# EUROPEAN PATENT SPECIFICATION

(11) **EP 0 960 114 B1**
(45) Date of publication and mention of the grant of the patent: **19.09.2007**
(21) Application number: 97910741.4
(22) Date of filing: 30.09.1997
(51) Int. Cl.: C12Q 1/68, C07H 21/04, C12N 15/63

(54) **POLYMORPHISMS AND NEW GENES IN THE REGION OF THE HUMAN HEMOCHROMATOSIS GENE**
POLYMORPHISMUS UND NEUE GENE IN DER REGION DES MENSCHLICHEN HEMOCHROMATOSISGENS
POLYMORPHISMES ET NOUVEAUX GENES DANS LA REGION DU GENE HUMAIN DE L'HEMOCHROMATOSE

(30) Priority: 01.10.1996 US 724394; 07.05.1997 US 852495
(43) Date of publication of application: 01.12.1999
(73) Proprietor: BIO-RAD LABORATORIES, INC., Hercules California 94547 (US)
(72) Inventor: FEDER, John, N., San Carlos, CA 94070 (US); KRONMAL, Gregory, S., Pacifica, CA 94044 (US); LAUER, Peter, M., San Francisco, CA 94131 (US); RUDDY, David, A., San Francisco, CA 94133 (US); THOMAS, Winston, J., San Mateo, CA 94402 (US); TSUCHIHASHI, Zenta, Menlo Park, CA 94025 (US); WOLFF, Roger, K., Mill Valley, CA 94941 (US)
(74) Representative: Jones Day
(86) International application number: PCT/US1997/017658
(87) International publication number: WO 1998/014466

(56) References cited:
- WO-A-97/38137
- FEDER ET AL: "A NOVEL MHC CLASS I-LIKE GENE IS MUTATED IN PATIENTS WITH HEREDITARY HAEMOCHROMATOSIS" NATURE GENETICS, NEW YORK, NY, US, vol. 13, August 1996 (1996-08), pages 399-408, XP002113585 ISSN: 1061-4036
- BEUTLER ERNEST ET AL: "Mutation analysis in hereditary hemochromatosis." BLOOD CELLS MOLECULES AND DISEASES, vol. 22, no. 2, 31 August 1996 (1996-08-31), pages 187-194, XP002227454 ISSN: 1079-9796
- GANDON GWENOLA ET AL: "Linkage disequilibrium and extended haplotypes in the HLA-A to D6S105 region: Implications for mapping the hemochromatosis gene (HFE)." HUMAN GENETICS, vol. 97, no. 1, January 1996 (1996-01), pages 103-113, XP002227451 ISSN: 0340-6717
- SEESE NICOLE K ET AL: "Localization of the hemochromatosis disease gene: Linkage disequilibrium analysis using an American patient collection." BLOOD CELLS MOLECULES AND DISEASES, vol. 22, no. 1, February 1996 (1996-02), pages 36-46, XP002227452 ISSN: 1079-9796
- RAHA-CHOWDHURY R ET AL: "New polymorphic microsatellite markers place the haemochromatosis gene telomeric to D6S105." HUMAN MOLECULAR GENETICS, OXFORD UNIVERSITY PRESS, SURREY, GB, vol. 4, no. 10, October 1995 (1995-10), pages 1869-1874, XP001024847 ISSN: 0964-6906
- GENOME RESEARCH, May 1997, Vol. 7, No. 5, RUDDY D.A. et al., "A 1.1-Mb Transcript Map of the Hereditary Hemochromatosis Locus", pages 441-456.
- SCIENCE, 04 September 1992, Vol. 257, FISCHER L. et al., "Cloning of the 62-Kilodalton Component of Basic Transcription Factor BTF2", pages 1392-1395.
- SCIENCE, 25 January 1991, Vol. 251, MARGOTTIN F. et al., "Participation of the TATA Factor in Transcription of the Yeast U6 Gene by RNA Polymerase C", pages 424-426.
- NATURE, 05 April 1990, Vol. 344, ZHENG X.M. et al., "Sequencing and Expression of Complementary DNA for the General Transcription Factor BTF3", pages 556-559.
- CLINICAL CHEMISTRY, February 1994, Vol. 40, No. 2, PANTEGHINI M., "Electrophoretic Fractionation of 5'-Nucleotidase", pages 190-196.
- GENOMICS, 15 April 1996, Vol. 33, No. 2, BURT M.J. et al., "A 4.5-Megabase YAC Contig and Physical Map over the Hemochromatosis Gene Region", pages 153-158.
- J. MOL. EVOL., November 1993, Vol. 37, No. 6, VERNET C. et al., "Evolutionary Study of Multigenic Families Mapping Close to the Human MHC Class I Region", pages 600-612.

## Description

### BACKGROUND OF THE INVENTION

Hereditary hemochromatosis (HH) is an inherited disorder of iron metabolism wherein the body accumulates excess iron. In symptomatic individuals, this excess iron leads to deleterious effects by being deposited in a variety of organs leading to their failure, and resulting in cirrhosis, diabetes, sterility, and other serious illnesses. The gene which is defective in this disease was disclosed in copending U.S.S.N. 08/652265, (Corresponds to US6025130, published 15.02.2000).

Fine structure mapping of the region to which the gene responsible for HH, HFE (denoted HH or HFE in some publications), was mapped makes possible the identification of candidate sequences comprising the HFE gene, along with structural elements for regulation and expression and neighboring genes.

A variety of techniques is available for fine structure mapping, including direct cDNA selection, exon-trapping, and genomic sample sequencing. The direct selection approach (Lovett et al. Proc Natl. Acad. Sci. U.SA. 88:9628-9623 (1991)) involves the hybridization of cDNA fragments to genomic DNA. This technique is extremely sensitive and capable of isolating portions of rare transcripts. Exon-trapping (Church et al. Nature Genetics 6:98-105 (1994)) recovers spliced introns from *in vivo* expressed genomic DNA clones and produces candidate exons without requiring any prior knowledge of the target's gene expression. High-throughput genomic DNA sequencing with comparison of the sequence data to databases of expressed sequences has also been used, such as in the positional cloning of the Werner syndrome gene (Yu et al. Science 277:258-262 (1996)) and in cloning by homology of the second Alzheimer's disease gene on chromosome 1 (Levy-Lahad et al. Science 269:973-977 (1995)).

HH is typically inherited as a recessive trait: in the current state of knowledge, homozygotes carrying two defective copies of the gene are most frequently affected by the disease. In addition, heterozygotes for the HFE gene are more susceptible to sporadic porphyria cutanea tarda and potentially other disorders (Roberts et al., Lancet 349:321-323 (1997). It is estimated that approximately 10-15% of Caucasians carry one copy of the HFE gene mutation and that there are about one million homozygotes in the United States. HH, thus, represents one of the most common genetic disease mutations in Caucasian individuals. Although ultimately HH produces debilitating symptoms, the majority of homozygotes and heterozygotes have not been diagnosed.

The need for such diagnostics is documented, for example, in Barton, J.C. et al. Nature Medicine 2:394-395 (1996); Finch, CA. West J Med 153:323-325 (1990); McCusick. V. Mendelian Inheritance in Man pp. 1882-1887, 11th ed., (Johns Hopkins University Press, Baltimore (1994)); Report of a joint World Health Omanization/Hemochromatosis Foundation/French Hemochromatosis Association Meeting on the Prevention and Control of Hemochromatosis (1993); Edwards. C.Q. et al. New Enol J Med 328:1616-1620 (1993): Bacon. B.R. New Engl J Med 326:126-127 (1992); Balan, V. et al. Gastroenterology 107:453-459 (1994); Phatak, P.D. et al. Arch Int Med 154:769-776 (1994).

A single mutation in the HFE gene, designated 24d1 in copending U.S.S.N. 08/630,912, gave rise to the majority of disease-causing chromosomes present in the population today. This is referred to herein as the "common" or "ancestral" or "common ancestral" mutation. These terms are used interchangeably. It appears that about 80% to 90% of all HH patients carry at least one copy of the common ancestral mutation which is closely linked to specific alleles of certain genetic markers close to this.ancestral HFE gene defect. These markers are, as a first approximation, in the allelic form in which they were present at the time the ancestral HFE mutation occurred. See, *for example,* Simon, M. et al. Am J Hum Genet 41:89-105 (1987); Jazwinska, E.C. et al. Am J Hum Genet. 53:242-257 (1993); Jazwinska, E.C. et al. Am J Hum Genet 56:428-433 (1995); Worwood, M. et al. Brit J Hematol 86:863-866 (1994); Summers, K.M. et al. Am J Hum Genet 45:41-48 (1989).

Several polymorphic markers in the HFE region have been described and shown to have alleles that are associated with HH disease. These markers include the published microsatellite markers D6S258, D6S306 (Gyapay, G. et al. Nature Genetics 7:246-339 (1994)), D6S265 (Worwood, M. et al. Brit J. Hematol 86:833-846 (1994)), D6S105 (Jazwinska, E.C. et al. Am J Hum Genet 53:242-257 (1993); Jazwinska, E.C. et al. Am J Hum Genet 56:428-433 (1995)), D6S1001 (Stone, C. et al. Hum Molec Genet 3:2043-2046 (1994)), D6S1260 (Raha-Chowdhury et al. Hum Molec Genet-4:1869-1874 (1995)) as well as additional microsatellite and single-nucleotide-polymorphism markers disclosed in co-pending PCT application WO 96/06583, Additionally, copending U.S.S.N. 08/630,912 disclosed additional markers 24d2 and 24d7.

The symptoms of HH are often similar to those of other conditions, and the severe effects of the disease often do not appear immediately. Accordingly, it would be desirable to provide a method to identify persons who may be destined to become symptomatic in order to intervene in time to prevent excessive tissue damage associated with iron overload. One reason for the lack of early diagnosis is the inadequacy of presently available diagnostic methods to ascertain which individuals are at risk, especially while such individuals are presymptomatic.

Although blood iron parameters can be used as a screening tool, a confirmed diagnosis often employs liver biopsy which is undesirably invasive, costly, and carries a risk of mortality. Thus, there is a clear need for the development of an inexpensive and noninvasive diagnostic test for detection of homozygotes and heterozygotes in order to facilitate diagnosis in symptomatic individuals, provide presymptomatic detection to guide intervention in order to prevent organ damage, and for identification of heterozygote carriers.

Furthermore, a need exists for both methods for fine structure mapping and a fine structure map of the region of the chromosome to which the HH focus maps. This and other needs are addressed by the present invention.

### SUMMARY OF THE INVENTION

One aspect of the invention is an oligonucleotide consisting of at least 8 to about 100 consecutive bases from the sequence of SEQ ID NO: 21, or its complement, including polymorphic site at base 35935 of SEQ ID NO: 21 ("polymorphic site C182.1G7").

Another aspect of the invention is an oligonucleotide pair consisting of SEQ ID NO: 40 and SEQ ID NO: 41 for amplification of an nucleic acid sequence from SEQ ID NO: 21 or its complement that includes the polymorphic site at base 35935 of SEQ ID NO: 21 ("polymorphic site C182.107").

Another aspect of the invention is an *in vitro* method to determine the present or absence of the common hereditary hemochromatosis (HFE) gene mutation in a sample of DNA or RNA comprising:
assessing the DNA or RNA for the present or absence of a haplotype comprising the allele of SEQ ID NO: 21 or its complement at polymorphic site at base 35935 of SEQ ID NO: 21 ("polymorphic site C182.1G7"),
wherein the absence of the haplotype indicates the likely absence of the HFE mutation in the genome from which the sample has been obtained and the presence of the haplotype indicates the likely presence of the HFE gene mutation in the genome from which the sample has been obtained.

Another aspect of the invention is an *in vitro* method to determine the presence or absence of the common hereditary hemochromatosis (HFE) gene mutation in a sample of DNA or RNA comprising:
assessing the DNA or RNA for the presence or absence of a G genotype at polymorphic site at base 35935 of SEQ ID NO: 21 ("polymorphic site C182.1 G7") or a C geneotype at the complementary site,
wherein the absence of the C or G genotype at polymorphic site C182.1G7 indicates the likely absence of the HFE gene mutation in the genome from which the sample has been obtained and the presence of the C or G genotype at polymorphic site C182.1G7 indicates the likely presence of the HFE gene mutation in the genome from which the sample has been obtained.

### BRIEF DESCRIPTION OF THE DRAWINGS

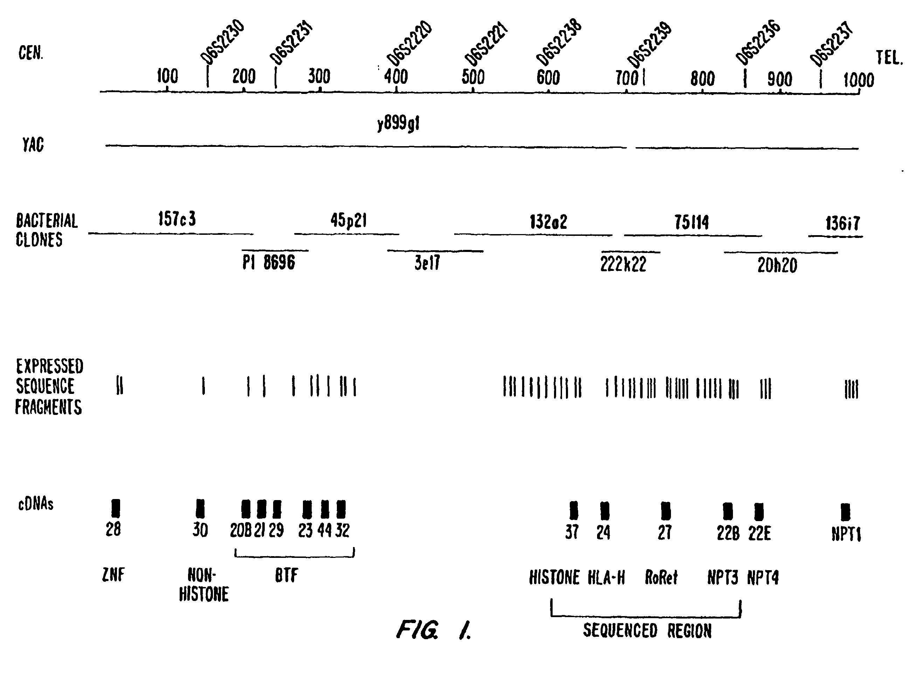

Figure 1 depicts a combination genetic, physical and transcription map of the HFE gene region. The first line shows the relative positions of selected genetic markers that define the HFE region. The heavy bar below represents the YAC clone used in the direct selection experiment. The order and positions af the bacterial clones employed in the exon-trapping and sample sequencing is indicated under the YAC. The thin bar under the bacterial clones represents the approximate locations of a subset of the expressed sequence fragments mapped to the contig. The thicker bars show the location of the cDNAs cloned. Two regions are bracketed: the butyrophilin family of genes (BTF), and the region where complete genomic sequencing was carried out

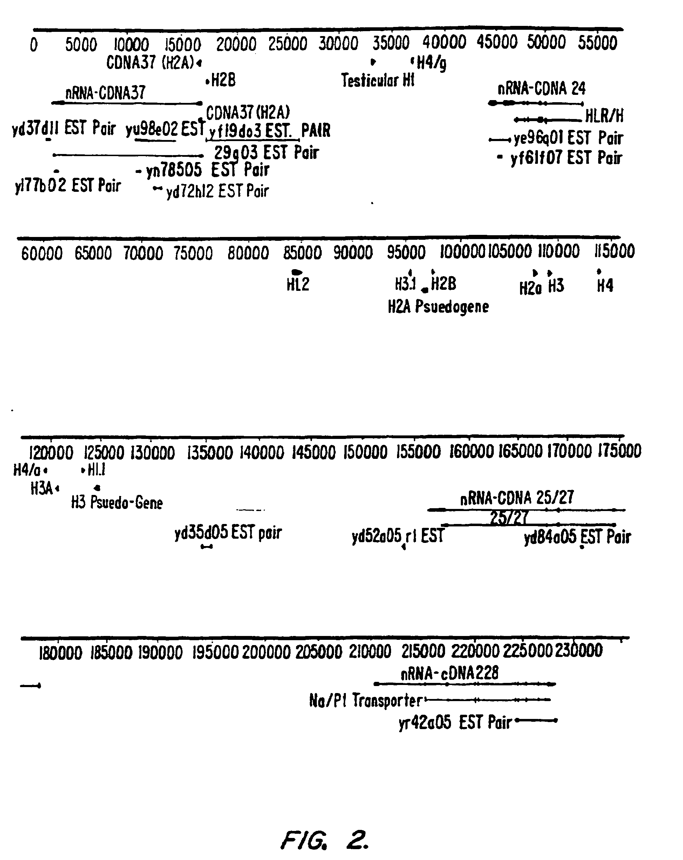

Figure 2 is a schematic of the 250 of genomic sequence including the HFE gene. genomic sequence including the HFE gene. Both the structure of the overall cDMA (top) end that corresponding to the coding regions (bottom), as wall as the direction of transcription are shown. The positions of the histone genes, the zinc a-2 glycoprotein pseudogene, and the ESTs are also shown.

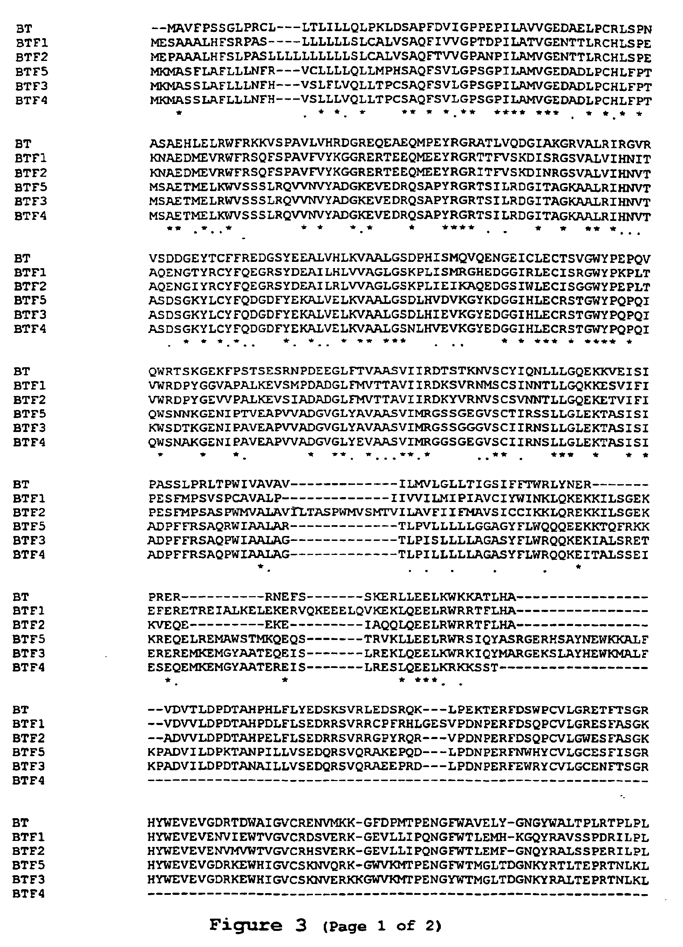

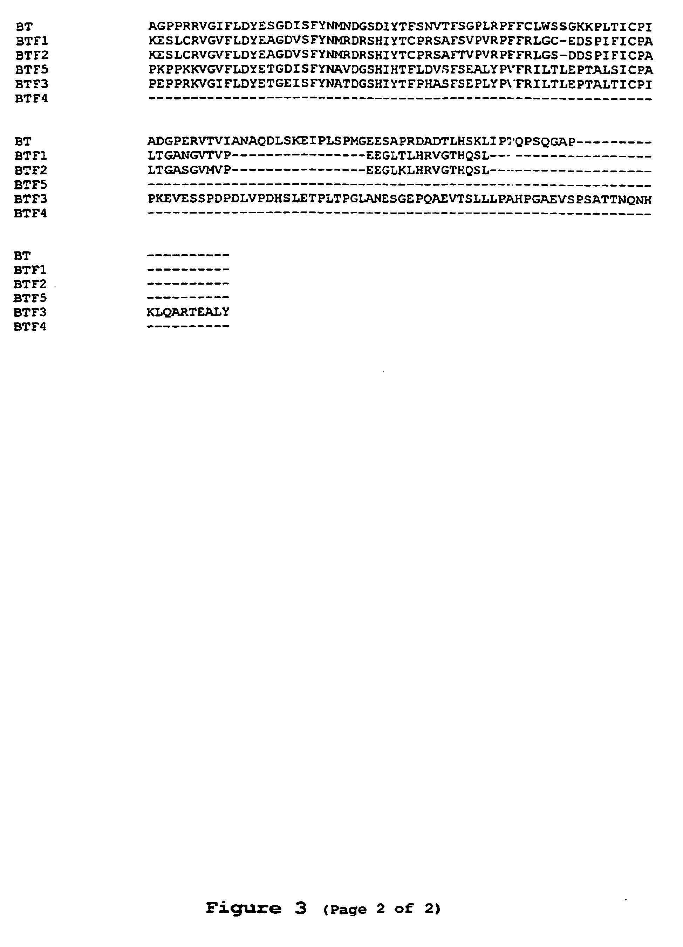

Figure 3 depicts an alignment of the predicted amino acid sequence of the BTF proteins. Sequences were aligned in a pair-wise fashion using CLUSTAL W (Thompson of al. Nucl. Acids Res. 22:4673-4680) to deduce the most parsimonious arrangement. The asterisks under the alignment represent amino acids conserved in all 6 proteins; the "dots" represent conserved amino acids substitutions. Boxed are the regions within the proteins which correspond to three conserved motifs: 1) the B-G domain, 2) the transmembrane domain (TM), and 3) the B30-2 exon domain.

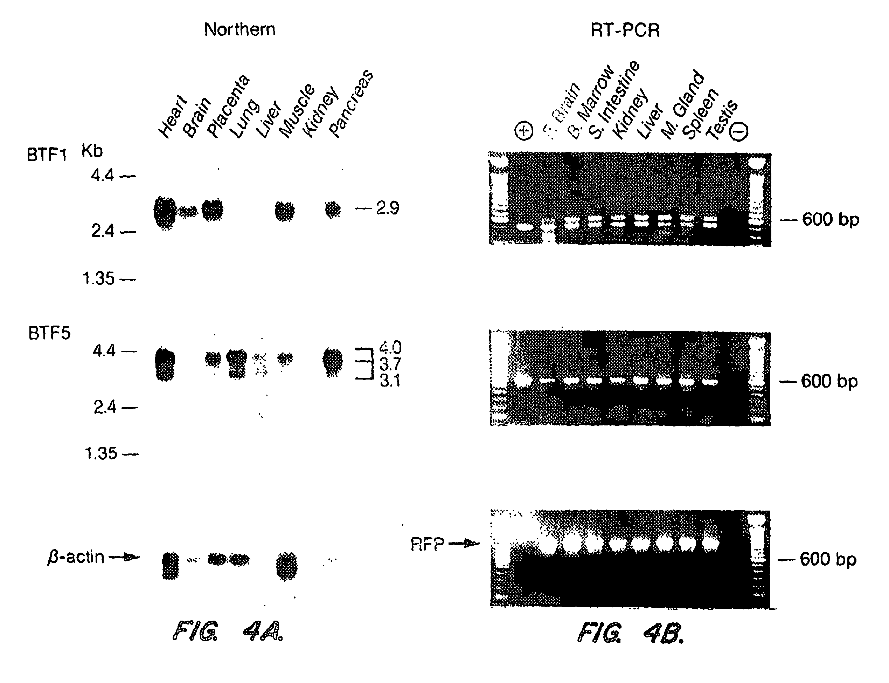

Figure 4, panel (A) depicts a Northern blot analysis of representative members of the two groups of BTF proteins, BTF1 and BTF5. BTF1 hybridized to all tissues on the blot as a major transcript at 2.9 kb and a minor one at 5.0 kb. BTF5 hybridized to several transcripts ranging between 4.0 and 3.1 kb and as a similar expression profile to BTF1. Autoradiography was for 24 hours. The β-actin hybridization demonstrated the variation in ploy (A)⁺ RNA between the lanes. Autoradiography was for 1 hour. In panel (B), RT-PCR analysis demonstrated that the expression of both genes was widespread. Included in the (+) lane are cDNA 21 and 44 as positive controls; the (-) lane represents the no-DNA control. Amplification using primers for the RFP gene (Isomura et al. Nucleic Acid Res, 20:5305-5310 (1992)) controlled for the integrity of the cDNA. All first strand cDNAs were checked for contaminating genomic DNA amplification by carrying out an identical experiment excluding the reverse transcriptase. In all cases, no amplification was obtained (data not shown).

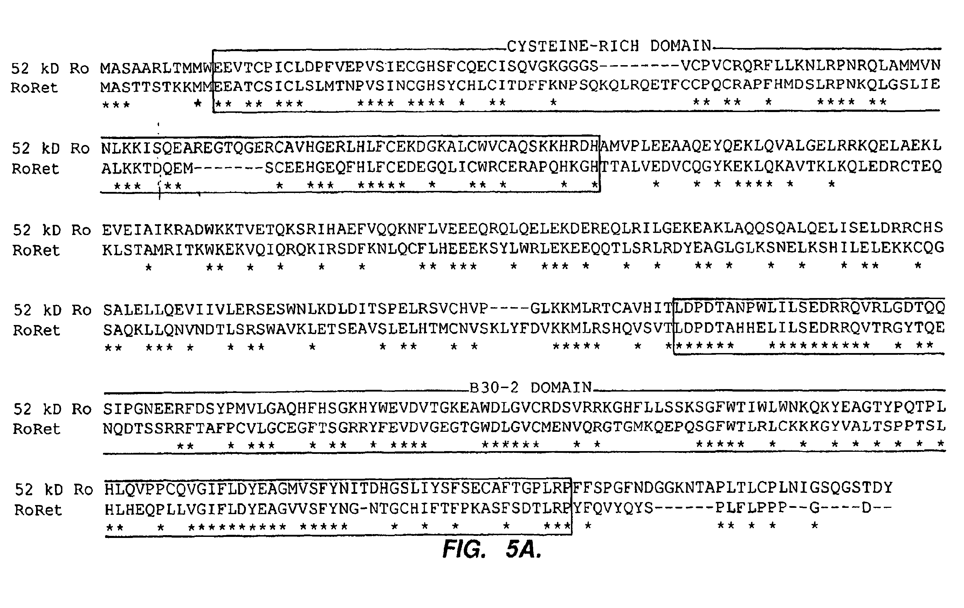

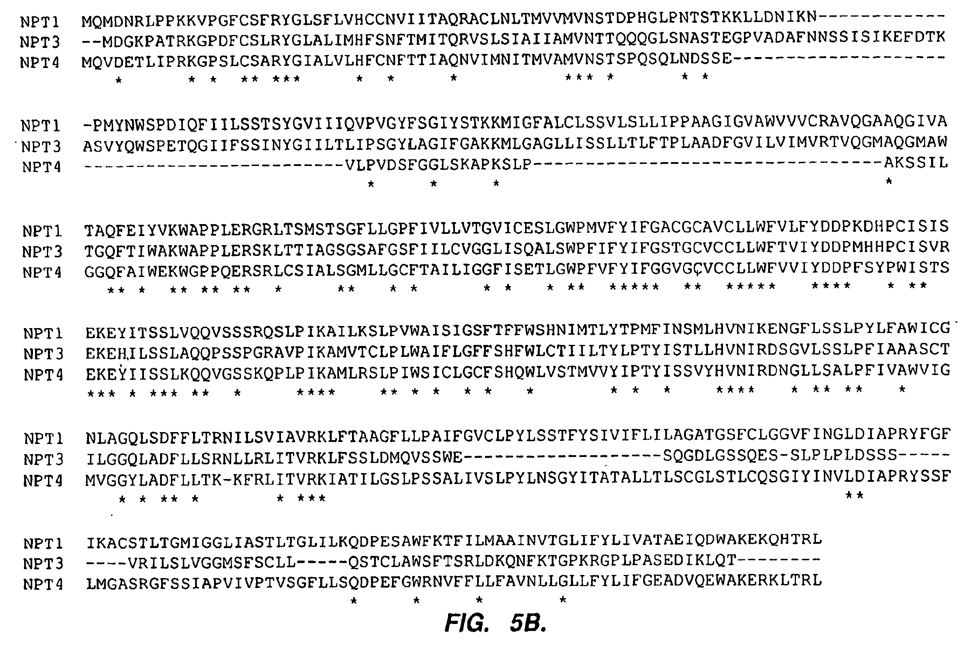

Figure 5(A) depicts an alignment of the predicted amino acid sequence of the RoRet gene to the 52 kD Ro/SSA auto-antigen protein. The asterisks under the alignment represent conserved amino acids; the "dots" represent conserved amino acids substitutions. The putative DNA binding cysteine-rich domain and the B30-2 exon domain are boxed. Figure 5(B) depicts an alignment of the predicted amino acid sequence of the two novel putative sodium phosphate transport proteins to that of the NPT1.

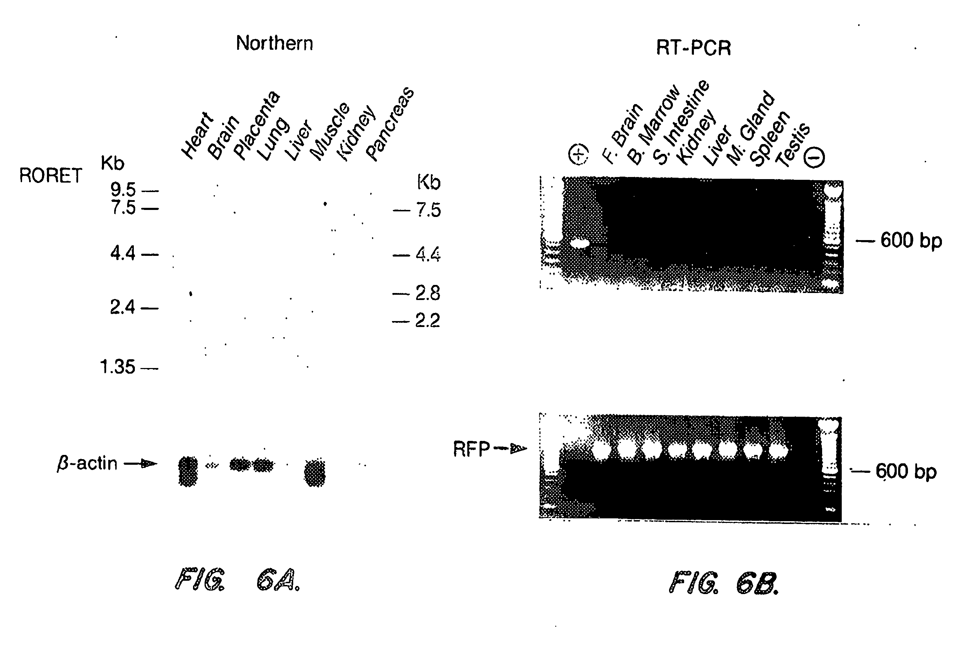

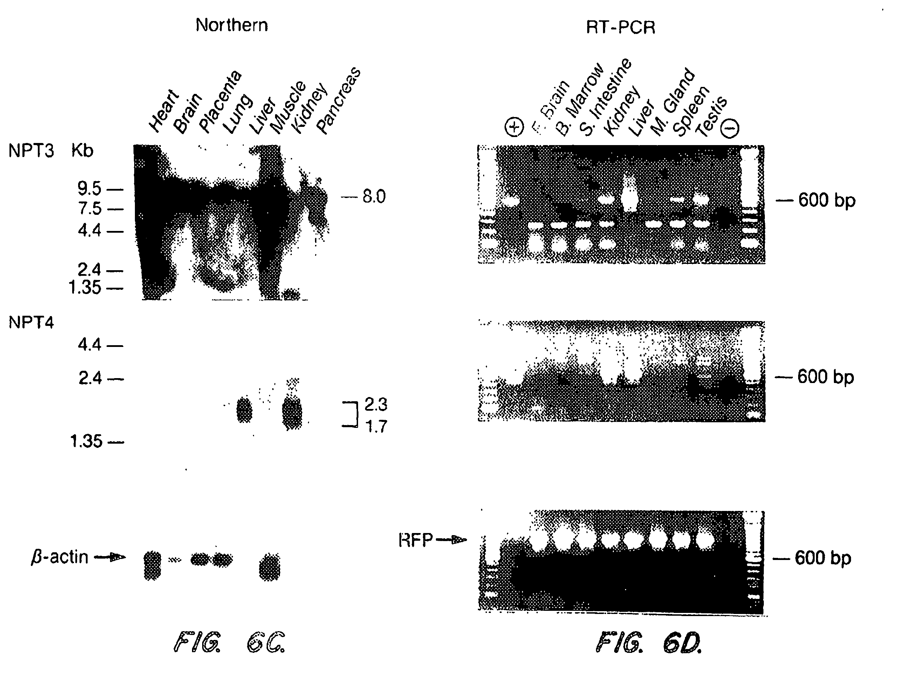

Figure 6, panel (A) depicts a Northern blot analysis of the RoRet gene. The RoRet cDNA hybridized to 4 different transcripts, ranging from 7.1 kb to 2.2 kb. Autoradiography was performed for 4 days. The re-hybridization of the blot with a β-actin probe showed the variation in poly (A)⁺ RNA between the lanes. Autoradiography was for 1 hour. Panel (B) depicts RT-PCR analysis of the RoRet gene. Included in the (+) lane was a cDNA 27 positive control. Weak amplification of the correct size was observed in the small intestine, kidney and liver. The other tissues were negative as was the no DNA control lane (-). The RFP primers demonstrated the integrity of the cDNA. Panel (C) depicts Northern blot analysis of NPT3 and NPT4. NPT3 was expressed at high abundance in the heart and muscle as a single 7.2 kb transcript. Lesser amounts were found in the other tissues. The expression pattern of NPT4 was more restricted, being found only in the liver and kidney as a smear of transcripts ranging from 2.6 to 1.7 kb. Panel (D) depicts RT-PCR analysis of the NPT3 and NPT4 genes. Included in the (+) lane were the respective cDNA22E and 22B positive controls. The NPT3 gene was expressed as the proper size PCR fragment in kidney, liver, spleen and testis. A smaller fragment was detected in all tissues with the exception of the liver. The no DNA control lane (-) was negative. NPT4 was expressed as the proper size fragment in the small intestine, kidney, liver and testis. Larger and smaller size fragments were found in all other tissues with the exception of the brain. For both genes these different size fragments may indicate alternative splice events. The no DNA control lane (-) was negative. The RFP primers demonstrated the integrity of the cDNA.

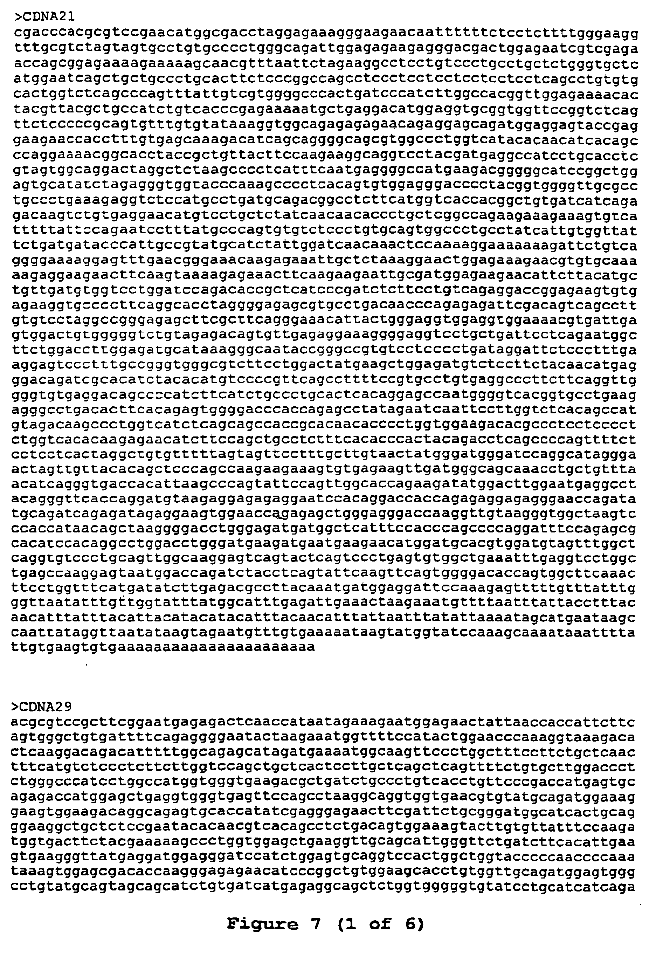

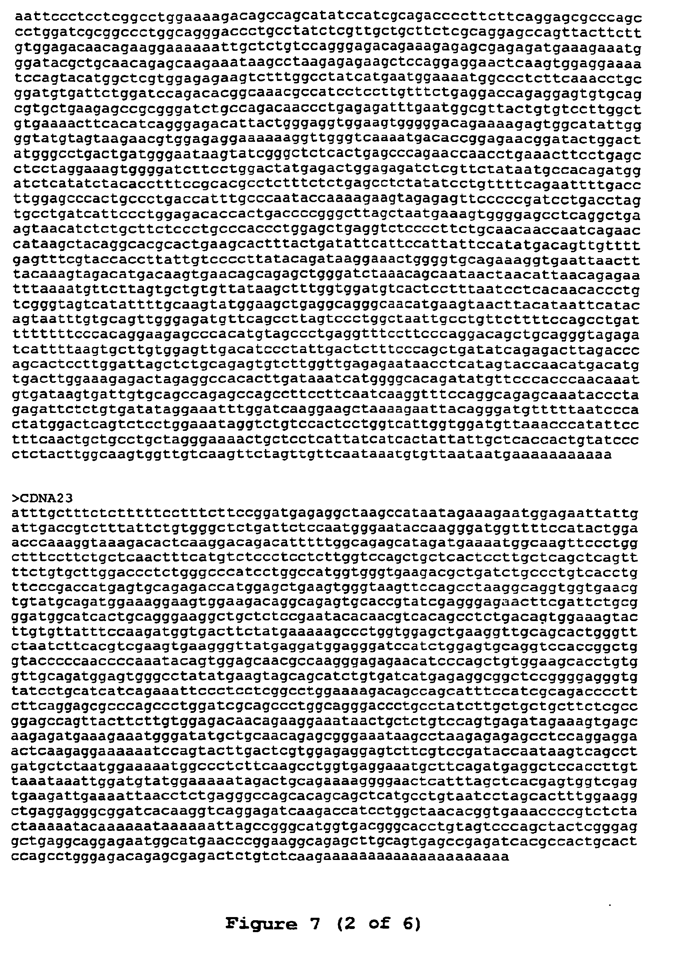

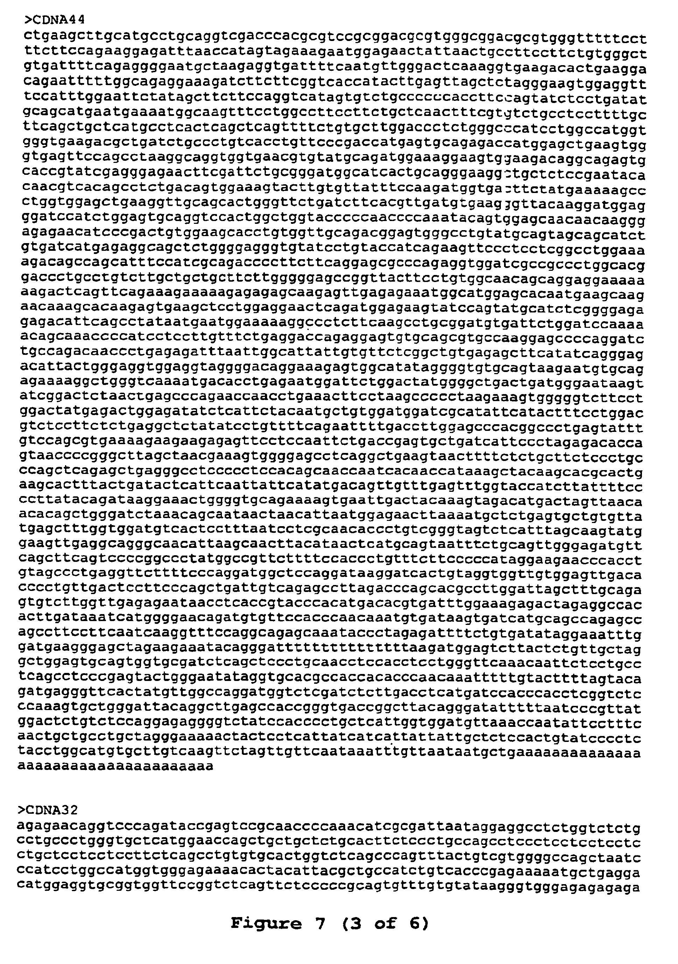

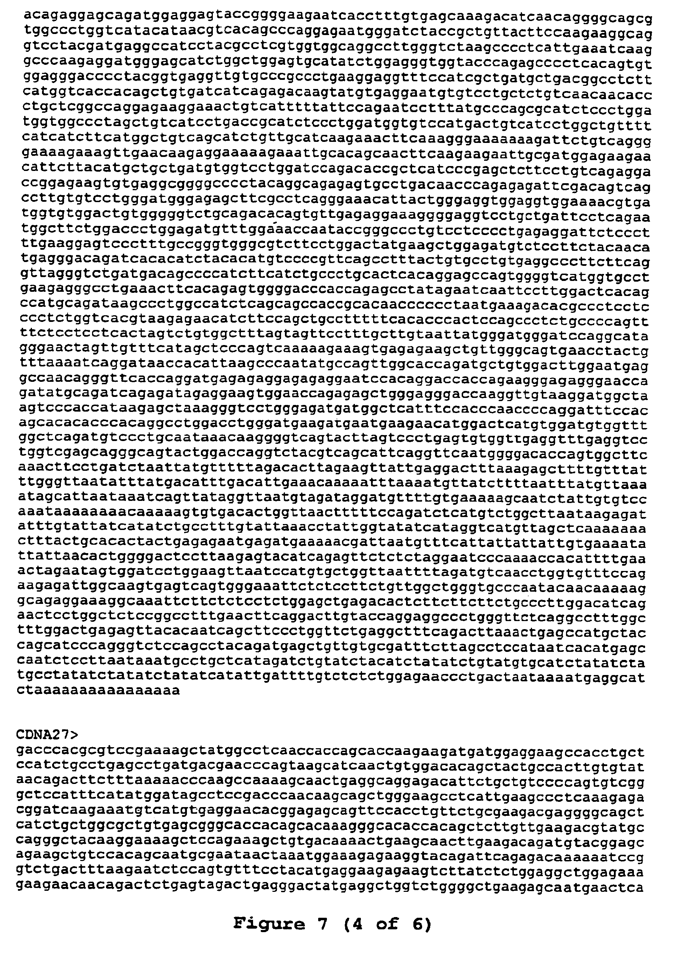

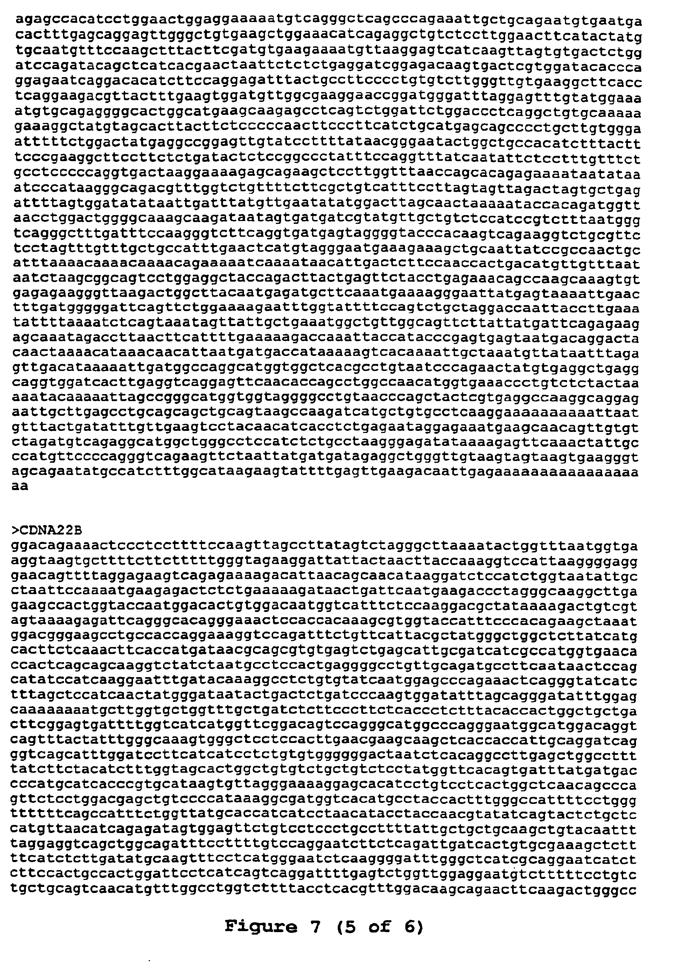

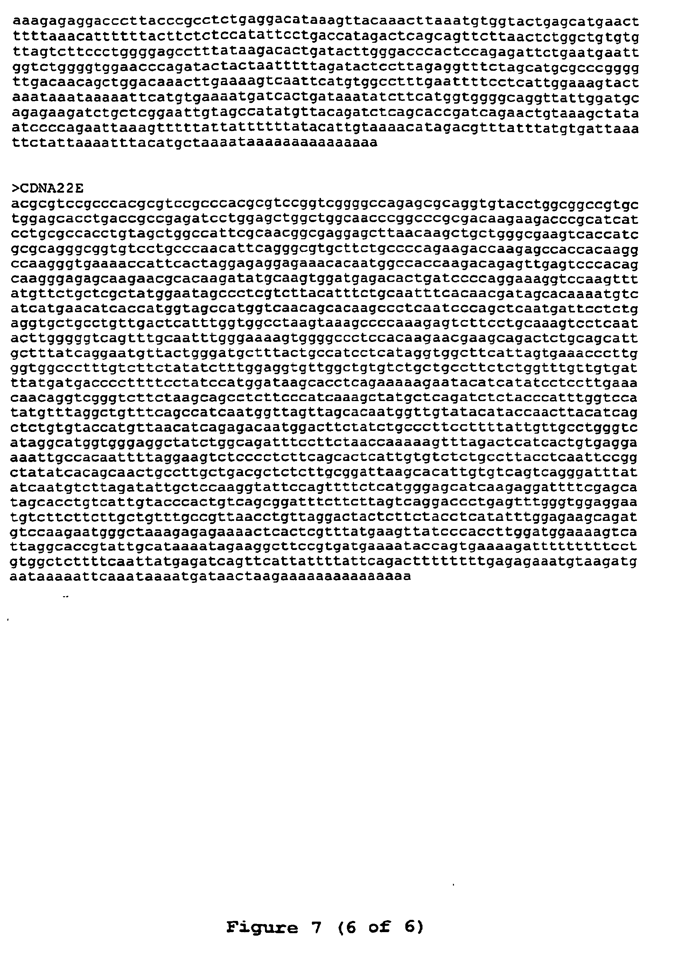

Figure 7 depicts the sequences of cDNA 21 (BTF1), cDNA 29 (BTF3), cDNA 23 (BTF4), cDNA 44 (BTF5), cDNA 32 (BTF2), cDNA 27 (RoRet), cDNA 22B (NPT3), cDNA22E (NPT4).

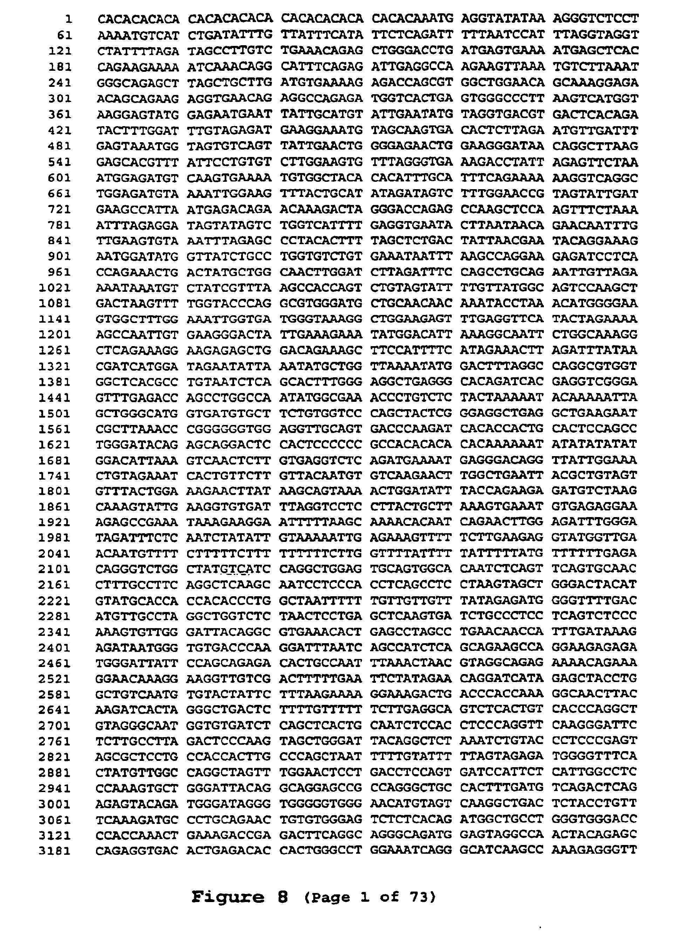

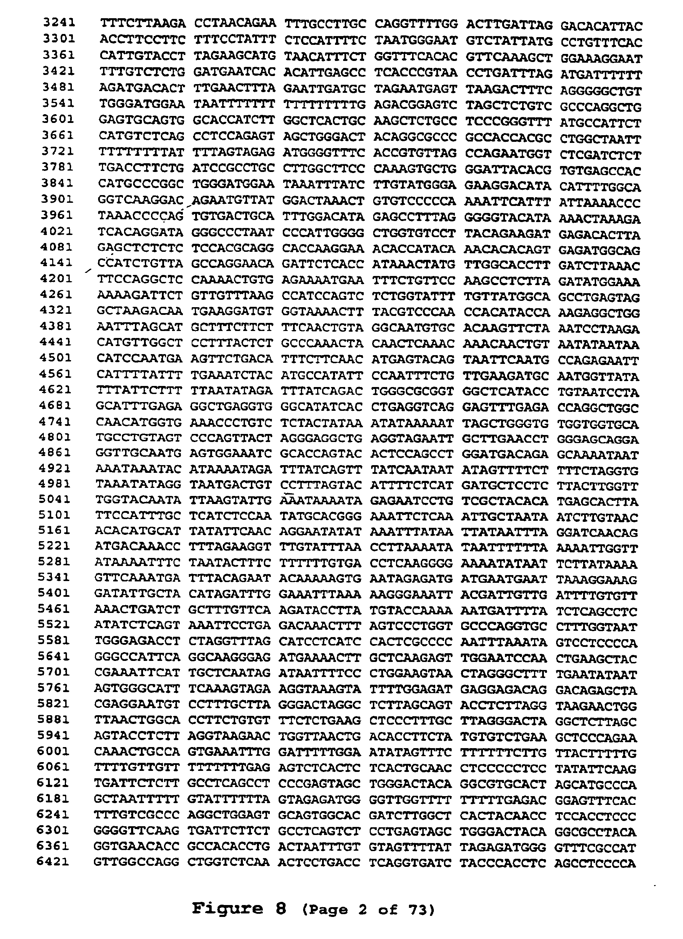

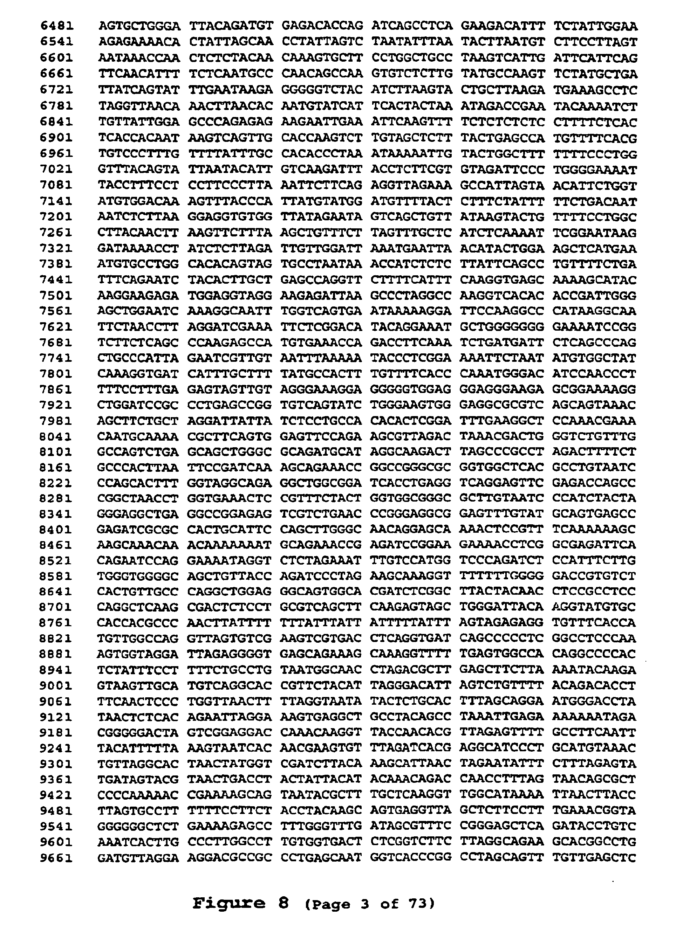

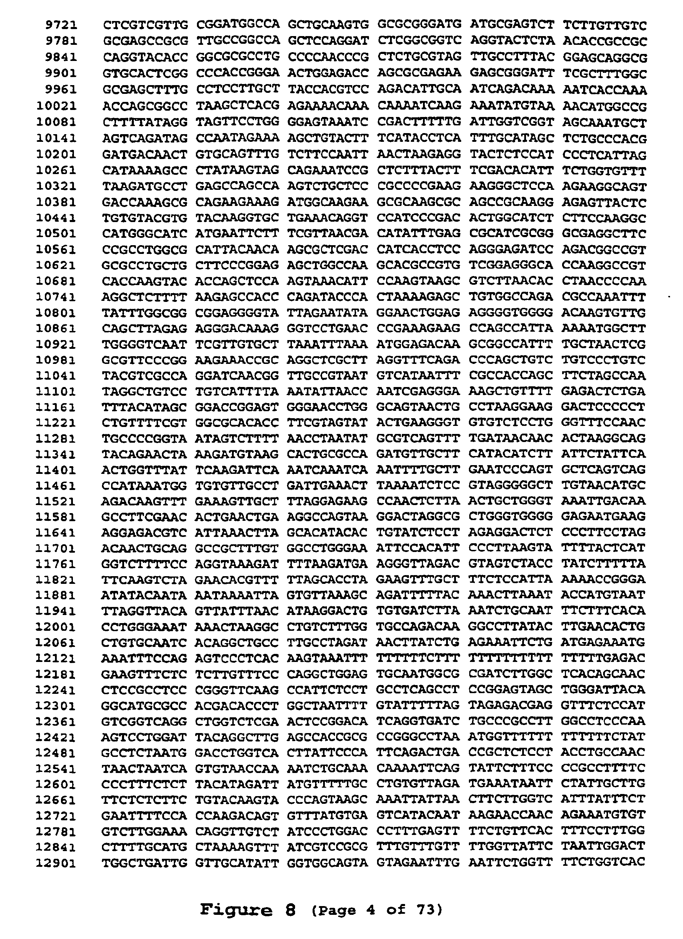

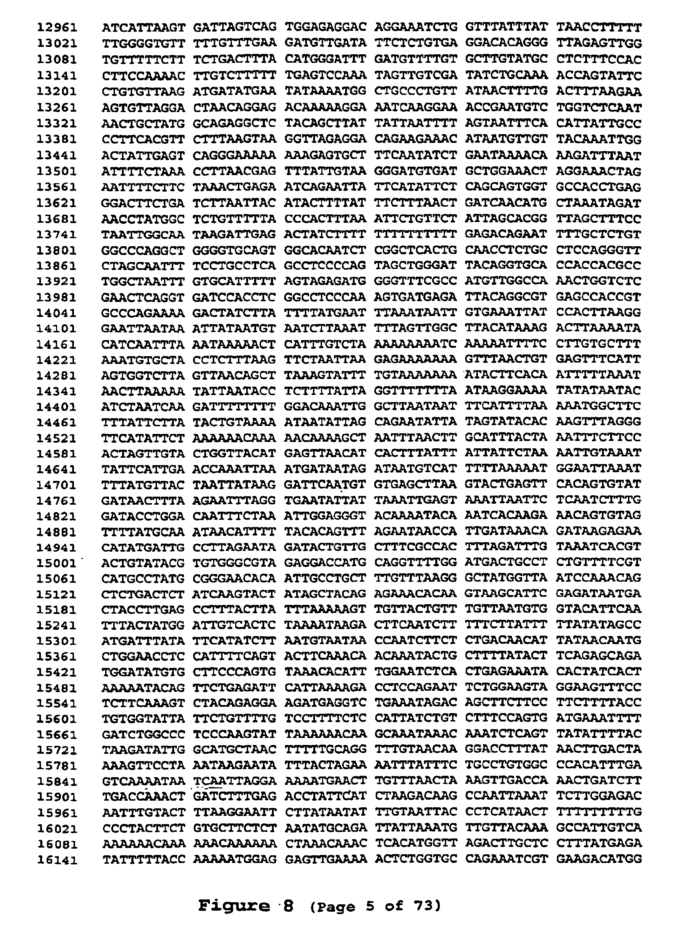

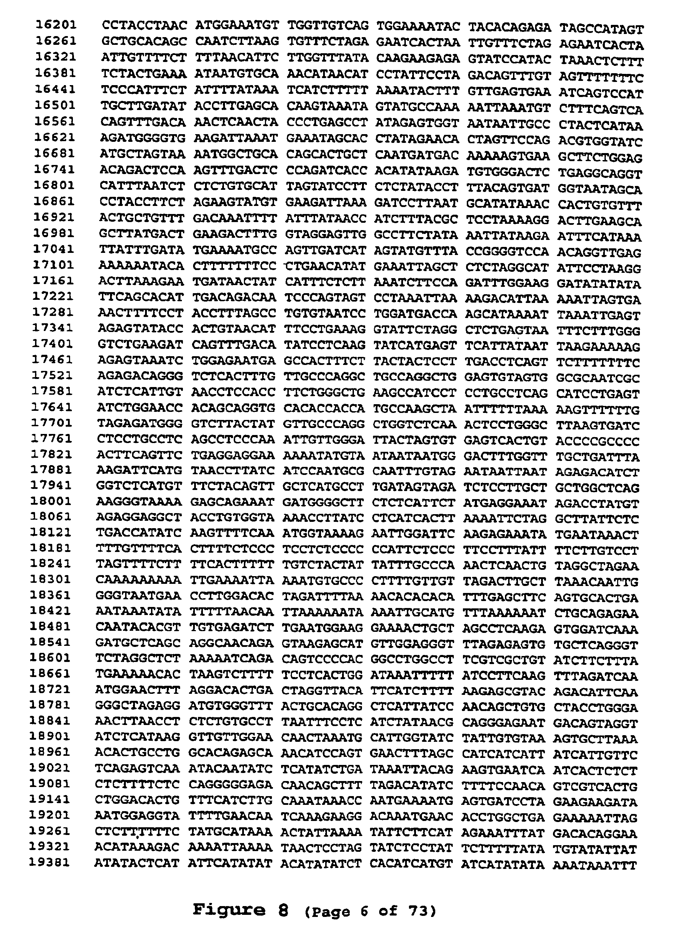

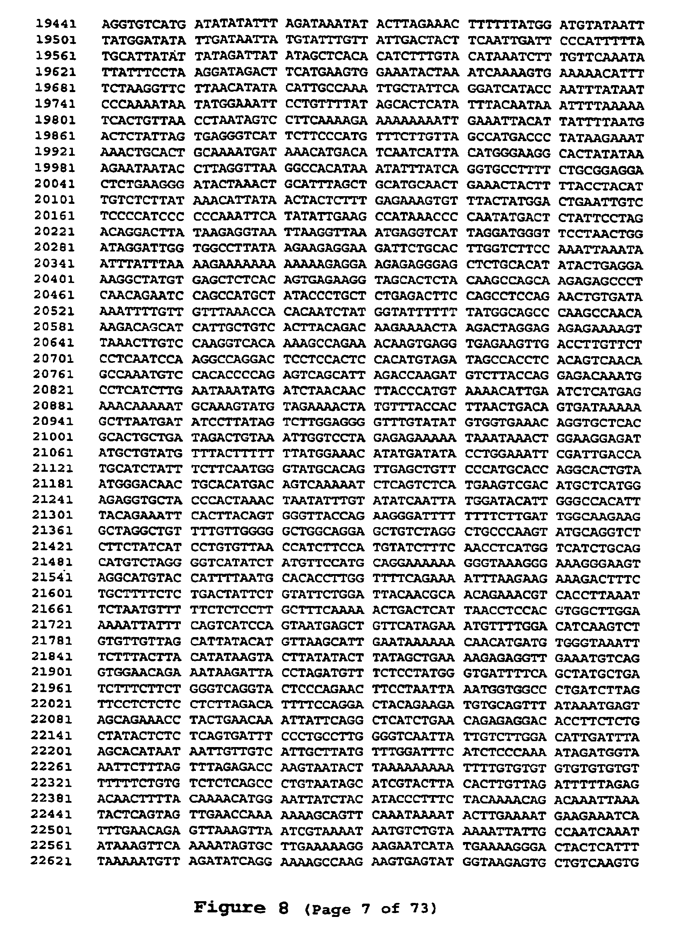

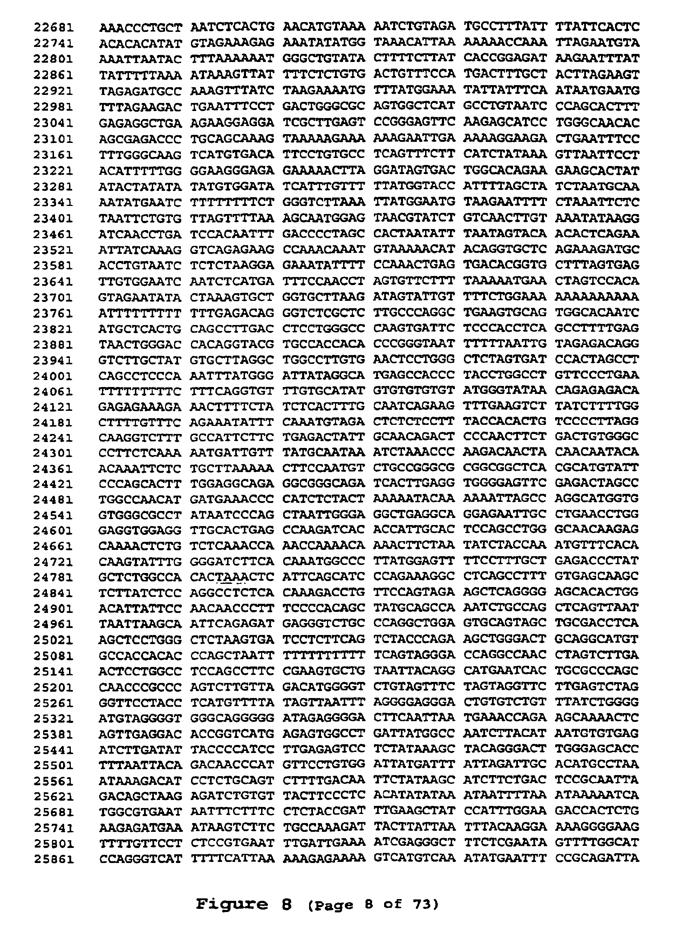

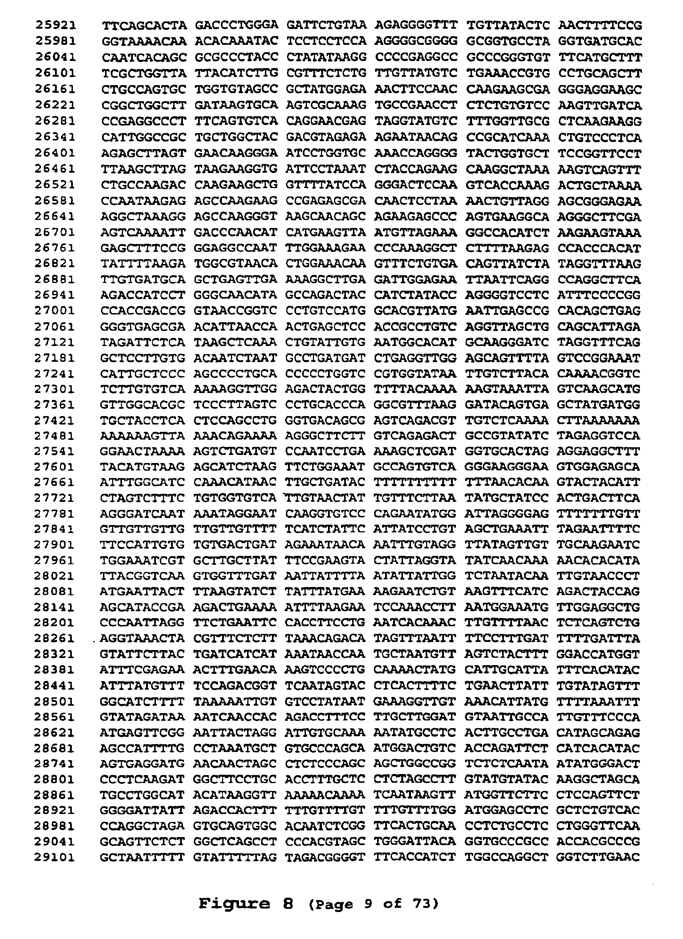

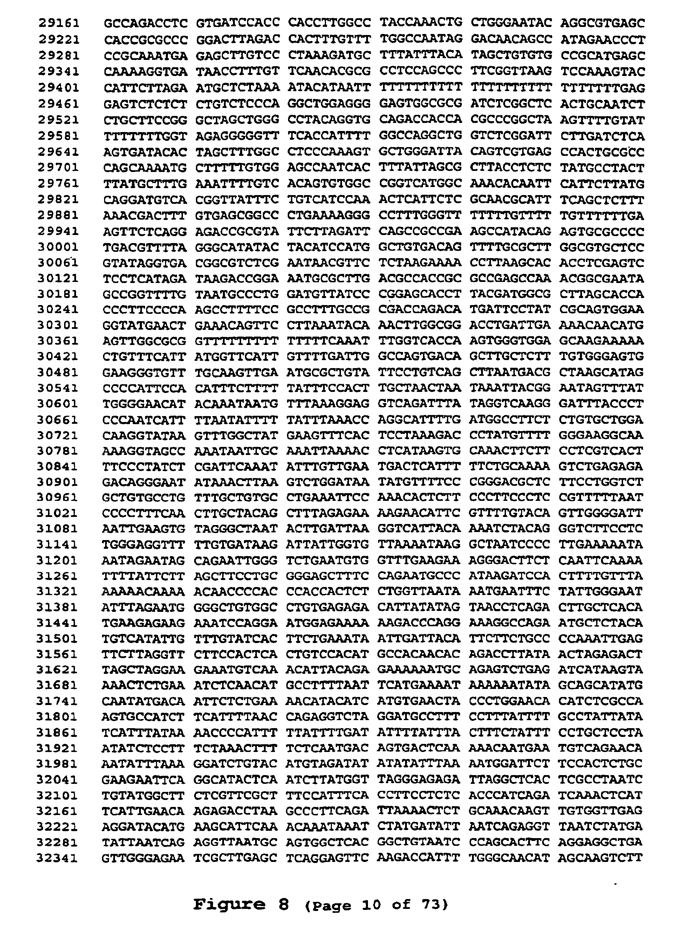

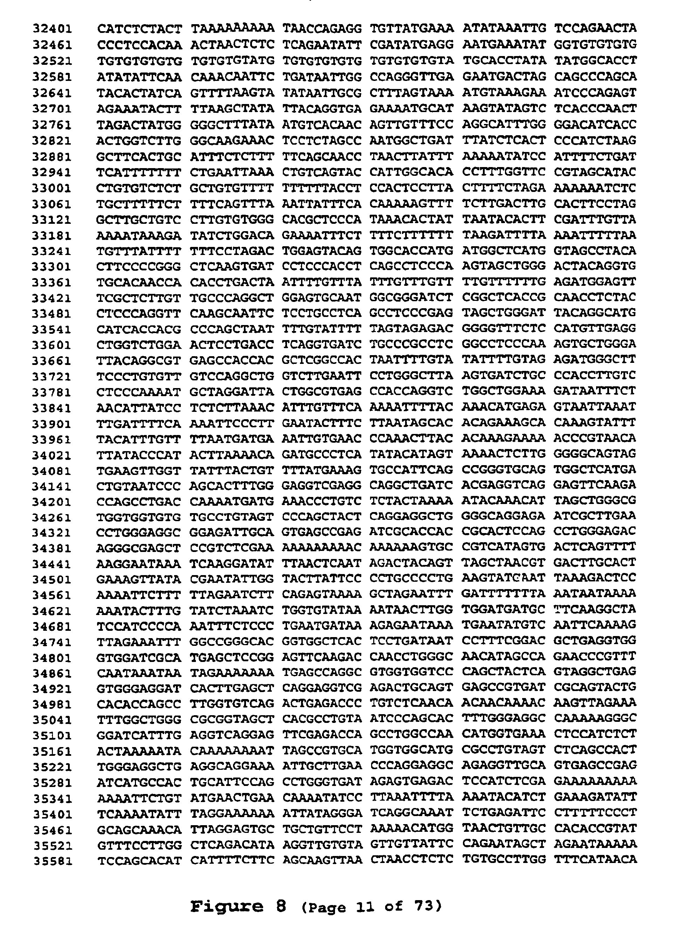

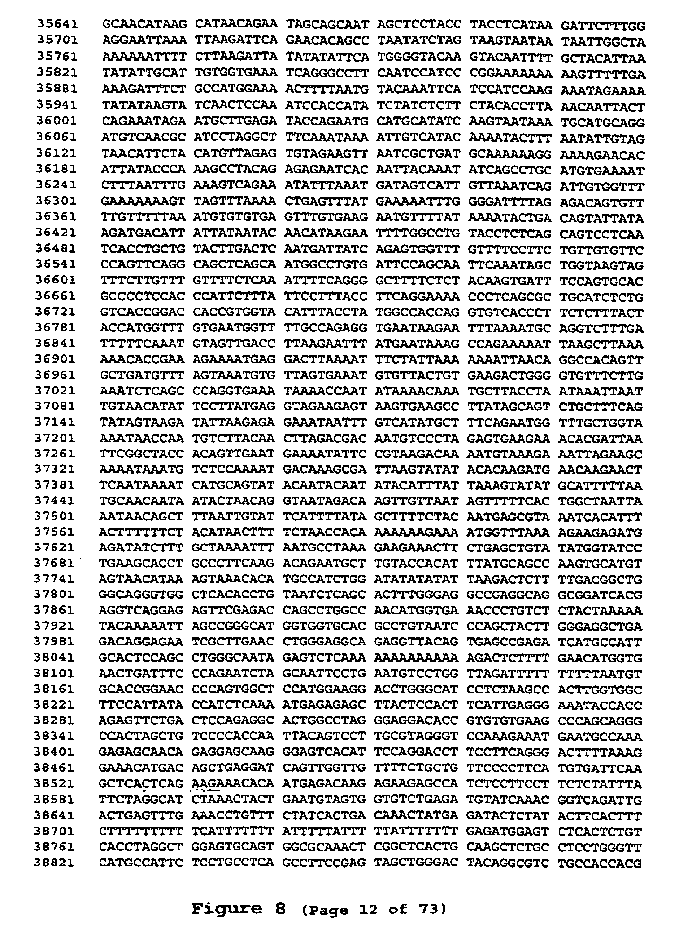

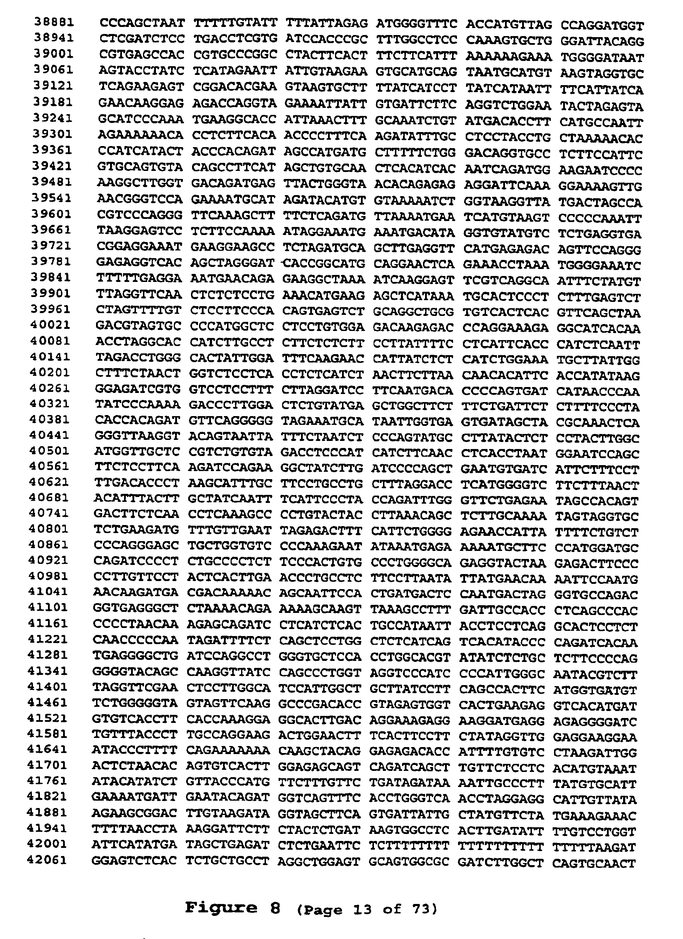

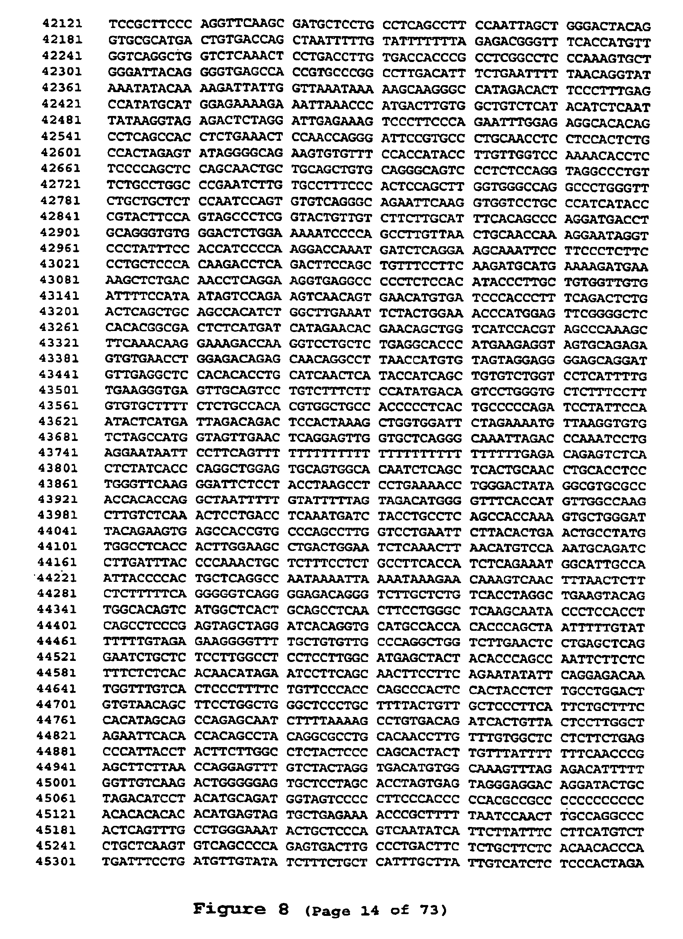

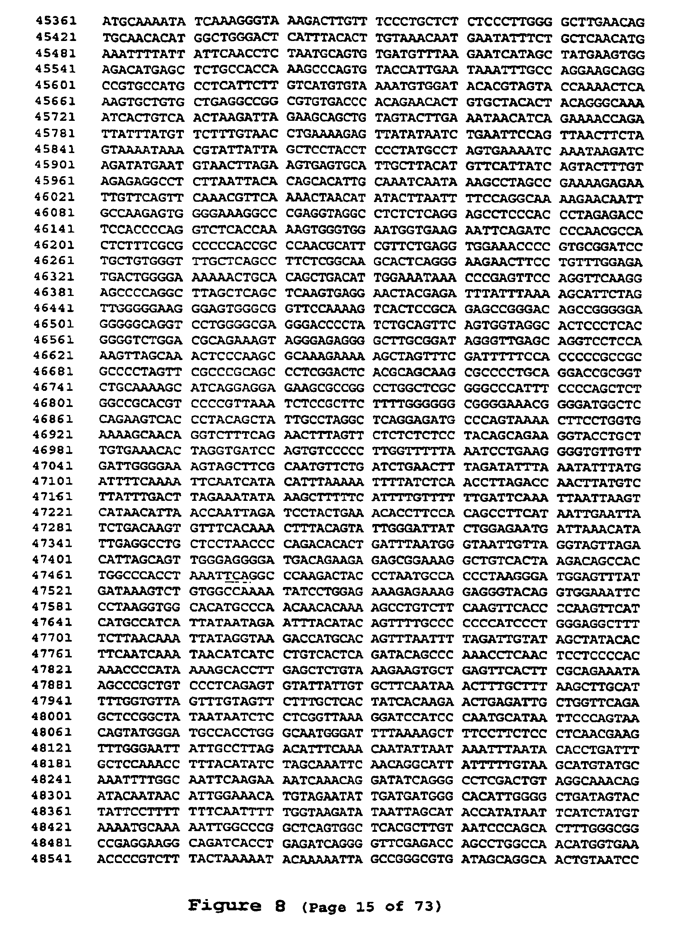

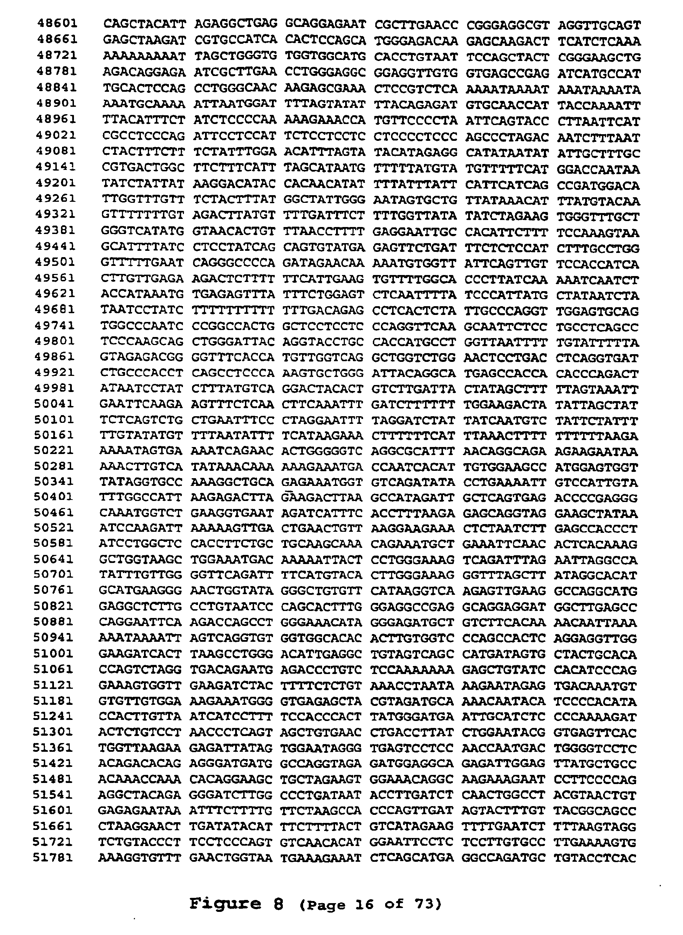

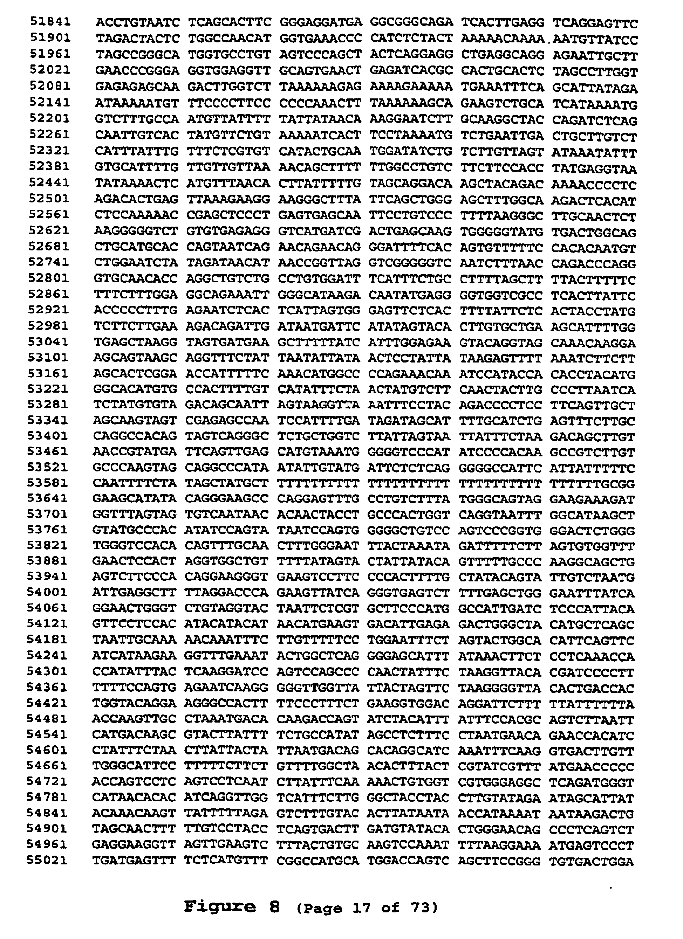

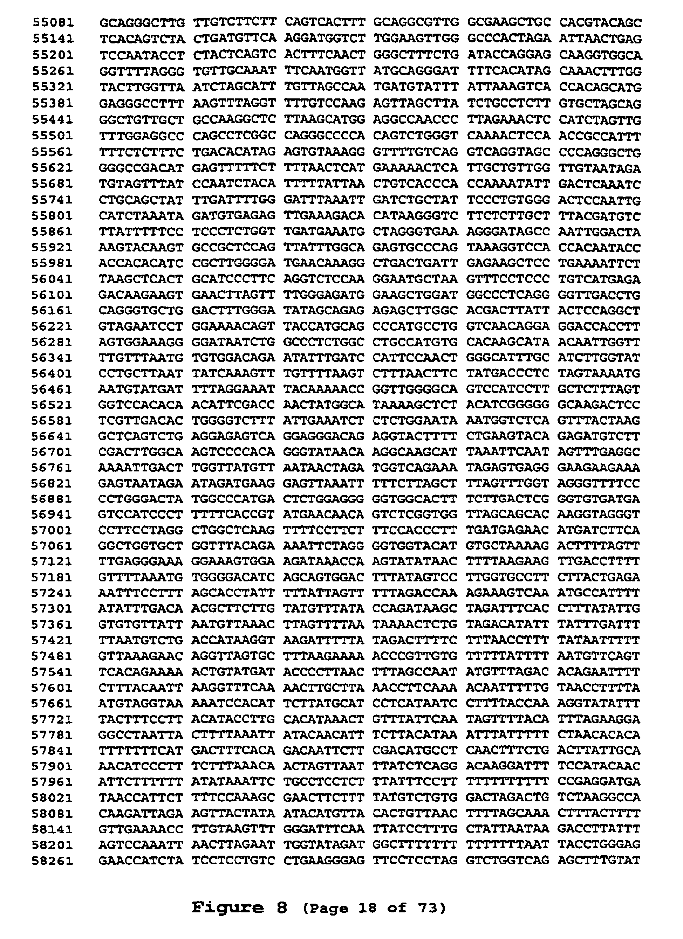

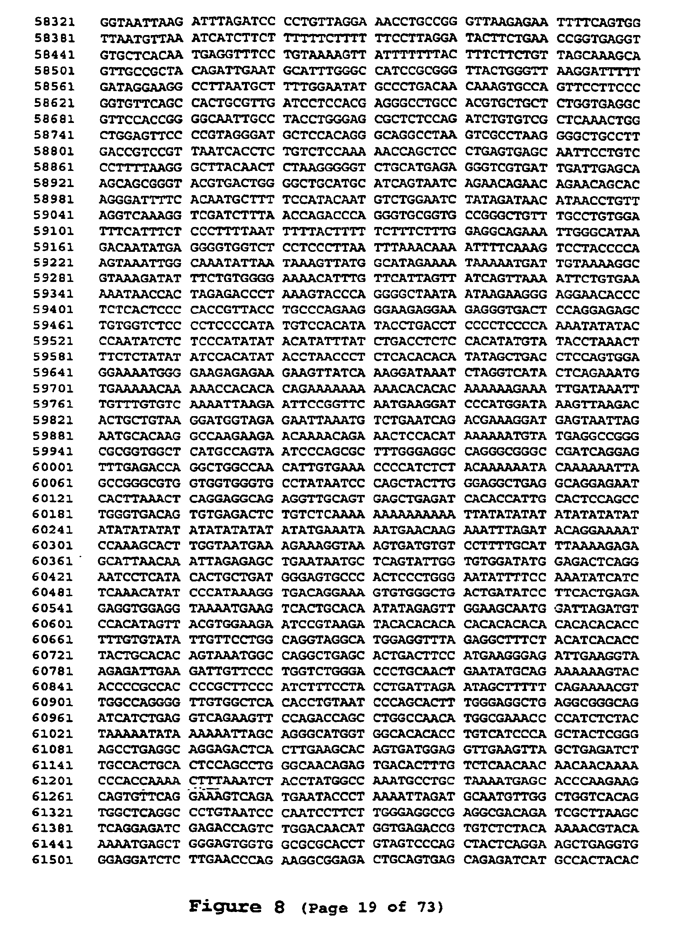

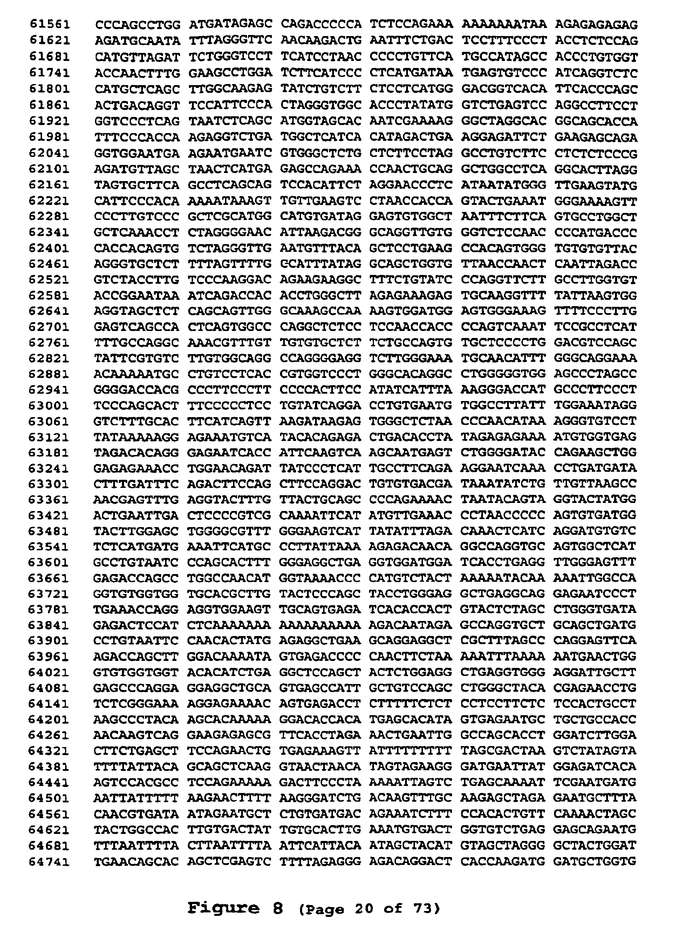

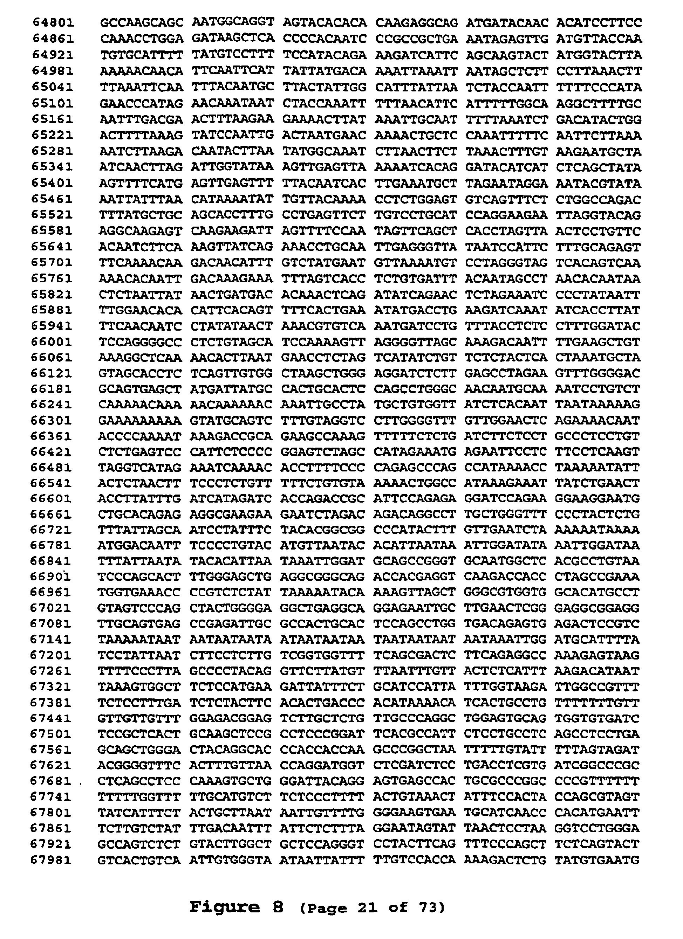

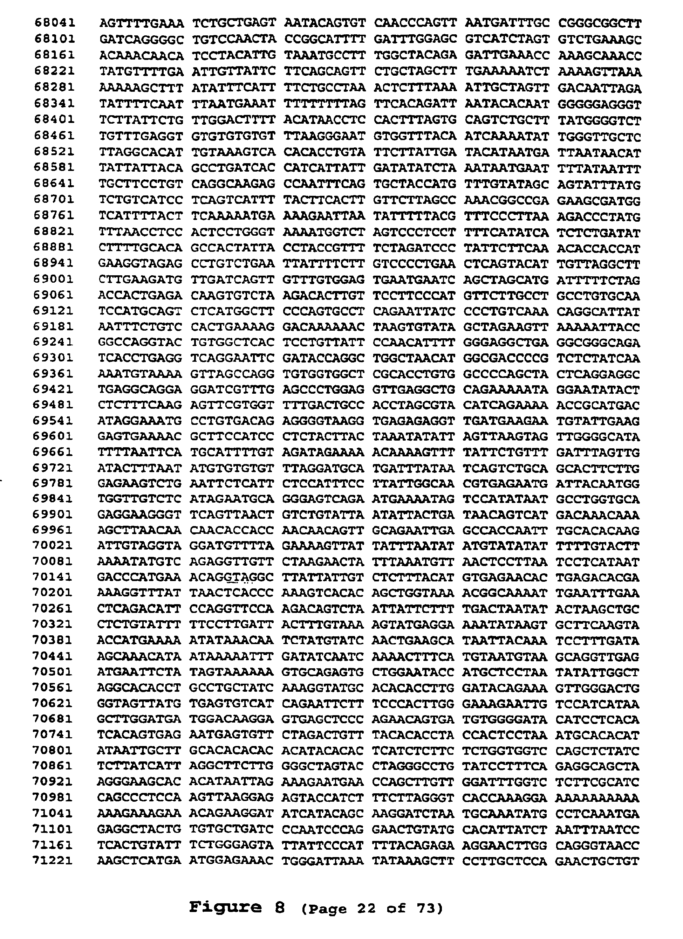

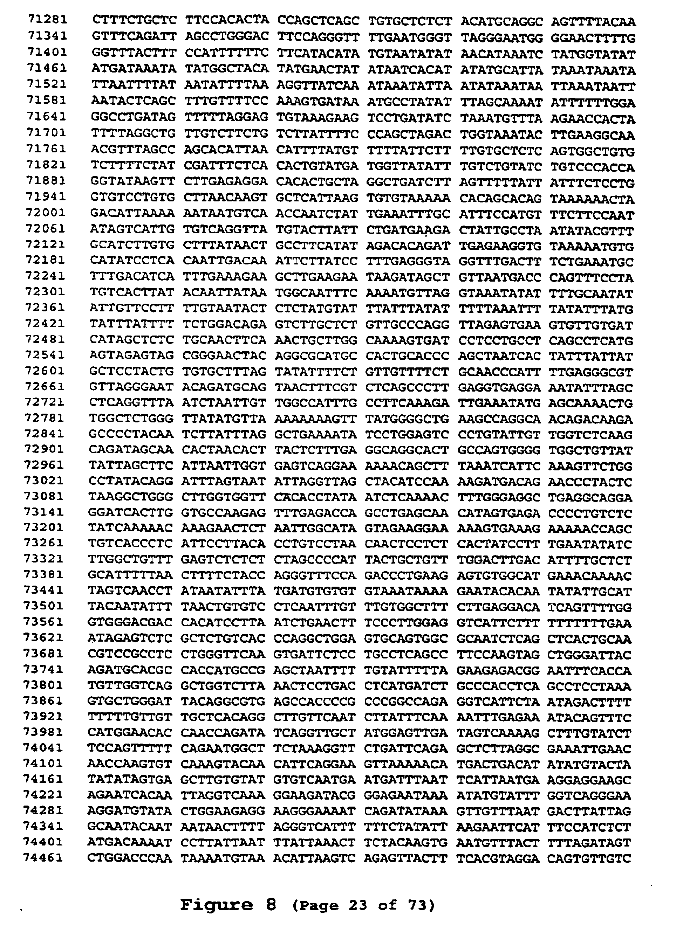

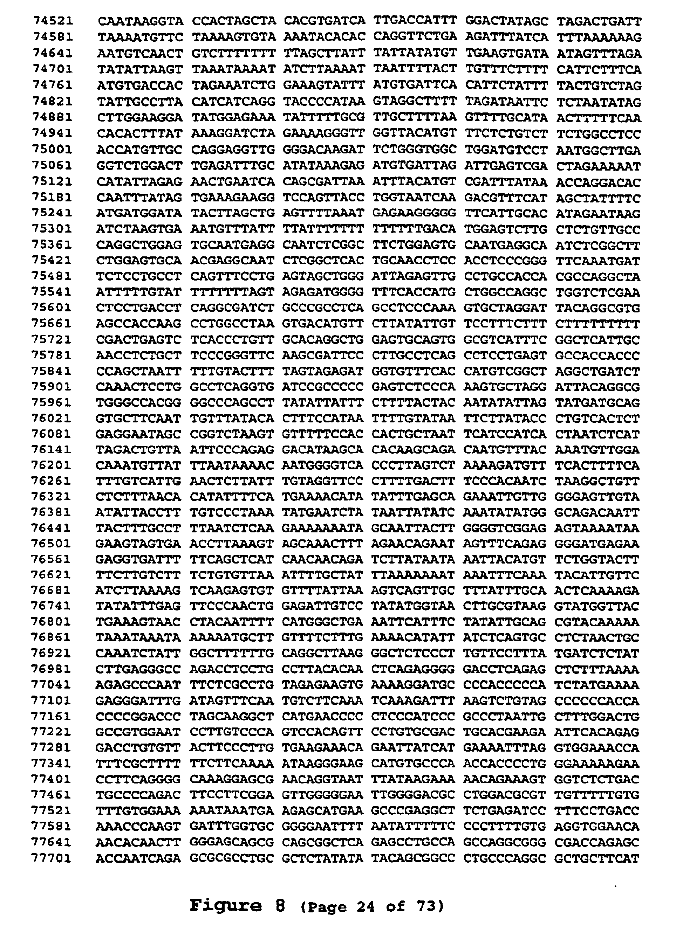

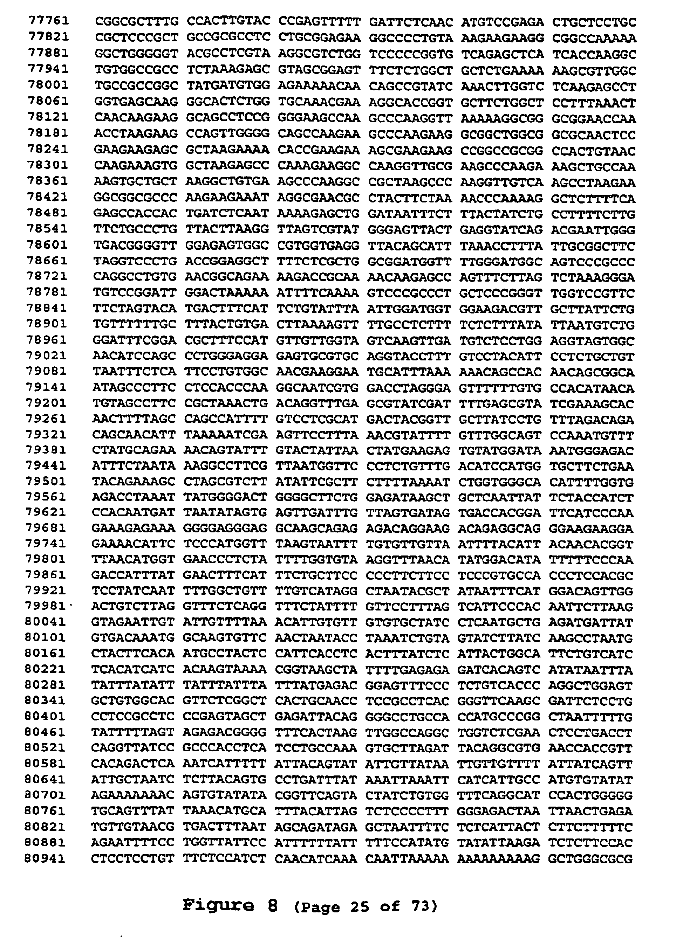

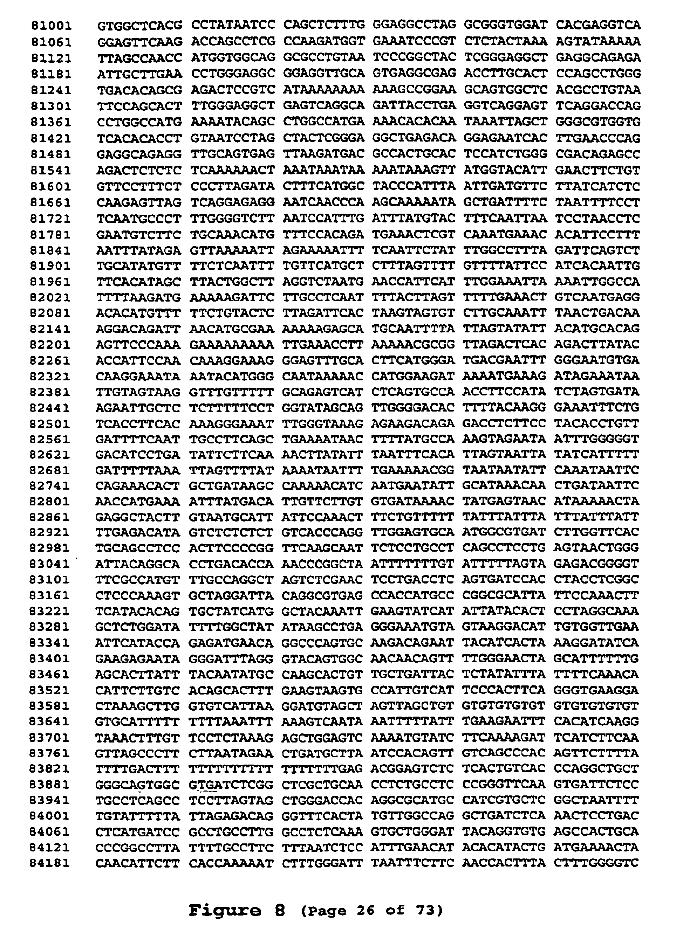

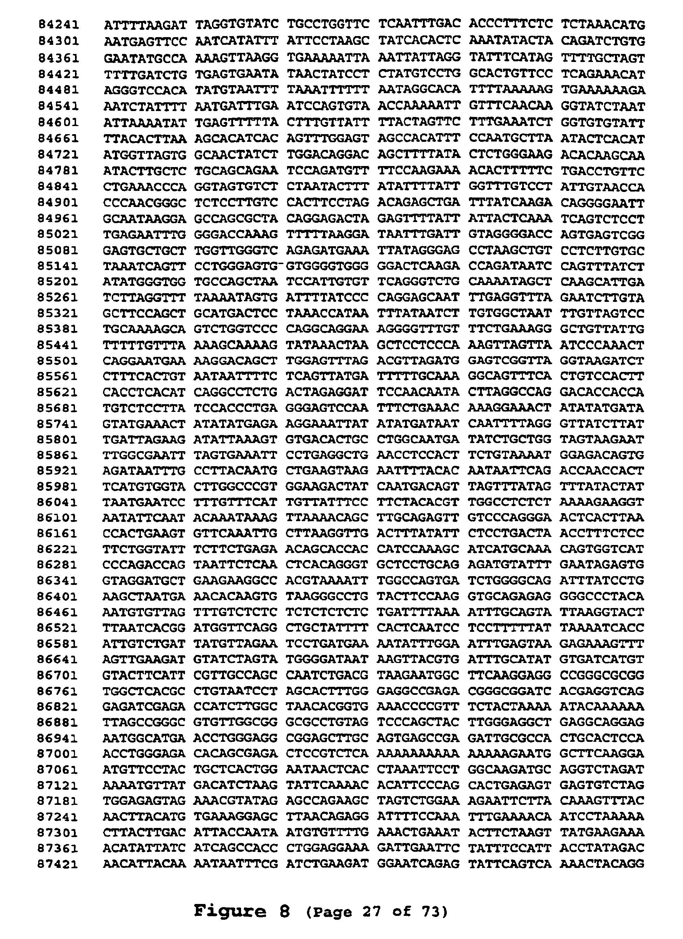

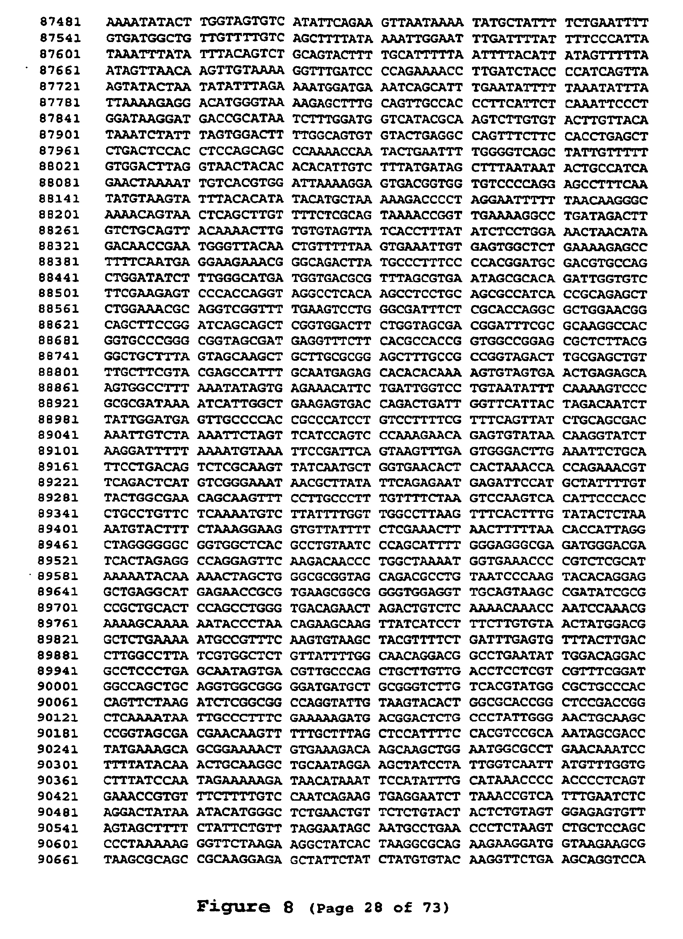

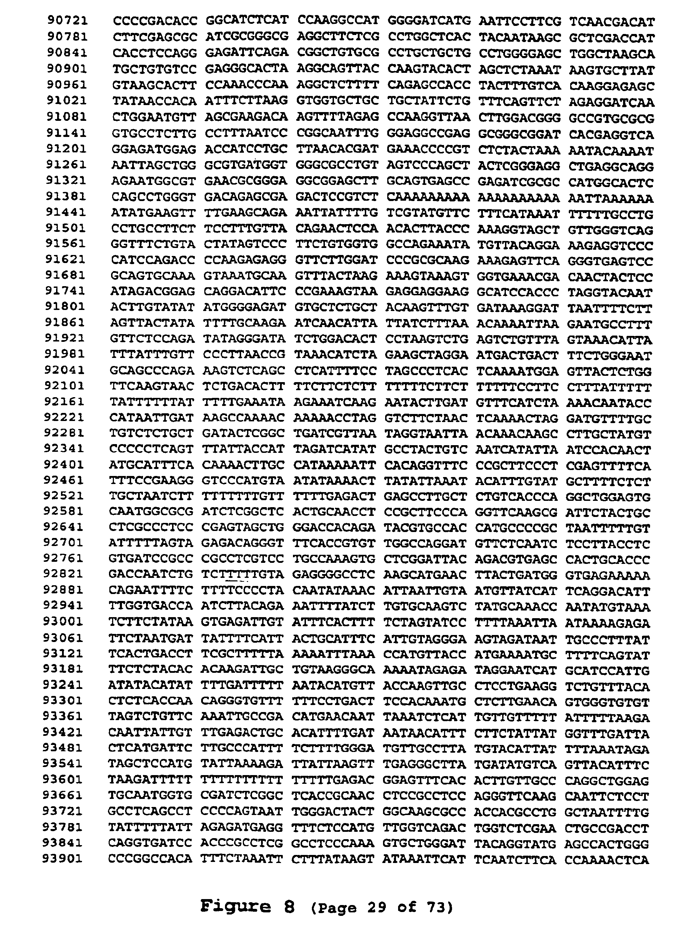

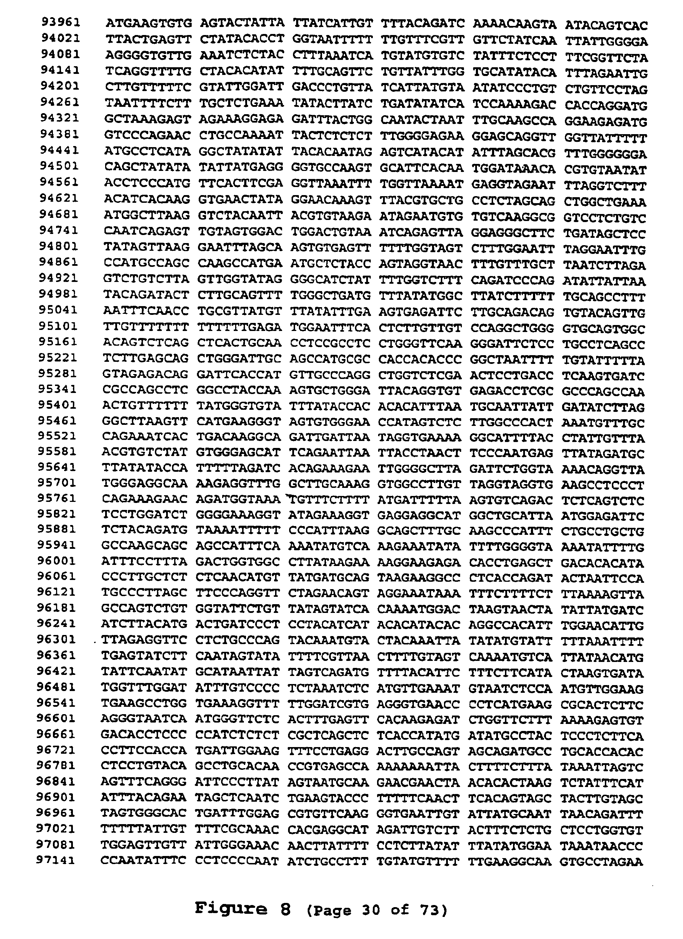

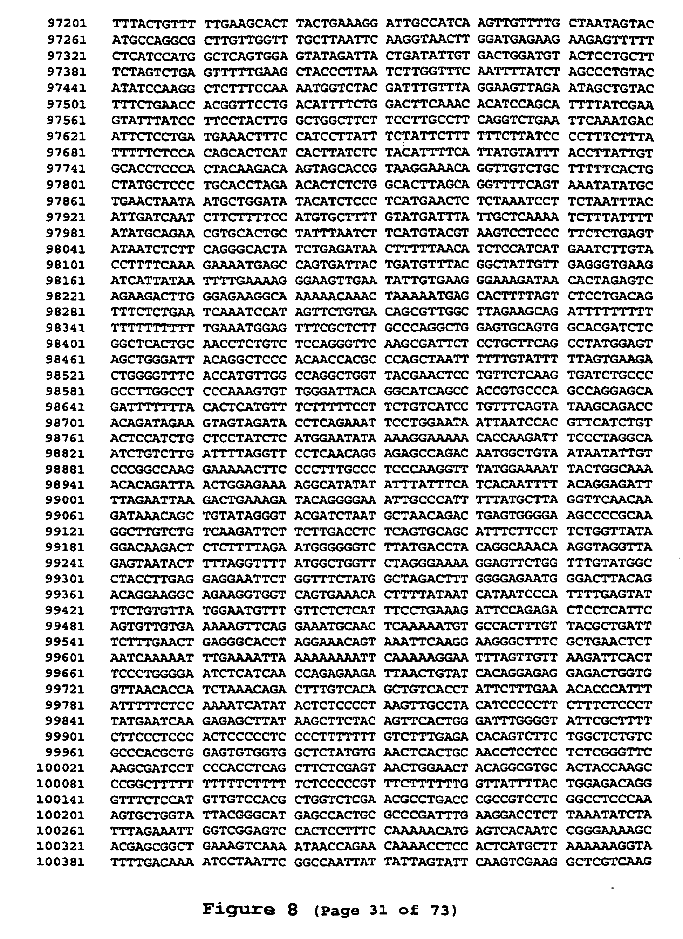

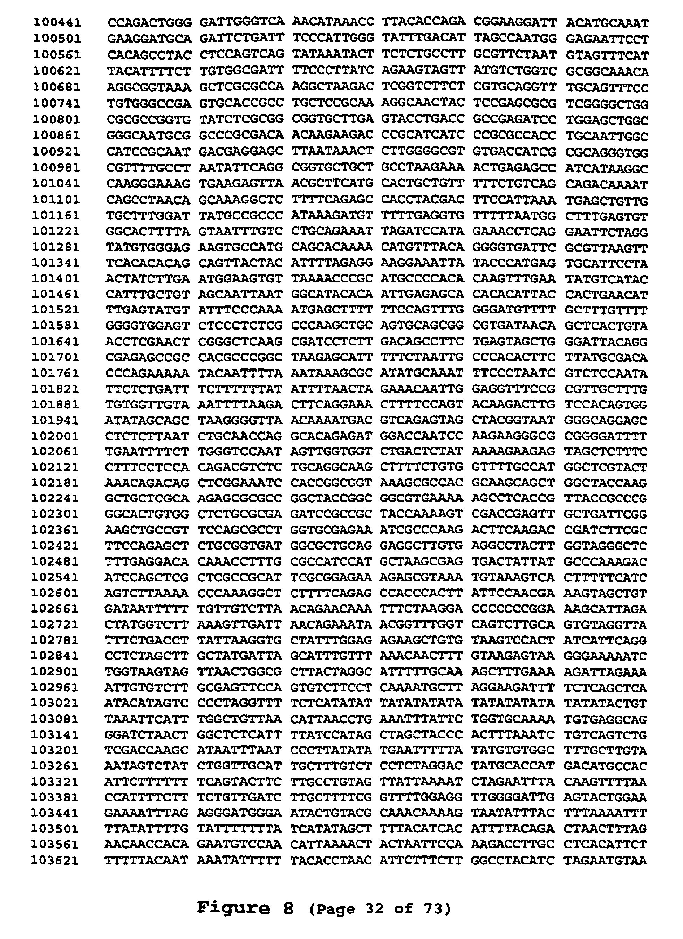

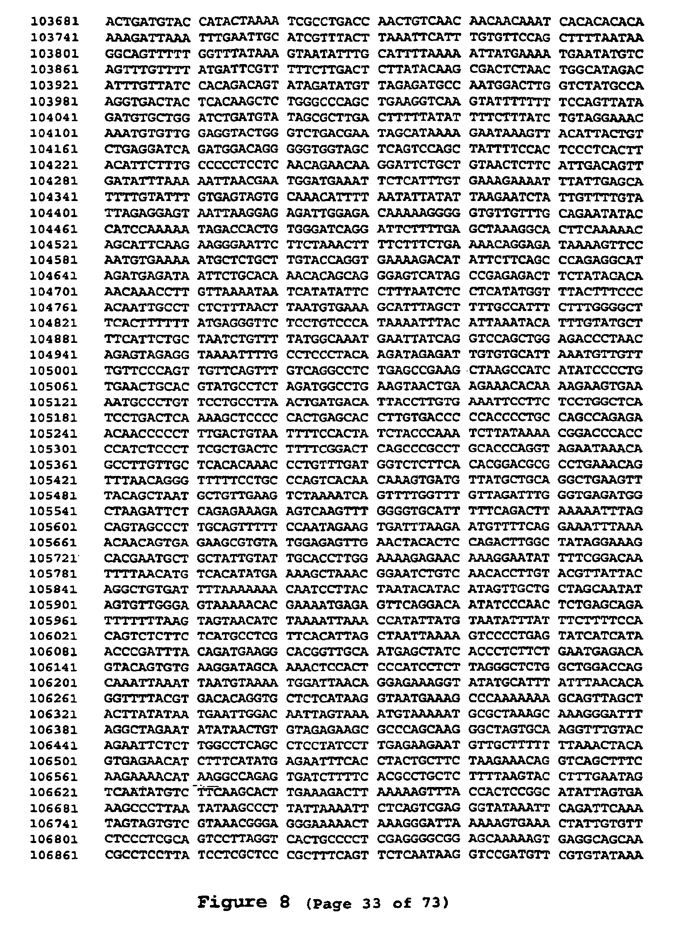

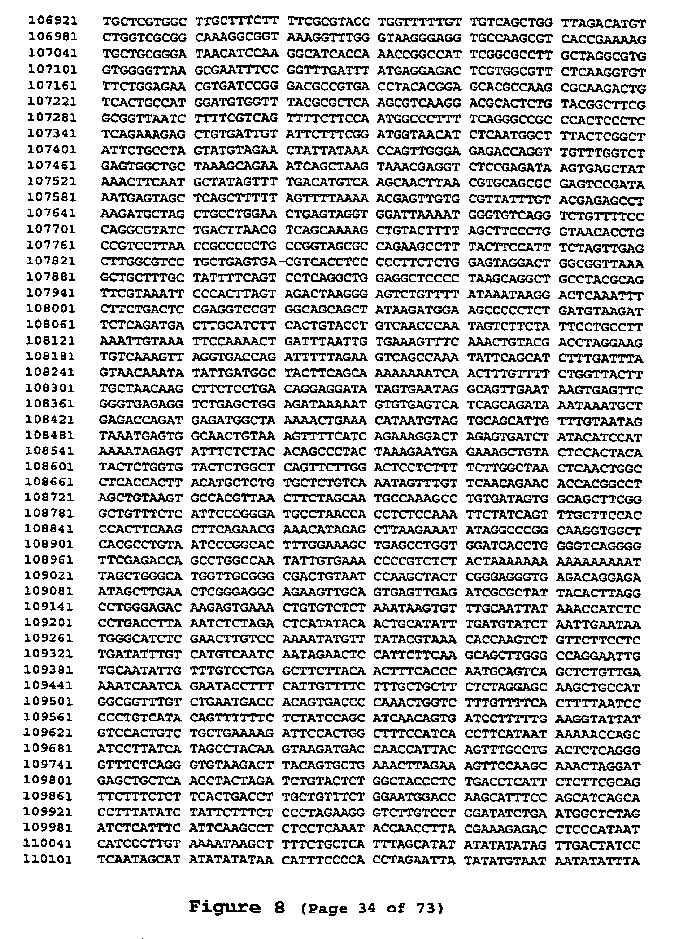

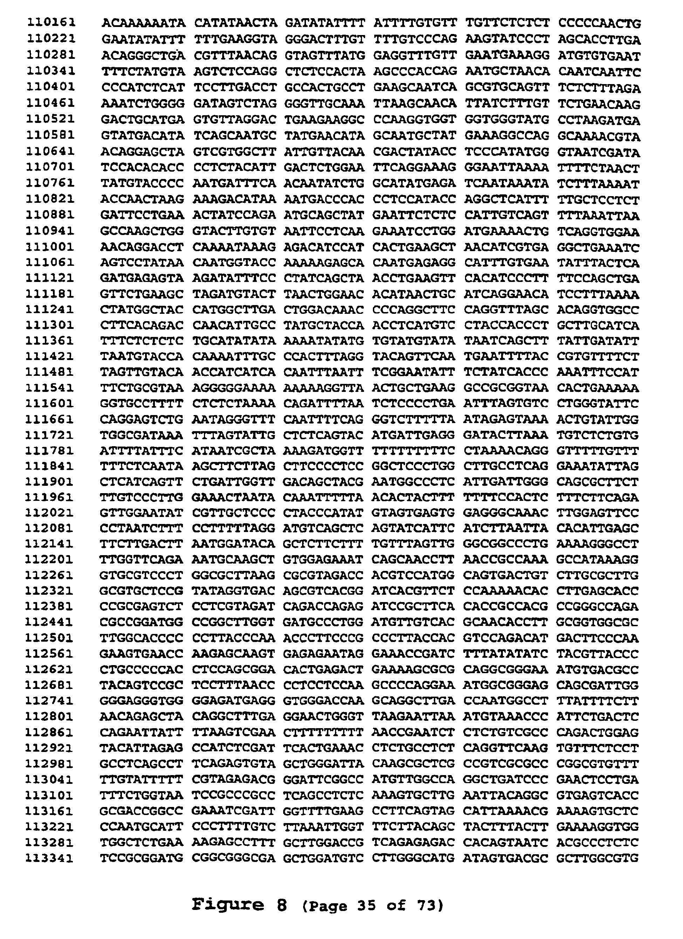

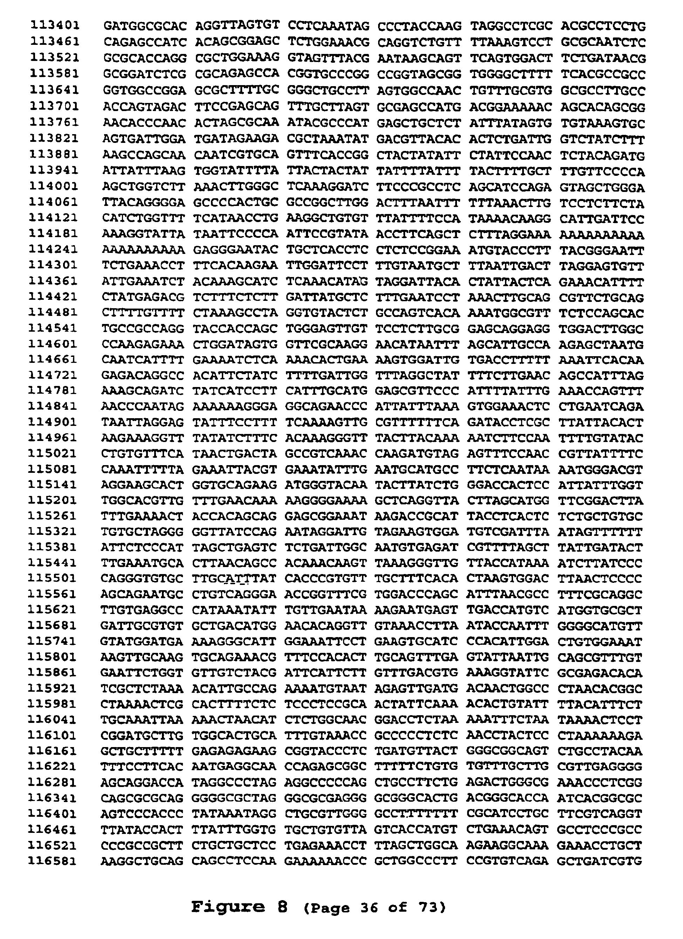

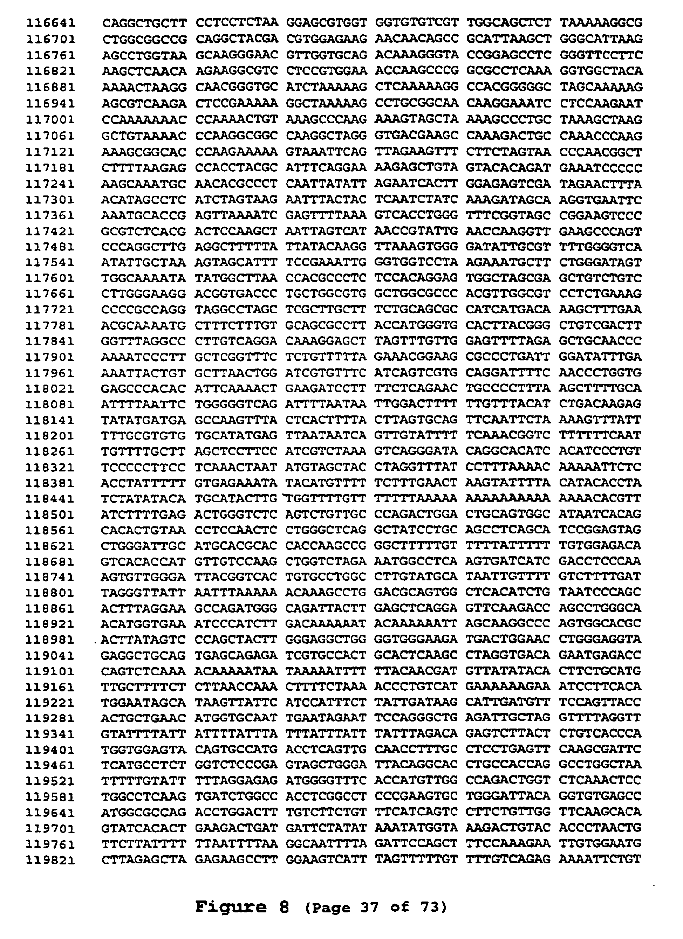

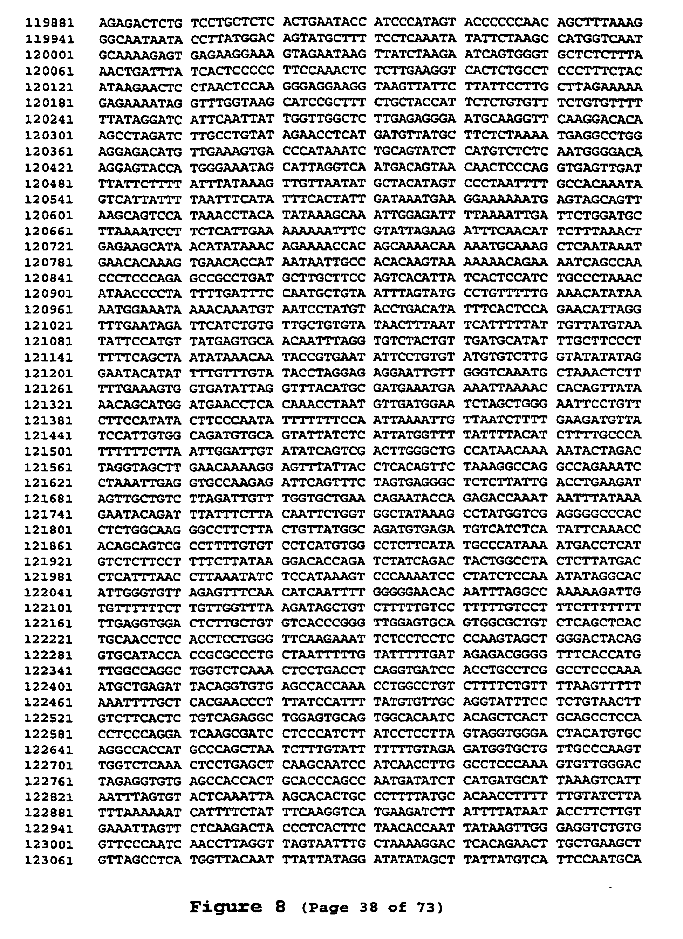

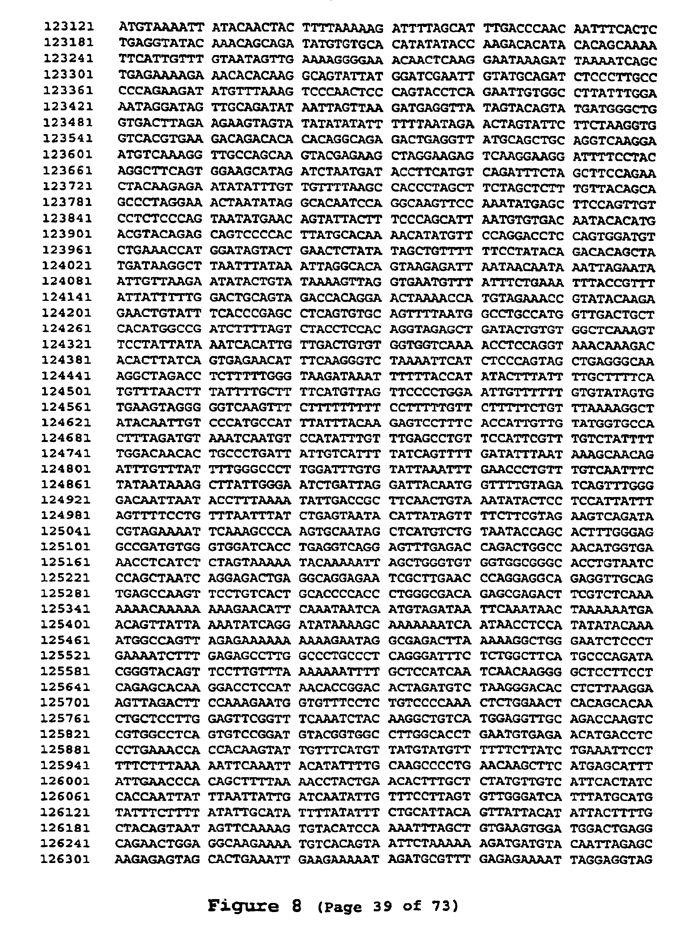

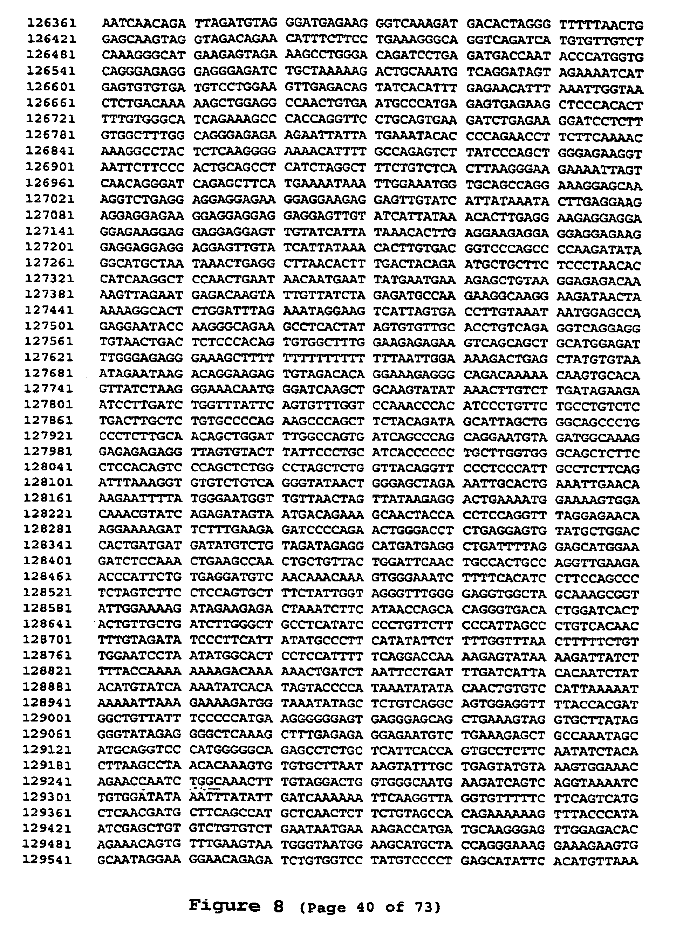

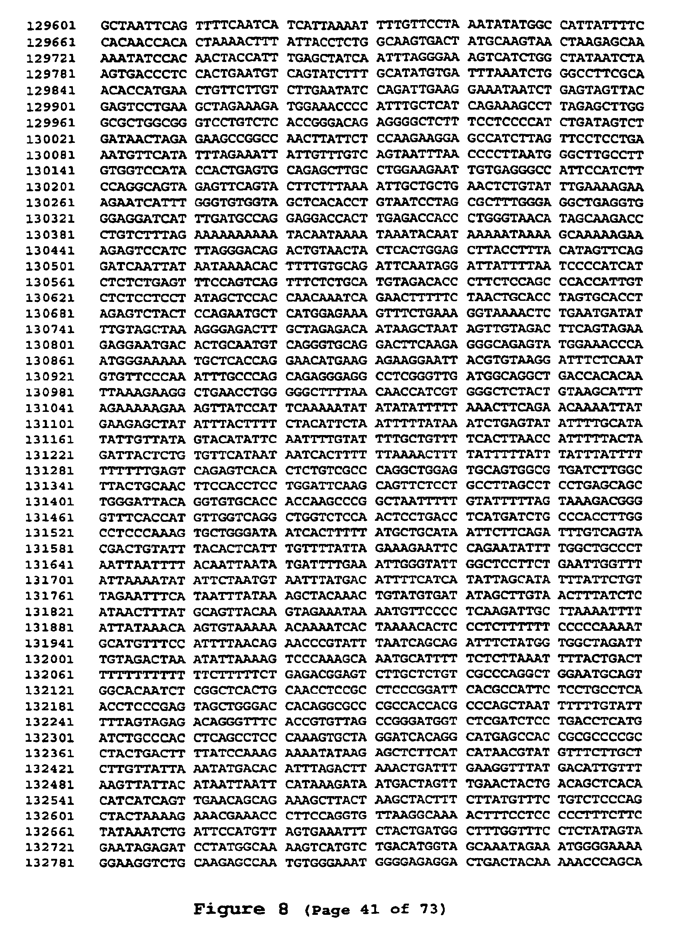

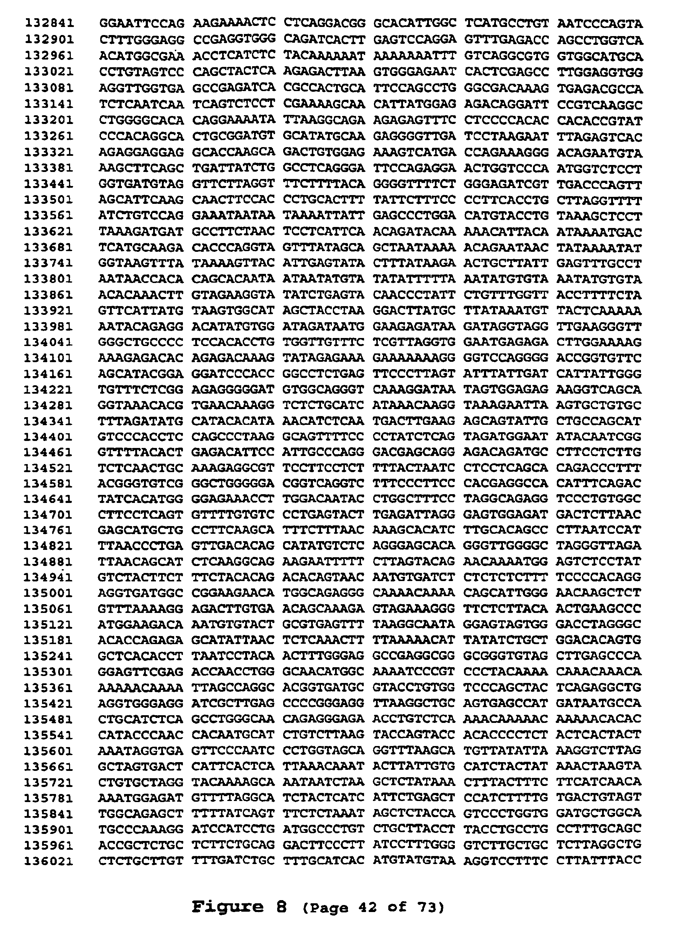

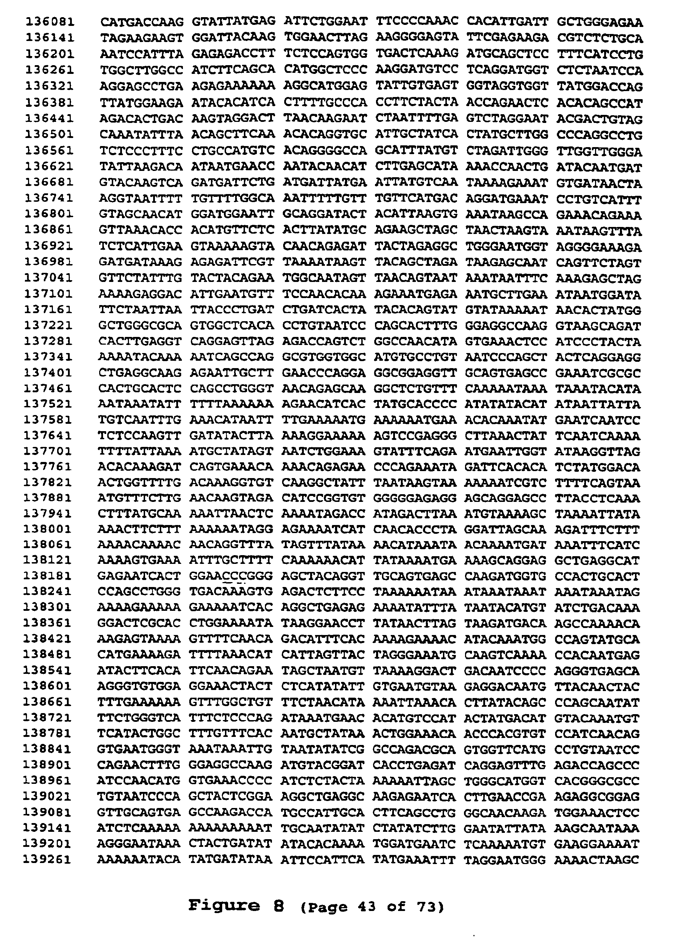

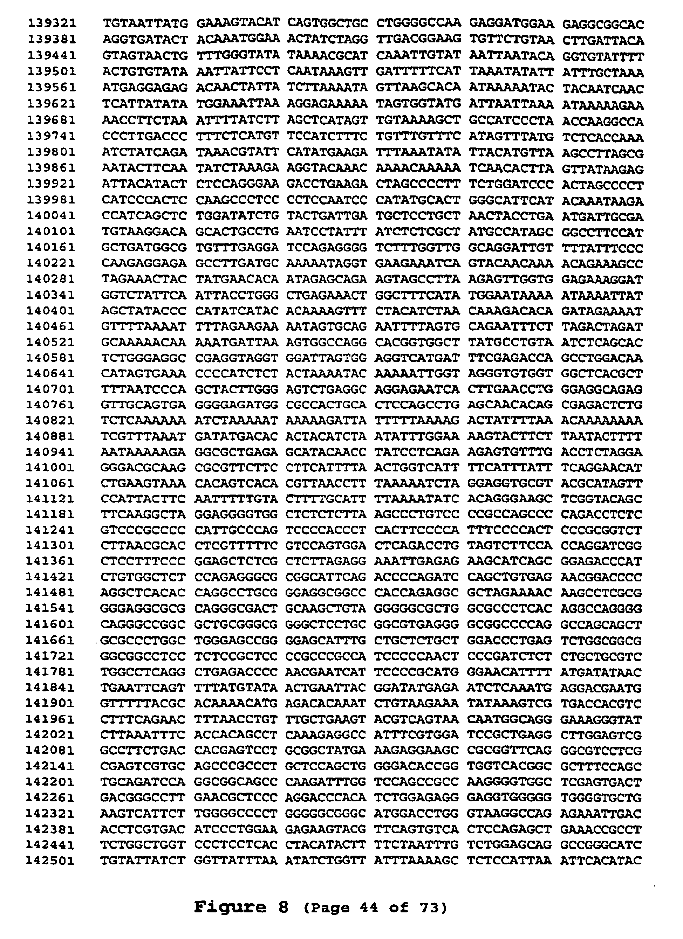

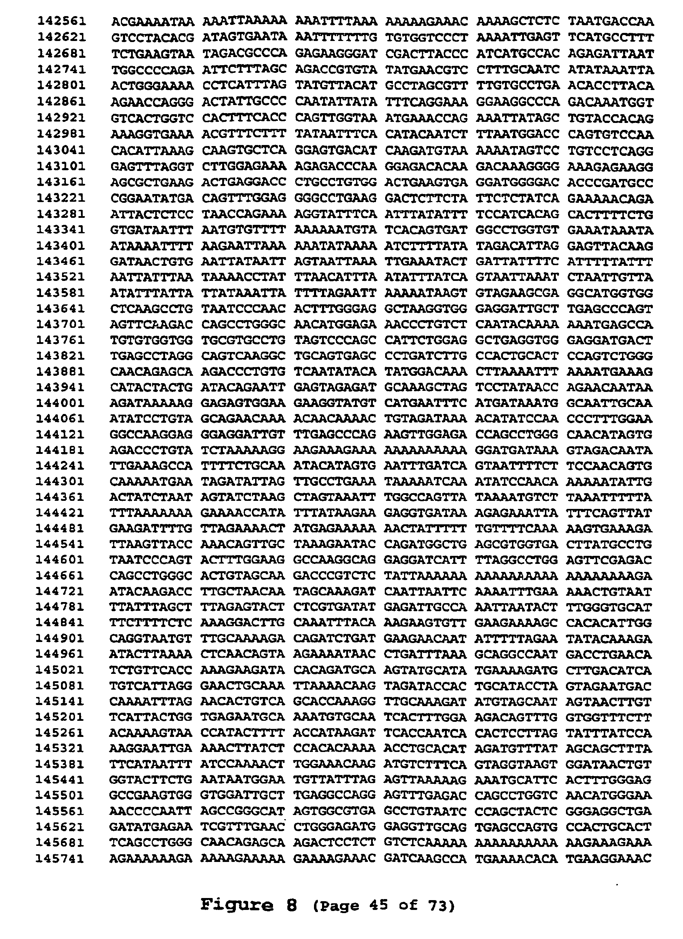

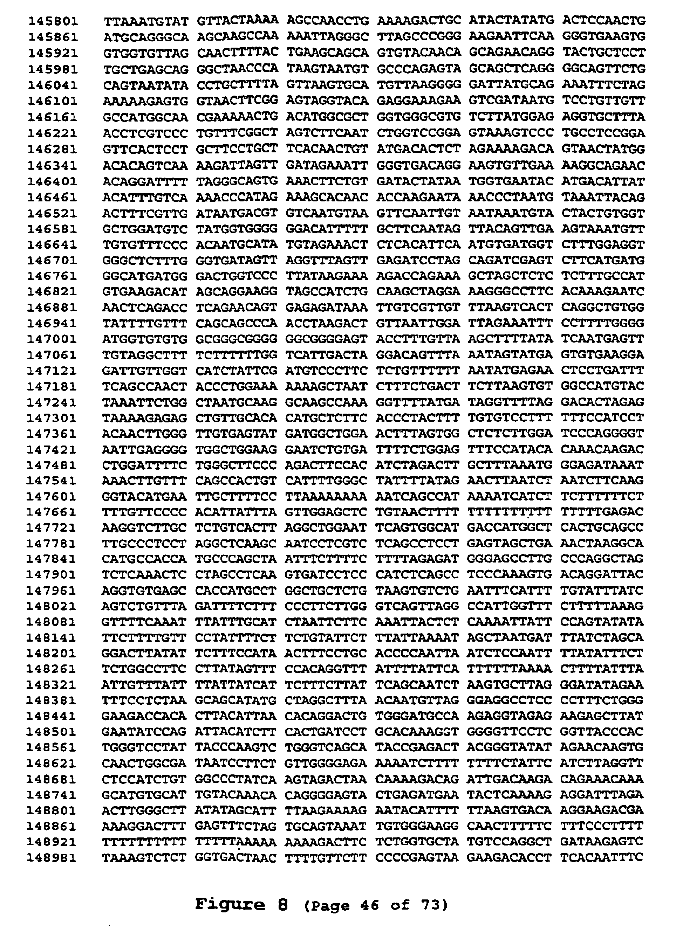

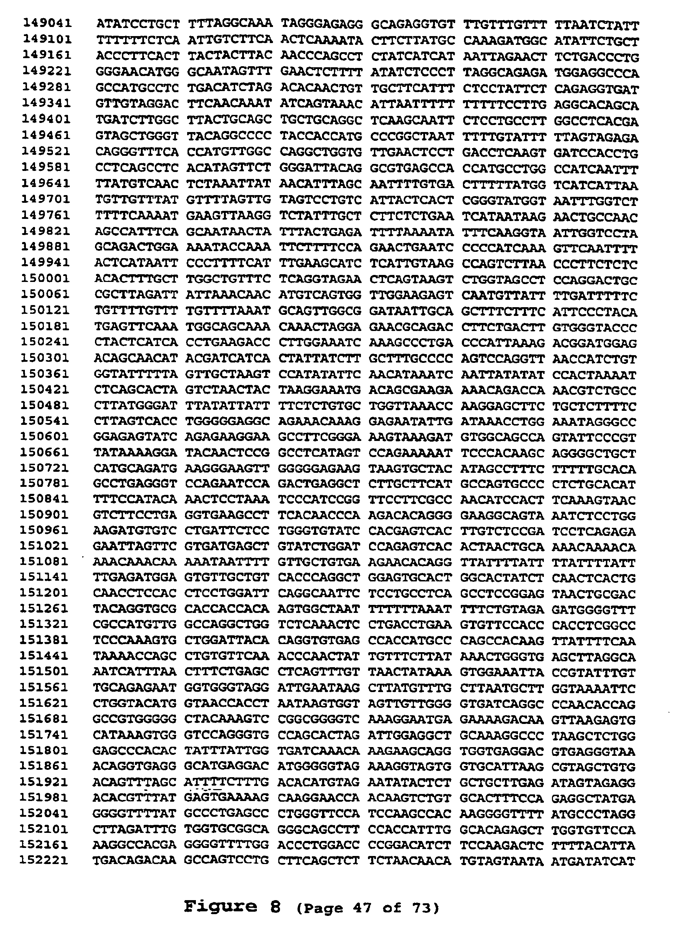

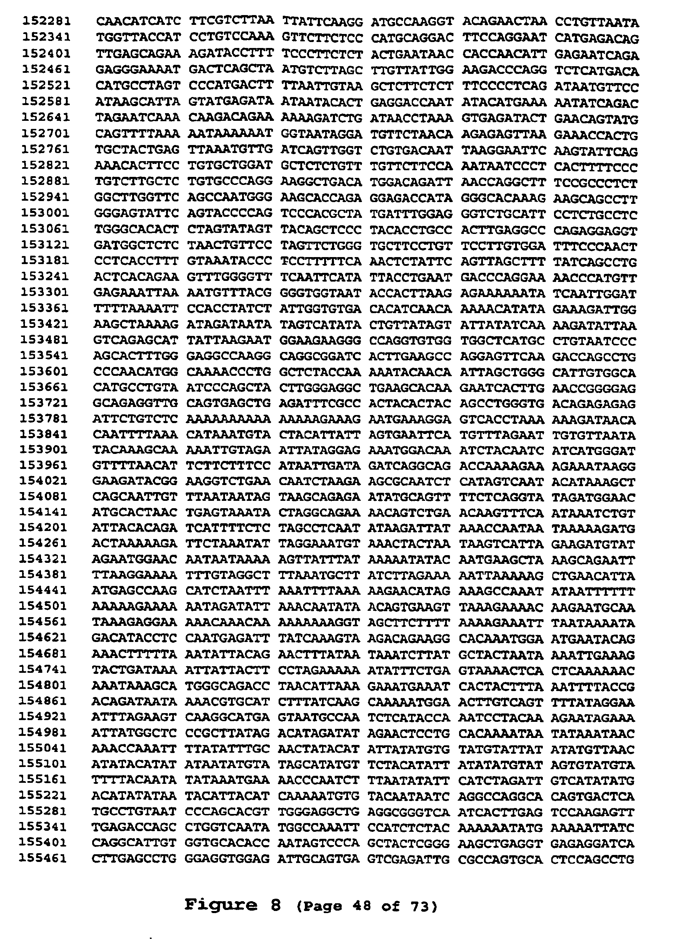

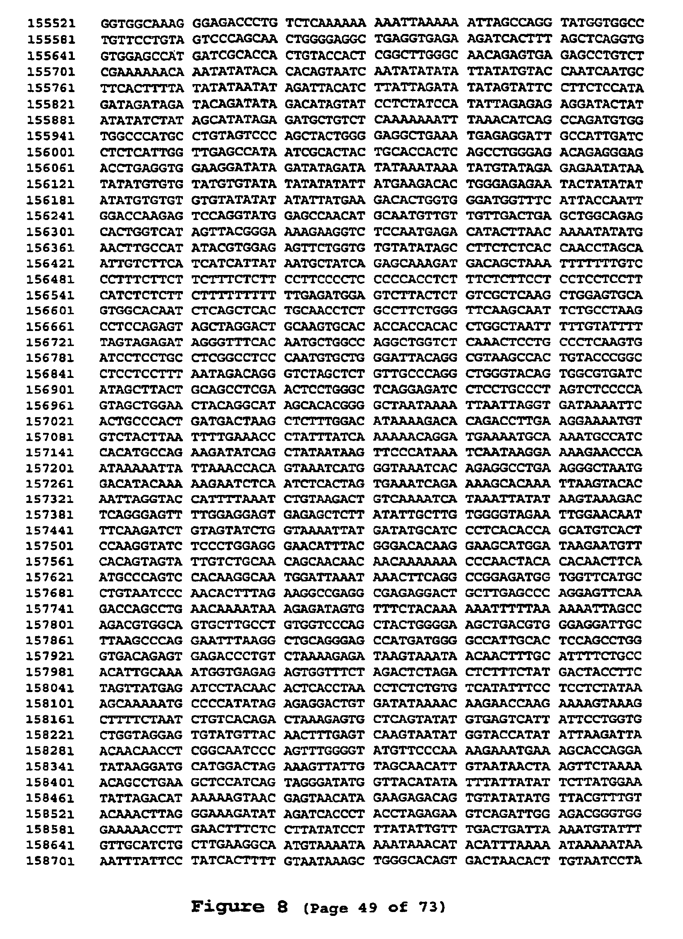

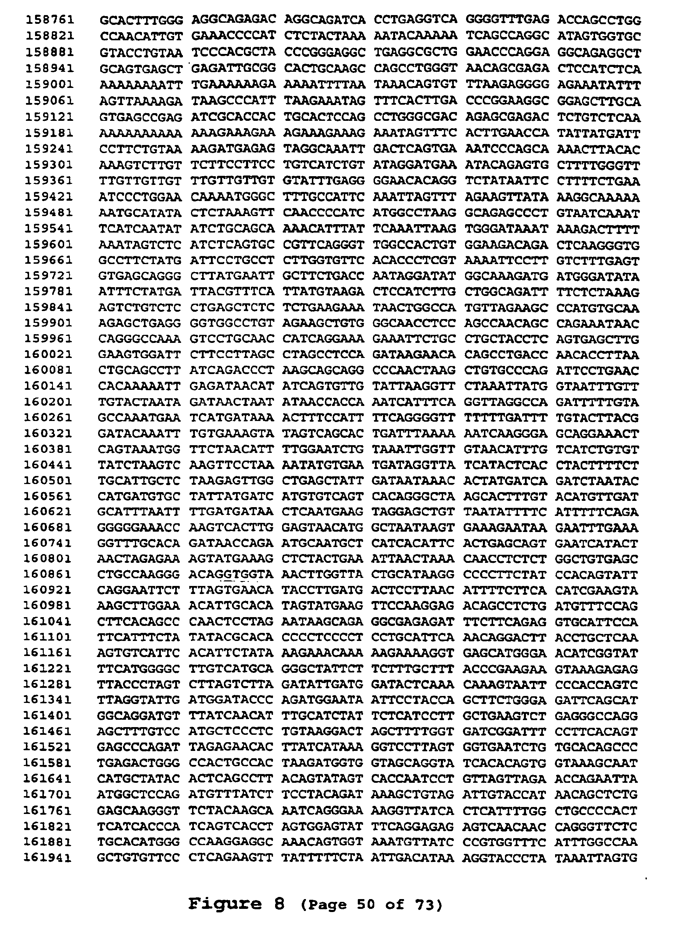

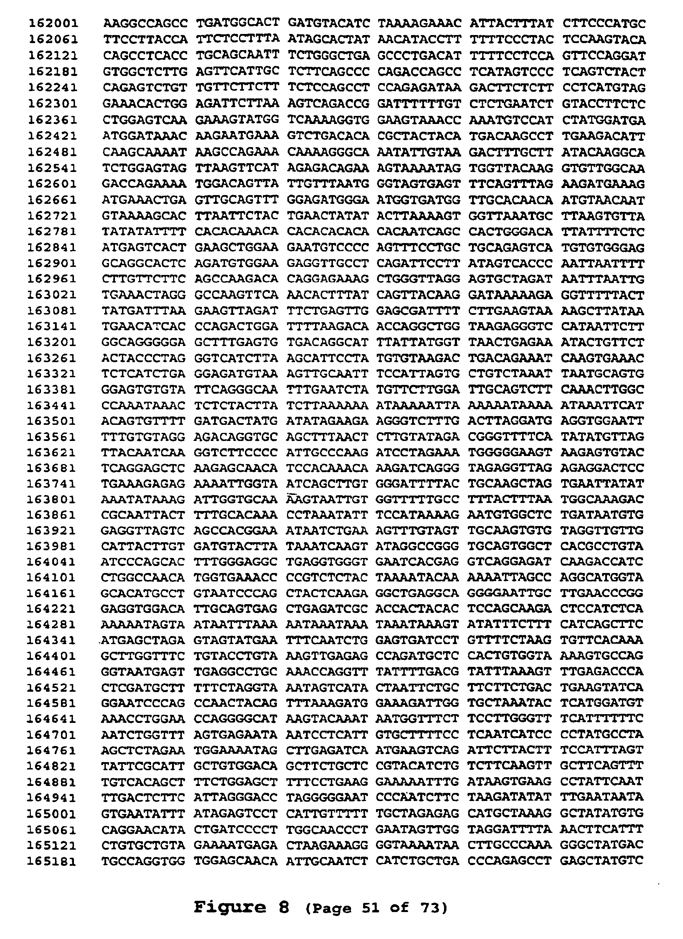

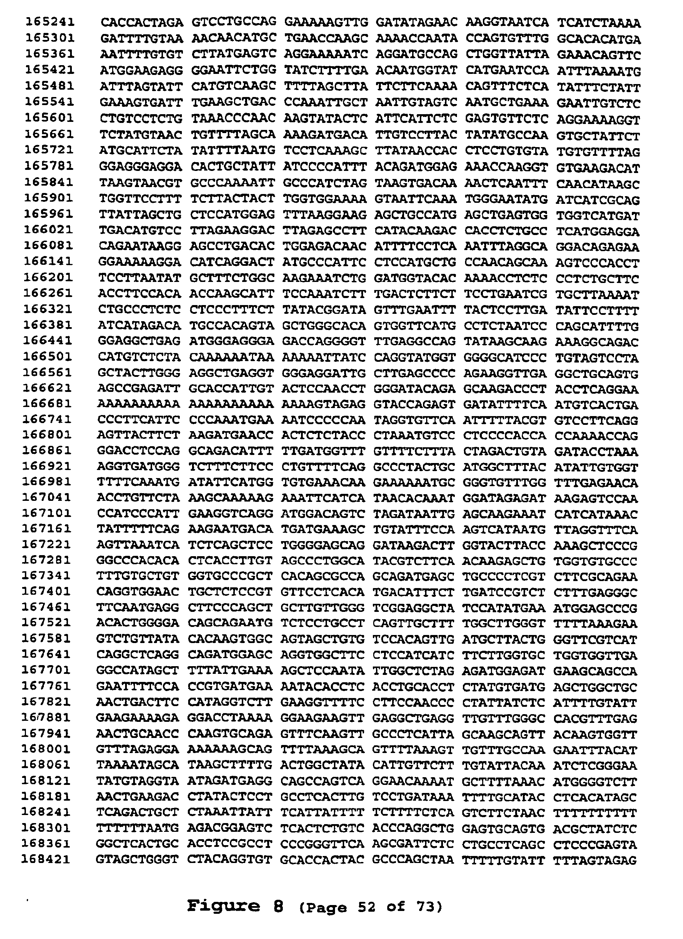

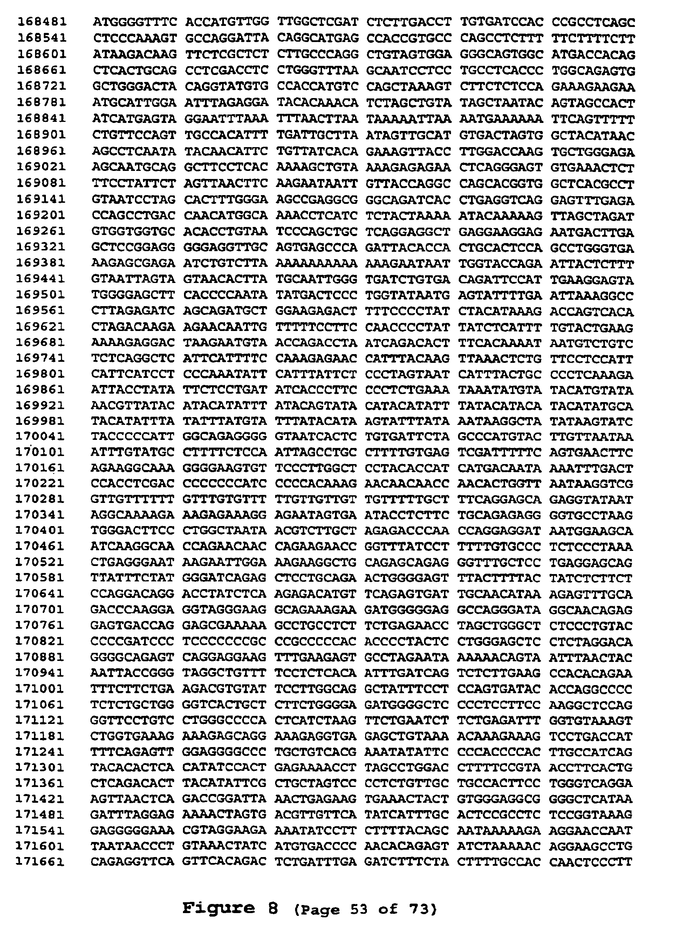

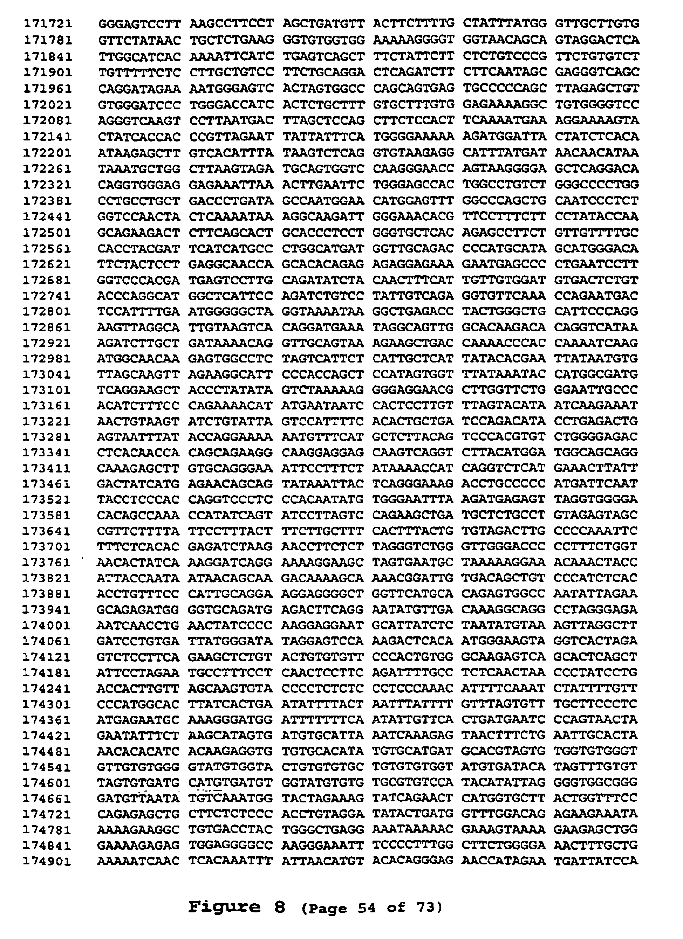

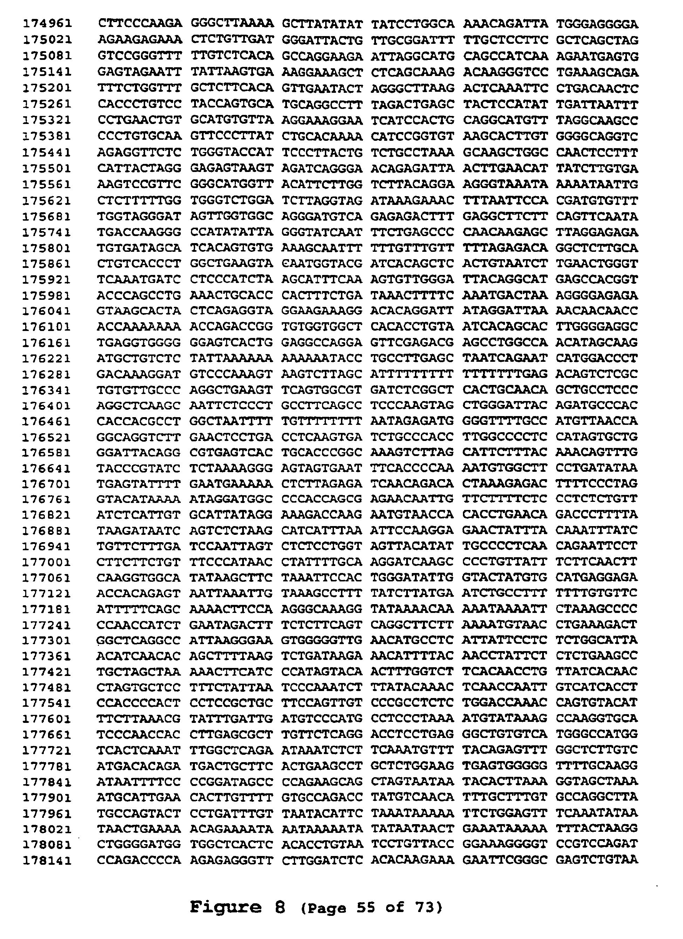

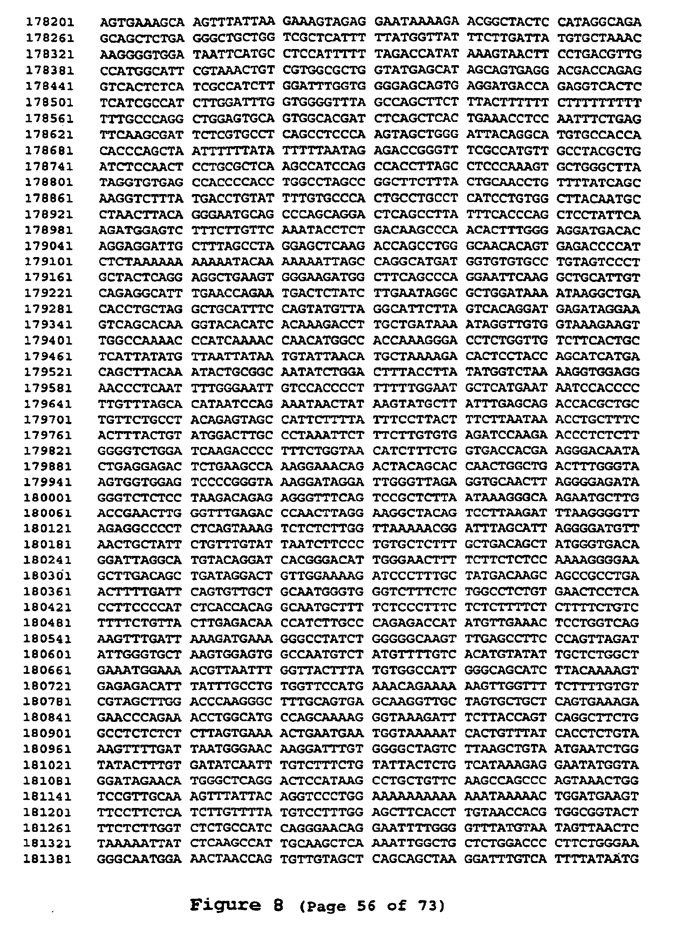

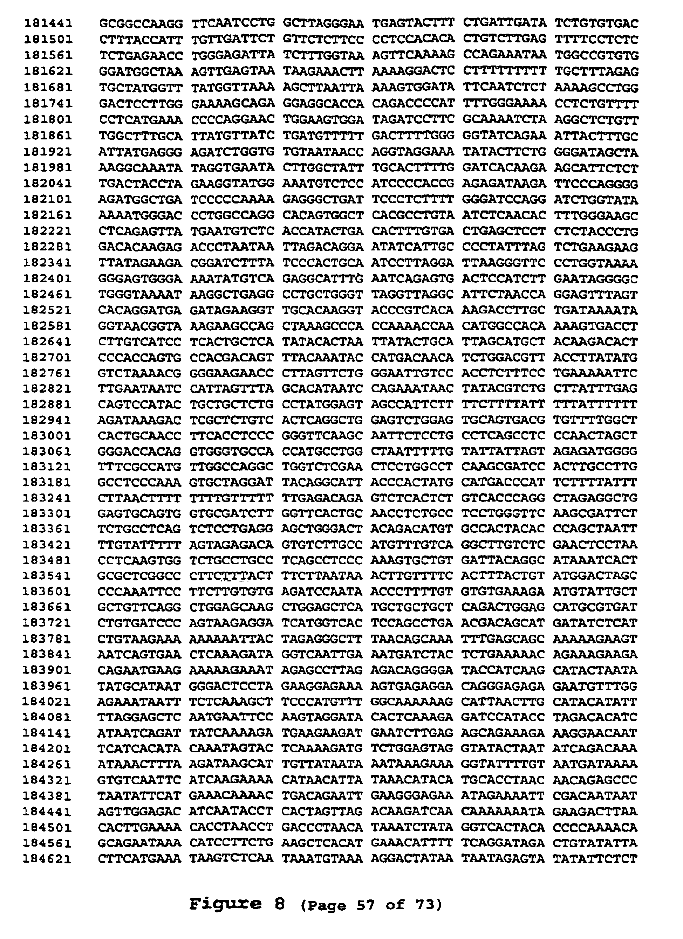

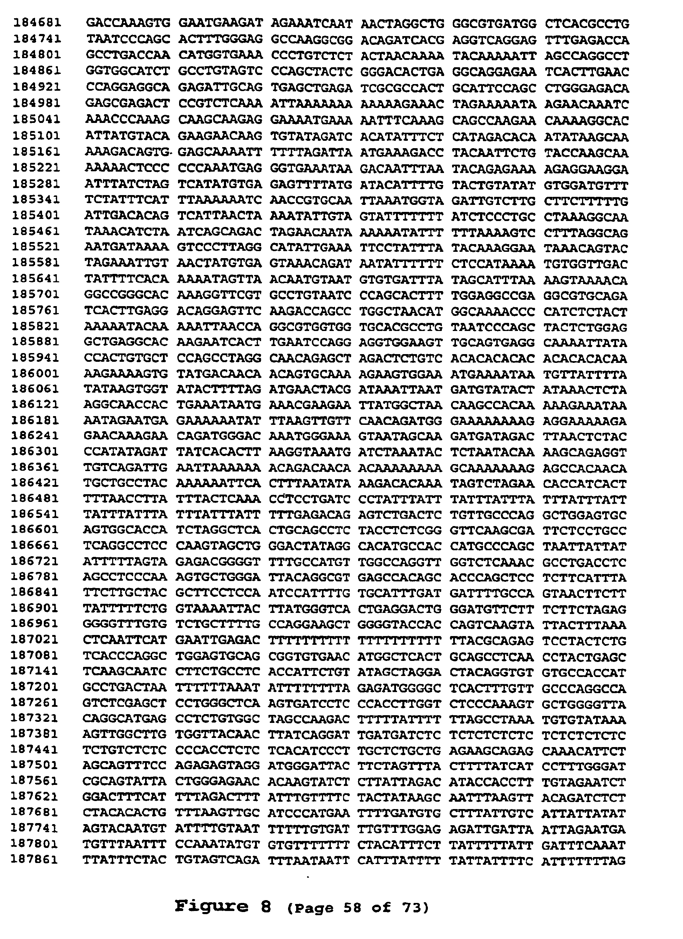

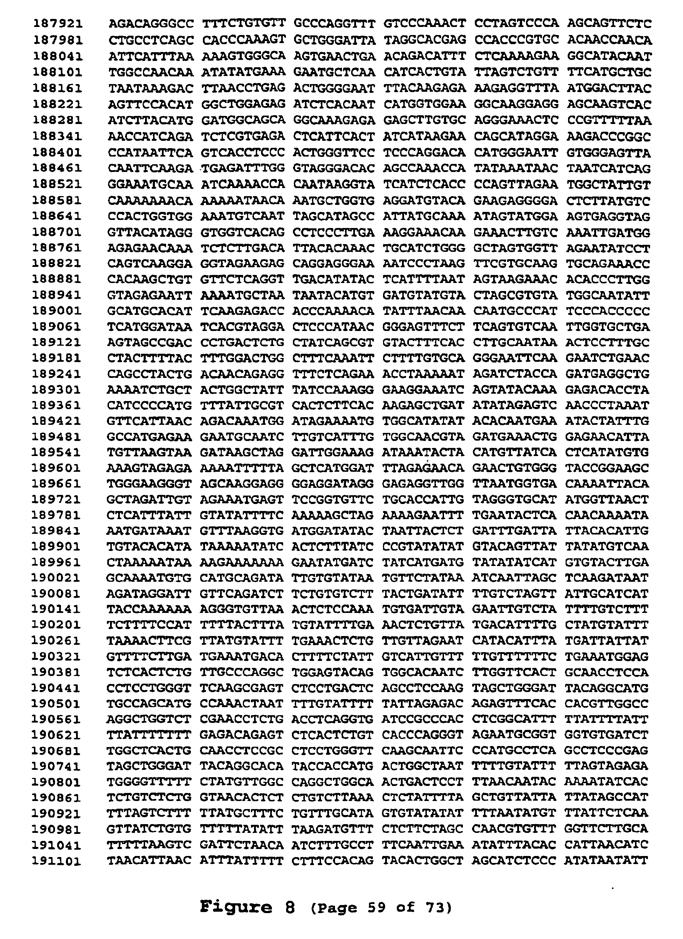

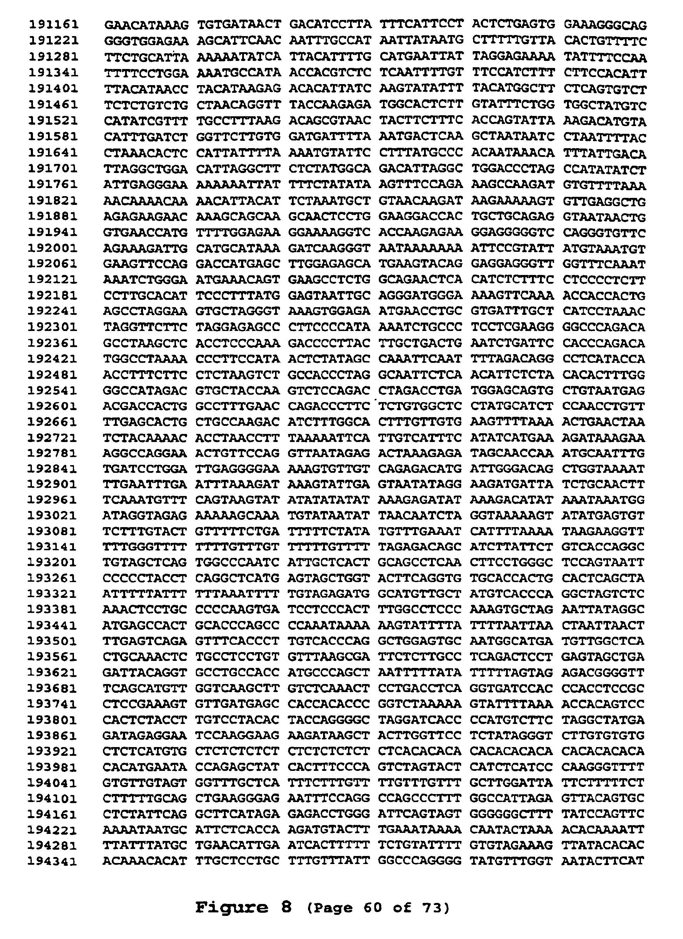

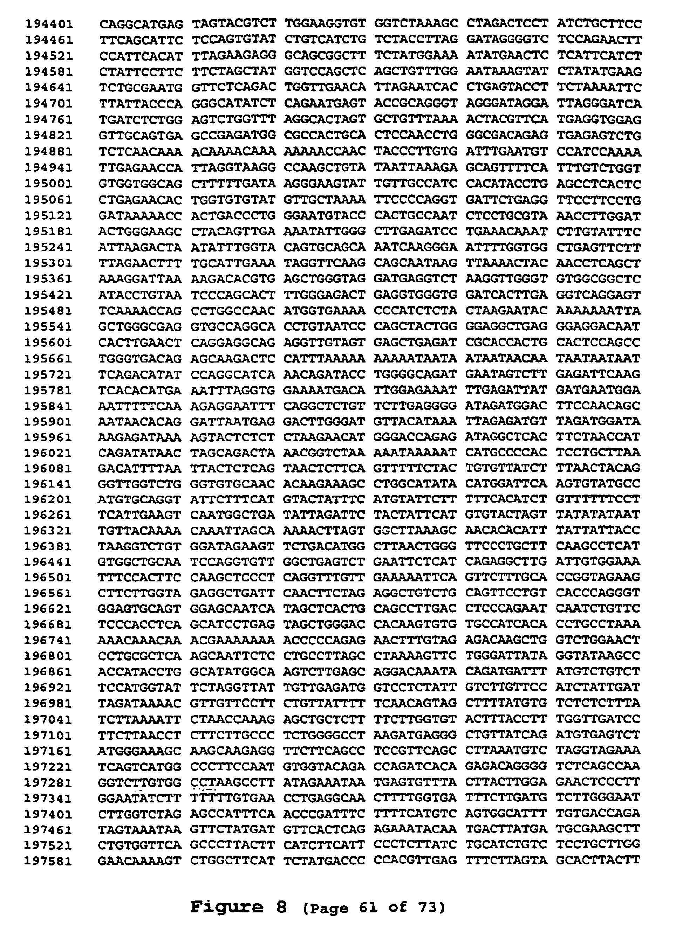

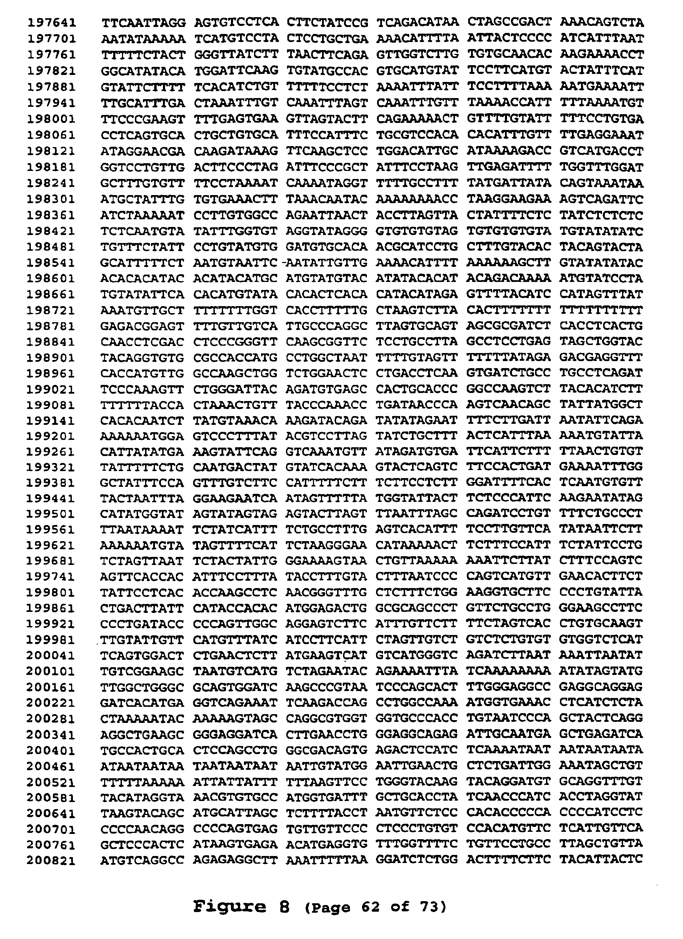

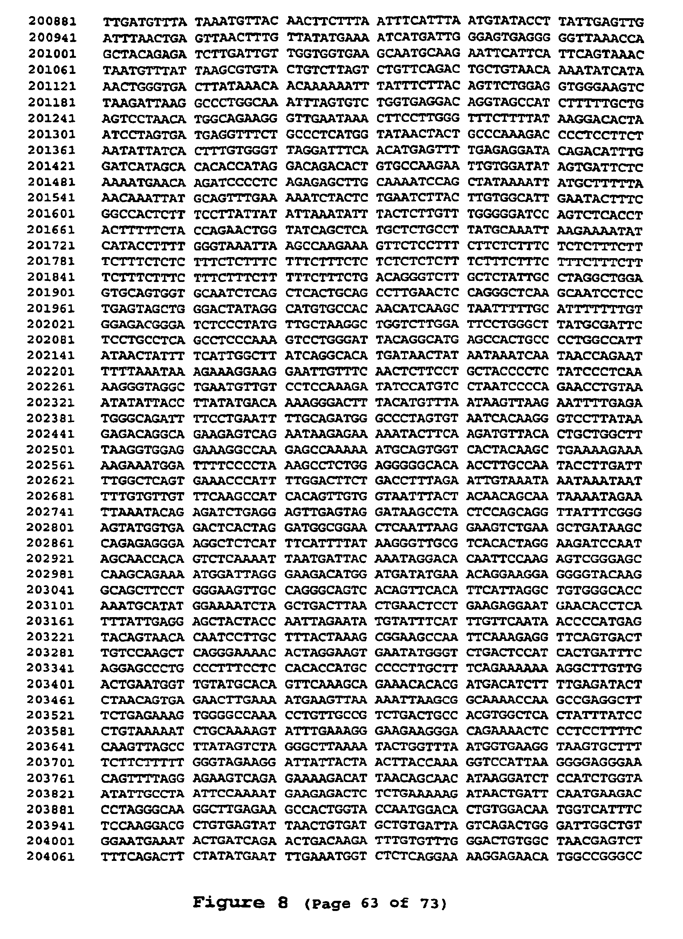

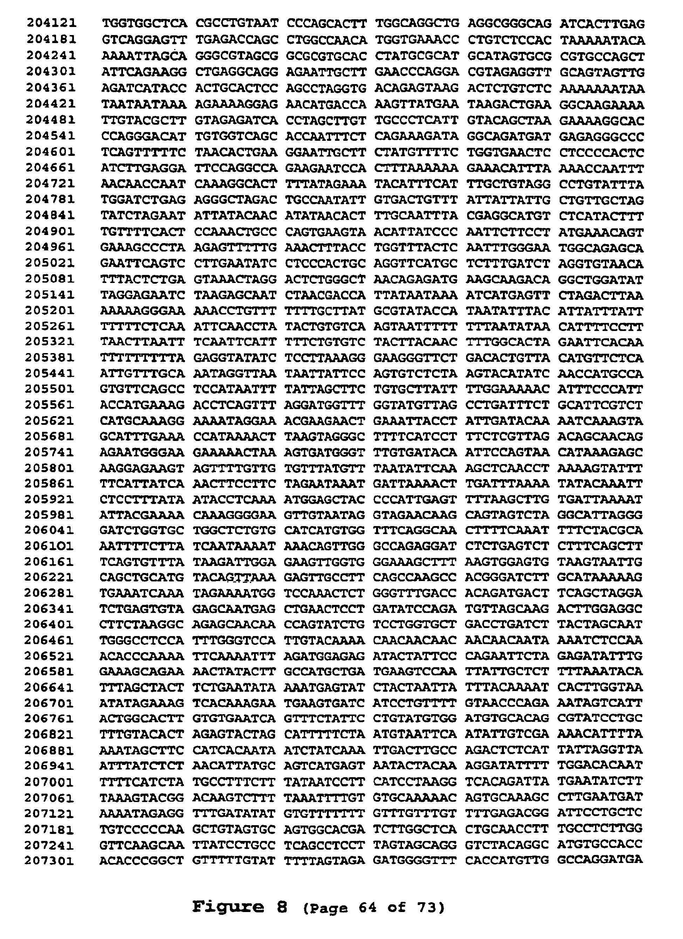

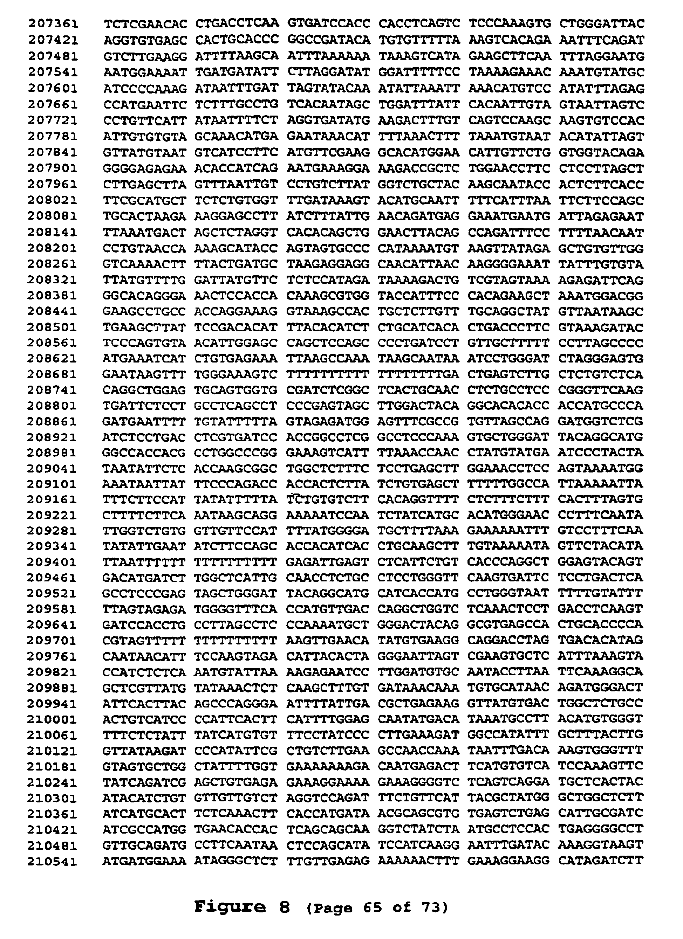

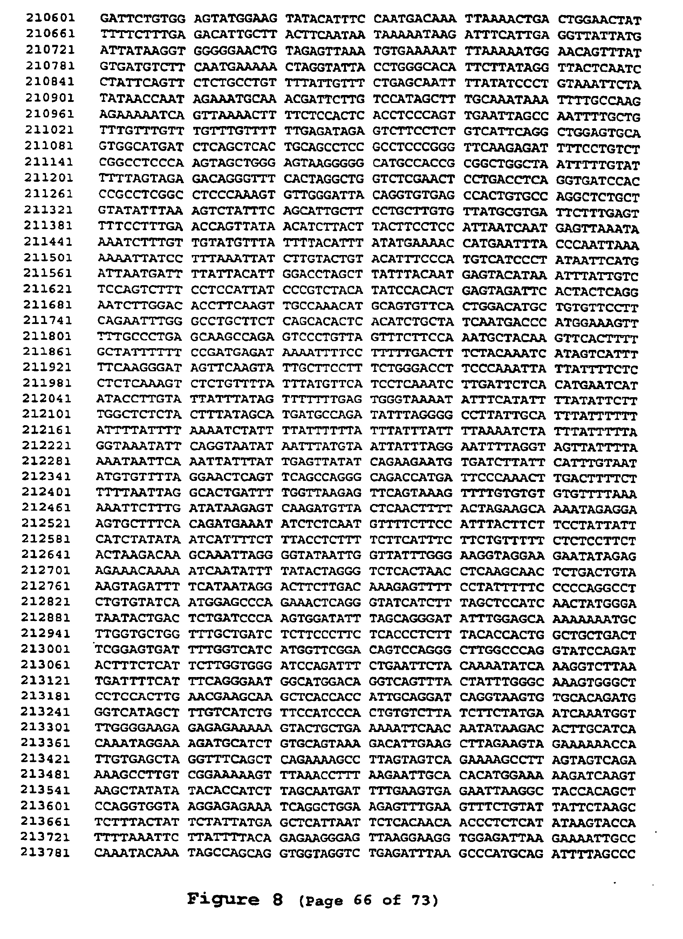

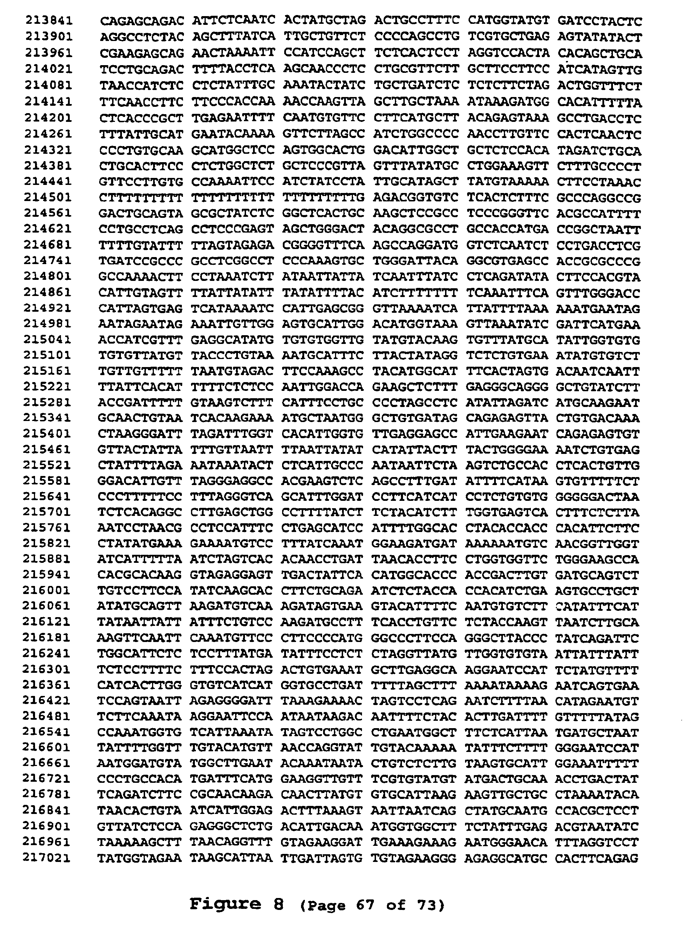

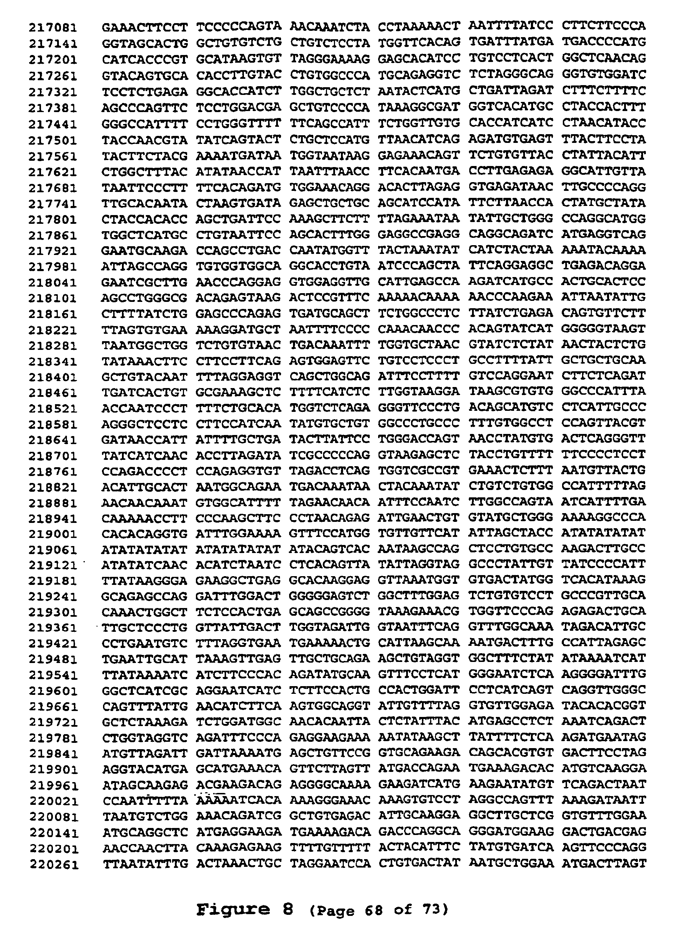

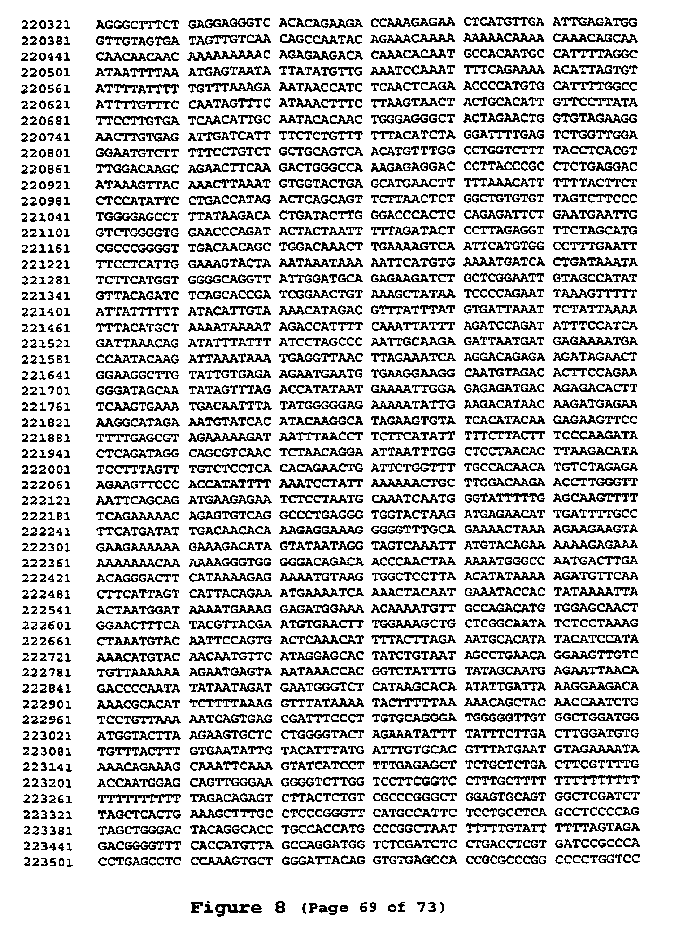

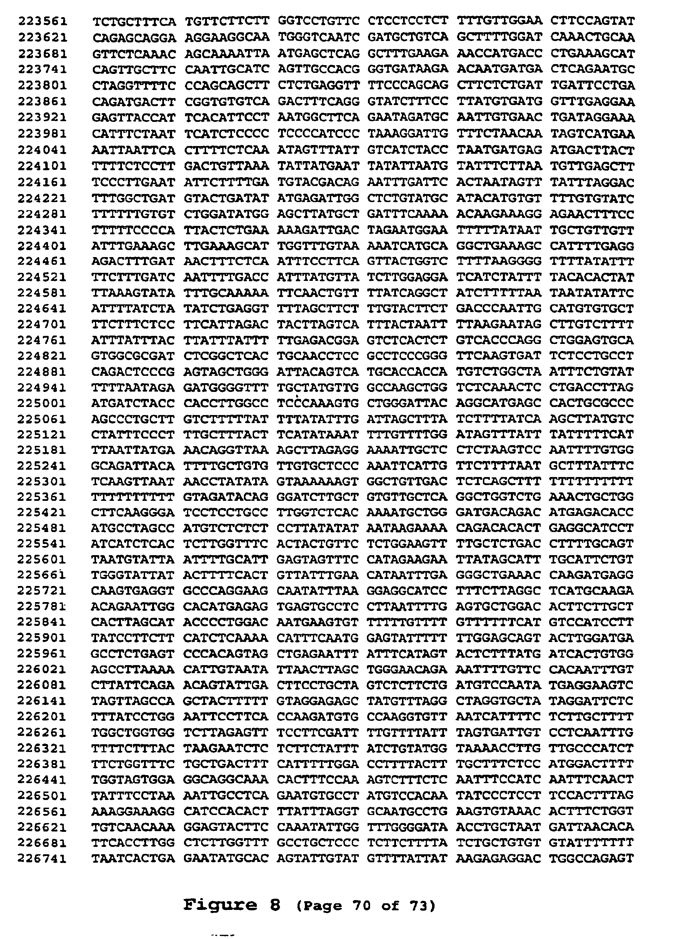

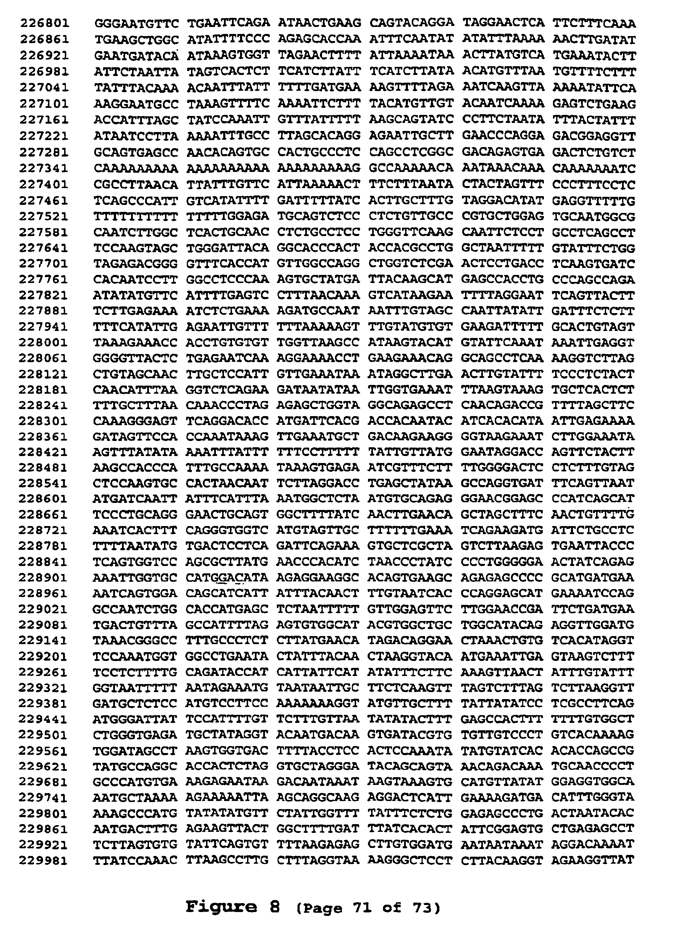

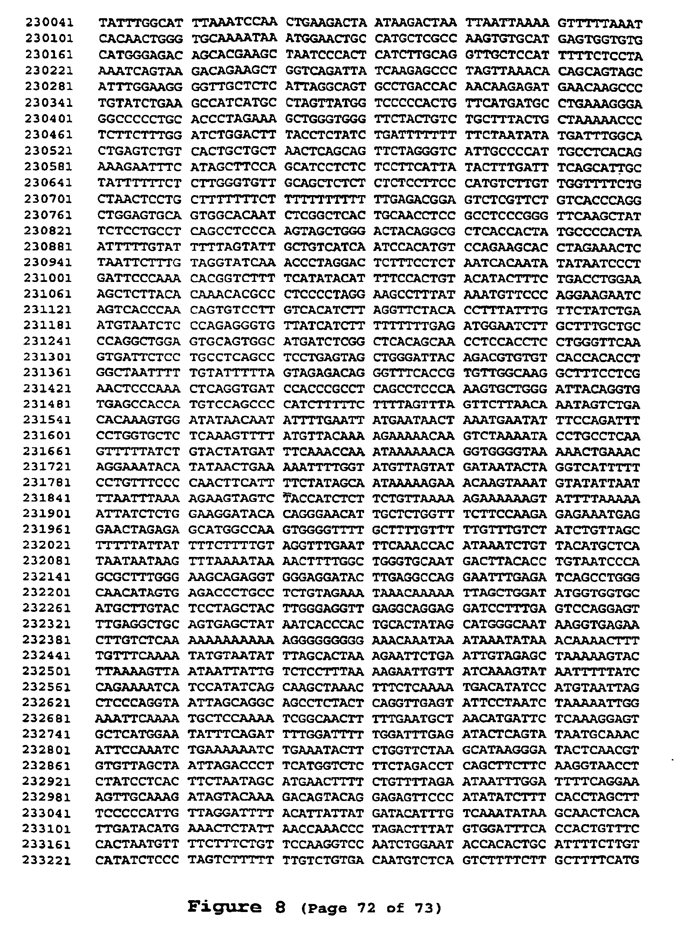

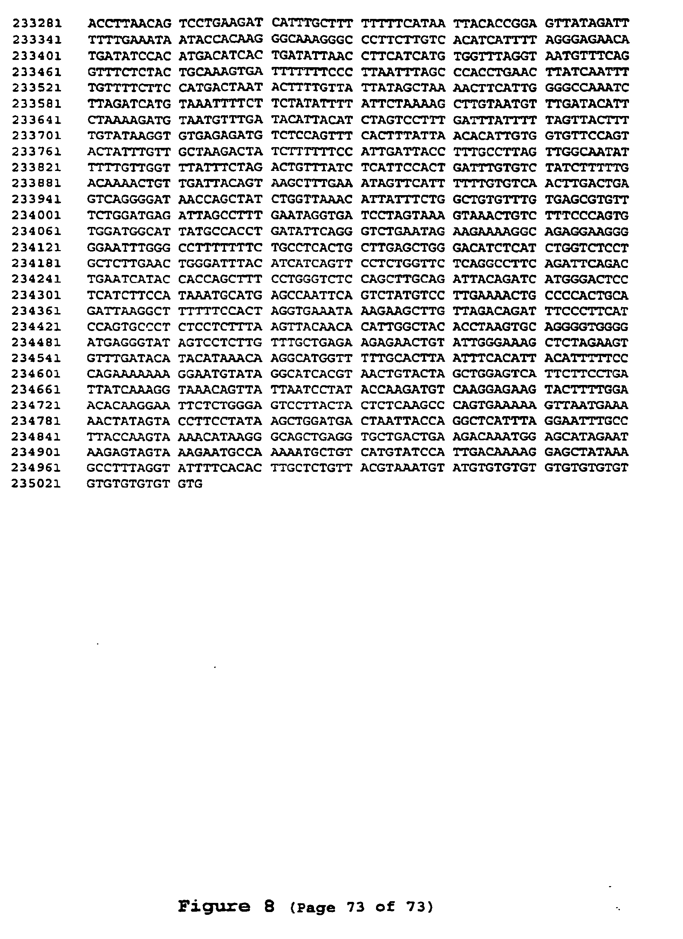

Figure 8 depicts the nucleotide sequence of approximately 235 kb in the HFE subregion from an unaffected individual.

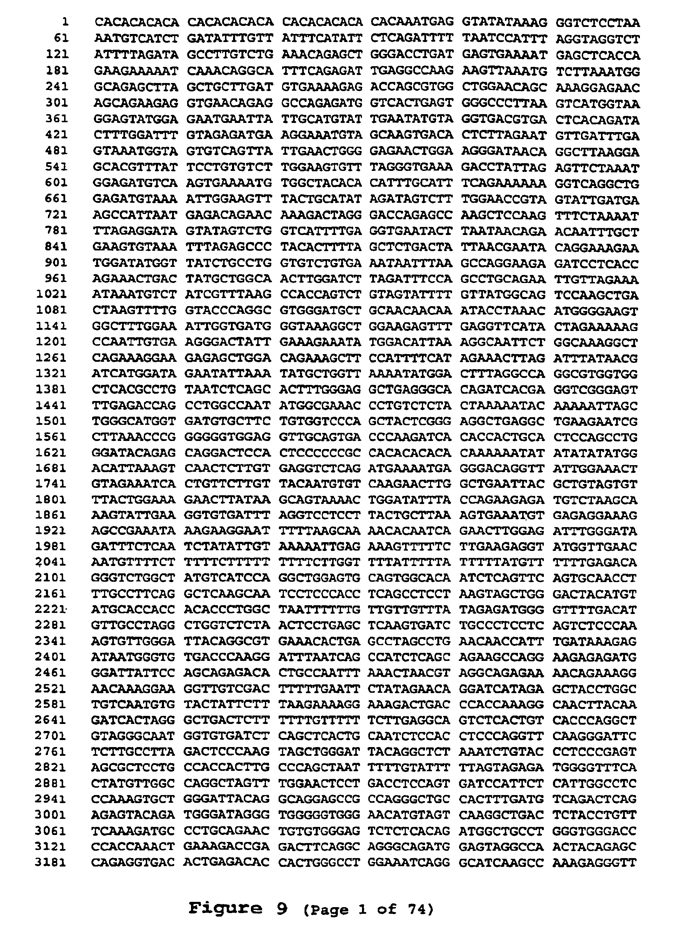

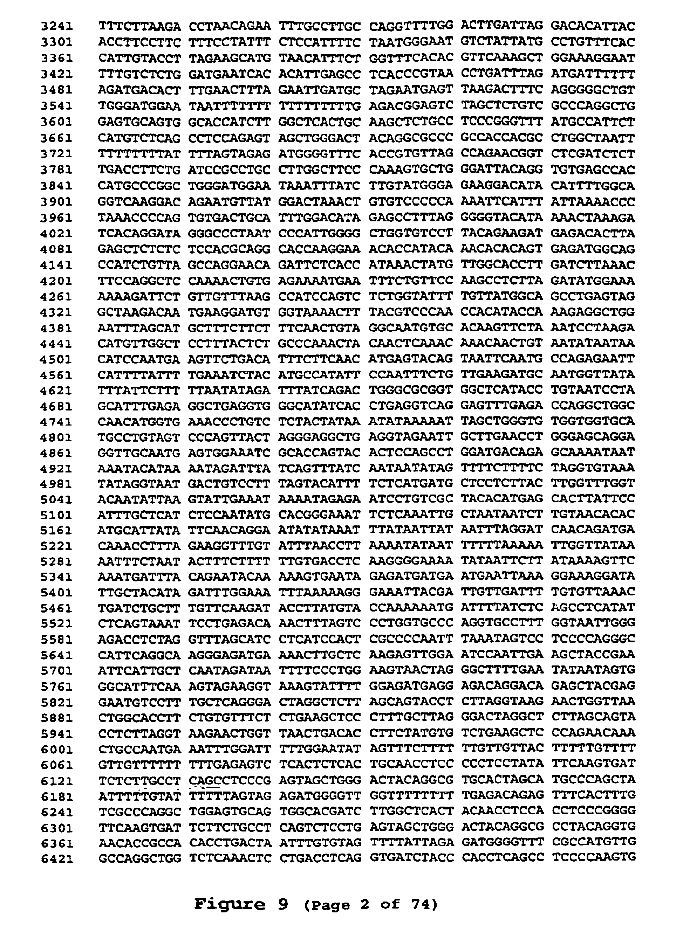

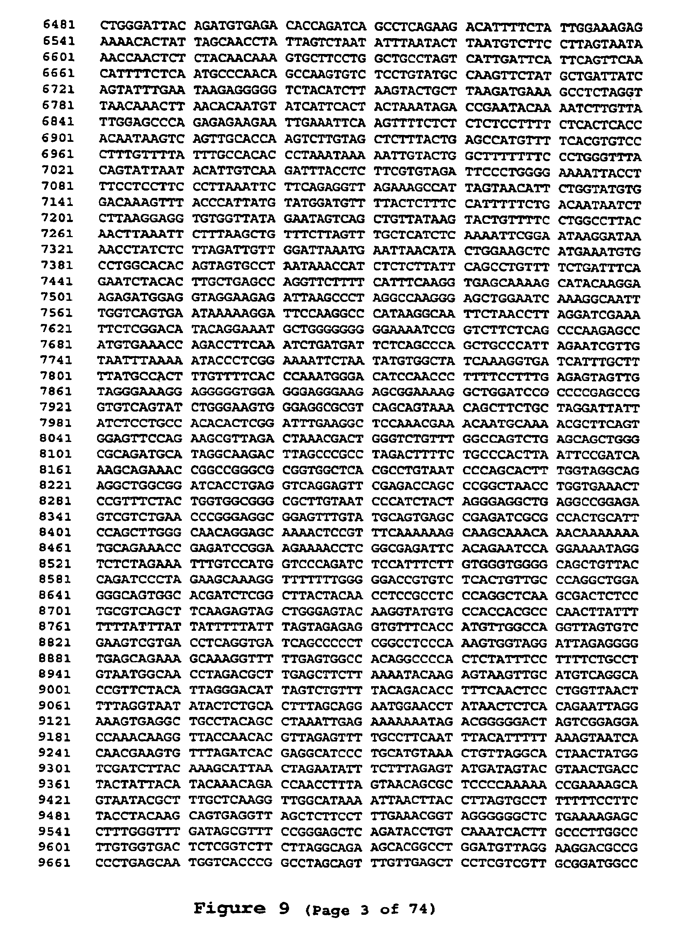

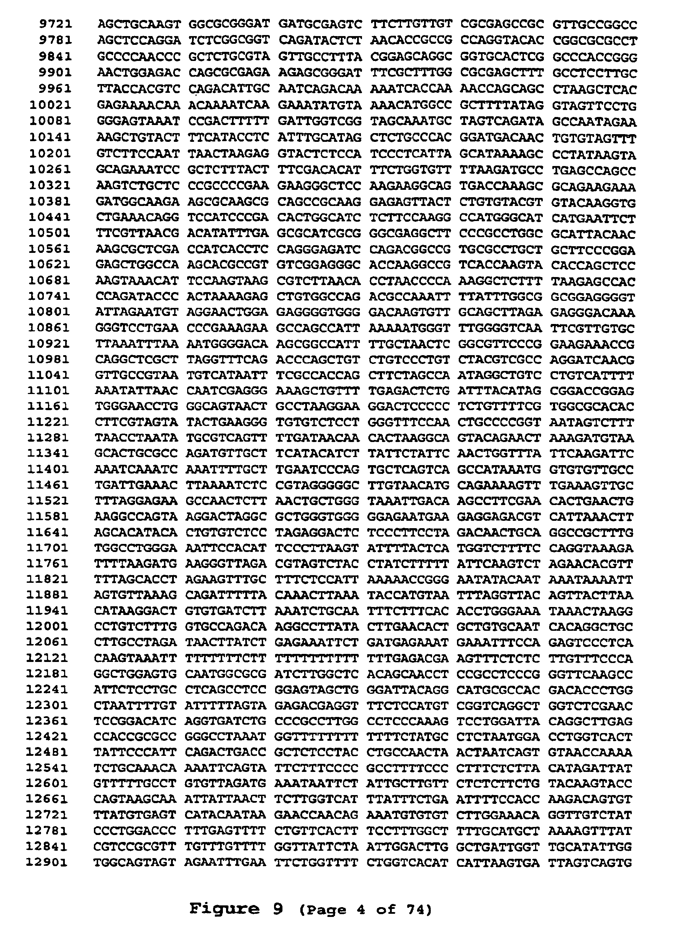

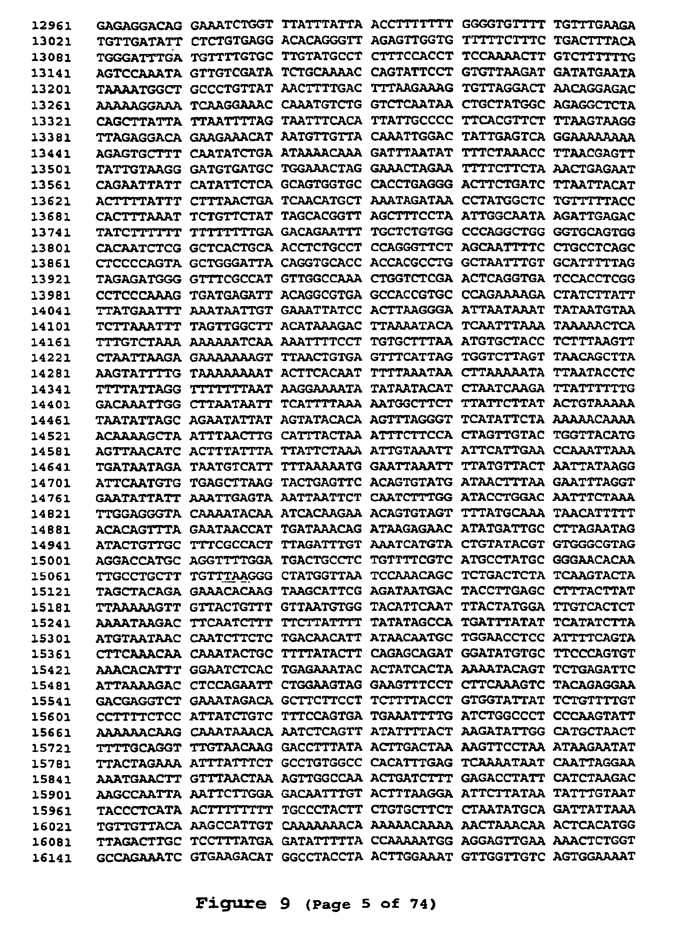

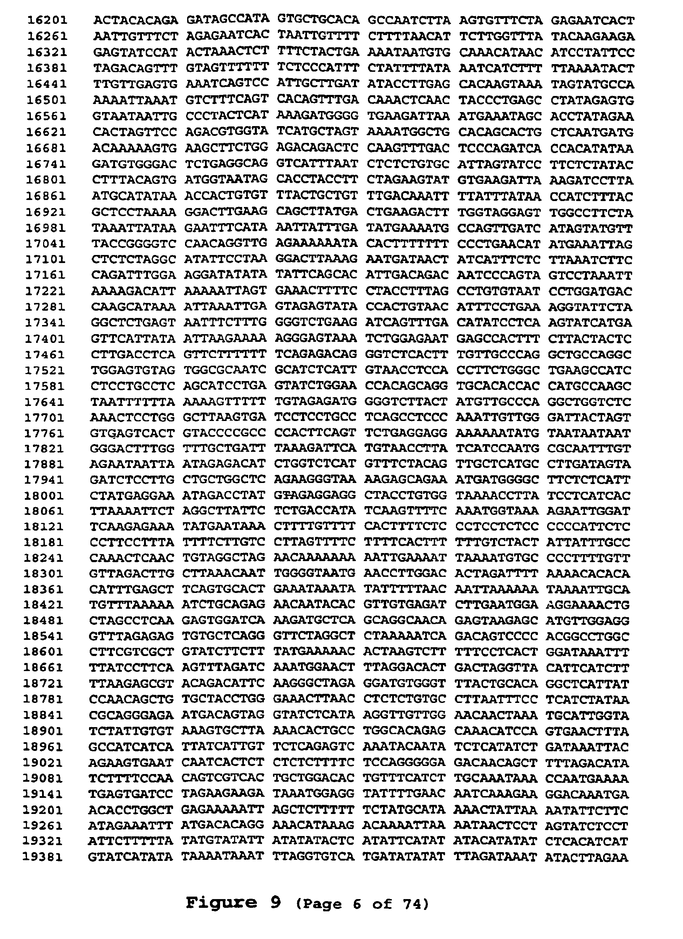

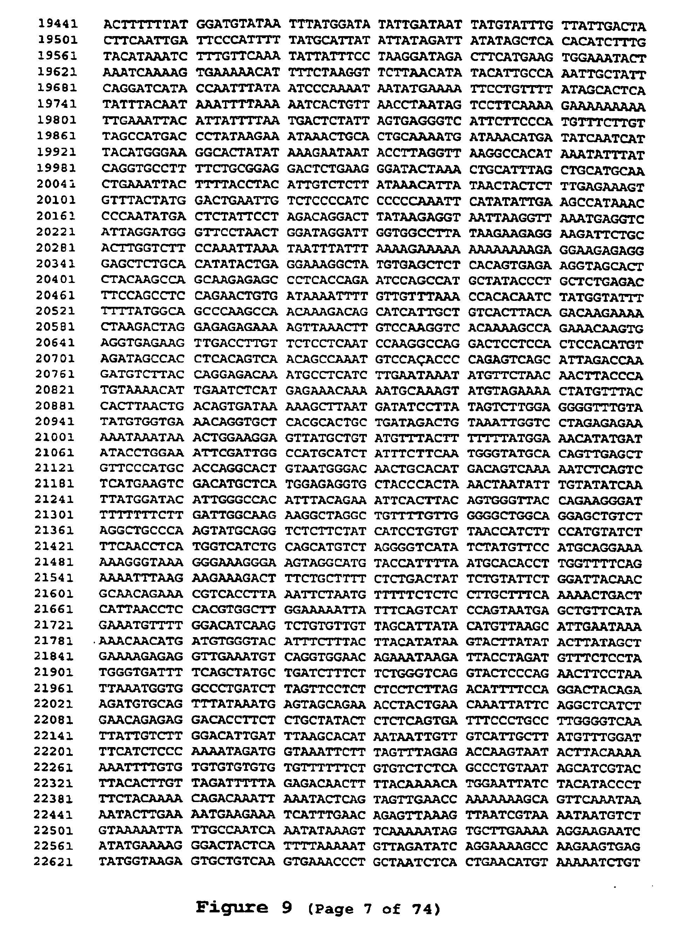

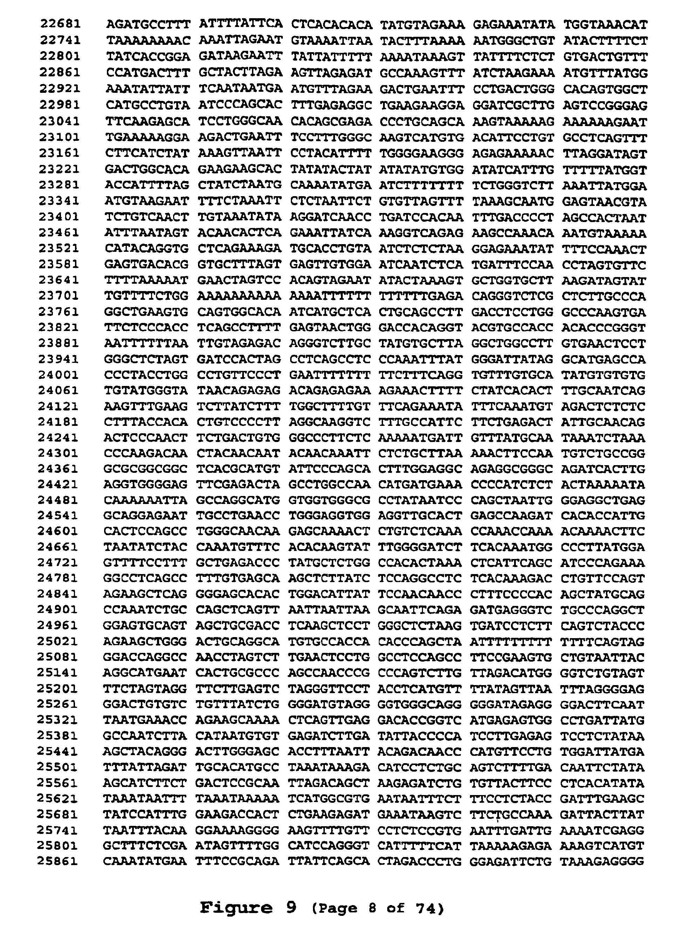

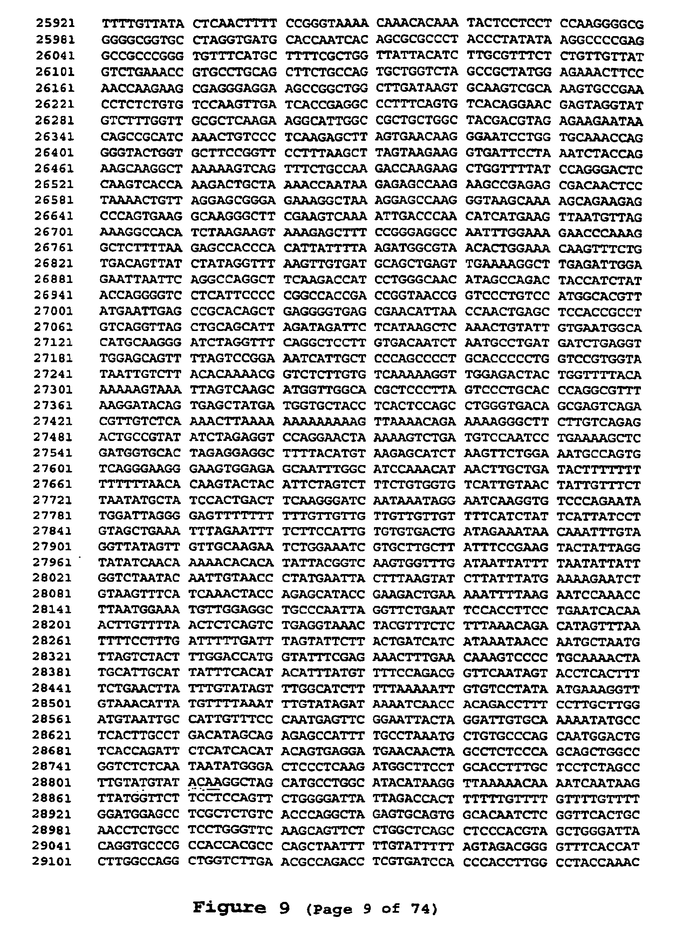

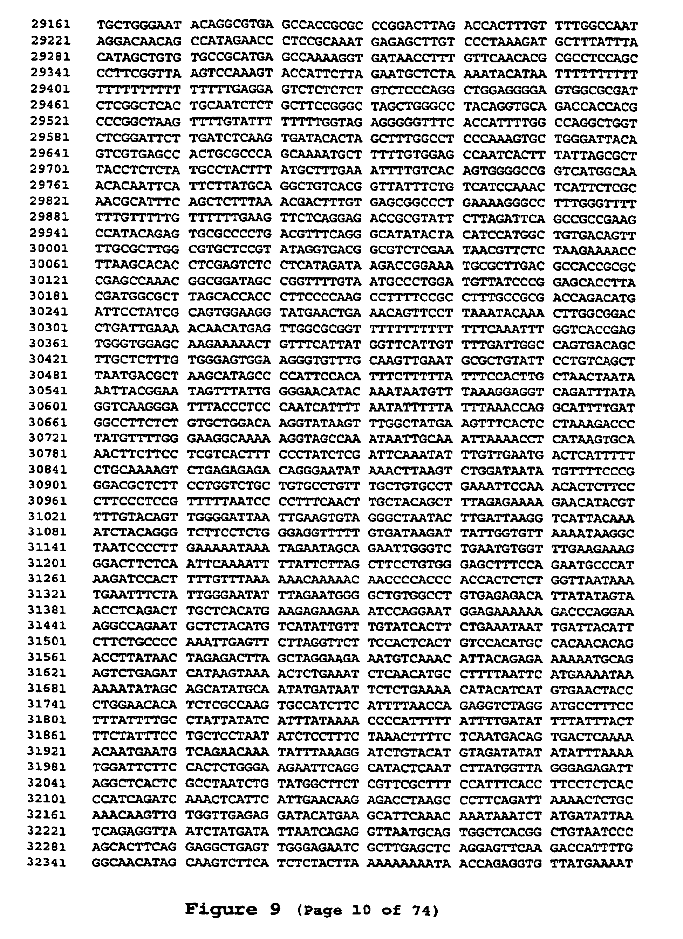

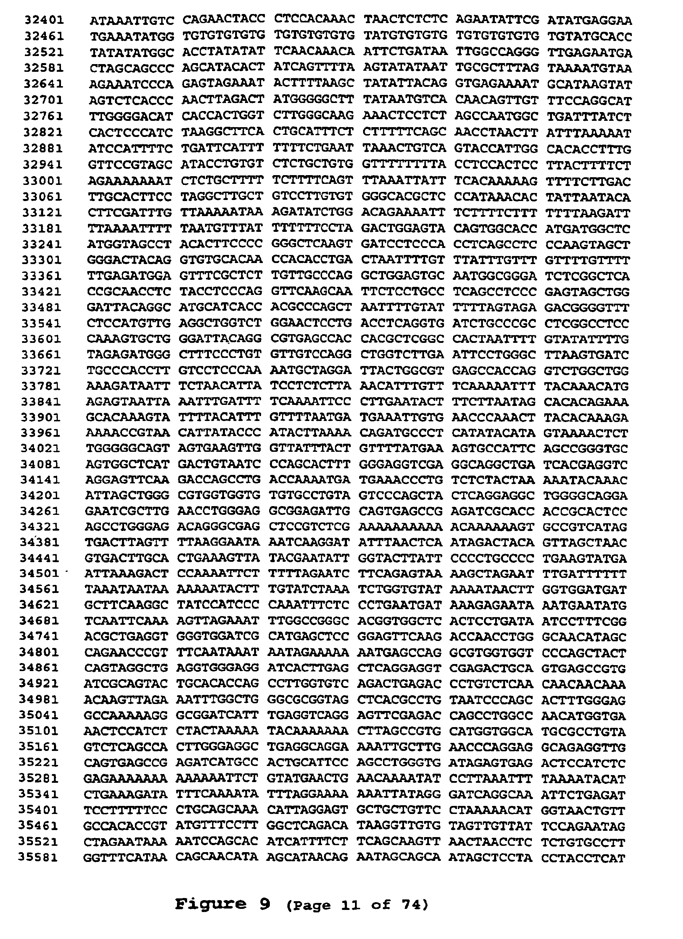

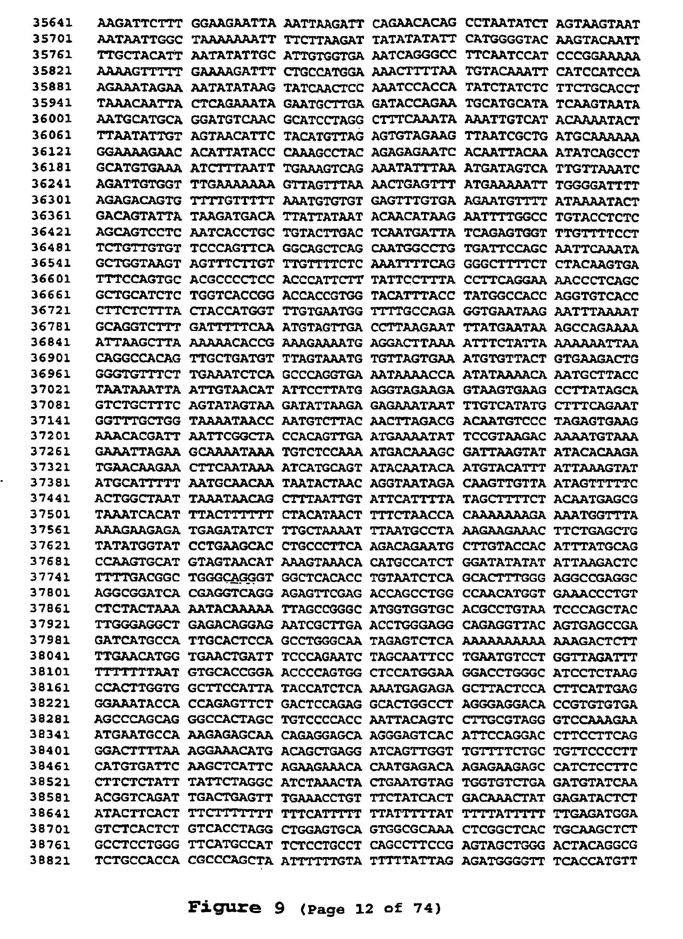

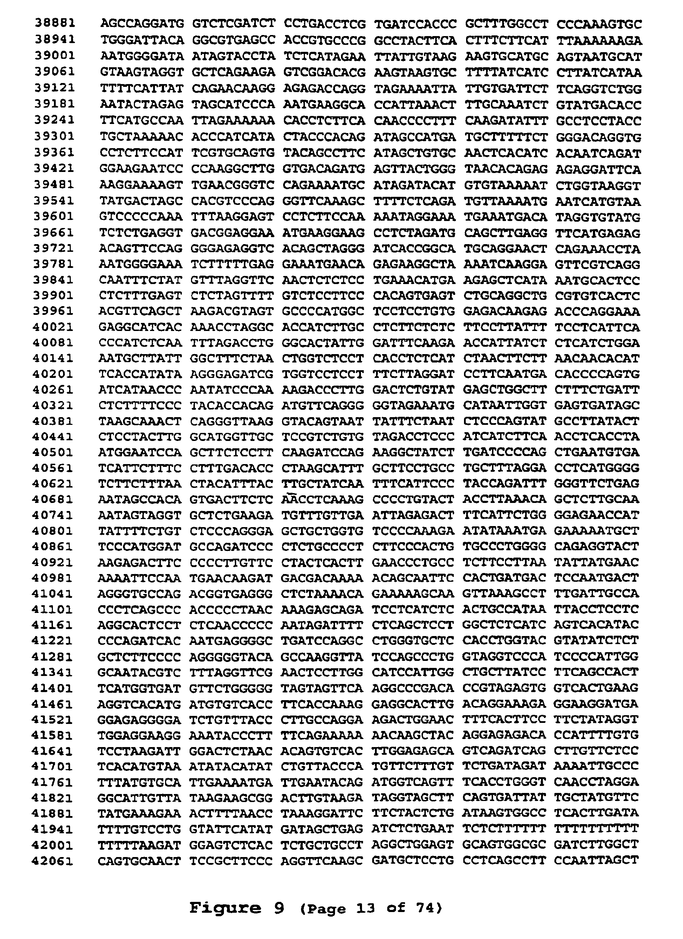

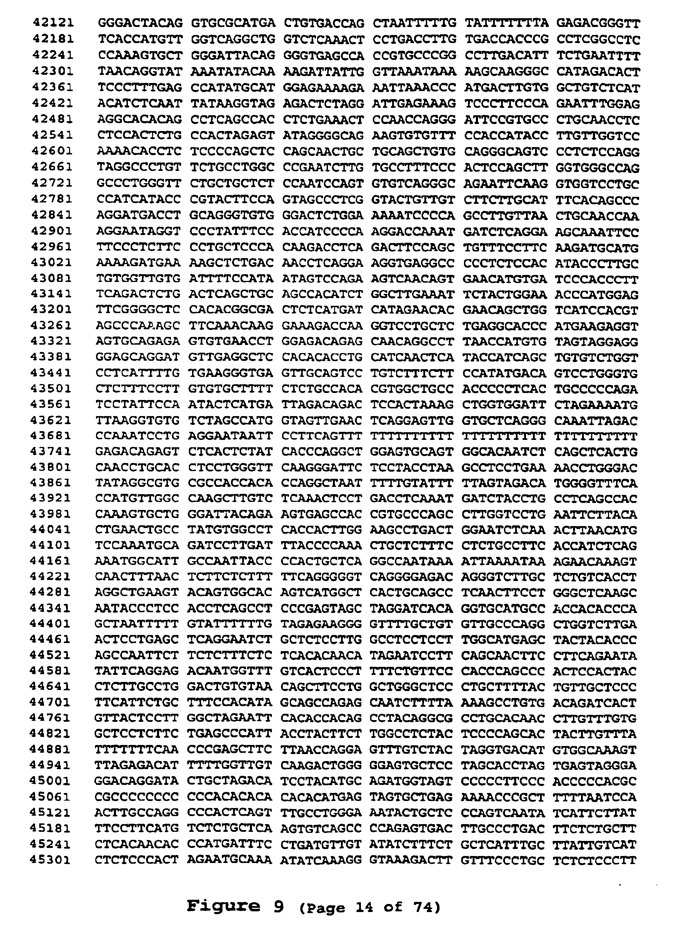

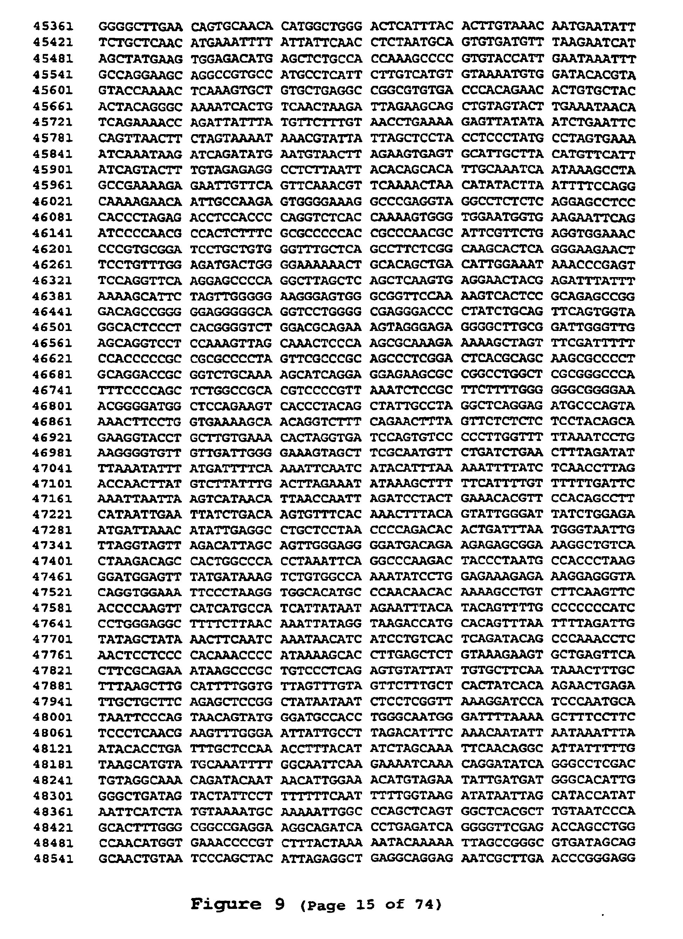

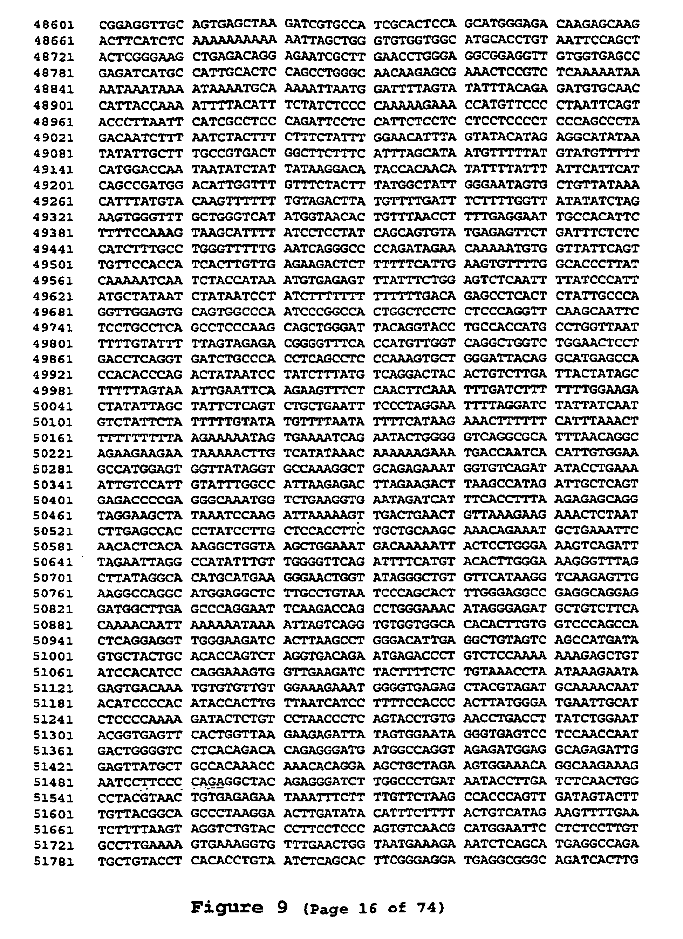

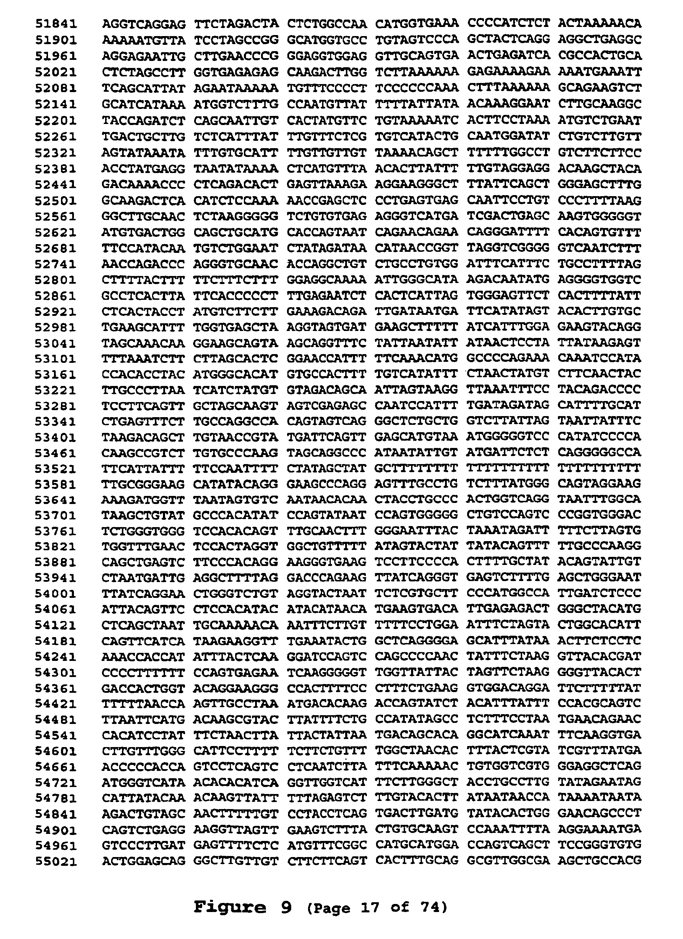

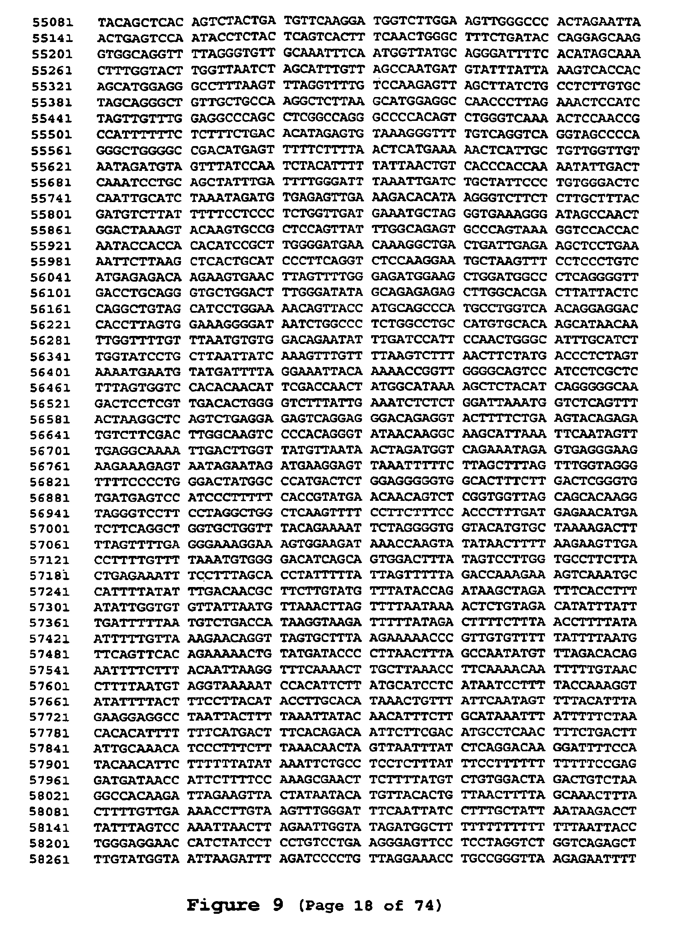

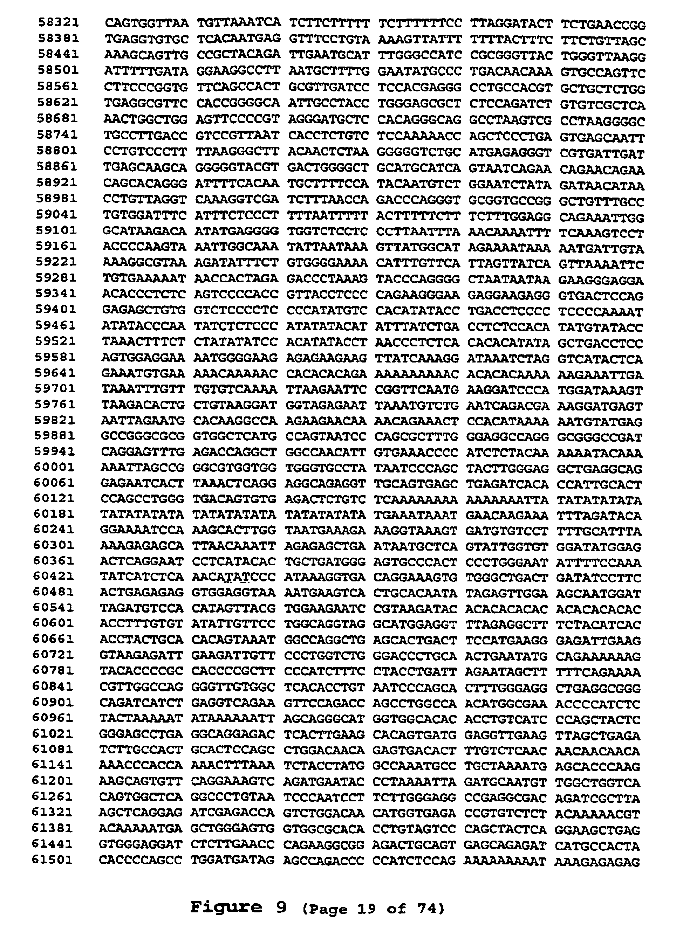

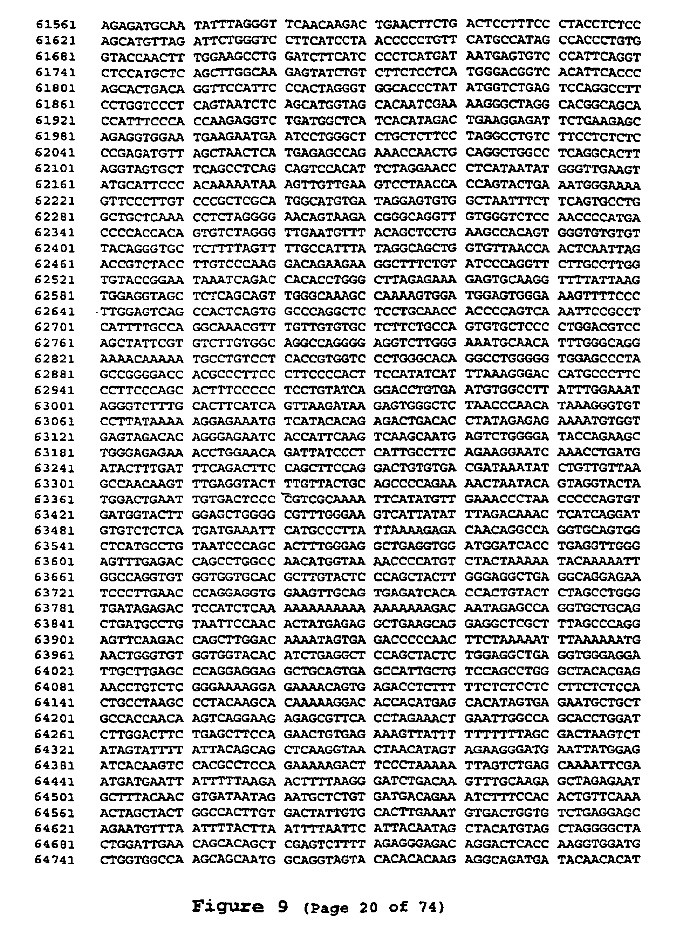

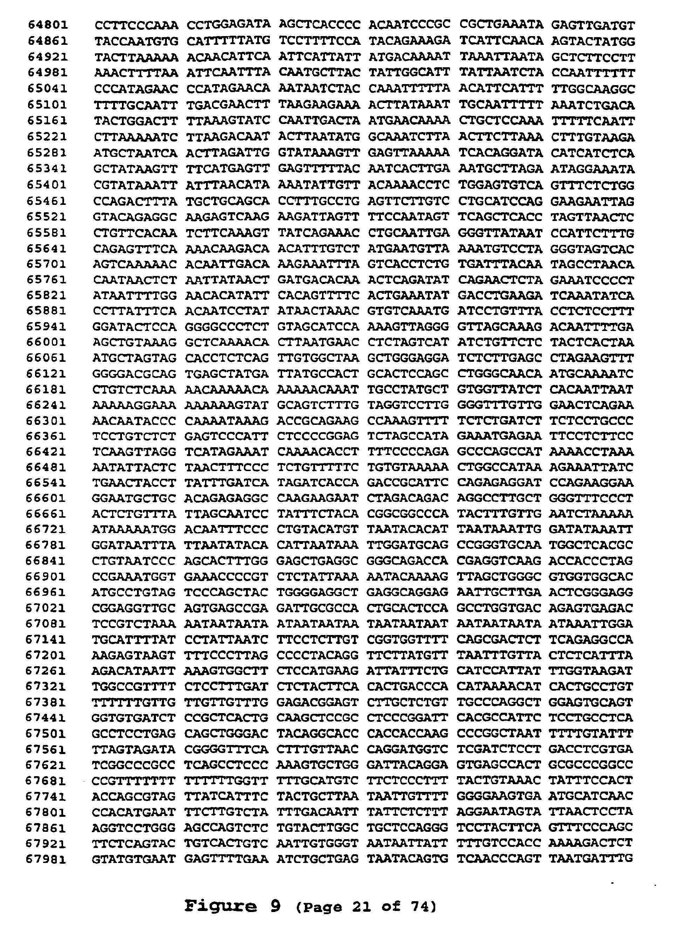

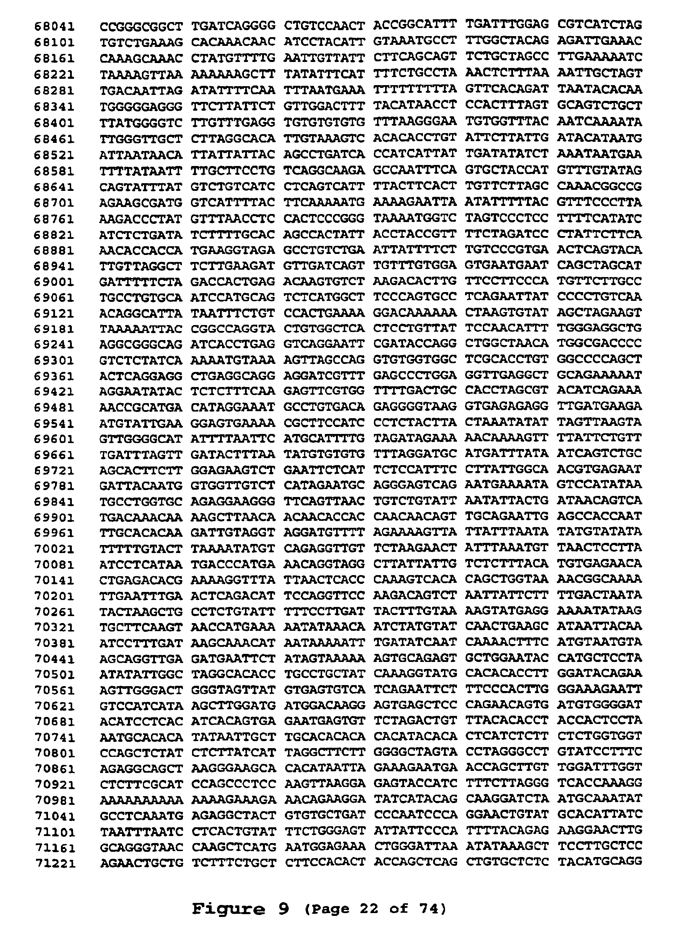

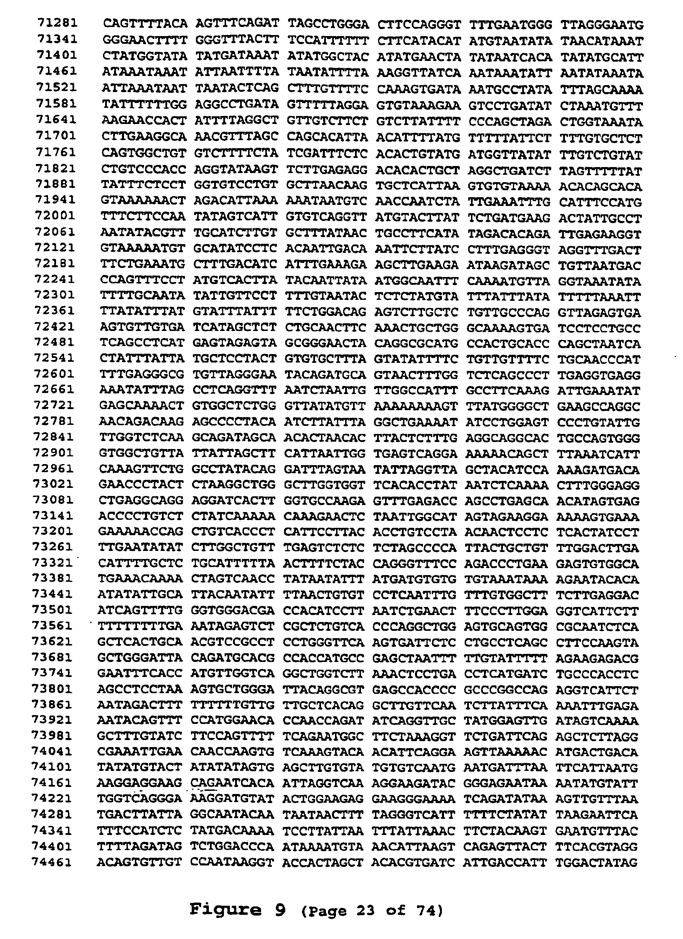

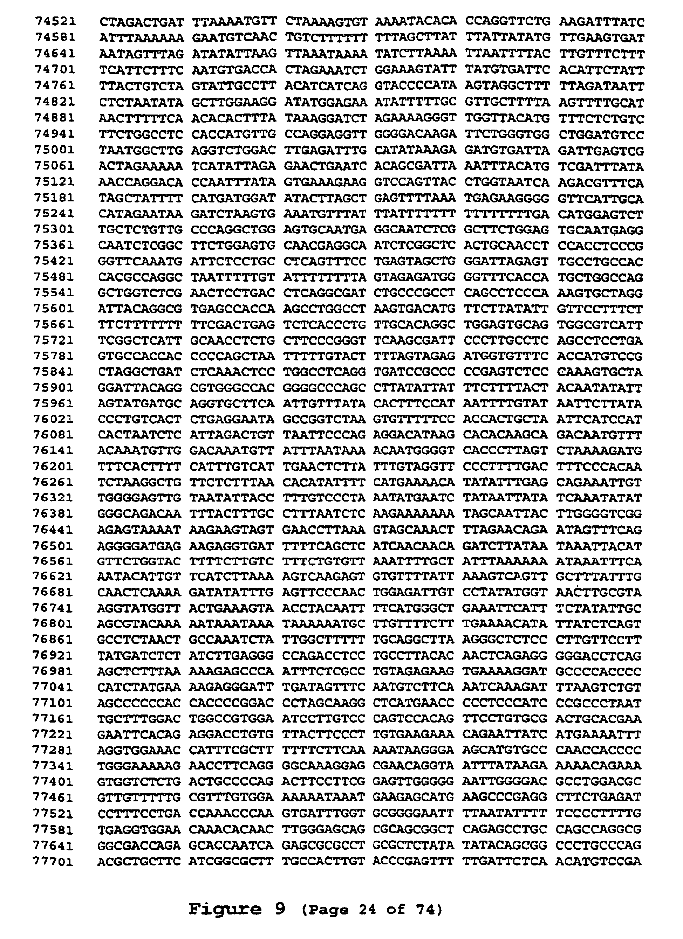

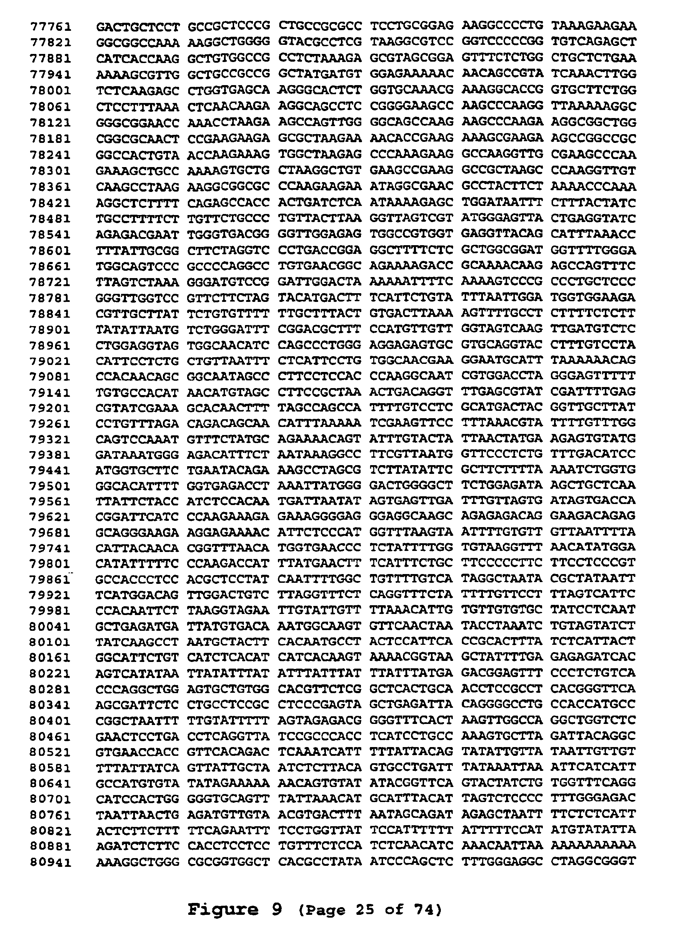

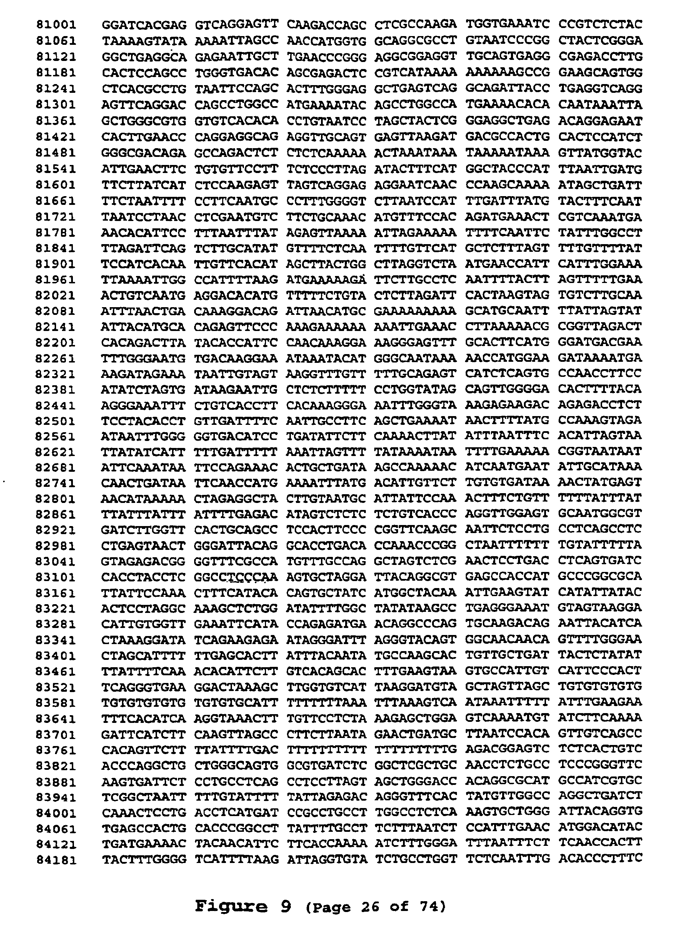

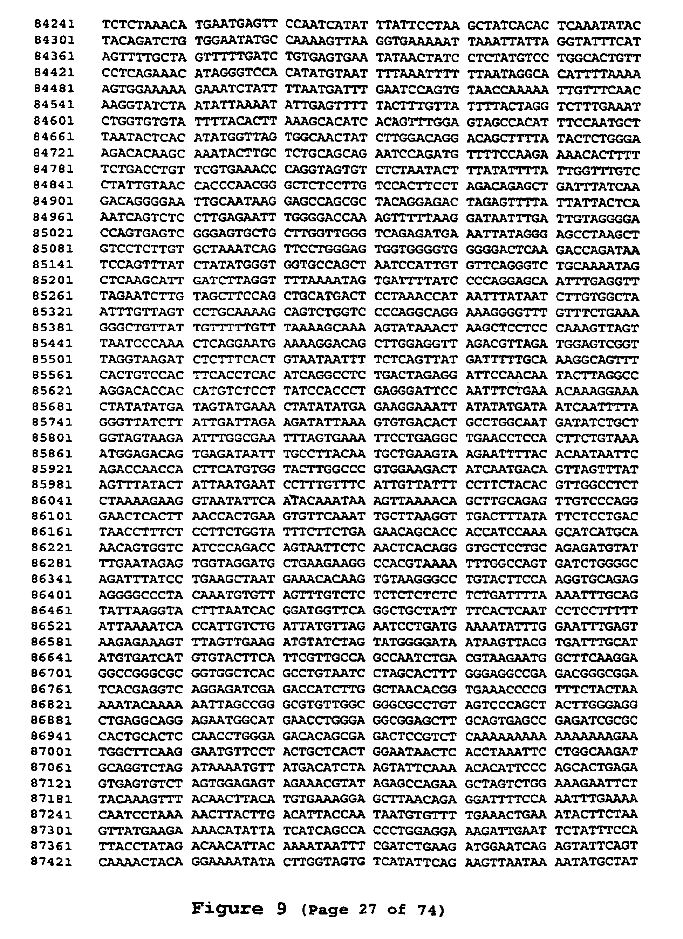

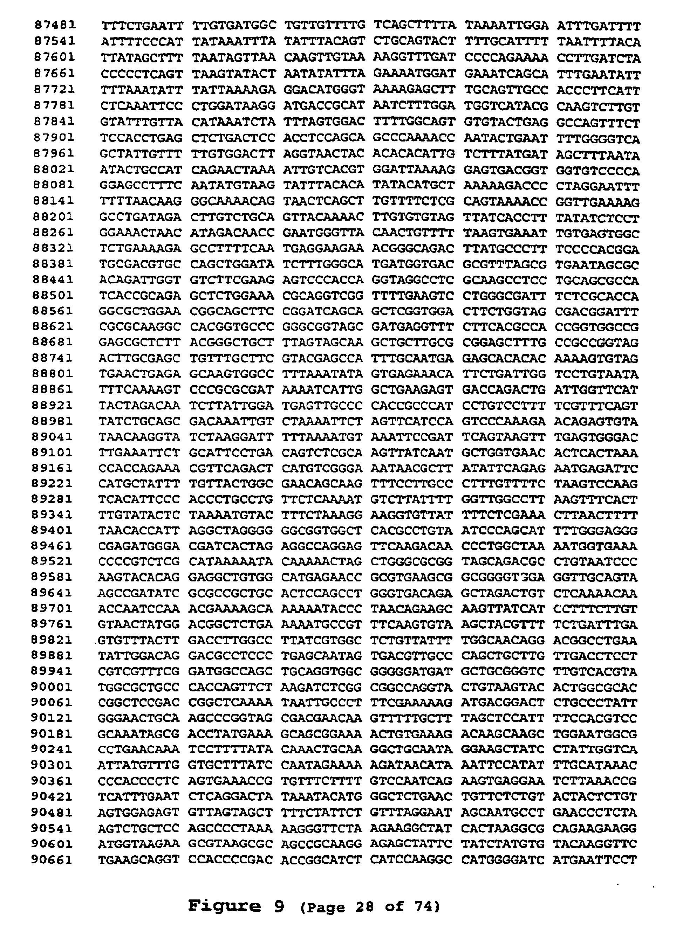

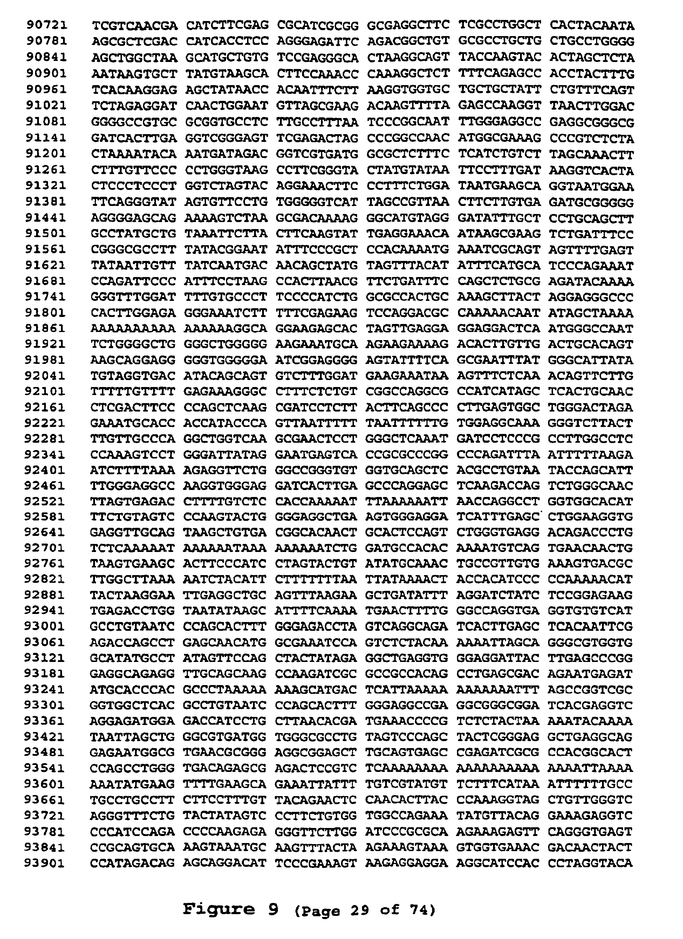

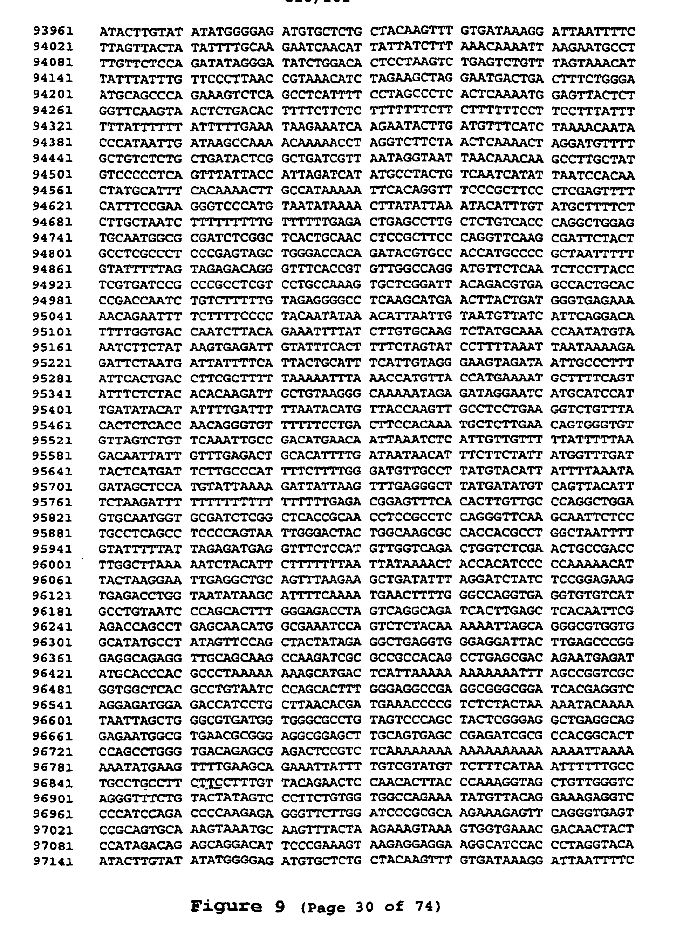

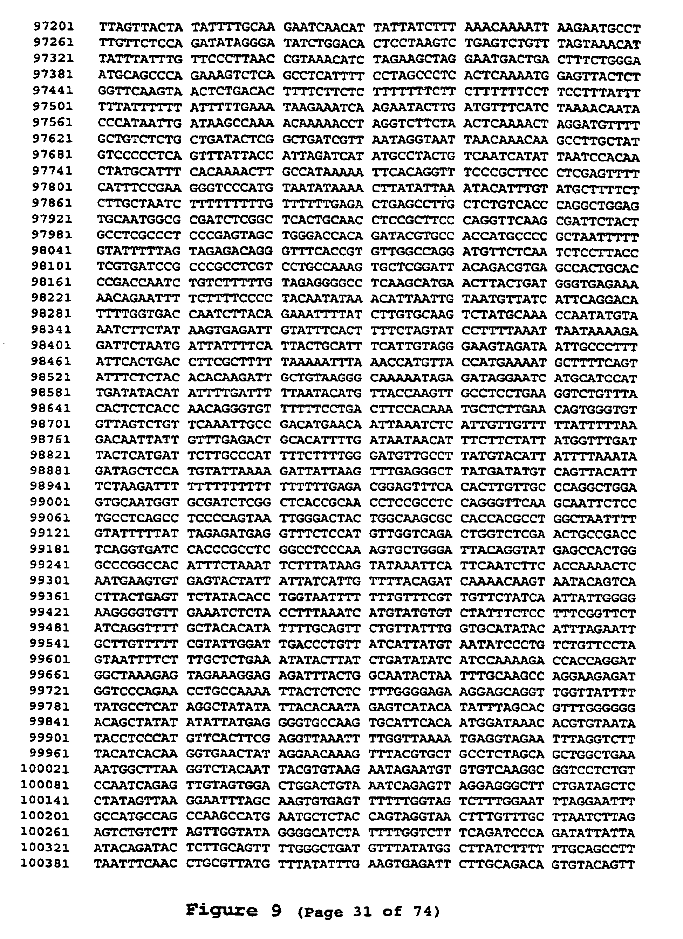

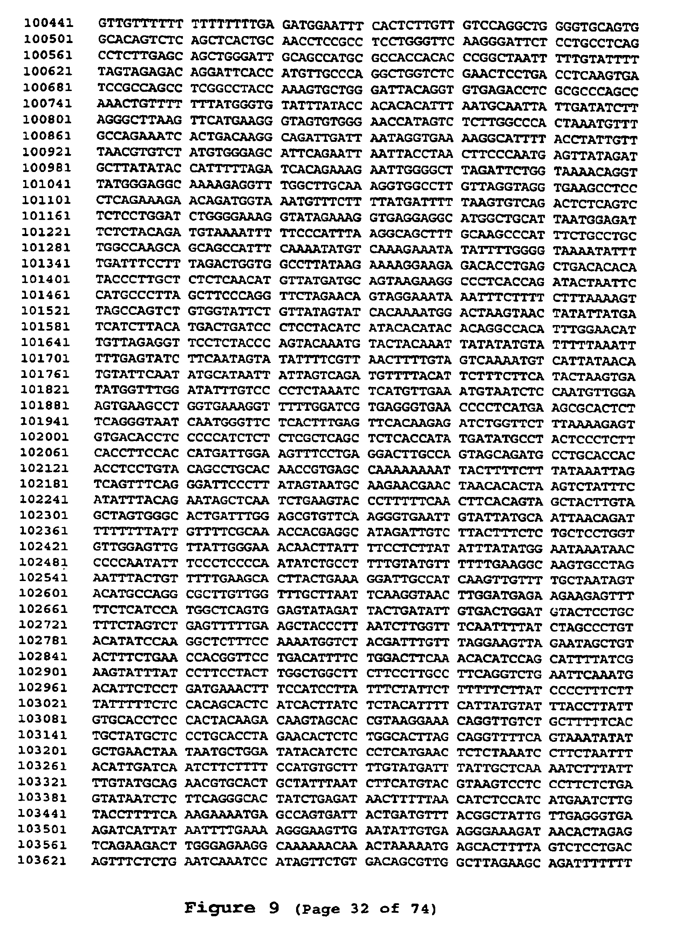

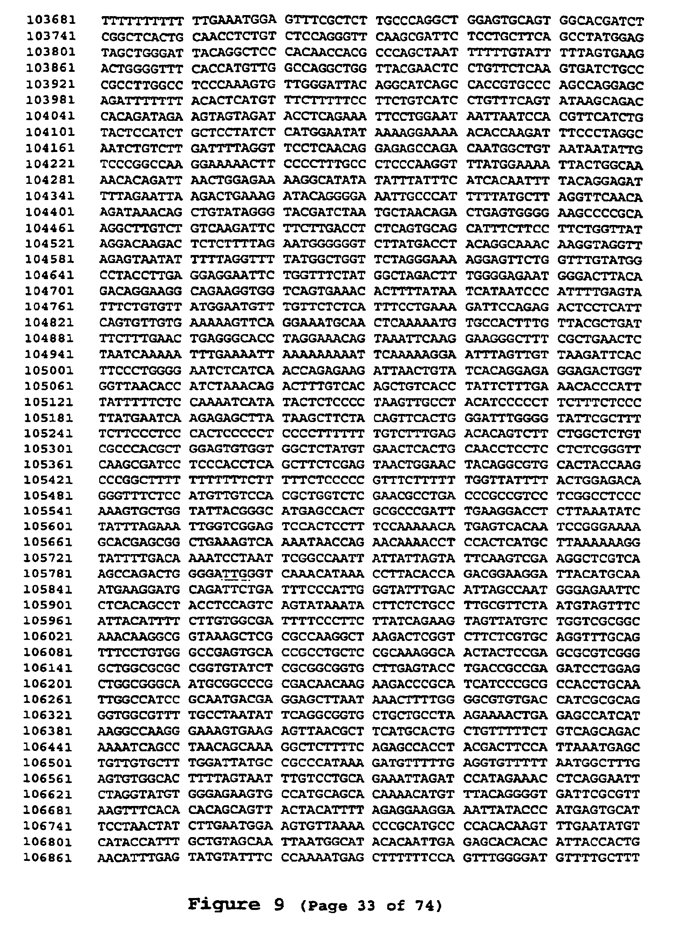

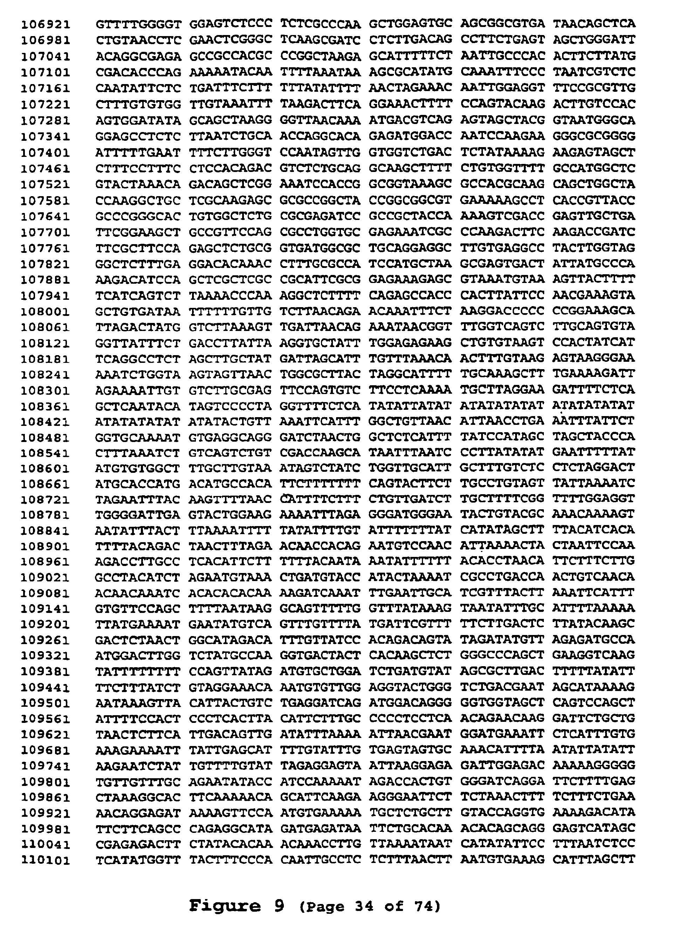

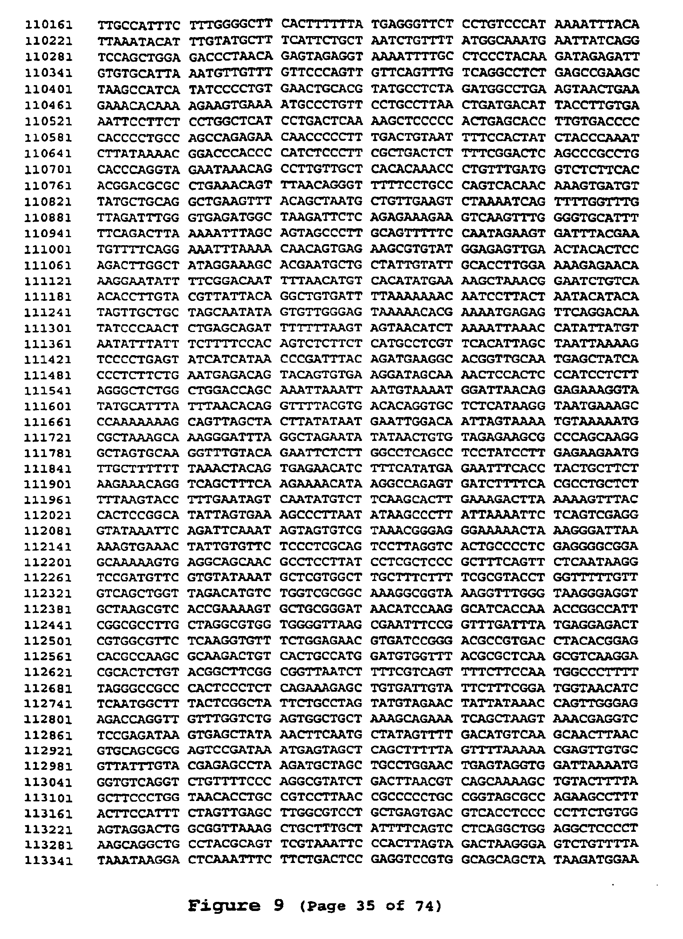

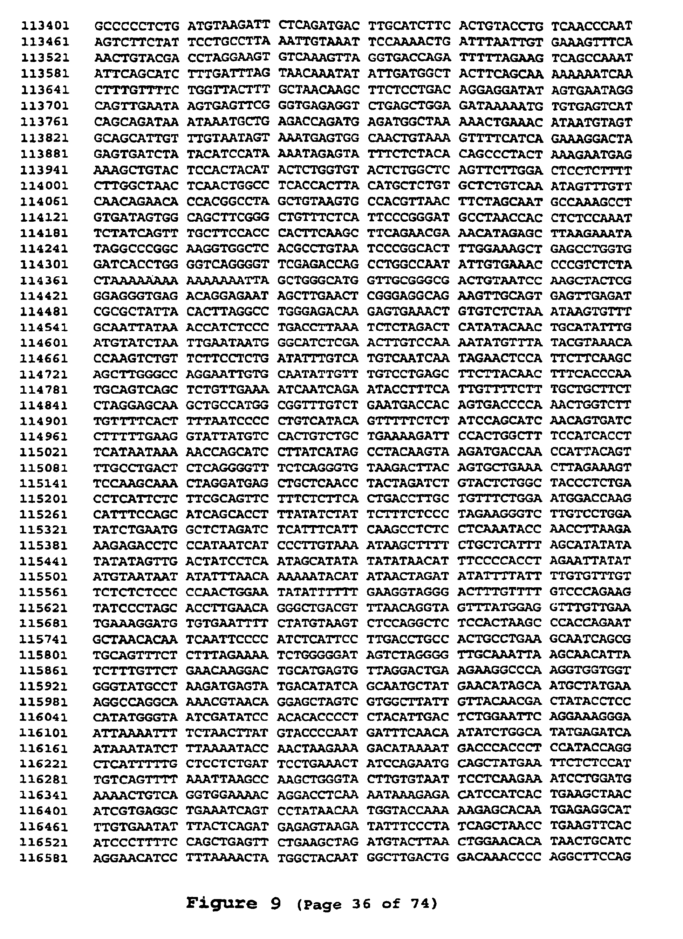

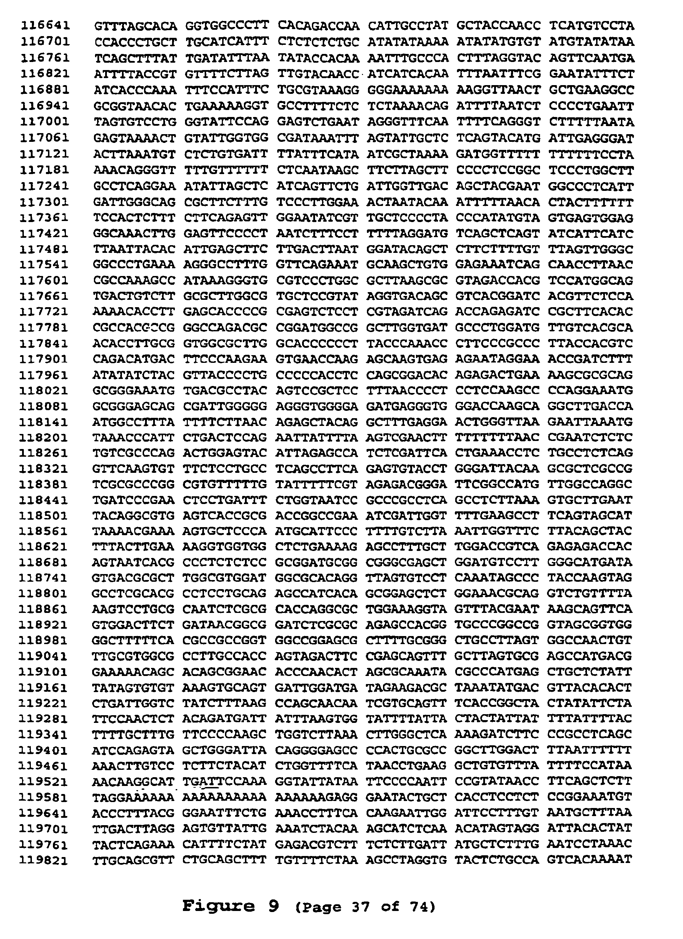

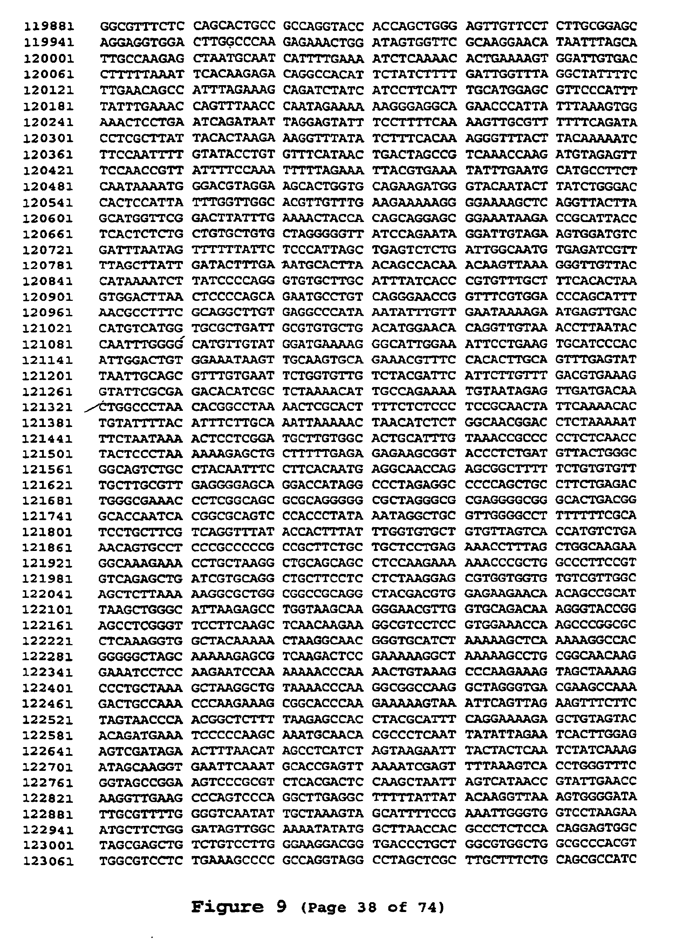

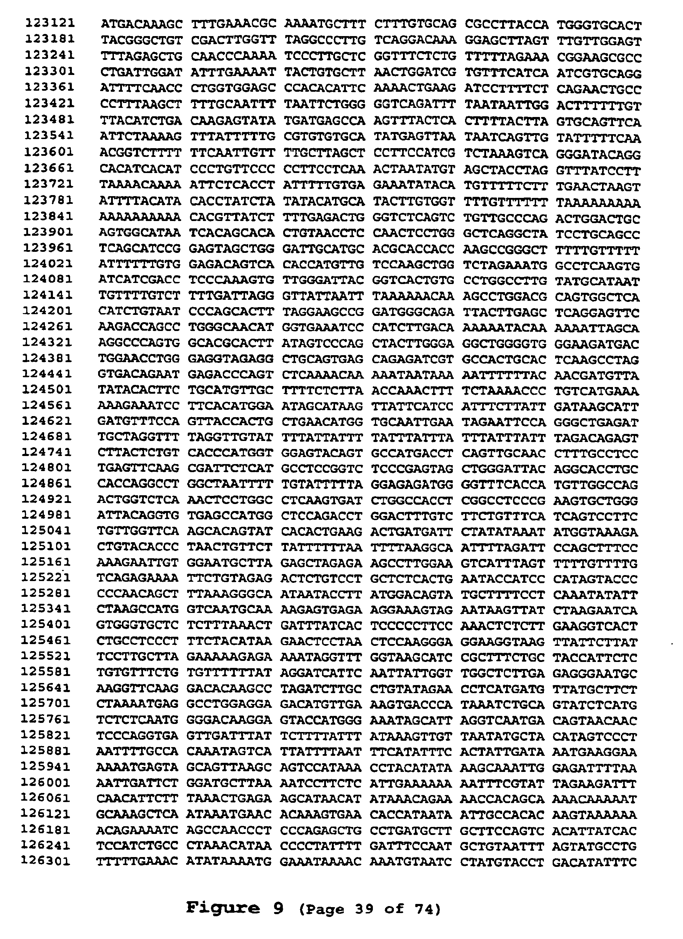

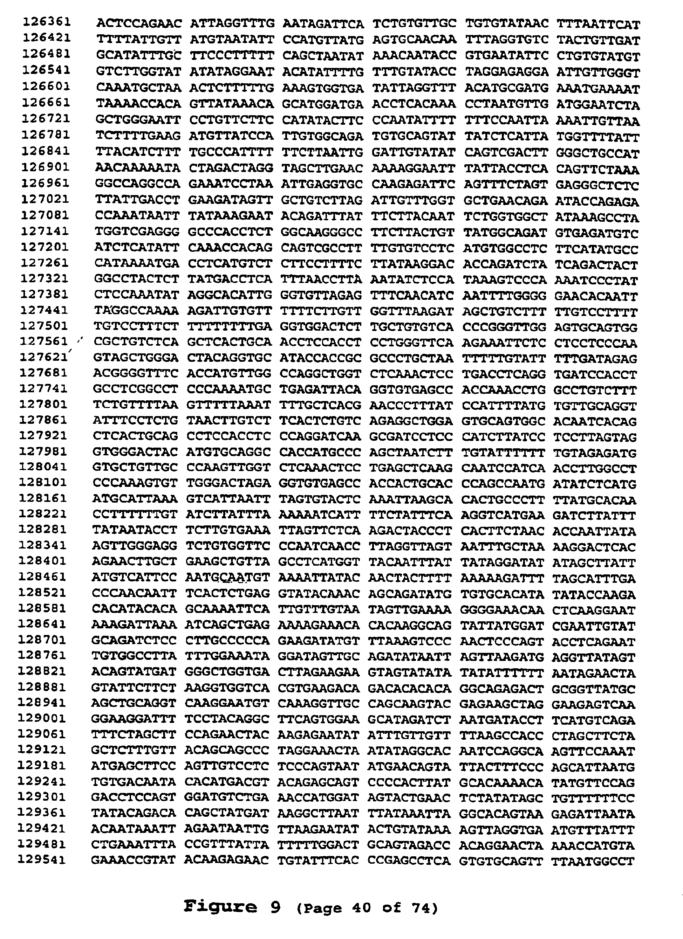

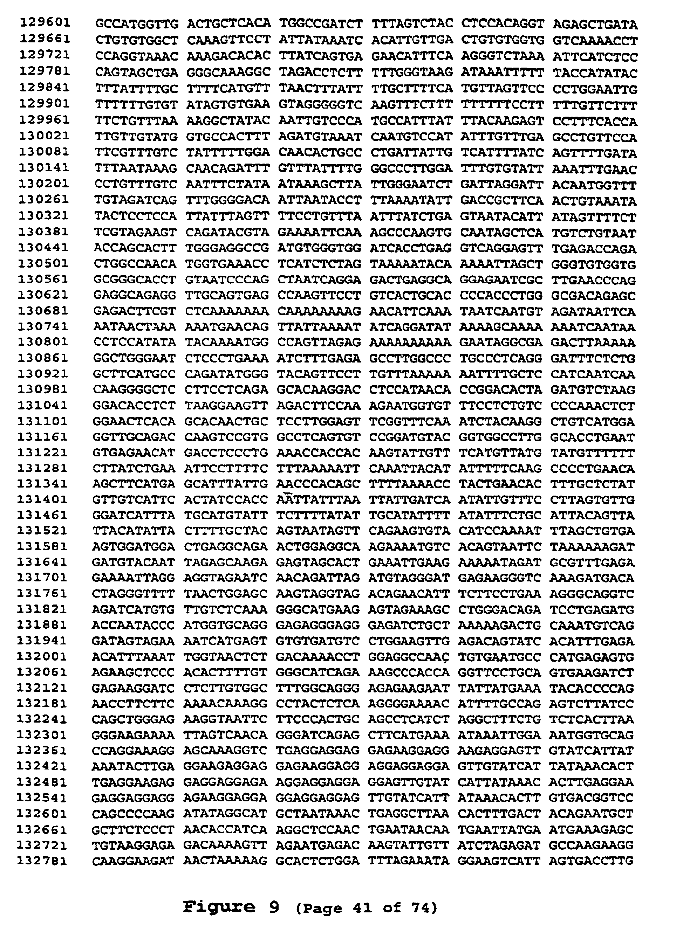

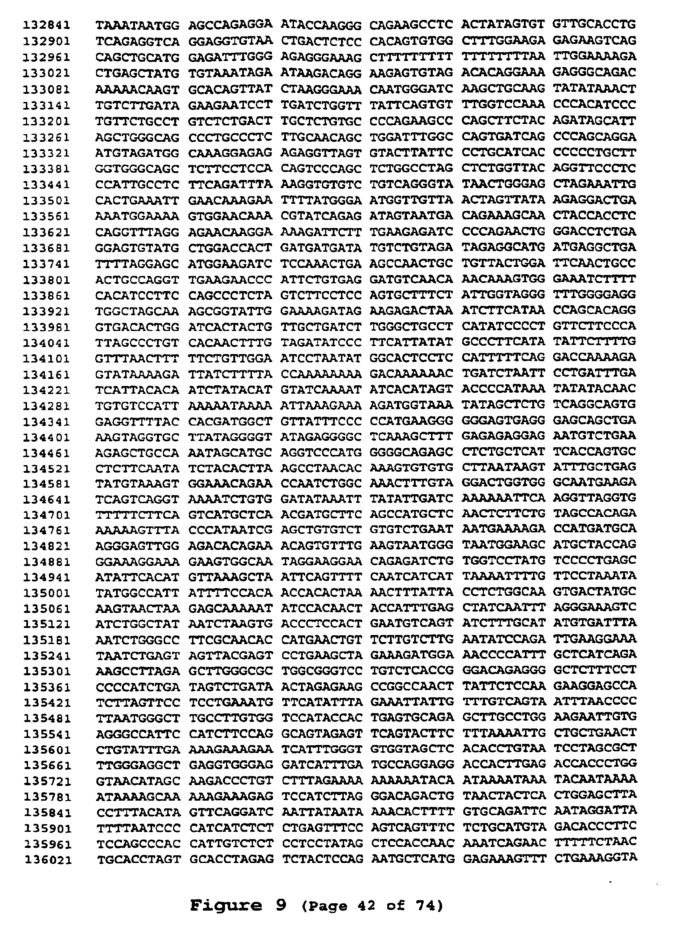

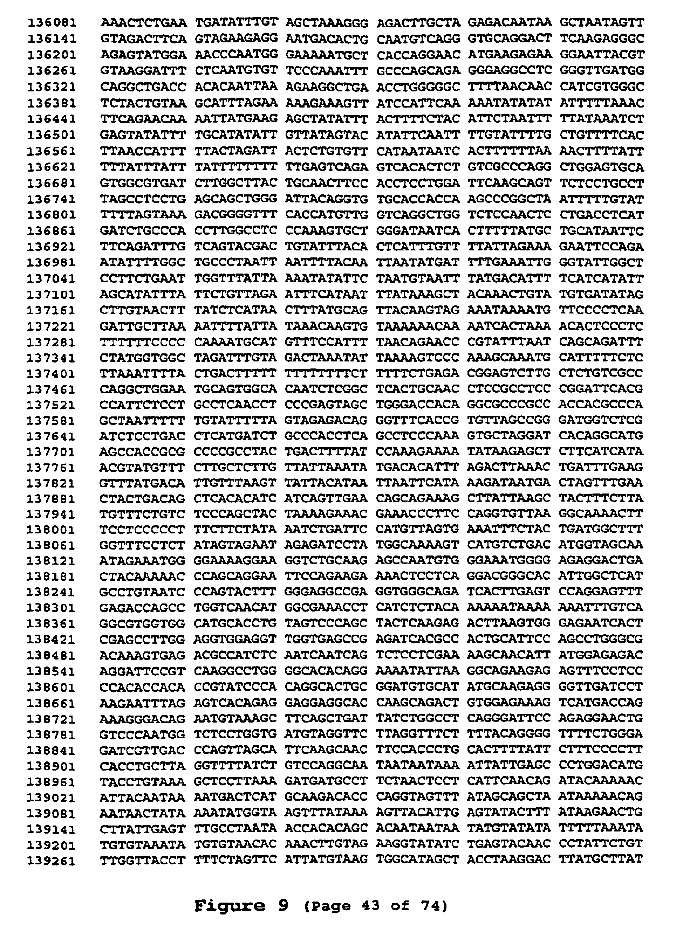

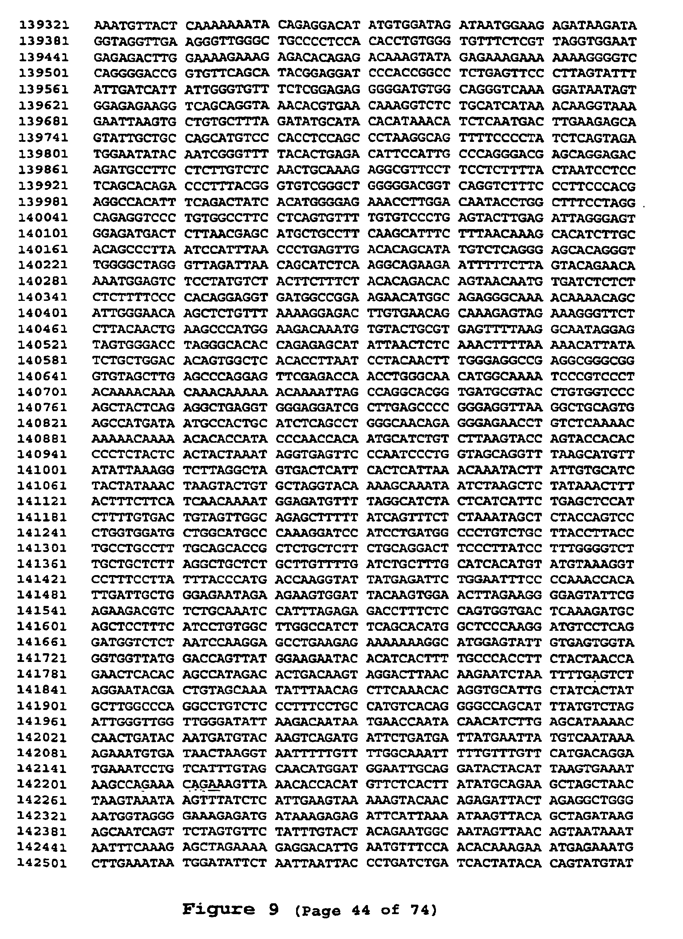

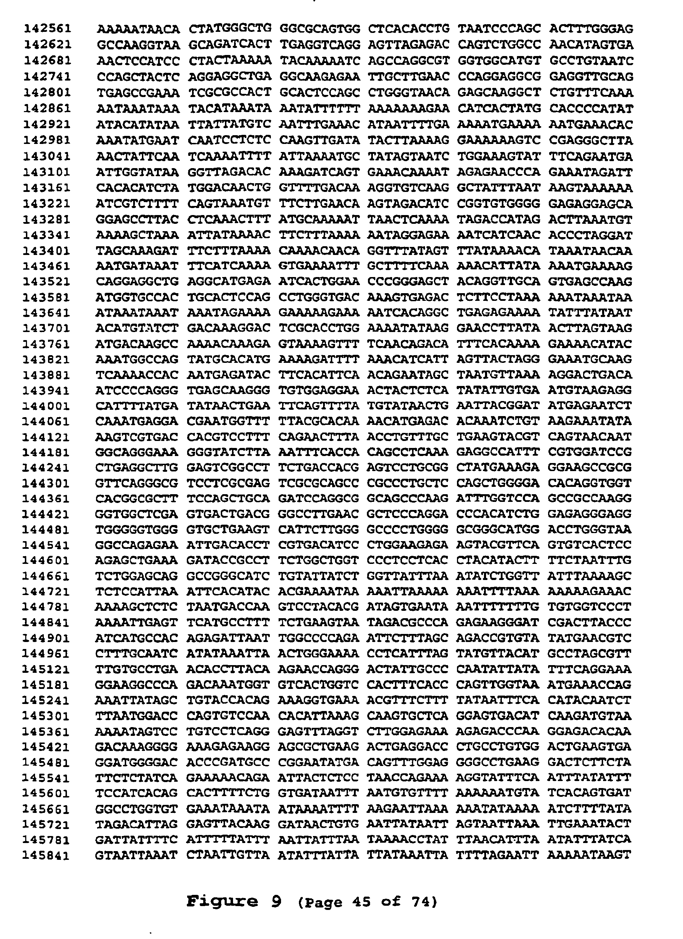

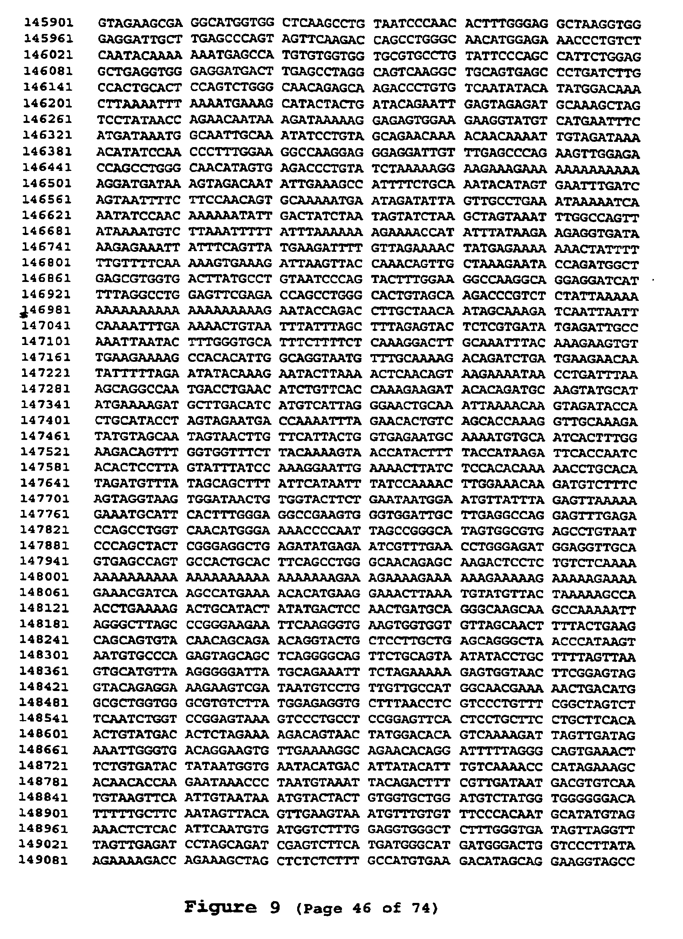

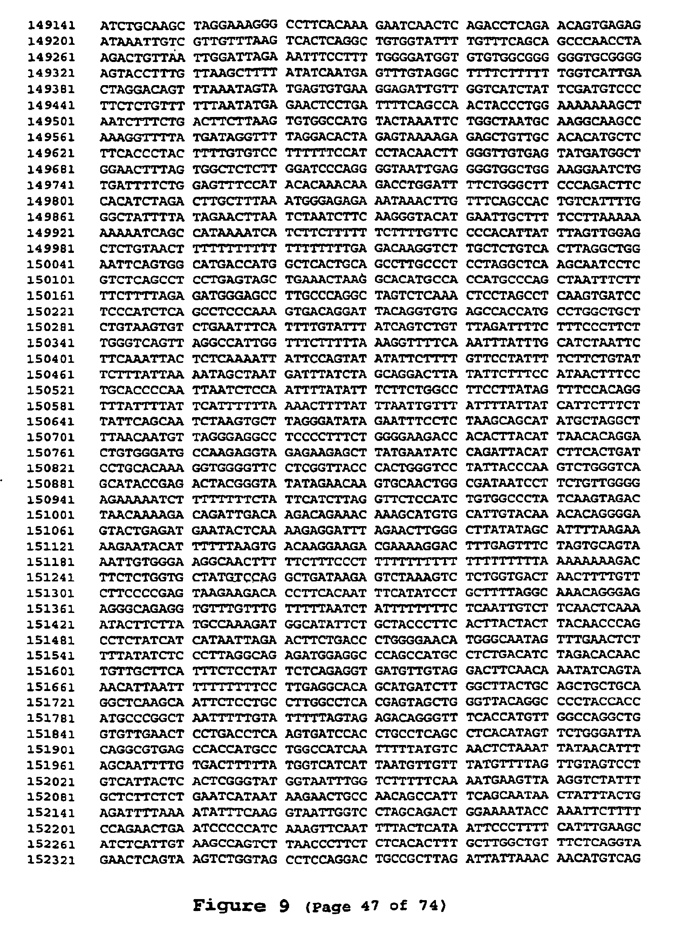

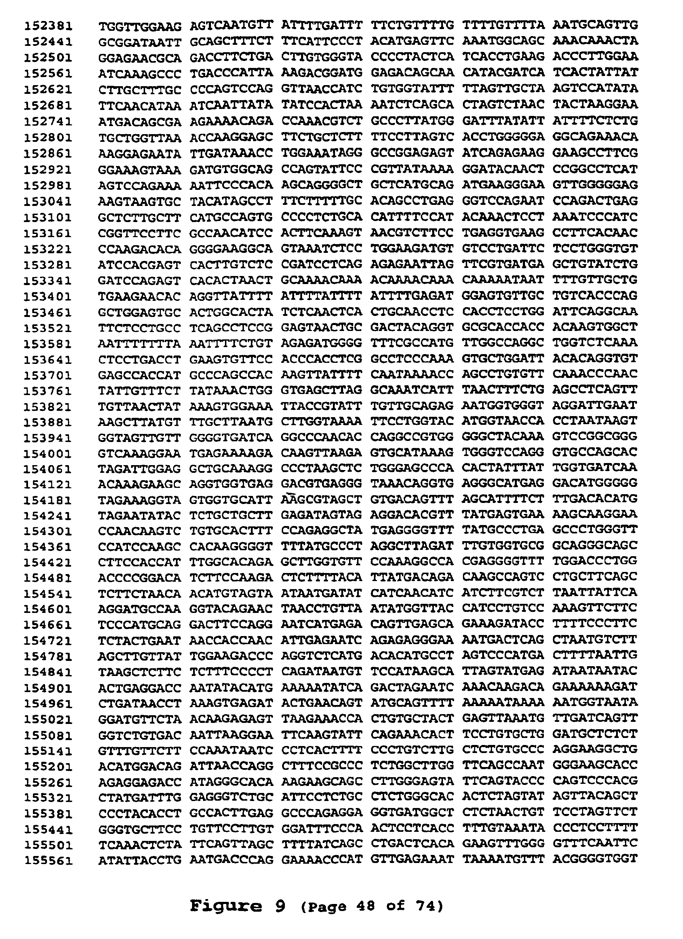

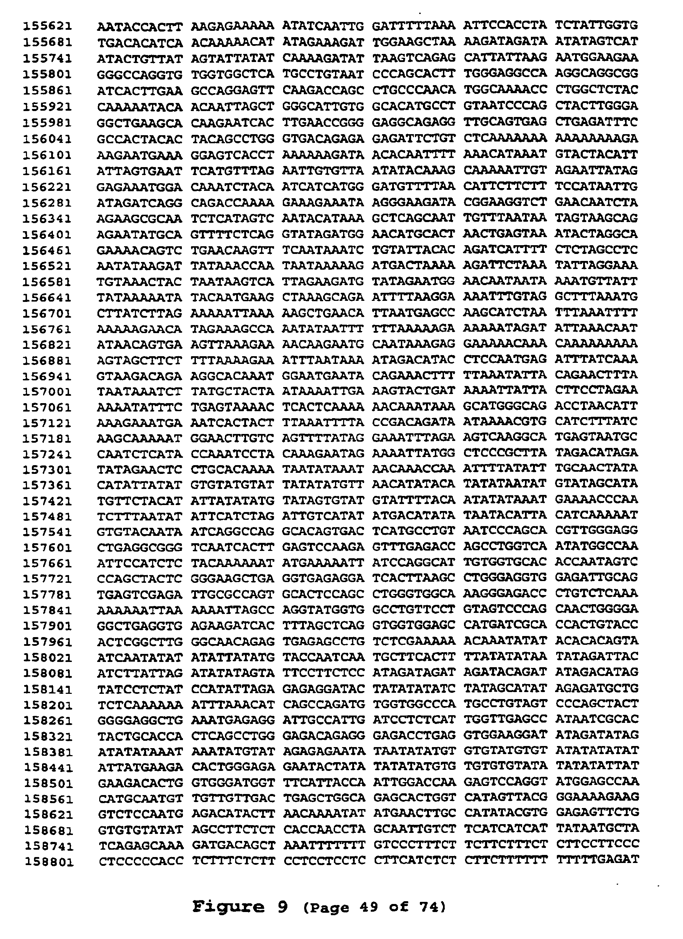

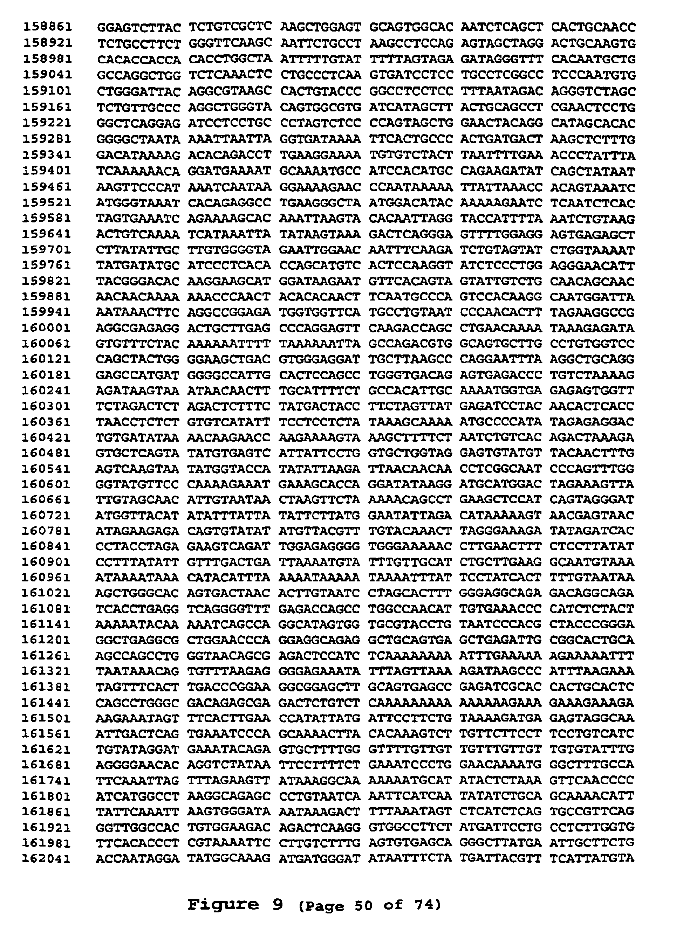

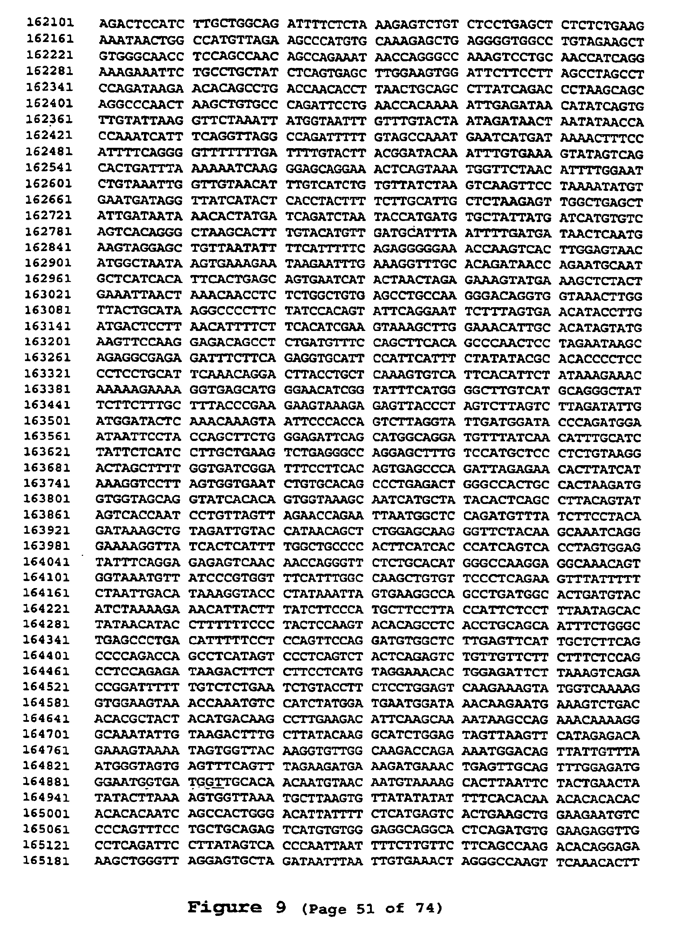

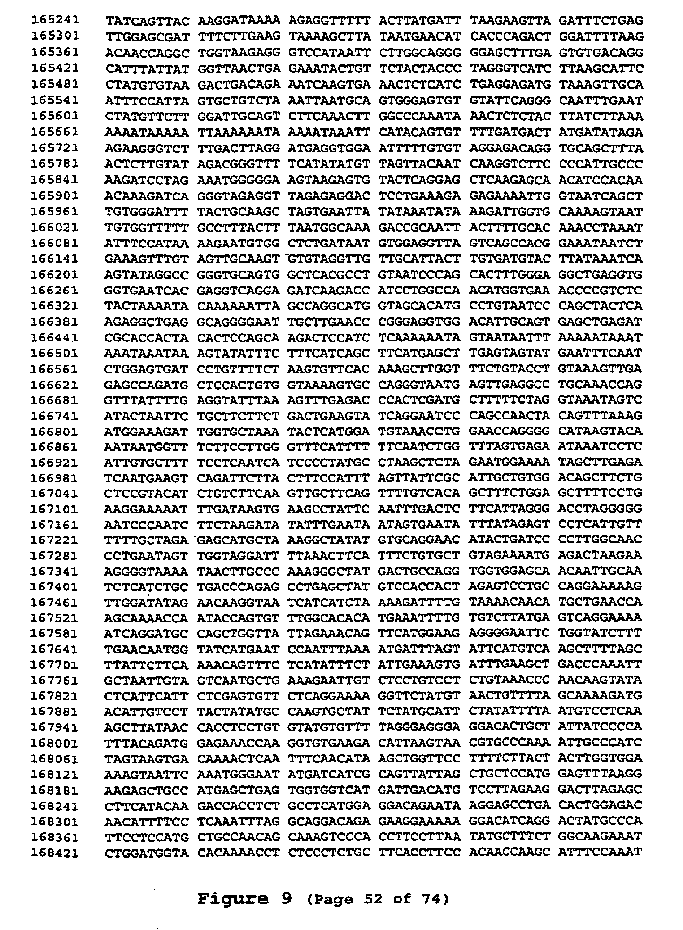

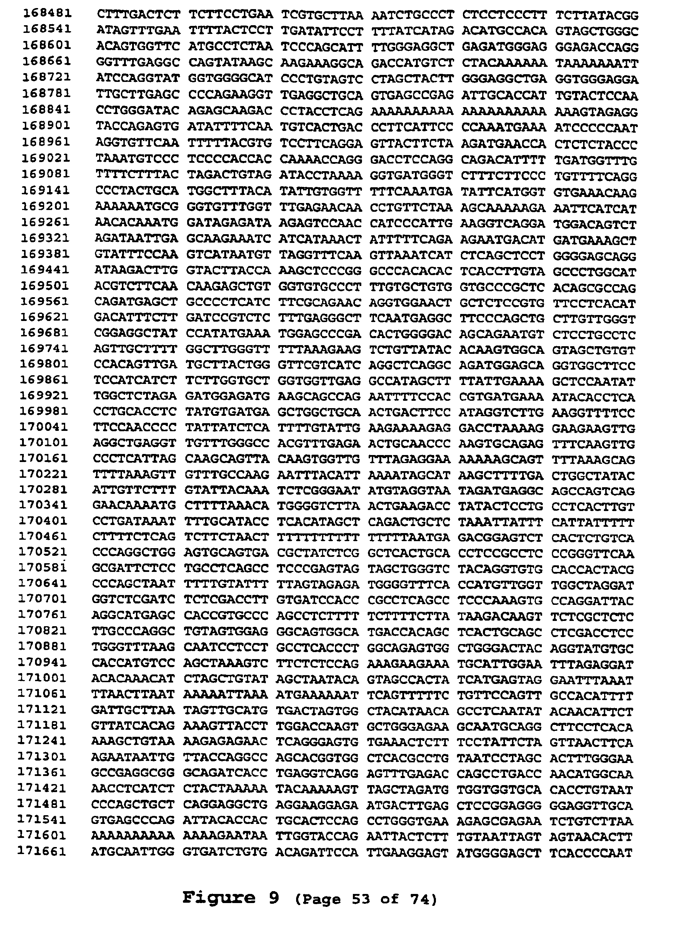

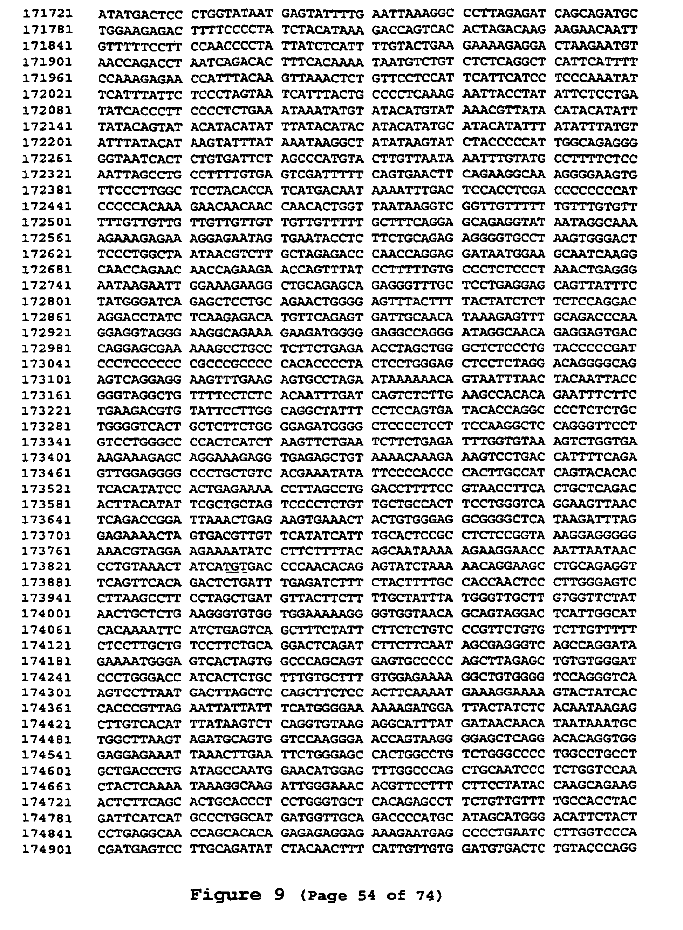

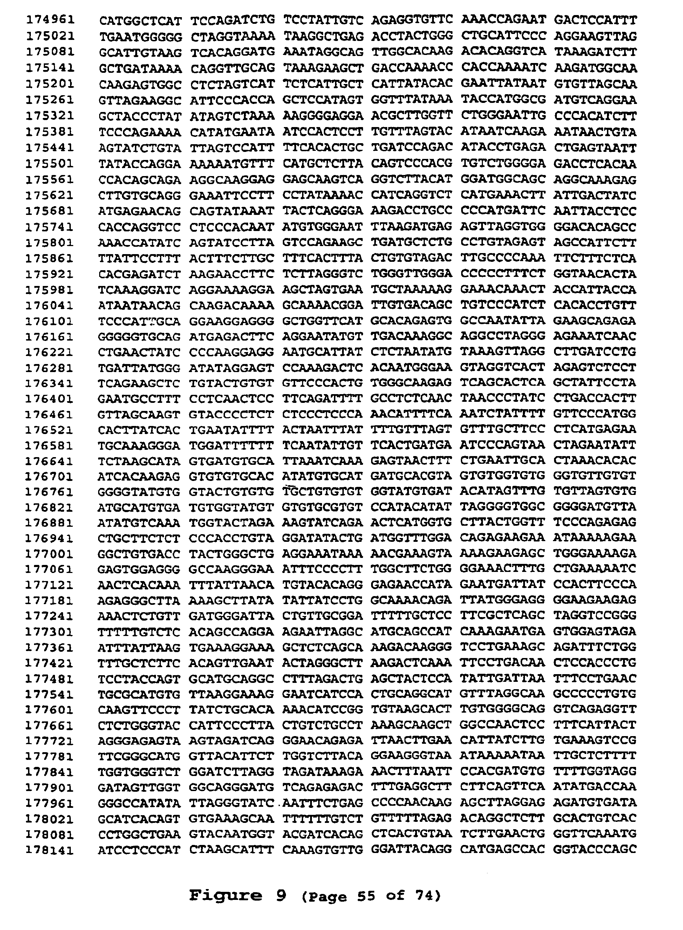

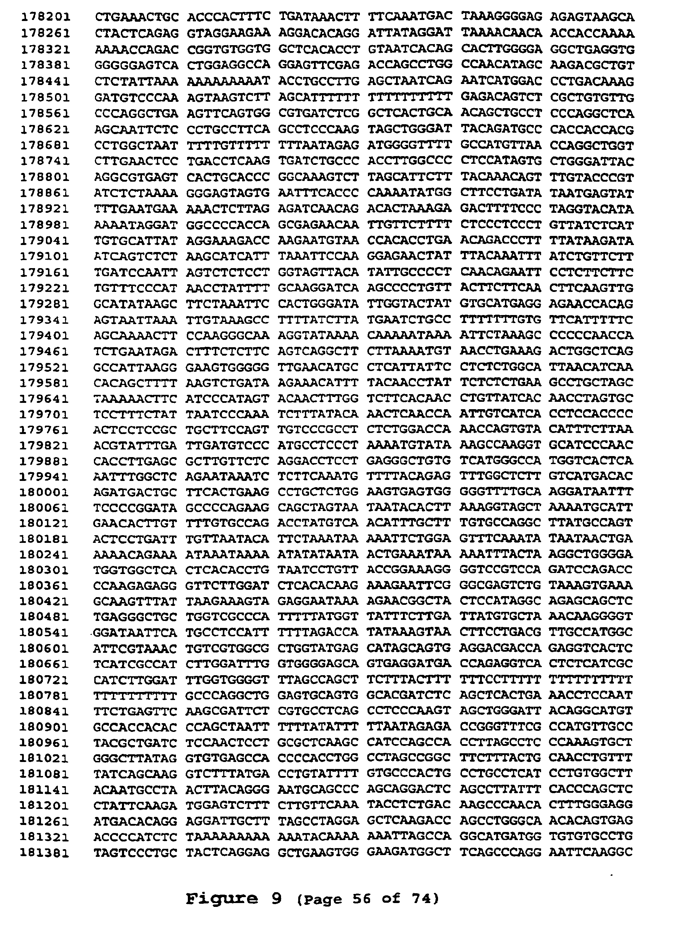

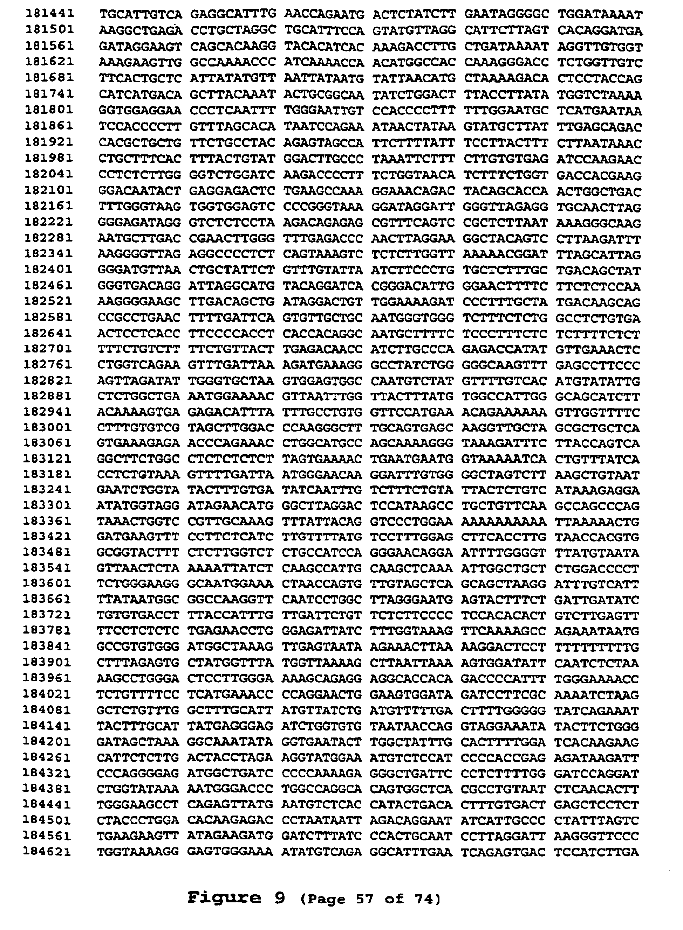

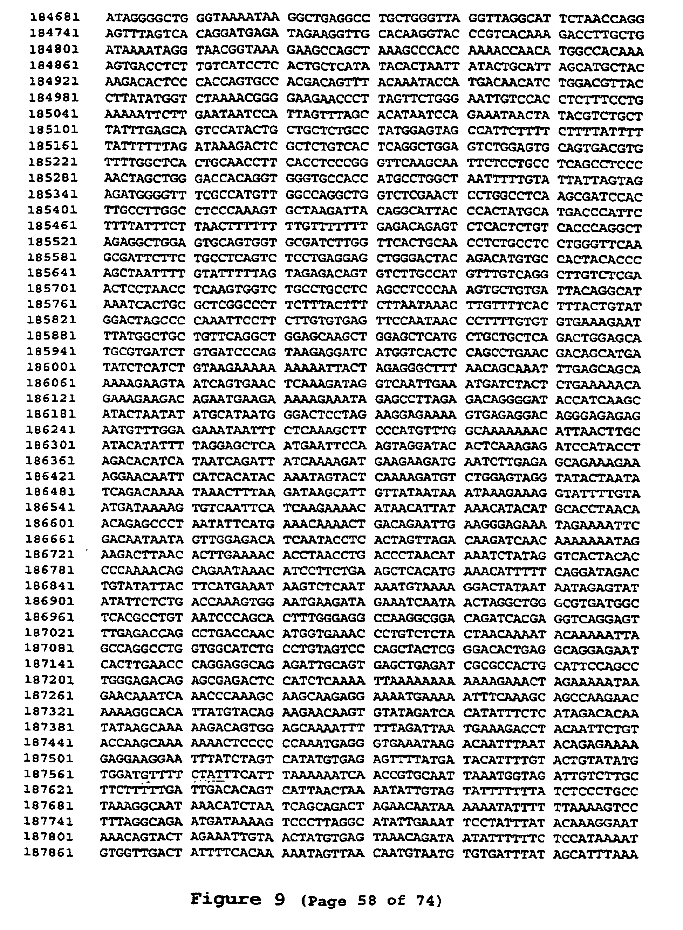

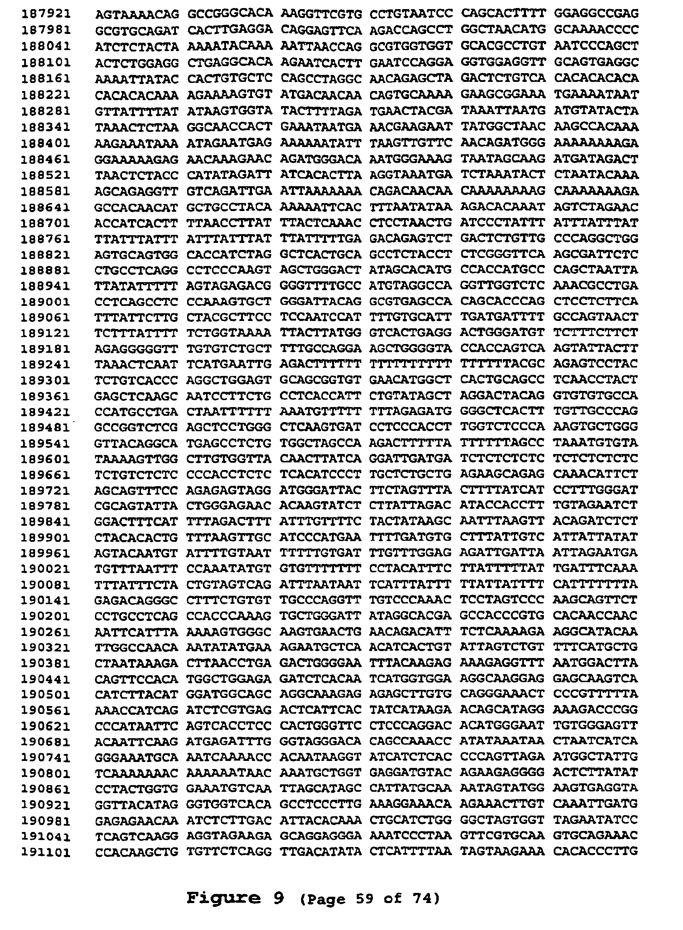

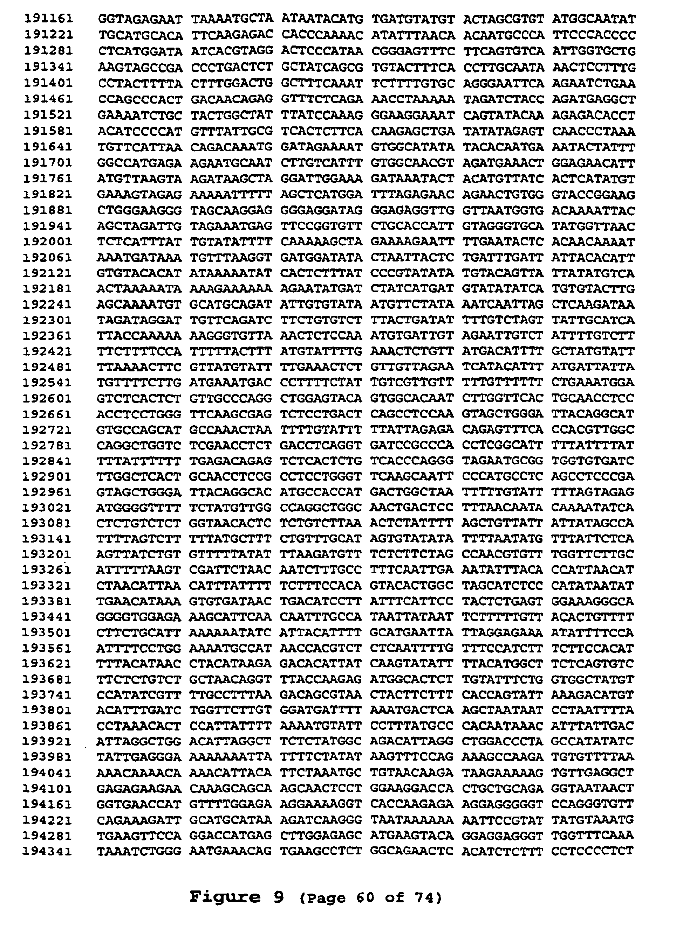

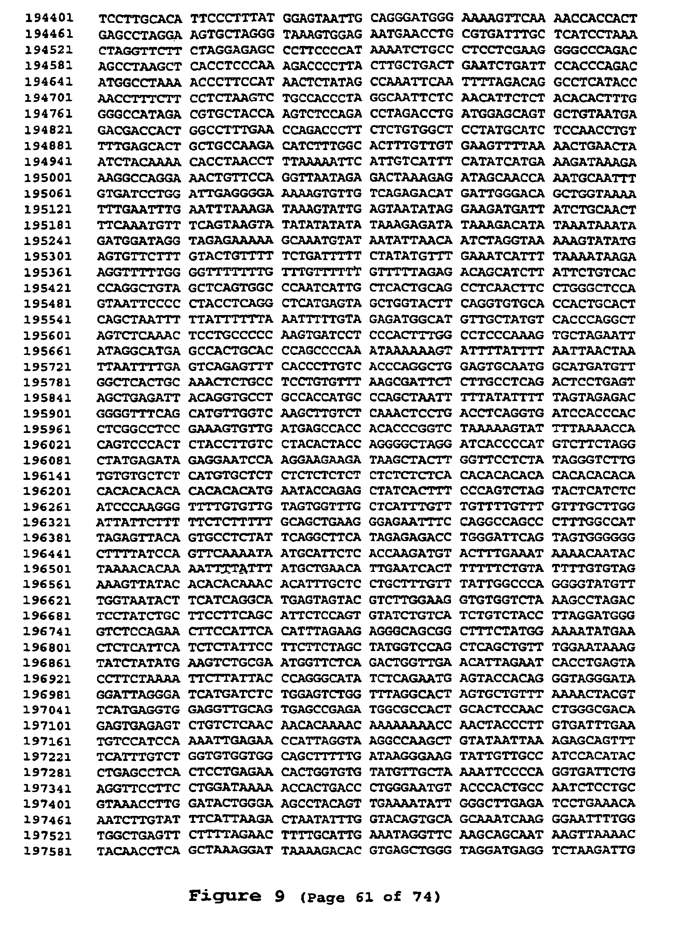

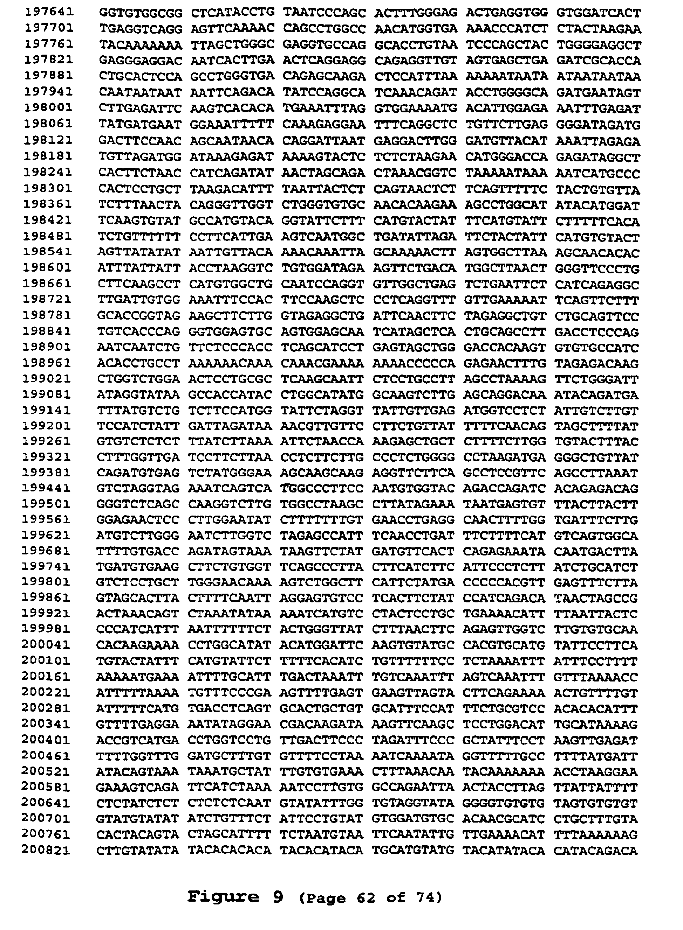

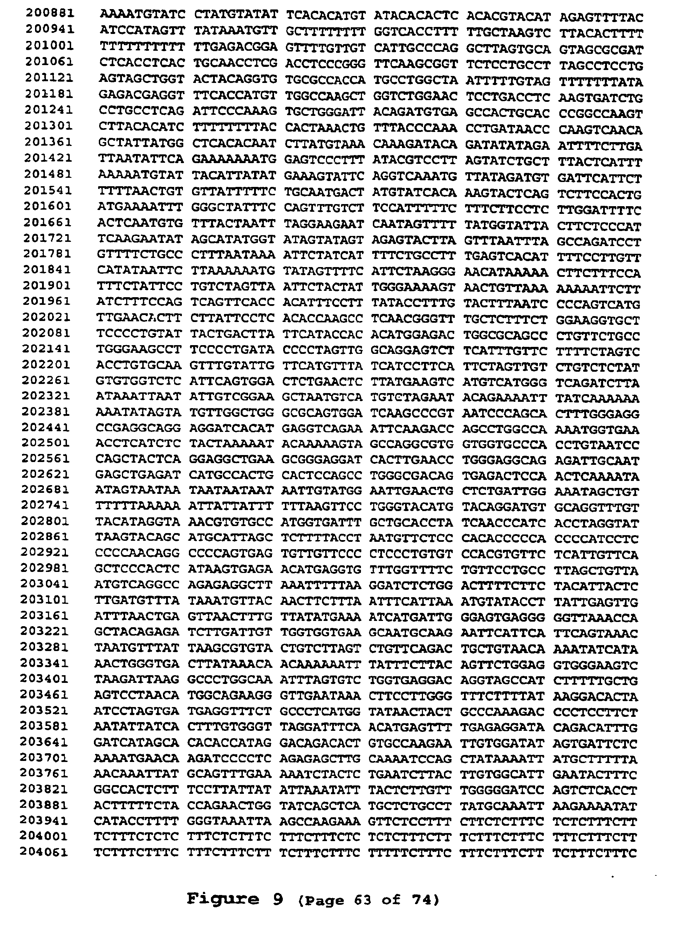

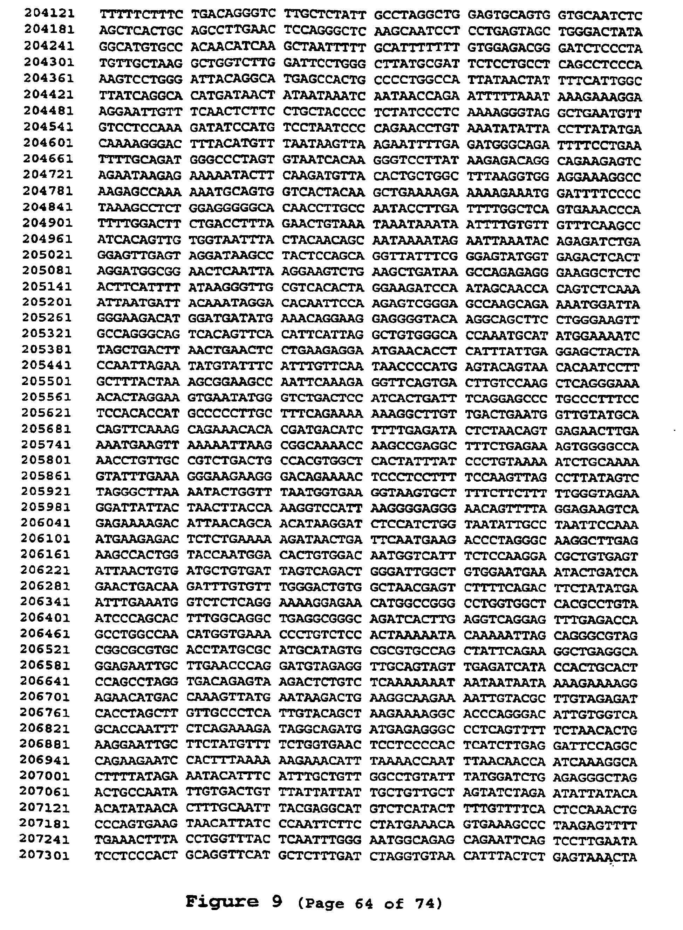

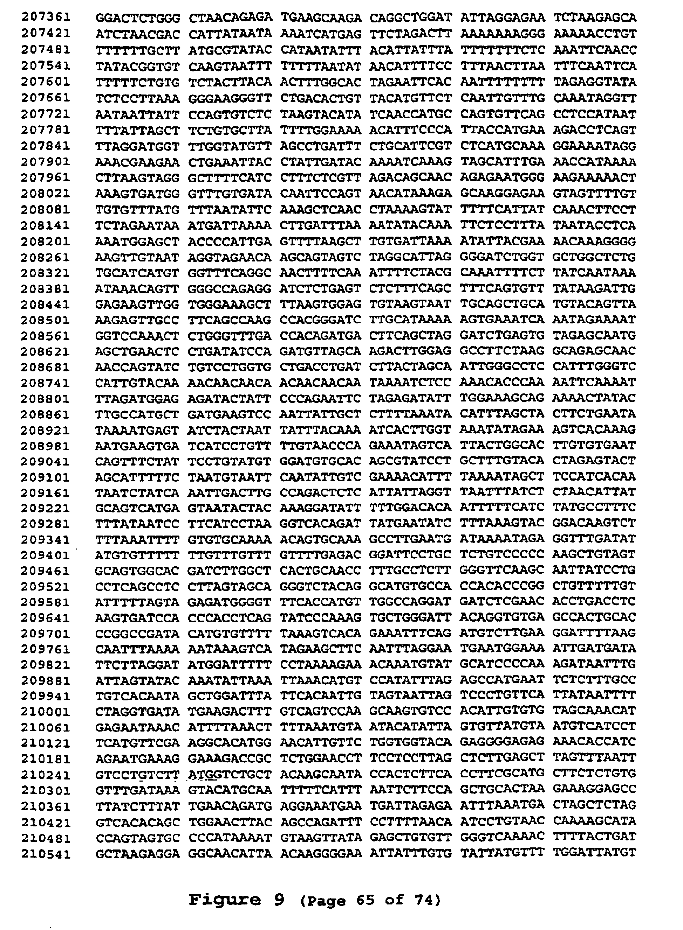

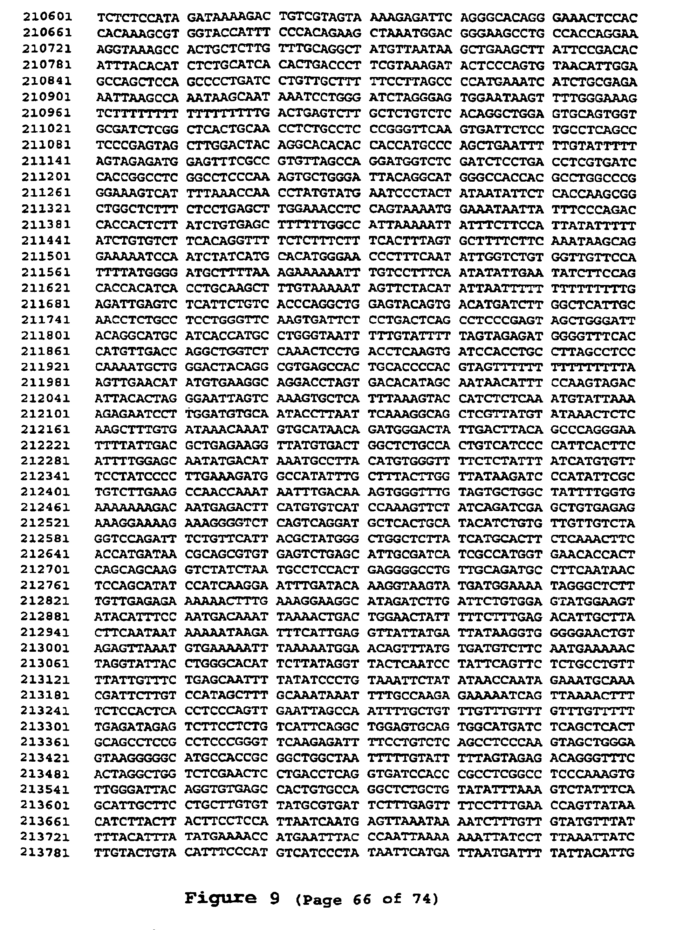

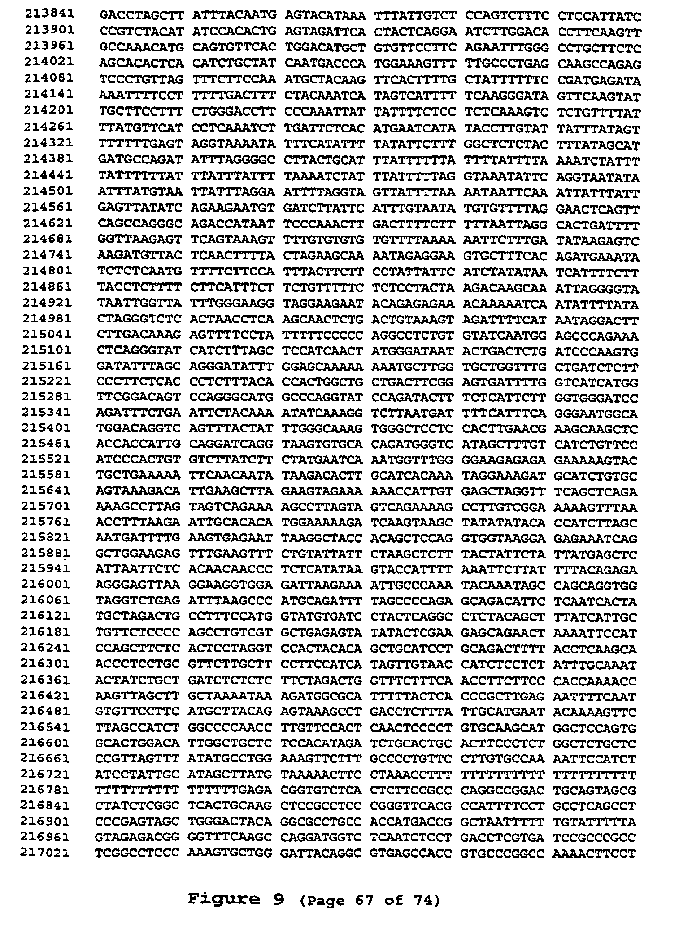

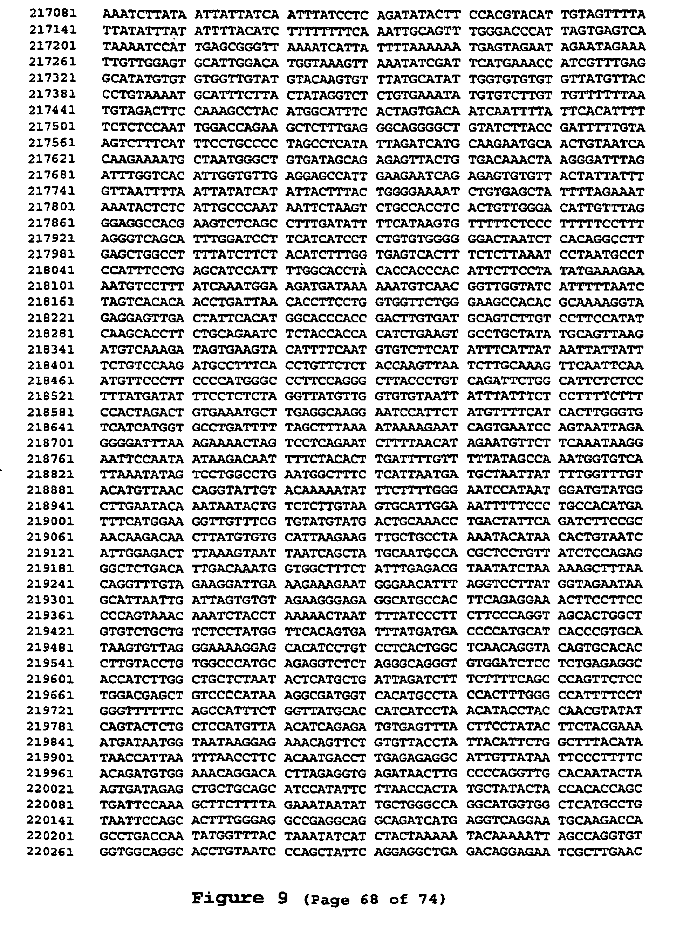

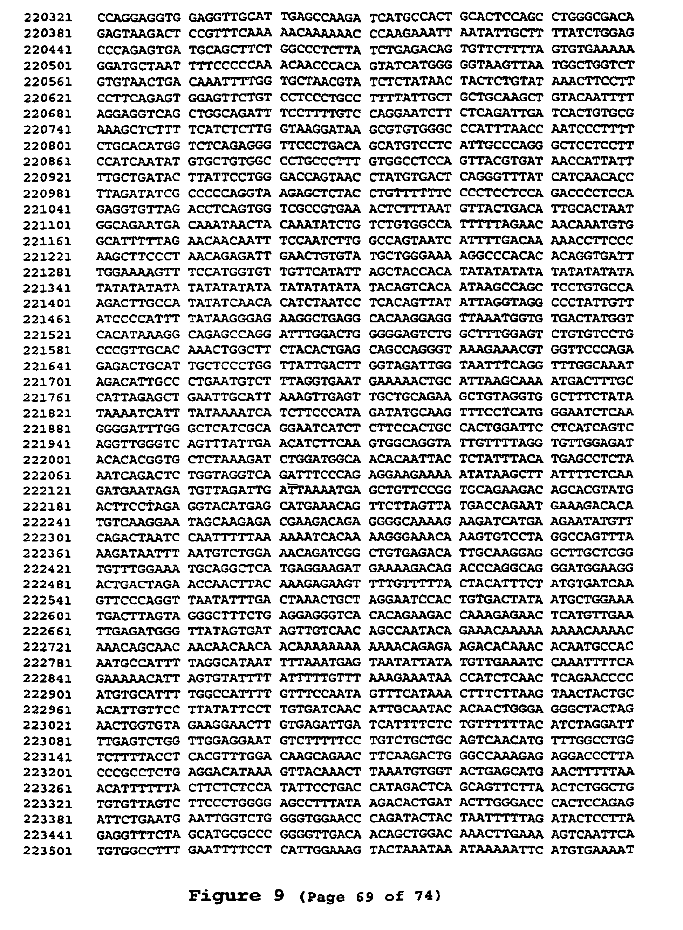

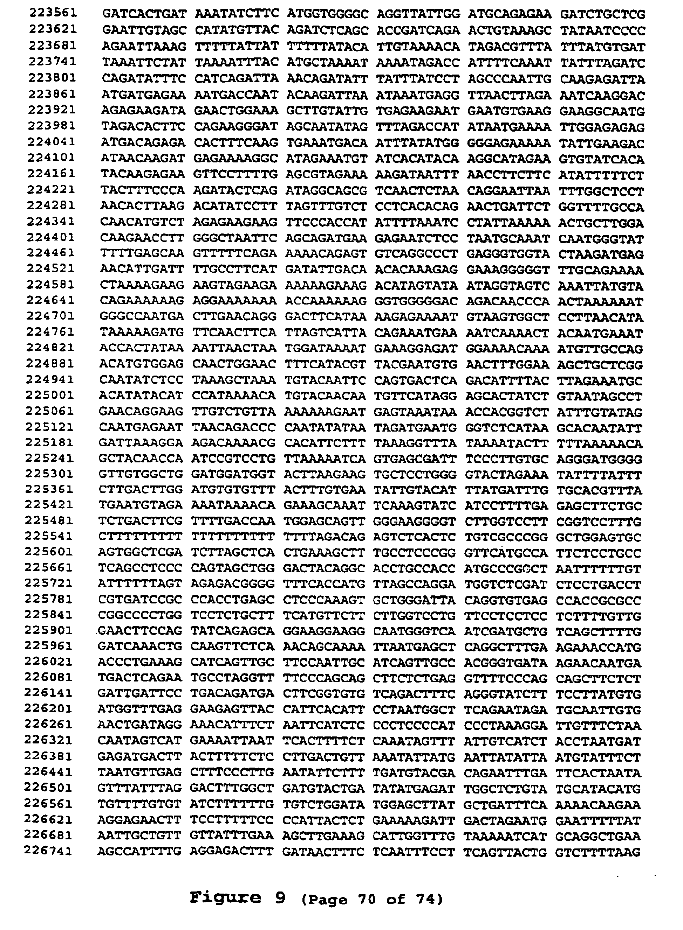

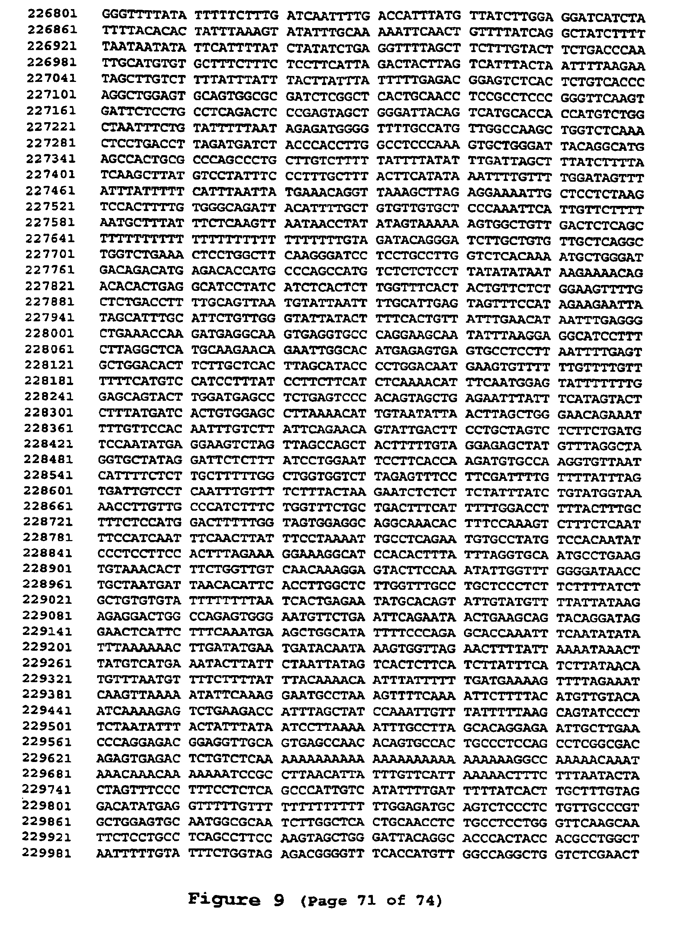

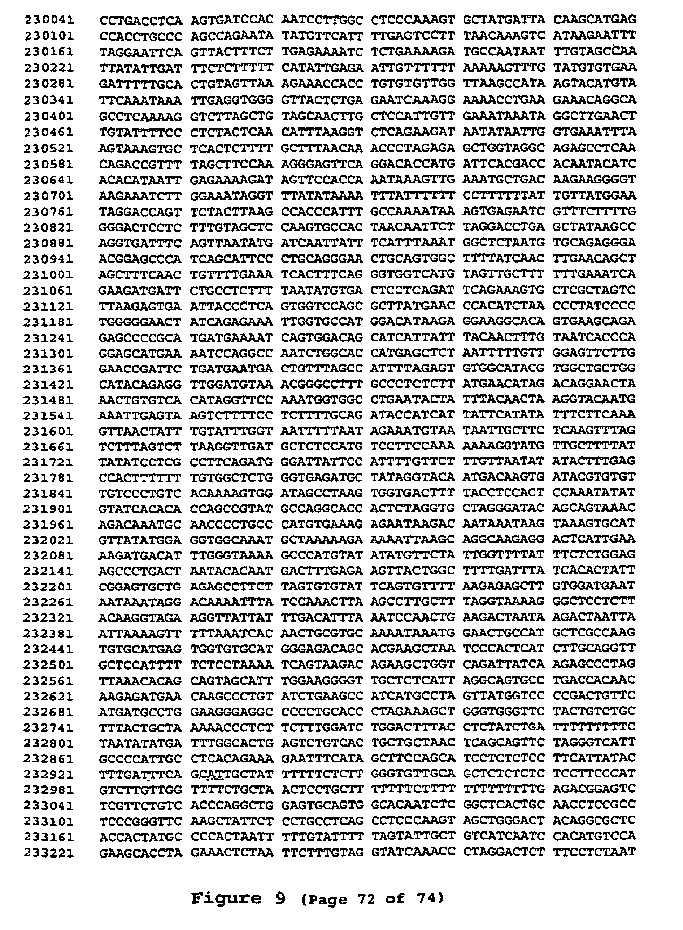

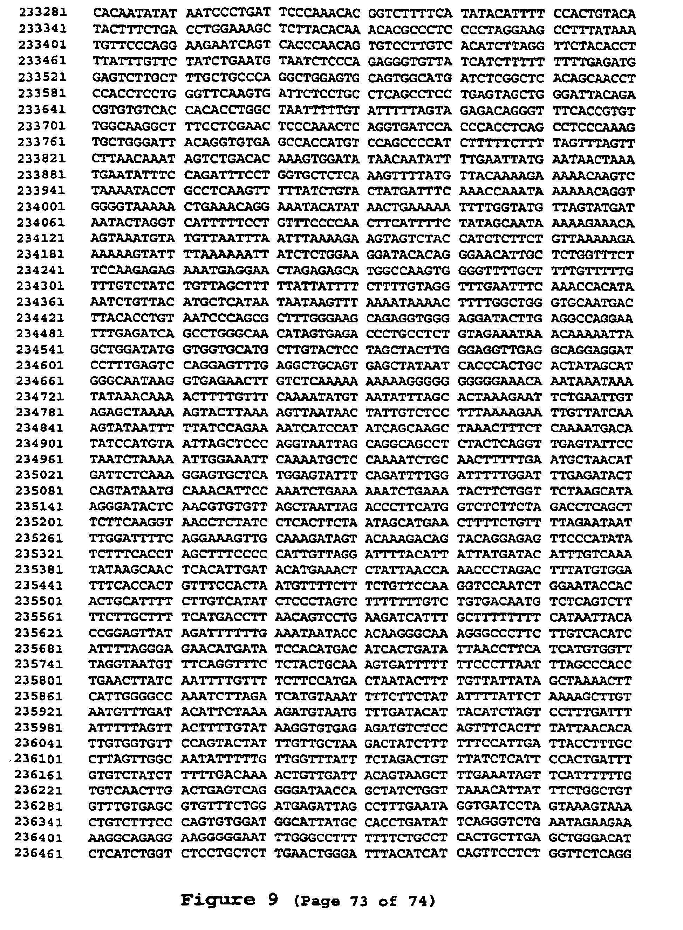

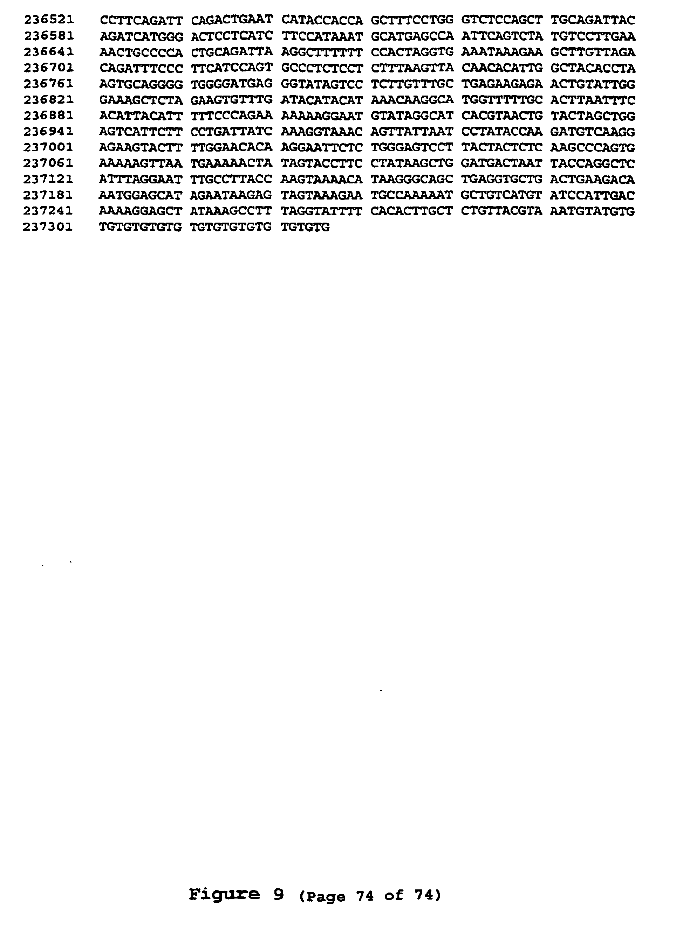

Figure 9 depicts the nucleotide sequence of approximately 235 kb in the HFE subregion from an HH affected individual. Polymorphic sites in the HH affected individual determined by comparing a sequence of the corresponding region from an HH unaffected individual are listed and described in Table I.

### DETAILED DESCRIPTION

### A. Definitions

Abbreviations for the twenty naturally occurring amino acids follow conventional usage. In the polypeptide notation used herein, the left-hand direction is the amino terminal direction and the right-hand direction is the carboxyl-terminal direction, in accordance with standard usage and convention. Similarly, unless specified otherwise, the left hand end of single-stranded polynucleotide sequences is the 5' end; the left hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

The term "nucleic acids", as used herein, refers to either DNA or RNA. "Nucleic acid sequence" or "polynucleotide sequence" refers to a single- or double-stranded polymer of deoxyribonucleotide or ribonucleotide bases read from the 5' to the 3' end. It includes both self-replicating plasmids, infectious polymers of DNA or RNA and nonfunctional DNA or RNA. The complement of any nucleic acid sequence of the invention is understood to be included in the definition of that sequence.

"Nucleic acid probes" may be DNA or RNA fragments. DNA fragments can be prepared, for example, by digesting plasmid DNA, or by use of PCR, or synthesized by either the phosphoramidite method described by Beaucage and Carruthers, Tetrahedron Lett. 22:1859-1862 (1981), or by the triester method according to Matteucci, et al., J. Am. Chem. Soc. 103:3185 (1981). A double stranded fragment may then be obtained, if desired, by annealing the chemically synthesized single strands together under appropriate conditions or by synthesizing the complementary strand using DNA polymerase with an appropriate primer sequence. Where a specific sequence for a nucleic acid probe is given, it is understood that the complementary strand is also identified and included. The complementary strand will work equally well in situations where the target is a double-stranded nucleic add.

The phrase "selectively hybridizing to" refers to a nucleic acid probe that hybridizes, duplexes or binds only to a particular target DNA or RNA sequence when the target sequences are present in a preparation of total cellular DNA or RNA. "Complementary" or "target" nucleic acid sequences refer to those nucleic acid sequences which selectively hybridize to a nucleic acid probe. Proper annealing conditions depend, for example, upon a probe's length, base composition, and the number of mismatches and their position on the probe, and must often be determined empirically. For discussions of nucleic acid probe design and annealing conditions, see, for example, Sambrook et al., Molecular Cloning: a Laboratory Manual (2nd ed.), Vols. 1-3. Cold Spring Harbor Laboratory, (1989) or Current Protocols in Molecular Biology, F. Ausubel et al., ed. Greene Publishing and Wiley-Interscience, New York (1987).

The phrase "nucleic acid sequence encoding" refers to a nucleic acid which directs the expression of a specific protein or peptide. The nucleic acid sequences include both the DNA strand sequence that is transcribed into RNA and the RNA sequence that is translated into protein. The nucleic acid sequences include both the full length nucleic acid sequences as well as non-full length sequences derived from the full length protein. It being further understood that the sequence includes the degenerate codons of the native sequence or sequences which may be introduced to provide codon preference in a specific host cell.

The phrase "isolated" or "substantially pure" refers to nucleic acid preparations that lack at least one protein or nucleic acid normally associated with the nucleic acid in a host cell.

The phrase "expression cassette", refers to nucleotide sequences which are capable of affecting expression of a structural gene in hosts compatible with such sequences. Such cassettes include at least promoters and optionally, transcription termination signals. Additional factors necessary or helpful in effecting expression may also be used as described herein.

The term "operably linked" as used herein refers to linkage of a promoter upstream from a DNA sequence such that the promoter mediates transcription of the DNA sequence.

The term "vector", refers to viral expression systems, autonomous self-replicating circular DNA (plasmids), and includes both expression and nonexpression plasmids. Where a recombinant microorganism or cell culture is described as hosting an "expression vector,'' this includes both extrachromosomal circular DNA and DNA that has been incorporated into the host chromosome(s). Where a vector is being maintained by a host cell, the vector may either be stably replicated by the cells during mitosis as an autonomous structure, or is incorporated within the host's genome.

The term "gene" as used herein is intended to refer to a nucleic acid sequence which encodes a polypeptide. This definition includes various sequence polymorphisms, mutations, and/or sequence variants wherein such alterations do not affect the function of the gene product. The term "gene" is intended to include not only coding sequences but also regulatory regions such as promoters, enhancers, and termination regions. The term further includes all introns and other DNA sequences spliced from the mRNA transcript, along with variants resulting from alternative splice sites.

The term "plasmid" refers to an autonomous circular DNA molecule capable of replication in a cell, and includes both the expression and nonexpression types. Where a recombinant microorganism or cell culture is described as hosting an "expression plasmid", this includes both extrachromosomal circular DNA molecules and DNA that has been incorporated into the host chromosome(s). Where a plasmid is being maintained by a host cell, the plasmid is either being stably replicated by the cells during mitosis as an autonomous structure or is incorporated within the host's genome.

The phrase "recombinant protein" or "recombinantly produced protein" refers to a peptide or protein produced using non-native cells that do not have an endogenous copy of DNA able to express the protein. The cells produce the protein because they have been genetically altered by the introduction of the appropriate nucleic acid sequence. The recombinant protein will not be found in association with proteins and other subcellular components normally associated with the cells producing the protein. The terms "protein" and "polypeptide" are used interchangeably herein.

The following terms are used to describe the sequence relationships between two or more nucleic acids or polynucleotides: "reference sequence" comparison window". "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, or may comprise a complete cDNA or gene sequence.

Optimal alignment of sequences for aligning a comparison window may, for example, be conducted by the local homology algorithm of Smith and Waterman Adv. Appl. Math. 2:482 (1981). by the homology alignment algorithm of Needleman and Wunsch J-Mol-Biol. 48:443 (1970), by the search for similarity method of Pearson and Lipman Proc, Natl. Acad. Sci. U.S.A. 85:2444 (1988), or by computerized implementations of these algorithms (for example, GAP, BESTFIT, FASTA, and TFASTA in the Wisconsin Genetics Software Package Release 7.0, Genetics Computer Group. 575 Science Dr., Madison, WI).

The terms "substantial identity" or "substantial sequence identity" as applied to nucleic acid sequences and as used herein and denote a characteristic of a polynucleotide sequence, wherein the polynucleotide comprises a sequence that has at least 85 percent sequence identity, preferably at least 90 to 95 percent sequence identity, and more preferably at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

As applied to polypeptides, the terms "substantial identity" or "substantial sequence identity" mean that two peptide sequences, when optimally aligned, such as by the programs GAP or BESTFIT using default gap weights, share at least 80 percent sequence identity, preferably at least 90 percent sequence identity, more preferably at least 95 percent sequence identity or more. "Percentage amino acid identity" or "percentage amino acid sequence identity" refers to a comparison of the amino acids of two polypeptides which, when optimally aligned, have approximately the designated percentage of the same amino acids. For example, "95% amino add identity" refers to a comparison of the amino acids of two polypeptides which when optimally aligned have 95% amino acid identity. Preferably, residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

The phrase "substantially purified" or "isolated" when referring to a peptide or protein, means a chemical composition which is essentially free of other cellular components. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. Generally, a substantially purified or isolated protein will comprise more than 80% of all macromolecular species present in the preparation. Preferably, the protein is purified to represent greater than 90% of all macromolecular species present. More preferably the protein is purified to greater than 95%, and most preferably the protein is purified to essential homogeneity, wherein other macromolecular species are not detected by conventional techniques.

The phrase "specifically binds to an antibody" or "specifically immunoreactive with*, when referring to a protein or peptide, refers to a binding reaction which is determinative of the presence of the protein in the presence of a heterogeneous population of proteins and other biologies. Thus, under designated immunoassay conditions, the specified antibodies bind to a particular protein and do not bind in a significant amount to other proteins present in the sample. Specific binding to an antibody under such conditions may require an antibody that is selected for its specificity for a particular protein. A variety of immunoassay formats may be used to select antibodies specifically immunoreactive with a particular protein. For example, solid-phase EUSA immunoassays are routinely used to select monoclonal antibodies specifically immunoreactive with a protein. See Harlow and Lane (1988) Antibodies, a Laboratory Manual, Cold Spring Harbor Publications, New York, for a description of immunoassay formats and conditions that can be used to determine specific immunoreactivity.

As used herein, "EST" or "Expressed Sequence Tag "refers to a partial DNA or cDNA sequence of about 150 to 500, more preferably about 300, sequential nucleotides of a longer sequence obtained from a genomic or cDNA library prepared from a selected cell, cell type, tissue or tissue type, or organisms which longer sequence corresponds to an mRNA or a gene found in that library. An EST is generally DNA. One or more libraries made from a single tissue type typically provide at least 3000 different (i.e. unique) ESTs and potentially the full complement of all possible ESTs representing all possible cDNAs, e.g., 50,000 -100,000 in an animal such as a human. (See, for example, Adams et al. Science 252:1651-1656 (1991)).

"Stringent" as used herein refers to hybridization and wash conditions of 50% formamide at 42°C. Other stringent hybridization conditions may also be selected. Generally, stringent conditions are selected to be about 5° C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 60°C. As other factors may significantly affect the stringency of hybridization, including, among others, base composition and size of the complementary strands, the presence of organic solvents and the extent of base mismatching, the combination of parameters is more important than the absolute measure of any one.

### B. Transcript Map and New Genes near HH

The instant invention provides a fine structure map of the 1 megabase region surrounding the HFE gene. As part of that map the instant invention provides approximately 250 kb of DNA sequence of which about 235 kb are provided in Figure 8 and eight too of particular interest corresponding to candidate genes within the 1 megabase region. These loci are useful as genetic and physical markers for further mapping studies- Additionally, the eight cDNA sequences corresponding to those loci are useful, for example, for the Isolation of other genes in putative gene families, the identification of homologs tram other spades, and as probes for diagnostic assays. In particular, isolated nucleic acid sequences of at least 18 nucleotides substantiate identical to contiguous nucleotides of a cDNA of the invention are useful as PCR primers. Typically, the PCR primer will be used as part of a pair of primers in a PCR reaction. Isolated nucleic acid sequences preferably comprising about 18-100 nucleotides, more preferably at least 18 nucleotides, substantially identical to contiguous nucleotides in a cDNA of the invention are useful in the design of PCR primers and probes for hybridization assays. Additionally, the proteins encoded by those cDNAs are useful in the generation of antibodies for analysis of gene expression and in diagnostic assays, and in the purification of related proteins.

A235 kb sequence is provided for the HFE subregion within the 1 megabase region mapped. This sequence can serve as a reference in genetic or physical analysis of deletions, substitutions, and insertions in that region. Additionally, the sequence information provides a resource for the further identification of new genes in that region.

### C. Polymorphic Markers

397 polymorphic sites have been identified in the region of the HFE gene. These polymorphisms are listed in Table 1. As described below, these polymorphisms were identified by comparison of the DNA sequence of an affected individual homozygous for the common ancestral HH mutation with that of an unaffected individual disclosed in copending U.S. 08/724,394.

**Table 1. Polymorphic Sites in the HH Region**

| **Base Location** | **Difference** | **Base Location** | **Difference** |
|---|---|---|---|
| 35-36 | AC DEL | 19755 | G-A |
| 841 | T-C | 19949 | C-T |
| 2662-2683 | TT DEL | 20085 | C-T |
| 3767 | T-C | 20366-20367 | A INS |
| 3829 | C-G | 20463 | C-A |
| 4925-4928 | TAAA DEL | 20841 | A-T |
| 5681 | C-T | 21059 | A-T |
| 5839 | T-C | 21117 | A-G |
| 6011 | G-A | 21837 | A-C |
| 6047 | C-G | 22283 | A-C |
| 6231 | G-A | 22786 | C-A |
| 6643 | ADEL | 23009 | G-A |
| 6698 | T-C | 24143 | T-A |
| 7186 | T-C | 26175 | G-C |
| 7273 | G-A | 26667 | C-A |
| 7545-7558 | TCACACACCGATTGG DEL | 26994 | T-C |
| 7672 | G DEL | 27838 | G-T |
| 7833 | T-C | 27881 | T DEL |
| - 8748 | T-G | 28132 | G-A |
| 9115 | G-A | 28100 | G-A |
| am | G-A | 29454-29457 | TTTT DEL |
| 10027 | G-A | 29787 | T-G |
| 10214 | C-T | 29625 | A-C |
| 10828 | A-G | 30008 | T-C |
| 10918 | C-G | 30177 | A-G |
| 10955 | A-G | 30400 | A-G |
| 11524 | C-A | 31059 | T-A |
| 11974 | A-G | 31280 | C-T |
| 11955 | T-C | 31749 | C-T |
| 12173-12175 | TTT DEL | 32040 | C-G |
| 13304 | G-A | 32558-32559 | TGTG DEL |
| 13455 | G-A | 33017 | T-G |
| 14416-14417 | A INS | 33026 | T DEL |
| 14998 | C-T | 34434 | C- T |
| 15564 | T-C | 35179 | A-C |
| 15887 | A-G | 35695 | G-A |
| 15804-15919 | CCAAACTGATCTTTGA DEL | 35702 | G-A |
| 16019 | T DEL | 35983 | A-G |
| 16211 | A-T | 37411 | A-G |
| 17461 | A-G | 38528 | C-T |
| 40431 | C-A | 72688 | C-G |
| 42054-42055 | TT DEL | 75323-75324 | T INS |
| 43783-43784 | TTTT INS | 75887 | G-C |
| 45120 | C DEL | 77519 | T-C |
| 45567 | A-C | 77749 | G-A |
| 46601 | A-T | 77908 | T-C |
| 47255 | C-G | 78385 | C-G |
| 47758 | C-A | 78592-78593 | AG INS |
| 47994 | G-C | 80189 | T-G |
| 48440 | G-A | 80279 | T DEL |
| 48650 | T-G | 80989-80990 | A INS |
| 48680 | A-G | 81193 | T-C |
| 50240 | C-T | 81273 | A DEL |
| 50553 | G-A | 82166 | G-A |
| 50586 | G-T | 83847 | T DEL |
| 51322 | G-C | 84161-84162 | CA-GG |
| 51747 | A-G | 84533 | A-G |
| 52474 | C-G | 84638 | T-G |
| 52733 | C-A | 85526 | T-G |
| 52875 | G-A | 85705 | G-T |
| 53631-53637 | TTTTTTT DEL | 86984 | T-C |
| 53707 | G-A | 87655 | T-C |
| 54819 | A-G | 87713 | A-C |
| 55913 | T-C | 87892 | C-T |
| 56225 | A-C | 88192 | T DEL |
| 56510 | T-C | 88528 | A-G |
| 56566 | G-A | 89645 | A-T |
| 56618 | A-T | 89728 | A-G |
| 57815 | A-G | 90088 | T-C |
| 58011 | T DEL | 91193-91194 | 2209bp INS |
| 58247-58248 | T INS | 91373 | T-C |
| 58926 | C-G | 91433-91434 | A INS |
| 59406 | C-G | 91747 | G-A |
| 59422 | G-C | 93625 | T DEL |
| 60221-60222 | A INS | 95116-95117 | T INS |
| 60656-60657 | CA DEL | 96315 | G-A |
| 61162 | G-A | 97981 | A-G |
| 61465 | G-A | 98351 | T DEL |
| 61607 | A DEL | 99249 | C-T |
| 61653 | T-C | 100094-100095 | TINS |
| 61794-61795 | T INS | 100647-100648 | TTC INS |
| 62061 | G-C | 100951 | C-T |
| 62362 | T-G | 101610 | C-G |
| 62732 | C-G | 102589 | C-T |
| 63364 | G-A | 103076-103077 | TATATATATATATA INS |
| 63430-63431 | GT INS | 103747 | T-C |
| 63754 | C-T | 105638 | A-C |
| 63785 | A-C | 107024 | C-T |
| 63870-63871 | A INS | 107322 | C-T |
| 64788 | A-G | 107858 | C-G |
| 64962 | G-A | 109019 | A DEL |
| 65891 | C-T | 109579 | T DEL |
| 66675 | G-C | 110021 | C-A |
| 67186-67187 | ATT INS | 111251 | C-A |
| 67746-67747 | TT INS | 111425 | G-A |
| 68259 | T-C | 112644 | T-A |
| 68836 | T-C | 113001 | G-C |
| 68976 | C-G | 113130 | C-T |
| 72508 | T-G | 114026 | G-A |
| 114250 | A DEL | 176222 | T-C |
| 115217 | C-G | 176524 | A-T |
| 117995 | G-A | 176684 | G-A |
| 118874 | A-G | 176815 | T-C |
| 119470 | T-C | 177049 | T-C |
| 119646 | G-T | 177065 | G-T |
| 120853 | C-T | 178285 | T-C |
| 121582 | G-A | 178551-178552 | CTTTTTTTTTTTTTT INS |
| 123576 | A-C | 179114-179115 | A INS |
| 125581 | C-T | 179260 | C-G |
| 125970 | G-T | 179281 | C-G |
| 126197 | A-G | 180023 | G-C |
| 126672 | A DEL | 180430 | T-C |
| 126672 | G-C | 180773 | T-C |
| 128220-128221 | A INS | 180824 | T-C |
| 132569 | C-T | 181097 | C-T |
| 133572 | A-C | 181183 | A-T |
| 134064 | T-G | 182351 | C-T |
| 136999 | G-A | 183197 | G-A |
| 137784 | C-T | 183623 | A-T |
| 138903 | G-A | 183653 | G-T |
| 139159-139160 | AINS | 183657 | T-G |
| 140359 | G-A | 183795-183796 | A INS |
| 140898 | C-T | 184060 | G-A |
| 141313 | C DEL | 184993 | G-A |
| 141343 | T-C | 185918 | A-G |
| 142148 | T-C | 186036 | T-C |
| 142178 | C-A | 186506-186507 | TAAC INS |
| 142433-142434 | ATAGA INS | 186561-186568 | TATTTATT DEL |
| 143783 | C-T | 186690 | G DEL |
| 144090 | C-T | 186751 | T-A |
| 144220-144221 | A INS | 187221 | A-G |
| 144725 | A-C | 187260 | A-G |
| 145732-145733 | AAAAAAAAAAAAAA INS | 187444-187447 | CTCT DEL |
| 147016-147017 | CG DEL | 187831-187832 | C INS |
| 147021 | G-T | 188638 | G-A |
| 147536 | T-G | 188642 | C-T |
| 148936 | T-A | 189246 | T-C |
| 149061 | T-C | 190340 | A-C |
| 154341 | A-T | 190354 | A-G |
| 154588 | G-A | 190762 | A-G |
| 155464 | G-A | 191260 | G-T |
| 158574 | C-G | 193018-193019 | AGAT INS |
| 160007 | C-T | 193147 | T-G |
| 164348 | A-T | 193196-193197 | C INS |
| 164499 | C-G | 193499 | C-T |
| 166677-166678 | AAAG INS | 193738 | C-G |
| 167389 | G-A | 193984-193985 | ACACACAC INS |
| 168506-168507 | AGGATGGTCT INS | 194064 | C-G |
| 168515 | T-C | 194504 | A DEL |
| 169413-169414 | AA INS | 194734 | G-A |
| 170300-170301 | TTGTTGTTGTTG INS | 194890 | A-C |
| 170491 | G-A | 195404 | G-A |
| 173428 | T-C | 195693 | A-T |
| 173642 | G-A | 196205 | G-A |
| 173948 | T-G | 197424 | C-T |
| 175330 | T-C | 197513 | C-T |
| 175836 | T-C | 197670 | G-A |
| 176200 | G-C | 198055 | C-A |
| 198401 | C-T | 215947 | C-A |
| 198692 | A-G | 216232 | A-G |
| 198780 | T DEL | 217478 | G-A |
| 199030 | T-G | 219052 | T-C |
| 199933 | C-T | 219082-219083 | ATATATATATATATATATAT INS |
| 200027 | G-A | 219314 | C-A |
| 200439 | T-A | 219327 | G-A |
| 200452 | A-G | 219560 | C-T |
| 200472-200483 | AATAATAATAAT DEL | 219660 | C-T |
| 200559 | A-T | 219889 | G-A |
| 200745 | A-G | 220198 | G-T |
| 200919 | T-A | 220384 | G-A |
| 201816 | C-T | 220451-220452 | CAAAAA INS |
| 201861-201862 | 42bp INS | 221363 | G-A |
| 202662 | T-C | 221645 | G-A |
| 202880 | T-C | 222119 | T-C |
| 204341 | C-T | 222358 | A-G |
| 204768 | A-T | 222367 | A-C |
| 205284 | T-G | 222686 | A-G |
| 207400 | C-A | 222959 | T-C |
| 208634 | T-C | 223270-223271 | TT DEL |
| 208718 | T DEL | 223283 | T-C |
| 208862 | A-C | 224964 | T-C |
| 209419-209420 | TT DEL | 225232 | A-C |
| 209802 | G-A | 225366-225367 | TTTT INS |
| 209944 | C-G | 225416 | G-C |
| 210299 | A-G | 225486 | T-C |
| 211142 | G-A | 226088 | A-G |
| 212072 | G-A | 228421 | A-G |
| 212146 | T-C | 230047 | G-A |
| 212379 | G-A | 230109 | G-C |
| 212637-212639 | TCT DEL | 230376 | C-G |
| 212696 | T-C | 230394 | A-G |
| 213042 | T-A | 231226 | A-G |
| 214192 | A-G | 231447 | G-A |
| 214529-214530 | TTTTTTTTTTT INS | 231835 | A-G |
| 214549 | T-C | 232400-232402 | AAA DEL |
| 214795 | C-T | 232402-232403 | GINS |
| 214908 | T-G | 232515 | T-C |
| 214977 | A-G | 232703 | G-T |
| 215769 | C-T | 232750 | A-G |

| | | | |
|---|---|---|---|
| * D6S2238 occurs at base 1. 24dl occurs at base 41316. D6S2239 occurs at base: 84841. D6S2241 occurs at base 235032 | | | |

**Table 2. Polymorphic Allele Frequencies**

| **Location** | **Frequency of ancestral variant in random chromosomes** | **Frequency of unaffected variant in random chromosomes** |
|---|---|---|
| 232703 | 53% | 47% |
| 231835 | 53% | 47% |
| 230394 | 85% | 15% |
| 230376 | 25% | 75% |
| 230109 | 53% | 47% |
| 225486 | 45% | 55% |
| 225416 | 75% | 25% |
| 220198 | 43% | 57% |
| 219660 | 58% | 42% |
| 219560 | 53% | 47% |
| 214977 | 65% | 35% |
| 214908 | 50% | 50% |
| 214795 | 24% | 76% |
| 214549 | 53% | 47% |
| 214192 | 65% | 35% |
| 210299 | 53% | 47% |
| 208862 | 80% | 20% |
| 208634 | 48% | 52% |
| 207400 | 25% | 75% |
| 205284 | 50% | 50% |
| 204341 | 53% | 47% |
| 202880 | 58% | 42% |
| 202662 | 98% | 2% |
| 200027 | 25% | 75% |
| 199030 | - 58% | 42% |
| 198692 | 55% | 45% |
| 198401 | 55% | 45% |
| 198055 | 55% | 45% |
| 195693 | 60% | 40% |
| 195404 | 25% | 75% |
| 194890 | 55% | 45% |
| 175330 | 53% | 47% |
| 173948 | 83% | 17% |
| 173642 | 55% | 45% |
| 173428 | 80% | 20% |
| 168515 | 80% | 20% |
| 160007 | 18% | 82% |
| 149061 | 58% | 42% |
| 148936 | 82% | 18% |
| 147536 | 100% | 0% |
| 147021 | 46% | 54% |
| 141343 | 55% | 45% |
| 140359 | 55% | 45% |
| 138903 | 55% | 45% |
| 132569 | 81% | 19% |
| 125581 | 18% | 82% |
| 121582 | 80% | 20% |
| 120853 | 18% | 82% |
| 118874 | 85% | 15% |
| 115217 | 50% | 50% |
| 113130 | 40% | 60% |
| 113001 | 48% | 52% |
| 107858 | 48% | 52% |
| 103747 | 50% | 50% |
| 96315 | 25% | 75% |
| 91194 | 80% | 20% |
| 90088 | 75% | 25% |
| 89728 | 50% | 50% |
| 89645 | 50% | 50% |
| 88528 | 63% | 37% |
| 87892 | 75% | 25% |
| 87713 | 60% | 40% |
| 87655 | 50% | 50% |
| 86984 | 79% | 21 % |
| 85705 | 50% | 50% |
| 85526 | 50% | 50% |
| 84638 | 50% | 50% |
| 84533 | 50% | 50% |
| 82168 | 78% | 22% |
| 81193 | 58% | 42% |
| 80189 | 50% | 50% |
| 78385 | 80% | 20% |
| 77908 | 88% | 12% |
| 68976 | 50% | 50% |
| 68259 | 51% | 49% |
| 68675 | 80% | 20% |
| 62732 | 50% | 50% |
| 62382 | 40% | 60% |
| 61653 | 48% | 52% |
| 61465 | 5% | 95% |
| 61162 | 60% | 40% |
| 53707 | 100% | 0% |
| 52875 | 50% | 50% |
| 52733 | 74% | 26% |
| S2474 | 47% | 53% |
| 50588 | 50% | 50% |
| 50553 | 50% | 50% |
| 50240 | 50% | 50% |
| 48680 | 53% | 47% |
| 48850 | 63% | 37% |
| 48440 | 50% | 50% |
| 47255 | 50% | 50% |
| 46601 | 53% | 47% |
| 45567 | 49% | 51% |
| 41316 | 5% | 85% |
| 40431 | 20% | 80% |
| 38526 | 23% | 77% |
| 37411 | 70% | 30% |
| 35983 | 5% | 95% |

By way of background. These polymorphisms provide surrogate markers for use in diagnostic assays to detect the likely presence of the mutations 24d1 and/or 24d2. in preferably 24d1, in homozygotes or heterozygotes. Thus, for example, DNA or RNA from an individual is assessed for the presence of absence of a genotype defined by a polymorphic allele of Table 1. wherein, as a result, the absence of a genotype defined by a polymorphic allele of Table 1 indicates the likely absence of the HFE gene mutation in the genome of the individual and the presence of the genotype indicates the likely presence of the HFE gene mutation in the genome of the individual.

These markers may be used singly, in combination with each other, or with other polymorphic markers (such as those disclosed in co-pending PCT application WO 96/06583) in diagnostic assays for the likely presence of the HFE gene mutation is an individual. For example, any of the markers defined by the polymorphic sites of Table 1 can be used in diagnostic assays in combination with 24d1 or 24d2, or at least one of polymorphisms HHP-1, HHP-19, or HHP-29, or microsatellite repeat alleles 19D9:205:1884:235:1A2:239;1E4:271;24E2:245;28:206;3321-1: 98; 4073-1.162;4440-1:180.4440-2:139, 731-1:171:5091-1:148; 3218-1221;4072-2:170; 950.1:142; 950.2:184: 950-3:185:950-4:128:950-6:151,850-8:137;63-1:151;63-2:113;63-3:168;65-1205; 65-2:159; 68-1:167;241-5:108;241-29:113;373-8:151: and 373-29:113,D6S258:199.D6S265:122, D6S105:124: DSS306:238:D6S464:206;and D6S1001:180.

Table 2 lists the frequency of about 100 of the alleles defined by the polymorphic sites identified. As is evident from the Table, certain of these alleles are present rarely in the general population. of the genotypes defined by the alleles listed in Table 2, polymorphisms occurring at base 35983 are the subject of the present invention.

It will be understood by those of skill in the art that because they were identified in an ancestral HH homozygote, the haplotypes defined by the polymorphic sites of Table 1 are predictive of the likely presence of the HFE gene mutation 24d1. Thus, for example, the likelihood of any affected individual having at least two or more of any of the polymorphic alleles defined by Table 1 is greater than that for any unaffected individual. Similarly, the likelihood of any affected individual having at least three or more of any of the polymorphic alleles defined by Table 1 is greater than that for any unaffected individual,

Thus, for example in a diagnostic assay for the likely presence of the HFE gene mutation in the genome of the individual, DNA or RNA from the individual is assessed for the presence or absence of a haplotype of Table 1, wherein, as a result, the absence of a haplotype of Table 1 indicates the likely absence of the HFE gene mutation in the genome of the individual and the presence of the haplotype indicates the likely presence of the HFE gene mutation in the genome of the individual.

The markers defined by the polymorphic sites of Table 1 are additionally useful as markers for genetic analysis of the inheritance of certain HFE alleles and other genes which occur within the chromosomal region corresponding to the sequence of Figure 9 which include, for example, those disclosed in copending U.S.S.N. 08/724,394.

As the entire nucleotide sequence of the region is provided in Figure 9, it will be evident to those of ordinary skill in the art which sequences to use as primers or probes for detecting each polymorphism of interest. Thus, in some embodiments of the invention, the nucleotide sequences of the invention include at least one oligonucleotide pair selected from the sequence of Figure 9 or its complement for amplification of the polymorphic site at base 35983 of Table 1. Furthermore, in some embodiments of the invention a preferred hybridization probe is an oligonucleotide comprising at least 8 to about 100 consecutive bases from the sequence of Figure 9, or the complement of the sequence, wherein the at least 8 to about 100 consecutive bases includes at least the polymorphic site 35983 of Table 1. Such isolated DNA sequences are useful as primers, probes, or as the component of a kit in diagnostic assays for detecting the likely presence of the HFE gene mutation in an individual.

### D. Nucleic Acid Based Screening

Individuals carrying polymorphic alleles of the invention may be detected at either the DNA, the RNA, or the protein level using a variety of technique that are well known. In the art. The genomic DNA used for the diagnosis may be obtained from body cells, such as those present in peripheral blood, urine, saliva, bucca, surgical specimen, and autopsy specimens. The DNA may be used directly or may be amplified enzymatically *in vitro* through use of PCR (Saiki at al. Science. 239:467-481 (1988)) or other *in vitro* amplification methods such as the ligase chain reaction (LCR) (Wu and Wallace Genomics 4:560-569 (1899)), strand displacement amplification (SDA) (Walker et al. Proc, Natl. Acad. Sci, U.S.A, 89:392-398 (1992)), self-sustained sequence replication (3SR) (Fahy et al. PCR Methods Appl. 1:25-33 (1992)), prior to mutation analysis. The methodology for preparing nucleic acids in a form that is suitable for mutation detection is well known in the art.

The detection of polymorphisms in specific DNA sequences, such as in the region of the HFE gene, can be accomplished by a variety of methods including, but not limited to, restriction-fragment-length-polymorphism detection based on allele-specific restriction-endonuclease cleavage (Kan and Dozy Lancet :910-912 (1978)), hybridization with allele-specific oligonucleotide probes (Wallace et al. Nucl Acids Res 8:3543-3557 (1978)), including immobilized oligonucleotides (Saiki et al. Proc. Natl. Acad- Sci. U.SA. 86:6230-6234 (1989)) or oligonucleotides arrays (Maskos and Southern Nucl Acids Res 21:2289-2270 (1993)), allele specific PCR (Newton et al. Nucl Acids Res 17:2503-2516 (1989)). mismatch-repair detection (MRD) (Faham and Cox Genome Res 5:474-482(1985)), binding of MutS protein (Wagner et al. Nucl Acids Res 23:3944-3948 (1995), densturing-gradient get electrophoresis (DGGE) (Fisher and Lerman et al. Proc. Natl. Acad. Sci. U S.A. 80:1579-1583 (1983)), single-strand-conformation-polymorphism detection (Orita et al. Genomics 5:874-879 (1983)), RNAase cleavage at mismatched base-pairs (Myers et al. Science 230:1242 (1985)), chemical (Cotton et al. Proc. Natl. Acad. Sci. U.S.A 85:4397-4401 (1988) or enzymatic (You et al. Proc, Natl, Acad, Sci. U.S.A 92:87.81 (1995)) cleavage of heteroduplex DNA methods based on allele specific primer extension (Syvänen et al. Genomics 8:684-692 (1990)), genetic bit analysis (GBA) (Nikiforov et al. Nucl Acids Res 22:4167-4175 (1994)), the oligonucleotide-ligation assay (OLA) (Landegren et aL Science 241:1077 (1988), the allele-specific ligation chain reaction (LCR) (Barrany Proc. Natl. Acad. Sci. U.S.A. 88:189-193 (1991)), gap-LCR (Abravaya et al. Nucl Acids Res 23:675-682 (1995)), radioactive and/or fluorescent DNA sequencing using standard procedures well known In the art, and peptide nucleic acid (PNA) assays (Orum et al., Nucl.Acids Res, 21:S332-5358 (1993); Thiede et al., Nucl. Acids Res. 24:983-984 (1998)).

In addition to the genotypes defined by the polymorphisms of the invention, as described in co-pending PCT application WO 98/35802 published November 14,1996, genotypes characterized by the presence of the alleles 1909:205; 1884:235; 1A2:238; 1E4:271; 24E2:245; 288:208; 3321-1:88 (denoted 3321-1:97 therein): 4073-1:182; 4440-1:180; 4440-2:139; 731-1:177; 5091-1:148:3216-1221;4072-2:170 (denoted 4072-2:148 therein); 950-1:142;950-2:164;950-3:165: 950-4:128;950-6:151;95-8:137;83-1:151;63-2:113;63-3:169;65-1206;65-2:159;68-1:167;241-5:108; 241-29:113; 373-8:151; and 373-29:113, alleles D6S258:199, D6SZ65:122, D6S105:124, D6S306:238, D6S464:206: and D6S1001:180, and/or alleles associates with the HHP-1. the HHP-19 or HHP-29 single base-pair polymorphisms can also be used to assist in the identification of an individual whose genome contains 24d1 and/or 24d2. For example, the assessing step can be performed by a process which comprises subjecting the DNA or RNA to amplification using oligonucleotide primers flanking the polymorphism at base 35983 of Table 1, and oligonucleotides flanking 24d1 and/or 24d2, oligonucleotide primers flanking at least one of the base-pair polymorphisms HHP-1, HHp-19, and HHP-29, oligonucleotide primers flanking at least one of the microsatellite repeat alleles, or oligonucleotide primers for any combination of polymorphisms or microsatellite repeat alleles thereof.

Oligonucleotides useful in diagnostic assays are typically at least 8 consecutive nucleotides in length, and may range upwards of 18 nucleotides in length to greater than 100 or more consecutive nucleotides. Such oligonucleotides can be derived from either the genomic DNA of Figure 8 or 9. or cDNA sequences derived therefrom, or may be synthesized.

Additionally, the proteins encoded by such cDNAs are useful In the generation of antibodies for analysis of gene expression and in diagnostic assays, and in the purification of related proteins.

### E. General Methods

The nucleic acid compositions of this invention, whether RNA, cDNA, genomic DNA. or a hybrid of the various combinations, may be isolated from natural sources, including cloned DNA, or may be synthesized *in vitro.* The nucleic acids claimed may be present in transformed or transfected whole cells, in a transformed or transfected cell lysate, or in a partially purified or substantially pure form.

Techniques for nucleic acid manipulation of the nucleic acid sequences of the invention such as subcloning nucleic acid sequences encoding polypeptides into expression vectors, labeling probes, DNA hybridization, and the like are described generally in Sambrook et al., Molecular Cloning- a Laboratory Manual (2nd Ed.). Vol. 1-3. Cold Spring Harbor Laboratory. Cold Spring Harbor, New York, (1989), which is incorporated herein by reference. This manual is hereinafter referred to as "Sambrook et *al".*

There are various methods of Isolating the nucleic acid sequences of the invention. For example. DNA is isolated from a genomic or cONA library using labeled oligonucleotide probes having sequences complementary to the sequences disclosed herein. Such probes can be used directly in hybridization assays. Alternatively probes can be designed for use in amplification techniques such as PCR.

To prepare a cDNA library, mRNA is isolated from tissue such as heart or pancreas, preferably a tissue wherein expression of the gene or gene family is likely to occur, cDNA is prepared from the mRNA and ligated into a recombinant vector. The vector is transfected into a recombinant host for propagation, screening and cloning. Methods for making and screening cDNA libraries are well known. See Gubler. U. and Hoffman. 8.J. Gene 25:263-289 (1983) and Sambrook *et al.*

For a genomic library, for example, the DNA is extracted from tissue and either mechanically sheared or enzymatically digested to yield fragments of about 12-20 kb. The fragments are then separated by gradient centrifugation from undesired sizes and are constructed in bacteriophage lambda vectors. These vectors and phage are packaged *in vitro,* as described in Sambrook, *et al.* Recombinant phage are analyzed by plaque hybridization as described in Benton and Davis. Science 196:180-182 (1977). Colony hybridization is carried out as generally described in M. Grunstein et al. Proc. Natl. Acad. Sci. USA. 72:3981-3965 (1975).

DNA of interest is identified In either cDNA or genomic libraries by its ability to hybridize with nucleic acid probes, for example on Southern blots, and these DNA regions are Isolated by standard methods familliar to those of skill in the art. See Sambrook *et al*.

In PCR techniques, oligonucleotide primers complementary to the two 3" borders of the DNA region to be amplified are synthesized. The polymerase chain reaction is then carried out using the two primers. See PCR Protocols: a Guids to Method and Applications (Innis. M. Gelland, D., Sninsky, J. and White. T.. eds.), Academic Press, San Diego (1990). Primers can be selected to amplify the entire regions encoding a full-length sequence of interest or to amplify smaller DNA segments as desired.

PCR can be used in a variety of protocols to isolate cDNA's encoding a sequence of interest. In these protocols, appropriate primers and probes for amplifying DNA encoding a sequence of interest are generated from analysis of the DNA sequences listed herein. Once such regions are PCR-amplified, they can be sequenced and oligonucleotide probes can be prepared from sequence obtained.

Oligonucleotides for use as primers or probes are chemically synthesized according to the solid phase phosphoramidite triester method first described by Beaucage. S.L. and Carruthers, M.H., Tetrahedron Lett., 22(20):1859-1862 (1981) using an automated synthesizer, as described in Needharn-VanDevanter, D.R., et al., Nucleic Acids Res. 12:8159-6168 (1984). Purification of oligonucleotides is by either native acrylamide gel electrophoresis or by anion-exchange HPLC as described In Pearson, J.D. and Regnier, F.E., J. Chrom., 255:137-149 (1983). The sequence of the synthetic oligonucleotide can be verified using the chemical degradation method of Maxam, A.M. and Gilbert. W., In Grossman. L. and Moldave, D., eds. Academic Press, New York, Methods in Enzymology 65:499-560 (1960).

This invention also embraces diagnostic kits for detecting DNA or RNA comprising the polymorphism at base 35983 of Table 1 in tissue or blood samples which comprise nucleic acid probes as described herein and instructional material. The kit may also contain additional components such as labeled compounds, as described herein for identification of duplexed nucleic acids.

The following examples are provided to illustrate the invention but not to limit its scope. Other variants of the invention will be readily apparent to one of ordinary skill in the art and are encompassed by the appended claims.

### F. EXPERIMENTAL EXAMPLES

### Sequencing of 235 kb from a Homozygous Ancestral (Affected) individual

In these studies the entire genomic sequence was determined from an HH affected individual for a region corresponding to a 235,033 bp region surrounding the HFE gene between the flanking markers D6S2238 and D6S2241. The sequence was derived from a human lymphoblastoid cell line. HC14. that is homozygous for the ancestral HH mutation and region. The sequence from the ancestral chromosome (Figure 9) was compared to the sequence of the region in an unaffected individual (Figure 8) disclosed in copending U.S.S.N. 08/724,394 to identify polymorphic sites. A subset of the polymorphic alleles so defined were further studied to determine their frequency in a collection of random individuals.

The cell line HC14 was deposited with the ATCC om June 25, 1997. and is designated ATCC CRL-12371.

### a. Cosmid Library Screening

The strategy and methodology for sequencing the genomic DNA for the affected individual was essentially as described in copending U.S.S.N. 08/724,394. Basically, a cosmid library was constructed using high molecular weight DNA from HC14 cells. The library was constructed in the supercos vector (Stratagene, La Jolla, CA). Colonies were replicated onto Biotrans nylon filters (ICN) using standard techniques. Probes from genomic subclones used in the generation of the sequence of the unaffected sequence disclosed in 08/724,394 were isolated by gel electrophoresis and electroporation. Subclones were chosen at a spacing of approximately 20 kb throughout the 235 kb region. The DNA was labeled by incorporation of 32P dCTP by the random primer labeling approach. Positively hybridizing clones were isolated to purity by a secondary screening step. Cosmid insert ends were sequenced to determine whether full coverage had been obtained, and which clones formed a minimal path of cosmids through the 235 kb region.

### b. Sample Sequencing

A minimal set of cosmid clones chosen to cover the 235 kb region were prepped with the Qiagen Maxi-Prep system. Ten micrograms of DNA from each cosmid preparation were sonicated in a Heat Systems Sonicator XL and end-repaired with Klenow (USB) and T4 DNA polymerase (USB). The sheared fragments were size selected between three to four kilobases on a 0.7% agarose gel and then ligated to BstXl linkers (Invitrogen). The ligations were gel purified on a 0.7% agarose gel and cloned into a pSP72 derivative plasmid vector. The resulting plasmids were transformed into electrocompetent DH5α cells and plated on LB-carbenicillin plates. A sufficient number of colonies was picked to achieve 15-fold clone coverage. The appropriate number of colonies was calculated by the following equation to generate a single-fold sequence coverage: Number of colonies = size of bacterial clone (in kb)/average sequence read length (0.4 kb). These colonies were prepped in the 96-well Qiagen REAL, and the 5' to 3' DNA Prep Kit, and AGCT end-sequenced with oligo MAP1 using standard ABI Dye Terminator protocols. MAP1 was CGTTAGAACGCGGCTACAAT.

### c. Genomic Sequencing

The MAP1 sequences from the cosmid clones HC182, HC187, HC189, HC195, HC199, HC200, HC201, HC206, HC207, and HC212 were assembled into contigs with the Staden package (available from Roger Staden, MRC). A minimal set of 3 kb clones was selected for sequencing with oligo labeled MAP2 that sits on the opposite end of the plasmid vector. The sequence of MAP2 was GCCGATTCATTAATGCAGGT. The MAP2 sequences were entered into the Staden database in conjunction with the MAP1 sequences to generate a tiling path of 3 kb clones across the region. The plasmid 3 kb libraries were concurrently transformed in 96 well format into pox38UR (available from C. Martin, Lawrence Berkeley Laboratories). The transformants were subsequently mated with JGM (Strathman et al. P.N.A.S. 88:1247-1250 (1991) in 96 well format. All matings of the 3 kb clones within the tiling path were streaked on LB-carbenicillin-kanamycin plates and a random selection of 12 colonies per 3 kb clone was prepped in the AGCT system. The oligos -21: CTGTAAAACGACGGCCAGTC, and REV: GCAGGAAACAGCTATGACC were used to sequence off both ends of the transposon. Each 3 kb clone was assembled in conjunction with the end sequence information from all cosmid clones in the region.

In some regions, the coverage of the genomic sequence by cosmids was incomplete. Any gaps in the sequence were filled by using standard PCR techniques to amplify genomic DNA in those regions and standard ABI dye terminator chemistry to sequence the amplification products.

### d. Identification of Polymorphic Sites

The assembled sequence of the cosmid clones in connection with the PCR amplified genomic DNA was compared to the genomic sequence of the unaffected individual using the FASTA algorithm. Numeric values were assigned to the sequenced regions of 1 to 235,303, wherein base 1 refers to the first C in the CA repeat of D6S2238 and base 235,303 is the last T in the GT repeat of D6S2241 of the unaffected sequence (Figure 8). Table 1 lists the differences between the two compared sequences. Note that previously disclosed (Feder et al., Nature Genetics 13:399-408 (1996)) polymorphic sites D6S2238 (base 1), D6S2241 (base 235,032), 24d1 (base 41316), and D6S2239 (base 84841) are not included in the list of new polymorphisms, although they are provided for reference in a footnote to the Table and were observed in the ancestral sequence. In the Table, a single base change such as C-T refers to a C in the unaffected sequence at the indicated base position that occurred as a T in the corresponding position in the affected sequence. Similarly, an insertion of one or more bases, such as TTT in the affected sequence, is represented as "TTT INS" between the indicated bases of the unaffected sequence. A deletion of one or more bases occurring in the affected sequence, such as AAA DEL, is represented as the deletion of the indicated bases in the unaffected sequence.

### e. Characterization of Rare Polymorphisms

In this study about 100 of the polymorphisms of Table 1 were arbitrarily chosen for further characterization. Allele frequencies in the general population were estimated by OLA analysis using a population of random DNAs (the "CEPH" collection, J. Dausset et al., Genomics 6(3):575-577 (1990)). These results are provided in Table 2.

One single base pair difference, occurring at base 35983 and designated C182.1 G7T/C (an A to G change on the opposite strand) was present in the ancestral chromosome and rare in the random DNAs. This change occurred in a noncoding region of the hemochromatosis gene near exon 7 approximately 5.3 kb from the 24d1 (Cys282Tyr) mutation. OLA was used to genotype 90 hemochromatosis patients for the C182.1 G7T/C base pair change. The frequency for C occurring at this position in the patients was 79.4% as compared to 5% in the random DNAs. Eighty-five of the 90 patients assayed contained identical 24d1 and C182.1G7T/C genotypes. Four of the remaining 5 patients were homozygous at 24d1 and heterozygous at C182.1 G7T/C; one was heterozygous at 24d1 and homozygous at C182.1 G7T/C. The primers used for this analysis were as follows.
PCR primer for detection:

| | |
|---|---|
| 182.1G7.F | 5'-GCATCAGCGATTAACTTCTAC-3' |
| 182.1G7.R | 5'-TTGCATTGTGGTGAAATCAGGG -3' |

For the detection assay, the biotinylated primers used were as follows.

| | |
|---|---|
| 182.1G7.C | 5' (b)CTGAGTAATTGTTTAAGGTGC -3' |
| 182.1G7.T | 5' (b)CTGAGTAATTGTTTAAGGTGT -3' |

The phosphorylated digoxigenin-labeled primer used was:

| | |
|---|---|
| **182.1G7.D** | **5'(p)AGAAGAGATAGATATGGTGG -3'** |

The above rare alleles is to be used as a marker for 24d1 and is especially useful in screening assays for the likely presence of 24d1 and/or 24d2.

## Claims

1. An oligonucleotide consisting of at least 8 to about 100 consecutive bases from the sequence of SEQ ID NO: 21, or its complement, including the polymorphic site at base 35935 of SEQ ID NO: 21 ("polymorphic site C182.1G7").

2. An oligonucleotide pair consisting of SEQ ID NO: 40 and SEQ ID NO: 41 for amplification of an nucleic acid sequence from SEQ ID NO: 21 or its complement that includes the polymorphic site at base 35935 of SEQ ID NO: 21 ("polymorphic site C182.1G7").

3. An *in vitro* method to determine the presence or absence of the common hereditary hemochromatosis (HFE) gene mutation in a sample of DNA or RNA comprising:
assessing the DNA or RNA for the presence or absence of a haplotype comprising the allele of SEQ ID NO: 21 or its complement at polymorphic site at base 35935 of SEQ ID NO: 21 ("polymorphic site C182.1G7"),
wherein the absence of the haplotype indicates the likely absence of the HFE mutation in the genome from which the sample has been obtained and the presence of the haplotype indicates the likely presence of the HFE gene mutation in the genome from which the sample has been obtained.

4. The method of claim 3, wherein the method further comprises assessing the DNA or RNA for the presence of at least one of the polymorphisms 24d1, 24d2, HHP-1, HHP-19, or HHP-29; or microsatellite repeat alleles 19D9:205, 18B4:235, 1A2:239, 1E4:271; 24E2:245, 2B8:206, 3321-1:98, 4073-1:182, 4440-1:180, 4440-2:139, 731-1:177, 5091-1:148, 3216-1:221, 4072-2:170, 950-1:142, 950-2:164, 950-3:165, 950-4:128, 950-6:151, 950-8.-137, 63-1:151, 63-2:113, 63-3:169, 65-1:206, 65-2:159, 68-1:167, 241-5:108, 241-29:113, 373-8:151, 373-29:113, D6S258:199, D6S265:122, D6S105:124, D6S306:238, D6S464:206, or D6S100I:180.

5. The method of claim 3, wherein the haplotype further comprises an allele of SEQ ID NO: 2I or its complement in at least one polymorphic site of Table 1 other than the polymorphic site C182.1 G7.

6. The method of claim 3, wherein the haplotype further comprises an allele of SEQ ID NO: 21 or its complement in at least two polymorphic sites of Table 1 other than the polymorphic site C182.1G7.

7. An *in vitro* method to determine the presence or absence of the common hereditary hemochromatosis (HFE) gene mutation in a sample of DNA or RNA comprising:
assessing the DNA or RNA for the presence or absence of a G genotype at polymorphic site at base 35935 of SEQ ID NO: 21 ("polymorphic site C182.1G7") or a C genotype at the complementary site,
wherein the absence of the C or G genotype at polymorphic site C182.1G7 indicates the likely absence of the HFE gene mutation in the genome from which the sample has been obtained and the presence of the C or G genotype at polymorphic site C182.1G7 indicates the likely presence of the HFE gene mutation in the genome from which the sample has been obtained.

8. A kit comprising one or more oligonucleotides of claim 1.

9. A kit comprising at least one oligonucleotide pair of claim 2.

## Patentansprüche

1. Oligonukleotid, welches aus mindestens acht bis ungefähr 100 aufeinander folgenden Basen aus der Sequenz der SEQ ID NO: 21 oder deren Komplement besteht, einschließlich der polymorphen Stelle (Site) an der Base 35935 der SEQ ID NO: 21 ("polymorphe Stelle C 182.1 G7").

2. Oligonukleotidpaar, welches aus SEQ ID NO: 40 und SEQ ID NO: 41 besteht zur Amplifikation einer Nukleinsäuresequenz aus SEQ ID NO: 21 oder deren Komplement, das die polymorphe Stelle an der Base 35935 der SEQ ID NO: 21 ("polymorphe Stelle C182.1G7") enthält.

3. *In* vitro-Verfahren zur Bestimmung der Anwesenheit oder Abwesenheit der Genmutation der allgemeinen hereditären Hämochromatose (HFE) in einer Probe von DNA oder RNA, welches umfasst:
Untersuchung der DNA oder RNA auf Anwesenheit oder Abwesenheit eines Haplotyps, welcher das Allel der SEQ ID NO: 21 oder deren Komplement an der polymorphen Stelle an der Base 35935 der SEQ ID NO: 21 (_{"}polymorphe Stelle C182.1G7") umfasst,
wobei die Abwesenheit des Haplotyps die wahrscheinliche Abwesenheit der HFE-Mutation im Genom anzeigt, aus welchem die Probe erhalten worden ist, und die Anwesenheit des Haplotyps die wahrscheinliche Anwesenheit der HFE-Genmutation im Genom anzeigt, aus welchem die Probe erhalten worden ist.

4. Verfahren nach Anspruch 3, wobei das Verfahren weiter umfasst die Untersuchung der DNA oder RNA auf Anwesenheit wenigstens eines der Polymorphismen 24d1, 24d2, HHP-1, HHP-19 oder HHP-29; oder Mikrosatelliten-Repeat-Allele 19D9:205, 18B4:235, 1A2:239, 1E4:271; 24E2:245, 2B8:206, 3321-1:98, 4073-1:182, 4440-1:180, 4440-2:139, 731-1:177, 5091-1:148, 3216-1:221, 4072-2:170, 950-1:142, 950-2:164, 950-3:165, 950-4:128, 950-6:151, 950-8:137, 63-1:151, 63-2:113, 63-3:169, 65-1:206, 65-2:159, 68-1:167, 241-5:108, 241-29:113, 373-8:151, 373-29:113, D6S258:199, D6S265:122, D6S105:124, D6S306:238, D6S464:206 oder D6S1001:180.

5. Verfahren nach Anspruch 3, wobei der Haplotyp weiter umfasst ein Allel der SEQ ID NO: 21 oder deren Komplement in wenigstens einer polymorphen Stelle der Tabelle 1 mit Ausnahme der polymorphen Stelle C182.1G7.

6. Verfahren nach Anspruch 3, wobei der Haplotyp weiter umfasst ein Allel der SEQ ID NO: 21 oder deren Komplement in wenigstens zwei polymorphen Stellen der Tabelle 1 mit Ausnahme der polymorphen Stelle C182.1G7.

7. *In* vitro-Verfahren zur Bestimmung der Anwesenheit oder Abwesenheit der Genmutation der allgemeinen hereditären Hämochromatose (HFE) in einer Probe von DNA oder RNA, welches umfasst:
Untersuchung der DNA oder RNA auf Anwesenheit oder Abwesenheit eines G-Genotyps an der polymorphen Stelle an der Base 35935 der SEQ ID NO: 21 ("polymorphe Stelle C182.1G7") oder eines C-Genotyps an der komplementären Stelle,
wobei die Abwesenheit des C- oder G-Genotyps an der polymorphen Stelle C182.1G7 die wahrscheinliche Abwesenheit der HFE-Genmutation im Genom anzeigt, aus welchem die Probe erhalten worden ist, und die Anwesenheit des C- oder G-Genotyps an der polymorphen Stelle C182.1G7 die wahrscheinliche Anwesenheit der HFE-Genmutation im Genom anzeigt, aus welchem die Probe erhalten worden ist.

8. Kit, welches ein oder mehrere Oligonukleotide des Anspruchs 1 umfasst.

9. Kit, welches wenigstens ein Oligonukleotidpaar des Anspruchs 2 umfasst.

## Revendications

1. Oligonucléotide consistant en au moins 8 à environ 100 bases consécutives provenant de la séquence de SEQ ID N° 21, ou de son complément, incluant le site polymorphe au niveau de la base 35 935 de SEQ ID N° 21 (« site polymorphe C182.1G7 »).

2. Paire d'oligonucléotides consistant en SEQ ID N° 40 et SEQ ID N° 41 pour amplification d'une séquence d'acide nucléique issue de SEQ ID N° 21 ou de son complément qui inclut le site polymorphe au niveau de la base 35 935 de SEQ ID N° 21 (« site polymorphe C182.1G7 »).

3. Procédé *in vitro* pour déterminer la présence ou l'absence de la mutation génétique de l'hémochromatose commune héréditaire (HFE) dans un échantillon d'ADN ou d'ARN comprenant :
l'évaluation de l'ADN ou de l'ARN pour déterminer la présence ou l'absence d'un haplotype comprenant l'allèle de SEQ ID N° 21 ou de son complément au niveau du site polymorphe au niveau de la base 35 935 de SEQ ID N° 21 (« site polymorphe C182.1G7 »),
dans lequel l'absence de l'haplotype indique l'absence probable de la mutation HFE dans le génome à partir duquel l'échantillon a été obtenu et la présence de l'haplotype indique la présence probable de la mutation génétique HFE dans le génome à partir duquel l'échantillon a été obtenu.

4. Procédé selon la revendication 3, dans lequel le procédé inclut en outre l'évaluation de l'ADN ou de l'ARN pour déterminer la présence d'au moins un des polymorphismes 24d1, 24d2, HHP-1, HHP-19 ou HHP-29 ; ou de microsatellites répétés des allèles 19D9:205, 18B4:235, 1A2:239, 1E4:271, 24E2:245, 2B8:206, 3321-1:98, 4073-1:182, 4440-1:180, 4440-2:139, 731-1:177, 5091-1:148, 3216-1:221, 4072-2:170, 950-1:142, 950-2:164, 950-3:165, 950-4:128, 950-6:151, 950-8:137, 63-1:151, 63-2:113, 63-3:169, 65-1:206, 65-2:159, 68-1:167, 241-5:108, 241-29:113, 373-8:151, 373-29:113, D6S258:199, D6S265:122, D6S105:124, D6S306:238, D6S464:206, ou D6S 1001:180.

5. Procédé selon la revendication 3, dans lequel l'haplotype comprend en outre un allèle de SEQ ID N° 21 ou de son complément dans au moins un site polymorphe du tableau 1 autre que le site polymorphe C 182.1 G7.

6. Procédé selon la revendication 3, dans lequel l'haplotype comprend en outre un allèle de SEQ ID N° 21 ou de son complément dans au moins deux sites polymorphes du tableau 1 autres que le site polymorphe C182.1G7.

7. Procédé *in vitro* pour déterminer la présence ou l'absence de la mutation génétique de l'hémochromatose commune héréditaire (HFE) dans un échantillon d'ADN ou d'ARN comprenant :
l'évaluation de l'ADN ou de l'ARN pour déterminer la présence ou l'absence d'un génotype G au niveau du site polymorphe au niveau de la base 35 935 de SEQ ID N° 21 (« site polymorphe C182.1G7 ») ou d'un génotype C au niveau du site complémentaire,
dans lequel l'absence du génotype C ou G au niveau du site polymorphe C182.1G7 indique l'absence probable de la mutation génétique HFE dans le génome à partir duquel l'échantillon a été obtenu et la présence du génotype C ou G au niveau du site polymorphe C182.1G7 indique la présence probable de la mutation génétique HFE dans le génome à partir duquel l'échantillon a été obtenu.

8. Kit comprenant un ou plusieurs oligonucléotides selon la revendication 1.

9. Kit comprenant au moins une paire d'oligonucléotides selon la revendication 2.


### SEQUENCE LISTING

<110> Progenitor, Inc.
<120> POLYMORPHISMS AND NEW GENES IN THE REGION OF THE HUMAN HEMOCHROMATOSIS GENE
<130> 8907-0057-227
<140> EP 97910741.4
   <141> 1997-09-30
<150> PCT/US97/17658
   <151> 1997-09-30
<150> US 08/852, 495
   <151> 1997-05-07
<150> US 08/724,394
   <151> 1997-10-01
<160> 49
<170> FastSEQ for Windows Version 3.0
<210> 1
   <211> 526
   <212> PRT
   <213> Homo sapien
<400> 1
   Met Ala Val Phe Pro Ser Ser Gly Leu Pro Arg Cys Leu Leu Thr Leu
    1               5                  10                  15
   Ile Leu Leu Gln Leu Pro Lys Leu Asp Ser Ala Pro Phe Asp Val Ile
               20                  25                  30
   Gly Pro Pro Glu Pro Ile Leu Ala Val Val Gly Glu Asp Ala Glu Leu
           35                  40                  45
   Pro Cys Arg Leu Ser Pro Asn Ala Ser Ala Glu His Leu Glu Leu Arg
       50                  55                  60
   Trp Phe Arg Lys Lys Val Ser Pro Ala Val Leu Val His Arg Asp Gly
   65                  70                  75                  80
   Arg Glu Gln Glu Ala Glu Gln Met Pro Glu Tyr Arg Gly Arg Ala Thr
                   85                  90                  95
   Leu Val Gln Asp Gly Ile Ala Lys Gly Arg Val Ala Leu Arg Ile Arg
               100                 105                 110
   Gly Val Arg Val Ser Asp Asp Gly Glu Tyr Thr Cys Phe Phe Arg Glu
           115                 120                 125
   Asp Gly Ser Tyr Glu Glu Ala Leu Val His Leu Lys Val Ala Ala Leu
       130                 135                 140
   Gly Ser Asp Pro His Ile Ser Met Gln Val Gln Glu Asn Gly Glu Ile
   145                 150                 155                 160
   Cys Leu Glu Cys Thr Ser Val Gly Trp Tyr Pro Glu Pro Gln Val Gln
                   165                 170                 175
   Trp Arg Thr Ser Lys Gly Glu Lys Phe Pro Ser Thr Ser Glu Ser Arg
               180                 185                 190
   Asn Pro Asp Glu Glu Gly Leu Phe Thr Val Ala Ala Ser Val Ile Ile
           195                 200                 205
   Arg Asp Thr Ser Thr Lys Asn Val Ser Cys Tyr Ile Gln Asn Leu Leu
       210                 215                 220
   Leu Gly Gln Glu Lys Lys Val Glu Ile Ser Ile Pro Ala Ser Ser Leu
   225                 230                 235                 240
   Pro Arg Leu Thr Pro Trp Ile Val Ala Val Ala Val Ile Leu Met Val
                   245                 250                 255
   Leu Gly Leu Leu Thr Ile Gly Ser Ile Phe Phe Thr Trp Arg Leu Tyr
               260                 265                 270
   Asn Glu Arg Pro Arg Glu Arg Arg Asn Glu Phe Ser Ser Lys Glu Arg
           275                 280                 285
   Leu Leu Glu Glu Leu Lys Trp Lys Lys Ala Thr Leu His Ala Val Asp
       290                 295                 300
   Val Thr Leu Asp Pro Asp Thr Ala His Pro His Leu Phe Leu Tyr Glu
   305                 310                 315                 320
   Asp Ser Lys Ser Val Arg Leu Glu Asp Ser Arg Gln Lys Leu Pro Glu
                   325                 330                 335
   Lys Thr Glu Arg Phe Asp Ser Trp Pro Cys Val Leu Gly Arg Glu Thr
               340                 345                 350
   Phe Thr Ser Gly Arg His Tyr Trp Glu Val Glu Val Gly Asp Arg Thr
           355                 360                 365
   Asp Trp Ala Ile Gly Val Cys Arg Glu Asn Val Met Lys Lys Gly Phe
       370                 375                 380
   Asp Pro Met Thr Pro Glu Asn Gly Phe Trp Ala Val Glu Leu Tyr Gly
   385                 390                 395                 400
   Asn Gly Tyr Trp Ala Leu Thr Pro Leu Arg Thr Pro Leu Pro Leu Ala
                   405                 410                 415
   Gly Pro Pro Arg Arg Val Gly Ile Phe Leu Asp Tyr Glu Ser Gly Asp
               420                 425                 430
   Ile Ser Phe Tyr Asn Met Asn Asp Gly Ser Asp Ile Tyr Thr Phe Ser
           435                 440                 445
   Asn Val Thr Phe Ser Gly Pro Leu Arg Phe Phe Phe Cys Leu Trp Ser
       450                 455                 460
   Ser Gly Lys Lys Pro Leu Thr Ile Cys Pro Ile Ala Asp Gly Pro Glu
   465                 470                 475                 480
   Arg Val Thr Val Ile Ala Asn Ala Gln Asp Leu Ser Lys Glu Ile Pro
                   485                 490                 495
   Leu Ser Pro Met Gly Glu Glu Ser Ala Pro Arg Asp Ala Asp Thr Leu
               500                 505                 510
   His Ser Lys Leu Ile Pro Thr Gln Pro Ser Gln Gly Ala Pro
           515                 520                 525
<210> 2
   <211> 527
   <212> PRT
   <213> Homo sapien
<400> 2
   Met Glu Ser Ala Ala Ala Leu His Phe Ser Arg Pro Ala Ser Leu Leu
    1               5                  10                  15
   Leu Leu Leu Leu Ser Leu Cys Ala Leu Val Ser Ala Gln Phe Ile Val
               20                  25                  30
   Val Gly Pro Thr Asp Pro Ile Leu Ala Thr Val Gly Glu Asn Thr Thr
           35                  40                  45
   Leu Arg Cys His Leu Ser Pro Glu Lys Asn Ala Glu Asp Met Glu Val
       50                  55                  60
   Arg Trp Phe Arg Ser Gln Phe Ser Pro Ala Val Phe Val Tyr Lys Gly
   65                  70                  75                  80
   Gly Arg Glu Arg Thr Glu Glu Gln Met Glu Glu Tyr Arg Gly Arg Thr
                   85                  90                  95
   Thr Phe Val Ser Lys Asp Ile Ser Arg Gly Ser Val Ala Leu Val Ile
               100                 105                 110
   His Asn Ile Thr Ala Gln Glu Asn Gly Thr Tyr Arg Cys Tyr Phe Gln
           115                 120                 125
   Glu Gly Arg Ser Tyr Asp Glu Ala Ile Leu His Leu Val Val Ala Gly
       130                 135                 140
   Leu Gly Ser Lys Pro Leu Ile Ser Met Arg Gly His Glu Asp Gly Gly
   145                 150                 155                 160
   Ile Arg Leu Glu Cys Ile Ser Arg Gly Trp Tyr Pro Lys Pro Leu Thr
   165 170 175
   Val Trp Arg Asp Pro Tyr Gly Gly Val Ala Pro Ala Leu Lys Glu Val
   180 185 190
   Ser Met Pro Asp Ala Asp Gly Leu Phe Met Val Thr Thr Ala Val Ile
   195 200 205
   Ile Arg Asp Lys Ser Val Arg Asn Met Ser Cys Ser Ile Asn Asn Thr
   210 215 220
   Leu Leu Gly Gln Lys Lys Glu Ser Val Ile Phe Ile Pro Glu Ser Phe
   225 230 235 240
   Met Pro Ser Val Ser Pro Cys Ala Val Ala Leu Pro Ile Ile Val Val
   245 250 255
   Ile Leu Met Ile Pro Ile Ala Val Cys Ile Tyr Trp Ile Asn Lys Leu
   260 265 270
   Gln Lys Glu Lys Lys Ile Leu Ser Gly Glu Lys Glu Phe Glu Arg Glu
   275 280 285
   Thr Arg Glu Ile Ala Leu Lys Glu Leu Glu Lys Glu Arg Val Gln Lys
   290 295 300
   Glu Glu Glu Leu Gln Val Lys Glu Lys Leu Gln Glu Glu Leu Arg Trp
   305 310 315 320
   Arg Arg Thr Phe Leu His Ala Val Asp Val Val Leu Asp Pro Asp Thr
   325 330 335
   Ala His Pro Asp Leu Phe Leu Ser Glu Asp Arg Arg Ser Val Arg Arg
   340 345 350
   Cys Pro Phe Arg His Leu Gly Glu Ser Val Pro Asp Asn Pro Glu Arg
   355 360 365
   Phe Asp Ser Gln Pro Cys Val Leu Gly Arg Glu Ser Phe Ala Ser Gly
   370 375 380
   Lys His Tyr Trp Glu Val Glu Val Glu Asn Val Ile Glu Trp Thr Val
   385 390 395 400
   Gly Val Cys Arg Asp Ser Val Glu Arg Lys Gly Glu Val Leu Leu Ile
   405 410 415
   Pro Gln Asn Gly Phe Trp Thr Leu Glu Met His Lys Gly Gln Tyr Arg
   420 425 430
   Ala Val Ser Ser Pro Asp Arg Ile Leu Pro Leu Lys Glu Ser Leu Cys
   435 440 445
   Arg Val Gly Val Phe Leu Asp Tyr Glu Ala Gly Asp Val Ser Phe Tyr
   450 455 460
   Asn Met Arg Asp Arg Ser His Ile Tyr Thr Cys Pro Arg Ser Ala Phe
   465 470 475 480
   Ser Val Pro Val Arg Phe Phe Phe Arg Leu Gly Cys Glu Asp Ser Pro
   485 490 495
   Ile Phe Ile Cys Pro Ala Leu Thr Gly Ala Asn Gly Val Thr Val Pro
   500 505 510
   Glu Glu Gly Leu Thr Leu His Arg Val Gly Thr His Gln Ser Leu
   515 520 525
<210> 3
   <211> 523
   <212> PRT
   <213> Homo sapien
<400> 3
   Met Glu Pro Ala Ala Ala Leu His Phe Ser Leu Pro Ala Ser Leu Leu
    1               5                   10                  15
   Leu Leu Leu Leu Leu Leu Leu Leu Ser Leu Cys Ala Leu Val Ser Ala
               20                  25                  30
   Gln Phe Thr Val Val Gly Pro Ala Asn Pro Ile Leu Ala Met Val Gly
           35                  40                  45
   Glu Asn Thr Thr Leu Arg Cys His Leu Ser Pro Glu Lys Asn Ala Glu
       50                  55                  60
   Asp Met Glu Val Arg Trp Phe Arg Ser Gln Phe Ser Pro Ala Val Phe
   65                  70                  75                  80
   Val Tyr Lys Gly Gly Arg Glu Arg Thr Glu Glu Gln Met Glu Glu Tyr
                   85                  90                  95
   Arg Gly Arg Ile Thr Phe Val Ser Lys Asp Ile Asn Arg Gly Ser Val
               100                 105                 110
   Ala Leu Val Ile His Asn Val Thr Ala Gln Glu Asn Gly Ile Tyr Arg
           115                 120                 125
   Cys Tyr Phe Gln Glu Gly Arg Ser Tyr Asp Glu Ala Ile Leu Arg Leu
       130                 135                 140
   Val Val Ala Gly Leu Gly Ser Lys Pro Leu Ile Glu Ile Lys Ala Gln
   145                 150                 155                 160
   Glu Asp Gly Ser Ile Trp Leu Glu Cys Ile Ser Gly Gly Trp Tyr Pro
                   165                 170                 175
   Glu Pro Leu Thr Val Trp Arg Asp Pro Tyr Gly Glu Val Val Pro Ala
               180                 185                 190
   Leu Lys Glu Val Ser Ile Ala Asp Ala Asp Gly Leu Phe Met Val Thr
           195                 200                 205
   Thr Ala Val Ile Ile Arg Asp Lys Tyr Val Arg Asn Val Ser Cys Ser
       210                 215                 220
   Val Asn Asn Thr Leu Leu Gly Gln Glu Lys Glu Thr Val Ile Phe Ile
   225                 230                 235                 240
   Pro Glu Ser Phe Met Pro Ser Ala Ser Pro Trp Met Val Ala Leu Ala
                   245                 250                 255
   Val Ile Leu Thr Ala Ser Pro Trp Met Val Ser Met Thr Val Ile Leu
               260                 265                 270
   Ala Val Phe Ile Ile Phe Met Ala Val Ser Ile Cys Cys Ile Lys Lys
           275                 280                 285
   Leu Gln Arg Glu Lys Lys Ile Leu Ser Gly Glu Lys Lys Val Glu Gln
       290                 295                 300
   Glu Glu Lys Glu Ile Ala Gln Gln Leu Gln Glu Glu Leu Arg Trp Arg
   305                 310                 315                 320
   Arg Thr Phe Leu His Ala Ala Asp Val Val Leu Asp Pro Asp Thr Ala
                   325                 330                 335
   His Pro Glu Leu Phe Leu Ser Glu Asp Arg Arg Ser Val Arg Arg Gly
               340                 345                 350
   Pro Tyr Arg Gln Arg Val Pro Asp Asn Pro Glu Arg Phe Asp Ser Gln
           355                 360                 365
   Pro Cys Val Leu Gly Trp Glu Ser Phe Ala Ser Gly Lys His Tyr Trp
       370                 375                 380
   Glu Val Glu Val Glu Asn Val Met Val Trp Thr Val Gly Val Cys Arg
   385                 390                 395                 400
   His Ser Val Glu Arg Lys Gly Glu Val Leu Leu Ile Pro Gln Asn Gly
                   405                 410                 415
   Phe Trp Thr Leu Glu Met Phe Gly Asn Gln Tyr Arg Ala Leu Ser Ser
               420                 425                 430
   Pro Glu Arg Ile Leu Pro Leu Lys Glu Ser Leu Cys Arg Val Gly Val
           435                 440                 445
   Phe Leu Asp Tyr Glu Ala Gly Asp Val Ser Phe Tyr Asn Met Arg Asp
       450                 455                 460
   Arg Ser His Ile Tyr Thr Cys Pro Arg Ser Ala Phe Thr Val Pro Val
   465                 470                 475                 480
   Arg Phe Phe Phe Arg Leu Gly Ser Asp Asp Ser Pro Ile Phe Ile Cys
                   485                 490                 495
   Pro Ala Leu Thr Gly Ala Ser Gly Val Met Val Pro Glu Glu Gly Leu
               500                 505                 510
   Lys Leu His Arg Val Gly Thr His Gln Ser Leu
           515                 520
<210> 4
   <211> 513
   <212> PRT
   <213> Homo sapien
<400> 4
   Met Lys Met Ala Ser Phe Leu Ala Phe Leu Leu Leu Asn Phe Arg Val
   1               5                   10                  15
   Cys Leu Leu Leu Leu Gln Leu Leu Met Pro His Ser Ala Gln Phe Ser
               20                  25                  30
   Val Leu Gly Pro Ser Gly Pro Ile Leu Ala Met Val Gly Glu Asp Ala
           35                  40                  45
   Asp Leu Pro Cys His Leu Phe Pro Thr Met Ser Ala Glu Thr Met Glu
       50                  55                  60
   Leu Lys Trp Val Ser Ser Ser Leu Arg Gln Val Val Asn Val Tyr Ala
   65                  70                  75                  80
   Asp Gly Lys Glu Val Glu Asp Arg Gln Ser Ala Pro Tyr Arg Gly Arg
                   85                  90                  95
   Thr Ser Ile Leu Arg Asp Gly Ile Thr Ala Gly Lys Ala Ala Leu Arg
               100                 105                 110
   Ile His Asn Val Thr Ala Ser Asp Ser Gly Lys Tyr Leu Cys Tyr Phe
           115                 120                 125
   Gln Asp Gly Asp Phe Tyr Glu Lys Ala Leu Val Glu Leu Lys Val Ala
       130                 135                 140
   Ala Leu Gly Ser Asp Leu His Val Asp Val Lys Gly Tyr Lys Asp Gly
   145                 150                 155                 160
   Gly Ile His Leu Glu Cys Arg Ser Thr Gly Trp Tyr Pro Gln Pro Gln
                   165                 170                 175
   Ile Gln Trp Ser Asn Asn Lys Gly Glu Asn Ile Pro Thr Val Glu Ala
               180                 185                 190
   Pro Val Val Ala Asp Gly Val Gly Leu Tyr Ala Val Ala Ala Ser Val
           195                 200                 205
   Ile Met Arg Gly Ser Ser Gly Glu Gly Val Ser Cys Thr Ile Arg Ser
       210                 215                 220
   Ser Leu Leu Gly Leu Glu Lys Thr Ala Ser Ile Ser Ile Ala Asp Pro
   225                 230                 235                 240
   Phe Phe Arg Ser Ala Gln Arg Trp Ile Ala Ala Leu Ala Arg Thr Leu
                   245                 250                 255
   Pro Val Leu Leu Leu Leu Leu Gly Gly Ala Gly Tyr Phe Leu Trp Gln
               260                 265                 270
   Gln Gln Glu Glu Lys Lys Thr Gln Phe Arg Lys Lys Lys Arg Glu Gln
           275                 280                 285
   Glu Leu Arg Glu Met Ala Trp Ser Thr Met Lys Gln Glu Gln Ser Thr
       290                 295                 300
   Arg Val Lys Leu Leu Glu Glu Leu Arg Trp Arg Ser Ile Gln Tyr Ala
   305                 310                 315                 320
   Ser Arg Gly Glu Arg His Ser Ala Tyr Asn Glu Trp Lys Lys Ala Leu
                   325                 330                 335
   Phe Lys Pro Ala Asp Val Ile Leu Asp Pro Lys Thr Ala Asn Pro Ile
               340                 345                 350
   Leu Leu Val Ser Glu Asp Gln Arg Ser Val Gln Arg Ala Lys Glu Pro
           355                 360                 365
   Gln Asp Leu Pro Asp Asn Pro Glu Arg Phe Asn Trp His Tyr Cys Val
       370                 375                 380
   Leu Gly Cys Glu Ser Phe Ile Ser Gly Arg His Tyr Trp Glu Val Glu
   385                 390                 395                 400
   Val Gly Asp Arg Lys Glu Trp His Ile Gly Val Cys Ser Lys Asn Val
                   405                 410                 415
   Gln Arg Lys Gly Trp Val Lys Met Thr Pro Glu Asn Gly Phe Trp Thr
               420                 425                 430
   Met Gly Leu Thr Asp Gly Asn Lys Tyr Arg Thr Leu Thr Glu Pro Arg
           435                 440                 445
   Thr Asn Leu Lys Leu Pro Lys Pro Pro Lys Lys Val Gly Val Phe Leu
       450                 455                 460
   Asp Tyr Glu Thr Gly Asp Ile Ser Phe Tyr Asn Ala Val Asp Gly Ser
   465                 470                 475                 480
   His Ile His Thr Phe Leu Asp Val Ser Phe Ser Glu Ala Leu Tyr Phe
                   485                 490                 495
   Val Phe Arg Ile Leu Thr Leu Glu Pro Thr Ala Leu Ser Ile Cys Pro
   500 505 510
   Ala
<210> 5
   <211> 584
   <212> PRT
   <213> Homo sapien
<400> 5
   Met Lys Met Ala Ser Ser Leu Ala Phe Leu Leu Leu Asn Phe His Val
    1               5                  10                  15
   Ser Leu Phe Leu Val Gln Leu Leu Thr Pro Cys Ser Ala Gln Phe Ser
               20                  25                  30
   Val Leu Gly Pro Ser Gly Pro Ile Leu Ala Met Val Gly Glu Asp Ala
           35                  40                  45
   Asp Leu Pro Cys His Leu Phe Pro Thr Met Ser Ala Glu Thr Met Glu
       50                  55                  60
   Leu Arg Trp Val Ser Ser Ser Leu Arg Gln Val Val Asn Val Tyr Ala
   65                  70                  75                  80
   Asp Gly Lys Glu Val Glu Asp Arg Gln Ser Ala Pro Tyr Arg Gly Arg
                   85                  90                  95
   Thr Ser Ile Leu Arg Asp Gly Ile Thr Ala Gly Lys Ala Ala Leu Arg
               100                 105                 110
   Ile His Asn Val Thr Ala Ser Asp Ser Gly Lys Tyr Leu Cys Tyr Phe
           115                 120                 125
   Gln Asp Gly Asp Phe Tyr Glu Lys Ala Leu Val Glu Leu Lys Val Ala
       130                 135                 140
   Ala Leu Gly Ser Asp Leu His Ile Glu Val Lys Gly Tyr Glu Asp Gly
   145                 150                 155                 160
   Gly Ile His Leu Glu Cys Arg Ser Thr Gly Trp Tyr Pro Gln Pro Gln
                   165                 170                 175
   Ile Lys Trp Ser Asp Thr Lys Gly Glu Asn Ile Pro Ala Val Glu Ala
               180                 185                 190
   Pro Val Val Ala Asp Gly Val Gly Leu Tyr Ala Val Ala Ala Ser Val
           195                 200                 205
   Ile Met Arg Gly Ser Ser Gly Gly Gly Val Ser Cys Ile Ile Arg Asn
       210                 215                 220
   Ser Leu Leu Gly Leu Glu Lys Thr Ala Ser Ile Ser Ile Ala Asp Pro
   225                 230                 235                 240
   Phe Phe Arg Ser Ala Gln Pro Trp Ile Ala Ala Leu Ala Gly Thr Leu
                   245                 250                 255
   Pro Ile Ser Leu Leu Leu Leu Ala Gly Ala Ser Tyr Phe Leu Trp Arg
               260                 265                 270
   Gln Gln Lys Glu Lys Ile Ala Leu Ser Arg Glu Thr Glu Arg Glu Arg
           275                 280                 285
   Glu Met Lys Glu Met Gly Tyr Ala Ala Thr Glu Gln Glu Ile Ser Leu
       290                 295                 300
   Arg Glu Lys Leu Gln Glu Glu Leu Lys Trp Arg Lys Ile Gln Tyr Met
   305                 310                 315                 320
   Ala Arg Gly Glu Lys Ser Leu Ala Tyr His Glu Trp Lys Met Ala Leu
                   325                 330                 335
   Phe Lys Pro Ala Asp Val Ile Leu Asp Pro Asp Thr Ala Asn Ala Ile
               340                 345                 350
   Leu Leu Val Ser Glu Asp Gln Arg Ser Val Gln Arg Ala Glu Glu Pro
           355                 360                 365
   Arg Asp Leu Pro Asp Asn Pro Glu Arg Phe Glu Trp Arg Tyr Cys Val
       370                 375                 380
   Leu Gly Cys Glu Asn Phe Thr Ser Gly Arg His Tyr Trp Glu Val Glu
   385                 390                 395                 400
   Val Gly Asp Arg Lys Glu Trp His Ile Gly Val Cys Ser Lys Asn Val
                   405                 410                 415
   Glu Arg Lys Lys Gly Trp Val Lys Met Thr Pro Glu Asn Gly Tyr Trp
               420                     425                     430
   Thr Met Gly Leu Thr Asp Gly Asn Lys Tyr Arg Ala Leu Thr Glu Pro
           435                     440                     445
   Arg Thr Asn Leu Lys Leu Pro Glu Pro Pro Arg Lys Val Gly Ile Phe
       450                     455                     460
   Leu Asp Tyr Glu Thr Gly Glu Ile Ser Phe Tyr Asn Ala Thr Asp Gly
   465                     470                     475                     480
   Ser His Ile Tyr Thr Phe Pro His Ala Ser Phe Ser Glu Pro Leu Tyr
                   485                     490                     495
   Phe Val Phe Arg Ile Leu Thr Leu Glu Pro Thr Ala Leu Thr Ile Cys
               500                     505                     510
   Pro Ile Pro Lys Glu Val Glu Ser Ser Pro Asp Pro Asp Leu Val Pro
           515                     520                     525
   Asp His Ser Leu Glu Thr Pro Leu Thr Pro Gly Leu Ala Asn Glu Ser
       530                     535                     540
   Gly Glu Pro Gln Ala Glu Val Thr Ser Leu Leu Leu Pro Ala His Pro
   545                     550                     555                     560
   Gly Ala Glu Val Ser Pro Ser Ala Thr Thr Asn Gln Asn His Lys Leu
                   565                     570                     575
   Gln Ala Arg Thr Glu Ala Leu Tyr
               580
<210> 6
   <211> 319
   <212> PRT
   <213> Homo sapien
<400> 6
   Met Lys Met Ala Ser Ser Leu Ala Phe Leu Leu Leu Asn Phe His Val
   1 5 10 15
   Ser Leu Leu Leu Val Gln Leu Leu Thr Pro Cys Ser Ala Gln Phe Ser
   20 25 30
   Val Leu Gly Pro Ser Gly Pro Ile Leu Ala Met Val Gly Glu Asp Ala
   35 40 45
   Asp Leu Pro Cys His Leu Phe Pro Thr Met Ser Ala Glu Thr Met Glu
   50 55 60
   Leu Lys Trp Val Ser Ser Ser Leu Arg Gln Val Val Asn Val Tyr Ala
   65 70 75 80
   Asp Gly Lys Glu Val Glu Asp Arg Gln Ser Ala Pro Tyr Arg Gly Arg
   85 90 95
   Thr Ser Ile Leu Arg Asp Gly Ile Thr Ala Gly Lys Ala Ala Leu Arg
   100 105 110
   Ile His Asn Val Thr Ala Ser Asp Ser Gly Lys Tyr Leu Cys Tyr Phe
   115 120 125
   Gln Asp Gly Asp Phe Tyr Glu Lys Ala Leu Val Glu Leu Lys Val Ala
   130 135 140
   Ala Leu Gly Ser Asn Leu His Val Glu Val Lys Gly Tyr Glu Asp Gly
   145 150 155 160
   Gly Ile His Leu Glu Cys Arg Ser Thr Gly Trp Tyr Pro Gln Pro Gln
   165 170 175
   Ile Gln Trp Ser Asn Ala Lys Gly Glu Asn Ile Pro Ala Val Glu Ala
   180 185 190
   Pro Val Val Ala Asp Gly Val Gly Leu Tyr Glu Val Ala Ala Ser Val
   195 200 205
   Ile Met Arg Gly Gly Ser Gly Glu Gly Val Ser Cys Ile Ile Arg Asn
   210 215 220
   Ser Leu Leu Gly Leu Glu Lys Thr Ala Ser Ile Ser Ile Ala Asp Pro
   225 230 235 240
   Phe Phe Arg Ser Ala Gln Pro Trp Ile Ala Ala Leu Ala Gly Thr Leu
   245 250 255
   Pro Ile Leu Leu Leu Leu Leu Ala Gly Ala Ser Tyr Phe Leu Trp Arg
   260 265 270
   Gln Gln Lys Glu Ile Thr Ala Leu Ser Ser Glu Ile Glu Ser Glu Gln
   275 280 285
   Glu Met Lys Glu Met Gly Tyr Ala Ala Thr Glu Arg Glu Ile Ser Leu
   290 295 300
   Arg Glu Ser Leu Gln Glu Glu Leu Lys Arg Lys Lys Ser Ser Thr
   305 310 315
<210> 7
   <211> 475
   <212> PRT
   <213> Homo sapien
<400> 7
<210> 8
   <211> 465
   <212> PRT
   <213> Homo sapien
<400> 8
   Met Ala Ser Thr Thr Ser Thr Lys Lys Met Met Glu Glu Ala Thr Cys
   1               5                   10                  15
   Ser Ile Cys Leu Ser Leu Met Thr Asn Pro Val Ser Ile Asn Cys Gly
               20                  25                  30
   His Ser Tyr Cys His Leu Cys Ile Thr Asp Phe Phe Lys Asn Pro Ser
           35                  40                  45
   Gln Lys Gln Leu Arg Gln Glu Thr Phe Cys Cys Pro Gln Cys Arg Ala
       50                  55                  60
   Pro Phe His Met Asp Ser Leu Arg Pro Asn Lys Gln Leu Gly Ser Leu
   65                  70                  75                  80
   Ile Glu Ala Leu Lys Glu Thr Asp Gln Glu Met Ser Cys Glu Glu His
                   85                  90                  95
   Gly Glu Gln Phe His Leu Phe Cys Glu Asp Glu Gly Gln Leu Ile Cys
               100                 105                 110
   Trp Arg Cys Glu Arg Ala Pro Gln His Lys Gly His Thr Thr Ala Leu
           115                 120                 125
   Val Glu Asp Val Cys Gln Gly Tyr Lys Glu Lys Leu Gln Lys Ala Val
       130                 135                 140
   Thr Lys Leu Lys Gln Leu Glu Asp Arg Cys Thr Glu Gln Lys Leu Ser
   145                 150                 155                 160
   Thr Ala Met Arg Ile Thr Lys Trp Lys Glu Lys Val Gln Ile Gln Arg
                   165                 170                 175
   Gln Lys Ile Arg Ser Asp Phe Lys Asn Leu Gln Cys Phe Leu His Glu
               180                 185                 190
   Glu Glu Lys Ser Tyr Leu Trp Arg Leu Glu Lys Glu Glu Gln Gln Thr
           195                 200                 205
   Leu Ser Arg Leu Arg Asp Tyr Glu Ala Gly Leu Gly Leu Lys Ser Asn
       210                 215                 220
   Glu Leu Lys Ser His Ile Leu Glu Leu Glu Glu Lys Cys Gln Gly Ser
   225                 230                 235                 240
   Ala Gln Lys Leu Leu Gln Asn Val Asn Asp Thr Leu Ser Arg Ser Trp
                   245                 250                 255
   Ala Val Lys Leu Glu Thr Ser Glu Ala Val Ser Leu Glu Leu His Thr
               260                 265                 270
   Met Cys Asn Val Ser Lys Leu Tyr Phe Asp Val Lys Lys Met Leu Arg
           275                 280                 285
   Ser His Gln Val Ser Val Thr Leu Asp Pro Asp Thr Ala His His Glu
       290                 295                 300
   Leu Ile Leu Ser Glu Asp Arg Arg Gln Val Thr Arg Gly Tyr Thr Gln
   305                 310                 315                 320
   Glu Asn Gln Asp Thr Ser Ser Arg Arg Phe Thr Ala Phe Pro Cys Val
                   325                 330                 335
   Leu Gly Cys Glu Gly Phe Thr Ser Gly Arg Arg Tyr Phe Glu Val Asp
               340                 345                 350
   Val Gly Glu Gly Thr Gly Trp Asp Leu Gly Val Cys Met Glu Asn Val
           355                 360                 365
   Gln Arg Gly Thr Gly Met Lys Gln Glu Pro Gln Ser Gly Phe Trp Thr
       370                     375                     380
   Leu Arg Leu Cys Lys Lys Lys Gly Tyr Val Ala Leu Thr Ser Pro Pro
   385                     390                     395                     400
   Thr Ser Leu His Leu His Glu Gln Pro Leu Leu Val Gly Ile Phe Leu
                   405                     410                     415
   Asp Tyr Glu Ala Gly Val Val Ser Phe Tyr Asn Gly Asn Thr Gly Cys
               420                     425                     430
   His Ile Phe Thr Phe Pro Lys Ala Ser Phe Ser Asp Thr Leu Arg Pro
           435                     440                     445
   Tyr Phe Gln Val Tyr Gln Tyr Ser Pro Leu Phe Leu Pro Pro Pro Gly
       450                     455                     460
   Asp
   465
<210> 9
   <211> 467
   <212> PRT
   <213> Homo sapien
<400> 9
   Met Gln Met Asp Asn Arg Leu Pro Pro Lys Lys Val Pro Gly Phe Cys
   1 5 10 15
   Ser Phe Arg Tyr Gly Leu Ser Phe Leu Val His Cys Cys Asn Val Ile
   20 25 30
   Ile Thr Ala Gln Arg Ala Cys Leu Asn Leu Thr Met Val Val Met Val
   35 40 45
   Asn Ser Thr Asp Pro His Gly Leu Pro Asn Thr Ser Thr Lys Lys Leu
   50 55 60
   Leu Asp Asn Ile Lys Asn Pro Met Tyr Asn Trp Ser Pro Asp Ile Gln
   65 70 75 80
   Gly Ile Ile Leu Ser Ser Thr Ser Tyr Gly Val Ile Ile Ile Gln Val
   85 90 95
   Pro Val Gly Tyr Phe Ser Gly Ile Tyr Ser Thr Lys Lys Met Ile Gly
   100 105 110
   Phe Ala Leu Cys Leu Ser Ser Val Leu Ser Leu Leu Ile Pro Pro Ala
   115 120 125
   Ala Gly Ile Gly Val Ala Trp Val Val Val Cys Arg Ala Val Gln Gly
   130 135 140
   Ala Ala Gln Gly Ile Val Ala Thr Ala Gln Phe Glu Ile Tyr Val Lys
   145 150 155 160
   Trp Ala Pro Pro Leu Glu Arg Gly Arg Leu Thr Ser Met Ser Thr Ser
   165 170 175
   Gly Phe Leu Leu Gly Pro Phe Ile Val Leu Leu Val Thr Gly Val Ile
   180 185 190
   Cys Glu Ser Leu Gly Trp Pro Met Val Phe Tyr Ile Phe Gly Ala Cys
   195 200 205
   Gly Cys Ala Val Cys Leu Leu Trp Phe Val Leu Phe Tyr Asp Asp Pro
   210 215 220
   Lys Asp His Pro Cys Ile Ser Ile Ser Glu Lys Glu Tyr Ile Thr Ser
   225 230 235 240
   Ser Leu Val Gln Gln Val Ser Ser Ser Arg Gln Ser Leu Pro Ile Lys
   245 250 255
   Ala Ile Leu Lys Ser Leu Pro Val Trp Ala Ile Ser Ile Gly Ser Phe
   260 265 270
   Thr Phe Phe Trp Ser His Asn Ile Met Thr Leu Tyr Thr Pro Met Phe
   275 280 285
   Ile Asn Ser Met Leu His Val Asn Ile Lys Glu Asn Gly Phe Leu Ser
   290 295 300
   Ser Leu Pro Tyr Leu Phe Ala Trp Ile Cys Gly Asn Leu Ala Gly Gln
   305 310 315 320
   Leu Ser Asp Phe Phe Leu Thr Arg Asn Ile Leu Ser Val Ile Ala Val
   325 330 335
   Arg Lys Leu Phe Thr Ala Ala Gly Phe Leu Leu Pro Ala Ile Phe Gly
   340 345 350
   Val Cys Leu Pro Tyr Leu Ser Ser Thr Phe Tyr Ser Ile Val Ile Phe
   355 360 365
   Leu Ile Leu Ala Gly Ala Thr Gly Ser Phe Cys Leu Gly Gly Val Phe
   370 375 380
   Ile Asn Gly Leu Asp Ile Ala Pro Arg Tyr Phe Gly Phe Ile Lys Ala
   385 390 395 400
   Cys Ser Thr Leu Thr Gly Met Ile Gly Gly Leu Ile Ala Ser Thr Leu
   405 410 415
   Thr Gly Leu Ile Leu Lys Gln Asp Pro Glu Ser Ala Trp Phe Lys Thr
   420 425 430
   Phe Ile Leu Met Ala Ala Ile Asn Val Thr Gly Leu Ile Phe Tyr Leu
   435 440 445
   Ile Val Ala Thr Ala Glu Ile Gln Asp Trp Ala Lys Glu Lys Gln His
   450 455 460
   Thr Arg Leu
   465
<210> 10
   <211> 436
   <212> PRT
   <213> Homo sapien
<400> 10
   Met Asp Gly Lys Pro Ala Thr Arg Lys Gly Pro Asp Phe Cys Ser Leu
   1               5                   10                  15
   Arg Tyr Gly Leu Ala Leu Ile Met His Phe Ser Asn Phe Thr Met Ile
               20                  25                  30
   Thr Gln Arg Val Ser Leu Ser Ile Ala Ile Ile Ala Met Val Asn Thr
           35                  40                  45
   Thr Gln Gln Gln Gly Leu Ser Asn Ala Ser Thr Glu Gly Pro Val Ala
       50                  55                  60
   Asp Ala Phe Asn Asn Ser Ser Ile Ser Ile Lys Glu Phe Asp Thr Lys
   65                  70                  75                  80
   Ala Ser Val Tyr Gln Trp Ser Pro Glu Thr Gln Gly Ile Ile Phe Ser
                   85                  90                  95
   Ser Ile Asn Tyr Gly Ile Ile Leu Thr Leu Ile Pro Ser Gly Tyr Leu
               100                 105                 110
   Ala Gly Ile Phe Gly Ala Lys Lys Met Leu Gly Ala Gly Leu Leu Ile
           115                 120                 125
   Ser Ser Leu Leu Thr Leu Phe Thr Pro Leu Ala Ala Asp Phe Gly Val
       130                 135                 140
   Ile Leu Val Ile Met Val Arg Thr Val Gln Gly Met Ala Gln Gly Met
   145                 150                 155                 160
   Ala Trp Thr Gly Gln Phe Thr Ile Trp Ala Lys Trp Ala Pro Pro Leu
                   165                 170                 175
   Glu Arg Ser Lys Leu Thr Thr Ile Ala Gly Ser Gly Ser Ala Phe Gly
               180                 185                 190
   Ser Phe Ile Ile Leu Cys Val Gly Gly Leu Ile Ser Gln Ala Leu Ser
           195                 200                 205
   Trp Pro Phe Ile Phe Tyr Ile Phe Gly Ser Thr Gly Cys Val Cys Cys
       210                 215                 220
   Leu Leu Trp Phe Thr Val Ile Tyr Asp Asp Pro Met His His Pro Cys
   225                 230                 235                 240
   Ile Ser Val Arg Glu Lys Glu His Ile Leu Ser Ser Leu Ala Gln Gln
                   245                 250                 255
   Pro Ser Ser Pro Gly Arg Ala Val Pro Ile Lys Ala Met Val Thr Cys
               260                 265                 270
   Leu Pro Leu Trp Ala Ile Phe Leu Gly Phe Phe Ser His Phe Trp Leu
           275                 280                 285
   Cys Thr Ile Ile Leu Thr Tyr Leu Pro Thr Tyr Ile Ser Thr Leu Leu
       290                 295                 300
   His Val Asn Ile Arg Asp Ser Gly Val Leu Ser Ser Leu Pro Phe Ile
   305                         310                                        315                   320
   Ala Ala Ala Ser Cys Thr Ile Leu Gly Gly Gln Leu Ala Asp Phe Leu
                       325                      330                  335
   Leu Ser Arg Asn Leu Leu Arg Leu Ile Thr Val Arg Lys Leu Phe Ser
               340                      345                  350
   Ser Leu Asp Met Gln Val Ser Ser Trp Glu Ser Gln Gly Asp Leu Gly
           355                      360                  365
   Ser Ser Gln Glu Ser Ser Leu Pro Leu Pro Leu Asp Ser Ser Ser Val
       370                      375                  380
   Arg Ile Leu Ser Leu Val Gly Gly Met Ser Phe Ser Cys Leu Leu Gln
   385                      390                  395                  400
   Ser Thr Cys Leu Ala Trp Ser Phe Thr Ser Arg Leu Asp Lys Gln Asn
                       405                  410                  415
   Phe Lys Thr Gly Pro Lys Arg Gly Pro Leu Pro Ala Ser Glu Asp Ile
               420                  425                  430
   Lys Leu Gln Thr
           435
<210> 11
   <211> 401
   <212> PRT
   <213> Homo sapien
<400> 11
   Met Gln Val Asp Glu Thr Leu Ile Pro Arg Lys Gly Pro Ser Leu Cys
   1               5                   10                  15
   Ser Ala Arg Tyr Gly Ile Ala Leu Val Leu His Phe Cys Asn Phe Thr
               20                  25                  30
   Thr Ile Ala Gln Asn Val Ile Met Asn Ile Thr Met Val Ala Met Val
           35                  40                  45
   Asn Ser Thr Ser Pro Gln Ser Gln Leu Asn Asp Ser Ser Glu Val Leu
       50                  55                  60
   Pro Val Asp Ser Phe Gly Gly Leu Ser Lys Ala Pro Lys Ser Leu Pro
   65                  70                  75                  80
   Ala Lys Ser Ser Ile Leu Gly Gly Gln Phe Ala Ile Trp Glu Lys Trp
                   85                  90                  95
   Gly Pro Pro Gln Glu Arg Ser Arg Leu Cys Ser Ile Ala Leu Ser Gly
               100                 105                 110
   Met Leu Leu Gly Cys Phe Thr Ala Ile Leu Ile Gly Gly Phe Ile Ser
           115                 120                 125
   Glu Thr Leu Gly Trp Pro Phe Val Phe Tyr Ile Phe Gly Gly Val Gly
       130                 135                 140
   Cys Val Cys Cys Leu Leu Trp Phe Val Val Ile Tyr Asp Asp Pro Phe
   145                 150                 155                 160
   Ser Tyr Pro Trp Ile Ser Thr Ser Glu Lys Glu Tyr Ile Ile Ser Ser
                   165                 170                 175
   Leu Lys Gln Gln Val Gly Ser Ser Lys Gln Pro Leu Pro Ile Lys Ala
               180                 185                 190
   Met Leu Arg Ser Leu Pro Ile Trp Ser Ile Cys Leu Gly Cys Phe Ser
           195                 200                 205
   His Gln Trp Leu Val Ser Thr Met Val Val Tyr Ile Pro Thr Tyr Ile
       210                 215                 220
   Ser Ser Val Tyr His Val Asn Ile Arg Asp Asn Gly Leu Leu Ser Ala
   225                 230                 235                 240
   Leu Pro Phe Ile Val Ala Trp Val Ile Gly Met Val Gly Gly Tyr Leu
                   245                 250                 255
   Ala Asp Phe Leu Leu Thr Lys Lys Phe Arg Leu Ile Thr Val Arg Lys
               260                 265                 270
   Ile Ala Thr Ile Leu Gly Ser Leu Pro Ser Ser Ala Leu Ile Val Ser
           275                 280                 285
   Leu Pro Tyr Leu Asn Ser Gly Tyr Ile Thr Ala Thr Ala Leu Leu Thr
       290                 295                 300
   Leu Ser Cys Gly Leu Ser Thr Leu Cys Gln Ser Gly Ile Tyr Ile Asn
   305                 310                 315                 320
   Val Leu Asp Ile Ala Pro Arg Tyr Ser Ser Phe Leu Met Gly Ala Ser
                   325                 330                 335
   Arg Gly Phe Ser Ser Ile Ala Pro Val Ile Val Pro Thr Val Ser Gly
               340                 345                 350
   Phe Leu Leu Ser Gln Asp Pro Glu Phe Gly Trp Arg Asn Val Phe Phe
           355                 360                 365
   Leu Leu Phe Ala Val Asn Leu Leu Gly Leu Leu Phe Tyr Leu Ile Phe
       370                 375                 380
   Gly Glu Ala Asp Val Gln Glu Trp Ala Lys Glu Arg Lys Leu Thr Arg
   385                 390                 395                 400
   Leu
<210> 12
   <211> 2882
   <212> DNA
   <213> Homo sapien
<400> 12
   cgacccacgc gtccgaacat ggcgacctag gagaaaggga agaacaattt tttctcctct     60
   tttgggaagg tttgcgtcta gtagtgcctg tgcccctggg cagattggag agaagaggga    120
   cgactggaga atcgtcgaga accagcggag aaaagaaaaa gcaacgttta attctagaag    180
   gcctcctgtc cctgcctgct ctgggtgctc atggaatcag ctgctgccct gcacttctcc    240
   cggccagcct ccctcctcct cctcctcctc agcctgtgtg cactggtctc agcccagttt    300
   attgtcgtgg ggcccactga tcccatcttg gccacggttg gagaaaacac tacgttacgc    360
   tgccatctgt cacccgagaa aaatgctgag gacatggagg tgcggtggtt ccggtctcag    420
   ttctcccccg cagtgtttgt gtataaaggt ggcagagaga gaacagagga gcagatggag    480
   gagtaccgag gaagaaccac ctttgtgagc aaagacatca gcaggggcag cgtggccctg    540
   gtcatacaca acatcacagc ccaggaaaac ggcacctacc gctgttactt ccaagaaggc    600
   aggtcctacg atgaggccat cctgcacctc gtagtggcag gactaggctc taagcccctc    660
   atttcaatga ggggccatga agacgggggc atccggctgg agtgcatatc tagagggtgg    720
   tacccaaagc ccctcacagt gtggagggac ccctacggtg gggttgcgcc tgccctgaaa    780
   gaggtctcca tgcctgatgc agacggcctc ttcatggtca ccacggctgt gatcatcaga    840
   gacaagtctg tgaggaacat gtcctgctct atcaacaaca ccctgctcgg ccagaagaaa    900
   gaaagtgtca tttttattcc agaatccttt atgcccagtg tgtctccctg tgcagtggcc    960
   ctgcctatca ttgtggttat tctgatgata cccattgccg tatgcatcta ttggatcaac   1020
   aaactccaaa aggaaaaaaa gattctgtca ggggaaaagg agtttgaacg ggaaacaaga   1080
   gaaattgctc taaaggaact ggagaaagaa cgtgtgcaaa aagaggaaga acttcaagta   1140
   aaagagaaac ttcaagaaga attgcgatgg agaagaacat tcttacatgc tgttgatgtg   1200
   gtcctggatc cagacaccgc tcatcccgat ctcttcctgt cagaggaccg gagaagtgtg   1260
   agaaggtgcc ccttcaggca cctaggggag agcgtgcctg acaacccaga gagattcgac   1320
   agtcagcctt gtgtcctagg ccgggagagc ttcgcttcag ggaaacatta ctgggaggtg   1380
   gaggtggaaa acgtgattga gtggactgtg ggggtctgta gagacagtgt tgagaggaaa   1440
   ggggaggtcc tgctgattcc tcagaatggc ttctggacct tggagatgca taaagggcaa   1500
   taccgggccg tgtcctcccc tgataggatt ctccctttga aggagtccct ttgccgggtg   1560
   ggcgtcttcc tggactatga agctggagat gtctccttct acaacatgag ggacagatcg   1620
   cacatctaca catgtccccg ttcagccttt tccgtgcctg tgaggccctt cttcaggttg   1680
   gggtgtgagg acagccccat cttcatctgc cctgcactca caggagccaa tggggtcacg   1740
   gtgcctgaag agggcctgac acttcacaga gtggggaccc accagagcct atagaatcaa   1800
   ttccttggtc tcacagccat gtagacaagc cctggtcatc tcagcagcca ccgcacaaca   1860
   cccctggtgg aagacacgcc ctcctcccct ctggtcacac aagagaacat cttccagctg   1920
   cctctttcac acccactaca gacctcagcc ccagttttct cctcctcact aggctgtgtt   1980
   tttagtagtt cctttgcttg taactatggg atgggatcca ggcataggga actagttgtt   2040
   acacagctcc cagccaagaa gaaagtgtga gaagttgatg ggcagcaaac ctgctgttta   2100
   acatcagggt gaccacatta agcccagtat tccagttggc accagaagat atggacttgg   2160
   aatgaggcct acagggttca ccaggatgta agaggagaga ggaatccaca ggaccaccag   2220
   agaggagagg gaaccagata tgcagatcag agatagagga agtggaacca gagagctggg   2280
   agggaccaag gttgtaaggg tggctaagtc ccaccataac agctaagggg acctgggaga   2340
   tgatggctca tttccaccca gccccaggat ttccagagcg cacatccaca ggcctggacc   2400
   tgggatgaag atgaatgaag aacatggatg cacgtggatg tagtttggct caggtgtccc   2460
   tgcagttggc aaggagtcag tactcagtcc ctgagtgtgg ctgaaatttg aggtcctggc   2520
   tgagccaagg agtaatggac cagatctacc tcagtattca agttcagtgg ggacaccagt   2580
   ggcttcaaac ttcctggttt catgatatct tgagacgcct tacaaatgat ggaggattcc   2640
   aaagagtttt tgtttatttg ggttaatatt tgttggtatt tatggcattt gagattgaaa   2700
   ctaagaaatg ttttaattta ttacctttac aacatttatt tacattacat acatacattt   2760
   acaacattta ttaatttata ttaaaatagc atgaataagc caattatagg ttaatataag   2820
   tagaatgttt gtgaaaaata agtatggtat ccaaagcaaa ataaatttta ttgtgaagtg   2880
   tg                                                                  2882
<210> 13
   <211> 2926
   <212> DNA
   <213> Homo sapien
<400> 13
   acgcgtccgc ttcggaatga gagactcaac cataatagaa agaatggaga actattaacc     60
   accattcttc agtgggctgt gattttcaga ggggaatact aagaaatggt tttccatact    120
   ggaacccaaa ggtaaagaca ctcaaggaca gacatttttg gcagagcata gatgaaaatg    180
   gcaagttccc tggctttcct tctgctcaac tttcatgtct ccctcttctt ggtccagctg    240
   ctcactcctt gctcagctca gttttctgtg cttggaccct ctgggcccat cctggccatg    300
   gtgggtgaag acgctgatct gccctgtcac ctgttcccga ccatgagtgc agagaccatg    360
   gagctgaggt gggtgagttc cagcctaagg caggtggtga acgtgtatgc agatggaaag    420
   gaagtggaag acaggcagag tgcaccatat cgagggagaa cttcgattct gcgggatggc    480
   atcactgcag ggaaggctgc tctccgaata cacaacgtca cagcctctga cagtggaaag    540
   tacttgtgtt atttccaaga tggtgacttc tacgaaaaag ccctggtgga gctgaaggtt    600
   gcagcattgg gttctgatct tcacattgaa gtgaagggtt atgaggatgg agggatccat    660
   ctggagtgca ggtccactgg ctggtacccc caaccccaaa taaagtggag cgacaccaag    720
   ggagagaaca tcccggctgt ggaagcacct gtggttgcag atggagtggg cctgtatgca    780
   gtagcagcat ctgtgatcat gagaggcagc tctggtgggg gtgtatcctg catcatcaga    840
   aattccctcc tcggcctgga aaagacagcc agcatatcca tcgcagaccc cttcttcagg    900
   agcgcccagc cctggatcgc ggccctggca gggaccctgc ctatctcgtt gctgcttctc    960
   gcaggagcca gttacttctt gtggagacaa cagaaggaaa aaattgctct gtccagggag   1020
   acagaaagag agcgagagat gaaagaaatg ggatacgctg caacagagca agaaataagc   1080
   ctaagagaga agctccagga ggaactcaag tggaggaaaa tccagtacat ggctcgtgga   1140
   gagaagtctt tggcctatca tgaatggaaa atggccctct tcaaacctgc ggatgtgatt   1200
   ctggatccag acacggcaaa cgccatcctc cttgtttctg aggaccagag gagtgtgcag   1260
   cgtgctgaag agccgcggga tctgccagac aaccctgaga gatttgaatg gcgttactgt   1320
   gtccttggct gtgaaaactt cacatcaggg agacattact gggaggtgga agtgggggac   1380
   agaaaagagt ggcatattgg ggtatgtagt aagaacgtgg agaggaaaaa aggttgggtc   1440
   aaaatgacac cggagaacgg atactggact atgggcctga ctgatgggaa taagtatcgg   1500
   gctctcactg agcccagaac caacctgaaa cttcctgagc ctcctaggaa agtggggatc   1560
   ttcctggact atgagactgg agagatctcg ttctataatg ccacagatgg atctcatatc   1620
   tacacctttc cgcacgcctc tttctctgag cctctatatc ctgttttcag aattttgacc   1680
   ttggagccca ctgccctgac catttgccca ataccaaaag aagtagagag ttcccccgat   1740
   cctgacctag tgcctgatca ttccctggag acaccactga ccccgggctt agctaatgaa   1800
   agtggggagc ctcaggctga agtaacatct ctgcttctcc ctgcccaccc tggagctgag   1860
   gtctcccctt ctgcaacaac caatcagaac cataagctac aggcacgcac tgaagcactt   1920
   tactgatatt cattccatta ttccatatga cagttgtttt gagtttcgta ccaccttatt   1980
   gtccccttat acagataagg aaactggggt gcagaaaggt gaattaactt tacaaagtag   2040
   acatgacaag tgaacagcag agctgggatc taaacagcaa taactaacat taacagagaa   2100
   tttaaaatgt tcttagtgct gtgttataag ctttggtgga tgtcactcct ttaatcctca   2160
   caacaccctg tcgggtagtc atattttgca agtatggaag ctgaggcagg gcaacatgaa   2220
   gtaacttaca taattcatac agtaatttgt gcagttggga gatgttcagc cttagtccct   2280
   ggctaattgc ctgttctttt ccagcctgat tttttttccc acaggaagag cccacatgta   2340
   gccctgaggt ttccttccca ggacagctgc agggtagaga tcattttaag tgcttgtgga   2400
   gttgacatcc ctattgactc tttcccagct gatatcagag acttagaccc agcactcctt   2460
   ggattagctc tgcagagtgt cttggttgag agaataacct catagtacca acatgacatg   2520
   tgacttggaa agagactaga ggccacactt gataaatcat ggggcacaga tatgttccca   2580
   cccaacaaat gtgataagtg attgtgcagc cagagccagc cttccttcaa tcaaggtttc   2640
   caggcagagc aaatacccta gagattctct gtgatatagg aaatttggat caaggaagct   2700
   aaaagaatta cagggatgtt tttaatccca ctatggactc agtctcctgg aaataggtct   2760
   gtccactcct ggtcattggt ggatgttaaa cccatattcc tttcaactgc tgcctgctag   2820
   ggaaaactgc tcctcattat catcactatt attgctcacc actgtatccc ctctacttgg   2880
   caagtggttg tcaagttcta gttgttcaat aaatgtgtta ataatg                  2926
<210> 14
   <211> 1645
   <212> DNA
   <213> Homo sapien
<400> 14
   atttgctttc tctttttcct ttcttccgga tgagaggcta agccataata gaaagaatgg   60
   agaattattg attgaccgtc tttattctgt gggctctgat tctccaatgg gaataccaag  120
   ggatggtttt ccatactgga acccaaaggt aaagacactc aaggacagac atttttggca  180
   gagcatagat gaaaatggca agttccctgg ctttccttct gctcaacttt catgtctccc  240
   tcctcttggt ccagctgctc actccttgct cagctcagtt ttctgtgctt ggaccctctg  300
   ggcccatcct ggccatggtg ggtgaagacg ctgatctgcc ctgtcacctg ttcccgacca  360
   tgagtgcaga gaccatggag ctgaagtggg taagttccag cctaaggcag gtggtgaacg  420
   tgtatgcaga tggaaaggaa gtggaagaca ggcagagtgc accgtatcga gggagaactt  480
   cgattctgcg ggatggcatc actgcaggga aggctgctct ccgaatacac aacgtcacag  540
   cctctgacag tggaaagtac ttgtgttatt tccaagatgg tgacttctat gaaaaagccc  600
   tggtggagct gaaggttgca gcactgggtt ctaatcttca cgtcgaagtg aagggttatg  660
   aggatggagg gatccatctg gagtgcaggt ccaccggctg gtacccccaa ccccaaatac  720
   agtggagcaa cgccaaggga gagaacatcc cagctgtgga agcacctgtg gttgcagatg  780
   gagtgggcct atatgaagta gcagcatctg tgatcatgag aggcggctcc ggggagggtg  840
   tatcctgcat catcagaaat tccctcctcg gcctggaaaa gacagccagc atttccatcg  900
   cagacccctt cttcaggagc gcccagccct ggatcgcagc cctggcaggg accctgccta  960
   tcttgctgct gcttctcgcc ggagccagtt acttcttgtg gagacaacag aaggaaataa 1020
   ctgctctgtc cagtgagata gaaagtgagc aagagatgaa agaaatggga tatgctgcaa 1080
   cagagcggga aataagccta agagagagcc tccaggagga actcaagagg aaaaaatcca 1140
   gtacttgact cgtggagagg agtcttcgtc cgataccaat aagtcagcct gatgctctaa 1200
   tggaaaaatg gccctcttca agcctggtga ggaaatgctt cagatgaggc tccaccttgt 1260
   taaataaatt ggatgtatgg aaaaatagac tgcagaaaag gggaactcat ttagctcacg 1320
   agtggtcgag tgaagattga aaattaacct ctgagggcca gcacagcagc tcatgcctgt 1380
   aatcctagca ctttggaagg ctgaggaggg cggatcacaa ggtcaggaga tcaagaccat 1440
   cctggctaac acggtgaaac cccgtctcta ctaaaaatac aaaaaataaa aaattagccg 1500
   ggcatggtga cgggcacctg tagtcccagc tactcgggag gctgaggcag gagaatggca 1560
   tgaacccgga aggcagagct tgcagtgagc cgagatcacg ccactgcact ccagcctggg 1620
   agacagagcg agactctgtc tcaag                                       1645
<210> 15
   <211> 3416
   <212> DNA
   <213> Homo sapien
<400> 15
   ctgaagcttg catgcctgca ggtcgaccca cgcgtccgcg gacgcgtggg cggacgcgtg   60
   ggtttttcct ttcttccaga aggagattta accatagtag aaagaatgga gaactattaa  120
   ctgccttcct tctgtgggct gtgattttca gaggggaatg ctaagaggtg attttcaatg  180
   ttgggactca aaggtgaaga cactgaagga cagaattttt ggcagaggaa agatcttctt  240
   cggtcaccat acttgagtta gctctaggga agtggaggtt tccatttgga attctatagc  300
   ttcttccagg tcatagtgtc tgccccccac cttccagtat ctcctgatat gcagcatgaa  360
   tgaaaatggc aagtttcctg gccttccttc tgctcaactt tcgtgtctgc ctccttttgc  420
   ttcagctgct catgcctcac tcagctcagt tttctgtgct tggaccctct gggcccatcc  480
   tggccatggt gggtgaagac gctgatctgc cctgtcacct gttcccgacc atgagtgcag  540
   agaccatgga gctgaagtgg gtgagttcca gcctaaggca ggtggtgaac gtgtatgcag  600
   atggaaagga agtggaagac aggcagagtg caccgtatcg agggagaact tcgattctgc  660
   gggatggcat cactgcaggg aaggctgctc tccgaataca caacgtcaca gcctctgaca  720
   gtggaaagta cttgtgttat ttccaagatg gtgacttcta tgaaaaagcc ctggtggagc  780
   tgaaggttgc agcactgggt tctgatcttc acgttgatgt gaagggttac aaggatggag  840
   ggatccatct ggagtgcagg tccactggct ggtaccccca accccaaata cagtggagca  900
   acaacaaggg agagaacatc ccgactgtgg aagcacctgt ggttgcagac ggagtgggcc  960
   tgtatgcagt agcagcatct gtgatcatga gaggcagctc tggggagggt gtatcctgta 1020
   ccatcagaag ttccctcctc ggcctggaaa agacagccag catttccatc gcagacccct 1080
   tcttcaggag cgcccagagg tggatcgccg ccctggcacg gaccctgcct gtcttgctgc 1140
   tgcttcttgg gggagccggt tacttcctgt ggcaacagca ggaggaaaaa aagactcagt 1200
   tcagaaagaa aaagagagag caagagttga gagaaatggc atggagcaca atgaagcaag 1260
   aacaaagcac aagagtgaag ctcctggagg aactcagatg gagaagtatc cagtatgcat 1320
   ctcggggaga gagacattca gcctataatg aatggaaaaa ggccctcttc aagcctgcgg    1380
   atgtgattct ggatccaaaa acagcaaacc ccatcctcct tgtttctgag gaccagagga    1440
   gtgtgcagcg tgccaaggag ccccaggatc tgccagacaa ccctgagaga tttaattggc    1500
   attattgtgt tctcggctgt gagagcttca tatcagggag acattactgg gaggtggagg    1560
   taggggacag gaaagagtgg catatagggg tgtgcagtaa gaatgtgcag agaaaaggct    1620
   gggtcaaaat gacacctgag aatggattct ggactatggg gctgactgat gggaataagt    1680
   atcggactct aactgagccc agaaccaacc tgaaacttcc taagccccct aagaaagtgg    1740
   gggtcttcct ggactatgag actggagata tctcattcta caatgctgtg gatggatcgc    1800
   atattcatac tttcctggac gtctccttct ctgaggctct atatcctgtt ttcagaattt    1860
   tgaccttgga gcccacggcc ctgagtattt gtccagcgtg aaaagaagaa gagagttcct    1920
   ccaattctga ccgagtgctg atcattccct agagacacca gtaaccccgg gcttagctaa    1980
   cgaaagtggg gagcctcagg ctgaagtaac ttttctctgc ttctccctgc ccagctcaga    2040
   gctgagggcc tccccctcca cagcaaccaa tcacaaccat aaagctacaa gcacgcactg    2100
   aagcacttta ctgatactca ttcaattatt catatgacag ttgtttgagt ttggtaccat    2160
   cttattttcc ccttatacag ataaggaaac tggggtgcag aaaagtgaat tgactacaaa    2220
   gtagacatga ctagttaaca acacagctgg gatctaaaca gcaataacta acattaatgg    2280
   agaacttaaa atgctctgag tgctgtgtta tgagctttgg tggatgtcac tcctttaatc    2340
   ctcgcaacac cctgtcgggt agtctcattt agcaagtatg gaagttgagg cagggcaaca    2400
   ttaagcaact tacataactc atgcagtaat ttctgcagtt gggagatgtt cagcttcagt    2460
   ccccggccct atggccgttc ttttccaccc tgtttcttcc cccataggaa gaacccacct    2520
   gtagccctga ggttcttttc ccaggatggc tccaggataa ggatcactgt aggtggttgt    2580
   ggagttgaca cccctgttga ctccttccca gctgattgtc agagccttag acccagcacg    2640
   ccttggatta gctttgcaga gtgtcttggt tgagagaata acctcaccgt acccacatga    2700
   cacgtgattt ggaaagagac tagaggccac acttgataaa tcatggggaa cagatgtgtt    2760
   ccacccaaca aatgtgataa gtgatcatgc agccagagcc agccttcctt caatcaaggt    2820
   ttccaggcag agcaaatacc ctagagattt tctgtgatat aggaaatttg gatgaaggga    2880
   gctagaagaa atacagggat tttttttttt ttttaagatg gagtcttact ctgttgctag    2940
   gctggagtgc agtggtgcga tctcagctcc ctgcaacctc cacctcctgg gttcaaacaa    3000
   ttctcctgcc tcagcctccc gagtactggg aatataggtg cacgccacca cacccaacaa    3060
   atttttgtac ttttagtaca gatgagggtt cactatgttg gccaggatgg tctcgatctc    3120
   ttgacctcat gatccaccca cctcggtctc ccaaagtgct gggattacag gcttgagcca    3180
   ccgggtgacc ggcttacagg gatattttta atcccgttat ggactctgtc tccaggagag    3240
   gggtctatcc acccctgctc attggtggat gttaaaccaa tattcctttc aactgctgcc    3300
   tgctagggaa aaactactcc tcattatcat cattattatt gctctccact gtatcccctc    3360
   tacctggcat gtgcttgtca agttctagtt gttcaataaa tttgttaata atgctg        3416
<210> 16
   <211> 3502
   <212> DNA
   <213> Homo sapien
<400> 16
   agagaacagg tcccagatac cgagtccgca accccaaaca tcgcgattaa taggaggcct 60
   ctggtctctg cctgccctgg gtgctcatgg aaccagctgc tgctctgcac ttctccctgc 120
   cagcctccct cctcctcctc ctgctcctcc tccttctcag cctgtgtgca ctggtctcag 180
   cccagtttac tgtcgtgggg ccagctaatc ccatcctggc catggtggga gaaaacacta 240
   cattacgctg ccatctgtca cccgagaaaa atgctgagga catggaggtg cggtggttcc 300
   ggtctcagtt ctcccccgca gtgtttgtgt ataagggtgg gagagagaga acagaggagc 360
   agatggagga gtaccgggga agaatcacct ttgtgagcaa agacatcaac aggggcagcg 420
   tggccctggt catacataac gtcacagccc aggagaatgg gatctaccgc tgttacttcc 480
   aagaaggcag gtcctacgat gaggccatcc tacgcctcgt ggtggcaggc cttgggtcta 540
   agcccctcat tgaaatcaag gcccaagagg atgggagcat ctggctggag tgcatatctg 600
   gagggtggta cccagagccc ctcacagtgt ggagggaccc ctacggtgag gttgtgcccg 660
   ccctgaagga ggtttccatc gctgatgctg acggcctctt catggtcacc acagctgtga 720
   tcatcagaga caagtatgtg aggaatgtgt cctgctctgt caacaacacc ctgctcggcc     780
   aggagaagga aactgtcatt tttattccag aatcctttat gcccagcgca tctccctgga     840
   tggtggccct agctgtcatc ctgaccgcat ctccctggat ggtgtccatg actgtcatcc 900
   tggctgtttt catcatcttc atggctgtca gcatctgttg catcaagaaa cttcaaaggg 960
   aaaaaaagat tctgtcaggg gaaaagaaag ttgaacaaga ggaaaaagaa attgcacagc 1020
   aacttcaaga agaattgcga tggagaagaa cattcttaca tgctgctgat gtggtcctgg 1080
   atccagacac cgctcatccc gagctcttcc tgtcagagga ccggagaagt gtgaggcggg 1140
   gcccctacag gcagagagtg cctgacaacc cagagagatt cgacagtcag ccttgtgtcc 1200
   tgggatggga gagcttcgcc tcagggaaac attactggga ggtggaggtg gaaaacgtga 1260
   tggtgtggac tgtgggggtc tgcagacaca gtgttgagag gaaaggggag gtcctgctga 1320
   ttcctcagaa tggcttctgg accctggaga tgtttggaaa ccaataccgg gccctgtcct 1380
   cccctgagag gattctccct ttgaaggagt ccctttgccg ggtgggcgtc ttcctggact 1440
   atgaagctgg agatgtctcc ttctacaaca tgagggacag atcacacatc tacacatgtc 1500
   cccgttcagc ctttactgtg cctgtgaggc ccttcttcag gttagggtct gatgacagcc 1560
   ccatcttcat ctgccctgca ctcacaggag ccagtggggt catggtgcct gaagagggcc 1620
   tgaaacttca cagagtgggg acccaccaga gcctatagaa tcaattcctt ggactcacag 1680
   ccatgcagat aagccctggc catctcagca gccaccgcac aaccccccta atgaaagaca 1740
   cgccctcctc ccctctggtc acgtaagaga acatcttcca gctgcctttt tcacacccac 1800
   tccagccctc tgccccagtt ttctcctcct cactagtctg tggctttagt agttcctttg 1860
   cttgtaatta tgggatggga tccaggcata gggaactagt tgtttcatag ctcccagtca 1920
   aaaagaaagt gagagaagct gttgggcagt gaacctactg tttaaaatca ggataaccac 1980
   attaagccca atatgccagt tggcaccaga tgctgtggac ttggaatgag gccaacaggg 2040
   ttcaccagga tgagagagga gagaggaatc cacaggacca ccagaaggga gagggaacca 2100
   gatatgcaga tcagagatag aggaagtgga accagagagc tgggagggac caaggttgta 2160
   aggatggcta agtcccacca taagagctaa agggtcctgg gagatgatgg ctcatttcca 2220
   cccaacccca ggatttccac agcacacacc cacaggcctg gacctgggat gaagatgaat 2280
   gaagaacatg gactcatgtg gatgtggttt ggctcagatg tccctgcaat aaacaagggg 2340
   tcagtactta gtccctgagt gtggttgagg tttgaggtcc tggtcgagca gggcagtact 2400
   ggaccaggtc tacgtcagca ttcaggttca atggggacac cagtggcttc aaacttcctg 2460
   atctaattat gtttttagac acttagaagt tattgaggac tttaaagagc ttttgtttat 2520
   ttgggttaat atttatgaca tttgacattg aaacaaaaat ttaaaatgtt atcttttaat 2580
   ttatgttaaa atagcattaa taaatcagtt ataggttaat gtagatagga tgttttgtga 2640
   aaaagcaatc tattgtgtcc aaataaaaaa aacaaaaagt gtgacactgg ttaacttttt 2700
   ccagatctca tgtctggctt aataagagat atttgtatta tcatatctgc ctttgtatta 2760
   aacctattgg tatatcatag gtcatgttag ctcaaaaaaa ctttactgca cactactgag 2820
   agaatgagat gaaaaacgat taatgtttca ttattattat tgtgaaaata ttattaacac 2880
   tggggactcc ttaagagtac atcagagttc tctctaggaa tcccaaaacc acattttgaa 2940
   actagaatag tggatcctgg aagttaatcc atgtgctggt taattttaga tgtcaacctg 3000
   gtgtttccag aagagattgg caagtgagtc agtgggaaat tctctccttc tgttggctgg 3060
   gtgcccaata caacaaaaag gcagaggaaa ggcaaattct tctctcctct ggagctgaga 3120
   cactcttctt cttctgccct tggacatcag aactcctggc tctccggcct ttgaacttca 3180
   ggacttgtac caggaggccc tgggttctca ggcctttggc tttggactga gagttacaca 3240
   atcagcttcc ctggttctga ggctttcaga cttaaactga gccatgctac cagcatccca 3300
   gggtctccag cctacagatg agctgttgtg cgatttctta gcctccataa tcacatgagc 3360
   caatctcctt aataaatgcc tgctcataga tctgtatcta catctatatc tgtatgtgca 3420
   tctatatcta tgcctatatc tatatctata tcatattgat tttgtctctc tggagaaccc 3480
   tgactaataa aatgaggcat ct 3502
<210> 17
   <211> 2854
   <212> DNA
   <213> Homo sapien
<400> 17
   gacccacgcg tccgaaaagc tatggcctca accaccagca ccaagaagat gatggaggaa      60
   gccacctgct ccatctgcct gagcctgatg acgaacccag taagcatcaa ctgtggacac     120
   agctactgcc acttgtgtat aacagacttc tttaaaaacc caagccaaaa gcaactgagg     180
   caggagacat tctgctgtcc ccagtgtcgg gctccatttc atatggatag cctccgaccc     240
   aacaagcagc tgggaagcct cattgaagcc ctcaaagaga cggatcaaga aatgtcatgt     300
   gaggaacacg gagagcagtt ccacctgttc tgcgaagacg aggggcagct catctgctgg     360
   cgctgtgagc gggcaccaca gcacaaaggg cacaccacag ctcttgttga agacgtatgc     420
   cagggctaca aggaaaagct ccagaaagct gtgacaaaac tgaagcaact tgaagacaga     480
   tgtacggagc agaagctgtc cacagcaatg cgaataacta aatggaaaga gaaggtacag     540
   attcagagac aaaaaatccg gtctgacttt aagaatctcc agtgtttcct acatgaggaa     600
   gagaagtctt atctctggag gctggagaaa gaagaacaac agactctgag tagactgagg     660
   gactatgagg ctggtctggg gctgaagagc aatgaactca agagccacat cctggaactg     720
   gaggaaaaat gtcagggctc agcccagaaa ttgctgcaga atgtgaatga cactttgagc     780
   aggagttggg ctgtgaagct ggaaacatca gaggctgtct ccttggaact tcatactatg     840
   tgcaatgttt ccaagcttta cttcgatgtg aagaaaatgt taaggagtca tcaagttagt     900
   gtgactctgg atccagatac agctcatcac gaactaattc tctctgagga tcggagacaa     960
   gtgactcgtg gatacaccca ggagaatcag gacacatctt ccaggagatt tactgccttc    1020
   ccctgtgtct tgggttgtga aggcttcacc tcaggaagac gttactttga agtggatgtt    1080
   ggcgaaggaa ccggatggga tttaggagtt tgtatggaaa atgtgcagag gggcactggc    1140
   atgaagcaag agcctcagtc tggattctgg accctcaggc tgtgcaaaaa gaaaggctat    1200
   gtagcactta cttctccccc aacttccctt catctgcatg agcagcccct gcttgtggga    1260
   atttttctgg actatgaggc cggagttgta tccttttata acgggaatac tggctgccac   1320
   atctttactt tcccgaaggc ttccttctct gatactctcc ggccctattt ccaggtttat    1380
   caatattctc ctttgtttct gcctccccca ggtgactaag gaaaagagca gaagctcctt    1440
   ggtttaacca gcacagagaa aataatataa atcccataag ggcagacgtt tggtctgttt    1500
   tcttcgctgt catttcctta gtagttagac tagtgctgag attttagtgg atatataatt    1560
   gatttatgtt gaatatatgg acttagcaac taaaaatacc acagatggtt aacctggact    1620
   ggggcaaagc aagataatag tgatgatcgt atgttgctgt ctccatccgt ctttaatggg    1680
   tcagggcttt gatttccaag ggtcttcagg tgatgagtag gggtacccac aagtcagaag    1740
   gtctgcgttc tcctagtttg tttgctgcca tttgaactca tgtagggaat gaaagaaagc    1800
   tgcaattatc cgccaactgc atttaaaaca aaacaaaaca gaaaaatcaa aataacattg    1860
   actcttccaa ccactgacat gttgtttaat aatctaagcg gcagtcctgg aggctaccag    1920
   acttactgag ttctacctga gaaacagcca agcaaagtgt gagagaaggg ttaagactgg    1980
   cttacaatga gatgcttcaa atgaaaaggg aattatgagt aaaattgaac tttgatgggg    2040
   gattcagttc tggaaaagaa tttggtattt tccagtctgc taggaccaat taccttgaaa    2100
   tattttaaaa tctcagtaaa tagttattgc tgaaatggct gttggcagtt cttattatga    2160
   ttcagagaag agcaaataga ccttaacttc attttgaaaa agaccaaatt accatacccg    2220
   agtgagtaat gacaggacta caactaaaac ataaacaaca ttaatgatga ccataaaaag    2280
   tcacaaaatt gctaaatgtt ataatttaga gttgacataa aaattgatgg ccaggcatgg    2340
   tggctcacgc ctgtaatccc agaactatgt gaggctgagg caggtggatc acttgaggtc    2400
   aggagttcaa caccagcctg gccaacatgg tgaaaccctg tctctactaa aaatacaaaa    2460
   attagccggg catggtggta ggggcctgta acccagctac tcgtgaggcc aaggcaggag    2520
   aattgcttga gcctgcagca gctgcagtaa gccaagatca tgctgtgcct caaggaaaaa    2580
   aaaaattaat gtttactgat atttgttgaa gtcctacaac atcacctctg agaataggag    2640
   aaatgaagca acagttgtgt ctagatgtca gaggcatggc tgggcctcca tctctgccta    2700
   agggagatat aaaagagttc aaactattgc ccatgttccc cagggtcaga agttctaatt    2760
   atgatgatag aggctgggtt gtaagtagta agtgaagggt agcagaatat gccatctttg    2820
   gcataagaag tattttgagt tgaagacaat tgag                                2854
<210> 18
   <211> 2266
   <212> DNA
   <213> Homo sapien
<400> 18
   ggacagaaaa ctccctcctt ttccaagtta gccttatagt ctagggctta aaatactggt     60
   ttaatggtga aggtaagtgc ttttcttctt tttgggtaga aggattatta ctaacttacc    120
   aaaggtccat taaggggagg gaacagtttt aggagaagtc agagaaaaga cattaacagc    180
   aacataagga tctccatctg gtaatattgc ctaattccaa aatgaagaga ctctctgaaa    240
   aagataactg attcaatgaa gaccctaggg caaggcttga gaagccactg gtaccaatgg    300
   acactgtgga caatggtcat ttctccaagg acgctataaa agactgtcgt agtaaaagag    360
   attcagggca cagggaaact ccaccacaaa gcgtggtacc atttcccaca gaagctaaat    420
   ggacgggaag cctgccacca ggaaaggtcc agatttctgt tcattacgct atgggctggc    480
   tcttatcatg cacttctcaa acttcaccat gataacgcag cgtgtgagtc tgagcattgc    540
   gatcatcgcc atggtgaaca ccactcagca gcaaggtcta tctaatgcct ccactgaggg    600
   gcctgttgca gatgccttca ataactccag catatccatc aaggaatttg atacaaaggc    660
   ctctgtgtat caatggagcc cagaaactca gggtatcatc tttagctcca tcaactatgg    720
   gataatactg actctgatcc caagtggata tttagcaggg atatttggag caaaaaaaat    780
   gcttggtgct ggtttgctga tctcttccct tctcaccctc tttacaccac tggctgctga    840
   cttcggagtg attttggtca tcatggttcg gacagtccag ggcatggccc agggaatggc    900
   atggacaggt cagtttacta tttgggcaaa gtgggctcct ccacttgaac gaagcaagct    960
   caccaccatt gcaggatcag ggtcagcatt tggatccttc atcatcctct gtgtgggggg   1020
   actaatctca caggccttga gctggccttt tatcttctac atctttggta gcactggctg   1080
   tgtctgctgt ctcctatggt tcacagtgat ttatgatgac cccatgcatc acccgtgcat   1140
   aagtgttagg gaaaaggagc acatcctgtc ctcactggct caacagccca gttctcctgg   1200
   acgagctgtc cccataaagg cgatggtcac atgcctacca ctttgggcca ttttcctggg   1260
   ttttttcagc catttctggt tatgcaccat catcctaaca tacctaccaa cgtatatcag   1320
   tactctgctc catgttaaca tcagagatag tggagttctg tcctccctgc cttttattgc   1380
   tgctgcaagc tgtacaattt taggaggtca gctggcagat ttccttttgt ccaggaatct   1440
   tctcagattg atcactgtgc gaaagctctt ttcatctctt gatatgcaag tttcctcatg   1500
   ggaatctcaa ggggatttgg gctcatcgca ggaatcatct cttccactgc cactggattc   1560
   ctcatcagtc aggattttga gtctggttgg aggaatgtct ttttcctgtc tgctgcagtc   1620
   aacatgtttg gcctggtctt ttacctcacg tttggacaag cagaacttca agactgggcc   1680
   aaagagagga cccttacccg cctctgagga cataaagtta caaacttaaa tgtggtactg   1740
   agcatgaact ttttaaacat tttttacttc tctccatatt cctgaccata gactcagcag   1800
   ttcttaactc tggctgtgtg ttagtcttcc ctggggagcc tttataagac actgatactt   1860
   gggacccact ccagagattc tgaatgaatt ggtctggggt ggaacccaga tactactaat   1920
   ttttagatac tccttagagg tttctagcat gcgcccgggg ttgacaacag ctggacaaac   1980
   ttgaaaagtc aattcatgtg gcctttgaat tttcctcatt ggaaagtact aaataaataa   2040
   aaattcatgt gaaaatgatc actgataaat atcttcatgg tggggcaggt tattggatgc   2100
   agagaagatc tgctcggaat tgtagccata tgttacagat ctcagcaccg atcagaactg   2160
   taaagctata atccccagaa ttaaagtttt tattattttt tatacattgt aaaacataga   2220
   cgtttattta tgtgattaaa ttctattaaa atttacatgc taaaat                  2266
<210> 19
   <211> 1780
   <212> DNA
   <213> Homo sapien
<400> 19
   acgcgtccgc ccacgcgtcc gcccacgcgt ccggtcgggg ccagagcgca ggtgtacctg     60
   gcggccgtgc tggagcacct gaccgccgag atcctggagc tggctggcaa cccggcccgc    120
   gacaagaaga cccgcatcat cctgcgccac ctgtagctgg ccattcgcaa cggcgaggag    180
   cttaacaagc tgctgggcga agtcaccatc gcgcagggcg gtgtcctgcc caacattcag    240
   ggcgtgcttc tgccccagaa gaccaagagc caccacaagg ccaagggtga aaaccattca    300
   ctaggagagg agaaacacaa tggccaccaa gacagagttg agtcccacag caagggagag    360
   caagaacgca caagatatgc aagtggatga gacactgatc cccaggaaag gtccaagttt    420
   atgttctgct cgctatggaa tagccctcgt cttacatttc tgcaatttca caacgatagc    480
   acaaaatgtc atcatgaaca tcaccatggt agccatggtc aacagcacaa gccctcaatc    540
   ccagctcaat gattcctctg aggtgctgcc tgttgactca tttggtggcc taagtaaagc    600
   cccaaagagt cttcctgcaa agtcctcaat acttgggggt cagtttgcaa tttgggaaaa     660
   gtggggccct ccacaagaac gaagcagact ctgcagcatt gctttatcag gaatgttact    720
   gggatgcttt actgccatcc tcataggtgg cttcattagt gaaacccttg ggtggccctt    780
   tgtcttctat atctttggag gtgttggctg tgtctgctgc cttctctggt ttgttgtgat    840
   ttatgatgac cccttttcct atccatggat aagcacctca gaaaaagaat acatcatatc   900
   ctccttgaaa caacaggtcg ggtcttctaa gcagcctctt cccatcaaag ctatgctcag    960
   atctctaccc atttggtcca tatgtttagg ctgtttcagc catcaatggt tagttagcac   1020
   aatggttgta tacataccaa cttacatcag ctctgtgtac catgttaaca tcagagacaa   1080
   tggacttcta tctgcccttc cttttattgt tgcctgggtc ataggcatgg tgggaggcta   1140
   tctggcagat ttccttctaa ccaaaaagtt tagactcatc actgtgagga aaattgccac   1200
   aattttagga agtctcccct cttcagcact cattgtgtct ctgccttacc tcaattccgg   1260
   ctatatcaca gcaactgcct tgctgacgct ctcttgcgga ttaagcacat tgtgtcagtc   1320
   agggatttat atcaatgtct tagatattgc tccaaggtat tccagttttc tcatgggagc   1380
   atcaagagga ttttcgagca tagcacctgt cattgtaccc actgtcagcg gatttcttct   1440
   tagtcaggac cctgagtttg ggtggaggaa tgtcttcttc ttgctgtttg ccgttaacct   1500
   gttaggacta ctcttctacc tcatatttgg agaagcagat gtccaagaat gggctaaaga   1560
   gagaaaactc actcgtttat gaagttatcc caccttggat ggaaaagtca ttaggcaccg   1620
   tattgcataa aatagaaggc ttccgtgatg aaaataccag tgaaaagatt tttttttcct   1680
   gtggctcttt tcaattatga gatcagttca ttattttatt cagacttttt tttgagagaa   1740
   atgtaagatg aataaaaatt caaataaaat gataactaag                          1780
<210> 20
   <211> 235033
   <212> DNA
   <213> Homo sapien
<400> 20
   cacacacaca cacacacaca cacacacaca cacacaaatg aggtatataa agggtctcct     60
   aaaatgtcat ctgatatttg ttatttcata ttctcagatt tttaatccat ttaggtaggt    120
   ctattttaga tagccttgtc tgaaacagag ctgggacctg atgagtgaaa atgagctcac    180
   cagaagaaaa atcaaacagg catttcagag attgaggcca agaagttaaa tgtcttaaat    240
   gggcagagct tagctgcttg atgtgaaaag agaccagcgt ggctggaaca gcaaaggaga    300
   acagcagaag aggtgaacag aggccagaga tggtcactga gtgggccctt aagtcatggt    360
   aaggagtatg gagaatgaat tattgcatgt attgaatatg taggtgacgt gactcacaga    420
   tactttggat ttgtagagat gaaggaaatg tagcaagtga cactcttaga atgttgattt 480
   gagtaaatgg tagtgtcagt tattgaactg gggagaactg gaagggataa caggcttaag 540
   gagcacgttt attcctgtgt cttggaagtg tttagggtga aagacctatt agagttctaa 600
   atggagatgt caagtgaaaa tgtggctaca cacatttgca tttcagaaaa aaggtcaggc 660
   tggagatgta aaattggaag tttactgcat atagatagtc tttggaaccg tagtattgat 720
   gaagccatta atgagacaga acaaagacta gggaccagag ccaagctcca agtttctaaa 780
   atttagagga tagtatagtc tggtcatttt gaggtgaata cttaataaca gaacaatttg 840
   ttgaagtgta aatttagagc cctacacttt tagctctgac tattaacgaa tacaggaaag 900
   aatggatatg gttatctgcc tggtgtctgt gaaataattt aagccaggaa gagatcctca 960
   ccagaaactg actatgctgg caacttggat cttagatttc cagcctgcag aattgttaga 1020
   aaataaatgt ctatcgttta agccaccagt ctgtagtatt ttgttatggc agtccaagct 1080
   gactaagttt tggtacccag gcgtgggatg ctgcaacaac aaatacctaa acatggggaa 1140
   gtggctttgg aaattggtga tgggtaaagg ctggaagagt ttgaggttca tactagaaaa 1200
   agccaattgt gaagggacta ttgaaagaaa tatggacatt aaaggcaatt ctggcaaagg 1260
   ctcagaaagg aagagagctg gacagaaagc ttccatttcc atagaaactt agatttataa 1320
   cgatcatgga tagaatatta aatatgctgg ttaaaatatg gactttaggc caggcgtggt 1380
   ggctcacgcc tgtaatctca gcactttggg aggctgaggg cacagatcac gaggtcggga 1440
   gtttgagacc agcctggcca atatggcgaa accctgtctc tactaaaaat acaaaaatta 1500
   gctgggcatg gtgatgtgct tctgtggtcc cagctactcg ggaggctgag gctgaagaat 1560
   cgcttaaacc cggggggtgg aggttgcagt gacccaagat cacaccactg cactccagcc 1620
   tgggatacag agcaggactc cactcccccc gccacacaca cacaaaaaat atatatatat 1680
   ggacattaaa gtcaactctt gtgaggtctc agatgaaaat gagggacagg ttattggaaa 1740
   ctgtagaaat cactgttctt gttacaatgt gtcaagaact tggctgaatt acgctgtagt 1800
   gtttactgga aagaacttat aagcagtaaa actggatatt taccagaaga gatgtctaag 1860
   caaagtattg aaggtgtgat ttaggtcctc cttactgctt aaagtgaaat gtgagaggaa 1920
   agagccgaaa taaagaagga atttttaagc aaaacacaat cagaacttgg agatttggga 1980
   tagatttctc aatctatatt gtaaaaattg agaaagtttt tcttgaagag gtatggttga 2040
   acaatgtttt ctttttcttt ttttttcttg gttttatttt tatttttatg ttttttgaga 2100
   cagggtctgg ctatgtcatc caggctggag tgcagtggca caatctcagt tcagtgcaac 2160
   ctttgccttc aggctcaagc aatcctccca cctcagcctc ctaagtagct gggactacat 2220
   gtatgcacca ccacaccctg gctaattttt tgttgttgtt tatagagatg gggttttgac 2280
   atgttgccta ggctggtctc taactcctga gctcaagtga tctgccctcc tcagtctccc 2340
   aaagtgttgg gattacaggc gtgaaacact gagcctagcc tgaacaacca tttgataaag 2400
   agataatggg tgtgacccaa ggatttaatc agccatctca gcagaagcca ggaagagaga 2460
   tgggattatt ccagcagaga cactgccaat ttaaactaac gtaggcagag aaaacagaaa 2520
   ggaacaaagg aaggttgtcg actttttgaa ttctatagaa caggatcata gagctacctg 2580
   gctgtcaatg tgtactattc tttaagaaaa ggaaagactg acccaccaaa ggcaacttac 2640
   aagatcacta gggctgactc ttttgttttt tcttgaggca gtctcactgt cacccaggct 2700
   gtagggcaat ggtgtgatct cagctcactg caatctccac ctcccaggtt caagggattc 2760
   tcttgcctta gactcccaag tagctgggat tacaggctct aaatctgtac cctcccgagt 2820
   agcgctcctg ccaccacttg cccagctaat ttttgtattt ttagtagaga tggggtttca 2880
   ctatgttggc caggctagtt tggaactcct gacctccagt gatccattct cattggcctc 2940
   ccaaagtgct gggattacag gcaggagccg ccagggctgc cactttgatg tcagactcag 3000
   agagtacaga tgggataggg tgggggtggg aacatgtagt caaggctgac tctacctgtt 3060
   tcaaagatgc cctgcagaac tgtgtgggag tctctcacag atggctgcct gggtgggacc 3120
   ccaccaaact gaaagaccga gacttcaggc agggcagatg gagtaggcca actacagagc 3180
   cagaggtgac actgagacac cactgggcct ggaaatcagg gcatcaagcc aaagagggtt 3240
   tttcttaaga cctaacagaa tttgccttgc caggttttgg acttgattag gacacattac 3300
   accttccttc tttcctattt ctccattttc taatgggaat gtctattatg cctgtttcac 3360
   cattgtacct tagaagcatg taacatttct ggtttcacac gttcaaagct ggaaaggaat 3420
   tttgtctctg gatgaatcac acattgagcc tcacccgtaa cctgatttag atgatttttt 3480
   agatgacact ttgaacttta gaattgatgc tagaatgagt taagactttc agggggctgt 3540
   tgggatggaa taattttttt tttttttttg agacggagtc tagctctgtc gcccaggctg 3600
   gagtgcagtg gcaccatctt ggctcactgc aagctctgcc tcccgggttt atgccattct 3660
   catgtctcag cctccagagt agctgggact acaggcgccc gccaccacgc ctggctaatt 3720
   ttttttttat tttagtagag atggggtttc accgtgttag ccagaatggt ctcgatctct 3780
   tgaccttctg atccgcctgc cttggcttcc caaagtgctg ggattacacg tgtgagccac 3840
   catgcccggc tgggatggaa taaatttatc ttgtatggga gaaggacata cattttggca 3900
   ggtcaaggac agaatgttat ggactaaact gtgtccccca aaattcattt attaaaaccc 3960
   taaaccccag tgtgactgca tttggacata gagcctttag gggtacataa aaactaaaga 4020
   tcacaggata gggccctaat cccattgggg ctggtgtcct tacagaagat gagacactta 4080
   gagctctctc tccacgcagg caccaaggaa acaccataca aacacacagt gagatggcag 4140
   ccatctgtta gccaggaaca gattctcacc ataaactatg ttggcacctt gatcttaaac 4200
   ttccaggctc caaaactgtg agaaaatgaa tttctgttcc aagcctctta gatatggaaa 4260
   aaaagattct gttgtttaag ccatccagtc tctggtattt tgttatggca gcctgagtag 4320
   gctaagacaa tgaaggatgt ggtaaaactt tacgtcccaa ccacatacca aagaggctgg 4380
   aatttagcat gctttcttct ttcaactgta ggcaatgtgc acaagttcta aatcctaaga 4440
   catgttggct cctttactct gcccaaacta caactcaaac aaacaactgt aatataataa 4500
   catccaatga agttctgaca tttcttcaac atgagtacag taattcaatg ccagagaatt 4560
   cattttattt tgaaatctac atgccatatt ccaatttctg ttgaagatgc aatggttata 4620
   tttattcttt ttaatataga tttatcagac tgggcgcggt ggctcatacc tgtaatccta 4680
   gcatttgaga ggctgaggtg ggcatatcac ctgaggtcag gagtttgaga ccaggctggc 4740
   caacatggtg aaaccctgtc tctactataa atataaaaat tagctgggtg tggtggtgca 4800
   tgcctgtagt cccagttact agggaggctg aggtagaatt gcttgaacct gggagcagga 4860
   ggttgcaatg agtggaaatc gcaccagtac actccagcct ggatgacaga gcaaaataat 4920
   aaataaatac ataaaataga tttatcagtt tatcaataat atagttttct tttctaggtg 4980
   taaatatagg taatgactgt cctttagtac attttctcat gatgctcctc ttacttggtt 5040
   tggtacaata ttaagtattg aaataaaata gagaatcctg tcgctacaca tgagcactta 5100
   ttccatttgc tcatctccaa tatgcacggg aaattctcaa attgctaata atcttgtaac 5160
   acacatgcat tatattcaac aggaatatat aaatttataa ttataattta ggatcaacag 5220
   atgacaaacc tttagaaggt ttgtatttaa ccttaaaata taatttttta aaaattggtt 5280
   ataaaatttc taatactttc ttttttgtga cctcaagggg aaaatataat tcttataaaa 5340
   gttcaaatga tttacagaat acaaaaagtg aatagagatg atgaatgaat taaaggaaag 5400
   gatattgcta catagatttg gaaatttaaa aagggaaatt acgattgttg attttgtgtt 5460
   aaactgatct gctttgttca agatacctta tgtaccaaaa aatgatttta tctcagcctc 5520
   atatctcagt aaattcctga gacaaacttt agtccctggt gcccaggtgc ctttggtaat 5580
   tgggagacct ctaggtttag catcctcatc cactcgcccc aatttaaata gtcctcccca 5640
   gggccattca ggcaagggag atgaaaactt gctcaagagt tggaatccaa ctgaagctac 5700
   cgaaattcat tgctcaatag ataattttcc ctggaagtaa ctagggcttt tgaatataat 5760
   agtgggcatt tcaaagtaga aggtaaagta ttttggagat gaggagacag gacagagcta 5820
   cgaggaatgt cctttgctta gggactaggc tcttagcagt acctcttagg taagaactgg 5880
   ttaactggca ccttctgtgt ttctctgaag ctccctttgc ttagggacta ggctcttagc 5940
   agtacctctt aggtaagaac tggttaactg acaccttcta tgtgtctgaa gctcccagaa 6000
   caaactgcca gtgaaatttg gatttttgga atatagtttc ttttttcttg ttactttttg 6060
   ttttgttgtt tttttttgag agtctcactc tcactgcaac ctccccctcc tatattcaag 6120
   tgattctctt gcctcagcct cccgagtagc tgggactaca ggcgtgcact agcatgccca 6180
   gctaattttt gtatttttta gtagagatgg ggttggtttt tttttgagac ggagtttcac 6240
   tttgtcgccc aggctggagt gcagtggcac gatcttggct cactacaacc tccacctccc 6300
   ggggttcaag tgattcttct gcctcagtct cctgagtagc tgggactaca ggcgcctaca 6360
   ggtgaacacc gccacacctg actaatttgt gtagttttat tagagatggg gtttcgccat 6420
   gttggccagg ctggtctcaa actcctgacc tcaggtgatc tacccacctc agcctcccca 6480
   agtgctggga ttacagatgt gagacaccag atcagcctca gaagacattt tctattggaa 6540
   agagaaaaca ctattagcaa cctattagtc taatatttaa tacttaatgt cttccttagt 6600
   aataaaccaa ctctctacaa caaagtgctt cctggctgcc taagtcattg attcattcag 6660
   ttcaacattt tctcaatgcc caacagccaa gtgtctcttg tatgccaagt tctatgctga 6720
   ttatcagtat ttgaataaga gggggtctac atcttaagta ctgcttaaga tgaaagcctc 6780
   taggttaaca aacttaacac aatgtatcat tcactactaa atagaccgaa tacaaaatct 6840
   tgttattgga gcccagagag aagaattgaa attcaagttt tctctctctc cttttctcac 6900
   tcaccacaat aagtcagttg caccaagtct tgtagctctt tactgagcca tgttttcacg 6960
   tgtccctttg ttttatttgc cacaccctaa ataaaaattg tactggcttt ttttccctgg 7020
   gtttacagta ttaatacatt gtcaagattt acctcttcgt gtagattccc tggggaaaat 7080
   tacctttcct ccttccctta aattcttcag aggttagaaa gccattagta acattctggt 7140
   atgtggacaa agtttaccca ttatgtatgg atgttttact ctttctattt ttctgacaat 7200
   aatctcttaa ggaggtgtgg ttatagaata gtcagctgtt ataagtactg ttttcctggc 7260
   cttacaactt aagttcttta agctgtttct tagtttgctc atctcaaaat tcggaataag 7320
   gataaaacct atctcttaga ttgttggatt aaatgaatta acatactgga agctcatgaa 7380
   atgtgcctgg cacacagtag tgcctaataa accatctctc ttattcagcc tgttttctga 7440
   tttcagaatc tacacttgct gagccaggtt cttttcattt caaggtgagc aaaagcatac 7500
   aaggaagaga tggaggtagg aagagattaa gccctaggcc aaggtcacac accgattggg 7560
   agctggaatc aaaggcaatt tggtcagtga ataaaaagga ttccaaggcc cataaggcaa 7620
   ttctaacctt aggatcgaaa ttctcggaca tacaggaaat gctggggggg gaaaatccgg 7680
   tcttctcagc ccaagagcca tgtgaaacca gaccttcaaa tctgatgatt ctcagcccag 7740
   ctgcccatta gaatcgttgt aatttaaaaa taccctcgga aaattctaat atgtggctat 7800
   caaaggtgat catttgcttt tatgccactt tgttttcacc caaatgggac atccaaccct 7860
   tttcctttga gagtagttgt agggaaagga gggggtggag ggagggaaga gcggaaaagg 7920
   ctggatccgc cctgagccgg tgtcagtatc tgggaagtgg gaggcgcgtc agcagtaaac 7980
   agcttctgct aggattatta tctcctgcca cacactcgga tttgaaggct ccaaacgaaa 8040
   caatgcaaaa cgcttcagtg gagttccaga agcgttagac taaacgactg ggtctgtttg 8100
   gccagtctga gcagctgggc gcagatgcat aggcaagact tagcccgcct agacttttct 8160
   gcccacttaa ttccgatcaa agcagaaacc ggccgggcgc ggtggctcac gcctgtaatc 8220
   ccagcacttt ggtaggcaga ggctggcgga tcacctgagg tcaggagttc gagaccagcc 8280
   cggctaacct ggtgaaactc cgtttctact ggtggcgggc gcttgtaatc ccatctacta 8340
   gggaggctga ggccggagag tcgtctgaac ccgggaggcg gagtttgtat gcagtgagcc 8400
   gagatcgcgc cactgcattc cagcttgggc aacaggagca aaactccgtt tcaaaaaagc 8460
   aagcaaacaa acaaaaaaat gcagaaaccg agatccggaa gaaaacctcg gcgagattca 8520
   cagaatccag gaaaataggt ctctagaaat ttgtccatgg tcccagatct ccatttcttg 8580
   tgggtggggc agctgttacc agatccctag aagcaaaggt ttttttgggg gaccgtgtct 8640
   cactgttgcc caggctggag ggcagtggca cgatctcggc ttactacaac ctccgcctcc 8700
   caggctcaag cgactctcct gcgtcagctt caagagtagc tgggattaca aggtatgtgc 8760
   caccacgccc aacttatttt tttatttatt atttttattt agtagagagg tgtttcacca 8820
   tgttggccag gttagtgtcg aagtcgtgac ctcaggtgat cagccccctc ggcctcccaa 8880
   agtggtagga ttagaggggt gagcagaaag caaaggtttt tgagtggcca caggccccac 8940
   tctatttcct tttctgcctg taatggcaac ctagacgctt gagcttctta aaatacaaga 9000
   gtaagttgca tgtcaggcac cgttctacat tagggacatt agtctgtttt acagacacct 9060
   ttcaactccc tggttaactt ttaggtaata tactctgcac tttagcagga atgggaccta 9120
   taactctcac agaattagga aagtgaggct gcctacagcc taaattgaga aaaaaataga 9180
   cgggggacta gtcggaggac caaacaaggt taccaacacg ttagagtttt gccttcaatt 9240
   tacattttta aagtaatcac aacgaagtgt ttagatcacg aggcatccct gcatgtaaac 9300
   tgttaggcac taactatggt cgatcttaca aagcattaac tagaatattt ctttagagta 9360
   tgatagtacg taactgacct actattacat acaaacagac caacctttag taacagcgct 9420
   ccccaaaaac cgaaaagcag taatacgctt tgctcaaggt tggcataaaa ttaacttacc 9480
   ttagtgcctt ttttccttct acctacaagc agtgaggtta gctcttcctt tgaaacggta 9540
   ggggggctct gaaaagagcc tttgggtttg atagcgtttc cgggagctca gatacctgtc 9600
   aaatcacttg cccttggcct tgtggtgact ctcggtcttc ttaggcagaa gcacggcctg 9660
   gatgttagga aggacgccgc cctgagcaat ggtcacccgg cctagcagtt tgttgagctc 9720
   ctcgtcgttg cggatggcca gctgcaagtg gcgcgggatg atgcgagtct tcttgttgtc 9780
   gcgagccgcg ttgccggcca gctccaggat ctcggcggtc aggtactcta acaccgccgc 9840
   caggtacacc ggcgcgcctg ccccaacccg ctctgcgtag ttgcctttac ggagcaggcg 9900
   gtgcactcgg cccaccggga actggagacc agcgcgagaa gagcgggatt tcgctttggc 9960
   gcgagctttg cctccttgct taccacgtcc agacattgca atcagacaaa aatcaccaaa 10020
   accagcggcc taagctcacg agaaaacaaa caaaatcaag aaatatgtaa aacatggccg 10080
   cttttatagg tagttcctgg ggagtaaatc cgactttttg attggtcggt agcaaatgct 10140
   agtcagatag ccaatagaaa agctgtactt tcatacctca tttgcatagc tctgcccacg 10200
   gatgacaact gtgcagtttg tcttccaatt aactaagagg tactctccat ccctcattag 10260
   cataaaagcc ctataagtag cagaaatccg ctctttactt tcgacacatt tctggtgttt 10320
   taagatgcct gagccagcca agtctgctcc cgccccgaag aagggctcca agaaggcagt 10380
   gaccaaagcg cagaagaaag atggcaagaa gcgcaagcgc agccgcaagg agagttactc 10440
   tgtgtacgtg tacaaggtgc tgaaacaggt ccatcccgac actggcatct cttccaaggc 10500
   catgggcatc atgaattctt tcgttaacga catatttgag cgcatcgcgg gcgaggcttc 10560
   ccgcctggcg cattacaaca agcgctcgac catcacctcc agggagatcc agacggccgt 10620
   gcgcctgctg cttcccggag agctggccaa gcacgccgtg tcggagggca ccaaggccgt 10680
   caccaagtac accagctcca agtaaacatt ccaagtaagc gtcttaacac ctaaccccaa 10740
   aggctctttt aagagccacc cagataccca ctaaaagagc tgtggccaga cgccaaattt 10800
   tatttggcgg cggaggggta ttagaatata ggaactggag aggggtgggg acaagtgttg 10860
   cagcttagag agggacaaag ggtcctgaac ccgaaagaag ccagccatta aaaatggctt 10920
   tggggtcaat tcgttgtgct taaatttaaa atggagacaa gcggccattt tgctaactcg 10980
   gcgttcccgg aagaaaccgc aggctcgctt aggtttcaga cccagctgtc tgtccctgtc 11040
   tacgtcgcca ggatcaacgg ttgccgtaat gtcataattt cgccaccagc ttctagccaa 11100
   taggctgtcc tgtcatttta aatattaacc aatcgaggga aagctgtttt gagactctga 11160
   tttacatagc ggaccggagt gggaacctgg gcagtaactg cctaaggaag gactccccct 11220
   ctgttttcgt ggcgcacacc ttcgtagtat actgaagggt gtgtctcctg ggtttccaac 11280
   tgccccggta atagtctttt aacctaatat gcgtcagttt tgataacaac actaaggcag 11340
   tacagaacta aagatgtaag cactgcgcca gatgttgctt catacatctt attctattca 11400
   actggtttat tcaagattca aatcaaatca aattttgctt gaatcccagt gctcagtcag 11460
   ccataaatgg tgtgttgcct gattgaaact taaaatctcc gtagggggct tgtaacatgc 11520
   agacaagttt gaaagttgct ttaggagaag ccaactctta actgctgggt aaattgacaa 11580
   gccttcgaac actgaactga aggccagtaa ggactaggcg ctgggtgggg gagaatgaag 11640
   aggagacgtc attaaactta gcacatacac tgtatctcct agaggactct cccttcctag 11700
   acaactgcag gccgctttgt ggcctgggaa attccacatt cccttaagta ttttactcat 11760
   ggtcttttcc aggtaaagat tttaagatga agggttagac gtagtctacc tatcttttta 11820
   ttcaagtcta gaacacgttt ttagcaccta gaagtttgct ttctccatta aaaaccggga 11880
   atatacaata aataaaatta gtgttaaagc agatttttac aaacttaaat accatgtaat 11940
   ttaggttaca gttatttaac ataaggactg tgtgatctta aatctgcaat ttctttcaca 12000
   cctgggaaat aaactaaggc ctgtctttgg tgccagacaa ggccttatac ttgaacactg 12060
   ctgtgcaatc acaggctgcc ttgcctagat aacttatctg agaaattctg atgagaaatg 12120
   aaatttccag agtccctcac aagtaaattt ttttttcttt tttttttttt tttttgagac 12180
   gaagtttctc tcttgtttcc caggctggag tgcaatggcg cgatcttggc tcacagcaac 12240
   ctccgcctcc cgggttcaag ccattctcct gcctcagcct ccggagtagc tgggattaca 12300
   ggcatgcgcc acgacaccct ggctaatttt gtatttttag tagagacgag gtttctccat 12360
   gtcggtcagg ctggtctcga actccggaca tcaggtgatc tgcccgcctt ggcctcccaa 12420
   agtcctggat tacaggcttg agccaccgcg ccgggcctaa atggtttttt ttttttctat 12480
   gcctctaatg gacctggtca cttattccca ttcagactga ccgctctcct acctgccaac 12540
   taactaatca gtgtaaccaa aatctgcaaa caaaattcag tattctttcc ccgccttttc 12600
   ccctttctct tacatagatt atgtttttgc ctgtgttaga tgaaataatt ctattgcttg 12660
   ttctctcttc tgtacaagta cccagtaagc aaattattaa cttcttggtc atttatttct 12720
   gaattttcca ccaagacagt gtttatgtga gtcatacaat aagaaccaac agaaatgtgt 12780
   gtcttggaaa caggttgtct atccctggac cctttgagtt ttctgttcac tttcctttgg 12840
   cttttgcatg ctaaaagttt atcgtccgcg tttgtttgtt ttggttattc taattggact 12900
   tggctgattg gttgcatatt ggtggcagta gtagaatttg aattctggtt ttctggtcac 12960
   atcattaagt gattagtcag tggagaggac aggaaatctg gtttatttat taaccttttt 13020
   ttggggtgtt tttgtttgaa gatgttgata ttctctgtga ggacacaggg ttagagttgg 13080
   tgtttttctt tctgacttta catgggattt gatgttttgt gcttgtatgc ctctttccac 13140
   cttccaaaac ttgtcttttt tgagtccaaa tagttgtcga tatctgcaaa accagtattc 13200
   ctgtgttaag atgatatgaa tataaaatgg ctgccctgtt ataacttttg actttaagaa 13260
   agtgttagga ctaacaggag acaaaaagga aatcaaggaa accgaatgtc tggtctcaat 13320
   aactgctatg gcagaggctc tacagcttat tattaatttt agtaatttca cattattgcc 13380
   ccttcacgtt ctttaagtaa ggttagagga cagaagaaac ataatgttgt tacaaattgg 13440
   actattgagt cagggaaaaa aaagagtgct ttcaatatct gaataaaaca aagatttaat 13500
   attttctaaa ccttaacgag tttattgtaa gggatgtgat gctggaaact aggaaactag 13560
   aattttcttc taaactgaga atcagaatta ttcatattct cagcagtggt gccacctgag 13620
   ggacttctga tcttaattac atacttttat ttctttaact gatcaacatg ctaaatagat 13680
   aacctatggc tctgttttta cccactttaa attctgttct attagcacgg ttagctttcc 13740
   taattggcaa taagattgag actatctttt tttttttttt gagacagaat tttgctctgt 13800
   ggcccaggct ggggtgcagt ggcacaatct cggctcactg caacctctgc ctccagggtt 13860
   ctagcaattt tcctgcctca gcctccccag tagctgggat tacaggtgca ccaccacgcc 13920
   tggctaattt gtgcattttt agtagagatg gggtttcgcc atgttggcca aactggtctc 13980
   gaactcaggt gatccacctc ggcctcccaa agtgatgaga ttacaggcgt gagccaccgt 14040
   gcccagaaaa gactatctta ttttatgaat ttaaataatt gtgaaattat ccacttaagg 14100
   gaattaataa attataatgt aatcttaaat tttagttggc ttacataaag acttaaaata 14160
   catcaattta aataaaaact catttgtcta aaaaaaaatc aaaaattttc cttgtgcttt 14220
   aaatgtgcta cctctttaag ttctaattaa gagaaaaaaa gtttaactgt gagtttcatt 14280
   agtggtctta gttaacagct taaagtattt tgtaaaaaaa atacttcaca atttttaaat 14340
   aacttaaaaa tattaatacc tcttttatta ggttttttta ataaggaaaa tatataatac 14400
   atctaatcaa gatttttttt ggacaaattg gcttaataat ttcattttaa aaatggcttc 14460
   tttattctta tactgtaaaa ataatattag cagaatatta tagtatacac aagtttaggg 14520
   ttcatattct aaaaaacaaa aacaaaagct aatttaactt gcatttacta aatttcttcc 14580
   actagttgta ctggttacat gagttaacat cactttattt attattctaa aattgtaaat 14640
   tattcattga accaaattaa atgataatag ataatgtcat ttttaaaaat ggaattaaat 14700
   tttatgttac taattataag gattcaatgt gtgagcttaa gtactgagtt cacagtgtat 14760
   gataacttta agaatttagg tgaatattat taaattgagt aaattaattc tcaatctttg 14820
   gatacctgga caatttctaa attggagggt acaaaataca aatcacaaga aacagtgtag 14880
   ttttatgcaa ataacatttt tacacagttt agaataacca ttgataaaca gataagagaa 14940
   catatgattg ccttagaata gatactgttg ctttcgccac tttagatttg taaatcacgt 15000
   actgtatacg tgtgggcgta gaggaccatg caggttttgg atgactgcct ctgttttcgt 15060
   catgcctatg cgggaacaca attgcctgct ttgtttaagg gctatggtta atccaaacag 15120
   ctctgactct atcaagtact atagctacag agaaacacaa gtaagcattc gagataatga 15180
   ctaccttgag cctttactta tttaaaaagt tgttactgtt tgttaatgtg gtacattcaa 15240
   tttactatgg attgtcactc taaaataaga cttcaatctt tttcttattt ttatatagcc 15300
   atgatttata ttcatatctt aatgtaataa ccaatcttct ctgacaacat tataacaatg 15360
   ctggaacctc cattttcagt acttcaaaca acaaatactg cttttatact tcagagcaga 15420
   tggatatgtg cttcccagtg taaacacatt tggaatctca ctgagaaata cactatcact 15480
   aaaaatacag ttctgagatt cattaaaaga cctccagaat tctggaagta ggaagtttcc 15540
   tcttcaaagt ctacagagga agatgaggtc tgaaatagac agcttcttcc ttcttttacc   15600
   tgtggtatta ttctgttttg tccttttctc cattatctgt ctttccagtg atgaaatttt   15660
   gatctggccc tcccaagtat taaaaaacaa gcaaataaac aaatctcagt tatattttac   15720
   taagatattg gcatgctaac tttttgcagg tttgtaacaa ggacctttat aacttgacta   15780
   aaagttccta aataagaata tttactagaa aatttatttc tgcctgtggc ccacatttga   15840
   gtcaaaataa tcaattagga aaaatgaact tgtttaacta aagttgacca aactgatctt   15900
   tgaccaaact gatctttgag acctattcat ctaagacaag ccaattaaat tcttggagac   15960
   aatttgtact ttaaggaatt cttataatat ttgtaattac cctcataact tttttttttg   16020
   ccctacttct gtgcttctct aatatgcaga ttattaaatg ttgttacaaa gccattgtca   16080
   aaaaaacaaa aaacaaaaaa ctaaacaaac tcacatggtt agacttgctc ctttatgaga   16140
   tatttttacc aaaaatggag gagttgaaaa actctggtgc cagaaatcgt gaagacatgg   16200
   cctacctaac atggaaatgt tggttgtcag tggaaaatac tacacagaga tagccatagt   16260
   gctgcacagc caatcttaag tgtttctaga gaatcactaa ttgtttctag agaatcacta   16320
   attgttttct tttaacattc ttggtttata caagaagaga gtatccatac taaactcttt   16380
   tctactgaaa ataatgtgca aacataacat cctattccta gacagtttgt agtttttttc   16440
   tcccatttct attttataaa tcatcttttt aaaatacttt gttgagtgaa atcagtccat   16500
   tgcttgatat accttgagca caagtaaata gtatgccaaa aattaaatgt ctttcagtca   16560
   cagtttgaca aactcaacta ccctgagcct atagagtggt aataattgcc ctactcataa   16620
   agatggggtg aagattaaat gaaatagcac ctatagaaca ctagttccag acgtggtatc   16680
   atgctagtaa aatggctgca cagcactgct caatgatgac aaaaagtgaa gcttctggag   16740
   acagactcca agtttgactc ccagatcacc acatataaga tgtgggactc tgaggcaggt   16800
   catttaatct ctctgtgcat tagtatcctt ctctatacct ttacagtgat ggtaatagca   16860
   cctaccttct agaagtatgt gaagattaaa gatccttaat gcatataaac cactgtgttt   16920
   actgctgttt gacaaatttt atttataacc atctttacgc tcctaaaagg acttgaagca   16980
   gcttatgact gaagactttg gtaggagttg gccttctata aattataaga atttcataaa   17040
   ttatttgata tgaaaatgcc agttgatcat agtatgttta ccggggtcca acaggttgag   17100
   aaaaaataca ctttttttcc ctgaacatat gaaattagct ctctaggcat attcctaagg   17160
   acttaaagaa tgataactat catttctctt aaatcttcca gatttggaag gatatatata   17220
   ttcagcacat tgacagacaa tcccagtagt cctaaattaa aagacattaa aaattagtga   17280
   aacttttcct acctttagcc tgtgtaatcc tggatgacca agcataaaat taaattgagt   17340
   agagtatacc actgtaacat ttcctgaaag gtattctagg ctctgagtaa tttctttggg   17400
   gtctgaagat cagtttgaca tatcctcaag tatcatgagt tcattataat taagaaaaag   17460
   agagtaaatc tggagaatga gccactttct tactactcct tgacctcagt tctttttttc   17520
   agagacaggg tctcactttg ttgcccaggc tgccaggctg gagtgtagtg gcgcaatcgc   17580
   atctcattgt aacctccacc ttctgggctg aagccatcct cctgcctcag catcctgagt   17640
   atctggaacc acagcaggtg cacaccacca tgccaagcta attttttaaa aagttttttg   17700
   tagagatggg gtcttactat gttgcccagg ctggtctcaa actcctgggc ttaagtgatc   17760
   ctcctgcctc agcctcccaa attgttggga ttactagtgt gagtcactgt accccgcccc   17820
   acttcagttc tgaggaggaa aaaatatgta ataataatgg gactttggtt tgctgattta   17880
   aagattcatg taaccttatc atccaatgcg caatttgtag aataattaat agagacatct   17940
   ggtctcatgt ttctacagtt gctcatgcct tgatagtaga tctccttgct gctggctcag   18000
   aagggtaaaa gagcagaaat gatggggctt ctctcattct atgaggaaat agacctatgt   18060
   agaggaggct acctgtggta aaaccttatc ctcatcactt aaaattctag gcttattctc   18120
   tgaccatatc aagttttcaa atggtaaaag aattggattc aagagaaata tgaataaact   18180
   tttgttttca cttttctccc tcctctcccc ccattctccc ttcctttatt ttcttgtcct   18240
   tagttttctt ttcacttttt tgtctactat tatttgccca aactcaactg taggctagaa   18300
   caaaaaaaaa ttgaaaatta aaatgtgccc cttttgttgt tagacttgct taaacaattg   18360
   gggtaatgaa ccttggacac tagattttaa aacacacaca tttgagcttc agtgcactga   18420
   aataaatata tttttaacaa ttaaaaaata aaattgcatg tttaaaaaat ctgcagagaa   18480
   caatacacgt tgtgagatct tgaatggaag gaaaactgct agcctcaaga gtggatcaaa   18540
   gatgctcagc aggcaacaga gtaagagcat gttggagggt ttagagagtg tgctcagggt   18600
   tctaggctct aaaaatcaga cagtccccac ggcctggcct tcgtcgctgt atcttcttta   18660
   tgaaaaacac taagtctttt tcctcactgg ataaattttt atccttcaag tttagatcaa   18720
   atggaacttt aggacactga ctaggttaca ttcatctttt aagagcgtac agacattcaa   18780
   gggctagagg atgtgggttt actgcacagg ctcattatcc aacagctgtg ctacctggga   18840
   aacttaacct ctctgtgcct taatttcctc atctataacg cagggagaat gacagtaggt   18900
   atctcataag gttgttggaa caactaaatg cattggtatc tattgtgtaa agtgcttaaa   18960
   acactgcctg gcacagagca aacatccagt gaactttagc catcatcatt atcattgttc   19020
   tcagagtcaa atacaatatc tcatatctga taaattacag aagtgaatca atcactctct   19080
   ctcttttctc cagggggaga caacagcttt tagacatatc ttttccaaca gtcgtcactg   19140
   ctggacactg tttcatcttg caaataaacc aatgaaaatg agtgatccta gaagaagata   19200
   aatggaggta ttttgaacaa tcaaagaagg acaaatgaac acctggctga gaaaaattag   19260
   ctcttttttc tatgcataaa actattaaaa tattcttcat agaaatttat gacacaggaa   19320
   acataaagac aaaattaaaa taactcctag tatctcctat tctttttata tgtatattat 19380
   atatactcat attcatatat acatatatct cacatcatgt atcatatata aaataaattt 19440
   aggtgtcatg atatatattt agataaatat acttagaaac ttttttatgg atgtataatt 19500
   tatggatata ttgataatta tgtatttgtt attgactact tcaattgatt cccattttta 19560
   tgcattatat tatagattat atagctcaca catctttgta cataaatctt tgttcaaata 19620
   ttatttccta aggatagact tcatgaagtg gaaatactaa atcaaaagtg aaaaacattt 19680
   tctaaggttc ttaacatata cattgccaaa ttgctattca ggatcatacc aatttataat 19740
   cccaaaataa tatggaaatt cctgttttat agcactcata tttacaataa attttaaaaa 19800
   tcactgttaa cctaatagtc cttcaaaaga aaaaaaaatt gaaattacat tattttaatg 19860
   actctattag tgagggtcat tcttcccatg tttcttgtta gccatgaccc tataagaaat 19920
   aaactgcact gcaaaatgat aaacatgaca tcaatcatta catgggaagg cactatataa 19980
   agaataatac cttaggttaa ggccacataa atatttatca ggtgcctttt ctgcggagga 20040
   ctctgaaggg atactaaact gcatttagct gcatgcaact gaaactactt ttacctacat 20100
   tgtctcttat aaacattata actactcttt gagaaagtgt ttactatgga ctgaattgtc 20160
   tccccatccc cccaaattca tatattgaag ccataaaccc caatatgact ctattcctag 20220
   acaggactta taagaggtaa ttaaggttaa atgaggtcat taggatgggt tcctaactgg 20280
   ataggattgg tggccttata agaagaggaa gattctgcac ttggtcttcc aaattaaata 20340
   atttatttaa aagaaaaaaa aaaaagagga agagagggag ctctgcacat atactgagga 20400
   aaggctatgt gagctctcac agtgagaagg tagcactcta caagccagca agagagccct 20460
   caacagaatc cagccatgct ataccctgct ctgagacttc cagcctccag aactgtgata 20520
   aaattttgtt gtttaaacca cacaatctat ggtatttttt tatggcagcc caagccaaca 20580
   aagacagcat cattgctgtc acttacagac aagaaaacta agactaggag agagaaaagt 20640
   taaacttgtc caaggtcaca aaagccagaa acaagtgagg tgagaagttg accttgttct 20700
   cctcaatcca aggccaggac tcctccactc cacatgtaga tagccacctc acagtcaaca 20760
   gccaaatgtc cacaccccag agtcagcatt agaccaagat gtcttaccag gagacaaatg 20820
   cctcatcttg aataaatatg atctaacaac ttacccatgt aaaacattga atctcatgag 20880
   aaacaaaaat gcaaagtatg tagaaaacta tgtttaccac ttaactgaca gtgataaaaa 20940
   gcttaatgat atccttatag tcttggaggg gtttgtatat gtggtgaaac aggtgctcac 21000
   gcactgctga tagactgtaa attggtccta gagagaaaaa taaataaact ggaaggagat 21060
   atgctgtatg tttacttttt ttatggaaac atatgatata cctggaaatt cgattgacca 21120
   tgcatctatt tcttcaatgg gtatgcacag ttgagctgtt cccatgcacc aggcactgta 21180
   atgggacaac tgcacatgac agtcaaaaat ctcagtctca tgaagtcgac atgctcatgg 21240
   agaggtgcta cccactaaac taatatttgt atatcaatta tggatacatt gggccacatt 21300
   tacagaaatt cacttacagt gggttaccag aagggatttt ttttcttgat tggcaagaag 21360
   gctaggctgt tttgttgggg gctggcagga gctgtctagg ctgcccaagt atgcaggtct 21420
   cttctatcat cctgtgttaa ccatcttcca tgtatctttc aacctcatgg tcatctgcag 21480
   catgtctagg ggtcatatct atgttccatg caggaaaaaa gggtaaaggg aaagggaagt 21540
   aggcatgtac cattttaatg cacaccttgg ttttcagaaa atttaagaag aaagactttc 21600
   tgcttttctc tgactattct gtattctgga ttacaacgca acagaaacgt caccttaaat 21660
   tctaatgttt ttctctcctt gctttcaaaa actgactcat taacctccac gtggcttgga 21720
   aaaattattt cagtcatcca gtaatgagct gttcatagaa atgttttgga catcaagtct 21780
   gtgttgttag cattatacat gttaagcatt gaataaaaaa caacatgatg tgggtaaatt 21840
   tctttactta catataagta cttatatact tatagctgaa aagagaggtt gaaatgtcag 21900
   gtggaacaga aataagatta cctagatgtt tctcctatgg gtgattttca gctatgctga 21960
   tctttcttct gggtcaggta ctcccagaac ttcctaatta aatggtggcc ctgatcttag 22020
   ttcctctctc ctcttagaca ttttccagga ctacagaaga tgtgcagttt ataaatgagt 22080
   agcagaaacc tactgaacaa attattcagg ctcatctgaa cagagaggac accttctctg 22140
   ctatactctc tcagtgattt ccctgccttg gggtcaatta ttgtcttgga cattgattta 22200
   agcacataat aattgttgtc attgcttatg tttggatttc atctcccaaa atagatggta 22260
   aattctttag tttagagacc aagtaatact taaaaaaaaa ttttgtgtgt gtgtgtgtgt 22320
   tttttctgtg tctctcagcc ctgtaatagc atcgtactta cacttgttag atttttagag 22380
   acaactttta caaaacatgg aattatctac ataccctttc tacaaaacag acaaattaaa 22440
   tactcagtag ttgaaccaaa aaaagcagtt caaataaaat acttgaaaat gaagaaatca 22500
   tttgaacaga gttaaagtta atcgtaaaat aatgtctgta aaaattattg ccaatcaaat 22560
   ataaagttca aaaatagtgc ttgaaaaagg aagaatcata tgaaaaggga ctactcattt 22620
   taaaaatgtt agatatcagg aaaagccaag aagtgagtat ggtaagagtg ctgtcaagtg 22680
   aaaccctgct aatctcactg aacatgtaaa aatctgtaga tgcctttatt ttattcactc 22740
   acacacatat gtagaaagag aaatatatgg taaacattaa aaaaaccaaa ttagaatgta 22800
   aaattaatac tttaaaaaat gggctgtata cttttcttat caccggagat aagaatttat 22860
   tatttttaaa ataaagttat tttctctgtg actgtttcca tgactttgct acttagaagt 22920
   tagagatgcc aaagtttatc taagaaaatg tttatggaaa tattatttca ataatgaatg 22980
   tttagaagac tgaatttcct gactgggcgc agtggctcat gcctgtaatc ccagcacttt 23040
   gagaggctga agaaggagga tcgcttgagt ccgggagttc aagagcatcc tgggcaacac 23100
   agcgagaccc tgcagcaaag taaaaagaaa aaagaattga aaaaggaaga ctgaatttcc 23160
   tttgggcaag tcatgtgaca ttcctgtgcc tcagtttctt catctataaa gttaattcct 23220
   acatttttgg ggaagggaga gaaaaactta ggatagtgac tggcacagaa gaagcactat 23280
   atactatata tatgtggata tcatttgttt ttatggtacc attttagcta tctaatgcaa 23340
   aatatgaatc ttttttttct gggtcttaaa ttatggaatg taagaatttt ctaaattctc 23400
   taattctgtg ttagttttaa agcaatggag taacgtatct gtcaacttgt aaatataagg 23460
   atcaacctga tccacaattt gacccctagc cactaatatt taatagtaca acactcagaa 23520
   attatcaaag gtcagagaag ccaaacaaat gtaaaaacat acaggtgctc agaaagatgc 23580
   acctgtaatc tctctaagga gaaatatttt ccaaactgag tgacacggtg ctttagtgag 23640
   ttgtggaatc aatctcatga tttccaacct agtgttcttt taaaaatgaa ctagtccaca 23700
   gtagaatata ctaaagtgct ggtgcttaag atagtattgt tttctggaaa aaaaaaaaaa 23760
   attttttttt tttgagacag ggtctcgctc ttgcccaggc tgaagtgcag tggcacaatc 23820
   atgctcactg cagccttgac ctcctgggcc caagtgattc tcccacctca gccttttgag 23880
   taactgggac cacaggtacg tgccaccaca cccgggtaat tttttaattg tagagacagg 23940
   gtcttgctat gtgcttaggc tggccttgtg aactcctggg ctctagtgat ccactagcct 24000
   cagcctccca aatttatggg attataggca tgagccaccc tacctggcct gttccctgaa 24060
   tttttttttc tttcaggtgt ttgtgcatat gtgtgtgtgt atgggtataa cagagagaca 24120
   gagagaaaga aacttttcta tctcactttg caatcagaag tttgaagtct tatcttttgg 24180
   cttttgtttc agaaatattt caaatgtaga ctctctcctt taccacactg tccccttagg 24240
   caaggtcttt gccattcttc tgagactatt gcaacagact cccaacttct gactgtgggc 24300
   ccttctcaaa aatgattgtt tatgcaataa atctaaaccc aagacaacta caacaataca 24360
   acaaattctc tgcttaaaaa cttccaatgt ctgccgggcg cggcggctca cgcatgtatt 24420
   cccagcactt tggaggcaga ggcgggcaga tcacttgagg tggggagttc gagactagcc 24480
   tggccaacat gatgaaaccc catctctact aaaaatacaa aaaattagcc aggcatggtg 24540
   gtgggcgcct ataatcccag ctaattggga ggctgaggca ggagaattgc ctgaacctgg 24600
   gaggtggagg ttgcactgag ccaagatcac accattgcac tccagcctgg gcaacaagag 24660
   caaaactctg tctcaaacca aaccaaaaca aaacttctaa tatctaccaa atgtttcaca 24720
   caagtatttg gggatcttca caaatggccc ttatggagtt ttcctttgct gagaccctat 24780
   gctctggcca cactaaactc attcagcatc ccagaaaggc ctcagccttt gtgagcaagc 24840
   tcttatctcc aggcctctca caaagacctg ttccagtaga agctcagggg agcacactgg 24900
   acattattcc aacaaccctt tccccacagc tatgcagcca aatctgccag ctcagttaat 24960
   taattaagca attcagagat gagggtctgc ccaggctgga gtgcagtagc tgcgacctca 25020
   agctcctggg ctctaagtga tcctcttcag tctacccaga agctgggact gcaggcatgt 25080
   gccaccacac ccagctaatt tttttttttt tcagtaggga ccaggccaac ctagtcttga 25140
   actcctggcc tccagccttc cgaagtgctg taattacagg catgaatcac tgcgcccagc 25200
   caacccgccc agtcttgtta gacatggggt ctgtagtttc tagtaggttc ttgagtctag 25260
   ggttcctacc tcatgtttta tagttaattt aggggaggga ctgtgtctgt ttatctgggg 25320
   atgtaggggt gggcaggggg atagagggga cttcaattaa tgaaaccaga agcaaaactc 25380
   agttgaggac accggtcatg agagtggcct gattatggcc aatcttacat aatgtgtgag 25440
   atcttgatat taccccatcc ttgagagtcc tctataaagc tacagggact tgggagcacc 25500
   tttaattaca gacaacccat gttcctgtgg attatgattt attagattgc acatgcctaa 25560
   ataaagacat cctctgcagt cttttgacaa ttctataagc atcttctgac tccgcaatta 25620
   gacagctaag agatctgtgt tacttccctc acatatataa ataattttaa ataaaaatca 25680
   tggcgtgaat aatttctttc ctctaccgat ttgaagctat ccatttggaa gaccactctg 25740
   aagagatgaa ataagtcttc tgccaaagat tacttattaa tttacaagga aaaggggaag 25800
   ttttgttcct ctccgtgaat ttgattgaaa atcgagggct ttctcgaata gttttggcat 25860
   ccagggtcat ttttcattaa aaagagaaaa gtcatgtcaa atatgaattt ccgcagatta 25920
   ttcagcacta gaccctggga gattctgtaa agaggggttt tgttatactc aacttttccg 25980
   ggtaaaacaa acacaaatac tcctcctcca aggggcgggg gcggtgccta ggtgatgcac 26040
   caatcacagc gcgccctacc ctatataagg ccccgaggcc gcccgggtgt ttcatgcttt 26100
   tcgctggtta ttacatcttg cgtttctctg ttgttatgtc tgaaaccgtg cctgcagctt 26160
   ctgccagtgc tggtgtagcc gctatggaga aacttccaac caagaagcga gggaggaagc 26220
   cggctggctt gataagtgca agtcgcaaag tgccgaacct ctctgtgtcc aagttgatca 26280
   ccgaggccct ttcagtgtca caggaacgag taggtatgtc tttggttgcg ctcaagaagg 26340
   cattggccgc tgctggctac gacgtagaga agaataacag ccgcatcaaa ctgtccctca 26400
   agagcttagt gaacaaggga atcctggtgc aaaccagggg tactggtgct tccggttcct 26460
   ttaagcttag taagaaggtg attcctaaat ctaccagaag caaggctaaa aagtcagttt 26520
   ctgccaagac caagaagctg gttttatcca gggactccaa gtcaccaaag actgctaaaa 26580
   ccaataagag agccaagaag ccgagagcga caactcctaa aactgttagg agcgggagaa 26640
   aggctaaagg agccaagggt aagcaacagc agaagagccc agtgaaggca agggcttcga 26700
   agtcaaaatt gacccaacat catgaagtta atgttagaaa ggccacatct aagaagtaaa 26760
   gagctttccg ggaggccaat ttggaaagaa cccaaaggct cttttaagag ccacccacat 26820
   tattttaaga tggcgtaaca ctggaaacaa gtttctgtga cagttatcta taggtttaag 26880
   ttgtgatgca gctgagttga aaaggcttga gattggagaa ttaattcagg ccaggcttca 26940
   agaccatcct gggcaacata gccagactac catctatacc aggggtcctc atttccccgg 27000
   ccaccgaccg gtaaccggtc cctgtccatg gcacgttatg aattgagccg cacagctgag 27060
   gggtgagcga acattaacca actgagctcc accgcctgtc aggttagctg cagcattaga 27120
   tagattctca taagctcaaa ctgtattgtg aatggcacat gcaagggatc taggtttcag 27180
   gctccttgtg acaatctaat gcctgatgat ctgaggttgg agcagtttta gtccggaaat 27240
   cattgctccc agcccctgca ccccctggtc cgtggtataa ttgtcttaca caaaacggtc 27300
   tcttgtgtca aaaaggttgg agactactgg ttttacaaaa aagtaaatta gtcaagcatg 27360
   gttggcacgc tcccttagtc cctgcaccca ggcgtttaag gatacagtga gctatgatgg 27420
   tgctacctca ctccagcctg ggtgacagcg agtcagacgt tgtctcaaaa cttaaaaaaa 27480
   aaaaaagtta aaacagaaaa agggcttctt gtcagagact gccgtatatc tagaggtcca 27540
   ggaactaaaa agtctgatgt ccaatcctga aaagctcgat ggtgcactag aggaggcttt 27600
   tacatgtaag agcatctaag ttctggaaat gccagtgtca gggaagggaa gtggagagca 27660
   atttggcatc caaacataac ttgctgatac tttttttttt tttaacacaa gtactacatt 27720
   ctagtctttc tgtggtgtca ttgtaactat tgtttcttaa tatgctatcc actgacttca 27780
   agggatcaat aaataggaat caaggtgtcc cagaatatgg attaggggag tttttttgtt 27840
   gttgttgttg ttgttgtttt tcatctattc attatcctgt agctgaaatt tagaattttc 27900
   ttccattgtg tgtgactgat agaaataaca aatttgtagg ttatagttgt tgcaagaatc 27960
   tggaaatcgt gcttgcttat ttccgaagta ctattaggta tatcaacaaa aacacacata 28020
   ttacggtcaa gtggtttgat aattatttta atattattgg tctaatacaa ttgtaaccct 28080
   atgaattact ttaagtatct tatttatgaa aagaatctgt aagtttcatc agactaccag 28140
   agcataccga agactgaaaa attttaagaa tccaaacctt aatggaaatg ttggaggctg 28200
   cccaattagg ttctgaattc caccttcctg aatcacaaac ttgttttaac tctcagtctg 28260
   aggtaaacta cgtttctctt taaacagaca tagtttaatt ttcctttgat ttttgattta 28320
   gtattcttac tgatcatcat aaataaccaa tgctaatgtt agtctacttt ggaccatggt 28380
   atttcgagaa actttgaaca aagtcccctg caaaactatg cattgcatta tttcacatac 28440
   atttatgttt tccagacggt tcaatagtac ctcacttttc tgaacttatt tgtatagttt 28500
   ggcatctttt taaaaattgt gtcctataat gaaaggttgt aaacattatg ttttaaattt 28560
   gtatagataa aatcaaccac agacctttcc ttgcttggat gtaattgcca ttgtttccca 28620
   atgagttcgg aattactagg attgtgcaaa aatatgcctc acttgcctga catagcagag 28680
   agccattttg cctaaatgct gtgcccagca atggactgtc accagattct catcacatac 28740
   agtgaggatg aacaactagc ctctcccagc agctggccgg tctctcaata atatgggact 28800
   ccctcaagat ggcttcctgc acctttgctc ctctagcctt gtatgtatac aaggctagca 28860
   tgcctggcat acataaggtt aaaaacaaaa tcaataagtt atggttcttc ctccagttct 28920
   ggggattatt agaccacttt tttgttttgt tttgttttgg atggagcctc gctctgtcac 28980
   ccaggctaga gtgcagtggc acaatctcgg ttcactgcaa cctctgcctc ctgggttcaa 29040
   gcagttctct ggctcagcct cccacgtagc tgggattaca ggtgcccgcc accacgcccg 29100
   gctaattttt gtatttttag tagacggggt ttcaccatct tggccaggct ggtcttgaac 29160
   gccagacctc gtgatccacc caccttggcc taccaaactg ctgggaatac aggcgtgagc 29220
   caccgcgccc ggacttagac cactttgttt tggccaatag gacaacagcc atagaaccct 29280
   ccgcaaatga gagcttgtcc ctaaagatgc tttatttaca tagctgtgtg ccgcatgagc 29340
   caaaaggtga taacctttgt tcaacacgcg cctccagccc ttcggttaag tccaaagtac 29400
   cattcttaga atgctctaaa atacataatt tttttttttt tttttttttt tttttttgag 29460
   gagtctctct ctgtctccca ggctggaggg gagtggcgcg atctcggctc actgcaatct 29520
   ctgcttccgg gctagctggg cctacaggtg cagaccacca cgcccggcta agttttgtat 29580
   tttttttggt agagggggtt tcaccatttt ggccaggctg gtctcggatt cttgatctca 29640
   agtgatacac tagctttggc ctcccaaagt gctgggatta cagtcgtgag ccactgcgcc 29700
   cagcaaaatg ctttttgtgg agccaatcac tttattagcg cttacctctc tatgcctact 29760
   ttatgctttg aaattttgtc acagtgtggc cggtcatggc aaacacaatt cattcttatg 29820
   caggatgtca cggttatttc tgtcatccaa actcattctc gcaacgcatt tcagctcttt 29880
   aaacgacttt gtgagcggcc ctgaaaaggg cctttgggtt tttttgtttt tgttttttga 29940
   agttctcagg agaccgcgta ttcttagatt cagccgccga agccatacag agtgcgcccc 30000
   tgacgtttta gggcatatac tacatccatg gctgtgacag ttttgcgctt ggcgtgctcc 30060
   gtataggtga cggcgtctcg aataacgttc tctaagaaaa ccttaagcac acctcgagtc 30120
   tcctcataga taagaccgga aatgcgcttg acgccaccgc gccgagccaa acggcgaata 30180
   gccggttttg taatgccctg gatgttatcc cggagcacct tacgatggcg cttagcacca 30240
   cccttcccca agccttttcc gcctttgccg cgaccagaca tgattcctat cgcagtggaa 30300
   ggtatgaact gaaacagttc cttaaataca aacttggcgg acctgattga aaacaacatg 30360
   agttggcgcg gttttttttt tttttcaaat ttggtcacca agtgggtgga gcaagaaaaa 30420
   ctgtttcatt atggttcatt gttttgattg gccagtgaca gcttgctctt tgtgggagtg 30480
   gaagggtgtt tgcaagttga atgcgctgta ttcctgtcag cttaatgacg ctaagcatag 30540
   ccccattcca catttctttt tatttccact tgctaactaa taaattacgg aatagtttat 30600
   tggggaacat acaaataatg tttaaaggag gtcagattta taggtcaagg gatttaccct 30660
   cccaatcatt ttaatatttt tatttaaacc aggcattttg atggccttct ctgtgctgga 30720
   caaggtataa gtttggctat gaagtttcac tcctaaagac cctatgtttt gggaaggcaa 30780
   aaaggtagcc aaataattgc aaattaaaac ctcataagtg caaacttctt cctcgtcact 30840
   ttccctatct cgattcaaat atttgttgaa tgactcattt ttctgcaaaa gtctgagaga 30900
   gacagggaat ataaacttaa gtctggataa tatgttttcc cgggacgctc ttcctggtct 30960
   gctgtgcctg tttgctgtgc ctgaaattcc aaacactctt cccttccctc cgtttttaat 31020
   cccctttcaa cttgctacag ctttagagaa aagaacattc gttttgtaca gttggggatt 31080
   aattgaagtg tagggctaat acttgattaa ggtcattaca aaatctacag ggtcttcctc 31140
   tgggaggttt ttgtgataag attattggtg ttaaaataag gctaatcccc ttgaaaaata 31200
   aatagaatag cagaattggg tctgaatgtg gtttgaagaa agggacttct caattcaaaa 31260
   ttttattctt agcttcctgc gggagctttc cagaatgccc ataagatcca cttttgttta 31320
   aaaaacaaaa acaaccccac ccaccactct ctggttaata aatgaatttc tattgggaat 31380
   atttagaatg gggctgtggc ctgtgagaga cattatatag taacctcaga cttgctcaca 31440
   tgaagagaag aaatccagga atggagaaaa aagacccagg aaaggccaga atgctctaca 31500
   tgtcatattg tttgtatcac ttctgaaata attgattaca ttcttctgcc ccaaattgag 31560
   ttcttaggtt cttccactca ctgtccacat gccacaacac agaccttata actagagact 31620
   tagctaggaa gaaatgtcaa acattacaga gaaaaaatgc agagtctgag atcataagta 31680
   aaactctgaa atctcaacat gccttttaat tcatgaaaat aaaaaatata gcagcatatg 31740
   caatatgaca attctctgaa aacatacatc atgtgaacta ccctggaaca catctcgcca 31800
   agtgccatct tcattttaac cagaggtcta ggatgccttt cctttatttt gcctattata 31860
   tcatttataa aaccccattt ttattttgat attttattta ctttctattt cctgctccta 31920
   atatctcctt tctaaacttt tctcaatgac agtgactcaa aaacaatgaa tgtcagaaca 31980
   aatatttaaa ggatctgtac atgtagatat atatatttaa aatggattct tccactctgc 32040
   gaagaattca ggcatactca atcttatggt tagggagaga ttaggctcac tcgcctaatc 32100
   tgtatggctt ctcgttcgct ttccatttca ccttcctctc acccatcaga tcaaactcat 32160
   tcattgaaca agagacctaa gcccttcaga ttaaaactct gcaaacaagt tgtggttgag 32220
   aggatacatg aagcattcaa acaaataaat ctatgatatt aatcagaggt taatctatga 32280
   tattaatcag aggttaatgc agtggctcac ggctgtaatc ccagcacttc aggaggctga 32340
   gttgggagaa tcgcttgagc tcaggagttc aagaccattt tgggcaacat agcaagtctt 32400
   catctctact taaaaaaaaa taaccagagg tgttatgaaa atataaattg tccagaacta 32460
   ccctccacaa actaactctc tcagaatatt cgatatgagg aatgaaatat ggtgtgtgtg 32520
   tgtgtgtgtg tgtgtgtatg tgtgtgtgtg tgtgtgtgta tgcacctata tatggcacct 32580
   atatattcaa caaacaattc tgataattgg ccagggttga gaatgactag cagcccagca 32640
   tacactatca gttttaagta tataattgcg ctttagtaaa atgtaaagaa atcccagagt 32700
   agaaatactt ttaagctata ttacaggtga gaaaatgcat aagtatagtc tcacccaact 32760
   tagactatgg gggctttata atgtcacaac agttgtttcc aggcatttgg ggacatcacc 32820
   actggtcttg ggcaagaaac tcctctagcc aatggctgat ttatctcact cccatctaag 32880
   gcttcactgc atttctcttt ttcagcaacc taacttattt aaaaatatcc attttctgat 32940
   tcattttttt ctgaattaaa ctgtcagtac cattggcaca cctttggttc cgtagcatac 33000
   ctgtgtctct gctgtgtttt ttttttacct ccactcctta cttttctaga aaaaaatctc 33060
   tgctttttct tttcagttta aattatttca caaaaagttt tcttgacttg cacttcctag 33120
   gcttgctgtc cttgtgtggg cacgctccca taaacactat taatacactt cgatttgtta 33180
   aaaataaaga tatctggaca gaaaatttct tttctttttt taagatttta aaatttttaa 33240
   tgtttatttt tttcctagac tggagtacag tggcaccatg atggctcatg gtagcctaca 33300
   cttccccggg ctcaagtgat cctcccacct cagcctccca agtagctggg actacaggtg 33360
   tgcacaacca cacctgacta atttgtttta tttgtttgtt ttgttttttg agatggagtt 33420
   tcgctcttgt tgcccaggct ggagtgcaat ggcgggatct cggctcaccg caacctctac 33480
   ctcccaggtt caagcaattc tcctgcctca gcctcccgag tagctgggat tacaggcatg 33540
   catcaccacg cccagctaat tttgtatttt tagtagagac ggggtttctc catgttgagg 33600
   ctggtctgga actcctgacc tcaggtgatc tgcccgcctc ggcctcccaa agtgctggga 33660
   ttacaggcgt gagccaccac gctcggccac taattttgta tattttgtag agatgggctt 33720
   tccctgtgtt gtccaggctg gtcttgaatt cctgggctta agtgatctgc ccaccttgtc 33780
   ctcccaaaat gctaggatta ctggcgtgag ccaccaggtc tggctggaaa gataatttct 33840
   aacattatcc tctcttaaac atttgtttca aaaattttac aaacatgaga gtaattaaat 33900
   ttgattttca aaattccctt gaatactttc ttaatagcac acagaaagca caaagtattt 33960
   tacatttgtt ttaatgatga aattgtgaac ccaaacttac acaaagaaaa acccgtaaca 34020
   ttatacccat acttaaaaca gatgccctca tatacatagt aaaactcttg ggggcagtag 34080
   tgaagttggt tatttactgt tttatgaaag tgccattcag ccgggtgcag tggctcatga 34140
   ctgtaatccc agcactttgg gaggtcgagg caggctgatc acgaggtcag gagttcaaga 34200
   ccagcctgac caaaatgatg aaaccctgtc tctactaaaa atacaaacat tagctgggcg 34260
   tggtggtgtg tgcctgtagt cccagctact caggaggctg gggcaggaga atcgcttgaa 34320
   cctgggaggc ggagattgca gtgagccgag atcgcaccac cgcactccag cctgggagac 34380
   agggcgagct ccgtctcgaa aaaaaaaaac aaaaaagtgc cgtcatagtg actcagtttt 34440
   aaggaataaa tcaaggatat ttaactcaat agactacagt tagctaacgt gacttgcact 34500
   gaaagttata cgaatattgg tacttattcc cctgcccctg aagtatgaat taaagactcc 34560
   aaaattcttt ttagaatctt cagagtaaaa gctagaattt gattttttta aataataaaa 34620
   aaatactttg tatctaaatc tggtgtataa aataacttgg tggatgatgc ttcaaggcta 34680
   tccatcccca aatttctccc tgaatgataa agagaataaa tgaatatgtc aattcaaaag 34740
   ttagaaattt ggccgggcac ggtggctcac tcctgataat cctttcggac gctgaggtgg 34800
   gtggatcgca tgagctccgg agttcaagac caacctgggc aacatagcca gaacccgttt 34860
   caataaataa tagaaaaaaa tgagccaggc gtggtggtcc cagctactca gtaggctgag 34920
   gtgggaggat cacttgagct caggaggtcg agactgcagt gagccgtgat cgcagtactg 34980
   cacaccagcc ttggtgtcag actgagaccc tgtctcaaca acaacaaaac aagttagaaa 35040
   tttggctggg cgcggtagct cacgcctgta atcccagcac tttgggaggc caaaaagggc 35100
   ggatcatttg aggtcaggag ttcgagacca gcctggccaa catggtgaaa ctccatctct 35160
   actaaaaata caaaaaaaat tagccgtgca tggtggcatg cgcctgtagt ctcagccact 35220
   tgggaggctg aggcaggaaa attgcttgaa cccaggaggc agaggttgca gtgagccgag 35280
   atcatgccac tgcattccag cctgggtgat agagtgagac tccatctcga gaaaaaaaaa 35340
   aaaattctgt atgaactgaa caaaatatcc ttaaatttta aaatacatct gaaagatatt 35400
   tcaaaatatt taggaaaaaa attataggga tcaggcaaat tctgagattc ctttttccct 35460
   gcagcaaaca ttaggagtgc tgctgttcct aaaaacatgg taactgttgc cacaccgtat 35520
   gtttccttgg ctcagacata aggttgtgta gttgttattc cagaatagct agaataaaaa 35580
   tccagcacat cattttcttc agcaagttaa ctaacctctc tgtgccttgg tttcataaca 35640
   gcaacataag cataacagaa tagcagcaat agctcctacc tacctcataa gattctttgg 35700
   aggaattaaa ttaagattca gaacacagcc taatatctag taagtaataa taattggcta 35760
   aaaaaatttt cttaagatta tatatattca tggggtacaa gtacaatttt gctacattaa 35820
   tatattgcat tgtggtgaaa tcagggcctt caatccatcc cggaaaaaaa aagtttttga 35880
   aaagatttct gccatggaaa acttttaatg tacaaattca tccatccaag aaatagaaaa 35940
   tatataagta tcaactccaa atccaccata tctatctctt ctacacctta aacaattact 36000
   cagaaataga atgcttgaga taccagaatg catgcatatc aagtaataaa tgcatgcagg 36060
   atgtcaacgc atcctaggct ttcaaataaa attgtcatac aaaatacttt aatattgtag 36120
   taacattcta catgttagag tgtagaagtt aatcgctgat gcaaaaaagg aaaagaacac 36180
   attataccca aagcctacag agagaatcac aattacaaat atcagcctgc atgtgaaaat 36240
   ctttaatttg aaagtcagaa atatttaaat gatagtcatt gttaaatcag attgtggttt 36300
   gaaaaaaagt tagtttaaaa ctgagtttat gaaaaatttg gggattttag agacagtgtt 36360
   ttgtttttaa atgtgtgtga gtttgtgaag aatgttttat aaaatactga cagtattata 36420
   agatgacatt attataatac aacataagaa ttttggcctg tacctctcag cagtcctcaa 36480
   tcacctgctg tacttgactc aatgattatc agagtggttt gttttccttc tgttgtgttc 36540
   ccagttcagg cagctcagca atggcctgtg attccagcaa ttcaaatagc tggtaagtag 36600
   tttcttgttt gttttctcaa attttcaggg gcttttctct acaagtgatt tccagtgcac 36660
   gcccctccac ccattcttta ttcctttacc ttcaggaaaa ccctcagcgc tgcatctctg 36720
   gtcaccggac caccgtggta catttaccta tggccaccag gtgtcaccct tctctttact 36780
   accatggttt gtgaatggtt ttgccagagg tgaataagaa tttaaaatgc aggtctttga 36840
   tttttcaaat gtagttgacc ttaagaattt atgaataaag ccagaaaaat taagcttaaa 36900
   aaacaccgaa agaaaatgag gacttaaaat ttctattaaa aaaattaaca ggccacagtt 36960
   gctgatgttt agtaaatgtg ttagtgaaat gtgttactgt gaagactggg gtgtttcttg 37020
   aaatctcagc ccaggtgaaa taaaaccaat ataaaacaaa tgcttaccta ataaattaat 37080
   tgtaacatat tccttatgag gtagaagagt aagtgaagcc ttatagcagt ctgctttcag 37140
   tatagtaaga tattaagaga gaaataattt gtcatatgct ttcagaatgg tttgctggta 37200
   aaataaccaa tgtcttacaa cttagacgac aatgtcccta gagtgaagaa acacgattaa 37260
   ttcggctacc acagttgaat gaaaatattc cgtaagacaa aatgtaaaga aattagaagc 37320
   aaaataaatg tctccaaaat gacaaagcga ttaagtatat acacaagatg aacaagaact 37380
   tcaataaaat catgcagtat acaatacaat atacatttat taaagtatat gcatttttaa 37440
   tgcaacaata atactaacag gtaatagaca agttgttaat agtttttcac tggctaatta 37500
   aataacagct ttaattgtat tcattttata gcttttctac aatgagcgta aatcacattt 37560
   acttttttct acataacttt tctaaccaca aaaaaagaaa atggtttaaa agaagagatg 37620
   agatatcttt gctaaaattt aatgcctaaa gaagaaactt ctgagctgta tatggtatcc 37680
   tgaagcacct gcccttcaag acagaatgct tgtaccacat ttatgcagcc aagtgcatgt 37740
   agtaacataa agtaaacaca tgccatctgg atatatatat taagactctt ttgacggctg 37800
   ggcagggtgg ctcacacctg taatctcagc actttgggag gccgaggcag gcggatcacg 37860
   aggtcaggag agttcgagac cagcctggcc aacatggtga aaccctgtct ctactaaaaa 37920
   tacaaaaatt agccgggcat ggtggtgcac gcctgtaatc ccagctactt gggaggctga 37980
   gacaggagaa tcgcttgaac ctgggaggca gaggttacag tgagccgaga tcatgccatt 38040
   gcactccagc ctgggcaata gagtctcaaa aaaaaaaaaa agactctttt gaacatggtg 38100
   aactgatttc ccagaatcta gcaattcctg aatgtcctgg ttagattttt tttttaatgt 38160
   gcaccggaac cccagtggct ccatggaagg acctgggcat cctctaagcc acttggtggc 38220
   ttccattata ccatctcaaa atgagagagc ttactccact tcattgaggg aaataccacc   38280
   agagttctga ctccagaggc actggcctag ggaggacacc gtgtgtgaag cccagcaggg   38340
   ccactagctg tccccaccaa ttacagtcct tgcgtagggt ccaaagaaat gaatgccaaa   38400
   gagagcaaca gaggagcaag ggagtcacat tccaggacct tccttcaggg acttttaaag   38460
   gaaacatgac agctgaggat cagttggttg ttttctgctg ttccccttca tgtgattcaa   38520
   gctcactcag aagaaacaca atgagacaag agaagagcca tctccttcct tctctattta   38580
   ttctaggcat ctaaactact gaatgtagtg gtgtctgaga tgtatcaaac ggtcagattg   38640
   actgagtttg aaacctgttt ctatcactga caaactatga gatactctat acttcacttt   38700
   cttttttttt tcattttttt atttttattt ttattttttt gagatggagt ctcactctgt   38760
   cacctaggct ggagtgcagt ggcgcaaact cggctcactg caagctctgc ctcctgggtt   38820
   catgccattc tcctgcctca gccttccgag tagctgggac tacaggcgtc tgccaccacg   38880
   cccagctaat tttttgtatt tttattagag atggggtttc accatgttag ccaggatggt   38940
   ctcgatctcc tgacctcgtg atccacccgc tttggcctcc caaagtgctg ggattacagg   39000
   cgtgagccac cgtgcccggc ctacttcact ttcttcattt aaaaaagaaa tggggataat   39060
   agtacctatc tcatagaatt attgtaagaa gtgcatgcag taatgcatgt aagtaggtgc   39120
   tcagaagagt cggacacgaa gtaagtgctt ttatcatcct tatcataatt ttcattatca   39180
   gaacaaggag agaccaggta gaaaattatt gtgattcttc aggtctggaa tactagagta   39240
   gcatcccaaa tgaaggcacc attaaacttt gcaaatctgt atgacacctt catgccaatt   39300
   agaaaaaaca cctcttcaca acccctttca agatatttgc ctcctacctg ctaaaaacac   39360
   ccatcatact acccacagat agccatgatg ctttttctgg gacaggtgcc tcttccattc   39420
   gtgcagtgta cagccttcat agctgtgcaa ctcacatcac aatcagatgg aagaatcccc   39480
   aaggcttggt gacagatgag ttactgggta acacagagag aggattcaaa ggaaaagttg   39540
   aacgggtcca gaaaatgcat agatacatgt gtaaaaatct ggtaaggtta tgactagcca   39600
   cgtcccaggg ttcaaagctt ttctcagatg ttaaaatgaa tcatgtaagt cccccaaatt   39660
   taaggagtcc tcttccaaaa ataggaaatg aaatgacata ggtgtatgtc tctgaggtga   39720
   cggaggaaat gaaggaagcc tctagatgca gcttgaggtt catgagagac agttccaggg   39780
   gagaggtcac agctagggat caccggcatg caggaactca gaaacctaaa tggggaaatc   39840
   tttttgagga aatgaacaga gaaggctaaa atcaaggagt tcgtcaggca atttctatgt   39900
   ttaggttcaa ctctctcctg aaacatgaag agctcataaa tgcactccct ctttgagtct   39960
   ctagttttgt ctccttccca cagtgagtct gcaggctgcg tgtcactcac gttcagctaa   40020
   gacgtagtgc cccatggctc ctcctgtgga gacaagagac ccaggaaaga ggcatcacaa   40080
   acctaggcac catcttgcct cttctctctt ccttattttc ctcattcacc catctcaatt   40140
   tagacctggg cactattgga tttcaagaac cattatctct catctggaaa tgcttattgg   40200
   ctttctaact ggtctcctca cctctcatct aacttcttaa caacacattc accatataag   40260
   ggagatcgtg gtcctccttt cttaggatcc ttcaatgaca ccccagtgat cataacccaa   40320
   tatcccaaaa gacccttgga ctctgtatga gctggcttct ttctgattct cttttcccta   40380
   caccacagat gttcaggggg tagaaatgca taattggtga gtgatagcta cgcaaactca   40440
   gggttaaggt acagtaatta tttctaatct cccagtatgc cttatactct cctacttggc   40500
   atggttgctc cgtctgtgta gacctcccat catcttcaac ctcacctaat ggaatccagc   40560
   ttctccttca agatccagaa ggctatcttg atccccagct gaatgtgatc attctttcct   40620
   ttgacaccct aagcatttgc ttcctgcctg ctttaggacc tcatggggtc ttctttaact   40680
   acatttactt gctatcaatt tcattcccta ccagatttgg gttctgagaa tagccacagt   40740
   gacttctcaa cctcaaagcc cctgtactac cttaaacagc tcttgcaaaa tagtaggtgc   40800
   tctgaagatg tttgttgaat tagagacttt cattctgggg agaaccatta ttttctgtct   40860
   cccagggagc tgctggtgtc cccaaagaat ataaatgaga aaaatgcttc ccatggatgc   40920
   cagatcccct ctgcccctct tcccactgtg ccctggggca gaggtactaa gagacttccc   40980
   ccttgttcct actcacttga accctgcctc ttccttaata ttatgaacaa aattccaatg   41040
   aacaagatga cgacaaaaac agcaattcca ctgatgactc caatgactag ggtgccagac   41100
   ggtgagggct ctaaaacaga aaaagcaagt taaagccttt gattgccacc ctcagcccac   41160
   cccctaacaa agagcagatc ctcatctcac tgccataatt acctcctcag gcactcctct   41220
   caacccccaa tagattttct cagctcctgg ctctcatcag tcacataccc cagatcacaa   41280
   tgaggggctg atccaggcct gggtgctcca cctggcacgt atatctctgc tcttccccag   41340
   ggggtacagc caaggttatc cagccctggt aggtcccatc cccattgggc aatacgtctt   41400
   taggttcgaa ctccttggca tccattggct gcttatcctt cagccacttc atggtgatgt   41460
   tctgggggta gtagttcaag gcccgacacc gtagagtggt cactgaagag gtcacatgat   41520
   gtgtcacctt caccaaagga ggcacttgac aggaaagagg aaggatgagg agaggggatc   41580
   tgtttaccct tgccaggaag actggaactt tcacttcctt ctataggttg gaggaaggaa   41640
   ataccctttt cagaaaaaaa caagctacag gagagacacc attttgtgtc ctaagattgg   41700
   actctaacac agtgtcactt ggagagcagt cagatcagct tgttctcctc acatgtaaat   41760
   atacatatct gttacccatg ttctttgttc tgatagataa aattgccctt tatgtgcatt   41820
   gaaaatgatt gaatacagat ggtcagtttc acctgggtca acctaggagg cattgttata   41880
   agaagcggac ttgtaagata ggtagcttca gtgattattg ctatgttcta tgaaagaaac   41940
   ttttaaccta aaggattctt ctactctgat aagtggcctc acttgatatt ttgtcctggt   42000
   attcatatga tagctgagat ctctgaattc tctttttttt tttttttttt tttttaagat 42060
   ggagtctcac tctgctgcct aggctggagt gcagtggcgc gatcttggct cagtgcaact 42120
   tccgcttccc aggttcaagc gatgctcctg cctcagcctt ccaattagct gggactacag 42180
   gtgcgcatga ctgtgaccag ctaatttttg tattttttta gagacgggtt tcaccatgtt 42240
   ggtcaggctg gtctcaaact cctgaccttg tgaccacccg cctcggcctc ccaaagtgct 42300
   gggattacag gggtgagcca ccgtgcccgg ccttgacatt tctgaatttt taacaggtat 42360
   aaatatacaa aagattattg gttaaataaa aagcaagggc catagacact tccctttgag 42420
   ccatatgcat ggagaaaaga aattaaaccc atgacttgtg gctgtctcat acatctcaat 42480
   tataaggtag agactctagg attgagaaag tcccttccca gaatttggag aggcacacag 42540
   cctcagccac ctctgaaact ccaaccaggg attccgtgcc ctgcaacctc ctccactctg 42600
   ccactagagt ataggggcag aagtgtgttt ccaccatacc ttgttggtcc aaaacacctc 42660
   tccccagctc cagcaactgc tgcagctgtg cagggcagtc cctctccagg taggccctgt 42720
   tctgcctggc ccgaatcttg tgcctttccc actccagctt ggtgggccag gccctgggtt 42780
   ctgctgctct ccaatccagt gtgtcagggc agaattcaag gtggtcctgc ccatcatacc 42840
   cgtacttcca gtagccctcg gtactgttgt cttcttgcat ttcacagccc aggatgacct 42900
   gcagggtgtg ggactctgga aaaatcccca gccttgttaa ctgcaaccaa aggaataggt 42960
   ccctatttcc accatcccca aggaccaaat gatctcagga agcaaattcc ttccctcttc 43020
   cctgctccca caagacctca gacttccagc tgtttccttc aagatgcatg aaaagatgaa 43080
   aagctctgac aacctcagga aggtgaggcc ccctctccac atacccttgc tgtggttgtg 43140
   attttccata atagtccaga agtcaacagt gaacatgtga tcccacccct tcagactctg 43200
   actcagctgc agccacatct ggcttgaaat tctactggaa acccatggag ttcggggctc 43260
   cacacggcga ctctcatgat catagaacac gaacagctgg tcatccacgt agcccaaagc 43320
   ttcaaacaag gaaagaccaa ggtcctgctc tgaggcaccc atgaagaggt agtgcagaga 43380
   gtgtgaacct ggagacagag caacaggcct taaccatgtg tagtaggagg ggagcaggat 43440
   gttgaggctc cacacacctg catcaactca taccatcagc tgtgtctggt cctcattttg 43500
   tgaagggtga gttgcagtcc tgtctttctt ccatatgaca gtcctgggtg ctctttcctt 43560
   gtgtgctttt ctctgccaca cgtggctgcc accccctcac tgcccccaga tcctattcca 43620
   atactcatga ttagacagac tccactaaag ctggtggatt ctagaaaatg ttaaggtgtg 43680
   tctagccatg gtagttgaac tcaggagttg gtgctcaggg caaattagac ccaaatcctg 43740
   aggaataatt ccttcagttt tttttttttt tttttttttt ttttttgaga cagagtctca 43800
   ctctatcacc caggctggag tgcagtggca caatctcagc tcactgcaac ctgcacctcc 43860
   tgggttcaag ggattctcct acctaagcct cctgaaaacc tgggactata ggcgtgcgcc 43920
   accacaccag gctaattttt gtatttttag tagacatggg gtttcaccat gttggccaag 43980
   cttgtctcaa actcctgacc tcaaatgatc tacctgcctc agccaccaaa gtgctgggat 44040
   tacagaagtg agccaccgtg cccagccttg gtcctgaatt cttacactga actgcctatg 44100
   tggcctcacc acttggaagc ctgactggaa tctcaaactt aacatgtcca aatgcagatc 44160
   cttgatttac cccaaactgc tctttcctct gccttcacca tctcagaaat ggcattgcca 44220
   attaccccac tgctcaggcc aataaaatta aaataaagaa caaagtcaac tttaactctt 44280
   ctctttttca gggggtcagg ggagacaggg tcttgctctg tcacctaggc tgaagtacag 44340
   tggcacagtc atggctcact gcagcctcaa cttcctgggc tcaagcaata ccctccacct 44400
   cagcctcccg agtagctagg atcacaggtg catgccacca cacccagcta atttttgtat 44460
   tttttgtaga gaaggggttt tgctgtgttg cccaggctgg tcttgaactc ctgagctcag 44520
   gaatctgctc tccttggcct cctccttggc atgagctact acacccagcc aattcttctc 44580
   tttctctcac acaacataga atccttcagc aacttccttc agaatatatt caggagacaa 44640
   tggtttgtca ctcccctttc tgttcccacc cagcccactc cactacctct tgcctggact 44700
   gtgtaacagc ttcctggctg ggctccctgc ttttactgtt gctcccttca ttctgctttc 44760
   cacatagcag ccagagcaat cttttaaaag cctgtgacag atcactgtta ctccttggct 44820
   agaattcaca ccacagccta caggcgcctg cacaaccttg tttgtggctc ctcttctgag 44880
   cccattacct acttcttggc ctctactccc cagcactact tgtttatttt tttcaacccg 44940
   agcttcttaa ccaggagttt gtctactagg tgacatgtgg caaagtttag agacattttt 45000
   ggttgtcaag actgggggag tgctcctagc acctagtgag tagggaggac aggatactgc 45060
   tagacatcct acatgcagat ggtagtcccc cttcccaccc ccacgccgcc cccccccccc 45120
   acacacacac acatgagtag tgctgagaaa acccgctttt taatccaact tgccaggccc 45180
   actcagtttg cctgggaaat actgctccca gtcaatatca ttcttatttc cttcatgtct 45240
   ctgctcaagt gtcagcccca gagtgacttg ccctgacttc tctgcttctc acaacaccca 45300
   tgatttcctg atgttgtata tctttctgct catttgctta ttgtcatctc tcccactaga 45360
   atgcaaaata tcaaagggta aagacttgtt tccctgctct ctcccttggg gcttgaacag 45420
   tgcaacacat ggctgggact catttacact tgtaaacaat gaatatttct gctcaacatg 45480
   aaattttatt attcaacctc taatgcagtg tgatgtttaa gaatcatagc tatgaagtgg 45540
   agacatgagc tctgccacca aagcccagtg taccattgaa taaatttgcc aggaagcagg 45600
   ccgtgccatg cctcattctt gtcatgtgta aaatgtggat acacgtagta ccaaaactca 45660
   aagtgctgtg ctgaggccgg cgtgtgaccc acagaacact gtgctacact acagggcaaa 45720
   atcactgtca actaagatta gaagcagctg tagtacttga aataacatca gaaaaccaga 45780
   ttatttatgt tctttgtaac ctgaaaagag ttatataatc tgaattccag ttaacttcta 45840
   gtaaaataaa cgtattatta gctcctacct ccctatgcct agtgaaaatc aaataagatc 45900
   agatatgaat gtaacttaga agtgagtgca ttgcttacat gttcattatc agtactttgt 45960
   agagaggcct cttaattaca cagcacattg caaatcaata aagcctagcc gaaaagagaa 46020
   ttgttcagtt caaacgttca aaactaacat atacttaatt ttccaggcaa aagaacaatt 46080
   gccaagagtg gggaaaggcc cgaggtaggc ctctctcagg agcctcccac cctagagacc 46140
   tccaccccag gtctcaccaa aagtgggtgg aatggtgaag aattcagatc cccaacgcca 46200
   ctctttcgcg cccccaccgc ccaacgcatt cgttctgagg tggaaacccc gtgcggatcc 46260
   tgctgtgggt ttgctcagcc ttctcggcaa gcactcaggg aagaacttcc tgtttggaga 46320
   tgactgggga aaaaactgca cagctgacat tggaaataaa cccgagttcc aggttcaagg 46380
   agccccaggc ttagctcagc tcaagtgagg aactacgaga tttatttaaa agcattctag 46440
   ttgggggaag ggagtgggcg gttccaaaag tcactccgca gagccgggac agccggggga 46500
   gggggcaggt cctggggcga gggaccccta tctgcagttc agtggtaggc actccctcac 46560
   ggggtctgga cgcagaaagt agggagaggg gcttgcggat agggttgagc aggtcctcca 46620
   aagttagcaa actcccaagc gcaaagaaaa agctagtttc gatttttcca cccccgccgc 46680
   gcccctagtt cgcccgcagc cctcggactc acgcagcaag cgcccctgca ggaccgcggt 46740
   ctgcaaaagc atcaggagga gaagcgccgg cctggctcgc gggcccattt ccccagctct 46800
   ggccgcacgt ccccgttaaa tctccgcttc ttttgggggg cggggaaacg gggatggctc 46860
   cagaagtcac cctacagcta ttgcctaggc tcaggagatg cccagtaaaa cttcctggtg 46920
   aaaagcaaca ggtctttcag aactttagtt ctctctctcc tacagcagaa ggtacctgct 46980
   tgtgaaacac taggtgatcc agtgtccccc ttggttttta aatcctgaag gggtgttgtt 47040
   gattggggaa agtagcttcg caatgttctg atctgaactt tagatattta aatatttatg 47100
   attttcaaaa ttcaatcata catttaaaaa ttttatctca accttagacc aacttatgtc 47160
   ttatttgact tagaaatata aagctttttc attttgtttt ttgattcaaa ttaattaagt 47220
   cataacatta accaattaga tcctactgaa acaccttcca cagccttcat aattgaatta 47280
   tctgacaagt gtttcacaaa ctttacagta ttgggattat ctggagaatg attaaacata 47340
   ttgaggcctg ctcctaaccc cagacacact gatttaatgg gtaattgtta ggtagttaga 47400
   cattagcagt tgggagggga tgacagaaga gagcggaaag gctgtcacta agacagccac 47460
   tggcccacct aaattcaggc ccaagactac cctaatgcca ccctaaggga tggagtttat 47520
   gataaagtct gtggccaaaa tatcctggag aaagagaaag gagggtacag gtggaaattc 47580
   cctaaggtgg cacatgccca acaacacaaa agcctgtctt caagttcacc ccaagttcat 47640
   catgccatca ttataataga atttacatac agttttgccc ccccatccct gggaggcttt 47700
   tcttaacaaa ttataggtaa gaccatgcac agtttaattt tagattgtat agctatacac 47760
   ttcaatcaaa taacatcatc ctgtcactca gatacagccc aaacctcaac tcctccccac 47820
   aaaccccata aaagcacctt gagctctgta aagaagtgct gagttcactt cgcagaaata 47880
   agcccgctgt ccctcagagt gtattattgt gcttcaataa actttgcttt aagcttgcat 47940
   tttggtgtta gtttgtagtt ctttgctcac tatcacaaga actgagattg ctggttcaga 48000
   gctccggcta taataatctc ctcggttaaa ggatccatcc caatgcataa ttcccagtaa 48060
   cagtatggga tgccacctgg gcaatgggat tttaaaagct ttccttctcc ctcaacgaag 48120
   tttgggaatt attgccttag acatttcaaa caatattaat aaatttaata cacctgattt 48180
   gctccaaacc tttacatatc tagcaaattc aacaggcatt atttttgtaa gcatgtatgc 48240
   aaattttggc aattcaagaa aatcaaacag gatatcaggg cctcgactgt aggcaaacag 48300
   atacaataac attggaaaca tgtagaatat tgatgatggg cacattgggg ctgatagtac 48360
   tattcctttt tttcaatttt tggtaagata taattagcat accatataat tcatctatgt 48420
   aaaatgcaaa aattggcccg gctcagtggc tcacgcttgt aatcccagca ctttgggcgg 48480
   ccgaggaagg cagatcacct gagatcaggg gttcgagacc agcctggcca acatggtgaa 48540
   accccgtctt tactaaaaat acaaaaatta gccgggcgtg atagcaggca actgtaatcc 48600
   cagctacatt agaggctgag gcaggagaat cgcttgaacc cgggaggcgt aggttgcagt 48660
   gagctaagat cgtgccatca cactccagca tgggagacaa gagcaagact tcatctcaaa 48720
   aaaaaaaaat tagctgggtg tggtggcatg cacctgtaat tccagctact cgggaagctg 48780
   agacaggaga atcgcttgaa cctgggaggc ggaggttgtg gtgagccgag atcatgccat 48840
   tgcactccag cctgggcaac aagagcgaaa ctccgtctca aaaataaaat aaataaaata 48900
   aaatgcaaaa attaatggat tttagtatat ttacagagat gtgcaaccat taccaaaatt 48960
   ttacatttct atctccccaa aaagaaacca tgttccccta attcagtacc cttaattcat 49020
   cgcctcccag attcctccat tctcctcctc ctcccctccc agccctagac aatctttaat 49080
   ctactttctt tctatttgga acatttagta tacatagagg catataatat attgctttgc 49140
   cgtgactggc ttctttcatt tagcataatg tttttatgta tgtttttcat ggaccaataa 49200
   tatctattat aaggacatac cacaacatat tttatttatt cattcatcag ccgatggaca 49260
   ttggtttgtt tctactttat ggctattggg aatagtgctg ttataaacat ttatgtacaa 49320
   gtttttttgt agacttatgt tttgatttct tttggttata tatctagaag tgggtttgct 49380
   gggtcatatg gtaacactgt ttaacctttt gaggaattgc cacattcttt tccaaagtaa 49440
   gcattttatc ctcctatcag cagtgtatga gagttctgat ttctctccat ctttgcctgg 49500
   gtttttgaat cagggcccca gatagaacaa aaatgtggtt attcagttgt tccaccatca 49560
   cttgttgaga agactctttt ttcattgaag tgttttggca cccttatcaa aaatcaatct 49620
   accataaatg tgagagttta tttctggagt ctcaatttta tcccattatg ctataatcta 49680
   taatcctatc tttttttttt tttgacagag cctcactcta ttgcccaggt tggagtgcag 49740
   tggcccaatc ccggccactg gctcctcctc ccaggttcaa gcaattctcc tgcctcagcc 49800
   tcccaagcag ctgggattac aggtacctgc caccatgcct ggttaatttt tgtattttta 49860
   gtagagacgg ggtttcacca tgttggtcag gctggtctgg aactcctgac ctcaggtgat 49920
   ctgcccacct cagcctccca aagtgctggg attacaggca tgagccacca cacccagact 49980
   ataatcctat ctttatgtca ggactacact gtcttgatta ctatagcttt ttagtaaatt 50040
   gaattcaaga agtttctcaa cttcaaattt gatctttttt tggaagacta tattagctat 50100
   tctcagtctg ctgaatttcc ctaggaattt taggatctat tatcaatgtc tattctattt 50160
   ttgtatatgt tttaatattt tcataagaaa cttttttcat ttaaactttt ttttttaaga 50220
   aaaatagtga aaatcagaac actgggggtc aggcgcattt aacaggcaga agaagaataa 50280
   aaacttgtca tataaacaaa aaagaaatga ccaatcacat tgtggaagcc atggagtggt 50340
   tataggtgcc aaaggctgca gagaaatggt gtcagatata cctgaaaatt gtccattgta 50400
   tttggccatt aagagactta gaagacttaa gccatagatt gctcagtgag accccgaggg 50460
   caaatggtct gaaggtgaat agatcatttc acctttaaga gagcaggtag gaagctataa 50520
   atccaagatt aaaaagttga ctgaactgtt aaggaagaaa ctctaatctt gagccaccct 50580
   atcctggctc caccttctgc tgcaagcaaa cagaaatgct gaaattcaac actcacaaag 50640
   gctggtaagc tggaaatgac aaaaattact cctgggaaag tcagatttag aattaggcca 50700
   tatttgttgg ggttcagatt ttcatgtaca cttgggaaag ggtttagctt ataggcacat 50760
   gcatgaaggg aactggtata gggctgtgtt cataaggtca agagttgaag gccaggcatg 50820
   gaggctcttg cctgtaatcc cagcactttg ggaggccgag gcaggaggat ggcttgagcc 50880
   caggaattca agaccagcct gggaaacata gggagatgct gtcttcacaa aacaattaaa 50940
   aaataaaatt agtcaggtgt ggtggcacac acttgtggtc ccagccactc aggaggttgg 51000
   gaagatcact taagcctggg acattgaggc tgtagtcagc catgatagtg ctactgcaca 51060
   ccagtctagg tgacagaatg agaccctgtc tccaaaaaaa gagctgtatc cacatcccag 51120
   gaaagtggtt gaagatctac ttttctctgt aaacctaata aagaatagag tgacaaatgt 51180
   gtgttgtgga aagaaatggg gtgagagcta cgtagatgca aaacaataca tccccacata 51240
   ccacttgtta atcatccttt tccacccact tatgggatga attgcatctc cccaaaagat 51300
   actctgtcct aaccctcagt agctgtgaac ctgaccttat ctggaatacg gtgagttcac 51360
   tggttaagaa gagattatag tggaataggg tgagtcctcc aaccaatgac tggggtcctc 51420
   acagacacag agggatgatg gccaggtaga gatggaggca gagattggag ttatgctgcc 51480
   acaaaccaaa cacaggaagc tgctagaagt ggaaacaggc aagaaagaat ccttccccag 51540
   aggctacaga gggatcttgg ccctgataat accttgatct caactggcct acgtaactgt 51600
   gagagaataa atttcttttg ttctaagcca cccagttgat agtactttgt tacggcagcc 51660
   ctaaggaact tgatatacat ttcttttact gtcatagaag ttttgaatct tttaagtagg 51720
   tctgtaccct tcctcccagt gtcaacacat ggaattcctc tccttgtgcc ttgaaaagtg 51780
   aaaggtgttt gaactggtaa tgaaagaaat ctcagcatga ggccagatgc tgtacctcac 51840
   acctgtaatc tcagcacttc gggaggatga ggcgggcaga tcacttgagg tcaggagttc 51900
   tagactactc tggccaacat ggtgaaaccc catctctact aaaaacaaaa aatgttatcc 51960
   tagccgggca tggtgcctgt agtcccagct actcaggagg ctgaggcagg agaattgctt 52020
   gaacccggga ggtggaggtt gcagtgaact gagatcacgc cactgcactc tagccttggt 52080
   gagagagcaa gacttggtct taaaaaagag aaaagaaaaa tgaaatttca gcattataga 52140
   ataaaaatgt ttccccttcc ccccaaactt taaaaaagca gaagtctgca tcataaaatg 52200
   gtctttgcca atgttatttt tattataaca aaggaatctt gcaaggctac cagatctcag 52260
   caattgtcac tatgttctgt aaaaatcact tcctaaaatg tctgaattga ctgcttgtct 52320
   catttatttg tttctcgtgt catactgcaa tggatatctg tcttgttagt ataaatattt 52380
   gtgcattttg ttgttgttaa aacagctttt ttggcctgtc ttcttccacc tatgaggtaa 52440
   tataaaactc atgtttaaca cttatttttg tagcaggaca agctacagac aaaacccctc 52500
   agacactgag ttaaagaagg aagggcttta ttcagctggg agctttggca agactcacat 52560
   ctccaaaaac cgagctccct gagtgagcaa ttcctgtccc ttttaagggc ttgcaactct 52620
   aagggggtct gtgtgagagg gtcatgatcg actgagcaag tgggggtatg tgactggcag 52680
   ctgcatgcac cagtaatcag aacagaacag ggattttcac agtgtttttc cacacaatgt 52740
   ctggaatcta tagataacat aaccggttag gtcgggggtc aatctttaac cagacccagg 52800
   gtgcaacacc aggctgtctg cctgtggatt tcatttctgc cttttagctt ttactttttc 52860
   tttctttgga ggcagaaatt gggcataaga caatatgagg ggtggtcgcc tcacttattc 52920
   accccctttg agaatctcac tcattagtgg gagttctcac ttttattctc actacctatg 52980
   tcttcttgaa agacagattg ataatgattc atatagtaca cttgtgctga agcattttgg 53040
   tgagctaagg tagtgatgaa gctttttatc atttggagaa gtacaggtag caaacaagga 53100
   agcagtaagc aggtttctat taatattata actcctatta taagagtttt aaatcttctt 53160
   agcactcgga accatttttc aaacatggcc ccagaaacaa atccatacca cacctacatg 53220
   ggcacatgtg ccacttttgt catatttcta actatgtctt caactacttg cccttaatca 53280
   tctatgtgta gacagcaatt agtaaggtta aatttcctac agacccctcc ttcagttgct 53340
   agcaagtagt cgagagccaa tccattttga tagatagcat tttgcatctg agtttcttgc 53400
   caggccacag tagtcagggc tctgctggtc ttattagtaa ttatttctaa gacagcttgt 53460
   aaccgtatga ttcagttgag catgtaaatg ggggtcccat atccccacaa gccgtcttgt 53520
   gcccaagtag caggcccata atattgtatg attctctcag ggggccattc attatttttc 53580
   caattttcta tagctatgct tttttttttt tttttttttt tttttttttt ttttttgcgg 53640
   gaagcatata cagggaagcc caggagtttg cctgtcttta tgggcagtag gaagaaagat 53700
   ggtttagtag tgtcaataac acaactacct gcccactggt caggtaattt ggcataagct 53760
   gtatgcccac atatccagta taatccagtg ggggctgtcc agtcccggtg ggactctggg 53820
   tgggtccaca cagtttgcaa ctttgggaat ttactaaata gatttttctt agtgtggttt 53880
   gaactccact aggtggctgt ttttatagta ctattataca gtttttgccc aaggcagctg 53940
   agtcttccca caggaagggt gaagtccttc cccacttttg ctatacagta ttgtctaatg 54000
   attgaggctt ttaggaccca gaagttatca gggtgagtct tttgagctgg gaatttatca 54060
   ggaactgggt ctgtaggtac taattctcgt gcttcccatg gccattgatc tcccattaca 54120
   gttcctccac atacatacat aacatgaagt gacattgaga gactgggcta catgctcagc 54180
   taattgcaaa aacaaatttc ttgtttttcc tggaatttct agtactggca cattcagttc 54240
   atcataagaa ggtttgaaat actggctcag gggagcattt ataaacttct cctcaaacca 54300
   ccatatttac tcaaggatcc agtccagccc caactatttc taaggttaca cgatcccctt 54360
   ttttccagtg agaatcaagg gggttggtta ttactagttc taaggggtta cactgaccac 54420
   tggtacagga agggccactt ttccctttct gaaggtggac aggattcttt ttatttttta 54480
   accaagttgc ctaaatgaca caagaccagt atctacattt atttccacgc agtcttaatt 54540
   catgacaagc gtacttattt tctgccatat agcctctttc ctaatgaaca gaaccacatc 54600
   ctatttctaa cttattacta ttaatgacag cacaggcatc aaatttcaag gtgacttgtt 54660
   tgggcattcc tttttcttct gttttggcta acactttact cgtatcgttt atgaaccccc 54720
   accagtcctc agtcctcaat cttatttcaa aaactgtggt cgtgggaggc tcagatgggt 54780
   cataacacac atcaggttgg tcatttcttg ggctacctac cttgtataga atagcattat 54840
   acaaacaagt tatttttaga gtctttgtac acttataata accataaaat aataagactg 54900
   tagcaacttt ttgtcctacc tcagtgactt gatgtataca ctgggaacag ccctcagtct 54960
   gaggaaggtt agttgaagtc tttactgtgc aagtccaaat tttaaggaaa atgagtccct 55020
   tgatgagttt tctcatgttt cggccatgca tggaccagtc agcttccggg tgtgactgga 55080
   gcagggcttg ttgtcttctt cagtcacttt gcaggcgttg gcgaagctgc cacgtacagc 55140
   tcacagtcta ctgatgttca aggatggtct tggaagttgg gcccactaga attaactgag 55200
   tccaatacct ctactcagtc actttcaact gggctttctg ataccaggag caaggtggca 55260
   ggttttaggg tgttgcaaat ttcaatggtt atgcagggat tttcacatag caaactttgg 55320
   tacttggtta atctagcatt tgttagccaa tgatgtattt attaaagtca ccacagcatg 55380
   gagggccttt aagtttaggt tttgtccaag agttagctta tctgcctctt gtgctagcag 55440
   ggctgttgct gccaaggctc ttaagcatgg aggccaaccc ttagaaactc catctagttg 55500
   tttggaggcc cagcctcggc cagggcccca cagtctgggt caaaactcca accgccattt 55560
   tttctctttc tgacacatag agtgtaaagg gttttgtcag gtcaggtagc cccagggctg 55620
   gggccgacat gagtttttct tttaactcat gaaaaactca ttgctgttgg ttgtaataga 55680
   tgtagtttat ccaatctaca tttttattaa ctgtcaccca ccaaaatatt gactcaaatc 55740
   ctgcagctat ttgattttgg gatttaaatt gatctgctat tccctgtggg actccaattg 55800
   catctaaata gatgtgagag ttgaaagaca cataagggtc ttctcttgct ttacgatgtc 55860
   ttatttttcc tccctctggt tgatgaaatg ctagggtgaa agggatagcc aattggacta 55920
   aagtacaagt gccgctccag ttatttggca gagtgcccag taaaggtcca ccacaatacc 55980
   accacacatc cgcttgggga tgaacaaagg ctgactgatt gagaagctcc tgaaaattct 56040
   taagctcact gcatcccttc aggtctccaa ggaatgctaa gtttcctccc tgtcatgaga 56100
   gacaagaagt gaacttagtt ttgggagatg gaagctggat ggccctcagg ggttgacctg 56160
   cagggtgctg gactttggga tatagcagag agagcttggc acgacttatt actccaggct 56220
   gtagaatcct ggaaaacagt taccatgcag cccatgcctg gtcaacagga ggaccacctt 56280
   agtggaaagg ggataatctg gccctctggc ctgccatgtg cacaagcata acaattggtt 56340
   ttgtttaatg tgtggacaga atatttgatc cattccaact gggcatttgc atcttggtat 56400
   cctgcttaat tatcaaagtt tgttttaagt ctttaacttc tatgaccctc tagtaaaatg 56460
   aatgtatgat tttaggaaat tacaaaaacc ggttggggca gtccatcctt gctctttagt 56520
   ggtccacaca acattcgacc aactatggca taaaagctct acatcggggg gcaagactcc 56580
   tcgttgacac tggggtcttt attgaaatct ctctggaata aatggtctca gtttactaag 56640
   gctcagtctg aggagagtca ggagggacag aggtactttt ctgaagtaca gagatgtctt 56700
   cgacttggca agtccccaca gggtataaca aggcaagcat taaattcaat agtttgaggc 56760
   aaaattgact tggttatgtt aataactaga tggtcagaaa tagagtgagg gaagaagaaa 56820
   gagtaataga atagatgaag gagttaaatt tttcttagct ttagtttggt agggttttcc 56880
   cctgggacta tggcccatga ctctggaggg ggtggcactt tcttgactcg ggtgtgatga 56940
   gtccatccct ttttcaccgt atgaacaaca gtctcggtgg ttagcagcac aaggtagggt 57000
   ccttcctagg ctggctcaag ttttccttct ttccaccctt tgatgagaac atgatcttca 57060
   ggctggtgct ggtttacaga aaattctagg ggtggtacat gtgctaaaag acttttagtt 57120
   ttgagggaaa ggaaagtgga agataaacca agtatataac ttttaagaag ttgacctttt 57180
   gttttaaatg tggggacatc agcagtggac tttatagtcc ttggtgcctt cttactgaga 57240
   aatttccttt agcacctatt tttattagtt tttagaccaa agaaagtcaa atgccatttt 57300
   atatttgaca acgcttcttg tatgtttata ccagataagc tagatttcac ctttatattg 57360
   gtgtgttatt aatgttaaac ttagttttaa taaaactctg tagacatatt tatttgattt 57420
   ttaatgtctg accataaggt aagattttta tagacttttc tttaaccttt tataattttt 57480
   gttaaagaac aggttagtgc tttaagaaaa acccgttgtg tttttatttt aatgttcagt 57540
   tcacagaaaa actgtatgat accccttaac tttagccaat atgtttagac acagaatttt 57600
   ctttacaatt aaggtttcaa aacttgctta aaccttcaaa acaatttttg taacctttta 57660
   atgtaggtaa aaatccacat tcttatgcat cctcataatc cttttaccaa aggtatattt 57720
   tactttcctt acataccttg cacataaact gtttattcaa tagttttaca tttagaagga 57780
   ggcctaatta cttttaaatt atacaacatt tcttacataa atttattttt ctaacacaca 57840
   tttttttcat gactttcaca gacaattctt cgacatgcct caactttctg acttattgca 57900
   aacatccctt tctttaaaca actagttaat ttatctcagg acaaggattt tccatacaac 57960
   attctttttt atataaattc tgcctcctct ttatttcctt tttttttttt ccgaggatga 58020
   taaccattct tttccaaagc gaacttcttt tatgtctgtg gactagactg tctaaggcca 58080
   caagattaga agttactata atacatgtta cactgttaac ttttagcaaa ctttactttt 58140
   gttgaaaacc ttgtaagttt gggatttcaa ttatcctttg ctattaataa gaccttattt 58200
   agtccaaatt aacttagaat tggtatagat ggcttttttt tttttttaat tacctgggag 58260
   gaaccatcta tcctcctgtc ctgaagggag ttcctcctag gtctggtcag agctttgtat 58320
   ggtaattaag atttagatcc cctgttagga aacctgccgg gttaagagaa ttttcagtgg 58380
   ttaatgttaa atcatcttct tttttctttt ttccttagga tacttctgaa ccggtgaggt 58440
   gtgctcacaa tgaggtttcc tgtaaaagtt atttttttac tttcttctgt tagcaaagca 58500
   gttgccgcta cagattgaat gcatttgggc catccgcggg ttactgggtt aaggattttt 58560
   gataggaagg ccttaatgct tttggaatat gccctgacaa caaagtgcca gttccttccc 58620
   ggtgttcagc cactgcgttg atcctccacg agggcctgcc acgtgctgct ctggtgaggc 58680
   gttccaccgg ggcaattgcc tacctgggag cgctctccag atctgtgtcg ctcaaactgg 58740
   ctggagttcc ccgtagggat gctccacagg gcaggcctaa gtcgcctaag gggctgcctt 58800
   gaccgtccgt taatcacctc tgtctccaaa aaccagctcc ctgagtgagc aattcctgtc 58860
   ccttttaagg gcttacaact ctaagggggt ctgcatgaga gggtcgtgat tgattgagca 58920
   agcagcgggt acgtgactgg ggctgcatgc atcagtaatc agaacagaac agaacagcac 58980
   agggattttc acaatgcttt tccatacaat gtctggaatc tatagataac ataacctgtt 59040
   aggtcaaagg tcgatcttta accagaccca gggtgcggtg ccgggctgtt tgcctgtgga 59100
   tttcatttct cccttttaat ttttactttt tctttctttg gaggcagaaa ttgggcataa 59160
   gacaatatga ggggtggtct cctcccttaa tttaaacaaa attttcaaag tcctacccca 59220
   agtaaattgg caaatattaa taaagttatg gcatagaaaa taaaaatgat tgtaaaaggc 59280
   gtaaagatat ttctgtgggg aaaacatttg ttcattagtt atcagttaaa attctgtgaa 59340
   aaataaccac tagagaccct aaagtaccca ggggctaata ataagaaggg aggaacaccc 59400
   tctcactccc caccgttacc tgcccagaag ggaagaggaa gagggtgact ccaggagagc 59460
   tgtggtctcc cctccccata tgtccacata tacctgacct cccctcccca aaatatatac 59520
   ccaatatctc tcccatatat acatatttat ctgacctctc cacatatgta tacctaaact 59580
   ttctctatat atccacatat acctaaccct ctcacacaca tatagctgac ctccagtgga 59640
   ggaaaatggg gaagagagaa gaagttatca aaggataaat ctaggtcata ctcagaaatg 59700
   tgaaaaacaa aaaccacaca cagaaaaaaa aaacacacac aaaaaagaaa ttgataaatt 59760
   tgtttgtgtc aaaattaaga attccggttc aatgaaggat cccatggata aagttaagac 59820
   actgctgtaa ggatggtaga gaattaaatg tctgaatcag acgaaaggat gagtaattag 59880
   aatgcacaag gccaagaaga acaaaacaga aactccacat aaaaaatgta tgaggccggg 59940
   cgcggtggct catgccagta atcccagcgc tttgggaggc cagggcgggc cgatcaggag 60000
   tttgagacca ggctggccaa cattgtgaaa ccccatctct acaaaaaata caaaaaatta 60060
   gccgggcgtg gtggtgggtg cctataatcc cagctacttg ggaggctgag gcaggagaat 60120
   cacttaaact caggaggcag aggttgcagt gagctgagat cacaccattg cactccagcc 60180
   tgggtgacag tgtgagactc tgtctcaaaa aaaaaaaaaa ttatatatat atatatatat 60240
   atatatatat atatatatat atatgaaata aatgaacaag aaatttagat acaggaaaat 60300
   ccaaagcact tggtaatgaa agaaaggtaa agtgatgtgt ccttttgcat ttaaaagaga 60360
   gcattaacaa attagagagc tgaataatgc tcagtattgg tgtggatatg gagactcagg 60420
   aatcctcata cactgctgat gggagtgccc actccctggg aatattttcc aaatatcatc 60480
   tcaaacatat cccataaagg tgacaggaaa gtgtgggctg actgatatcc ttcactgaga 60540
   gaggtggagg taaaatgaag tcactgcaca atatagagtt ggaagcaatg gattagatgt 60600
   ccacatagtt acgtggaaga atccgtaaga tacacacaca cacacacaca cacacacacc 60660
   tttgtgtata ttgttcctgg caggtaggca tggaggttta gaggctttct acatcacacc 60720
   tactgcacac agtaaatggc caggctgagc actgacttcc atgaagggag attgaaggta 60780
   agagattgaa gattgttccc tggtctggga ccctgcaact gaatatgcag aaaaaagtac 60840
   accccgccac cccgcttccc atctttccta cctgattaga atagcttttt cagaaaacgt 60900
   tggccagggg ttgtggctca cacctgtaat cccagcactt tgggaggctg aggcgggcag 60960
   atcatctgag gtcagaagtt ccagaccagc ctggccaaca tggcgaaacc ccatctctac 61020
   taaaaatata aaaaattagc agggcatggt ggcacacacc tgtcatccca gctactcggg 61080
   agcctgaggc aggagactca cttgaagcac agtgatggag gttgaagtta gctgagatct 61140
   tgccactgca ctccagcctg ggcaacagag tgacactttg tctcaacaac aacaacaaaa 61200
   cccaccaaaa ctttaaatct acctatggcc aaatgcctgc taaaatgagc acccaagaag 61260
   cagtgttcag gaaagtcaga tgaataccct aaaattagat gcaatgttgg ctggtcacag 61320
   tggctcaggc cctgtaatcc caatccttct tgggaggccg aggcgacaga tcgcttaagc 61380
   tcaggagatc gagaccagtc tggacaacat ggtgagaccg tgtctctaca aaaacgtaca 61440
   aaaatgagct gggagtggtg gcgcgcacct gtagtcccag ctactcagga agctgaggtg 61500
   ggaggatctc ttgaacccag aaggcggaga ctgcagtgag cagagatcat gccactacac 61560
   cccagcctgg atgatagagc cagaccccca tctccagaaa aaaaaaataa agagagagag 61620
   agatgcaata tttagggttc aacaagactg aatttctgac tcctttccct acctctccag 61680
   catgttagat tctgggtcct tcatcctaac cccctgttca tgccatagcc accctgtggt 61740
   accaactttg gaagcctgga tcttcatccc ctcatgataa tgagtgtccc atcaggtctc 61800
   catgctcagc ttggcaagag tatctgtctt ctcctcatgg gacggtcaca ttcacccagc 61860
   actgacaggt tccattccca ctagggtggc accctatatg gtctgagtcc aggccttcct 61920
   ggtccctcag taatctcagc atggtagcac aatcgaaaag ggctaggcac ggcagcacca 61980
   tttcccacca agaggtctga tggctcatca catagactga aggagattct gaagagcaga 62040
   ggtggaatga agaatgaatc gtgggctctg ctcttcctag gcctgtcttc ctctctcccg 62100
   agatgttagc taactcatga gagccagaaa ccaactgcag gctggcctca ggcacttagg 62160
   tagtgcttca gcctcagcag tccacattct aggaaccctc ataatatggg ttgaagtatg 62220
   cattcccaca aaaataaagt tgttgaagtc ctaaccacca gtactgaaat gggaaaagtt 62280
   cccttgtccc gctcgcatgg catgtgatag gagtgtggct aatttcttca gtgcctggct 62340
   gctcaaacct ctaggggaac attaagacgg gcaggttgtg ggtctccaac cccatgaccc 62400
   caccacagtg tctagggttg aatgtttaca gctcctgaag ccacagtggg tgtgtgttac 62460
   agggtgctct tttagttttg ccatttatag gcagctggtg ttaaccaact caattagacc 62520
   gtctaccttg tcccaaggac agaagaaggc tttctgtatc ccaggttctt gccttggtgt 62580
   accggaataa atcagaccac acctgggctt agagaaagag tgcaaggttt tattaagtgg 62640
   aggtagctct cagcagttgg gcaaagccaa aagtggatgg agtgggaaag ttttcccttg 62700
   gagtcagcca ctcagtggcc caggctctcc tccaaccacc ccagtcaaat tccgcctcat 62760
   tttgccaggc aaacgtttgt tgtgtgctct tctgccagtg tgctcccctg gacgtccagc 62820
   tattcgtgtc ttgtggcagg ccaggggagg tcttgggaaa tgcaacattt gggcaggaaa 62880
   acaaaaatgc ctgtcctcac cgtggtccct gggcacaggc ctgggggtgg agccctagcc 62940
   ggggaccacg cccttccctt ccccacttcc atatcattta aagggaccat gcccttccct 63000
   tcccagcact ttccccctcc tgtatcagga cctgtgaatg tggccttatt tggaaatagg 63060
   gtctttgcac ttcatcagtt aagataagag tgggctctaa cccaacataa agggtgtcct 63120
   tataaaaagg agaaatgtca tacacagaga ctgacaccta tagagagaaa atgtggtgag 63180
   tagacacagg gagaatcacc attcaagtca agcaatgagt ctggggatac cagaagctgg 63240
   gagagaaacc tggaacagat tatccctcat tgccttcaga aggaatcaaa cctgatgata 63300
   ctttgatttc agacttccag cttccaggac tgtgtgacga taaatatctg ttgttaagcc 63360
   aacgagtttg aggtactttg ttactgcagc cccagaaaac taatacagta ggtactatgg 63420
   actgaattga ctccccgtcg caaaattcat atgttgaaac cctaaccccc agtgtgatgg 63480
   tacttggagc tggggcgttt gggaagtcat tatatttaga caaactcatc aggatgtgtc 63540
   tctcatgatg aaattcatgc ccttattaaa agagacaaca ggccaggtgc agtggctcat 63600
   gcctgtaatc ccagcacttt gggaggctga ggtggatgga tcacctgagg ttgggagttt 63660
   gagaccagcc tggccaacat ggtaaaaccc catgtctact aaaaatacaa aaattggcca 63720
   ggtgtggtgg tgcacgcttg tactcccagc tacctgggag gctgaggcag gagaatccct 63780
   tgaaaccagg aggtggaagt tgcagtgaga tcacaccact gtactctagc ctgggtgata 63840
   gagactccat ctcaaaaaaa aaaaaaaaaa agacaataga gccaggtgct gcagctgatg 63900
   cctgtaattc caacactatg agaggctgaa gcaggaggct cgctttagcc caggagttca 63960
   agaccagctt ggacaaaata gtgagacccc caacttctaa aaatttaaaa aatgaactgg 64020
   gtgtggtggt acacatctga ggctccagct actctggagg ctgaggtggg aggattgctt 64080
   gagcccagga ggaggctgca gtgagccatt gctgtccagc ctgggctaca cgagaacctg 64140
   tctcgggaaa aggagaaaac agtgagacct ctttttctct cctccttctc tccactgcct 64200
   aagccctaca agcacaaaaa ggacaccaca tgagcacata gtgagaatgc tgctgccacc 64260
   aacaagtcag gaagagagcg ttcacctaga aactgaattg gccagcacct ggatcttgga 64320
   cttctgagct tccagaactg tgagaaagtt attttttttt tagcgactaa gtctatagta 64380
   ttttattaca gcagctcaag gtaactaaca tagtagaagg gatgaattat ggagatcaca 64440
   agtccacgcc tccagaaaaa gacttcccta aaaattagtc tgagcaaaat tcgaatgatg 64500
   aattattttt aagaactttt aagggatctg acaagtttgc aagagctaga gaatgcttta 64560
   caacgtgata atagaatgct ctgtgatgac agaaatcttt ccacactgtt caaaactagc 64620
   tactggccac ttgtgactat tgtgcacttg aaatgtgact ggtgtctgag gagcagaatg 64680
   tttaatttta cttaatttta attcattaca atagctacat gtagctaggg gctactggat   64740
   tgaacagcac agctcgagtc ttttagaggg agacaggact caccaagatg gatgctggtg   64800
   gccaagcagc aatggcaggt agtacacaca caagaggcag atgatacaac acatccttcc   64860
   caaacctgga gataagctca ccccacaatc ccgccgctga aatagagttg atgttaccaa   64920
   tgtgcatttt tatgtccttt tccatacaga aagatcattc agcaagtact atggtactta   64980
   aaaaacaaca ttcaattcat tattatgaca aaattaaatt aatagctctt ccttaaactt   65040
   ttaaattcaa tttacaatgc ttactattgg catttattaa tctaccaatt ttttcccata   65100
   gaacccatag aacaaataat ctaccaaatt tttaacattc atttttggca aggcttttgc   65160
   aatttgacga actttaagaa gaaaacttat aaattgcaat ttttaaatct gacatactgg   65220
   acttttaaag tatccaattg actaatgaac aaaactgctc caaatttttc aattcttaaa   65280
   aatcttaaga caatacttaa tatggcaaat cttaacttct taaactttgt aagaatgcta   65340
   atcaacttag attggtataa agttgagtta aaaatcacag gatacatcat ctcagctata   65400
   agttttcatg agttgagttt ttacaatcac ttgaaatgct tagaatagga aatacgtata   65460
   aattatttaa cataaaatat tgttacaaaa cctctggagt gtcagtttct ctggccagac   65520
   tttatgctgc agcacctttg cctgagttct tgtcctgcat ccaggaagaa ttaggtacag   65580
   aggcaagagt caagaagatt agttttccaa tagttcagct cacctagtta actcctgttc   65640
   acaatcttca aagttatcag aaacctgcaa ttgagggtta taatccattc tttgcagagt   65700
   ttcaaaacaa gacaacattt gtctatgaat gttaaaatgt cctagggtag tcacagtcaa   65760
   aaacacaatt gacaaagaaa tttagtcacc tctgtgattt acaatagcct aacacaataa   65820
   ctctaattat aactgatgac acaaactcag atatcagaac tctagaaatc ccctataatt   65880
   ttggaacaca cattcacagt tttcactgaa atatgacctg aagatcaaat atcaccttat   65940
   ttcaacaatc ctatataact aaacgtgtca aatgatcctg tttacctctc ctttggatac   66000
   tccaggggcc ctctgtagca tccaaaagtt aggggttagc aaagacaatt ttgaagctgt   66060
   aaaggctcaa aacacttaat gaacctctag tcatatctgt tctctactca ctaaatgcta   66120
   gtagcacctc tcagttgtgg ctaagctggg aggatctctt gagcctagaa gtttggggac   66180
   gcagtgagct atgattatgc cactgcactc cagcctgggc aacaatgcaa aatcctgtct   66240
   caaaaacaaa aacaaaaaac aaattgccta tgctgtggtt atctcacaat taataaaaag   66300
   gaaaaaaaaa gtatgcagtc tttgtaggtc cttggggttt gttggaactc agaaaacaat   66360
   accccaaaat aaagaccgca gaagccaaag tttttctctg atcttctcct gccctcctgt   66420
   ctctgagtcc cattctcccc ggagtctagc catagaaatg agaattcctc ttcctcaagt   66480
   taggtcatag aaatcaaaac accttttccc cagagcccag ccataaaacc taaaaatatt   66540
   actctaactt tccctctgtt tttctgtgta aaaactggcc ataaagaaat tatctgaact   66600
   accttatttg atcatagatc accagaccgc attccagaga ggatccagaa ggaaggaatg   66660
   ctgcacagag aggcgaagaa gaatctagac agacaggcct tgctgggttt ccctactctg   66720
   tttattagca atcctatttc tacacggcgg cccatacttt gttgaatcta aaaaataaaa   66780
   atggacaatt tcccctgtac atgttaatac acattaataa attggatata aattggataa   66840
   tttattaata tacacattaa taaattggat gcagccgggt gcaatggctc acgcctgtaa   66900
   tcccagcact ttgggagctg aggcgggcag accacgaggt caagaccacc ctagccgaaa    66960
   tggtgaaacc ccgtctctat taaaaataca aaagttagct gggcgtggtg gcacatgcct   67020
   gtagtcccag ctactgggga ggctgaggca ggagaattgc ttgaactcgg gaggcggagg   67080
   ttgcagtgag ccgagattgc gccactgcac tccagcctgg tgacagagtg agactccgtc   67140
   taaaaataat aataataata ataataataa taataataat aataaattgg atgcatttta   67200
   tcctattaat cttcctcttg tcggtggttt tcagcgactc ttcagaggcc aaagagtaag   67260
   ttttccctta gcccctacag gttcttatgt ttaatttgtt actctcattt aagacataat   67320
   taaagtggct tctccatgaa gattatttct gcatccatta tttggtaaga ttggccgttt   67380
   tctcctttga tctctacttc acactgaccc acataaaaca tcactgcctg tttttttgtt   67440
   gttgttgttt ggagacggag tcttgctctg ttgcccaggc tggagtgcag tggtgtgatc   67500
   tccgctcact gcaagctccg cctcccggat tcacgccatt ctcctgcctc agcctcctga   67560
   gcagctggga ctacaggcac ccaccaccaa gcccggctaa tttttgtatt tttagtagat   67620
   acggggtttc actttgttaa ccaggatggt ctcgatctcc tgacctcgtg atcggcccgc   67680
   ctcagcctcc caaagtgctg ggattacagg agtgagccac tgcgcccggc cccgtttttt   67740
   tttttggttt ttgcatgtct tctccctttt actgtaaact atttccacta ccagcgtagt   67800
   tatcatttct actgcttaat aattgttttg gggaagtgaa tgcatcaacc cacatgaatt   67860
   tcttgtctat ttgacaattt attctcttta ggaatagtat taactcctaa ggtcctggga   67920
   gccagtctct gtacttggct gctccagggt cctacttcag tttcccagct tctcagtact   67980
   gtcactgtca attgtgggta ataattattt ttgtccacca aaagactctg tatgtgaatg   68040
   agttttgaaa tctgctgagt aatacagtgt caacccagtt aatgatttgc cgggcggctt   68100
   gatcaggggc tgtccaacta ccggcatttt gatttggagc gtcatctagt gtctgaaagc   68160
   acaaacaaca tcctacattg taaatgcctt tggctacaga gattgaaacc aaagcaaacc   68220
   tatgttttga attgttattc ttcagcagtt ctgctagctt tgaaaaatct aaaagttaaa   68280
   aaaaagcttt atatttcatt ttctgcctaa actctttaaa attgctagtt gacaattaga   68340
   tattttcaat ttaatgaaat ttttttttag ttcacagatt aatacacaat gggggagggt    68400
   tcttattctg ttggactttt acataacctc cactttagtg cagtctgctt tatggggtct   68460
   tgtttgaggt gtgtgtgtgt ttaagggaat gtggtttaca atcaaaatat tgggttgctc 68520
   ttaggcacat tgtaaagtca cacacctgta ttcttattga tacataatga ttaataacat 68580
   tattattaca gcctgatcac catcattatt gatatatcta aataatgaat tttataattt 68640
   tgcttcctgt caggcaagag ccaatttcag tgctaccatg tttgtatagc agtatttatg 68700
   tctgtcatcc tcagtcattt tacttcactt gttcttagcc aaacggccga gaagcgatgg 68760
   tcattttact tcaaaaatga aaagaattaa tatttttacg tttcccttaa agaccctatg 68820
   tttaacctcc actcctgggt aaaatggtct agtccctcct tttcatatca tctctgatat 68880
   cttttgcaca gccactatta cctaccgttt tctagatccc tattcttcaa acaccaccat 68940
   gaaggtagag cctgtctgaa ttattttctt gtcccctgaa ctcagtacat tgttaggctt 69000
   cttgaagatg ttgatcagtt gtttgtggag tgaatgaatc agctagcatg atttttctag 69060
   accactgaga caagtgtcta agacacttgt tccttcccat gttcttgcct gcctgtgcaa 69120
   tccatgcagt ctcatggctt cccagtgcct cagaattatc ccctgtcaaa caggcattat 69180
   aatttctgtc cactgaaaag gacaaaaaac taagtgtata gctagaagtt aaaaattacc 69240
   ggccaggtac tgtggctcac tcctgttatt ccaacatttt gggaggctga ggcgggcaga 69300
   tcacctgagg tcaggaattc gataccaggc tggctaacat ggcgaccccg tctctatcaa 69360
   aaatgtaaaa gttagccagg tgtggtggct cgcacctgtg gccccagcta ctcaggaggc 69420
   tgaggcagga ggatcgtttg agccctggag gttgaggctg cagaaaaata ggaatatact 69480
   ctctttcaag agttcgtggt tttgactgcc acctagcgta catcagaaaa accgcatgac 69540
   ataggaaatg cctgtgacag aggggtaagg tgagagaggt tgatgaagaa tgtattgaag 69600
   gagtgaaaac gcttccatcc ctctacttac taaatatatt agttaagtag ttggggcata 69660
   ttttaattca tgcattttgt agatagaaaa acaaaagttt tattctgttt gatttagttg 69720
   atactttaat atgtgtgtgt ttaggatgca tgatttataa tcagtctgca gcacttcttg 69780
   gagaagtctg aattctcatt ctccatttcc ttattggcaa cgtgagaatg attacaatgg 69840
   tggttgtctc atagaatgca gggagtcaga atgaaaatag tccatataat gcctggtgca 69900
   gaggaagggt tcagttaact gtctgtatta atattactga taacagtcat gacaaacaaa 69960
   agcttaacaa caacaccacc aacaacagtt gcagaattga gccaccaatt tgcacacaag 70020
   attgtaggta ggatgtttta gaaaagttat tatttaatat atgtatatat ttttgtactt 70080
   aaaatatgtc agaggttgtt ctaagaacta tttaaatgtt aactccttaa tcctcataat 70140
   gacccatgaa acaggtaggc ttattattgt ctctttacat gtgagaacac tgagacacga 70200
   aaaggtttat taactcaccc aaagtcacac agctggtaaa acggcaaaat tgaatttgaa 70260
   ctcagacatt ccaggttcca agacagtcta attattcttt tgactaatat actaagctgc 70320
   ctctgtattt ttccttgatt actttgtaaa agtatgagga aaatataagt gcttcaagta 70380
   accatgaaaa atataaacaa tctatgtatc aactgaagca taattacaaa tcctttgata 70440
   agcaaacata ataaaaattt gatatcaatc aaaactttca tgtaatgtaa gcaggttgag 70500
   atgaattcta tagtaaaaaa gtgcagagtg ctggaatacc atgctcctaa tatattggct 70560
   aggcacacct gcctgctatc aaaggtatgc acacaccttg gatacagaaa gttgggactg 70620
   ggtagttatg tgagtgtcat cagaattctt tcccacttgg gaaagaattg tccatcataa 70680
   gcttggatga tggacaagga gtgagctccc agaacagtga tgtggggata catcctcaca 70740
   tcacagtgag aatgagtgtt ctagactgtt tacacaccta ccactcctaa atgcacacat 70800
   ataattgctt gcacacacac acatacacac tcatctcttc tctggtggtc cagctctatc 70860
   tcttatcatt aggcttcttg gggctagtac ctagggcctg tatcctttca gaggcagcta 70920
   agggaagcac acataattag aaagaatgaa ccagcttgtt ggatttggtc tcttcgcatc 70980
   cagccctcca agttaaggag agtaccatct ttcttagggt caccaaagga aaaaaaaaaa 71040
   aaagaaagaa acagaaggat atcatacagc aaggatctaa tgcaaatatg cctcaaatga 71100
   gaggctactg tgtgctgatc ccaatcccag gaactgtatg cacattatct aatttaatcc 71160
   tcactgtatt tctgggagta ttattcccat tttacagaga aggaacttgg cagggtaacc 71220
   aagctcatga atggagaaac tgggattaaa tataaagctt ccttgctcca gaactgctgt 71280
   ctttctgctc ttccacacta ccagctcagc tgtgctctct acatgcaggc agttttacaa 71340
   gtttcagatt agcctgggac ttccagggtt ttgaatgggt tagggaatgg ggaacttttg 71400
   ggtttacttt ccattttttc ttcatacata tgtaatatat aacataaatc tatggtatat 71460
   atgataaata tatggctaca tatgaactat ataatcacat atatgcatta taaataaata 71520
   ttaattttat aatattttaa aggttatcaa ataaatatta atataaataa ttaaataatt 71580
   aatactcagc tttgttttcc aaagtgataa atgcctatat ttagcaaaat attttttgga 71640
   ggcctgatag tttttaggag tgtaaagaag tcctgatatc taaatgttta agaaccacta 71700
   ttttaggctg ttgtcttctg tcttattttc ccagctagac tggtaaatac ttgaaggcaa 71760
   acgtttagcc agcacattaa cattttatgt ttttattctt ttgtgctctc agtggctgtg 71820
   tcttttctat cgatttctca cactgtatga tggttatatt tgtctgtatc tgtcccacca 71880
   ggtataagtt cttgagagga cacactgcta ggctgatctt agtttttatt atttctcctg 71940
   gtgtcctgtg cttaacaagt gctcattaag tgtgtaaaaa cacagcacag taaaaaacta 72000
   gacattaaaa aataatgtca accaatctat tgaaatttgc atttccatgt ttcttccaat 72060
   atagtcattg tgtcaggtta tgtacttatt ctgatgaaga ctattgccta atatacgttt 72120
   gcatcttgtg ctttataact gccttcatat agacacagat tgagaaggtg taaaaatgtg 72180
   catatcctca caattgacaa attcttatcc tttgagggta ggtttgactt tctgaaatgc 72240
   tttgacatca tttgaaagaa gcttgaagaa taagatagct gttaatgacc cagtttccta 72300
   tgtcacttat acaattataa tggcaatttc aaaatgttag gtaaatatat tttgcaatat 72360
   attgttcctt ttgtaatact ctctatgtat ttatttatat ttttaaattt tatatttatg 72420
   tatttatttt tctggacaga gtcttgctct gttgcccagg ttagagtgaa gtgttgtgat 72480
   catagctctc tgcaacttca aactgcttgg caaaagtgat cctcctgcct cagcctcatg 72540
   agtagagtag cgggaactac aggcgcatgc cactgcaccc agctaatcac tatttattat 72600
   gctcctactg tgtgctttag tatattttct gttgttttct gcaacccatt ttgagggcgt 72660
   gttagggaat acagatgcag taactttcgt ctcagccctt gaggtgagga aatatttagc 72720
   ctcaggttta atctaattgt tggccatttg ccttcaaaga ttgaaatatg agcaaaactg 72780
   tggctctggg ttatatgtta aaaaaaagtt tatggggctg aagccaggca acagacaaga 72840
   gcccctacaa tcttatttag gctgaaaata tcctggagtc cctgtattgt tggtctcaag 72900
   cagatagcaa cactaacact tactctttga ggcaggcact gccagtgggg tggctgttat 72960
   tattagcttc attaattggt gagtcaggaa aaaacagctt taaatcattc aaagttctgg 73020
   cctatacagg atttagtaat attaggttag ctacatccaa aagatgacag aaccctactc 73080
   taaggctggg cttggtggtt cacacctata atctcaaaac tttgggaggc tgaggcagga 73140
   ggatcacttg gtgccaagag tttgagacca gcctgagcaa catagtgaga cccctgtctc 73200
   tatcaaaaac aaagaactct aattggcata gtagaaggaa aaagtgaaag aaaaaccagc 73260
   tgtcaccctc attccttaca cctgtcctaa caactcctct cactatcctt tgaatatatc 73320
   ttggctgttt gagtctctct ctagccccat tactgctgtt tggacttgac attttgctct 73380
   gcatttttaa cttttctacc agggtttcca gaccctgaag agtgtggcat gaaacaaaac 73440
   tagtcaacct ataatattta tgatgtgtgt gtaaataaaa gaatacacaa tatattgcat 73500
   tacaatattt taactgtgtc ctcaatttgt ttgtggcttt cttgaggaca tcagttttgg 73560
   gtgggacgac cacatcctta atctgaactt tcccttggag gtcattcttt tttttttgaa 73620
   atagagtctc gctctgtcac ccaggctgga gtgcagtggc gcaatctcag ctcactgcaa 73680
   cgtccgcctc ctgggttcaa gtgattctcc tgcctcagcc ttccaagtag ctgggattac 73740
   agatgcacgc caccatgccg agctaatttt tgtattttta gaagagacgg aatttcacca 73800
   tgttggtcag gctggtctta aactcctgac ctcatgatct gcccacctca gcctcctaaa 73860
   gtgctgggat tacaggcgtg agccaccccg cccggccaga ggtcattcta atagactttt 73920
   tttttgttgt tgctcacagg cttgttcaat cttatttcaa aatttgagaa atacagtttc 73980
   catggaacac caaccagata tcaggttgct atggagttga tagtcaaaag ctttgtatct 74040
   tccagttttt cagaatggct tctaaaggtt ctgattcaga gctcttaggc gaaattgaac 74100
   aaccaagtgt caaagtacaa cattcaggaa gttaaaaaca tgactgacat atatgtacta 74160
   tatatagtga gcttgtgtat gtgtcaatga atgatttaat tcattaatga aggaggaagc 74220
   agaatcacaa ttaggtcaaa ggaagatacg ggagaataaa atatgtattt ggtcagggaa 74280
   aggatgtata ctggaagagg aagggaaaat cagatataaa gttgtttaat gacttattag 74340
   gcaatacaat aataactttt agggtcattt tttctatatt aagaattcat ttccatctct 74400
   atgacaaaat ccttattaat ttattaaact tctacaagtg aatgtttact tttagatagt 74460
   ctggacccaa taaaatgtaa acattaagtc agagttactt tcacgtagga cagtgttgtc 74520
   caataaggta ccactagcta cacgtgatca ttgaccattt ggactatagc tagactgatt 74580
   taaaatgttc taaaagtgta aaatacacac caggttctga agatttatca tttaaaaaag 74640
   aatgtcaact gtcttttttt ttagcttatt tattatatgt tgaagtgata atagtttaga 74700
   tatattaagt taaataaaat atcttaaaat taattttact tgtttctttt cattctttca 74760
   atgtgaccac tagaaatctg gaaagtattt atgtgattca cattctattt tactgtctag 74820
   tattgcctta catcatcagg taccccataa gtaggctttt tagataattc tctaatatag 74880
   cttggaagga tatggagaaa tatttttgcg ttgcttttaa gttttgcata actttttcaa 74940
   cacactttat aaaggatcta gaaaagggtt ggttacatgt ttctctgtct tctggcctcc 75000
   accatgttgc caggaggttg gggacaagat tctgggtggc tggatgtcct aatggcttga 75060
   ggtctggact tgagatttgc atataaagag atgtgattag attgagtcga ctagaaaaat 75120
   catattagag aactgaatca cagcgattaa atttacatgt cgatttataa accaggacac 75180
   caatttatag tgaaagaagg tccagttacc tggtaatcaa gacgtttcat agctattttc 75240
   atgatggata tacttagctg agttttaaat gagaaggggg ttcattgcac atagaataag 75300
   atctaagtga aatgtttatt ttattttttt ttttttgaca tggagtcttg ctctgttgcc 75360
   caggctggag tgcaatgagg caatctcggc ttctggagtg caatgaggca atctcggctt 75420
   ctggagtgca acgaggcaat ctcggctcac tgcaacctcc acctcccggg ttcaaatgat 75480
   tctcctgcct cagtttcctg agtagctggg attagagttg cctgccacca cgccaggcta 75540
   atttttgtat tttttttagt agagatgggg tttcaccatg ctggccaggc tggtctcgaa 75600
   ctcctgacct caggcgatct gcccgcctca gcctcccaaa gtgctaggat tacaggcgtg 75660
   agccaccaag cctggcctaa gtgacatgtt cttatattgt tcctttcttt cttttttttt 75720
   cgactgagtc tcaccctgtt gcacaggctg gagtgcagtg gcgtcatttc ggctcattgc 75780
   aacctctgct tcccgggttc aagcgattcc cttgcctcag cctcctgagt gccaccaccc 75840
   ccagctaatt tttgtacttt tagtagagat ggtgtttcac catgtcggct aggctgatct 75900
   caaactcctg gcctcaggtg atccgccccc gagtctccca aagtgctagg attacaggcg 75960
   tgggccacgg ggcccagcct tatattattt cttttactac aatatattag tatgatgcag 76020
   gtgcttcaat tgtttataca ctttccataa ttttgtataa ttcttatacc ctgtcactct 76080
   gaggaatagc cggtctaagt gtttttccac cactgctaat tcatccatca ctaatctcat 76140
   tagactgtta attcccagag gacataagca cacaagcaga caatgtttac aaatgttgga 76200
   caaatgttat ttaataaaac aatggggtca cccttagtct aaaagatgtt tcacttttca 76260
   tttgtcattg aactcttatt tgtaggttcc cttttgactt tcccacaatc taaggctgtt 76320
   ctctttaaca catattttca tgaaaacata tatttgagca gaaattgttg gggagttgta 76380
   atattacctt tgtccctaaa tatgaatcta taattatatc aaatatatgg gcagacaatt 76440
   tactttgcct ttaatctcaa gaaaaaaata gcaattactt ggggtcggag agtaaaataa 76500
   gaagtagtga accttaaagt agcaaacttt agaacagaat agtttcagag gggatgagaa 76560
   gaggtgattt ttcagctcat caacaacaga tcttataata aattacatgt tctggtactt 76620
   ttcttgtctt tctgtgttaa attttgctat ttaaaaaaat aaatttcaaa tacattgttc 76680
   atcttaaaag tcaagagtgt gttttattaa agtcagttgc tttatttgca actcaaaaga 76740
   tatatttgag ttcccaactg gagattgtcc tatatggtaa cttgcgtaag gtatggttac 76800
   tgaaagtaac ctacaatttt catgggctga aattcatttc tatattgcag cgtacaaaaa 76860
   taaataaata aaaaatgctt gttttctttg aaaacatatt atctcagtgc ctctaactgc 76920
   caaatctatt ggcttttttg caggcttaag ggctctccct tgttccttta tgatctctat 76980
   cttgagggcc agacctcctg ccttacacaa ctcagagggg gacctcagag ctctttaaaa 77040
   agagcccaat ttctcgcctg tagagaagtg aaaaggatgc cccaccccca tctatgaaaa 77100
   gagggatttg atagtttcaa tgtcttcaaa tcaaagattt aagtctgtag ccccccacca 77160
   ccccggaccc tagcaaggct catgaacccc ctcccatccc gccctaattg ctttggactg 77220
   gccgtggaat ccttgtccca gtccacagtt cctgtgcgac tgcacgaaga attcacagag 77280
   gacctgtgtt acttcccttg tgaagaaaca gaattatcat gaaaatttag gtggaaacca 77340
   tttcgctttt ttcttcaaaa ataagggaag catgtgccca accacccctg ggaaaaagaa 77400
   ccttcagggg caaaggagcg aacaggtaat ttataagaaa aacagaaagt ggtctctgac 77460
   tgccccagac ttccttcgga gttgggggaa ttggggacgc ctggacgcgt tgtttttgtg 77520
   tttgtggaaa aaataaatga agagcatgaa gcccgaggct tctgagatcc tttcctgacc 77580
   aaacccaagt gatttggtgc ggggaatttt aatatttttc cccttttgtg aggtggaaca 77640
   aacacaactt gggagcagcg cagcggctca gagcctgcca gccaggcggg cgaccagagc 77700
   accaatcaga gcgcgcctgc gctctatata tacagcggcc ctgcccaggc gctgcttcat 77760
   cggcgctttg ccacttgtac ccgagttttt gattctcaac atgtccgaga ctgctcctgc 77820
   cgctcccgct gccgcgcctc ctgcggagaa ggcccctgta aagaagaagg cggccaaaaa 77880
   ggctgggggt acgcctcgta aggcgtctgg tcccccggtg tcagagctca tcaccaaggc 77940
   tgtggccgcc tctaaagagc gtagcggagt ttctctggct gctctgaaaa aagcgttggc 78000
   tgccgccggc tatgatgtgg agaaaaacaa cagccgtatc aaacttggtc tcaagagcct 78060
   ggtgagcaag ggcactctgg tgcaaacgaa aggcaccggt gcttctggct cctttaaact 78120
   caacaagaag gcagcctccg gggaagccaa gcccaaggtt aaaaaggcgg gcggaaccaa 78180
   acctaagaag ccagttgggg cagccaagaa gcccaagaag gcggctggcg gcgcaactcc 78240
   gaagaagagc gctaagaaaa caccgaagaa agcgaagaag ccggccgcgg ccactgtaac 78300
   caagaaagtg gctaagagcc caaagaaggc caaggttgcg aagcccaaga aagctgccaa 78360
   aagtgctgct aaggctgtga agcccaaggc cgctaagccc aaggttgtca agcctaagaa 78420
   ggcggcgccc aagaagaaat aggcgaacgc ctacttctaa aacccaaaag gctcttttca 78480
   gagccaccac tgatctcaat aaaagagctg gataatttct ttactatctg ccttttcttg 78540
   ttctgccctg ttacttaagg ttagtcgtat gggagttact gaggtatcag acgaattggg 78600
   tgacggggtt ggagagtggc cgtggtgagg ttacagcatt taaaccttta ttgcggcttc 78660
   taggtccctg accggaggct tttctcgctg gcggatggtt ttgggatggc agtcccgccc 78720
   caggcctgtg aacggcagaa aagaccgcaa aacaagagcc agtttcttag tctaaaggga 78780
   tgtccggatt ggactaaaaa attttcaaaa gtcccgccct gctcccgggt tggtccgttc 78840
   ttctagtaca tgactttcat tctgtattta attggatggt ggaagacgtt gcttattctg 78900
   tgttttttgc tttactgtga cttaaaagtt ttgcctcttt tctctttata ttaatgtctg 78960
   ggatttcgga cgctttccat gttgttggta gtcaagttga tgtctcctgg aggtagtggc 79020
   aacatccagc cctgggagga gagtgcgtgc aggtaccttt gtcctacatt cctctgctgt 79080
   taatttctca ttcctgtggc aacgaaggaa tgcatttaaa aaacagccac aacagcggca 79140
   atagcccttc ctccacccaa ggcaatcgtg gacctaggga gttttttgtg ccacataaca 79200
   tgtagccttc cgctaaactg acaggtttga gcgtatcgat tttgagcgta tcgaaagcac 79260
   aacttttagc cagccatttt gtcctcgcat gactacggtt gcttatcctg tttagacaga 79320
   cagcaacatt taaaaatcga agttccttta aacgtatttt gtttggcagt ccaaatgttt 79380
   ctatgcagaa aacagtattt gtactattaa ctatgaagag tgtatggata aatgggagac 79440
   atttctaata aaggccttcg ttaatggttc cctctgtttg acatccatgg tgcttctgaa 79500
   tacagaaagc ctagcgtctt atattcgctt cttttaaaat ctggtgggca cattttggtg 79560
   agacctaaat tatggggact ggggcttctg gagataagct gctcaattat tctaccatct 79620
   ccacaatgat taatatagtg agttgatttg ttagtgatag tgaccacgga ttcatcccaa 79680
   gaaagagaaa ggggagggag gcaagcagag agacaggaag acagaggcag ggaagaagga 79740
   gaaaacattc tcccatggtt taagtaattt tgtgttgtta attttacatt acaacacggt 79800
   ttaacatggt gaaccctcta ttttggtgta aggtttaaca tatggacata tttttcccaa 79860
   gaccatttat gaactttcat ttctgcttcc cccttcttcc tcccgtgcca ccctccacgc 79920
   tcctatcaat tttggctgtt ttgtcatagg ctaatacgct ataatttcat ggacagttgg 79980
   actgtcttag gtttctcagg tttctatttt gttcctttag tcattcccac aattcttaag 80040
   gtagaattgt attgttttaa acattgtgtt gtgtgctatc ctcaatgctg agatgattat 80100
   gtgacaaatg gcaagtgttc aactaatacc taaatctgta gtatcttatc aagcctaatg 80160
   ctacttcaca atgcctactc cattcacctc actttatctc attactggca ttctgtcatc 80220
   tcacatcatc acaagtaaaa cggtaagcta ttttgagaga gatcacagtc atataattta 80280
   tatttatatt tatttattta tttatgagac ggagtttccc tctgtcaccc aggctggagt 80340
   gctgtggcac gttctcggct cactgcaacc tccgcctcac gggttcaagc gattctcctg 80400
   cctccgcctc ccgagtagct gagattacag gggcctgcca ccatgcccgg ctaatttttg 80460
   tatttttagt agagacgggg tttcactaag ttggccaggc tggtctcgaa ctcctgacct 80520
   caggttatcc gcccacctca tcctgccaaa gtgcttagat tacaggcgtg aaccaccgtt 80580
   cacagactca aatcattttt attacagtat attgttataa ttgttgtttt attatcagtt 80640
   attgctaatc tcttacagtg cctgatttat aaattaaatt catcattgcc atgtgtatat 80700
   agaaaaaaac agtgtatata cggttcagta ctatctgtgg tttcaggcat ccactggggg 80760
   tgcagtttat taaacatgca tttacattag tctccccttt gggagactaa ttaactgaga 80820
   tgttgtaacg tgactttaat agcagataga gctaattttc tctcattact cttctttttc 80880
   agaattttcc tggttattcc atttttattt tttccatatg tatattaaga tctcttccac 80940
   ctcctcctgt ttctccatct caacatcaaa caattaaaaa aaaaaaaaag gctgggcgcg 81000
   gtggctcacg cctataatcc cagctctttg ggaggcctag gcgggtggat cacgaggtca 81060
   ggagttcaag accagcctcg ccaagatggt gaaatcccgt ctctactaaa agtataaaaa 81120
   ttagccaacc atggtggcag gcgcctgtaa tcccggctac tcgggaggct gaggcagaga 81180
   attgcttgaa cctgggaggc ggaggttgca gtgaggcgag accttgcact ccagcctggg 81240
   tgacacagcg agactccgtc ataaaaaaaa aaagccggaa gcagtggctc acgcctgtaa 81300
   ttccagcact ttgggaggct gagtcaggca gattacctga ggtcaggagt tcaggaccag 81360
   cctggccatg aaaatacagc ctggccatga aaacacacaa taaattagct gggcgtggtg 81420
   tcacacacct gtaatcctag ctactcggga ggctgagaca ggagaatcac ttgaacccag 81480
   gaggcagagg ttgcagtgag ttaagatgac gccactgcac tccatctggg cgacagagcc 81540
   agactctctc tcaaaaaact aaataaataa aaataaagtt atggtacatt gaacttctgt 81600
   gttcctttct cccttagata ctttcatggc tacccattta attgatgttc ttatcatctc 81660
   caagagttag tcaggagagg aatcaaccca agcaaaaata gctgattttc taattttcct 81720
   tcaatgccct ttggggtctt aatccatttg atttatgtac tttcaattaa tcctaacctc 81780
   gaatgtcttc tgcaaacatg tttccacaga tgaaactcgt caaatgaaac acattccttt 81840
   aatttataga gttaaaaatt agaaaaattt tcaattctat ttggccttta gattcagtct 81900
   tgcatatgtt ttctcaattt tgttcatgct ctttagtttt gttttattcc atcacaattg 81960
   ttcacatagc ttactggctt aggtctaatg aaccattcat ttggaaatta aaattggcca 82020
   ttttaagatg aaaaagattc ttgcctcaat tttacttagt ttttgaaact gtcaatgagg 82080
   acacatgttt ttctgtactc ttagattcac taagtagtgt cttgcaaatt taactgacaa 82140
   aggacagatt aacatgcgaa aaaaagagca tgcaatttta ttagtatatt acatgcacag 82200
   agttcccaaa gaaaaaaaaa ttgaaacctt aaaaacgcgg ttagactcac agacttatac 82260
   accattccaa caaaggaaag ggagtttgca cttcatggga tgacgaattt gggaatgtga 82320
   caaggaaata aatacatggg caataaaaac catggaagat aaaatgaaag atagaaataa 82380
   ttgtagtaag gtttgttttt gcagagtcat ctcagtgcca accttccata tctagtgata 82440
   agaattgctc tctttttcct ggtatagcag ttggggacac ttttacaagg gaaatttctg 82500
   tcaccttcac aaagggaaat ttgggtaaag agaagacaga gacctcttcc tacacctgtt 82560
   gattttcaat tgccttcagc tgaaaataac ttttatgcca aagtagaata atttgggggt 82620
   gacatcctga tattcttcaa aacttatatt taatttcaca ttagtaatta tatcattttt 82680
   gatttttaaa ttagttttat aaaataattt tgaaaaacgg taataatatt caaataattc 82740
   cagaaacact gctgataagc caaaaacatc aatgaatatt gcataaacaa ctgataattc 82800
   aaccatgaaa atttatgaca ttgttcttgt gtgataaaac tatgagtaac ataaaaacta 82860
   gaggctactt gtaatgcatt attccaaact ttctgttttt tatttattta tttatttatt 82920
   ttgagacata gtctctctct gtcacccagg ttggagtgca atggcgtgat cttggttcac 82980
   tgcagcctcc acttccccgg ttcaagcaat tctcctgcct cagcctcctg agtaactggg 83040
   attacaggca cctgacacca aacccggcta atttttttgt atttttagta gagacggggt 83100
   ttcgccatgt ttgccaggct agtctcgaac tcctgacctc agtgatccac ctacctcggc 83160
   ctcccaaagt gctaggatta caggcgtgag ccaccatgcc cggcgcatta ttccaaactt 83220
   tcatacacag tgctatcatg gctacaaatt gaagtatcat attatacact cctaggcaaa 83280
   gctctggata ttttggctat ataagcctga gggaaatgta gtaaggacat tgtggttgaa 83340
   attcatacca gagatgaaca ggcccagtgc aagacagaat tacatcacta aaggatatca 83400
   gaagagaata gggatttagg gtacagtggc aacaacagtt ttgggaacta gcattttttg 83460
   agcacttatt tacaatatgc caagcactgt tgctgattac tctatattta ttttcaaaca 83520
   cattcttgtc acagcacttt gaagtaagtg ccattgtcat tcccacttca gggtgaagga 83580
   ctaaagcttg gtgtcattaa ggatgtagct agttagctgt gtgtgtgtgt gtgtgtgtgt 83640
   gtgcattttt ttttaaattt aaagtcaata aatttttatt tgaagaattt cacatcaagg 83700
   taaactttgt tcctctaaag agctggagtc aaaatgtatc ttcaaaagat tcatcttcaa 83760
   gttagccctt cttaatagaa ctgatgctta atccacagtt gtcagcccac agttctttta 83820
   ttttgacttt tttttttttt tttttttgag acggagtctc tcactgtcac ccaggctgct 83880
   gggcagtggc gtgatctcgg ctcgctgcaa cctctgcctc ccgggttcaa gtgattctcc 83940
   tgcctcagcc tccttagtag ctgggaccac aggcgcatgc catcgtgctc ggctaatttt 84000
   tgtattttta ttagagacag ggtttcacta tgttggccag gctgatctca aactcctgac 84060
   ctcatgatcc gcctgccttg gcctctcaaa gtgctgggat tacaggtgtg agccactgca 84120
   cccggcctta ttttgccttc tttaatctcc atttgaacat acacatactg atgaaaacta 84180
   caacattctt caccaaaaat ctttgggatt taatttcttc aaccacttta ctttggggtc 84240
   attttaagat taggtgtatc tgcctggttc tcaatttgac accctttctc tctaaacatg 84300
   aatgagttcc aatcatattt attcctaagc tatcacactc aaatatacta cagatctgtg 84360
   gaatatgcca aaagttaagg tgaaaaatta aattattagg tatttcatag ttttgctagt 84420
   ttttgatctg tgagtgaata taactatcct ctatgtcctg gcactgttcc tcagaaacat 84480
   agggtccaca tatgtaattt taaatttttt aataggcaca ttttaaaaag tgaaaaaaga 84540
   aatctatttt aatgatttga atccagtgta accaaaaatt gtttcaacaa ggtatctaat 84600
   attaaaatat tgagttttta ctttgttatt ttactagttc tttgaaatct ggtgtgtatt 84660
   ttacacttaa agcacatcac agtttggagt agccacattt ccaatgctta atactcacat 84720
   atggttagtg gcaactatct tggacaggac agcttttata ctctgggaag acacaagcaa 84780
   atacttgctc tgcagcagaa tccagatgtt ttccaagaaa acactttttc tgacctgttc 84840
   ctgaaaccca ggtagtgtct ctaatacttt atattttatt ggtttgtcct attgtaacca 84900
   cccaacgggc tctccttgtc cacttcctag acagagctga tttatcaaga caggggaatt 84960
   gcaataagga gccagcgcta caggagacta gagttttatt attactcaaa tcagtctcct 85020
   tgagaatttg gggaccaaag tttttaagga taatttgatt gtaggggacc agtgagtcgg 85080
   gagtgctgct tggttgggtc agagatgaaa ttatagggag cctaagctgt cctcttgtgc 85140
   taaatcagtt cctgggagtg gtggggtggg ggactcaaga ccagataatc cagtttatct 85200
   atatgggtgg tgccagctaa tccattgtgt tcagggtctg caaaatagct caagcattga 85260
   tcttaggttt taaaatagtg attttatccc caggagcaat ttgaggttta gaatcttgta 85320
   gcttccagct gcatgactcc taaaccataa tttataatct tgtggctaat ttgttagtcc 85380
   tgcaaaagca gtctggtccc caggcaggaa aggggtttgt ttctgaaagg gctgttattg 85440
   tttttgttta aaagcaaaag tataaactaa gctcctccca aagttagtta atcccaaact 85500
   caggaatgaa aaggacagct tggagtttag acgttagatg gagtcggtta ggtaagatct 85560
   ctttcactgt aataattttc tcagttatga tttttgcaaa ggcagtttca ctgtccactt 85620
   cacctcacat caggcctctg actagaggat tccaacaata cttaggccag gacaccacca 85680
   tgtctcctta tccaccctga gggagtccaa tttctgaaac aaaggaaact atatatgata 85740
   gtatgaaact atatatgaga aggaaattat atatgataat caattttagg gttatcttat 85800
   tgattagaag atattaaagt gtgacactgc ctggcaatga tatctgctgg tagtaagaat 85860
   ttggcgaatt tagtgaaatt cctgaggctg aacctccact tctgtaaaat ggagacagtg 85920
   agataatttg ccttacaatg ctgaagtaag aattttacac aataattcag accaaccact 85980
   tcatgtggta cttggcccgt ggaagactat caatgacagt tagtttatag tttatactat 86040
   taatgaatcc tttgtttcat tgttatttcc ttctacacgt tggcctctct aaaagaaggt 86100
   aatattcaat acaaataaag ttaaaacagc ttgcagagtt gtcccaggga actcacttaa 86160
   ccactgaagt gttcaaattg cttaaggttg actttatatt ctcctgacta acctttctcc 86220
   ttctggtatt tcttctgaga acagcaccac catccaaagc atcatgcaaa cagtggtcat 86280
   cccagaccag taattctcaa ctcacagggt gctcctgcag agatgtattt gaatagagtg 86340
   gtaggatgct gaagaaggcc acgtaaaatt tggccagtga tctggggcag atttatcctg 86400
   aagctaatga aacacaagtg taagggcctg tacttccaag gtgcagagag gggccctaca 86460
   aatgtgttag tttgtctctc tctctctctc tgattttaaa atttgcagta ttaaggtact 86520
   ttaatcacgg atggttcagg ctgctatttt cactcaatcc tcctttttat taaaatcacc 86580
   attgtctgat tatgttagaa tcctgatgaa aatatttgga atttgagtaa gagaaagttt 86640
   agttgaagat gtatctagta tggggataat aagttacgtg atttgcatat gtgatcatgt 86700
   gtacttcatt cgttgccagc caatctgacg taagaatggc ttcaaggagg ccgggcgcgg 86760
   tggctcacgc ctgtaatcct agcactttgg gaggccgaga cgggcggatc acgaggtcag 86820
   gagatcgaga ccatcttggc taacacggtg aaaccccgtt tctactaaaa atacaaaaaa 86880
   ttagccgggc gtgttggcgg gcgcctgtag tcccagctac ttgggaggct gaggcaggag 86940
   aatggcatga acctgggagg cggagcttgc agtgagccga gattgcgcca ctgcactcca 87000
   acctgggaga cacagcgaga ctccgtctca aaaaaaaaaa aaaaagaatg gcttcaagga 87060
   atgttcctac tgctcactgg aataactcac ctaaattcct ggcaagatgc aggtctagat 87120
   aaaatgttat gacatctaag tattcaaaac acattcccag cactgagagt gagtgtctag 87180
   tggagagtag aaacgtatag agccagaagc tagtctggaa agaattctta caaagtttac 87240
   aacttacatg tgaaaggagc ttaacagagg attttccaaa tttgaaaaca atcctaaaaa 87300
   cttacttgac attaccaata atgtgttttg aaactgaaat acttctaagt tatgaagaaa 87360
   acatattatc atcagccacc ctggaggaaa gattgaattc tatttccatt acctatagac  87420
   aacattacaa aataatttcg atctgaagat ggaatcagag tattcagtca aaactacagg  87480
   aaaatatact tggtagtgtc atattcagaa gttaataaaa tatgctattt tctgaatttt  87540
   gtgatggctg ttgttttgtc agcttttata aaattggaat ttgattttat tttcccatta  87600
   taaatttata tttacagtct gcagtacttt tgcattttta attttacatt atagttttta  87660
   atagttaaca agttgtaaaa ggtttgatcc ccagaaaacc ttgatctacc ccatcagtta  87720
   agtatactaa tatatttaga aaatggatga aatcagcatt tgaatatttt taaatattta  87780
   ttaaaagagg acatgggtaa aagagctttg cagttgccac ccttcattct caaattccct  87840
   ggataaggat gaccgcataa tctttggatg gtcatacgca agtcttgtgt acttgttaca  87900
   taaatctatt tagtggactt ttggcagtgt gtactgaggc cagtttcttc cacctgagct  87960
   ctgactccac ctccagcagc ccaaaaccaa tactgaattt tggggtcagc tattgttttt  88020
   gtggacttag gtaactacac acacattgtc tttatgatag ctttaataat actgccatca  88080
   gaactaaaat tgtcacgtgg attaaaagga gtgacggtgg tgtccccagg agcctttcaa  88140
   tatgtaagta tttacacata tacatgctaa aaagacccct aggaattttt taacaagggc  88200
   aaaacagtaa ctcagcttgt tttctcgcag taaaaccggt tgaaaaggcc tgatagactt  88260
   gtctgcagtt acaaaacttg tgtgtagtta tcacctttat atctcctgga aactaacata  88320
   gacaaccgaa tgggttacaa ctgtttttaa gtgaaattgt gagtggctct gaaaagagcc  88380
   ttttcaatga ggaagaaacg ggcagactta tgccctttcc ccacggatgc gacgtgccag  88440
   ctggatatct ttgggcatga tggtgacgcg tttagcgtga atagcgcaca gattggtgtc  88500
   ttcgaagagt cccaccaggt aggcctcaca agcctcctgc agcgccatca ccgcagagct  88560
   ctggaaacgc aggtcggttt tgaagtcctg ggcgatttct cgcaccaggc gctggaacgg  88620
   cagcttccgg atcagcagct cggtggactt ctggtagcga cggatttcgc gcaaggccac  88680
   ggtgcccggg cggtagcgat gaggtttctt cacgccaccg gtggccggag cgctcttacg  88740
   ggctgcttta gtagcaagct gcttgcgcgg agctttgccg ccggtagact tgcgagctgt  88800
   ttgcttcgta cgagccattt gcaatgagag cacacacaaa agtgtagtga actgagagca  88860
   agtggccttt aaatatagtg agaaacattc tgattggtcc tgtaatattt caaaagtccc  88920
   gcgcgataaa atcattggct gaagagtgac cagactgatt ggttcattac tagacaatct  88980
   tattggatga gttgccccac cgcccatcct gtccttttcg tttcagttat ctgcagcgac  89040
   aaattgtcta aaattctagt tcatccagtc ccaaagaaca gagtgtataa caaggtatct  89100
   aaggattttt aaaatgtaaa ttccgattca gtaagtttga gtgggacttg aaattctgca  89160
   ttcctgacag tctcgcaagt tatcaatgct ggtgaacact cactaaacca ccagaaacgt  89220
   tcagactcat gtcgggaaat aacgcttata ttcagagaat gagattccat gctattttgt  89280
   tactggcgaa cagcaagttt ccttgccctt tgttttctaa gtccaagtca cattcccacc  89340
   ctgcctgttc tcaaaatgtc ttattttggt tggccttaag tttcactttg tatactctaa  89400
   aatgtacttt ctaaaggaag gtgttatttt ctcgaaactt aactttttaa caccattagg  89460
   ctaggggggc ggtggctcac gcctgtaatc ccagcatttt gggagggcga gatgggacga  89520
   tcactagagg ccaggagttc aagacaaccc tggctaaaat ggtgaaaccc cgtctcgcat  89580
   aaaaatacaa aaactagctg ggcgcggtag cagacgcctg taatcccaag tacacaggag  89640
   gctgaggcat gagaaccgcg tgaagcggcg gggtggaggt tgcagtaagc cgatatcgcg  89700
   ccgctgcact ccagcctggg tgacagaact agactgtctc aaaacaaacc aatccaaacg  89760
   aaaagcaaaa aataccctaa cagaagcaag ttatcatcct ttcttgtgta actatggacg  89820
   gctctgaaaa atgccgtttc aagtgtaagc tacgttttct gatttgagtg tttacttgac  89880
   cttggcctta tcgtggctct gttattttgg caacaggacg gcctgaatat tggacaggac  89940
   gcctccctga gcaatagtga cgttgcccag ctgcttgttg acctcctcgt cgtttcggat  90000
   ggccagctgc aggtggcggg ggatgatgct gcgggtcttg tcacgtatgg cgctgcccac  90060
   cagttctaag atctcggcgg ccaggtattg taagtacact ggcgcaccgg ctccgaccgg  90120
   ctcaaaataa ttgccctttc gaaaaagatg acggactctg ccctattggg aactgcaagc  90180
   ccggtagcga cgaacaagtt tttgctttag ctccattttc cacgtccgca aatagcgacc  90240
   tatgaaagca gcggaaaact gtgaaagaca agcaagctgg aatggcgcct gaacaaatcc  90300
   ttttatacaa actgcaaggc tgcaatagga agctatccta ttggtcaatt atgtttggtg  90360
   ctttatccaa tagaaaaaga taacataaat tccatatttg cataaacccc acccctcagt  90420
   gaaaccgtgt ttcttttgtc caatcagaag tgaggaatct taaaccgtca tttgaatctc  90480
   aggactataa atacatgggc tctgaactgt tctctgtact actctgtagt ggagagtgtt  90540
   agtagctttt ctattctgtt taggaatagc aatgcctgaa ccctctaagt ctgctccagc  90600
   ccctaaaaag ggttctaaga aggctatcac taaggcgcag aagaaggatg gtaagaagcg  90660
   taagcgcagc cgcaaggaga gctattctat ctatgtgtac aaggttctga agcaggtcca  90720
   ccccgacacc ggcatctcat ccaaggccat ggggatcatg aattccttcg tcaacgacat  90780
   cttcgagcgc atcgcgggcg aggcttctcg cctggctcac tacaataagc gctcgaccat  90840
   cacctccagg gagattcaga cggctgtgcg cctgctgctg cctggggagc tggctaagca  90900
   tgctgtgtcc gagggcacta aggcagttac caagtacact agctctaaat aagtgcttat  90960
   gtaagcactt ccaaacccaa aggctctttt cagagccacc tactttgtca caaggagagc  91020
   tataaccaca atttcttaag gtggtgctgc tgctattctg tttcagttct agaggatcaa  91080
   ctggaatgtt agcgaagaca agttttagag ccaaggttaa cttggacggg gccgtgcgcg  91140
   gtgcctcttg cctttaatcc cggcaatttg ggaggccgag gcgggcggat cacgaggtca 91200
   ggagatggag accatcctgc ttaacacgat gaaaccccgt ctctactaaa aatacaaaat 91260
   aattagctgg gcgtgatggt gggcgcctgt agtcccagct actcgggagg ctgaggcagg 91320
   agaatggcgt gaacgcggga ggcggagctt gcagtgagcc gagatcgcgc catggcactc 91380
   cagcctgggt gacagagcga gactccgtct caaaaaaaaa aaaaaaaaaa aattaaaaaa 91440
   atatgaagtt ttgaagcaga aattattttg tcgtatgttc tttcataaat tttttgcctg 91500
   cctgccttct tcctttgtta cagaactcca acacttaccc aaaggtagct gttgggtcag 91560
   ggtttctgta ctatagtccc ttctgtggtg gccagaaata tgttacagga aagaggtccc 91620
   catccagacc ccaagagagg gttcttggat cccgcgcaag aaagagttca gggtgagtcc 91680
   gcagtgcaaa gtaaatgcaa gtttactaag aaagtaaagt ggtgaaacga caactactcc 91740
   atagacggag caggacattc ccgaaagtaa gaggaggaag gcatccaccc taggtacaat 91800
   acttgtatat atggggagat gtgctctgct acaagtttgt gataaaggat taattttctt 91860
   agttactata ttttgcaaga atcaacatta ttatctttaa acaaaattaa gaatgccttt 91920
   gttctccaga tatagggata tctggacact cctaagtctg agtctgttta gtaaacatta 91980
   tttatttgtt cccttaaccg taaacatcta gaagctagga atgactgact ttctgggaat 92040
   gcagcccaga aagtctcagc ctcattttcc tagccctcac tcaaaatgga gttactctgg 92100
   ttcaagtaac tctgacactt ttcttctctt tttttcttct tttttccttc ctttattttt 92160
   tattttttat ttttgaaata agaaatcaag aatacttgat gtttcatcta aaacaatacc 92220
   cataattgat aagccaaaac aaaaacctag gtcttctaac tcaaaactag gatgttttgc 92280
   tgtctctgct gatactcggc tgatcgttaa taggtaatta acaaacaagc cttgctatgt 92340
   ccccctcagt ttattaccat tagatcatat gcctactgtc aatcatatta atccacaact 92400
   atgcatttca caaaacttgc cataaaaatt cacaggtttc ccgcttccct cgagttttca 92460
   tttccgaagg gtcccatgta atataaaact tatattaaat acatttgtat gcttttctct 92520
   tgctaatctt tttttttgtt ttttgagact gagccttgct ctgtcaccca ggctggagtg 92580
   caatggcgcg atctcggctc actgcaacct ccgcttccca ggttcaagcg attctactgc 92640
   ctcgccctcc cgagtagctg ggaccacaga tacgtgccac catgccccgc taatttttgt 92700
   atttttagta gagacagggt ttcaccgtgt tggccaggat gttctcaatc tccttacctc 92760
   gtgatccgcc cgcctcgtcc tgccaaagtg ctcggattac agacgtgagc cactgcaccc 92820
   gaccaatctg tctttttgta gaggggcctc aagcatgaac ttactgatgg gtgagaaaaa 92880
   cagaattttc ttttccccta caatataaac attaattgta atgttatcat tcaggacatt 92940
   ttggtgacca atcttacaga aattttatct tgtgcaagtc tatgcaaacc aatatgtaaa 93000
   tcttctataa gtgagattgt atttcacttt tctagtatcc ttttaaatta ataaaagaga 93060
   ttctaatgat tattttcatt actgcatttc attgtaggga agtagataat tgccctttat 93120
   tcactgacct tcgcttttta aaaatttaaa ccatgttacc atgaaaatgc ttttcagtat 93180
   ttctctacac acaagattgc tgtaagggca aaaatagaga taggaatcat gcatccattg 93240
   atatacatat tttgattttt aatacatgtt accaagttgc ctcctgaagg tctgtttaca 93300
   ctctcaccaa cagggtgttt tttcctgact tccacaaatg ctcttgaaca gtgggtgtgt 93360
   tagtctgttc aaattgccga catgaacaat taaatctcat tgttgttttt atttttaaga 93420
   caattattgt ttgagactgc acattttgat aataacattt cttctattat ggtttgatta 93480
   ctcatgattc ttgcccattt tcttttggga tgttgcctta tgtacattat tttaaataga 93540
   tagctccatg tattaaaaga ttattaagtt tgagggctta tgatatgtca gttacatttc 93600
   taagattttt tttttttttt tttttgagac ggagtttcac acttgttgcc caggctggag 93660
   tgcaatggtg cgatctcggc tcaccgcaac ctccgcctcc agggttcaag caattctcct 93720
   gcctcagcct ccccagtaat tgggactact ggcaagcgcc accacgcctg gctaattttg 93780
   tatttttatt agagatgagg tttctccatg ttggtcagac tggtctcgaa ctgccgacct 93840
   caggtgatcc acccgcctcg gcctcccaaa gtgctgggat tacaggtatg agccactggg 93900
   cccggccaca tttctaaatt ctttataagt ataaattcat tcaatcttca ccaaaactca 93960
   atgaagtgtg agtactatta ttatcattgt tttacagatc aaaacaagta atacagtcac 94020
   ttactgagtt ctatacacct ggtaattttt ttgtttcgtt gttctatcaa ttattgggga 94080
   aggggtgttg aaatctctac ctttaaatca tgtatgtgtc tatttctcct ttcggttcta 94140
   tcaggttttg ctacacatat tttgcagttc tgttatttgg tgcatataca tttagaattg 94200
   cttgtttttc gtattggatt gaccctgtta tcattatgta atatccctgt ctgttcctag 94260
   taattttctt tgctctgaaa tatacttatc tgatatatca tccaaaagac caccaggatg 94320
   gctaaagagt agaaaggaga gatttactgg caatactaat ttgcaagcca ggaagagatg 94380
   gtcccagaac ctgccaaaat tactctctct ttggggagaa ggagcaggtt ggttattttt 94440
   atgcctcata ggctatatat tacacaatag agtcatacat atttagcacg tttgggggga 94500
   cagctatata tattatgagg ggtgccaagt gcattcacaa tggataaaca cgtgtaatat 94560
   acctcccatg ttcacttcga ggttaaattt tggttaaaat gaggtagaat ttaggtcttt 94620
   acatcacaag gtgaactata ggaacaaagt ttacgtgctg cctctagcag ctggctgaaa 94680
   atggcttaag gtctacaatt acgtgtaaga atagaatgtg tgtcaaggcg gtcctctgtc 94740
   caatcagagt tgtagtggac tggactgtaa atcagagtta ggagggcttc tgatagctcc 94800
   tatagttaag gaatttagca agtgtgagtt ttttggtagt ctttggaatt taggaatttg 94860
   ccatgccagc caagccatga atgctctacc agtaggtaac tttgtttgct taatcttaga 94920
   gtctgtctta gttggtatag gggcatctat tttggtcttt cagatcccag atattattaa 94980
   tacagatact cttgcagttt tgggctgatg tttatatggc ttatcttttt tgcagccttt 95040
   aatttcaacc tgcgttatgt ttatatttga agtgagattc ttgcagacag tgtacagttg 95100
   ttgttttttt ttttttgaga tggaatttca ctcttgttgt ccaggctggg gtgcagtggc 95160
   acagtctcag ctcactgcaa cctccgcctc ctgggttcaa gggattctcc tgcctcagcc 95220
   tcttgagcag ctgggattgc agccatgcgc caccacaccc ggctaatttt tgtattttta 95280
   gtagagacag gattcaccat gttgcccagg ctggtctcga actcctgacc tcaagtgatc 95340
   cgccagcctc ggcctaccaa agtgctggga ttacaggtgt gagacctcgc gcccagccaa 95400
   actgtttttt tatgggtgta tttataccac acacatttaa tgcaattatt gatatcttag 95460
   ggcttaagtt catgaagggt agtgtgggaa ccatagtctc ttggcccact aaatgtttgc 95520
   cagaaatcac tgacaaggca gattgattaa taggtgaaaa ggcattttac ctattgttta 95580
   acgtgtctat gtgggagcat tcagaattaa ttacctaact tcccaatgag ttatagatgc 95640
   ttatatacca tttttagatc acagaaagaa ttggggctta gattctggta aaacaggtta 95700
   tgggaggcaa aagaggtttg gcttgcaaag gtggccttgt taggtaggtg aagcctccct 95760
   cagaaagaac agatggtaaa tgtttctttt atgattttta agtgtcagac tctcagtctc 95820
   tcctggatct ggggaaaggt atagaaaggt gaggaggcat ggctgcatta atggagattc 95880
   tctacagatg taaaattttt cccatttaag gcagctttgc aagcccattt ctgcctgctg 95940
   gccaagcagc agccatttca aaatatgtca aagaaatata ttttggggta aaatattttg 96000
   atttccttta gactggtggc cttataagaa aaggaagaga cacctgagct gacacacata 96060
   cccttgctct ctcaacatgt tatgatgcag taagaaggcc ctcaccagat actaattcca 96120
   tgcccttagc ttcccaggtt ctagaacagt aggaaataaa tttcttttct ttaaaagtta 96180
   gccagtctgt ggtattctgt tatagtatca caaaatggac taagtaacta tattatgatc 96240
   atcttacatg actgatccct cctacatcat acacatacac aggccacatt tggaacattg 96300
   ttagaggttc ctctgcccag tacaaatgta ctacaaatta tatatgtatt tttaaatttt 96360
   tgagtatctt caatagtata ttttcgttaa cttttgtagt caaaatgtca ttataacatg 96420
   tattcaatat gcataattat tagtcagatg ttttacattc tttcttcata ctaagtgata 96480
   tggtttggat atttgtcccc tctaaatctc atgttgaaat gtaatctcca atgttggaag 96540
   tgaagcctgg tgaaaggttt ttggatcgtg agggtgaacc cctcatgaag cgcactcttc 96600
   agggtaatca atgggttctc actttgagtt cacaagagat ctggttcttt aaaagagtgt 96660
   gacacctccc ccatctctct cgctcagctc tcaccatatg atatgcctac tccctcttca 96720
   ccttccacca tgattggaag tttcctgagg acttgccagt agcagatgcc tgcaccacac 96780
   ctcctgtaca gcctgcacaa ccgtgagcca aaaaaaatta cttttcttta taaattagtc 96840
   agtttcaggg attcccttat agtaatgcaa gaacgaacta acacactaag tctatttcat 96900
   atttacagaa tagctcaatc tgaagtaccc tttttcaact tcacagtagc tacttgtagc 96960
   tagtgggcac tgatttggag cgtgttcaag ggtgaattgt attatgcaat taacagattt 97020
   tttttattgt tttcgcaaac cacgaggcat agattgtctt actttctctg ctcctggtgt 97080
   tggagttgtt attgggaaac aacttatttt cctcttatat ttatatggaa taaataaccc 97140
   ccaatatttc cctccccaat atctgccttt tgtatgtttt ttgaaggcaa gtgcctagaa 97200
   tttactgttt ttgaagcact tactgaaagg attgccatca agttgttttg ctaatagtac 97260
   atgccaggcg cttgttggtt tgcttaattc aaggtaactt ggatgagaag aagagttttt 97320
   ctcatccatg gctcagtgga gtatagatta ctgatattgt gactggatgt actcctgctt 97380
   tctagtctga gtttttgaag ctacccttaa tcttggtttc aattttatct agccctgtac 97440
   atatccaagg ctctttccaa aatggtctac gatttgttta ggaagttaga atagctgtac 97500
   tttctgaacc acggttcctg acattttctg gacttcaaac acatccagca ttttatcgaa 97560
   gtatttatcc ttcctacttg gctggcttct tccttgcctt caggtctgaa ttcaaatgac 97620
   attctcctga tgaaactttc catccttatt tctattcttt tttcttatcc cctttcttta 97680
   tttttctcca cagcactcat cacttatctc tacattttca ttatgtattt accttattgt 97740
   gcacctccca ctacaagaca agtagcaccg taaggaaaca ggttgtctgc tttttcactg 97800
   ctatgctccc tgcacctaga acactctctg gcacttagca ggttttcagt aaatatatgc 97860
   tgaactaata atgctggata tacatctccc tcatgaactc tctaaatcct tctaatttac 97920
   attgatcaat cttcttttcc atgtgctttt gtatgattta ttgctcaaaa tctttatttt 97980
   atatgcagaa cgtgcactgc tatttaatct tcatgtacgt aagtcctccc ttctctgagt 98040
   ataatctctt cagggcacta tctgagataa ctttttaaca tctccatcat gaatcttgta 98100
   ccttttcaaa gaaaatgagc cagtgattac tgatgtttac ggctattgtt gagggtgaag 98160
   atcattataa ttttgaaaag ggaagttgaa tattgtgaag ggaaagataa cactagagtc 98220
   agaagacttg ggagaaggca aaaaacaaac taaaaatgag cacttttagt ctcctgacag 98280
   tttctctgaa tcaaatccat agttctgtga cagcgttggc ttagaagcag attttttttt 98340
   tttttttttt tgaaatggag tttcgctctt gcccaggctg gagtgcagtg gcacgatctc 98400
   ggctcactgc aacctctgtc tccagggttc aagcgattct cctgcttcag cctatggagt 98460
   agctgggatt acaggctccc acaaccacgc ccagctaatt ttttgtattt ttagtgaaga 98520
   ctggggtttc accatgttgg ccaggctggt tacgaactcc tgttctcaag tgatctgccc 98580
   gccttggcct cccaaagtgt tgggattaca ggcatcagcc accgtgccca gccaggagca 98640
   gattttttta cactcatgtt tctttttcct tctgtcatcc tgtttcagta taagcagacc 98700
   acagatagaa gtagtagata cctcagaaat tcctggaata attaatccac gttcatctgt  98760
   actccatctg ctcctatctc atggaatata aaaggaaaaa caccaagatt tccctaggca  98820
   atctgtcttg attttaggtt cctcaacagg agagccagac aatggctgta ataatattgt  98880
   cccggccaag gaaaaacttc ccctttgccc tcccaaggtt tatggaaaat tactggcaaa  98940
   acacagatta actggagaaa aggcatatat atttatttca tcacaatttt acaggagatt  99000
   ttagaattaa gactgaaaga tacaggggaa attgcccatt tttatgctta ggttcaacaa  99060
   gataaacagc tgtatagggt acgatctaat gctaacagac tgagtgggga agccccgcaa  99120
   ggcttgtctg tcaagattct tcttgacctc tcagtgcagc atttcttcct tctggttata  99180
   ggacaagact ctcttttaga atggggggtc ttatgaccta caggcaaaca aggtaggtta  99240
   gagtaatact tttaggtttt atggctggtt ctagggaaaa ggagttctgg tttgtatggc  99300
   ctaccttgag gaggaattct ggtttctatg gctagacttt ggggagaatg ggacttacag  99360
   acaggaaggc agaaggtggt cagtgaaaca cttttataat cataatccca ttttgagtat  99420
   ttctgtgtta tggaatgttt gttctctcat ttcctgaaag attccagaga ctcctcattc  99480
   agtgttgtga aaaagttcag gaaatgcaac tcaaaaatgt gccactttgt tacgctgatt  99540
   tctttgaact gagggcacct aggaaacagt aaattcaagg aagggctttc gctgaactct  99600
   aatcaaaaat ttgaaaatta aaaaaaaatt caaaaaggaa tttagttgtt aagattcact  99660
   tccctgggga atctcatcaa ccagagaaga ttaactgtat cacaggagag gagactggtg  99720
   gttaacacca tctaaacaga ctttgtcaca gctgtcacct attctttgaa acacccattt  99780
   atttttctcc aaaatcatat actctcccct aagttgccta catccccctt ctttctccct  99840
   tatgaatcaa gagagcttat aagcttctac agttcactgg gatttggggt attcgctttt  99900
   cttccctccc actccccctc cccttttttt gtctttgaga cacagtcttc tggctctgtc  99960
   gcccacgctg gagtgtggtg gctctatgtg aactcactgc aacctcctcc tctcgggttc  100020
   aagcgatcct cccacctcag cttctcgagt aactggaact acaggcgtgc actaccaagc  100080
   ccggcttttt tttttctttt tctcccccgt ttcttttttg gttattttac tggagacagg  100140
   gtttctccat gttgtccacg ctggtctcga acgcctgacc cgccgtcctc ggcctcccaa  100200
   agtgctggta ttacgggcat gagccactgc gcccgatttg aaggacctct taaatatcta  100260
   tttagaaatt ggtcggagtc cactcctttc caaaaacatg agtcacaatc cgggaaaagc  100320
   acgagcggct gaaagtcaaa ataaccagaa caaaacctcc actcatgctt aaaaaaggta  100380
   ttttgacaaa atcctaattc ggccaattat tattagtatt caagtcgaag gctcgtcaag  100440
   ccagactggg gattgggtca aacataaacc ttacaccaga cggaaggatt acatgcaaat  100500
   gaaggatgca gattctgatt tcccattggg tatttgacat tagccaatgg gagaattcct  100560
   cacagcctac ctccagtcag tataaatact tctctgcctt gcgttctaat gtagtttcat  100620
   tacattttct tgtggcgatt ttcccttatc agaagtagtt atgtctggtc gcggcaaaca  100680
   aggcggtaaa gctcgcgcca aggctaagac tcggtcttct cgtgcaggtt tgcagtttcc  100740
   tgtgggccga gtgcaccgcc tgctccgcaa aggcaactac tccgagcgcg tcggggctgg  100800
   cgcgccggtg tatctcgcgg cggtgcttga gtacctgacc gccgagatcc tggagctggc  100860
   gggcaatgcg gcccgcgaca acaagaagac ccgcatcatc ccgcgccacc tgcaattggc  100920
   catccgcaat gacgaggagc ttaataaact cttggggcgt gtgaccatcg cgcagggtgg  100980
   cgttttgcct aatattcagg cggtgctgct gcctaagaaa actgagagcc atcataaggc  101040
   caagggaaag tgaagagtta acgcttcatg cactgctgtt tttctgtcag cagacaaaat  101100
   cagcctaaca gcaaaggctc ttttcagagc cacctacgac ttccattaaa tgagctgttg  101160
   tgctttggat tatgccgccc ataaagatgt ttttgaggtg tttttaatgg ctttgagtgt  101220
   ggcactttta gtaatttgtc ctgcagaaat tagatccata gaaacctcag gaattctagg  101280
   tatgtgggag aagtgccatg cagcacaaaa catgtttaca ggggtgattc gcgttaagtt  101340
   tcacacacag cagttactac attttagagg aaggaaatta tacccatgag tgcattccta  101400
   actatcttga atggaagtgt taaaacccgc atgccccaca caagtttgaa tatgtcatac  101460
   catttgctgt agcaattaat ggcatacaca attgagagca cacacattac cactgaacat  101520
   ttgagtatgt atttcccaaa atgagctttt ttccagtttg gggatgtttt gctttgtttt  101580
   ggggtggagt ctccctctcg cccaagctgc agtgcagcgg cgtgataaca gctcactgta  101640
   acctcgaact cgggctcaag cgatcctctt gacagccttc tgagtagctg ggattacagg  101700
   cgagagccgc cacgcccggc taagagcatt tttctaattg cccacacttc ttatgcgaca  101760
   cccagaaaaa tacaatttta aataaagcgc atatgcaaat ttccctaatc gtctccaata  101820
   ttctctgatt tcttttttat attttaacta gaaacaattg gaggtttccg cgttgctttg  101880
   tgtggttgta aattttaaga cttcaggaaa cttttccagt acaagacttg tccacagtgg  101940
   atatagcagc taaggggtta acaaaatgac gtcagagtag ctacggtaat gggcaggagc  102000
   ctctcttaat ctgcaaccag gcacagagat ggaccaatcc aagaagggcg cggggatttt  102060
   tgaattttct tgggtccaat agttggtggt ctgactctat aaaagaagag tagctctttc  102120
   ctttcctcca cagacgtctc tgcaggcaag cttttctgtg gttttgccat ggctcgtact  102180
   aaacagacag ctcggaaatc caccggcggt aaagcgccac gcaagcagct ggctaccaag  102240
   gctgctcgca agagcgcgcc ggctaccggc ggcgtgaaaa agcctcaccg ttaccgcccg  102300
   ggcactgtgg ctctgcgcga gatccgccgc taccaaaagt cgaccgagtt gctgattcgg  102360
   aagctgccgt tccagcgcct ggtgcgagaa atcgcccaag acttcaagac cgatcttcgc  102420
   ttccagagct ctgcggtgat ggcgctgcag gaggcttgtg aggcctactt ggtagggctc  102480
   tttgaggaca caaacctttg cgccatccat gctaagcgag tgactattat gcccaaagac 102540
   atccagctcg ctcgccgcat tcgcggagaa agagcgtaaa tgtaaagtca ctttttcatc 102600
   agtcttaaaa cccaaaggct cttttcagag ccacccactt attccaacga aagtagctgt 102660
   gataattttt tgttgtctta acagaacaaa tttctaagga cccccccgga aagcattaga 102720
   ctatggtctt aaagttgatt aacagaaata acggtttggt cagtcttgca gtgtaggtta 102780
   tttctgacct tattaaggtg ctatttggag agaagctgtg taagtccact atcattcagg 102840
   cctctagctt gctatgatta gcatttgttt aaacaacttt gtaagagtaa gggaaaaatc 102900
   tggtaagtag ttaactggcg cttactaggc atttttgcaa agctttgaaa agattagaaa 102960
   attgtgtctt gcgagttcca gtgtcttcct caaaatgctt aggaagattt tctcagctca 103020
   atacatagtc ccctaggttt tctcatatat tatatatata tatatatata tatatactgt 103080
   taaattcatt tggctgttaa cattaacctg aaatttattc tggtgcaaaa tgtgaggcag 103140
   ggatctaact ggctctcatt ttatccatag ctagctaccc actttaaatc tgtcagtctg 103200
   tcgaccaagc ataatttaat cccttatata tgaattttta tatgtgtggc tttgcttgta 103260
   aatagtctat ctggttgcat tgctttgtct cctctaggac tatgcaccat gacatgccac 103320
   attctttttt tcagtacttc ttgcctgtag ttattaaaat ctagaattta caagttttaa 103380
   ccattttctt tctgttgatc ttgcttttcg gttttggagg ttggggattg agtactggaa 103440
   gaaaatttag agggatggga atactgtacg caaacaaaag taatatttac tttaaaattt 103500
   ttatattttg tattttttta tcatatagct tttacatcac attttacaga ctaactttag 103560
   aacaaccaca gaatgtccaa cattaaaact actaattcca aagaccttgc ctcacattct 103620
   tttttacaat aaatattttt tacacctaac attctttctt ggcctacatc tagaatgtaa 103680
   actgatgtac catactaaaa tcgcctgacc aactgtcaac aacaacaaat cacacacaca 103740
   aaagattaaa tttgaattgc atcgtttact taaattcatt tgtgttccag cttttaataa 103800
   ggcagttttt ggtttataaa gtaatatttg cattttaaaa attatgaaaa tgaatatgtc 103860
   agtttgtttt atgattcgtt tttcttgact cttatacaag cgactctaac tggcatagac 103920
   atttgttatc cacagacagt atagatatgt tagagatgcc aatggacttg gtctatgcca 103980
   aggtgactac tcacaagctc tgggcccagc tgaaggtcaa gtattttttt tccagttata 104040
   gatgtgctgg atctgatgta tagcgcttga ctttttatat tttctttatc tgtaggaaac 104100
   aaatgtgttg gaggtactgg gtctgacgaa tagcataaaa gaataaagtt acattactgt 104160
   ctgaggatca gatggacagg gggtggtagc tcagtccagc tattttccac tccctcactt 104220
   acattctttg ccccctcctc aacagaacaa ggattctgct gtaactcttc attgacagtt 104280
   gatatttaaa aattaacgaa tggatgaaat tctcatttgt gaaagaaaat ttattgagca 104340
   ttttgtattt gtgagtagtg caaacatttt aatattatat taagaatcta ttgttttgta 104400
   ttagaggagt aattaaggag agattggaga caaaaagggg gtgttgtttg cagaatatac 104460
   catccaaaaa tagaccactg tgggatcagg attcttttga gctaaaggca cttcaaaaac 104520
   agcattcaag aagggaattc ttctaaactt ttctttctga aaacaggaga taaaagttcc 104580
   aatgtgaaaa atgctctgct tgtaccaggt gaaaagacat attcttcagc ccagaggcat 104640
   agatgagata attctgcaca aacacagcag ggagtcatag ccgagagact tctatacaca 104700
   aacaaacctt gttaaaataa tcatatattc ctttaatctc ctcatatggt ttactttccc 104760
   acaattgcct ctctttaact taatgtgaaa gcatttagct tttgccattt ctttggggct 104820
   tcactttttt atgagggttc tcctgtccca taaaatttac attaaataca tttgtatgct 104880
   ttcattctgc taatctgttt tatggcaaat gaattatcag gtccagctgg agaccctaac 104940
   agagtagagg taaaattttg cctccctaca agatagagat tgtgtgcatt aaatgttgtt 105000
   tgttcccagt tgttcagttt gtcaggcctc tgagccgaag ctaagccatc atatcccctg 105060
   tgaactgcac gtatgcctct agatggcctg aagtaactga agaaacacaa aagaagtgaa 105120
   aatgccctgt tcctgcctta actgatgaca ttaccttgtg aaattccttc tcctggctca 105180
   tcctgactca aaagctcccc cactgagcac cttgtgaccc ccacccctgc cagccagaga 105240
   acaaccccct ttgactgtaa ttttccacta tctacccaaa tcttataaaa cggacccacc 105300
   ccatctccct tcgctgactc ttttcggact cagcccgcct gcacccaggt agaataaaca 105360
   gccttgttgc tcacacaaac cctgtttgat ggtctcttca cacggacgcg cctgaaacag 105420
   tttaacaggg tttttcctgc ccagtcacaa caaagtgatg ttatgctgca ggctgaagtt 105480
   tacagctaat gctgttgaag tctaaaatca gttttggttt gttagatttg ggtgagatgg 105540
   ctaagattct cagagaaaga agtcaagttt ggggtgcatt tttcagactt aaaaatttag 105600
   cagtagccct tgcagttttt ccaatagaag tgatttaaga atgttttcag gaaatttaaa 105660
   acaacagtga gaagcgtgta tggagagttg aactacactc cagacttggc tataggaaag 105720
   cacgaatgct gctattgtat tgcaccttgg aaaagagaac aaaggaatat tttcggacaa 105780
   ttttaacatg tcacatatga aaagctaaac ggaatctgtc aacaccttgt acgttattac 105840
   aggctgtgat tttaaaaaaa caatccttac taatacatac atagttgctg ctagcaatat 105900
   agtgttggga gtaaaaacac gaaaatgaga gttcaggaca atatcccaac tctgagcaga 105960
   tttttttaag tagtaacatc taaaattaaa ccatattatg taatatttat ttcttttcca 106020
   cagtctcttc tcatgcctcg ttcacattag ctaattaaaa gtcccctgag tatcatcata 106080
   acccgattta cagatgaagg cacggttgca atgagctatc accctcttct gaatgagaca 106140
   gtacagtgtg aaggatagca aaactccact cccatcctct tagggctctg gctggaccag 106200
   caaattaaat taatgtaaaa tggattaaca ggagaaaggt atatgcattt atttaacaca 106260
   ggttttacgt gacacaggtg ctctcataag gtaatgaaag cccaaaaaaa gcagttagct 106320
   acttatataa tgaattggac aattagtaaa atgtaaaaat gcgctaaagc aaagggattt 106380
   aggctagaat atataactgt gtagagaagc gcccagcaag ggctagtgca aggtttgtac 106440
   agaattctct tggcctcagc ctcctatcct tgagaagaat gttgcttttt ttaaactaca 106500
   gtgagaacat ctttcatatg agaatttcac ctactgcttc taagaaacag gtcagctttc 106560
   aagaaaacat aaggccagag tgatcttttc acgcctgctc ttttaagtac ctttgaatag 106620
   tcaatatgtc ttcaagcact tgaaagactt aaaaagttta ccactccggc atattagtga 106680
   aagcccttaa tataagccct tattaaaatt ctcagtcgag ggtataaatt cagattcaaa 106740
   tagtagtgtc gtaaacggga gggaaaaact aaagggatta aaaagtgaaa ctattgtgtt 106800
   ctccctcgca gtccttaggt cactgcccct cgaggggcgg agcaaaaagt gaggcagcaa 106860
   cgcctcctta tcctcgctcc cgctttcagt tctcaataag gtccgatgtt cgtgtataaa 106920
   tgctcgtggc ttgctttctt ttcgcgtacc tggtttttgt tgtcagctgg ttagacatgt 106980
   ctggtcgcgg caaaggcggt aaaggtttgg gtaagggagg tgccaagcgt caccgaaaag 107040
   tgctgcggga taacatccaa ggcatcacca aaccggccat tcggcgcctt gctaggcgtg 107100
   gtggggttaa gcgaatttcc ggtttgattt atgaggagac tcgtggcgtt ctcaaggtgt 107160
   ttctggagaa cgtgatccgg gacgccgtga cctacacgga gcacgccaag cgcaagactg 107220
   tcactgccat ggatgtggtt tacgcgctca agcgtcaagg acgcactctg tacggcttcg 107280
   gcggttaatc ttttcgtcag ttttcttcca atggcccttt tcagggccgc ccactccctc 107340
   tcagaaagag ctgtgattgt attctttcgg atggtaacat ctcaatggct ttactcggct 107400
   attctgccta gtatgtagaa ctattataaa ccagttggga gagaccaggt tgtttggtct 107460
   gagtggctgc taaagcagaa atcagctaag taaacgaggt ctccgagata agtgagctat 107520
   aaacttcaat gctatagttt tgacatgtca agcaacttaa cgtgcagcgc gagtccgata 107580
   aatgagtagc tcagcttttt agttttaaaa acgagttgtg cgttatttgt acgagagcct 107640
   aagatgctag ctgcctggaa ctgagtaggt ggattaaaat gggtgtcagg tctgttttcc 107700
   caggcgtatc tgacttaacg tcagcaaaag ctgtactttt agcttccctg gtaacacctg 107760
   ccgtccttaa ccgccccctg ccggtagcgc cagaagcctt tacttccatt tctagttgag 107820
   cttggcgtcc tgctgagtga cgtcacctcc cccttctctg gagtaggact ggcggttaaa 107880
   gctgctttgc tattttcagt cctcaggctg gaggctcccc taagcaggct gcctacgcag 107940
   ttcgtaaatt cccacttagt agactaaggg agtctgtttt ataaataagg actcaaattt 108000
   cttctgactc cgaggtccgt ggcagcagct ataagatgga agccccctct gatgtaagat 108060
   tctcagatga cttgcatctt cactgtacct gtcaacccaa tagtcttcta ttcctgcctt 108120
   aaattgtaaa ttccaaaact gatttaattg tgaaagtttc aaactgtacg acctaggaag 108180
   tgtcaaagtt aggtgaccag atttttagaa gtcagccaaa tattcagcat ctttgattta 108240
   gtaacaaata tattgatggc tacttcagca aaaaaaatca actttgtttt ctggttactt 108300
   tgctaacaag cttctcctga caggaggata tagtgaatag gcagttgaat aagtgagttc 108360
   gggtgagagg tctgagctgg agataaaaat gtgtgagtca tcagcagata aataaatgct 108420
   gagaccagat gagatggcta aaaactgaaa cataatgtag tgcagcattg tttgtaatag 108480
   taaatgagtg gcaactgtaa agttttcatc agaaaggact agagtgatct atacatccat 108540
   aaaatagagt atttctctac acagccctac taaagaatga gaaagctgta ctccactaca 108600
   tactctggtg tactctggct cagttcttgg actcctcttt tcttggctaa ctcaactggc 108660
   ctcaccactt acatgctctg tgctctgtca aatagtttgt tcaacagaac accacggcct 108720
   agctgtaagt gccacgttaa cttctagcaa tgccaaagcc tgtgatagtg gcagcttcgg 108780
   gctgtttctc attcccggga tgcctaacca cctctccaaa ttctatcagt ttgcttccac 108840
   ccacttcaag cttcagaacg aaacatagag cttaagaaat ataggcccgg caaggtggct 108900
   cacgcctgta atcccggcac tttggaaagc tgagcctggt ggatcacctg gggtcagggg 108960
   ttcgagacca gcctggccaa tattgtgaaa ccccgtctct actaaaaaaa aaaaaaaaat 109020
   tagctgggca tggttgcggg cgactgtaat ccaagctact cgggagggtg agacaggaga 109080
   atagcttgaa ctcgggaggc agaagttgca gtgagttgag atcgcgctat tacacttagg 109140
   cctgggagac aagagtgaaa ctgtgtctct aaataagtgt ttgcaattat aaaccatctc 109200
   cctgacctta aatctctaga ctcatataca actgcatatt tgatgtatct aattgaataa 109260
   tgggcatctc gaacttgtcc aaaatatgtt tatacgtaaa caccaagtct gttcttcctc 109320
   tgatatttgt catgtcaatc aatagaactc cattcttcaa gcagcttggg ccaggaattg 109380
   tgcaatattg tttgtcctga gcttcttaca actttcaccc aatgcagtca gctctgttga 109440
   aaatcaatca gaataccttt cattgttttc tttgctgctt ctctaggagc aagctgccat 109500
   ggcggtttgt ctgaatgacc acagtgaccc caaactggtc tttgttttca cttttaatcc 109560
   ccctgtcata cagttttttc tctatccagc atcaacagtg atcctttttg aaggtattat 109620
   gtccactgtc tgctgaaaag attccactgg ctttccatca ccttcataat aaaaaccagc 109680
   atccttatca tagcctacaa gtaagatgac caaccattac agtttgcctg actctcaggg 109740
   gtttctcagg gtgtaagact tacagtgctg aaacttagaa agttccaagc aaactaggat 109800
   gagctgctca acctactaga tctgtactct ggctaccctc tgacctcatt ctcttcgcag 109860
   ttctttctct tcactgacct tgctgtttct ggaatggacc aagcatttcc agcatcagca 109920
   cctttatatc tattctttct ccctagaagg gtcttgtcct ggatatctga atggctctag 109980
   atctcatttc attcaagcct ctcctcaaat accaacctta cgaaagagac ctcccataat 110040
   catcccttgt aaaataagct tttctgctca tttagcatat atatatatag ttgactatcc 110100
   tcaatagcat atatatataa catttcccca cctagaatta tatatgtaat aatatattta 110160
   acaaaaaata catataacta gatatatttt attttgtgtt tgttctctct cccccaactg 110220
   gaatatattt tttgaaggta gggactttgt tttgtcccag aagtatccct agcaccttga 110280
   acagggctga cgtttaacag gtagtttatg gaggtttgtt gaatgaaagg atgtgtgaat 110340
   tttctatgta agtctccagg ctctccacta agcccaccag aatgctaaca caatcaattc 110400
   cccatctcat tccttgacct gccactgcct gaagcaatca gcgtgcagtt tctctttaga 110460
   aaatctgggg gatagtctag gggttgcaaa ttaagcaaca ttatctttgt tctgaacaag 110520
   gactgcatga gtgttaggac tgaagaaggc ccaaggtggt ggtgggtatg cctaagatga 110580
   gtatgacata tcagcaatgc tatgaacata gcaatgctat gaaaggccag gcaaaacgta 110640
   acaggagcta gtcgtggctt attgttacaa cgactatacc tcccatatgg gtaatcgata 110700
   tccacacacc cctctacatt gactctggaa ttcaggaaag ggaattaaaa ttttctaact 110760
   tatgtacccc aatgatttca acaatatctg gcatatgaga tcaataaata tctttaaaat 110820
   accaactaag aaagacataa aatgacccac cctccatacc aggctcattt ttgctcctct 110880
   gattcctgaa actatccaga atgcagctat gaattctctc cattgtcagt tttaaattaa 110940
   gccaagctgg gtacttgtgt aattcctcaa gaaatcctgg atgaaaactg tcaggtggaa 111000
   aacaggacct caaaataaag agacatccat cactgaagct aacatcgtga ggctgaaatc 111060
   agtcctataa caatggtacc aaaaagagca caatgagagg catttgtgaa tatttactca 111120
   gatgagagta agatatttcc ctatcagcta acctgaagtt cacatccctt ttccagctga 111180
   gttctgaagc tagatgtact taactggaac acataactgc atcaggaaca tcctttaaaa 111240
   ctatggctac catggcttga ctggacaaac cccaggcttc caggtttagc acaggtggcc 111300
   cttcacagac caacattgcc tatgctacca acctcatgtc ctaccaccct gcttgcatca 111360
   tttctctctc tgcatatata aaaatatatg tgtatgtata taatcagctt tattgatatt 111420
   taatgtacca caaaatttgc ccactttagg tacagttcaa tgaattttac cgtgttttct 111480
   tagttgtaca accatcatca caatttaatt tcggaatatt tctatcaccc aaatttccat 111540
   ttctgcgtaa agggggaaaa aaaaaggtta actgctgaag gccgcggtaa cactgaaaaa 111600
   ggtgcctttt ctctctaaaa cagattttaa tctcccctga atttagtgtc ctgggtattc 111660
   caggagtctg aatagggttt caattttcag ggtcttttta atagagtaaa actgtattgg 111720
   tggcgataaa tttagtattg ctctcagtac atgattgagg gatacttaaa tgtctctgtg 111780
   attttatttc ataatcgcta aaagatggtt tttttttttc ctaaaacagg gtttttgttt 111840
   tttctcaata agcttcttag cttcccctcc ggctccctgg cttgcctcag gaaatattag 111900
   ctcatcagtt ctgattggtt gacagctacg aatggccctc attgattggg cagcgcttct 111960
   ttgtcccttg gaaactaata caaattttta acactacttt ttttccactc tttcttcaga 112020
   gttggaatat cgttgctccc ctacccatat gtagtgagtg gagggcaaac ttggagttcc 112080
   cctaatcttt cctttttagg atgtcagctc agtatcattc atcttaatta cacattgagc 112140
   ttcttgactt aatggataca gctcttcttt tgtttagttg ggcggccctg aaaagggcct 112200
   ttggttcaga aatgcaagct gtggagaaat cagcaacctt aaccgccaaa gccataaagg 112260
   gtgcgtccct ggcgcttaag cgcgtagacc acgtccatgg cagtgactgt cttgcgcttg 112320
   gcgtgctccg tataggtgac agcgtcacgg atcacgttct ccaaaaacac cttgagcacc 112380
   ccgcgagtct cctcgtagat cagaccagag atccgcttca caccgccacg ccgggccaga 112440
   cgccggatgg ccggcttggt gatgccctgg atgttgtcac gcaacacctt gcggtggcgc 112500
   ttggcacccc ccttacccaa acccttcccg cccttaccac gtccagacat gacttcccaa 112560
   gaagtgaacc aagagcaagt gagagaatag gaaaccgatc tttatatatc tacgttaccc 112620
   ctgcccccac ctccagcgga cactgagact gaaaagcgcg caggcgggaa atgtgacgcc 112680
   tacagtccgc tcctttaacc cctcctccaa gccccaggaa atggcgggag cagcgattgg 112740
   gggagggtgg ggagatgagg gtgggaccaa gcaggcttga ccaatggcct ttattttctt 112800
   aacagagcta caggctttga ggaactgggt taagaattaa atgtaaaccc attctgactc 112860
   cagaattatt ttaagtcgaa cttttttttt aaccgaatct ctctgtcgcc cagactggag 112920
   tacattagag ccatctcgat tcactgaaac ctctgcctct caggttcaag tgtttctcct 112980
   gcctcagcct tcagagtgta gctgggatta caagcgctcg ccgtcgcgcc cggcgtgttt 113040
   ttgtattttt cgtagagacg ggattcggcc atgttggcca ggctgatccc gaactcctga 113100
   tttctggtaa tccgcccgcc tcagcctctc aaagtgcttg aattacaggc gtgagtcacc 113160
   gcgaccggcc gaaatcgatt ggttttgaag ccttcagtag cattaaaacg aaaagtgctc 113220
   ccaatgcatt cccttttgtc ttaaattggt ttcttacagc tactttactt gaaaaggtgg 113280
   tggctctgaa aagagccttt gcttggaccg tcagagagac cacagtaatc acgccctctc 113340
   tccgcggatg cggcgggcga gctggatgtc cttgggcatg atagtgacgc gcttggcgtg 113400
   gatggcgcac aggttagtgt cctcaaatag ccctaccaag taggcctcgc acgcctcctg 113460
   cagagccatc acagcggagc tctggaaacg caggtctgtt ttaaagtcct gcgcaatctc 113520
   gcgcaccagg cgctggaaag gtagtttacg aataagcagt tcagtggact tctgataacg 113580
   gcggatctcg cgcagagcca cggtgcccgg ccggtagcgg tggggctttt tcacgccgcc 113640
   ggtggccgga gcgcttttgc gggctgcctt agtggccaac tgtttgcgtg gcgccttgcc 113700
   accagtagac ttccgagcag tttgcttagt gcgagccatg acggaaaaac agcacagcgg 113760
   aacacccaac actagcgcaa atacgcccat gagctgctct atttatagtg tgtaaagtgc 113820
   agtgattgga tgatagaaga cgctaaatat gacgttacac actctgattg gtctatcttt 113880
   aagccagcaa caatcgtgca gtttcaccgg ctactatatt ctattccaac tctacagatg 113940
   attatttaag tggtatttta ttactactat tattttattt tacttttgct ttgttcccca 114000
   agctggtctt aaacttgggc tcaaaggatc ttcccgcctc agcatccaga gtagctggga 114060
   ttacagggga gccccactgc gccggcttgg actttaattt tttaaacttg tcctcttcta 114120
   catctggttt tcataacctg aaggctgtgt ttattttcca taaaacaagg cattgattcc 114180
   aaaggtatta taattcccca attccgtata accttcagct ctttaggaaa aaaaaaaaaa 114240
   aaaaaaaaaa gagggaatac tgctcacctc ctctccggaa atgtaccctt tacgggaatt 114300
   tctgaaacct ttcacaagaa ttggattcct ttgtaatgct ttaattgact taggagtgtt 114360
   attgaaatct acaaagcatc tcaaacatag taggattaca ctattactca gaaacatttt 114420
   ctatgagacg tctttctctt gattatgctc tttgaatcct aaacttgcag cgttctgcag 114480
   cttttgtttt ctaaagccta ggtgtactct gccagtcaca aaatggcgtt tctccagcac 114540
   tgccgccagg taccaccagc tgggagttgt tcctcttgcg gagcaggagg tggacttggc 114600
   ccaagagaaa ctggatagtg gttcgcaagg aacataattt agcattgcca agagctaatg 114660
   caatcatttt gaaaatctca aaacactgaa aagtggattg tgaccttttt aaattcacaa 114720
   gagacaggcc acattctatc ttttgattgg tttaggctat tttcttgaac agccatttag 114780
   aaagcagatc tatcatcctt catttgcatg gagcgttccc attttatttg aaaccagttt 114840
   aacccaatag aaaaaaggga ggcagaaccc attatttaaa gtggaaactc ctgaatcaga 114900
   taattaggag tatttccttt tcaaaagttg cgttttttca gatacctcgc ttattacact 114960
   aagaaaggtt tatatctttc acaaagggtt tacttacaaa aatcttccaa ttttgtatac 115020
   ctgtgtttca taactgacta gccgtcaaac caagatgtag agtttccaac cgttattttc 115080
   caaattttta gaaattacgt gaaatatttg aatgcatgcc ttctcaataa aatgggacgt 115140
   aggaagcact ggtgcagaag atgggtacaa tacttatctg ggaccactcc attatttggt 115200
   tggcacgttg tttgaacaaa aaggggaaaa gctcaggtta cttagcatgg ttcggactta 115260
   tttgaaaact accacagcag gagcggaaat aagaccgcat tacctcactc tctgctgtgc 115320
   tgtgctaggg ggttatccag aataggattg tagaagtgga tgtcgattta atagtttttt 115380
   attctcccat tagctgagtc tctgattggc aatgtgagat cgttttagct tattgatact 115440
   ttgaaatgca cttaacagcc acaaacaagt taaagggttg ttaccataaa atcttatccc 115500
   cagggtgtgc ttgcatttat cacccgtgtt tgctttcaca ctaagtggac ttaactcccc 115560
   agcagaatgc ctgtcaggga accggtttcg tggacccagc atttaacgcc tttcgcaggc 115620
   ttgtgaggcc cataaatatt tgttgaataa aagaatgagt tgaccatgtc atggtgcgct 115680
   gattgcgtgt gctgacatgg aacacaggtt gtaaacctta ataccaattt ggggcatgtt 115740
   gtatggatga aaagggcatt ggaaattcct gaagtgcatc ccacattgga ctgtggaaat 115800
   aagttgcaag tgcagaaacg tttccacact tgcagtttga gtattaattg cagcgtttgt 115860
   gaattctggt gttgtctacg attcattctt gtttgacgtg aaaggtattc gcgagacaca 115920
   tcgctctaaa acattgccag aaaatgtaat agagttgatg acaactggcc ctaacacggc 115980
   ctaaaactcg cacttttctc tccctccgca actattcaaa acactgtatt ttacatttct 116040
   tgcaaattaa aaactaacat ctctggcaac ggacctctaa aaatttctaa taaaactcct 116100
   cggatgcttg tggcactgca tttgtaaacc gccccctctc aacctactcc ctaaaaaaga 116160
   gctgcttttt gagagagaag cggtaccctc tgatgttact gggcggcagt ctgcctacaa 116220
   tttccttcac aatgaggcaa ccagagcggc tttttctgtg tgtttgcttg cgttgagggg 116280
   agcaggacca taggccctag aggcccccag ctgccttctg agactgggcg aaaccctcgg 116340
   cagcgcgcag gggcgctagg ggcgcgaggg gcgggcactg acgggcacca atcacggcgc 116400
   agtcccaccc tataaatagg ctgcgttggg gccttttttt cgcatcctgc ttcgtcaggt 116460
   ttataccact ttatttggtg tgctgtgtta gtcaccatgt ctgaaacagt gcctcccgcc 116520
   cccgccgctt ctgctgctcc tgagaaacct ttagctggca agaaggcaaa gaaacctgct 116580
   aaggctgcag cagcctccaa gaaaaaaccc gctggccctt ccgtgtcaga gctgatcgtg 116640
   caggctgctt cctcctctaa ggagcgtggt ggtgtgtcgt tggcagctct taaaaaggcg 116700
   ctggcggccg caggctacga cgtggagaag aacaacagcc gcattaagct gggcattaag 116760
   agcctggtaa gcaagggaac gttggtgcag acaaagggta ccggagcctc gggttccttc 116820
   aagctcaaca agaaggcgtc ctccgtggaa accaagcccg gcgcctcaaa ggtggctaca 116880
   aaaactaagg caacgggtgc atctaaaaag ctcaaaaagg ccacgggggc tagcaaaaag 116940
   agcgtcaaga ctccgaaaaa ggctaaaaag cctgcggcaa caaggaaatc ctccaagaat 117000
   ccaaaaaaac ccaaaactgt aaagcccaag aaagtagcta aaagccctgc taaagctaag 117060
   gctgtaaaac ccaaggcggc caaggctagg gtgacgaagc caaagactgc caaacccaag 117120
   aaagcggcac ccaagaaaaa gtaaattcag ttagaagttt cttctagtaa cccaacggct 117180
   cttttaagag ccacctacgc atttcaggaa aagagctgta gtacacagat gaaatccccc 117240
   aagcaaatgc aacacgccct caattatatt agaatcactt ggagagtcga tagaacttta 117300
   acatagcctc atctagtaag aatttactac tcaatctatc aaagatagca aggtgaattc 117360
   aaatgcaccg agttaaaatc gagttttaaa gtcacctggg tttcggtagc cggaagtccc 117420
   gcgtctcacg actccaagct aattagtcat aaccgtattg aaccaaggtt gaagcccagt 117480
   cccaggcttg aggcttttta ttatacaagg ttaaagtggg gatattgcgt tttggggtca 117540
   atattgctaa agtagcattt tccgaaattg ggtggtccta agaaatgctt ctgggatagt 117600
   tggcaaaata tatggcttaa ccacgccctc tccacaggag tggctagcga gctgtctgtc 117660
   cttgggaagg acggtgaccc tgctggcgtg gctggcgccc acgttggcgt cctctgaaag 117720
   ccccgccagg taggcctagc tcgcttgctt tctgcagcgc catcatgaca aagctttgaa 117780
   acgcaaaatg ctttctttgt gcagcgcctt accatgggtg cacttacggg ctgtcgactt 117840
   ggtttaggcc cttgtcagga caaaggagct tagtttgttg gagttttaga gctgcaaccc 117900
   aaaatccctt gctcggtttc tctgttttta gaaacggaag cgccctgatt ggatatttga 117960
   aaattactgt gcttaactgg atcgtgtttc atcagtcgtg caggattttc aaccctggtg 118020
   gagcccacac attcaaaact gaagatcctt ttctcagaac tgccccttta agcttttgca 118080
   attttaattc tgggggtcag attttaataa ttggactttt ttgtttacat ctgacaagag 118140
   tatatgatga gccaagttta ctcactttta cttagtgcag ttcaattcta aaagtttatt 118200
   tttgcgtgtg tgcatatgag ttaataatca gttgtatttt tcaaacggtc ttttttcaat 118260
   tgttttgctt agctccttcc atcgtctaaa gtcagggata caggcacatc acatccctgt 118320
   tcccccttcc tcaaactaat atgtagctac ctaggtttat cctttaaaac aaaaattctc 118380
   acctattttt gtgagaaata tacatgtttt tctttgaact aagtatttta catacaccta 118440
   tctatataca tgcatacttg tggttttgtt tttttaaaaa aaaaaaaaaa aaaacacgtt 118500
   atcttttgag actgggtctc agtctgttgc ccagactgga ctgcagtggc ataatcacag 118560
   cacactgtaa cctccaactc ctgggctcag gctatcctgc agcctcagca tccggagtag 118620
   ctgggattgc atgcacgcac caccaagccg ggctttttgt ttttattttt tgtggagaca 118680
   gtcacaccat gttgtccaag ctggtctaga aatggcctca agtgatcatc gacctcccaa 118740
   agtgttggga ttacggtcac tgtgcctggc cttgtatgca taattgtttt gtcttttgat 118800
   tagggttatt aatttaaaaa acaaagcctg gacgcagtgg ctcacatctg taatcccagc 118860
   actttaggaa gccagatggg cagattactt gagctcagga gttcaagacc agcctgggca 118920
   acatggtgaa atcccatctt gacaaaaaat acaaaaaatt agcaaggccc agtggcacgc 118980
   acttatagtc ccagctactt gggaggctgg ggtgggaaga tgactggaac ctgggaggta 119040
   gaggctgcag tgagcagaga tcgtgccact gcactcaagc ctaggtgaca gaatgagacc 119100
   cagtctcaaa acaaaaataa taaaaatttt ttacaacgat gttatataca cttctgcatg 119160
   ttgcttttct cttaaccaaa cttttctaaa accctgtcat gaaaaaagaa atccttcaca 119220
   tggaatagca taagttattc atccatttct tattgataag cattgatgtt tccagttacc 119280
   actgctgaac atggtgcaat tgaatagaat tccagggctg agattgctag gttttaggtt 119340
   gtattttatt atttatttta tttatttatt tatttagaca gagtcttact ctgtcaccca 119400
   tggtggagta cagtgccatg acctcagttg caacctttgc ctcctgagtt caagcgattc 119460
   tcatgcctct ggtctcccga gtagctggga ttacaggcac ctgccaccag gcctggctaa 119520
   tttttgtatt tttaggagag atggggtttc accatgttgg ccagactggt ctcaaactcc 119580
   tggcctcaag tgatctggcc acctcggcct cccgaagtgc tgggattaca ggtgtgagcc 119640
   atggcgccag acctggactt tgtcttctgt ttcatcagtc cttctgttgg ttcaagcaca 119700
   gtatcacact gaagactgat gattctatat aaatatggta aagactgtac accctaactg 119760
   ttcttatttt ttaattttaa ggcaatttta gattccagct ttccaaagaa ttgtggaatg 119820
   cttagagcta gagaagcctt ggaagtcatt tagtttttgt tttgtcagag aaaattctgt 119880
   agagactctg tcctgctctc actgaatacc atcccatagt accccccaac agctttaaag 119940
   ggcaataata ccttatggac agtatgcttt tcctcaaata tattctaagc catggtcaat 120000
   gcaaaagagt gagaaggaaa gtagaataag ttatctaaga atcagtgggt gctctcttta 120060
   aactgattta tcactccccc ttccaaactc tcttgaaggt cactctgcct ccctttctac 120120
   ataagaactc ctaactccaa gggaggaagg taagttattc ttattccttg cttagaaaaa 120180
   gagaaaatag gtttggtaag catccgcttt ctgctaccat tctctgtgtt tctgtgtttt 120240
   ttataggatc attcaattat tggttggctc ttgagaggga atgcaaggtt caaggacaca 120300
   agcctagatc ttgcctgtat agaacctcat gatgttatgc ttctctaaaa tgaggcctgg 120360
   aggagacatg ttgaaagtga cccataaatc tgcagtatct catgtctctc aatggggaca 120420
   aggagtacca tgggaaatag cattaggtca atgacagtaa caactcccag gtgagttgat 120480
   ttattctttt atttataaag ttgttaatat gctacatagt ccctaatttt gccacaaata 120540
   gtcattattt taatttcata tttcactatt gataaatgaa ggaaaaaatg agtagcagtt 120600
   aagcagtcca taaacctaca tataaagcaa attggagatt ttaaaattga ttctggatgc 120660
   ttaaaatcct tctcattgaa aaaaaatttc gtattagaag atttcaacat tctttaaact 120720
   gagaagcata acatataaac agaaaaccac agcaaaacaa aaatgcaaag ctcaataaat 120780
   gaacacaaag tgaacaccat aataattgcc acacaagtaa aaaaacagaa aatcagccaa 120840
   ccctcccaga gccgcctgat gcttgcttcc agtcacatta tcactccatc tgccctaaac 120900
   ataaccccta ttttgatttc caatgctgta atttagtatg cctgtttttg aaacatataa 120960
   aatggaaata aaacaaatgt aatcctatgt acctgacata tttcactcca gaacattagg 121020
   tttgaataga ttcatctgtg ttgctgtgta taactttaat tcatttttat tgttatgtaa 121080
   tattccatgt tatgagtgca acaatttagg tgtctactgt tgatgcatat ttgcttccct 121140
   ttttcagcta atataaacaa taccgtgaat attcctgtgt atgtgtcttg gtatatatag 121200
   gaatacatat tttgtttgta tacctaggag aggaattgtt gggtcaaatg ctaaactctt 121260
   tttgaaagtg gtgatattag gtttacatgc gatgaaatga aaattaaaac cacagttata 121320
   aacagcatgg atgaacctca caaacctaat gttgatggaa tctagctggg aattcctgtt 121380
   cttccatata cttcccaata tttttttcca attaaaattg ttaatctttt gaagatgtta 121440
   tccattgtgg cagatgtgca gtattatctc attatggttt tattttacat cttttgccca 121500
   ttttttctta attggattgt atatcagtcg acttgggctg ccataacaaa aatactagac 121560
   taggtagctt gaacaaaagg agtttattac ctcacagttc taaaggccag gccagaaatc 121620
   ctaaattgag gtgccaagag attcagtttc tagtgagggc tctcttattg acctgaagat 121680
   agttgctgtc ttagattgtt tggtgctgaa cagaatacca gagaccaaat aatttataaa 121740
   gaatacagat ttatttctta caattctggt ggctataaag cctatggtcg aggggcccac 121800
   ctctggcaag ggccttctta ctgttatggc agatgtgaga tgtcatctca tattcaaacc 121860
   acagcagtcg ccttttgtgt cctcatgtgg cctcttcata tgcccataaa atgacctcat 121920
   gtctcttcct tttcttataa ggacaccaga tctatcagac tactggccta ctcttatgac 121980
   ctcatttaac cttaaatatc tccataaagt cccaaaatcc ctatctccaa atataggcac 122040
   attgggtgtt agagtttcaa catcaatttt gggggaacac aatttaggcc aaaaagattg 122100
   tgttttttct tgttggttta agatagctgt ctttttgtcc tttttgtcct ttcttttttt 122160
   ttgaggtgga ctcttgctgt gtcacccggg ttggagtgca gtggcgctgt ctcagctcac 122220
   tgcaacctcc acctcctggg ttcaagaaat tctcctcctc ccaagtagct gggactacag 122280
   gtgcatacca ccgcgccctg ctaatttttg tatttttgat agagacgggg tttcaccatg 122340
   ttggccaggc tggtctcaaa ctcctgacct caggtgatcc acctgcctcg gcctcccaaa 122400
   atgctgagat tacaggtgtg agccaccaaa cctggcctgt cttttctgtt ttaagttttt 122460
   aaattttgct cacgaaccct ttatccattt tatgtgttgc aggtatttcc tctgtaactt 122520
   gtcttcactc tgtcagaggc tggagtgcag tggcacaatc acagctcact gcagcctcca 122580
   cctcccagga tcaagcgatc ctcccatctt atcctcctta gtaggtggga ctacatgtgc 122640
   aggccaccat gcccagctaa tctttgtatt tttttgtaga gatggtgctg ttgcccaagt 122700
   tggtctcaaa ctcctgagct caagcaatcc atcaaccttg gcctcccaaa gtgttgggac 122760
   tagaggtgtg agccaccact gcacccagcc aatgatatct catgatgcat taaagtcatt 122820
   aatttagtgt actcaaatta agcacactgc ccttttatgc acaacctttt ttgtatctta 122880
   tttaaaaaat cattttctat ttcaaggtca tgaagatctt attttataat accttcttgt 122940
   gaaattagtt ctcaagacta ccctcacttc taacaccaat tataagttgg gaggtctgtg 123000
   gttcccaatc aaccttaggt tagtaatttg ctaaaaggac tcacagaact tgctgaagct 123060
   gttagcctca tggttacaat ttattatagg atatatagct tattatgtca ttccaatgca 123120
   atgtaaaatt atacaactac ttttaaaaag attttagcat ttgacccaac aatttcactc 123180
   tgaggtatac aaacagcaga tatgtgtgca catatatacc aagacacata cacagcaaaa 123240
   ttcattgttt gtaatagttg aaaaggggaa acaactcaag gaataaagat taaaatcagc 123300
   tgagaaaaga aacacacaag gcagtattat ggatcgaatt gtatgcagat ctcccttgcc 123360
   cccagaagat atgtttaaag tcccaactcc cagtacctca gaattgtggc cttatttgga 123420
   aataggatag ttgcagatat aattagttaa gatgaggtta tagtacagta tgatgggctg 123480
   gtgacttaga agaagtagta tatatatatt ttttaataga actagtattc ttctaaggtg 123540
   gtcacgtgaa gacagacaca cacaggcaga gactgaggtt atgcagctgc aggtcaagga 123600
   atgtcaaagg ttgccagcaa gtacgagaag ctaggaagag tcaaggaagg attttcctac 123660
   aggcttcagt ggaagcatag atctaatgat accttcatgt cagatttcta gcttccagaa 123720
   ctacaagaga atatatttgt tgttttaagc caccctagct tctagctctt tgttacagca 123780
   gccctaggaa actaatatag gcacaatcca ggcaagttcc aaatatgagc ttccagttgt 123840
   cctctcccag taatatgaac agtattactt tcccagcatt aatgtgtgac aatacacatg 123900
   acgtacagag cagtccccac ttatgcacaa aacatatgtt ccaggacctc cagtggatgt 123960
   ctgaaaccat ggatagtact gaactctata tagctgtttt ttcctataca gacacagcta 124020
   tgataaggct taatttataa attaggcaca gtaagagatt aataacaata aattagaata 124080
   attgttaaga atatactgta taaaagttag gtgaatgttt atttctgaaa tttaccgttt 124140
   attatttttg gactgcagta gaccacagga actaaaacca tgtagaaacc gtatacaaga 124200
   gaactgtatt tcacccgagc ctcagtgtgc agttttaatg gcctgccatg gttgactgct 124260
   cacatggccg atcttttagt ctacctccac aggtagagct gatactgtgt ggctcaaagt 124320
   tcctattata aatcacattg ttgactgtgt ggtggtcaaa acctccaggt aaacaaagac 124380
   acacttatca gtgagaacat ttcaagggtc taaaattcat ctcccagtag ctgagggcaa 124440
   aggctagacc tctttttggg taagataaat tttttaccat atactttatt ttgcttttca 124500
   tgtttaactt tattttgctt ttcatgttag ttcccctgga attgtttttt gtgtatagtg 124560
   tgaagtaggg ggtcaagttt cttttttttt cctttttgtt ctttttctgt ttaaaaggct 124620
   atacaattgt cccatgccat ttatttacaa gagtcctttc accattgttg tatggtgcca 124680
   ctttagatgt aaatcaatgt ccatatttgt ttgagcctgt tccattcgtt tgtctatttt 124740
   tggacaacac tgccctgatt attgtcattt tatcagtttt gatatttaat aaagcaacag 124800
   atttgtttat tttgggccct tggatttgtg tattaaattt gaaccctgtt tgtcaatttc 124860
   tataataaag cttattggga atctgattag gattacaatg gttttgtaga tcagtttggg 124920
   gacaattaat acctttaaaa tattgaccgc ttcaactgta aatatactcc tccattattt 124980
   agttttcctg tttaatttat ctgagtaata cattatagtt ttcttcgtag aagtcagata 125040
   cgtagaaaat tcaaagccca agtgcaatag ctcatgtctg taataccagc actttgggag 125100
   gccgatgtgg gtggatcacc tgaggtcagg agtttgagac cagactggcc aacatggtga 125160
   aacctcatct ctagtaaaaa tacaaaaatt agctgggtgt ggtggcgggc acctgtaatc 125220
   ccagctaatc aggagactga ggcaggagaa tcgcttgaac ccaggaggca gaggttgcag 125280
   tgagccaagt tcctgtcact gcaccccacc ctgggcgaca gagcgagact tcgtctcaaa 125340
   aaaacaaaaa aaagaacatt caaataatca atgtagataa ttcaaataac taaaaaatga 125400
   acagttatta aaatatcagg atataaaagc aaaaaaatca ataacctcca tatatacaaa 125460
   atggccagtt agagaaaaaa aaaagaatag gcgagactta aaaaggctgg gaatctccct 125520
   gaaaatcttt gagagccttg gccctgccct cagggatttc tctggcttca tgcccagata 125580
   cgggtacagt tccttgttta aaaaaatttt gctccatcaa tcaacaaggg gctccttcct 125640
   cagagcacaa ggacctccat aacaccggac actagatgtc taagggacac ctcttaagga 125700
   agttagactt ccaaagaatg gtgtttcctc tgtccccaaa ctctggaact cacagcacaa 125760
   ctgctccttg gagttcggtt tcaaatctac aaggctgtca tggaggttgc agaccaagtc 125820
   cgtggcctca gtgtccggat gtacggtggc cttggcacct gaatgtgaga acatgacctc 125880
   cctgaaacca ccacaagtat tgtttcatgt tatgtatgtt ttttcttatc tgaaattcct 125940
   tttctttaaa aattcaaatt acatattttg caagcccctg aacaagcttc atgagcattt 126000
   attgaaccca cagcttttaa aacctactga acactttgct ctatgttgtc attcactatc 126060
   caccaattat ttaattattg atcaatattg tttccttagt gttgggatca tttatgcatg 126120
   tatttctttt atattgcata ttttatattt ctgcattaca gttattacat attacttttg 126180
   ctacagtaat agttcaaaag tgtacatcca aaatttagct gtgaagtgga tggactgagg 126240
   cagaactgga ggcaagaaaa tgtcacagta attctaaaaa agatgatgta caattagagc 126300
   aagagagtag cactgaaatt gaagaaaaat agatgcgttt gagagaaaat taggaggtag 126360
   aatcaacaga ttagatgtag ggatgagaag ggtcaaagat gacactaggg tttttaactg 126420
   gagcaagtag gtagacagaa catttcttcc tgaaagggca ggtcagatca tgtgttgtct 126480
   caaagggcat gaagagtaga aagcctggga cagatcctga gatgaccaat acccatggtg 126540
   cagggagagg gagggagatc tgctaaaaag actgcaaatg tcaggatagt agaaaatcat 126600
   gagtgtgtga tgtcctggaa gttgagacag tatcacattt gagaacattt aaattggtaa 126660
   ctctgacaaa aagctggagg ccaactgtga atgcccatga gagtgagaag ctcccacact 126720
   tttgtgggca tcagaaagcc caccaggttc ctgcagtgaa gatctgagaa ggatcctctt 126780
   gtggctttgg cagggagaga agaattatta tgaaatacac cccagaacct tcttcaaaac 126840
   aaaggcctac tctcaagggg aaaacatttt gccagagtct tatcccagct gggagaaggt 126900
   aattcttccc actgcagcct catctaggct ttctgtctca cttaaggaaa gaaaattagt 126960
   caacagggat cagagcttca tgaaaataaa ttggaaatgg tgcagccagg aaaggagcaa 127020
   aggtctgagg aggaggagaa ggaggaagag gagttgtatc attataaata cttgaggaag 127080
   aggaggagaa ggaggaggag gaggagttgt atcattataa acacttgagg aagaggagga 127140
   ggagaaggag gaggaggagt tgtatcatta taaacacttg aggaagagga ggaggagaag 127200
   gaggaggagg aggagttgta tcattataaa cacttgtgac ggtcccagcc ccaagatata 127260
   ggcatgctaa taaactgagg cttaacactt tgactacaga atgctgcttc tccctaacac 127320
   catcaaggct ccaactgaat aacaatgaat tatgaatgaa agagctgtaa ggagagacaa 127380
   aagttagaat gagacaagta ttgttatcta gagatgccaa gaaggcaagg aagataacta 127440
   aaaaggcact ctggatttag aaataggaag tcattagtga ccttgtaaat aatggagcca 127500
   gaggaatacc aagggcagaa gcctcactat agtgtgttgc acctgtcaga ggtcaggagg 127560
   tgtaactgac tctcccacag tgtggctttg gaagagagaa gtcagcagct gcatggagat 127620
   ttgggagagg gaaagctttt tttttttttt tttaattgga aaagactgag ctatgtgtaa 127680
   atagaataag acaggaagag tgtagacaca ggaaagaggg cagacaaaaa caagtgcaca 127740
   gttatctaag ggaaacaatg ggatcaagct gcaagtatat aaacttgtct tgatagaaga 127800
   atccttgatc tggtttattc agtgtttggt ccaaacccac atccctgttc tgcctgtctc 127860
   tgacttgctc tgtgccccag aagcccagct tctacagata gcattagctg ggcagccctg 127920
   ccctcttgca acagctggat ttggccagtg atcagcccag caggaatgta gatggcaaag 127980
   gagagagagg ttagtgtact tattccctgc atcacccccc tgcttggtgg gcagctcttc 128040
   ctccacagtc ccagctctgg cctagctctg gttacaggtt ccctcccatt gcctcttcag 128100
   atttaaaggt gtgtctgtca gggtataact gggagctaga aattgcactg aaattgaaca 128160
   aagaatttta tgggaatggt tgttaactag ttataagagg actgaaaatg gaaaagtgga 128220
   caaacgtatc agagatagta atgacagaaa gcaactacca cctccaggtt taggagaaca 128280
   aggaaaagat tctttgaaga gatccccaga actgggacct ctgaggagtg tatgctggac 128340
   cactgatgat gatatgtctg tagatagagg catgatgagg ctgattttag gagcatggaa 128400
   gatctccaaa ctgaagccaa ctgctgttac tggattcaac tgccactgcc aggttgaaga 128460
   acccattctg tgaggatgtc aacaaacaaa gtgggaaatc ttttcacatc cttccagccc 128520
   tctagtcttc ctccagtgct ttctattggt agggtttggg gaggtggcta gcaaagcggt 128580
   attggaaaag atagaagaga ctaaatcttc ataaccagca cagggtgaca ctggatcact 128640
   actgttgctg atcttgggct gcctcatatc ccctgttctt cccattagcc ctgtcacaac 128700
   tttgtagata tcccttcatt atatgccctt catatattct tttggtttaa ctttttctgt 128760
   tggaatccta atatggcact cctccatttt tcaggaccaa aagagtataa aagattatct 128820
   tttaccaaaa aaaagacaaa aaactgatct aattcctgat ttgatcatta cacaatctat 128880
   acatgtatca aaatatcaca tagtacccca taaatatata caactgtgtc cattaaaaat 128940
   aaaaattaaa gaaaagatgg taaatatagc tctgtcaggc agtggaggtt ttaccacgat  129000
   ggctgttatt tcccccatga aggggggagt gagggagcag ctgaaagtag gtgcttatag  129060
   gggtatagag gggctcaaag ctttgagaga ggagaatgtc tgaaagagct gccaaatagc  129120
   atgcaggtcc catgggggca gagcctctgc tcattcacca gtgcctcttc aatatctaca  129180
   cttaagccta acacaaagtg tgtgcttaat aagtatttgc tgagtatgta aagtggaaac  129240
   agaaccaatc tggcaaactt tgtaggactg gtgggcaatg aagatcagtc aggtaaaatc  129300
   tgtggatata aatttatatt gatcaaaaaa ttcaaggtta ggtgtttttc ttcagtcatg  129360
   ctcaacgatg cttcagccat gctcaactct tctgtagcca cagaaaaaag tttacccata  129420
   atcgagctgt gtctgtgtct gaataatgaa aagaccatga tgcaagggag ttggagacac  129480
   agaaacagtg tttgaagtaa tgggtaatgg aagcatgcta ccagggaaag gaaagaagtg  129540
   gcaataggaa ggaacagaga tctgtggtcc tatgtcccct gagcatattc acatgttaaa  129600
   gctaattcag ttttcaatca tcattaaaat tttgttccta aatatatggc cattattttc  129660
   cacaaccaca ctaaaacttt attacctctg gcaagtgact atgcaagtaa ctaagagcaa  129720
   aaatatccac aactaccatt tgagctatca atttagggaa agtcatctgg ctataatcta  129780
   agtgaccctc cactgaatgt cagtatcttt gcatatgtga tttaaatctg ggccttcgca  129840
   acaccatgaa ctgttcttgt cttgaatatc cagattgaag gaaataatct gagtagttac  129900
   gagtcctgaa gctagaaaga tggaaacccc atttgctcat cagaaagcct tagagcttgg  129960
   gcgctggcgg gtcctgtctc accgggacag aggggctctt tcctccccat ctgatagtct  130020
   gataactaga gaagccggcc aacttattct ccaagaagga gccatcttag ttcctcctga  130080
   aatgttcata tttagaaatt attgtttgtc agtaatttaa ccccttaatg ggcttgcctt  130140
   gtggtccata ccactgagtg cagagcttgc ctggaagaat tgtgagggcc attccatctt  130200
   ccaggcagta gagttcagta cttctttaaa attgctgctg aactctgtat ttgaaaagaa  130260
   agaatcattt gggtgtggta gctcacacct gtaatcctag cgctttggga ggctgaggtg  130320
   ggaggatcat ttgatgccag gaggaccact tgagaccacc ctgggtaaca tagcaagacc  130380
   ctgtctttag aaaaaaaaaa tacaataaaa taaatacaat aaaaataaaa gcaaaaagaa  130440
   agagtccatc ttagggacag actgtaacta ctcactggag cttaccttta catagttcag  130500
   gatcaattat aataaaacac ttttgtgcag attcaatagg attattttaa tccccatcat  130560
   ctctctgagt ttccagtcag tttctctgca tgtagacacc cttctccagc ccaccattgt  130620
   ctctcctcct atagctccac caacaaatca gaactttttc taactgcacc tagtgcacct  130680
   agagtctact ccagaatgct catggagaaa gtttctgaaa ggtaaaactc tgaatgatat  130740
   ttgtagctaa agggagactt gctagagaca ataagctaat agttgtagac ttcagtagaa  130800
   gaggaatgac actgcaatgt cagggtgcag gacttcaaga gggcagagta tggaaaccca  130860
   atgggaaaaa tgctcaccag gaacatgaag agaaggaatt acgtgtaagg atttctcaat  130920
   gtgttcccaa atttgcccag cagagggagg cctcgggttg atggcaggct gaccacacaa  130980
   ttaaagaagg ctgaacctgg gggcttttaa caaccatcgt gggctctact gtaagcattt  131040
   agaaaaagaa agttatccat tcaaaaatat atatattttt aaacttcaga acaaaattat  131100
   gaagagctat atttactttt ctacattcta atttttataa atctgagtat attttgcata  131160
   tattgttata gtacatattc aattttgtat tttgctgttt tcacttaacc atttttacta  131220
   gattactctg tgttcataat aatcactttt ttaaaacttt tatttttatt tatttatttt  131280
   ttttttgagt cagagtcaca ctctgtcgcc caggctggag tgcagtggcg tgatcttggc  131340
   ttactgcaac ttccacctcc tggattcaag cagttctcct gccttagcct cctgagcagc  131400
   tgggattaca ggtgtgcacc accaagcccg gctaattttt gtatttttag taaagacggg  131460
   gtttcaccat gttggtcagg ctggtctcca actcctgacc tcatgatctg cccaccttgg  131520
   cctcccaaag tgctgggata atcacttttt atgctgcata attcttcaga tttgtcagta  131580
   cgactgtatt tacactcatt tgttttatta gaaagaattc cagaatattt tggctgccct  131640
   aattaatttt acaattaata tgattttgaa attgggtatt ggctccttct gaattggttt  131700
   attaaaatat attctaatgt aatttatgac attttcatca tattagcata tttattctgt  131760
   tagaatttca taatttataa agctacaaac tgtatgtgat atagcttgta actttatctc  131820
   ataactttat gcagttacaa gtagaaataa aatgttcccc tcaagattgc ttaaaatttt  131880
   attataaaca agtgtaaaaa acaaaatcac taaaacactc cctctttttt cccccaaaat  131940
   gcatgtttcc attttaacag aacccgtatt taatcagcag atttctatgg tggctagatt  132000
   tgtagactaa atattaaaag tcccaaagca aatgcatttt tctcttaaat tttactgact  132060
   tttttttttt ttctttttct gagacggagt cttgctctgt cgcccaggct ggaatgcagt  132120
   ggcacaatct cggctcactg caacctccgc ctcccggatt cacgccattc tcctgcctca  132180
   acctcccgag tagctgggac cacaggcgcc cgccaccacg cccagctaat tttttgtatt  132240
   tttagtagag acagggtttc accgtgttag ccgggatggt ctcgatctcc tgacctcatg  132300
   atctgcccac ctcagcctcc caaagtgcta ggatcacagg catgagccac cgcgccccgc  132360
   ctactgactt ttatccaaag aaaatataag agctcttcat cataacgtat gtttcttgct  132420
   cttgttatta aatatgacac atttagactt aaactgattt gaaggtttat gacattgttt  132480
   aagttattac ataattaatt cataaagata atgactagtt tgaactactg acagctcaca  132540
   catcatcagt tgaacagcag aaagcttact aagctacttt cttatgtttc tgtctcccag  132600
   ctactaaaag aaacgaaacc cttccaggtg ttaaggcaaa actttcctcc ccctttcttc  132660
   tataaatctg attccatgtt agtgaaattt ctactgatgg ctttggtttc ctctatagta  132720
   gaatagagat cctatggcaa aagtcatgtc tgacatggta gcaaatagaa atggggaaaa 132780
   ggaaggtctg caagagccaa tgtgggaaat ggggagagga ctgactacaa aaacccagca 132840
   ggaattccag aagaaaactc ctcaggacgg gcacattggc tcatgcctgt aatcccagta 132900
   ctttgggagg ccgaggtggg cagatcactt gagtccagga gtttgagacc agcctggtca 132960
   acatggcgaa acctcatctc tacaaaaaat aaaaaaattt gtcaggcgtg gtggcatgca 133020
   cctgtagtcc cagctactca agagacttaa gtgggagaat cactcgagcc ttggaggtgg 133080
   aggttggtga gccgagatca cgccactgca ttccagcctg ggcgacaaag tgagacgcca 133140
   tctcaatcaa tcagtctcct cgaaaagcaa cattatggag agacaggatt ccgtcaaggc 133200
   ctggggcaca caggaaaata ttaaggcaga agagagtttc ctccccacac cacaccgtat 133260
   cccacaggca ctgcggatgt gcatatgcaa gaggggttga tcctaagaat ttagagtcac 133320
   agaggaggag gcaccaagca gactgtggag aaagtcatga ccagaaaggg acagaatgta 133380
   aagcttcagc tgattatctg gcctcaggga ttccagagga actggtccca atggtctcct 133440
   ggtgatgtag gttcttaggt ttcttttaca ggggttttct gggagatcgt tgacccagtt 133500
   agcattcaag caacttccac cctgcacttt tattctttcc ccttcacctg cttaggtttt 133560
   atctgtccag gaaataataa taaaattatt gagccctgga catgtacctg taaagctcct 133620
   taaagatgat gccttctaac tcctcattca acagatacaa aaacattaca ataaaatgac 133680
   tcatgcaaga cacccaggta gtttatagca gctaataaaa acagaataac tataaaatat 133740
   ggtaagttta taaaagttac attgagtata ctttataaga actgcttatt gagtttgcct 133800
   aataaccaca cagcacaata ataatatgta tatattttta aatatgtgta aatatgtgta 133860
   acacaaactt gtagaaggta tatctgagta caaccctatt ctgtttggtt accttttcta 133920
   gttcattatg taagtggcat agctacctaa ggacttatgc ttataaatgt tactcaaaaa 133980
   aatacagagg acatatgtgg atagataatg gaagagataa gataggtagg ttgaagggtt 134040
   gggctgcccc tccacacctg tggttgtttc tcgttaggtg gaatgagaga cttggaaaag 134100
   aaagagacac agagacaaag tatagagaaa gaaaaaaagg ggtccagggg accggtgttc 134160
   agcatacgga ggatcccacc ggcctctgag ttcccttagt atttattgat cattattggg 134220
   tgtttctcgg agagggggat gtggcagggt caaaggataa tagtggagag aaggtcagca 134280
   ggtaaacacg tgaacaaagg tctctgcatc ataaacaagg taaagaatta agtgctgtgc 134340
   tttagatatg catacacata aacatctcaa tgacttgaag agcagtattg ctgccagcat 134400
   gtcccacctc cagccctaag gcagttttcc cctatctcag tagatggaat atacaatcgg 134460
   gttttacact gagacattcc attgcccagg gacgagcagg agacagatgc cttcctcttg 134520
   tctcaactgc aaagaggcgt tccttcctct tttactaatc ctcctcagca cagacccttt 134580
   acgggtgtcg ggctggggga cggtcaggtc tttcccttcc cacgaggcca catttcagac 134640
   tatcacatgg ggagaaacct tggacaatac ctggctttcc taggcagagg tccctgtggc 134700
   cttcctcagt gttttgtgtc cctgagtact tgagattagg gagtggagat gactcttaac 134760
   gagcatgctg ccttcaagca tttctttaac aaagcacatc ttgcacagcc cttaatccat 134820
   ttaaccctga gttgacacag catatgtctc agggagcaca gggttggggc tagggttaga 134880
   ttaacagcat ctcaaggcag aagaattttt cttagtacag aacaaaatgg agtctcctat 134940
   gtctacttct ttctacacag acacagtaac aatgtgatct ctctctcttt tccccacagg 135000
   aggtgatggc cggaagaaca tggcagaggg caaaacaaaa cagcattggg aacaagctct 135060
   gtttaaaagg agacttgtga acagcaaaga gtagaaaggg ttctcttaca actgaagccc 135120
   atggaagaca aatgtgtact gcgtgagttt taaggcaata ggagtagtgg gacctagggc 135180
   acaccagaga gcatattaac tctcaaactt ttaaaaacat tatatctgct ggacacagtg 135240
   gctcacacct taatcctaca actttgggag gccgaggcgg gcgggtgtag cttgagccca 135300
   ggagttcgag accaacctgg gcaacatggc aaaatcccgt ccctacaaaa caaacaaaca 135360
   aaaaacaaaa ttagccaggc acggtgatgc gtacctgtgg tcccagctac tcagaggctg 135420
   aggtgggagg atcgcttgag ccccgggagg ttaaggctgc agtgagccat gataatgcca 135480
   ctgcatctca gcctgggcaa cagagggaga acctgtctca aaacaaaaac aaaaacacac 135540
   catacccaac cacaatgcat ctgtcttaag taccagtacc acacccctct actcactact 135600
   aaataggtga gttcccaatc cctggtagca ggtttaagca tgttatatta aaggtcttag 135660
   gctagtgact cattcactca ttaaacaaat acttattgtg catctactat aaactaagta 135720
   ctgtgctagg tacaaaagca aataatctaa gctctataaa ctttactttc ttcatcaaca 135780
   aaatggagat gttttaggca tctactcatc attctgagct ccatcttttg tgactgtagt 135840
   tggcagagct ttttatcagt ttctctaaat agctctacca gtccctggtg gatgctggca 135900
   tgcccaaagg atccatcctg atggccctgt ctgcttacct tacctgcctg cctttgcagc 135960
   accgctctgc tcttctgcag gacttccctt atcctttggg gtcttgctgc tcttaggctg 136020
   ctctgcttgt tttgatctgc tttgcatcac atgtatgtaa aggtcctttc cttatttacc 136080
   catgaccaag gtattatgag attctggaat ttccccaaac cacattgatt gctgggagaa 136140
   tagaagaagt ggattacaag tggaacttag aaggggagta ttcgagaaga cgtctctgca 136200
   aatccattta gagagacctt tctccagtgg tgactcaaag atgcagctcc tttcatcctg 136260
   tggcttggcc atcttcagca catggctccc aaggatgtcc tcaggatggt ctctaatcca 136320
   aggagcctga agagaaaaaa aggcatggag tattgtgagt ggtaggtggt tatggaccag 136380
   ttatggaaga atacacatca cttttgccca ccttctacta accagaactc acacagccat 136440
   agacactgac aagtaggact taacaagaat ctaattttga gtctaggaat acgactgtag 136500
   caaatattta acagcttcaa acacaggtgc attgctatca ctatgcttgg cccaggcctg 136560
   tctccctttc ctgccatgtc acaggggcca gcatttatgt ctagattggg ttggttggga 136620
   tattaagaca ataatgaacc aatacaacat cttgagcata aaaccaactg atacaatgat 136680
   gtacaagtca gatgattctg atgattatga attatgtcaa taaaagaaat gtgataacta 136740
   aggtaatttt tgttttggca aatttttgtt tgttcatgac aggatgaaat cctgtcattt 136800
   gtagcaacat ggatggaatt gcaggatact acattaagtg aaataagcca gaaacagaaa 136860
   gttaaacacc acatgttctc acttatatgc agaagctagc taactaagta aataagttta 136920
   tctcattgaa gtaaaaagta caacagagat tactagaggc tgggaatggt aggggaaaga 136980
   gatgataaag agagattcgt taaaataagt tacagctaga taagagcaat cagttctagt 137040
   gttctatttg tactacagaa tggcaatagt taacagtaat aaataatttc aaagagctag 137100
   aaaagaggac attgaatgtt tccaacacaa agaaatgaga aatgcttgaa ataatggata 137160
   ttctaattaa ttaccctgat ctgatcacta tacacagtat gtataaaaat aacactatgg 137220
   gctgggcgca gtggctcaca cctgtaatcc cagcactttg ggaggccaag gtaagcagat 137280
   cacttgaggt caggagttag agaccagtct ggccaacata gtgaaactcc atccctacta 137340
   aaaatacaaa aatcagccag gcgtggtggc atgtgcctgt aatcccagct actcaggagg 137400
   ctgaggcaag agaattgctt gaacccagga ggcggaggtt gcagtgagcc gaaatcgcgc 137460
   cactgcactc cagcctgggt aacagagcaa ggctctgttt caaaaataaa taaatacata 137520
   aataaatatt ttttaaaaaa agaacatcac tatgcacccc atatatacat ataattatta 137580
   tgtcaatttg aaacataatt ttgaaaaatg aaaaaatgaa acacaaatat gaatcaatcc 137640
   tctccaagtt gatatactta aaaggaaaaa agtccgaggg cttaaactat tcaatcaaaa 137700
   ttttattaaa atgctatagt aatctggaaa gtatttcaga atgaattggt ataaggttag 137760
   acacaaagat cagtgaaaca aaacagagaa cccagaaata gattcacaca tctatggaca 137820
   actggttttg acaaaggtgt caaggctatt taataagtaa aaaaatcgtc ttttcagtaa 137880
   atgtttcttg aacaagtaga catccggtgt gggggagagg agcaggagcc ttacctcaaa 137940
   ctttatgcaa aaattaactc aaaatagacc atagacttaa atgtaaaagc taaaattata 138000
   aaacttcttt aaaaaatagg agaaaatcat caacacccta ggattagcaa agatttcttt 138060
   aaaacaaaac aacaggttta tagtttataa aacataaata acaaaatgat aaatttcatc 138120
   aaaagtgaaa atttgctttt caaaaaacat tataaaatga aaagcaggag gctgaggcat 138180
   gagaatcact ggaacccggg agctacaggt tgcagtgagc caagatggtg ccactgcact 138240
   ccagcctggg tgacaaagtg agactcttcc taaaaaataa ataaataaat aaataaatag 138300
   aaaagaaaaa gaaaaatcac aggctgagag aaaatattta taatacatgt atctgacaaa 138360
   ggactcgcac ctggaaaata taaggaacct tataacttag taagatgaca agccaaaaca 138420
   aagagtaaaa gttttcaaca gacatttcac aaaagaaaac atacaaatgg ccagtatgca 138480
   catgaaaaga ttttaaacat cattagttac tagggaaatg caagtcaaaa ccacaatgag 138540
   atacttcaca ttcaacagaa tagctaatgt taaaaggact gacaatcccc agggtgagca 138600
   agggtgtgga ggaaactact ctcatatatt gtgaatgtaa gaggacaatg ttacaactac 138660
   tttgaaaaaa gtttggctgt ttctaacata aaattaaaca cttatacagc ccagcaatat 138720
   ttctgggtca tttctcccag ataaatgaac acatgtccat actatgacat gtacaaatgt 138780
   tcatactggc tttgtttcac aatgctataa actggaaaca acccacgtgt ccatcaacag 138840
   gtgaatgggt aaataaattg taatatatcg gccagacgca gtggttcatg cctgtaatcc 138900
   cagaactttg ggaggccaag atgtacggat cacctgagat caggagtttg agaccagccc 138960
   atccaacatg gtgaaacccc atctctacta aaaaattagc tgggcatggt cacgggcgcc 139020
   tgtaatccca gctactcgga aggctgaggc aagagaatca cttgaaccga agaggcggag 139080
   gttgcagtga gccaagacca tgccattgca cttcagcctg ggcaacaaga tggaaactcc 139140
   atctcaaaaa aaaaaaaaat tgcaatatat ctatatcttg gaatattata aagcaataaa 139200
   agggaataaa ctactgatat atacacaaaa tggatgaatc tcaaaaatgt gaaggaaaat 139260
   aaaaaataca tatgatataa attccattca tatgaaattt taggaatggg aaaactaagc 139320
   tgtaattatg gaaagtacat cagtggctgc ctggggccaa gaggatggaa gaggcggcac 139380
   aggtgatact acaaatggaa actatctagg ttgacggaag tgttctgtaa cttgattaca 139440
   gtagtaactg tttgggtata taaaacgcat caaattgtat aattaataca ggtgtatttt 139500
   actgtgtata aattattcct caataaagtt gatttttcat taaatatatt atttgctaaa 139560
   atgaggagag acaactatta tcttaaaata gttaagcaca ataaaaatac tacaatcaac 139620
   tcattatata tggaaattaa aggagaaaaa tagtggtatg attaattaaa ataaaaagaa 139680
   aaccttctaa attttatctt agctcatagt tgtaaaagct gccatcccta accaaggcca 139740
   cccttgaccc tttctcatgt tccatctttc tgtttgtttc atagtttatg tctcaccaaa 139800
   atctatcaga taaacgtatt catatgaaga tttaaatata ttacatgtta agccttagcg 139860
   aatacttcaa tatctaaaga aggtacaaac aaaacaaaaa tcaacactta gttataagag 139920
   attacatact ctccagggaa gacctgaaga ctagcccctt tctggatccc actagcccct 139980
   catcccactc caagccctcc cctccaatcc catatgcact gggcattcat acaaataaga 140040
   ccatcagctc tggatatctg tactgattga tgctcctgct aactacctga atgattgcga 140100
   tgtaaggaca gcactgcctg aatcctattt atctctcgct atgccatagc ggccttccat 140160
   gctgatggcg tgtttgagga tccagagggg tctttggttg gcaggattgt tttatttccc 140220
   caagaggaga gccttgatgc aaaaataggt gaagaaatca gtacaacaaa acagaaagcc 140280
   tagaaactac tatgaacaca atagagcaga agtagcctta agagttggtg gagaaaggat 140340
   ggtctattca attacctggg ctgagaaact ggctttcata tggaataaaa ataaaattat 140400
   agctataccc catatcatac acaaaagttt ctacatctaa caaagacaca gatagaaaat 140460
   gttttaaaat tttagaagaa aatagtgcag aattttagtg cagaatttct tagactagat 140520
   gcaaaaacaa aaatgattaa agtggccagg cacggtggct tatgcctgta atctcagcac 140580
   tctgggaggc cgaggtaggt ggattagtgg aggtcatgat ttcgagacca gcctggacaa 140640
   catagtgaaa ccccatctct actaaaatac aaaaattggt agggtgtggt ggctcacgct 140700
   tttaatccca gctacttggg agtctgaggc aggagaatca cttgaacctg ggaggcagag 140760
   gttgcagtga ggggagatgg cgccactgca ctccagcctg agcaacacag cgagactctg 140820
   tctcaaaaaa atctaaaaat aaaaagatta tttttaaaag actattttaa acaaaaaaaa 140880
   tcgtttaaat gatatgacac actacatcta atatttggaa aagtacttct taatactttt 140940
   aataaaaaga ggcgctgaga gcatacaacc tatcctcaga agagtgtttg acctctagga 141000
   gggacgcaag cgcgttcttc cttcatttta actggtcatt ttcatttatt tcaggaacat 141060
   ctgaagtaaa cacagtcaca cgttaacctt taaaaatcta ggaggtgcgt acgcatagtt 141120
   ccattacttc aatttttgta cttttgcatt ttaaaatatc acagggaagc tcggtacagc 141180
   ttcaaggcta ggaggggtgg ctctctctta agccctgtcc ccgccagccc cagacctctc 141240
   gtcccgcccc cattgcccag tccccaccct cacttcccca tttccccact cccgcggtct 141300
   cttaacgcac ctcgtttttc gtccagtgga ctcagacctg tagtcttcca ccaggatcgg 141360
   ctcctttccc ggagctctcg ctcttagagg aaattgagag aagcatcagc ggagacccat 141420
   ctgtggctct ccagagggcg cggcattcag accccagatc cagctgtgag aacggacccc 141480
   aggctcacac caggcctgcg ggaggcggcc caccagaggc gctagaaaac aagcctcgcg 141540
   gggaggcgcg cagggcgact gcaagctgta gggggcgctg gcgccctcac aggccagggg 141600
   cagggccggc gctgcgggcg gggctcctgc ggcgtgaggg gcggccccag gccagcagct 141660
   gcgccctggc tgggagccgg ggagcatttg ctgctctgct ggaccctgag tctggcggcg 141720
   ggcggcctcc tctccgctcc ccgcccgcca tcccccaact cccgatctct ctgctgcgtc 141780
   tggcctcagg ctgagacccc aacgaatcat tccccgcatg ggaacatttt atgatataac 141840
   tgaattcagt tttatgtata actgaattac ggatatgaga atctcaaatg aggacgaatg 141900
   gtttttacgc acaaaacatg agacacaaat ctgtaagaaa tataaagtcg tgaccacgtc 141960
   ctttcagaac tttaacctgt ttgctgaagt acgtcagtaa caatggcagg gaaagggtat 142020
   cttaaatttc accacagcct caaagaggcc atttcgtgga tccgctgagg cttggagtcg 142080
   gccttctgac cacgagtcct gcggctatga aagaggaagc cgcggttcag ggcgtcctcg 142140
   cgagtcgtgc agcccgccct gctccagctg gggacaccgg tggtcacggc gctttccagc 142200
   tgcagatcca ggcggcagcc caagatttgg tccagccgcc aaggggtggc tcgagtgact 142260
   gacgggcctt gaacgctccc aggacccaca tctggagagg gaggtggggg tggggtgctg 142320
   aagtcattct tggggcccct gggggcgggc atggacctgg gtaaggccag agaaattgac 142380
   acctcgtgac atccctggaa gagaagtacg ttcagtgtca ctccagagct gaaaccgcct 142440
   tctggctggt ccctcctcac ctacatactt ttctaatttg tctggagcag gccgggcatc 142500
   tgtattatct ggttatttaa atatctggtt atttaaaagc tctccattaa attcacatac 142560
   acgaaaataa aaattaaaaa aaattttaaa aaaaagaaac aaaagctctc taatgaccaa 142620
   gtcctacacg atagtgaata aatttttttg tgtggtccct aaaattgagt tcatgccttt 142680
   tctgaagtaa tagacgccca gagaagggat cgacttaccc atcatgccac agagattaat 142740
   tggccccaga attctttagc agaccgtgta tatgaacgtc ctttgcaatc atataaatta 142800
   actgggaaaa cctcatttag tatgttacat gcctagcgtt ttgtgcctga acaccttaca 142860
   agaaccaggg actattgccc caatattata tttcaggaaa ggaaggccca gacaaatggt 142920
   gtcactggtc cactttcacc cagttggtaa atgaaaccag aaattatagc tgtaccacag 142980
   aaaggtgaaa acgtttcttt tataatttca catacaatct ttaatggacc cagtgtccaa 143040
   cacattaaag caagtgctca ggagtgacat caagatgtaa aaaatagtcc tgtcctcagg 143100
   gagtttaggt cttggagaaa agagacccaa ggagacacaa gacaaagggg aaagagaagg 143160
   agcgctgaag actgaggacc ctgcctgtgg actgaagtga ggatggggac acccgatgcc 143220
   cggaatatga cagtttggag gggcctgaag gactcttcta ttctctatca gaaaaacaga 143280
   attactctcc taaccagaaa aggtatttca atttatattt tccatcacag cacttttctg 143340
   gtgataattt aatgtgtttt aaaaaatgta tcacagtgat ggcctggtgt gaaataaata 143400
   ataaaatttt aagaattaaa aaatataaaa atcttttata tagacattag gagttacaag 143460
   gataactgtg aattataatt agtaattaaa ttgaaatact gattattttc atttttattt 143520
   aattatttaa taaaacctat ttaacattta atatttatca gtaattaaat ctaattgtta 143580
   atatttatta ttataaatta ttttagaatt aaaaataagt gtagaagcga ggcatggtgg 143640
   ctcaagcctg taatcccaac actttgggag gctaaggtgg gaggattgct tgagcccagt 143700
   agttcaagac cagcctgggc aacatggaga aaccctgtct caatacaaaa aaatgagcca 143760
   tgtgtggtgg tgcgtgcctg tagtcccagc cattctggag gctgaggtgg gaggatgact 143820
   tgagcctagg cagtcaaggc tgcagtgagc cctgatcttg ccactgcact ccagtctggg 143880
   caacagagca agaccctgtg tcaatataca tatggacaaa cttaaaattt aaaatgaaag 143940
   catactactg atacagaatt gagtagagat gcaaagctag tcctataacc agaacaataa 144000
   agataaaaag gagagtggaa gaaggtatgt catgaatttc atgataaatg gcaattgcaa 144060
   atatcctgta gcagaacaaa acaacaaaac tgtagataaa acatatccaa ccctttggaa 144120
   ggccaaggag ggaggattgt ttgagcccag aagttggaga ccagcctggg caacatagtg 144180
   agaccctgta tctaaaaagg aagaaagaaa aaaaaaaaaa ggatgataaa gtagacaata 144240
   ttgaaagcca ttttctgcaa atacatagtg aatttgatca gtaattttct tccaacagtg 144300
   caaaaatgaa tagatattag ttgcctgaaa taaaaatcaa atatccaaca aaaaatattg 144360
   actatctaat agtatctaag ctagtaaatt tggccagtta taaaatgtct taaattttta 144420
   tttaaaaaaa gaaaaccata tttataagaa gaggtgataa agagaaatta tttcagttat 144480
   gaagattttg ttagaaaact atgagaaaaa aactattttt tgttttcaaa aagtgaaaga 144540
   ttaagttacc aaacagttgc taaagaatac cagatggctg agcgtggtga cttatgcctg 144600
   taatcccagt actttggaag gccaaggcag gaggatcatt ttaggcctgg agttcgagac 144660
   cagcctgggc actgtagcaa gacccgtctc tattaaaaaa aaaaaaaaaa aaaaaaaaga 144720
   atacaagacc ttgctaacaa tagcaaagat caattaattc aaaatttgaa aaactgtaat 144780
   ttatttagct ttagagtact ctcgtgatat gagattgcca aattaatact ttgggtgcat 144840
   ttcttttctc aaaggacttg caaatttaca aagaagtgtt gaagaaaagc cacacattgg 144900
   caggtaatgt ttgcaaaaga cagatctgat gaagaacaat atttttagaa tatacaaaga 144960
   atacttaaaa ctcaacagta agaaaataac ctgatttaaa gcaggccaat gacctgaaca 145020
   tctgttcacc aaagaagata cacagatgca agtatgcata tgaaaagatg cttgacatca 145080
   tgtcattagg gaactgcaaa ttaaaacaag tagataccac tgcataccta gtagaatgac 145140
   caaaatttag aacactgtca gcaccaaagg ttgcaaagat atgtagcaat agtaacttgt 145200
   tcattactgg tgagaatgca aaatgtgcaa tcactttgga agacagtttg gtggtttctt 145260
   acaaaagtaa ccatactttt accataagat tcaccaatca cactccttag tatttatcca 145320
   aaggaattga aaacttatct ccacacaaaa acctgcacat agatgtttat agcagcttta 145380
   ttcataattt atccaaaact tggaaacaag atgtctttca gtaggtaagt ggataactgt 145440
   ggtacttctg aataatggaa tgttatttag agttaaaaag aaatgcattc actttgggag 145500
   gccgaagtgg gtggattgct tgaggccagg agtttgagac cagcctggtc aacatgggaa 145560
   aaccccaatt agccgggcat agtggcgtga gcctgtaatc ccagctactc gggaggctga 145620
   gatatgagaa tcgtttgaac ctgggagatg gaggttgcag tgagccagtg ccactgcact 145680
   tcagcctggg caacagagca agactcctct gtctcaaaaa aaaaaaaaaa aagaaagaaa 145740
   agaaaaaaga aaaagaaaaa gaaaagaaac gatcaagcca tgaaaacaca tgaaggaaac 145800
   ttaaatgtat gttactaaaa agccaacctg aaaagactgc atactatatg actccaactg 145860
   atgcagggca agcaagccaa aaattagggc ttagcccggg aagaattcaa gggtgaagtg 145920
   gtggtgttag caacttttac tgaagcagca gtgtacaaca gcagaacagg tactgctcct 145980
   tgctgagcag ggctaaccca taagtaatgt gcccagagta gcagctcagg ggcagttctg 146040
   cagtaatata cctgctttta gttaagtgca tgttaagggg gattatgcag aaatttctag 146100
   aaaaagagtg gtaacttcgg agtaggtaca gaggaaagaa gtcgataatg tcctgttgtt 146160
   gccatggcaa cgaaaaactg acatggcgct ggtgggcgtg tcttatggag aggtgcttta 146220
   acctcgtccc tgtttcggct agtcttcaat ctggtccgga gtaaagtccc tgcctccgga 146280
   gttcactcct gcttcctgct tcacaactgt atgacactct agaaaagaca gtaactatgg 146340
   acacagtcaa aagattagtt gatagaaatt gggtgacagg aagtgttgaa aaggcagaac 146400
   acaggatttt tagggcagtg aaacttctgt gatactataa tggtgaatac atgacattat 146460
   acatttgtca aaacccatag aaagcacaac accaagaata aaccctaatg taaattacag 146520
   actttcgttg ataatgacgt gtcaatgtaa gttcaattgt aataaatgta ctactgtggt 146580
   gctggatgtc tatggtgggg ggacattttt gcttcaatag ttacagttga agtaaatgtt 146640
   tgtgtttccc acaatgcata tgtagaaact ctcacattca atgtgatggt ctttggaggt 146700
   gggctctttg ggtgatagtt aggtttagtt gagatcctag cagatcgagt cttcatgatg 146760
   ggcatgatgg gactggtccc ttataagaaa agaccagaaa gctagctctc tctttgccat 146820
   gtgaagacat agcaggaagg tagccatctg caagctagga aagggccttc acaaagaatc 146880
   aactcagacc tcagaacagt gagagataaa ttgtcgttgt ttaagtcact caggctgtgg 146940
   tattttgttt cagcagccca acctaagact gttaattgga ttagaaattt ccttttgggg 147000
   atggtgtgtg gcgggcgggg ggcggggagt acctttgtta agcttttata tcaatgagtt 147060
   tgtaggcttt tcttttttgg tcattgacta ggacagttta aatagtatga gtgtgaagga 147120
   gattgttggt catctattcg atgtcccttc tctgtttttt aatatgagaa ctcctgattt 147180
   tcagccaact accctggaaa aaaagctaat ctttctgact tcttaagtgt ggccatgtac 147240
   taaattctgg ctaatgcaag gcaagccaaa ggttttatga taggttttag gacactagag 147300
   taaaagagag ctgttgcaca catgctcttc accctacttt tgtgtccttt tttccatcct 147360
   acaacttggg ttgtgagtat gatggctgga actttagtgg ctctcttgga tcccaggggt 147420
   aattgagggg tggctggaag gaatctgtga ttttctggag tttccataca caaacaagac 147480
   ctggattttc tgggcttccc agacttccac atctagactt gctttaaatg ggagataaat 147540
   aaacttgttt cagccactgt cattttgggc tattttatag aacttaatct aatcttcaag 147600
   ggtacatgaa ttgcttttcc ttaaaaaaaa aatcagccat aaaatcatct tcttttttct 147660
   tttgttcccc acattattta gttggagctc tgtaactttt tttttttttt tttttgagac 147720
   aaggtcttgc tctgtcactt aggctggaat tcagtggcat gaccatggct cactgcagcc 147780
   ttgccctcct aggctcaagc aatcctcgtc tcagcctcct gagtagctga aactaaggca 147840
   catgccacca tgcccagcta atttcttttc ttttagagat gggagccttg cccaggctag 147900
   tctcaaactc ctagcctcaa gtgatcctcc catctcagcc tcccaaagtg acaggattac 147960
   aggtgtgagc caccatgcct ggctgctctg taagtgtctg aatttcattt tgtatttatc 148020
   agtctgttta gattttcttt cccttcttgg gtcagttagg ccattggttt ctttttaaag 148080
   gttttcaaat ttatttgcat ctaattcttc aaattactct caaaattatt ccagtatata 148140
   ttcttttgtt cctattttct tctgtattct ttattaaaat agctaatgat ttatctagca 148200
   ggacttatat tctttccata actttcctgc accccaatta atctccaatt ttatatttct 148260
   tctggccttc cttatagttt ccacaggttt attttattca ttttttaaaa cttttattta 148320
   attgtttatt ttattatcat tctttcttat tcagcaatct aagtgcttag ggatatagaa 148380
   tttcctctaa gcagcatatg ctaggcttta acaatgttag ggaggcctcc cctttctggg 148440
   gaagaccaca cttacattaa cacaggactg tgggatgcca agaggtagag aagagcttat 148500
   gaatatccag attacatctt cactgatcct gcacaaaggt ggggttcctc ggttacccac 148560
   tgggtcctat tacccaagtc tgggtcagca taccgagact acgggtatat agaacaagtg 148620
   caactggcga taatccttct gttggggaga aaaatctttt ttttctattc atcttaggtt 148680
   ctccatctgt ggccctatca agtagactaa caaaagacag attgacaaga cagaaacaaa 148740
   gcatgtgcat tgtacaaaca caggggagta ctgagatgaa tactcaaaag aggatttaga 148800
   acttgggctt atatagcatt ttaagaaaag aatacatttt ttaagtgaca aggaagacga 148860
   aaaggacttt gagtttctag tgcagtaaat tgtgggaagg caactttttc tttccctttt 148920
   tttttttttt tttttaaaaa aaaagacttc tctggtgcta tgtccaggct gataagagtc 148980
   taaagtctct ggtgactaac ttttgttctt ccccgagtaa gaagacacct tcacaatttc 149040
   atatcctgct tttaggcaaa tagggagagg gcagaggtgt ttgtttgttt ttaatctatt 149100
   ttttttctca attgtcttca actcaaaata cttcttatgc caaagatggc atattctgct 149160
   acccttcact tactacttac aacccagcct ctatcatcat aattagaact tctgaccctg 149220
   gggaacatgg gcaatagttt gaactctttt atatctccct taggcagaga tggaggccca 149280
   gccatgcctc tgacatctag acacaactgt tgcttcattt ctcctattct cagaggtgat 149340
   gttgtaggac ttcaacaaat atcagtaaac attaattttt tttttccttg aggcacagca 149400
   tgatcttggc ttactgcagc tgctgcaggc tcaagcaatt ctcctgcctt ggcctcacga 149460
   gtagctgggt tacaggcccc taccaccatg cccggctaat ttttgtattt ttagtagaga 149520
   cagggtttca ccatgttggc caggctggtg ttgaactcct gacctcaagt gatccacctg 149580
   cctcagcctc acatagttct gggattacag gcgtgagcca ccatgcctgg ccatcaattt 149640
   ttatgtcaac tctaaattat aacatttagc aattttgtga ctttttatgg tcatcattaa 149700
   tgttgtttat gttttagttg tagtcctgtc attactcact cgggtatggt aatttggtct 149760
   ttttcaaaat gaagttaagg tctatttgct cttctctgaa tcataataag aactgccaac 149820
   agccatttca gcaataacta tttactgaga ttttaaaata tttcaaggta attggtccta 149880
   gcagactgga aaataccaaa ttcttttcca gaactgaatc ccccatcaaa gttcaatttt 149940
   actcataatt cccttttcat ttgaagcatc tcattgtaag ccagtcttaa cccttctctc 150000
   acactttgct tggctgtttc tcaggtagaa ctcagtaagt ctggtagcct ccaggactgc 150060
   cgcttagatt attaaacaac atgtcagtgg ttggaagagt caatgttatt ttgatttttc 150120
   tgttttgttt tgttttaaat gcagttggcg gataattgca gctttctttc attccctaca 150180
   tgagttcaaa tggcagcaaa caaactagga gaacgcagac cttctgactt gtgggtaccc 150240
   ctactcatca cctgaagacc cttggaaatc aaagccctga cccattaaag acggatggag 150300
   acagcaacat acgatcatca ctattatctt gctttgcccc agtccaggtt aaccatctgt 150360
   ggtattttta gttgctaagt ccatatattc aacataaatc aattatatat ccactaaaat 150420
   ctcagcacta gtctaactac taaggaaatg acagcgaaga aaacagacca aacgtctgcc 150480
   cttatgggat ttatattatt ttctctgtgc tggttaaacc aaggagcttc tgctcttttc 150540
   cttagtcacc tgggggaggc agaaacaaag gagaatattg ataaacctgg aaatagggcc 150600
   ggagagtatc agagaaggaa gccttcggga aagtaaagat gtggcagcca gtattcccgt 150660
   tataaaagga tacaactccg gcctcatagt ccagaaaaat tcccacaagc aggggctgct 150720
   catgcagatg aagggaagtt gggggagaag taagtgctac atagcctttc tttttgcaca 150780
   gcctgagggt ccagaatcca gactgaggct cttgcttcat gccagtgccc ctctgcacat 150840
   tttccataca aactcctaaa tcccatccgg ttccttcgcc aacatccact tcaaagtaac 150900
   gtcttcctga ggtgaagcct tcacaaccca agacacaggg gaaggcagta aatctcctgg 150960
   aagatgtgtc ctgattctcc tgggtgtatc cacgagtcac ttgtctccga tcctcagaga 151020
   gaattagttc gtgatgagct gtatctggat ccagagtcac actaactgca aaacaaaaca 151080
   aaacaaacaa aaataatttt gttgctgtga agaacacagg ttattttatt ttattttatt 151140
   ttgagatgga gtgttgctgt cacccaggct ggagtgcact ggcactatct caactcactg 151200
   caacctccac ctcctggatt caggcaattc tcctgcctca gcctccggag taactgcgac 151260
   tacaggtgcg caccaccaca agtggctaat ttttttaaat tttctgtaga gatggggttt 151320
   cgccatgttg gccaggctgg tctcaaactc ctgacctgaa gtgttccacc cacctcggcc 151380
   tcccaaagtg ctggattaca caggtgtgag ccaccatgcc cagccacaag ttattttcaa 151440
   taaaaccagc ctgtgttcaa acccaactat tgtttcttat aaactgggtg agcttaggca 151500
   aatcatttaa ctttctgagc ctcagtttgt taactataaa gtggaaatta ccgtatttgt 151560
   tgcagagaat ggtgggtagg attgaataag cttatgtttg cttaatgctt ggtaaaattc 151620
   ctggtacatg gtaaccacct aataagtggt agttgttggg gtgatcaggc ccaacaccag 151680
   gccgtggggg ctacaaagtc cggcggggtc aaaggaatga gaaaagacaa gttaagagtg 151740
   cataaagtgg gtccagggtg ccagcactag attggaggct gcaaaggccc taagctctgg 151800
   gagcccacac tatttattgg tgatcaaaca aagaagcagg tggtgaggac gtgagggtaa 151860
   acaggtgagg gcatgaggac atgggggtag aaaggtagtg gtgcattaag cgtagctgtg 151920
   acagtttagc attttctttg acacatgtag aatatactct gctgcttgag atagtagagg 151980
   acacgtttat gagtgaaaag caaggaacca acaagtctgt gcactttcca gaggctatga 152040
   ggggttttat gccctgagcc ctgggttcca tccaagccac aaggggtttt atgccctagg 152100
   cttagatttg tggtgcggca gggcagcctt ccaccatttg gcacagagct tggtgttcca 152160
   aaggccacga ggggttttgg accctggacc ccggacatct tccaagactc ttttacatta 152220
   tgacagacaa gccagtcctg cttcagctct tctaacaaca tgtagtaata atgatatcat 152280
   caacatcatc ttcgtcttaa ttattcaagg atgccaaggt acagaactaa cctgttaata 152340
   tggttaccat cctgtccaaa gttcttctcc catgcaggac ttccaggaat catgagacag 152400
   ttgagcagaa agataccttt tcccttctct actgaataac caccaacatt gagaatcaga 152460
   gagggaaaat gactcagcta atgtcttagc ttgttattgg aagacccagg tctcatgaca 152520
   catgcctagt cccatgactt ttaattgtaa gctcttctct ttcccctcag ataatgttcc 152580
   ataagcatta gtatgagata ataatacact gaggaccaat atacatgaaa aatatcagac 152640
   tagaatcaaa caagacagaa aaaagatctg ataacctaaa gtgagatact gaacagtatg 152700
   cagttttaaa aataaaaaat ggtaatagga tgttctaaca agagagttaa gaaaccactg 152760
   tgctactgag ttaaatgttg atcagttggt ctgtgacaat taaggaattc aagtattcag 152820
   aaacacttcc tgtgctggat gctctctgtt tgttcttcca aataatccct cacttttccc 152880
   tgtcttgctc tgtgcccagg aaggctgaca tggacagatt aaccaggctt tccgccctct 152940
   ggcttggttc agccaatggg aagcaccaga ggagaccata gggcacaaag aagcagcctt 153000
   gggagtattc agtaccccag tcccacgcta tgatttggag ggtctgcatt cctctgcctc 153060
   tgggcacact ctagtatagt tacagctccc tacacctgcc acttgaggcc cagaggaggt 153120
   gatggctctc taactgttcc tagttctggg tgcttcctgt tccttgtgga tttcccaact 153180
   cctcaccttt gtaaataccc tcctttttca aactctattc agttagcttt tatcagcctg 153240
   actcacagaa gtttggggtt tcaattcata ttacctgaat gacccaggaa aacccatgtt 153300
   gagaaattaa aatgtttacg gggtggtaat accacttaag agaaaaaata tcaattggat 153360
   ttttaaaatt ccacctatct attggtgtga cacatcaaca aaaacatata gaaagattgg 153420
   aagctaaaag atagataata tagtcatata ctgttatagt attatatcaa aagatattaa 153480
   gtcagagcat tattaagaat ggaagaaggg ccaggtgtgg tggctcatgc ctgtaatccc 153540
   agcactttgg gaggccaagg caggcggatc acttgaagcc aggagttcaa gaccagcctg 153600
   cccaacatgg caaaaccctg gctctaccaa aaatacaaca attagctggg cattgtggca 153660
   catgcctgta atcccagcta cttgggaggc tgaagcacaa gaatcacttg aaccggggag 153720
   gcagaggttg cagtgagctg agatttcgcc actacactac agcctgggtg acagagagag 153780
   attctgtctc aaaaaaaaaa aaaaagaaag aatgaaagga gtcacctaaa aaagataaca 153840
   caattttaaa cataaatgta ctacattatt agtgaattca tgtttagaat tgtgttaata 153900
   tacaaagcaa aaattgtaga attataggag aaatggacaa atctacaatc atcatgggat 153960
   gttttaacat tcttctttcc ataattgata gatcaggcag accaaaagaa agaaataagg 154020
   gaagatacgg aaggtctgaa caatctaaga agcgcaatct catagtcaat acataaagct 154080
   cagcaattgt ttaataatag taagcagaga atatgcagtt ttctcaggta tagatggaac 154140
   atgcactaac tgagtaaata ctaggcagaa aacagtctga acaagtttca ataaatctgt 154200
   attacacaga tcattttctc tagcctcaat ataagattat aaaccaataa taaaaagatg 154260
   actaaaaaga ttctaaatat taggaaatgt aaactactaa taagtcatta gaagatgtat 154320
   agaatggaac aataataaaa agttatttat aaaaatatac aatgaagcta aagcagaatt 154380
   ttaaggaaaa tttgtaggct ttaaatgctt atcttagaaa aattaaaaag ctgaacatta 154440
   atgagccaag catctaattt aaattttaaa aagaacatag aaagccaaat ataatttttt 154500
   aaaaagaaaa aatagatatt aaacaatata acagtgaagt taaagaaaac aagaatgcaa 154560
   taaagaggaa aaacaaacaa aaaaaaaggt agcttctttt aaaagaaatt taataaaata 154620
   gacatacctc caatgagatt tatcaaagta agacagaagg cacaaatgga atgaatacag 154680
   aaacttttta aatattacag aactttataa taaatcttat gctactaata aaattgaaag 154740
   tactgataaa attattactt cctagaaaaa atatttctga gtaaaactca ctcaaaaaac 154800
   aaataaagca tgggcagacc taacattaaa gaaatgaaat cactacttta aattttaccg 154860
   acagataata aaacgtgcat ctttatcaag caaaaatgga acttgtcagt tttataggaa 154920
   atttagaagt caaggcatga gtaatgccaa tctcatacca aatcctacaa agaatagaaa 154980
   attatggctc ccgcttatag acatagatat agaactcctg cacaaaataa tataaataac 155040
   aaaccaaatt ttatatttgc aactatacat attatatgtg tatgtattat atatgttaac 155100
   atatacatat ataatatgta tagcatatgt tctacatatt atatatgtat agtgtatgta 155160
   ttttacaata tataaatgaa aacccaatct ttaatatatt catctagatt gtcatatatg 155220
   acatatataa tacattacat caaaaatgtg tacaataatc aggccaggca cagtgactca 155280
   tgcctgtaat cccagcacgt tgggaggctg aggcgggtca atcacttgag tccaagagtt 155340
   tgagaccagc ctggtcaata tggccaaatt ccatctctac aaaaaatatg aaaaattatc 155400
   caggcattgt ggtgcacacc aatagtccca gctactcggg aagctgaggt gagaggatca 155460
   cttgagcctg ggaggtggag attgcagtga gtcgagattg cgccagtgca ctccagcctg 155520
   ggtggcaaag ggagaccctg tctcaaaaaa aaattaaaaa attagccagg tatggtggcc 155580
   tgttcctgta gtcccagcaa ctggggaggc tgaggtgaga agatcacttt agctcaggtg 155640
   gtggagccat gatcgcacca ctgtaccact cggcttgggc aacagagtga gagcctgtct 155700
   cgaaaaaaca aatatataca cacagtaatc aatatatata ttatatgtac caatcaatgc 155760
   ttcactttta tatataatat agattacatc ttattagata tatagtattc cttctccata 155820
   gatagataga tacagatata gacatagtat cctctatcca tattagagag aggatactat 155880
   atatatctat agcatataga gatgctgtct caaaaaaatt taaacatcag ccagatgtgg 155940
   tggcccatgc ctgtagtccc agctactggg gaggctgaaa tgagaggatt gccattgatc 156000
   ctctcattgg ttgagccata atcgcactac tgcaccactc agcctgggag acagagggag 156060
   acctgaggtg gaaggatata gatatagata tataaataaa tatgtataga gagaatataa 156120
   tatatgtgtg tatgtgtata tatatatatt atgaagacac tgggagagaa tactatatat 156180
   atatgtgtgt gtgtatatat atattatgaa gacactggtg ggatggtttc attaccaatt 156240
   ggaccaagag tccaggtatg gagccaacat gcaatgttgt tgttgactga gctggcagag 156300
   cactggtcat agttacggga aaagaaggtc tccaatgaga catacttaac aaaatatatg 156360
   aacttgccat atacgtggag agttctggtg tgtatatagc cttctctcac caacctagca 156420
   attgtcttca tcatcattat aatgctatca gagcaaagat gacagctaaa tttttttgtc 156480
   cctttcttct tctttctctt ccttcccctc ccccacctct ttctcttcct cctcctcctt 156540
   catctctctt cttttttttt ttgagatgga gtcttactct gtcgctcaag ctggagtgca 156600
   gtggcacaat ctcagctcac tgcaacctct gccttctggg ttcaagcaat tctgcctaag 156660
   cctccagagt agctaggact gcaagtgcac accaccacac ctggctaatt tttgtatttt 156720
   tagtagagat agggtttcac aatgctggcc aggctggtct caaactcctg ccctcaagtg 156780
   atcctcctgc ctcggcctcc caatgtgctg ggattacagg cgtaagccac tgtacccggc 156840
   ctcctccttt aatagacagg gtctagctct gttgcccagg ctgggtacag tggcgtgatc 156900
   atagcttact gcagcctcga actcctgggc tcaggagatc ctcctgccct agtctcccca 156960
   gtagctggaa ctacaggcat agcacacggg gctaataaaa ttaattaggt gataaaattc 157020
   actgcccact gatgactaag ctctttggac ataaaagaca cagaccttga aggaaaatgt 157080
   gtctacttaa ttttgaaacc ctatttatca aaaaacagga tgaaaatgca aaatgccatc 157140
   cacatgccag aagatatcag ctataataag ttcccataaa tcaataagga aaagaaccca 157200
   ataaaaatta ttaaaccaca gtaaatcatg ggtaaatcac agaggcctga agggctaatg 157260
   gacatacaaa aagaatctca atctcactag tgaaatcaga aaagcacaaa ttaagtacac 157320
   aattaggtac cattttaaat ctgtaagact gtcaaaatca taaattatat aagtaaagac 157380
   tcagggagtt ttggaggagt gagagctctt atattgcttg tggggtagaa ttggaacaat 157440
   ttcaagatct gtagtatctg gtaaaattat gatatgcatc cctcacacca gcatgtcact 157500
   ccaaggtatc tccctggagg gaacatttac gggacacaag gaagcatgga taagaatgtt 157560
   cacagtagta ttgtctgcaa cagcaacaac aacaaaaaaa cccaactaca cacaacttca 157620
   atgcccagtc cacaaggcaa tggattaaat aaacttcagg ccggagatgg tggttcatgc 157680
   ctgtaatccc aacactttag aaggccgagg cgagaggact gcttgagccc aggagttcaa 157740
   gaccagcctg aacaaaataa agagatagtg tttctacaaa aaatttttaa aaaattagcc 157800
   agacgtggca gtgcttgcct gtggtcccag ctactgggga agctgacgtg ggaggattgc 157860
   ttaagcccag gaatttaagg ctgcagggag ccatgatggg gccattgcac tccagcctgg 157920
   gtgacagagt gagaccctgt ctaaaagaga taagtaaata acaactttgc attttctgcc 157980
   acattgcaaa atggtgagag agtggtttct agactctaga ctctttctat gactaccttc 158040
   tagttatgag atcctacaac actcacctaa cctctctgtg tcatatttcc tcctctataa 158100
   agcaaaaatg ccccatatag agaggactgt gatataaaac aagaaccaag aaaagtaaag 158160
   cttttctaat ctgtcacaga ctaaagagtg ctcagtatat gtgagtcatt attcctggtg 158220
   ctggtaggag tgtatgttac aactttgagt caagtaatat ggtaccatat attaagatta 158280
   acaacaacct cggcaatccc agtttggggt atgttcccaa aagaaatgaa agcaccagga 158340
   tataaggatg catggactag aaagttattg tagcaacatt gtaataacta agttctaaaa 158400
   acagcctgaa gctccatcag tagggatatg gttacatata tttattatat tcttatggaa 158460
   tattagacat aaaaagtaac gagtaacata gaagagacag tgtatatatg ttacgtttgt 158520
   acaaacttag ggaaagatat agatcaccct acctagagaa gtcagattgg agacgggtgg 158580
   gaaaaacctt gaactttctc cttatatcct ttatattgtt tgactgatta aaatgtattt 158640
   gttgcatctg cttgaaggca atgtaaaata aaataaacat acatttaaaa ataaaaataa 158700
   aatttattcc tatcactttt gtaataaagc tgggcacagt gactaacact tgtaatccta 158760
   gcactttggg aggcagagac aggcagatca cctgaggtca ggggtttgag accagcctgg 158820
   ccaacattgt gaaaccccat ctctactaaa aatacaaaaa tcagccaggc atagtggtgc 158880
   gtacctgtaa tcccacgcta cccgggaggc tgaggcgctg gaacccagga ggcagaggct 158940
   gcagtgagct gagattgcgg cactgcaagc cagcctgggt aacagcgaga ctccatctca 159000
   aaaaaaaatt tgaaaaaaga aaaattttaa taaacagtgt ttaagagggg agaaatattt 159060
   agttaaaaga taagcccatt taagaaatag tttcacttga cccggaaggc ggagcttgca 159120
   gtgagccgag atcgcaccac tgcactccag cctgggcgac agagcgagac tctgtctcaa 159180
   aaaaaaaaaa aaagaaagaa agaaagaaag aaatagtttc acttgaacca tattatgatt 159240
   ccttctgtaa aagatgagag taggcaaatt gactcagtga aatcccagca aaacttacac 159300
   aaagtcttgt tcttccttcc tgtcatctgt ataggatgaa atacagagtg cttttgggtt 159360
   ttgttgttgt ttgttgttgt gtatttgagg ggaacacagg tctataattc cttttctgaa 159420
   atccctggaa caaaatgggc tttgccattc aaattagttt agaagttata aaggcaaaaa 159480
   aatgcatata ctctaaagtt caaccccatc atggcctaag gcagagccct gtaatcaaat 159540
   tcatcaatat atctgcagca aaacatttat tcaaattaag tgggataaat aaagactttt 159600
   aaatagtctc atctcagtgc cgttcagggt tggccactgt ggaagacaga ctcaagggtg 159660
   gccttctatg attcctgcct cttggtgttc acaccctcgt aaaattcctt gtctttgagt 159720
   gtgagcaggg cttatgaatt gcttctgacc aataggatat ggcaaagatg atgggatata 159780
   atttctatga ttacgtttca ttatgtaaga ctccatcttg ctggcagatt ttctctaaag 159840
   agtctgtctc ctgagctctc tctgaagaaa taactggcca tgttagaagc ccatgtgcaa 159900
   agagctgagg ggtggcctgt agaagctgtg ggcaacctcc agccaacagc cagaaataac 159960
   cagggccaaa gtcctgcaac catcaggaaa gaaattctgc ctgctacctc agtgagcttg 160020
   gaagtggatt cttccttagc ctagcctcca gataagaaca cagcctgacc aacaccttaa 160080
   ctgcagcctt atcagaccct aagcagcagg cccaactaag ctgtgcccag attcctgaac 160140
   cacaaaaatt gagataacat atcagtgttg tattaaggtt ctaaattatg gtaatttgtt 160200
   tgtactaata gataactaat ataaccacca aatcatttca ggttaggcca gatttttgta 160260
   gccaaatgaa tcatgataaa actttccatt ttcaggggtt tttttgattt tgtacttacg 160320
   gatacaaatt tgtgaaagta tagtcagcac tgatttaaaa aatcaaggga gcaggaaact 160380
   cagtaaatgg ttctaacatt ttggaatctg taaattggtt gtaacatttg tcatctgtgt 160440
   tatctaagtc aagttcctaa aatatgtgaa tgataggtta tcatactcac ctacttttct 160500
   tgcattgctc taagagttgg ctgagctatt gataataaac actatgatca gatctaatac 160560
   catgatgtgc tattatgatc atgtgtcagt cacagggcta agcactttgt acatgttgat 160620
   gcatttaatt ttgatgataa ctcaatgaag taggagctgt taatattttc atttttcaga 160680
   gggggaaacc aagtcacttg gagtaacatg gctaataagt gaaagaataa gaatttgaaa 160740
   ggtttgcaca gataaccaga atgcaatgct catcacattc actgagcagt gaatcatact 160800
   aactagagaa agtatgaaag ctctactgaa attaactaaa caacctctct ggctgtgagc 160860
   ctgccaaggg acaggtggta aacttggtta ctgcataagg ccccttctat ccacagtatt 160920
   caggaattct ttagtgaaca taccttgatg actccttaac attttcttca catcgaagta 160980
   aagcttggaa acattgcaca tagtatgaag ttccaaggag acagcctctg atgtttccag 161040
   cttcacagcc caactcctag aataagcaga ggcgagagat ttcttcagag gtgcattcca 161100
   ttcatttcta tatacgcaca cccctcccct cctgcattca aacaggactt acctgctcaa 161160
   agtgtcattc acattctata aagaaacaaa aagaaaaggt gagcatggga acatcggtat 161220
   ttcatggggc ttgtcatgca gggctattct tctttgcttt acccgaagaa gtaaagagag 161280
   ttaccctagt cttagtctta gatattgatg gatactcaaa caaagtaatt cccaccagtc 161340
   ttaggtattg atggataccc agatggaata attcctacca gcttctggga gattcagcat 161400
   ggcaggatgt ttatcaacat ttgcatctat tctcatcctt gctgaagtct gagggccagg 161460
   agctttgtcc atgctccctc tgtaaggact agcttttggt gatcggattt ccttcacagt 161520
   gagcccagat tagagaacac ttatcataaa ggtccttagt ggtgaatctg tgcacagccc 161580
   tgagactggg ccactgccac taagatggtg gtagcaggta tcacacagtg gtaaagcaat 161640
   catgctatac actcagcctt acagtatagt caccaatcct gttagttaga accagaatta 161700
   atggctccag atgtttatct tcctacagat aaagctgtag attgtaccat aacagctctg 161760
   gagcaagggt tctacaagca aatcagggaa aaggttatca ctcattttgg ctgccccact 161820
   tcatcaccca tcagtcacct agtggagtat ttcaggagag agtcaacaac cagggttctc 161880
   tgcacatggg ccaaggaggc aaacagtggt aaatgttatc ccgtggtttc atttggccaa 161940
   gctgtgttcc ctcagaagtt tatttttcta attgacataa aggtacccta taaattagtg 162000
   aaggccagcc tgatggcact gatgtacatc taaaagaaac attactttat cttcccatgc 162060
   ttccttacca ttctccttta atagcactat aacatacctt ttttccctac tccaagtaca 162120
   cagcctcacc tgcagcaatt tctgggctga gccctgacat ttttcctcca gttccaggat 162180
   gtggctcttg agttcattgc tcttcagccc cagaccagcc tcatagtccc tcagtctact 162240
   cagagtctgt tgttcttctt tctccagcct ccagagataa gacttctctt cctcatgtag 162300
   gaaacactgg agattcttaa agtcagaccg gattttttgt ctctgaatct gtaccttctc 162360
   ctggagtcaa gaaagtatgg tcaaaaggtg gaagtaaacc aaatgtccat ctatggatga 162420
   atggataaac aagaatgaaa gtctgacaca cgctactaca tgacaagcct tgaagacatt 162480
   caagcaaaat aagccagaaa caaaagggca aatattgtaa gactttgctt atacaaggca 162540
   tctggagtag ttaagttcat agagacagaa agtaaaatag tggttacaag gtgttggcaa 162600
   gaccagaaaa tggacagtta ttgtttaatg ggtagtgagt ttcagtttag aagatgaaag 162660
   atgaaactga gttgcagttt ggagatggga atggtgatgg ttgcacaaca atgtaacaat 162720
   gtaaaagcac ttaattctac tgaactatat acttaaaagt ggttaaatgc ttaagtgtta 162780
   tatatatttt cacacaaaca cacacacaca cacaatcagc cactgggaca ttattttctc 162840
   atgagtcact gaagctggaa gaatgtcccc agtttcctgc tgcagagtca tgtgtgggag 162900
   gcaggcactc agatgtggaa gaggttgcct cagattcctt atagtcaccc aattaatttt 162960
   cttgttcttc agccaagaca caggagaaag ctgggttagg agtgctagat aatttaattg 163020
   tgaaactagg gccaagttca aacactttat cagttacaag gataaaaaga ggtttttact 163080
   tatgatttaa gaagttagat ttctgagttg gagcgatttt cttgaagtaa aagcttataa 163140
   tgaacatcac ccagactgga ttttaagaca accaggctgg taagagggtc cataattctt 163200
   ggcaggggga gctttgagtg tgacaggcat ttattatggt taactgagaa atactgttct 163260
   actaccctag ggtcatctta agcattccta tgtgtaagac tgacagaaat caagtgaaac 163320
   tctcatctga ggagatgtaa agttgcaatt tccattagtg ctgtctaaat taatgcagtg 163380
   ggagtgtgta ttcagggcaa tttgaatcta tgttcttgga ttgcagtctt caaacttggc 163440
   ccaaataaac tctctactta tcttaaaaaa ataaaaatta aaaaataaaa ataaattcat 163500
   acagtgtttt gatgactatg atatagaaga agggtctttg acttaggatg aggtggaatt 163560
   tttgtgtagg agacaggtgc agctttaact cttgtataga cgggttttca tatatgttag 163620
   ttacaatcaa ggtcttcccc attgcccaag atcctagaaa tgggggaagt aagagtgtac 163680
   tcaggagctc aagagcaaca tccacaaaca aagatcaggg tagaggttag agaggactcc 163740
   tgaaagagag aaaattggta atcagcttgt gggattttac tgcaagctag tgaattatat 163800
   aaatataaag attggtgcaa aagtaattgt ggtttttgcc tttactttaa tggcaaagac 163860
   cgcaattact tttgcacaaa cctaaatatt tccataaaag aatgtggctc tgataatgtg 163920
   gaggttagtc agccacggaa ataatctgaa agtttgtagt tgcaagtgtg taggttgttg 163980
   cattacttgt gatgtactta taaatcaagt ataggccggg tgcagtggct cacgcctgta 164040
   atcccagcac tttgggaggc tgaggtgggt gaatcacgag gtcaggagat caagaccatc 164100
   ctggccaaca tggtgaaacc ccgtctctac taaaatacaa aaaattagcc aggcatggta 164160
   gcacatgcct gtaatcccag ctactcaaga ggctgaggca ggggaattgc ttgaacccgg 164220
   gaggtggaca ttgcagtgag ctgagatcgc accactacac tccagcaaga ctccatctca 164280
   aaaaatagta ataatttaaa aataaataaa taaataaagt atatttcttt catcagcttc 164340
   atgagctaga gtagtatgaa tttcaatctg gagtgatcct gttttctaag tgttcacaaa 164400
   gcttggtttc tgtacctgta aagttgagag ccagatgctc cactgtggta aaagtgccag 164460
   ggtaatgagt tgaggcctgc aaaccaggtt tattttgacg tatttaaagt ttgagaccca 164520
   ctcgatgctt tttctaggta aatagtcata ctaattctgc ttcttctgac tgaagtatca 164580
   ggaatcccag ccaactacag tttaaagatg gaaagattgg tgctaaatac tcatggatgt 164640
   aaacctggaa ccaggggcat aagtacaaat aatggtttct tccttgggtt tcattttttc 164700
   aatctggttt agtgagaata aatcctcatt gtgcttttcc tcaatcatcc cctatgccta 164760
   agctctagaa tggaaaatag cttgagatca atgaagtcag attcttactt tccatttagt 164820
   tattcgcatt gctgtggaca gcttctgctc cgtacatctg tcttcaagtt gcttcagttt 164880
   tgtcacagct ttctggagct tttcctgaag gaaaaatttg ataagtgaag cctattcaat 164940
   ttgactcttc attagggacc tagggggaat cccaatcttc taagatatat ttgaataata 165000
   gtgaatattt atagagtcct cattgttttt tgctagagag catgctaaag gctatatgtg 165060
   caggaacata ctgatcccct tggcaaccct gaatagttgg taggatttta aacttcattt 165120
   ctgtgctgta gaaaatgaga ctaagaaagg ggtaaaataa cttgcccaaa gggctatgac 165180
   tgccaggtgg tggagcaaca attgcaatct catctgctga cccagagcct gagctatgtc 165240
   caccactaga gtcctgccag gaaaaagttg gatatagaac aaggtaatca tcatctaaaa 165300
   gattttgtaa aacaacatgc tgaaccaagc aaaaccaata ccagtgtttg gcacacatga 165360
   aattttgtgt cttatgagtc aggaaaaatc aggatgccag ctggttatta gaaacagttc 165420
   atggaagagg ggaattctgg tatcttttga acaatggtat catgaatcca atttaaaatg 165480
   atttagtatt catgtcaagc ttttagctta ttcttcaaaa cagtttctca tatttctatt 165540
   gaaagtgatt tgaagctgac ccaaattgct aattgtagtc aatgctgaaa gaattgtctc 165600
   ctgtcctctg taaacccaac aagtatactc attcattctc gagtgttctc aggaaaaggt 165660
   tctatgtaac tgttttagca aaagatgaca ttgtccttac tatatgccaa gtgctattct 165720
   atgcattcta tattttaatg tcctcaaagc ttataaccac ctcctgtgta tgtgttttag 165780
   ggagggagga cactgctatt atccccattt acagatggag aaaccaaggt gtgaagacat 165840
   taagtaacgt gcccaaaatt gcccatctag taagtgacaa aactcaattt caacataagc 165900
   tggttccttt tcttactact tggtggaaaa gtaattcaaa tgggaatatg atcatcgcag 165960
   ttattagctg ctccatggag tttaaggaag agctgccatg agctgagtgg tggtcatgat 166020
   tgacatgtcc ttagaaggac ttagagcctt catacaagac cacctctgcc tcatggagga 166080
   cagaataagg agcctgacac tggagacaac attttcctca aatttaggca ggacagagaa 166140
   ggaaaaagga catcaggact atgcccattc ctccatgctg ccaacagcaa agtcccacct 166200
   tccttaatat gctttctggc aagaaatctg gatggtacac aaaacctctc cctctgcttc 166260
   accttccaca accaagcatt tccaaatctt tgactcttct tcctgaatcg tgcttaaaat 166320
   ctgccctctc ctccctttct tatacggata gtttgaattt tactccttga tattcctttt 166380
   atcatagaca tgccacagta gctgggcaca gtggttcatg cctctaatcc cagcattttg 166440
   ggaggctgag atgggaggga gaccaggggt ttgaggccag tataagcaag aaaggcagac 166500
   catgtctcta caaaaaataa aaaaattatc caggtatggt ggggcatccc tgtagtccta 166560
   gctacttggg aggctgaggt gggaggattg cttgagcccc agaaggttga ggctgcagtg 166620
   agccgagatt gcaccattgt actccaacct gggatacaga gcaagaccct acctcaggaa 166680
   aaaaaaaaaa aaaaaaaaaa aaaagtagag gtaccagagt gatattttca atgtcactga 166740
   cccttcattc cccaaatgaa aatcccccaa taggtgttca atttttacgt gtccttcagg 166800
   agttacttct aagatgaacc actctctacc ctaaatgtcc ctccccacca ccaaaaccag 166860
   ggacctccag gcagacattt ttgatggttt gttttcttta ctagactgta gatacctaaa 166920
   aggtgatggg tctttcttcc ctgttttcag gccctactgc atggctttac atattgtggt 166980
   ttttcaaatg atattcatgg tgtgaaacaa gaaaaaatgc gggtgtttgg tttgagaaca 167040
   acctgttcta aagcaaaaag aaattcatca taacacaaat ggatagagat aagagtccaa 167100
   ccatcccatt gaaggtcagg atggacagtc tagataattg agcaagaaat catcataaac 167160
   tatttttcag aagaatgaca tgatgaaagc tgtatttcca agtcataatg ttaggtttca 167220
   agttaaatca tctcagctcc tggggagcag gataagactt ggtacttacc aaagctcccg 167280
   ggcccacaca ctcaccttgt agccctggca tacgtcttca acaagagctg tggtgtgccc 167340
   tttgtgctgt ggtgcccgct cacagcgcca gcagatgagc tgcccctcgt cttcgcagaa 167400
   caggtggaac tgctctccgt gttcctcaca tgacatttct tgatccgtct ctttgagggc 167460
   ttcaatgagg cttcccagct gcttgttggg tcggaggcta tccatatgaa atggagcccg 167520
   acactgggga cagcagaatg tctcctgcct cagttgcttt tggcttgggt ttttaaagaa 167580
   gtctgttata cacaagtggc agtagctgtg tccacagttg atgcttactg ggttcgtcat 167640
   caggctcagg cagatggagc aggtggcttc ctccatcatc ttcttggtgc tggtggttga 167700
   ggccatagct tttattgaaa agctccaata ttggctctag agatggagat gaagcagcca 167760
   gaattttcca ccgtgatgaa aatacacctc acctgcacct ctatgtgatg agctggctgc 167820
   aactgacttc cataggtctt gaaggttttc cttccaaccc ctattatctc attttgtatt 167880
   gaagaaaaga ggacctaaaa ggaagaagtt gaggctgagg ttgtttgggc cacgtttgag 167940
   aactgcaacc caagtgcaga gtttcaagtt gccctcatta gcaagcagtt acaagtggtt 168000
   gtttagagga aaaaaagcag ttttaaagca gttttaaagt tgtttgccaa gaatttacat 168060
   taaaatagca taagcttttg actggctata cattgttctt tgtattacaa atctcgggaa 168120
   tatgtaggta atagatgagg cagccagtca ggaacaaaat gcttttaaac atggggtctt 168180
   aactgaagac ctatactcct gcctcacttg tcctgataaa ttttgcatac ctcacatagc 168240
   tcagactgct ctaaattatt tcattatttt tcttttctca gtcttctaac tttttttttt 168300
   ttttttaatg agacggagtc tcactctgtc acccaggctg gagtgcagtg acgctatctc 168360
   ggctcactgc acctccgcct cccgggttca agcgattctc ctgcctcagc ctcccgagta 168420
   gtagctgggt ctacaggtgt gcaccactac gcccagctaa tttttgtatt tttagtagag 168480
   atggggtttc accatgttgg ttggctcgat ctcttgacct tgtgatccac ccgcctcagc 168540
   ctcccaaagt gccaggatta caggcatgag ccaccgtgcc cagcctcttt ttcttttctt 168600
   ataagacaag ttctcgctct cttgcccagg ctgtagtgga gggcagtggc atgaccacag 168660
   ctcactgcag cctcgacctc ctgggtttaa gcaatcctcc tgcctcaccc tggcagagtg 168720
   gctgggacta caggtatgtg ccaccatgtc cagctaaagt cttctctcca gaaagaagaa 168780
   atgcattgga atttagagga tacacaaaca tctagctgta tagctaatac agtagccact 168840
   atcatgagta ggaatttaaa tttaacttaa taaaaattaa aatgaaaaaa ttcagttttt 168900
   ctgttccagt tgccacattt tgattgctta atagttgcat gtgactagtg gctacataac 168960
   agcctcaata tacaacattc tgttatcaca gaaagttacc ttggaccaag tgctgggaga 169020
   agcaatgcag gcttcctcac aaaagctgta aaagagagaa ctcagggagt gtgaaactct 169080
   ttcctattct agttaacttc aagaataatt gttaccaggc cagcacggtg gctcacgcct 169140
   gtaatcctag cactttggga agccgaggcg ggcagatcac ctgaggtcag gagtttgaga 169200
   ccagcctgac caacatggca aaacctcatc tctactaaaa atacaaaaag ttagctagat 169260
   gtggtggtgc acacctgtaa tcccagctgc tcaggaggct gaggaaggag aatgacttga 169320
   gctccggagg gggaggttgc agtgagccca gattacacca ctgcactcca gcctgggtga 169380
   aagagcgaga atctgtctta aaaaaaaaaa aaagaataat tggtaccaga attactcttt 169440
   gtaattagta gtaacactta tgcaattggg tgatctgtga cagattccat tgaaggagta 169500
   tggggagctt caccccaata tatgactccc tggtataatg agtattttga attaaaggcc 169560
   cttagagatc agcagatgct ggaagagact tttcccctat ctacataaag accagtcaca 169620
   ctagacaaga agaacaattg tttttccttc caacccctat tatctcattt tgtactgaag 169680
   aaaagaggac taagaatgta accagaccta atcagacact ttcacaaaat aatgtctgtc 169740
   tctcaggctc attcattttc caaagagaac catttacaag ttaaactctg ttcctccatt 169800
   cattcatcct cccaaatatt catttattct ccctagtaat catttactgc ccctcaaaga 169860
   attacctata ttctcctgat atcacccttc ccctctgaaa taaatatgta tacatgtata 169920
   aacgttatac atacatattt atacagtata catacatatt tatacataca tacatatgca 169980
   tacatattta tatttatgta tttatacata agtatttata aataaggcta tataagtatc 170040
   tacccccatt ggcagagggg gtaatcactc tgtgattcta gcccatgtac ttgttaataa 170100
   atttgtatgc cttttctcca attagcctgc cttttgtgag tcgatttttc agtgaacttc 170160
   agaaggcaaa ggggaagtgt tcccttggct cctacaccat catgacaata aaatttgact 170220
   ccacctcgac cccccccatc ccccacaaag aacaacaacc aacactggtt aataaggtcg 170280
   gttgtttttt gtttgtgttt ttgttgttgt tgtttttgct ttcaggagca gaggtataat 170340
   aggcaaaaga aagagaaagg agaatagtga atacctcttc tgcagagagg ggtgcctaag 170400
   tgggacttcc ctggctaata acgtcttgct agagacccaa ccaggaggat aatggaagca 170460
   atcaaggcaa ccagaacaac cagaagaacc ggtttatcct ttttgtgccc tctccctaaa 170520
   ctgagggaat aagaattgga aagaaggctg cagagcagag ggtttgctcc tgaggagcag 170580
   ttatttctat gggatcagag ctcctgcaga actggggagt ttacttttac tatctcttct 170640
   ccaggacagg acctatctca agagacatgt tcagagtgat tgcaacataa agagtttgca 170700
   gacccaagga ggtagggaag gcagaaagaa gatgggggag gccagggata ggcaacagag 170760
   gagtgaccag gagcgaaaaa gcctgcctct tctgagaacc tagctgggct ctccctgtac 170820
   ccccgatccc tcccccccgc ccgcccccac acccctactc ctgggagctc ctctaggaca 170880
   ggggcagagt caggaggaag tttgaagagt gcctagaata aaaaacagta atttaactac 170940
   aattaccggg taggctgttt tcctctcaca atttgatcag tctcttgaag ccacacagaa 171000
   tttcttctga agacgtgtat tccttggcag gctatttcct ccagtgatac accaggcccc 171060
   tctctgctgg ggtcactgct cttctgggga gatggggctc ccctccttcc aaggctccag 171120
   ggttcctgtc ctgggcccca ctcatctaag ttctgaatct tctgagattt ggtgtaaagt 171180
   ctggtgaaag aaagagcagg aaagaggtga gagctgtaaa acaaagaaag tcctgaccat 171240
   tttcagagtt ggaggggccc tgctgtcacg aaatatattc cccaccccac ttgccatcag 171300
   tacacactca catatccact gagaaaacct tagcctggac cttttccgta accttcactg 171360
   ctcagacact tacatattcg ctgctagtcc cctctgttgc tgccacttcc tgggtcagga 171420
   agttaactca gaccggatta aactgagaag tgaaactact gtgggaggcg gggctcataa 171480
   gatttaggag aaaactagtg acgttgttca tatcatttgc actccgcctc tccggtaaag 171540
   gagggggaaa cgtaggaaga aaatatcctt cttttacagc aataaaaaga aggaaccaat 171600
   taataaccct gtaaactatc atgtgacccc aacacagagt atctaaaaac aggaagcctg 171660
   cagaggttca gttcacagac tctgatttga gatctttcta cttttgccac caactcccct 171720
   gggagtcctt aagccttcct agctgatgtt acttcttttg ctatttatgg gttgcttgtg 171780
   gttctataac tgctctgaag ggtgtggtgg aaaaaggggt ggtaacagca gtaggactca 171840
   ttggcatcac aaaattcatc tgagtcagct ttctattctt ctctgtcccg ttctgtgtct 171900
   tgtttttctc cttgctgtcc ttctgcagga ctcagatctt cttcaatagc gagggtcagc 171960
   caggatagaa aatgggagtc actagtggcc cagcagtgag tgcccccagc ttagagctgt 172020
   gtgggatccc tgggaccatc actctgcttt gtgctttgtg gagaaaaggc tgtggggtcc 172080
   agggtcaagt ccttaatgac ttagctccag cttctccact tcaaaatgaa aggaaaagta 172140
   ctatcaccac ccgttagaat tattatttca tggggaaaaa agatggatta ctatctcaca 172200
   ataagagctt gtcacattta taagtctcag gtgtaagagg catttatgat aacaacataa 172260
   taaatgctgg cttaagtaga tgcagtggtc caagggaacc agtaagggga gctcaggaca 172320
   caggtgggag gagaaattaa acttgaattc tgggagccac tggcctgtct gggcccctgg 172380
   cctgcctgct gaccctgata gccaatggaa catggagttt ggcccagctg caatccctct 172440
   ggtccaacta ctcaaaataa aggcaagatt gggaaacacg ttcctttctt cctataccaa 172500
   gcagaagact cttcagcact gcaccctcct gggtgctcac agagccttct gttgttttgc 172560
   cacctacgat tcatcatgcc ctggcatgat ggttgcagac cccatgcata gcatgggaca 172620
   ttctactcct gaggcaacca gcacacagag agaggagaaa gaatgagccc ctgaatcctt 172680
   ggtcccacga tgagtccttg cagatatcta caactttcat tgttgtggat gtgactctgt 172740
   acccaggcat ggctcattcc agatctgtcc tattgtcaga ggtgttcaaa ccagaatgac 172800
   tccattttga atgggggcta ggtaaaataa ggctgagacc tactgggctg cattcccagg 172860
   aagttaggca ttgtaagtca caggatgaaa taggcagttg gcacaagaca caggtcataa 172920
   agatcttgct gataaaacag gttgcagtaa agaagctgac caaaacccac caaaatcaag 172980
   atggcaacaa gagtggcctc tagtcattct cattgctcat tatacacgaa ttataatgtg 173040
   ttagcaagtt agaaggcatt cccaccagct ccatagtggt ttataaatac catggcgatg 173100
   tcaggaagct accctatata gtctaaaaag gggaggaacg cttggttctg ggaattgccc 173160
   acatctttcc cagaaaacat atgaataatc cactccttgt ttagtacata atcaagaaat 173220
   aactgtaagt atctgtatta gtccattttc acactgctga tccagacata cctgagactg 173280
   agtaatttat accaggaaaa aatgtttcat gctcttacag tcccacgtgt ctggggagac 173340
   ctcacaacca cagcagaagg caaggaggag caagtcaggt cttacatgga tggcagcagg 173400
   caaagagctt gtgcagggaa attcctttct ataaaaccat caggtctcat gaaacttatt 173460
   gactatcatg agaacagcag tataaattac tcagggaaag acctgccccc atgattcaat 173520
   tacctcccac caggtccctc ccacaatatg tgggaattta agatgagagt taggtgggga 173580
   cacagccaaa ccatatcagt atccttagtc cagaagctga tgctctgcct gtagagtagc 173640
   cgttctttta ttcctttact ttcttgcttt cactttactg tgtagacttg ccccaaattc 173700
   tttctcacac gagatctaag aaccttctct tagggtctgg gttgggaccc cctttctggt 173760
   aacactatca aaggatcagg aaaaggaagc tagtgaatgc taaaaaggaa acaaactacc 173820
   attaccaata ataacagcaa gacaaaagca aaacggattg tgacagctgt cccatctcac 173880
   acctgtttcc cattgcagga aggaggggct ggttcatgca cagagtggcc aatattagaa 173940
   gcagagatgg ggtgcagatg agacttcagg aatatgttga caaaggcagg cctagggaga 174000
   aatcaacctg aactatcccc aaggaggaat gcattatctc taatatgtaa agttaggctt 174060
   gatcctgtga ttatgggata taggagtcca aagactcaca atgggaagta ggtcactaga 174120
   gtctccttca gaagctctgt actgtgtgtt cccactgtgg gcaagagtca gcactcagct 174180
   attcctagaa tgcctttcct caactccttc agattttgcc tctcaactaa ccctatcctg 174240
   accacttgtt agcaagtgta cccctctctc cctcccaaac attttcaaat ctattttgtt 174300
   cccatggcac ttatcactga atattttact aatttatttt gtttagtgtt tgcttccctc 174360
   atgagaatgc aaagggatgg atttttttca atattgttca ctgatgaatc ccagtaacta 174420
   gaatatttct aagcatagtg atgtgcatta aatcaaagag taactttctg aattgcacta 174480
   aacacacatc acaagaggtg tgtgcacata tgtgcatgat gcacgtagtg tggtgtgggt 174540
   gttgtgtggg gtatgtggta ctgtgtgtgc tgtgtgtggt atgtgataca tagtttgtgt 174600
   tagtgtgatg catgtgatgt ggtatgtgtg tgcgtgtcca tacatattag gggtggcggg 174660
   gatgttaata tgtcaaatgg tactagaaag tatcagaact catggtgctt actggtttcc 174720
   cagagagctg cttctctccc acctgtagga tatactgatg gtttggacag agaagaaata 174780
   aaaagaaggc tgtgacctac tgggctgagg aaataaaaac gaaagtaaaa gaagagctgg 174840
   gaaaagagag tggaggggcc aagggaaatt tcccctttgg cttctgggga aactttgctg 174900
   aaaaatcaac tcacaaattt attaacatgt acacagggag aaccatagaa tgattatcca 174960
   cttcccaaga gggcttaaaa gcttatatat tatcctggca aaacagatta tgggagggga 175020
   agaagagaaa ctctgttgat gggattactg ttgcggattt ttgctccttc gctcagctag 175080
   gtccgggttt ttgtctcaca gccaggaaga attaggcatg cagccatcaa agaatgagtg 175140
   gagtagaatt tattaagtga aaggaaagct ctcagcaaag acaagggtcc tgaaagcaga 175200
   tttctggttt gctcttcaca gttgaatact agggcttaag actcaaattc ctgacaactc 175260
   caccctgtcc taccagtgca tgcaggcctt tagactgagc tactccatat tgattaattt 175320
   cctgaactgt gcatgtgtta aggaaaggaa tcatccactg caggcatgtt taggcaagcc 175380
   ccctgtgcaa gttcccttat ctgcacaaaa catccggtgt aagcacttgt ggggcaggtc 175440
   agaggttctc tgggtaccat tcccttactg tctgcctaaa gcaagctggc caactccttt 175500
   cattactagg gagagtaagt agatcaggga acagagatta acttgaacat tatcttgtga 175560
   aagtccgttc gggcatggtt acattcttgg tcttacagga agggtaaata aaaataattg 175620
   ctctttttgg tgggtctgga tcttaggtag ataaagaaac tttaattcca cgatgtgttt 175680
   tggtagggat agttggtggc agggatgtca gagagacttt gaggcttctt cagttcaata 175740
   tgaccaaggg ccatatatta gggtatcaat ttctgagccc caacaagagc ttaggagaga 175800
   tgtgatagca tcacagtgtg aaagcaattt tttgtttgtt tttagagaca ggctcttgca 175860
   ctgtcaccct ggctgaagta caatggtacg atcacagctc actgtaatct tgaactgggt 175920
   tcaaatgatc ctcccatcta agcatttcaa agtgttggga ttacaggcat gagccacggt 175980
   acccagcctg aaactgcacc cactttctga taaacttttc aaatgactaa aggggagaga 176040
   gtaagcacta ctcagaggta ggaagaaagg acacaggatt ataggattaa aacaacaacc 176100
   accaaaaaaa accagaccgg tgtggtggct cacacctgta atcacagcac ttggggaggc 176160
   tgaggtgggg ggagtcactg gaggccagga gttcgagacg agcctggcca acatagcaag 176220
   atgctgtctc tattaaaaaa aaaaaatacc tgccttgagc taatcagaat catggaccct 176280
   gacaaaggat gtcccaaagt aagtcttagc attttttttt tttttttgag acagtctcgc 176340
   tgtgttgccc aggctgaagt tcagtggcgt gatctcggct cactgcaaca gctgcctccc 176400
   aggctcaagc aattctccct gccttcagcc tcccaagtag ctgggattac agatgcccac 176460
   caccacgcct ggctaatttt tgtttttttt aatagagatg gggttttgcc atgttaacca 176520
   ggcaggtctt gaactcctga cctcaagtga tctgcccacc ttggcccctc catagtgctg 176580
   ggattacagg cgtgagtcac tgcacccggc aaagtcttag cattctttac aaacagtttg 176640
   tacccgtatc tctaaaaggg agtagtgaat ttcaccccaa aatgtggctt cctgatataa 176700
   tgagtatttt gaatgaaaaa ctcttagaga tcaacagaca ctaaagagac ttttccctag 176760
   gtacataaaa ataggatggc cccaccagcg agaacaattg ttcttttctc cctctctgtt 176820
   atctcattgt gcattatagg aaagaccaag aatgtaacca cacctgaaca gaccctttta 176880
   taagataatc agtctctaag catcatttaa attccaagga gaactattta caaatttatc 176940
   tgttctttga tccaattagt ctctcctggt agttacatat tgcccctcaa cagaattcct 177000
   cttcttctgt ttcccataac ctattttgca aggatcaagc ccctgttatt tcttcaactt 177060
   caaggtggca tataagcttc taaattccac tgggatattg gtactatgtg catgaggaga 177120
   accacagagt aattaaattg taaagccttt tatcttatga atctgccttt ttttgtgttc 177180
   atttttcagc aaaacttcca agggcaaagg tataaaacaa aaataaaatt ctaaagcccc 177240
   ccaaccatct gaatagactt tctcttcagt caggcttctt aaaatgtaac ctgaaagact 177300
   ggctcaggcc attaagggaa gtgggggttg aacatgcctc attattcctc tctggcatta 177360
   acatcaacac agcttttaag tctgataaga aacattttac aacctattct ctctgaagcc 177420
   tgctagctaa aaacttcatc ccatagtaca actttggtct tcacaacctg ttatcacaac 177480
   ctagtgctcc tttctattaa tcccaaatct ttatacaaac tcaaccaatt gtcatcacct 177540
   ccaccccact cctccgctgc ttccagttgt cccgcctctc tggaccaaac cagtgtacat 177600
   ttcttaaacg tatttgattg atgtcccatg cctccctaaa atgtataaag ccaaggtgca 177660
   tcccaaccac cttgagcgct tgttctcagg acctcctgag ggctgtgtca tgggccatgg 177720
   tcactcaaat ttggctcaga ataaatctct tcaaatgttt tacagagttt ggctcttgtc 177780
   atgacacaga tgactgcttc actgaagcct gctctggaag tgagtggggg ttttgcaagg 177840
   ataattttcc ccggatagcc ccagaagcag ctagtaataa tacacttaaa ggtagctaaa 177900
   atgcattgaa cacttgtttt gtgccagacc tatgtcaaca tttgctttgt gccaggctta 177960
   tgccagtact cctgatttgt taatacattc taaataaaaa ttctggagtt tcaaatataa 178020
   taactgaaaa acagaaaata aataaaaata tataataact gaaataaaaa tttactaagg 178080
   ctggggatgg tggctcactc acacctgtaa tcctgttacc ggaaaggggt ccgtccagat 178140
   ccagacccca agagagggtt cttggatctc acacaagaaa gaattcgggc gagtctgtaa 178200
   agtgaaagca agtttattaa gaaagtagag gaataaaaga acggctactc cataggcaga 178260
   gcagctctga gggctgctgg tcgctcattt ttatggttat ttcttgatta tgtgctaaac 178320
   aaggggtgga taattcatgc ctccattttt tagaccatat aaagtaactt cctgacgttg 178380
   ccatggcatt cgtaaactgt cgtggcgctg gtatgagcat agcagtgagg acgaccagag 178440
   gtcactctca tcgccatctt ggatttggtg gggagcagtg aggatgacca gaggtcactc 178500
   tcatcgccat cttggatttg gtggggttta gccagcttct ttactttttt cttttttttt 178560
   tttgcccagg ctggagtgca gtggcacgat ctcagctcac tgaaacctcc aatttctgag 178620
   ttcaagcgat tctcgtgcct cagcctccca agtagctggg attacaggca tgtgccacca 178680
   cacccagcta attttttata tttttaatag agaccgggtt tcgccatgtt gcctacgctg 178740
   atctccaact cctgcgctca agccatccag ccaccttagc ctcccaaagt gctgggctta 178800
   taggtgtgag ccaccccacc tggcctagcc ggcttcttta ctgcaacctg ttttatcagc 178860
   aaggtcttta tgacctgtat tttgtgccca ctgcctgcct catcctgtgg cttacaatgc 178920
   ctaacttaca gggaatgcag cccagcagga ctcagcctta tttcacccag ctcctattca 178980
   agatggagtc tttcttgttc aaatacctct gacaagccca acactttggg aggatgacac 179040
   aggaggattg ctttagccta ggagctcaag accagcctgg gcaacacagt gagaccccat 179100
   ctctaaaaaa aaaaatacaa aaaaattagc caggcatgat ggtgtgtgcc tgtagtccct 179160
   gctactcagg aggctgaagt gggaagatgg cttcagccca ggaattcaag gctgcattgt 179220
   cagaggcatt tgaaccagaa tgactctatc ttgaataggc gctggataaa ataaggctga 179280
   cacctgctag gctgcatttc cagtatgtta ggcattctta gtcacaggat gagataggaa 179340
   gtcagcacaa ggtacacatc acaaagacct tgctgataaa ataggttgtg gtaaagaagt 179400
   tggccaaaac ccatcaaaac caacatggcc accaaaggga cctctggttg tcttcactgc 179460
   tcattatatg ttaattataa tgtattaaca tgctaaaaga cactcctacc agcatcatga 179520
   cagcttacaa atactgcggc aatatctgga ctttacctta tatggtctaa aaggtggagg 179580
   aaccctcaat tttgggaatt gtccacccct tttttggaat gctcatgaat aatccacccc 179640
   ttgtttagca cataatccag aaataactat aagtatgctt atttgagcag accacgctgc 179700
   tgttctgcct acagagtagc cattctttta tttccttact ttcttaataa acctgctttc 179760
   actttactgt atggacttgc cctaaattct ttcttgtgtg agatccaaga accctctctt 179820
   ggggtctgga tcaagacccc tttctggtaa catctttctg gtgaccacga agggacaata 179880
   ctgaggagac tctgaagcca aaggaaacag actacagcac caactggctg actttgggta 179940
   agtggtggag tccccgggta aaggatagga ttgggttaga ggtgcaactt aggggagata 180000
   gggtctctcc taagacagag agggtttcag tccgctctta ataaagggca agaatgcttg 180060
   accgaacttg ggtttgagac ccaacttagg aaggctacag tccttaagat ttaaggggtt 180120
   agaggcccct ctcagtaaag tctctcttgg ttaaaaacgg atttagcatt aggggatgtt 180180
   aactgctatt ctgtttgtat taatcttccc tgtgctcttt gctgacagct atgggtgaca 180240
   ggattaggca tgtacaggat cacgggacat tgggaacttt tcttctctcc aaaaggggaa 180300
   gcttgacagc tgataggact gttggaaaag atccctttgc tatgacaagc agccgcctga 180360
   acttttgatt cagtgttgct gcaatgggtg ggtctttctc tggcctctgt gaactcctca 180420
   ccttccccat ctcaccacag gcaatgcttt tctccctttc tctcttttct cttttctgtc 180480
   ttttctgtta cttgagacaa ccatcttgcc cagagaccat atgttgaaac tcctggtcag 180540
   aagtttgatt aaagatgaaa gggcctatct gggggcaagt ttgagccttc ccagttagat 180600
   attgggtgct aagtggagtg gccaatgtct atgttttgtc acatgtatat tgctctggct 180660
   gaaatggaaa acgttaattt ggttacttta tgtggccatt gggcagcatc ttacaaaagt 180720
   gagagacatt tatttgcctg tggttccatg aaacagaaaa aagttggttt tcttttgtgt 180780
   cgtagcttgg acccaagggc tttgcagtga gcaaggttgc tagtgctgct cagtgaaaga 180840
   gaacccagaa acctggcatg ccagcaaaag ggtaaagatt tcttaccagt caggcttctg 180900
   gcctctctct cttagtgaaa actgaatgaa tggtaaaaat cactgtttat cacctctgta 180960
   aagttttgat taatgggaac aaggatttgt ggggctagtc ttaagctgta atgaatctgg 181020
   tatactttgt gatatcaatt tgtctttctg tattactctg tcataaagag gaatatggta 181080
   ggatagaaca tgggctcagg actccataag cctgctgttc aagccagccc agtaaactgg 181140
   tccgttgcaa agtttattac aggtccctgg aaaaaaaaaa aaataaaaac tggatgaagt 181200
   ttccttctca tcttgtttta tgtcctttgg agcttcacct tgtaaccacg tggcggtact 181260
   ttctcttggt ctctgccatc cagggaacag gaattttggg gtttatgtaa tagttaactc 181320
   taaaaattat ctcaagccat tgcaagctca aaattggctg ctctggaccc cttctgggaa 181380
   gggcaatgga aactaaccag tgttgtagct cagcagctaa ggatttgtca ttttataatg 181440
   gcggccaagg ttcaatcctg gcttagggaa tgagtacttt ctgattgata tctgtgtgac 181500
   ctttaccatt tgttgattct gttctcttcc cctccacaca ctgtcttgag ttttcctctc 181560
   tctgagaacc tgggagatta tctttggtaa agttcaaaag ccagaaataa tggccgtgtg 181620
   ggatggctaa agttgagtaa taagaaactt aaaaggactc cttttttttt tgctttagag 181680
   tgctatggtt tatggttaaa agcttaatta aaagtggata ttcaatctct aaaagcctgg 181740
   gactccttgg gaaaagcaga ggaggcacca cagaccccat tttgggaaaa cctctgtttt 181800
   cctcatgaaa ccccaggaac tggaagtgga tagatccttc gcaaaatcta aggctctgtt 181860
   tggctttgca ttatgttatc tgatgttttt gacttttggg ggtatcagaa attactttgc 181920
   attatgaggg agatctggtg tgtaataacc aggtaggaaa tatacttctg gggatagcta 181980
   aaggcaaata taggtgaata cttggctatt tgcacttttg gatcacaaga agcattctct 182040
   tgactaccta gaaggtatgg aaatgtctcc atccccaccg agagataaga ttcccagggg 182100
   agatggctga tcccccaaaa gagggctgat tccctctttt gggatccagg atctggtata 182160
   aaaatgggac cctggccagg cacagtggct cacgcctgta atctcaacac tttgggaagc 182220
   ctcagagtta tgaatgtctc accatactga cactttgtga ctgagctcct ctctaccctg 182280
   gacacaagag accctaataa ttagacagga atatcattgc ccctatttag tctgaagaag 182340
   ttatagaaga cggatcttta tcccactgca atccttagga ttaagggttc cctggtaaaa 182400
   gggagtggga aaatatgtca gaggcatttg aatcagagtg actccatctt gaataggggc 182460
   tgggtaaaat aaggctgagg cctgctgggt taggttaggc attctaacca ggagtttagt 182520
   cacaggatga gatagaaggt tgcacaaggt acccgtcaca aagaccttgc tgataaaata 182580
   ggtaacggta aagaagccag ctaaagccca ccaaaaccaa catggccaca aaagtgacct 182640
   cttgtcatcc tcactgctca tatacactaa ttatactgca ttagcatgct acaagacact 182700
   cccaccagtg ccacgacagt ttacaaatac catgacaaca tctggacgtt accttatatg 182760
   gtctaaaacg gggaagaacc cttagttctg ggaattgtcc acctctttcc tgaaaaattc 182820
   ttgaataatc cattagttta gcacataatc cagaaataac tatacgtctg cttatttgag 182880
   cagtccatac tgctgctctg cctatggagt agccattctt ttcttttatt tttatttttt 182940
   agataaagac tcgctctgtc actcaggctg gagtctggag tgcagtgacg tgttttggct 183000
   cactgcaacc ttcacctccc gggttcaagc aattctcctg cctcagcctc ccaactagct 183060
   gggaccacag gtgggtgcca ccatgcctgg ctaatttttg tattattagt agagatgggg 183120
   tttcgccatg ttggccaggc tggtctcgaa ctcctggcct caagcgatcc acttgccttg 183180
   gcctcccaaa gtgctaggat tacaggcatt acccactatg catgacccat tcttttattt 183240
   cttaactttt ttttgttttt ttgagacaga gtctcactct gtcacccagg ctagaggctg 183300
   gagtgcagtg gtgcgatctt ggttcactgc aacctctgcc tcctgggttc aagcgattct 183360
   tctgcctcag tctcctgagg agctgggact acagacatgt gccactacac ccagctaatt 183420
   ttgtattttt agtagagaca gtgtcttgcc atgtttgtca ggcttgtctc gaactcctaa 183480
   cctcaagtgg tctgcctgcc tcagcctccc aaagtgctgt gattacaggc ataaatcact 183540
   gcgctcggcc cttctttact ttcttaataa acttgttttc actttactgt atggactagc 183600
   cccaaattcc ttcttgtgtg agatccaata acccttttgt gtgtgaaaga atgtattgct 183660
   gctgttcagg ctggagcaag ctggagctca tgctgctgct cagactggag catgcgtgat 183720
   ctgtgatccc agtaagagga tcatggtcac tccagcctga acgacagcat gatatctcat 183780
   ctgtaagaaa aaaaaattac tagagggctt taacagcaaa tttgagcagc aaaaagaagt 183840
   aatcagtgaa ctcaaagata ggtcaattga aatgatctac tctgaaaaac agaaagaaga 183900
   cagaatgaag aaaaagaaat agagccttag agacagggga taccatcaag catactaata 183960
   tatgcataat gggactccta gaaggagaaa agtgagagga cagggagaga gaatgtttgg 184020
   agaaataatt tctcaaagct tcccatgttt ggcaaaaaag cattaacttg catacatatt 184080
   ttaggagctc aatgaattcc aagtaggata cactcaaaga gatccatacc tagacacatc 184140
   ataatcagat tatcaaaaga tgaagaagat gaatcttgag agcagaaaga aaggaacaat 184200
   tcatcacata caaatagtac tcaaaagatg tctggagtag gtatactaat atcagacaaa 184260
   ataaacttta agataagcat tgttataata aataaagaaa ggtattttgt aatgataaaa 184320
   gtgtcaattc atcaagaaaa cataacatta taaacataca tgcacctaac aacagagccc 184380
   taatattcat gaaacaaaac tgacagaatt gaagggagaa atagaaaatt cgacaataat 184440
   agttggagac atcaatacct cactagttag acaagatcaa caaaaaaata gaagacttaa 184500
   cacttgaaaa cacctaacct gaccctaaca taaatctata ggtcactaca ccccaaaaca 184560
   gcagaataaa catccttctg aagctcacat gaaacatttt tcaggataga ctgtatatta 184620
   cttcatgaaa taagtctcaa taaatgtaaa aggactataa taatagagta tatattctct 184680
   gaccaaagtg gaatgaagat agaaatcaat aactaggctg ggcgtgatgg ctcacgcctg 184740
   taatcccagc actttgggag gccaaggcgg acagatcacg aggtcaggag tttgagacca 184800
   gcctgaccaa catggtgaaa ccctgtctct actaacaaaa tacaaaaatt agccaggcct 184860
   ggtggcatct gcctgtagtc ccagctactc gggacactga ggcaggagaa tcacttgaac 184920
   ccaggaggca gagattgcag tgagctgaga tcgcgccact gcattccagc ctgggagaca 184980
   gagcgagact ccgtctcaaa attaaaaaaa aaaaagaaac tagaaaaata agaacaaatc 185040
   aaacccaaag caagcaagag gaaaatgaaa aatttcaaag cagccaagaa caaaaggcac 185100
   attatgtaca gaagaacaag tgtatagatc acatatttct catagacaca atataagcaa 185160
   aaagacagtg gagcaaaatt ttttagatta atgaaagacc tacaattctg taccaagcaa 185220
   aaaaactccc cccaaatgag ggtgaaataa gacaatttaa tacagagaaa agaggaagga 185280
   atttatctag tcatatgtga gagttttatg atacattttg tactgtatat gtggatgttt 185340
   tctatttcat ttaaaaaatc aaccgtgcaa ttaaatggta gattgtcttg cttctttttg 185400
   attgacacag tcattaacta aaatattgta gtattttttt atctccctgc ctaaaggcaa 185460
   taaacatcta atcagcagac tagaacaata aaaaatattt tttaaaagtc ctttaggcag 185520
   aatgataaaa gtcccttagg catattgaaa ttcctattta tacaaaggaa taaacagtac 185580
   tagaaattgt aactatgtga gtaaacagat aatatttttt ctccataaaa tgtggttgac 185640
   tattttcaca aaaatagtta acaatgtaat gtgtgattta tagcatttaa aagtaaaaca 185700
   ggccgggcac aaaggttcgt gcctgtaatc ccagcacttt tggaggccga ggcgtgcaga 185760
   tcacttgagg acaggagttc aagaccagcc tggctaacat ggcaaaaccc catctctact 185820
   aaaaatacaa aaattaacca ggcgtggtgg tgcacgcctg taatcccagc tactctggag 185880
   gctgaggcac aagaatcact tgaatccagg aggtggaagt tgcagtgagg caaaattata 185940
   ccactgtgct ccagcctagg caacagagct agactctgtc acacacacac acacacacaa 186000
   aagaaaagtg tatgacaaca acagtgcaaa agaagtggaa atgaaaataa tgttatttta 186060
   tataagtggt atacttttag atgaactacg ataaattaat gatgtatact ataaactcta 186120
   aggcaaccac tgaaataatg aaacgaagaa ttatggctaa caagccacaa aaagaaataa 186180
   aatagaatga gaaaaaatat ttaagttgtt caacagatgg gaaaaaaaag aggaaaaaga 186240
   gaacaaagaa cagatgggac aaatgggaaa gtaatagcaa gatgatagac ttaactctac 186300
   ccatatagat tatcacactt aaggtaaatg atctaaatac tctaatacaa aagcagaggt 186360
   tgtcagattg aattaaaaaa acagacaaca acaaaaaaaa gcaaaaaaag agccacaaca 186420
   tgctgcctac aaaaaattca ctttaatata aagacacaaa tagtctagaa caccatcact 186480
   tttaacctta tttactcaaa cctcctgatc cctatttatt tatttattta tttatttatt 186540
   tatttattta tttatttatt tttgagacag agtctgactc tgttgcccag gctggagtgc 186600
   agtggcacca tctaggctca ctgcagcctc tacctctcgg gttcaagcga ttctcctgcc 186660
   tcaggcctcc caagtagctg ggactatagg cacatgccac catgcccagc taattattat 186720
   atttttagta gagacggggt tttgccatgt tggccaggtt ggtctcaaac gcctgacctc 186780
   agcctcccaa agtgctggga ttacaggcgt gagccacagc acccagctcc tcttcattta 186840
   ttcttgctac gcttcctcca atccattttg tgcatttgat gattttgcca gtaacttctt 186900
   tatttttctg gtaaaattac ttatgggtca ctgaggactg ggatgttctt tcttctagag 186960
   ggggtttgtg tctgcttttg ccaggaagct ggggtaccac cagtcaagta ttactttaaa 187020
   ctcaattcat gaattgagac tttttttttt tttttttttt ttacgcagag tcctactctg 187080
   tcacccaggc tggagtgcag cggtgtgaac atggctcact gcagcctcaa cctactgagc 187140
   tcaagcaatc cttctgcctc accattctgt atagctagga ctacaggtgt gtgccaccat 187200
   gcctgactaa ttttttaaat atttttttta gagatggggc tcactttgtt gcccaggcca 187260
   gtctcgagct cctgggctca agtgatcctc ccaccttggt ctcccaaagt gctggggtta 187320
   caggcatgag cctctgtggc tagccaagac tttttatttt ttagcctaaa tgtgtataaa 187380
   agttggcttg tggttacaac ttatcaggat tgatgatctc tctctctctc tctctctctc 187440
   tctgtctctc cccacctctc tcacatccct tgctctgctg agaagcagag caaacattct 187500
   agcagtttcc agagagtagg atgggattac ttctagttta cttttatcat cctttgggat 187560
   cgcagtatta ctgggagaac acaagtatct cttattagac ataccacctt tgtagaatct 187620
   ggactttcat tttagacttt atttgttttc tactataagc aatttaagtt acagatctct 187680
   ctacacactg tttaagttgc atcccatgaa ttttgatgtg ctttattgtc attattatat 187740
   agtacaatgt attttgtaat tttttgtgat ttgtttggag agattgatta attagaatga 187800
   tgtttaattt ccaaatatgt gtgttttttt ctacatttct tatttttatt gatttcaaat 187860
   ttatttctac tgtagtcaga tttaataatt catttatttt tattattttc atttttttag 187920
   agacagggcc tttctgtgtt gcccaggttt gtcccaaact cctagtccca agcagttctc 187980
   ctgcctcagc cacccaaagt gctgggatta taggcacgag ccacccgtgc acaaccaaca 188040
   attcatttaa aaagtgggca agtgaactga acagacattt ctcaaaagaa ggcatacaat 188100
   tggccaacaa atatatgaaa gaatgctcaa catcactgta ttagtctgtt ttcatgctgc 188160
   taataaagac ttaacctgag actggggaat ttacaagaga aagaggttta atggacttac 188220
   agttccacat ggctggagag atctcacaat catggtggaa ggcaaggagg agcaagtcac 188280
   atcttacatg gatggcagca ggcaaagaga gagcttgtgc agggaaactc ccgtttttaa 188340
   aaccatcaga tctcgtgaga ctcattcact atcataagaa cagcatagga aagacccggc 188400
   ccataattca gtcacctccc actgggttcc tcccaggaca catgggaatt gtgggagtta 188460
   caattcaaga tgagatttgg gtagggacac agccaaacca tataaataac taatcatcag 188520
   ggaaatgcaa atcaaaacca caataaggta tcatctcacc ccagttagaa tggctattgt 188580
   caaaaaaaca aaaaataaca aatgctggtg aggatgtaca gaagagggga ctcttatgtc 188640
   ccactggtgg aaatgtcaat tagcatagcc attatgcaaa atagtatgga agtgaggtag 188700
   gttacatagg gtggtcacag cctcccttga aaggaaacaa gaaacttgtc aaattgatgg 188760
   agagaacaaa tctcttgaca ttacacaaac tgcatctggg gctagtggtt agaatatcct 188820
   cagtcaagga ggtagaagag caggagggaa aatccctaag ttcgtgcaag tgcagaaacc 188880
   cacaagctgt gttctcaggt tgacatatac tcattttaat agtaagaaac acacccttgg 188940
   gtagagaatt aaaatgctaa taatacatgt gatgtatgta ctagcgtgta tggcaatatt 189000
   gcatgcacat tcaagagacc acccaaaaca tatttaacaa caatgcccat tcccaccccc 189060
   tcatggataa tcacgtagga ctcccataac gggagtttct tcagtgtcaa ttggtgctga 189120
   agtagccgac cctgactctg ctatcagcgt gtactttcac cttgcaataa actcctttgc 189180
   ctacttttac tttggactgg ctttcaaatt cttttgtgca gggaattcaa gaatctgaac 189240
   cagcctactg acaacagagg tttctcagaa acctaaaaat agatctacca gatgaggctg 189300
   aaaatctgct actggctatt tatccaaagg gaaggaaatc agtatacaaa gagacaccta 189360
   catccccatg tttattgcgt cactcttcac aagagctgat atatagagtc aaccctaaat 189420
   gttcattaac agacaaatgg atagaaaatg tggcatatat acacaatgaa atactatttg 189480
   gccatgagaa gaatgcaatc ttgtcatttg tggcaacgta gatgaaactg gagaacatta 189540
   tgttaagtaa gataagctag gattggaaag ataaatacta catgttatca ctcatatgtg 189600
   aaagtagaga aaaattttta gctcatggat ttagagaaca gaactgtggg taccggaagc 189660
   tgggaagggt agcaaggagg ggaggatagg gagaggttgg ttaatggtga caaaattaca 189720
   gctagattgt agaaatgagt tccggtgttc tgcaccattg tagggtgcat atggttaact 189780
   ctcatttatt gtatattttc aaaaagctag aaaagaattt tgaatactca caacaaaata 189840
   aatgataaat gtttaaggtg atggatatac taattactct gatttgatta ttacacattg 189900
   tgtacacata taaaaatatc actctttatc ccgtatatat gtacagttat tatatgtcaa 189960
   ctaaaaataa aagaaaaaaa gaatatgatc tatcatgatg tatatatcat gtgtacttga 190020
   gcaaaatgtg catgcagata ttgtgtataa tgttctataa atcaattagc tcaagataat 190080
   agataggatt gttcagatct tctgtgtctt tactgatatt ttgtctagtt attgcatcat 190140
   taccaaaaaa agggtgttaa actctccaaa tgtgattgta gaattgtcta ttttgtcttt 190200
   tcttttccat ttttacttta tgtattttga aactctgtta tgacattttg ctatgtattt 190260
   taaaacttcg ttatgtattt tgaaactctg ttgttagaat catacattta tgattattat 190320
   gttttcttga tgaaatgaca cttttctatt gtcattgttt ttgttttttc tgaaatggag 190380
   tctcactctg ttgcccaggc tggagtacag tggcacaatc ttggttcact gcaacctcca 190440
   cctcctgggt tcaagcgagt ctcctgactc agcctccaag tagctgggat tacaggcatg 190500
   tgccagcatg ccaaactaat tttgtatttt tattagagac agagtttcac cacgttggcc 190560
   aggctggtct cgaacctctg acctcaggtg atccgcccac ctcggcattt ttattttatt 190620
   ttattttttt gagacagagt ctcactctgt cacccagggt agaatgcggt ggtgtgatct 190680
   tggctcactg caacctccgc ctcctgggtt caagcaattc ccatgcctca gcctcccgag 190740
   tagctgggat tacaggcaca taccaccatg actggctaat tttgtatttt ttagtagaga 190800
   tggggttttt ctatgttggc caggctggca actgactcct ttaacaatac aaaatatcac 190860
   tctgtctctg gtaacactct ctgtcttaaa ctctatttta gctgttatta ttatagccat 190920
   tttagtcttt ttatgctttc tgtttgcata gtgtatatat tttaatatgt ttattctcaa 190980
   gttatctgtg tttttatatt taagatgttt ctcttctagc caacgtgttt ggttcttgca 191040
   tttttaagtc gattctaaca atctttgcct ttcaattgaa atatttacac cattaacatc 191100
   taacattaac atttattttt ctttccacag tacactggct agcatctccc atataatatt 191160
   gaacataaag tgtgataact gacatcctta tttcattcct actctgagtg gaaagggcag 191220
   gggtggagaa agcattcaac aatttgccat aattataatg ctttttgtta cactgttttc 191280
   ttctgcatta aaaaatatca ttacattttg catgaattat taggagaaaa tattttccaa 191340
   ttttcctgga aaatgccata accacgtctc tcaattttgt ttccatcttt cttccacatt 191400
   ttacataacc tacataagag acacattatc aagtatattt tacatggctt ctcagtgtct 191460
   tctctgtctg ctaacaggtt taccaagaga tggcactctt gtatttctgg tggctatgtc 191520
   catatcgttt tgcctttaag acagcgtaac tacttctttc accagtatta aagacatgta 191580
   catttgatct ggttcttgtg gatgatttta aatgactcaa gctaataatc ctaattttac 191640
   ctaaacactc cattatttta aaatgtattc ctttatgccc acaataaaca tttattgaca 191700
   ttaggctgga cattaggctt ctctatggca gacattaggc tggaccctag ccatatatct 191760
   attgagggaa aaaaaattat tttctatata agtttccaga aagccaagat gtgttttaaa 191820
   aacaaaacaa aacattacat tctaaatgct gtaacaagat aagaaaaagt gttgaggctg 191880
   agagaagaac aaagcagcaa gcaactcctg gaaggaccac tgctgcagag gtaataactg 191940
   gtgaaccatg ttttggagaa ggaaaaggtc accaagagaa ggagggggtc cagggtgttc 192000
   agaaagattg catgcataaa gatcaagggt aataaaaaaa attccgtatt atgtaaatgt 192060
   gaagttccag gaccatgagc ttggagagca tgaagtacag gaggagggtt ggtttcaaat 192120
   aaatctggga atgaaacagt gaagcctctg gcagaactca catctctttc ctcccctctt 192180
   ccttgcacat tccctttatg gagtaattgc agggatggga aaagttcaaa accaccactg 192240
   agcctaggaa gtgctagggt aaagtggaga atgaacctgc gtgatttgct catcctaaac 192300
   taggttcttc taggagagcc cttccccata aaatctgccc tcctcgaagg ggcccagaca 192360
   gcctaagctc acctcccaaa gaccccttac ttgctgactg aatctgattc cacccagaca 192420
   tggcctaaaa cccttccata actctatagc caaattcaat tttagacagg cctcatacca 192480
   acctttcttc ctctaagtct gccaccctag gcaattctca acattctcta cacactttgg 192540
   ggccatagac gtgctaccaa gtctccagac ctagacctga tggagcagtg ctgtaatgag 192600
   acgaccactg gcctttgaac cagacccttc tctgtggctc ctatgcatct ccaacctgtt 192660
   ttgagcactg ctgccaagac atctttggca ctttgttgtg aagttttaaa actgaactaa 192720
   tctacaaaac acctaacctt taaaaattca ttgtcatttc atatcatgaa agataaagaa 192780
   aggccaggaa actgttccag gttaatagag actaaagaga tagcaaccaa atgcaatttg 192840
   tgatcctgga ttgaggggaa aaagtgttgt cagagacatg attgggacag ctggtaaaat 192900
   ttgaatttga atttaaagat aaagtattga gtaatatagg aagatgatta tctgcaactt 192960
   tcaaatgttt cagtaagtat atatatatat aaagagatat aaagacatat aaataaatgg 193020
   ataggtagag aaaaagcaaa tgtataatat taacaatcta ggtaaaaagt atatgagtgt 193080
   tctttgtact gtttttctga tttttctata tgtttgaaat cattttaaaa taagaaggtt 193140
   tttgggtttt ttttgtttgt tttttgtttt tagagacagc atcttattct gtcaccaggc 193200
   tgtagctcag tggcccaatc attgctcact gcagcctcaa cttcctgggc tccagtaatt 193260
   ccccctacct caggctcatg agtagctggt acttcaggtg tgcaccactg cactcagcta 193320
   atttttattt tttaaatttt tgtagagatg gcatgttgct atgtcaccca ggctagtctc 193380
   aaactcctgc ccccaagtga tcctcccact ttggcctccc aaagtgctag aattataggc 193440
   atgagccact gcacccagcc ccaaataaaa aagtatttta ttttaattaa ctaattaact 193500
   ttgagtcaga gtttcaccct tgtcacccag gctggagtgc aatggcatga tgttggctca 193560
   ctgcaaactc tgcctcctgt gtttaagcga ttctcttgcc tcagactcct gagtagctga 193620
   gattacaggt gcctgccacc atgcccagct aatttttata tttttagtag agacggggtt 193680
   tcagcatgtt ggtcaagctt gtctcaaact cctgacctca ggtgatccac ccacctccgc 193740
   ctccgaaagt gttgatgagc caccacaccc ggtctaaaaa gtattttaaa accacagtcc 193800
   cactctacct tgtcctacac taccaggggc taggatcacc ccatgtcttc taggctatga 193860
   gatagaggaa tccaaggaag aagataagct acttggttcc tctatagggt cttgtgtgtg 193920
   ctctcatgtg ctctctctct ctctctctct ctcacacaca cacacacaca cacacacaca 193980
   cacatgaata ccagagctat cactttccca gtctagtact catctcatcc caagggtttt 194040
   gtgttgtagt ggtttgctca tttctttgtt ttgtttgttt gcttggatta ttctttttct 194100
   ctttttgcag ctgaagggag aatttccagg ccagcccttt ggccattaga gttacagtgc 194160
   ctctattcag gcttcataga gagacctggg attcagtagt ggggggcttt tatccagttc 194220
   aaaataatgc attctcacca agatgtactt tgaaataaaa caatactaaa acacaaaatt 194280
   ttatttatgc tgaacattga atcacttttt tctgtatttt gtgtagaaag ttatacacac 194340
   acaaacacat ttgctcctgc tttgtttatt ggcccagggg tatgtttggt aatacttcat 194400
   caggcatgag tagtacgtct tggaaggtgt ggtctaaagc ctagactcct atctgcttcc 194460
   ttcagcattc tccagtgtat ctgtcatctg tctaccttag gataggggtc tccagaactt 194520
   ccattcacat ttagaagagg gcagcggctt tctatggaaa atatgaactc tcattcatct 194580
   ctattccttc ttctagctat ggtccagctc agctgtttgg aataaagtat ctatatgaag 194640
   tctgcgaatg gttctcagac tggttgaaca ttagaatcac ctgagtacct tctaaaattc 194700
   ttattaccca gggcatatct cagaatgagt accgcagggt agggatagga ttagggatca 194760
   tgatctctgg agtctggttt aggcactagt gctgtttaaa actacgttca tgaggtggag 194820
   gttgcagtga gccgagatgg cgccactgca ctccaacctg ggcgacagag tgagagtctg 194880
   tctcaacaaa acaaaacaaa aaaaaccaac tacccttgtg atttgaatgt ccatccaaaa 194940
   ttgagaacca ttaggtaagg ccaagctgta taattaaaga gcagttttca tttgtctggt 195000
   gtggtggcag ctttttgata agggaagtat tgttgccatc cacatacctg agcctcactc 195060
   ctgagaacac tggtgtgtat gttgctaaaa ttccccaggt gattctgagg ttccttcctg 195120
   gataaaaacc actgaccctg ggaatgtacc cactgccaat ctcctgcgta aaccttggat 195180
   actgggaagc ctacagttga aaatattggg cttgagatcc tgaaacaaat cttgtatttc 195240
   attaagacta atatttggta cagtgcagca aatcaaggga attttggtgg ctgagttctt 195300
   ttagaacttt tgcattgaaa taggttcaag cagcaataag ttaaaactac aacctcagct 195360
   aaaggattaa aagacacgtg agctgggtag gatgaggtct aaggttgggt gtggcggctc 195420
   atacctgtaa tcccagcact ttgggagact gaggtgggtg gatcacttga ggtcaggagt 195480
   tcaaaaccag cctggccaac atggtgaaaa cccatctcta ctaagaatac aaaaaaatta 195540
   gctgggcgag gtgccaggca cctgtaatcc cagctactgg ggaggctgag ggaggacaat 195600
   cacttgaact caggaggcag aggttgtagt gagctgagat cgcaccactg cactccagcc 195660
   tgggtgacag agcaagactc catttaaaaa aaaaataata ataataacaa taataataat 195720
   tcagacatat ccaggcatca aacagatacc tggggcagat gaatagtctt gagattcaag 195780
   tcacacatga aatttaggtg gaaaatgaca ttggagaaat ttgagattat gatgaatgga 195840
   aatttttcaa agaggaattt caggctctgt tcttgagggg atagatggac ttccaacagc 195900
   aataacacag gattaatgag gacttgggat gttacataaa ttagagatgt tagatggata 195960
   aagagataaa agtactctct ctaagaacat gggaccagag ataggctcac ttctaaccat 196020
   cagatataac tagcagacta aacggtctaa aaataaaaat catgccccac tcctgcttaa 196080
   gacattttaa ttactctcag taactcttca gtttttctac tgtgttatct ttaactacag 196140
   ggttggtctg ggtgtgcaac acaagaaagc ctggcatata catggattca agtgtatgcc 196200
   atgtgcaggt attctttcat gtactatttc atgtattctt tttcacatct gttttttcct 196260
   tcattgaagt caatggctga tattagattc tactattcat gtgtactagt tatatataat 196320
   tgttacaaaa caaattagca aaaacttagt ggcttaaagc aacacacatt tattattacc 196380
   taaggtctgt ggatagaagt tctgacatgg cttaactggg ttccctgctt caagcctcat 196440
   gtggctgcaa tccaggtgtt ggctgagtct gaattctcat cagaggcttg attgtggaaa 196500
   tttccacttc caagctccct caggtttgtt gaaaaattca gttctttgca ccggtagaag 196560
   cttcttggta gaggctgatt caacttctag aggctgtctg cagttcctgt cacccagggt 196620
   ggagtgcagt ggagcaatca tagctcactg cagccttgac ctcccagaat caatctgttc 196680
   tcccacctca gcatcctgag tagctgggac cacaagtgtg tgccatcaca cctgcctaaa 196740
   aaacaaacaa acgaaaaaaa acccccagag aactttgtag agacaagctg gtctggaact 196800
   cctgcgctca agcaattctc ctgccttagc ctaaaagttc tgggattata ggtataagcc 196860
   accatacctg gcatatggca agtcttgagc aggacaaata cagatgattt atgtctgtct 196920
   tccatggtat tctaggttat tgttgagatg gtcctctatt gtcttgttcc atctattgat 196980
   tagataaaac gttgttcctt ctgttatttt tcaacagtag cttttatgtg tctctcttta 197040
   tcttaaaatt ctaaccaaag agctgctctt ttcttggtgt actttacctt tggttgatcc 197100
   ttcttaacct cttcttgccc tctggggcct aagatgaggg ctgttatcag atgtgagtct 197160
   atgggaaagc aagcaagagg ttcttcagcc tccgttcagc cttaaatgtc taggtagaaa 197220
   tcagtcatgg cccttccaat gtggtacaga ccagatcaca gagacagggg tctcagccaa 197280
   ggtcttgtgg cctaagcctt atagaaataa tgagtgttta cttacttgga gaactccctt 197340
   ggaatatctt tttttgtgaa cctgaggcaa cttttggtga tttcttgatg tcttgggaat 197400
   cttggtctag agccatttca acccgatttc ttttcatgtc agtggcattt tgtgaccaga 197460
   tagtaaataa gttctatgat gttcactcag agaaatacaa tgacttatga tgcgaagctt 197520
   ctgtggttca gcccttactt catcttcatt ccctcttatc tgcatctgtc tcctgcttgg 197580
   gaacaaaagt ctggcttcat tctatgaccc ccacgttgag tttcttagta gcacttactt 197640
   ttcaattagg agtgtcctca cttctatccg tcagacataa ctagccgact aaacagtcta 197700
   aatataaaaa tcatgtccta ctcctgctga aaacatttta attactcccc atcatttaat 197760
   tttttctact gggttatctt taacttcaga gttggtcttg tgtgcaacac aagaaaacct 197820
   ggcatataca tggattcaag tgtatgccac gtgcatgtat tccttcatgt actatttcat 197880
   gtattctttt tcacatctgt tttttcctct aaaatttatt tccttttaaa aatgaaaatt 197940
   ttgcatttga ctaaatttgt caaatttagt caaatttgtt taaaaccatt tttaaaatgt 198000
   ttcccgaagt tttgagtgaa gttagtactt cagaaaaact gttttgtatt tttcctgtga 198060
   cctcagtgca ctgctgtgca tttccatttc tgcgtccaca cacatttgtt ttgaggaaat 198120
   ataggaacga caagataaag ttcaagctcc tggacattgc ataaaagacc gtcatgacct 198180
   ggtcctgttg acttccctag atttcccgct atttcctaag ttgagatttt tggtttggat 198240
   gctttgtgtt ttcctaaaat caaaataggt ttttgccttt tatgattata cagtaaataa 198300
   atgctatttg tgtgaaactt taaacaatac aaaaaaaacc taaggaagaa agtcagattc 198360
   atctaaaaat ccttgtggcc agaattaact accttagtta ctattttctc tatctctctc 198420
   tctcaatgta tatttggtgt aggtataggg gtgtgtgtag tgtgtgtgta tgtatatatc 198480
   tgtttctatt cctgtatgtg gatgtgcaca acgcatcctg ctttgtacac tacagtacta 198540
   gcatttttct aatgtaattc aatattgttg aaaacatttt aaaaaagctt gtatatatac 198600
   acacacatac acatacatgc atgtatgtac atatacacat acagacaaaa atgtatccta 198660
   tgtatattca cacatgtata cacactcaca catacataga gttttacatc catagtttat 198720
   aaatgttgct tttttttggt cacctttttg ctaagtctta cacttttttt tttttttttt 198780
   gagacggagt tttgttgtca ttgcccaggc ttagtgcagt agcgcgatct cacctcactg 198840
   caacctcgac ctcccgggtt caagcggttc tcctgcctta gcctcctgag tagctggtac 198900
   tacaggtgtg cgccaccatg cctggctaat ttttgtagtt tttttataga gacgaggttt 198960
   caccatgttg gccaagctgg tctggaactc ctgacctcaa gtgatctgcc tgcctcagat 199020
   tcccaaagtt ctgggattac agatgtgagc cactgcaccc ggccaagtct tacacatctt 199080
   ttttttacca ctaaactgtt tacccaaacc tgataaccca agtcaacagc tattatggct 199140
   cacacaatct tatgtaaaca aagatacaga tatatagaat tttcttgatt aatattcaga 199200
   aaaaaatgga gtccctttat acgtccttag tatctgcttt actcatttaa aaatgtatta 199260
   cattatatga aagtattcag gtcaaatgtt atagatgtga ttcattcttt ttaactgtgt 199320
   tatttttctg caatgactat gtatcacaaa gtactcagtc ttccactgat gaaaatttgg 199380
   gctatttcca gtttgtcttc catttttctt tcttcctctt ggattttcac tcaatgtgtt 199440
   tactaattta ggaagaatca atagttttta tggtattact tctcccattc aagaatatag 199500
   catatggtat agtatagtag agtacttagt ttaatttagc cagatcctgt tttctgccct 199560
   ttaataaaat tctatcattt tctgcctttg agtcacattt tccttgttca tataattctt 199620
   aaaaaatgta tagttttcat tctaagggaa cataaaaact tctttccatt tctattcctg 199680
   tctagttaat tctactattg ggaaaagtaa ctgttaaaaa aaattcttat ctttccagtc 199740
   agttcaccac atttccttta tacctttgta ctttaatccc cagtcatgtt gaacacttct 199800
   tattcctcac accaagcctc aacgggtttg ctctttctgg aaggtgcttc ccctgtatta 199860
   ctgacttatt cataccacac atggagactg gcgcagccct gttctgcctg ggaagccttc 199920
   ccctgatacc cccagttggc aggagtcttc atttgttctt ttctagtcac ctgtgcaagt 199980
   ttgtattgtt catgtttatc atccttcatt ctagttgtct gtctctgtgt gtggtctcat 200040
   tcagtggact ctgaactctt atgaagtcat gtcatgggtc agatcttaat aaattaatat 200100
   tgtcggaagc taatgtcatg tctagaatac agaaaattta tcaaaaaaaa atatagtatg 200160
   ttggctgggc gcagtggatc aagcccgtaa tcccagcact ttgggaggcc gaggcaggag 200220
   gatcacatga ggtcagaaat tcaagaccag cctggccaaa atggtgaaac ctcatctcta 200280
   ctaaaaatac aaaaagtagc caggcgtggt ggtgcccacc tgtaatccca gctactcagg 200340
   aggctgaagc gggaggatca cttgaacctg ggaggcagag attgcaatga gctgagatca 200400
   tgccactgca ctccagcctg ggcgacagtg agactccatc tcaaaataat aataataata 200460
   ataataataa taataataat aattgtatgg aattgaactg ctctgattgg aaatagctgt 200520
   tttttaaaaa attattattt tttaagttcc tgggtacaag tacaggatgt gcaggtttgt 200580
   tacataggta aacgtgtgcc atggtgattt gctgcaccta tcaacccatc acctaggtat 200640
   taagtacagc atgcattagc tcttttacct aatgttctcc cacaccccca ccccatcctc 200700
   ccccaacagg ccccagtgag tgttgttccc ctccctgtgt ccacatgttc tcattgttca 200760
   gctcccactc ataagtgaga acatgaggtg tttggttttc tgttcctgcc ttagctgtta 200820
   atgtcaggcc agagaggctt aaatttttaa ggatctctgg acttttcttc tacattactc 200880
   ttgatgttta taaatgttac aacttcttta atttcattta atgtatacct tattgagttg 200940
   atttaactga gttaactttg ttatatgaaa atcatgattg ggagtgaggg ggttaaacca 201000
   gctacagaga tcttgattgt tggtggtgaa gcaatgcaag aattcattca ttcagtaaac 201060
   taatgtttat taagcgtgta ctgtcttagt ctgttcagac tgctgtaaca aaatatcata 201120
   aactgggtga cttataaaca acaaaaaatt tatttcttac agttctggag gtgggaagtc 201180
   taagattaag gccctggcaa atttagtgtc tggtgaggac aggtagccat ctttttgctg 201240
   agtcctaaca tggcagaagg gttgaataaa cttccttggg tttcttttat aaggacacta 201300
   atcctagtga tgaggtttct gccctcatgg tataactact gcccaaagac ccctccttct 201360
   aatattatca ctttgtgggt taggatttca acatgagttt tgagaggata cagacatttg 201420
   gatcatagca cacaccatag gacagacact gtgccaagaa ttgtggatat agtgattctc 201480
   aaaatgaaca agatcccctc agagagcttg caaaatccag ctataaaatt atgcttttta 201540
   aacaaattat gcagtttgaa aaatctactc tgaatcttac ttgtggcatt gaatactttc 201600
   ggccactctt tccttattat attaaatatt tactcttgtt tgggggatcc agtctcacct 201660
   actttttcta ccagaactgg tatcagctca tgctctgcct tatgcaaatt aagaaaatat 201720
   catacctttt gggtaaatta agccaagaaa gttctccttt cttctctttc tctctttctt 201780
   tctttctctc tttctctttc tttctttctc tctctctctt tctttctttc tttctttctt 201840
   tctttctttc tttctttctt tttctttctg acagggtctt gctctattgc ctaggctgga 201900
   gtgcagtggt gcaatctcag ctcactgcag ccttgaactc cagggctcaa gcaatcctcc 201960
   tgagtagctg ggactatagg catgtgccac aacatcaagc taatttttgc atttttttgt 202020
   ggagacggga tctccctatg ttgctaaggc tggtcttgga ttcctgggct tatgcgattc 202080
   tcctgcctca gcctcccaaa gtcctgggat tacaggcatg agccactgcc cctggccatt 202140
   ataactattt tcattggctt atcaggcaca tgataactat aataaatcaa taaccagaat 202200
   ttttaaataa agaaaggaag gaattgtttc aactcttcct gctacccctc tatccctcaa 202260
   aagggtaggc tgaatgttgt cctccaaaga tatccatgtc ctaatcccca gaacctgtaa 202320
   atatattacc ttatatgaca aaagggactt tacatgttta ataagttaag aattttgaga 202380
   tgggcagatt ttcctgaatt ttgcagatgg gccctagtgt aatcacaagg gtccttataa 202440
   gagacaggca gaagagtcag aataagagaa aaatacttca agatgttaca ctgctggctt 202500
   taaggtggag gaaaggccaa gagccaaaaa atgcagtggt cactacaagc tgaaaagaaa 202560
   aagaaatgga ttttccccta aagcctctgg agggggcaca accttgccaa taccttgatt 202620
   ttggctcagt gaaacccatt ttggacttct gacctttaga attgtaaata aataaataat 202680
   tttgtgttgt ttcaagccat cacagttgtg gtaatttact acaacagcaa taaaatagaa 202740
   ttaaatacag agatctgagg agttgagtag gataagccta ctccagcagg ttatttcggg 202800
   agtatggtga gactcactag gatggcggaa ctcaattaag gaagtctgaa gctgataagc 202860
   cagagaggga aggctctcat ttcattttat aagggttgcg tcacactagg aagatccaat 202920
   agcaaccaca gtctcaaaat taatgattac aaataggaca caattccaag agtcgggagc 202980
   caagcagaaa atggattagg gaagacatgg atgatatgaa acaggaagga ggggtacaag 203040
   gcagcttcct gggaagttgc cagggcagtc acagttcaca ttcattaggc tgtgggcacc 203100
   aaatgcatat ggaaaatcta gctgacttaa ctgaactcct gaagaggaat gaacacctca 203160
   tttattgagg agctactacc aattagaata tgtatttcat ttgttcaata accccatgag 203220
   tacagtaaca caatccttgc tttactaaag cggaagccaa ttcaaagagg ttcagtgact 203280
   tgtccaagct cagggaaaac actaggaagt gaatatgggt ctgactccat cactgatttc 203340
   aggagccctg ccctttcctc cacaccatgc ccccttgctt tcagaaaaaa aggcttgttg 203400
   actgaatggt tgtatgcaca gttcaaagca gaaacacacg atgacatctt ttgagatact 203460
   ctaacagtga gaacttgaaa atgaagttaa aaattaagcg gcaaaaccaa gccgaggctt 203520
   tctgagaaag tggggccaaa cctgttgccg tctgactgcc acgtggctca ctatttatcc 203580
   ctgtaaaaat ctgcaaaagt atttgaaagg gaagaaggga cagaaaactc cctccttttc 203640
   caagttagcc ttatagtcta gggcttaaaa tactggttta atggtgaagg taagtgcttt 203700
   tcttcttttt gggtagaagg attattacta acttaccaaa ggtccattaa ggggagggaa 203760
   cagttttagg agaagtcaga gaaaagacat taacagcaac ataaggatct ccatctggta 203820
   atattgccta attccaaaat gaagagactc tctgaaaaag ataactgatt caatgaagac 203880
   cctagggcaa ggcttgagaa gccactggta ccaatggaca ctgtggacaa tggtcatttc 203940
   tccaaggacg ctgtgagtat taactgtgat gctgtgatta gtcagactgg gattggctgt 204000
   ggaatgaaat actgatcaga actgacaaga tttgtgtttg ggactgtggc taacgagtct 204060
   tttcagactt ctatatgaat ttgaaatggt ctctcaggaa aaggagaaca tggccgggcc 204120
   tggtggctca cgcctgtaat cccagcactt tggcaggctg aggcgggcag atcacttgag 204180
   gtcaggagtt tgagaccagc ctggccaaca tggtgaaacc ctgtctccac taaaaataca 204240
   aaaattagca gggcgtagcg gcgcgtgcac ctatgcgcat gcatagtgcg cgtgccagct 204300
   attcagaagg ctgaggcagg agaattgctt gaacccagga cgtagaggtt gcagtagttg 204360
   agatcatacc actgcactcc agcctaggtg acagagtaag actctgtctc aaaaaaataa 204420
   taataataaa agaaaaggag aacatgacca aagttatgaa taagactgaa ggcaagaaaa 204480
   ttgtacgctt gtagagatca cctagcttgt tgccctcatt gtacagctaa gaaaaggcac 204540
   ccagggacat tgtggtcagc accaatttct cagaaagata ggcagatgat gagagggccc 204600
   tcagtttttc taacactgaa ggaattgctt ctatgttttc tggtgaactc ctccccactc 204660
   atcttgagga ttccaggcca gaagaatcca ctttaaaaaa gaaacattta aaaccaattt 204720
   aacaaccaat caaaggcact tttatagaaa tacatttcat ttgctgtagg cctgtattta 204780
   tggatctgag agggctagac tgccaatatt gtgactgttt attattattg ctgttgctag 204840
   tatctagaat attatacaac atataacact ttgcaattta cgaggcatgt ctcatacttt 204900
   tgttttcact ccaaactgcc cagtgaagta acattatccc aattcttcct atgaaacagt 204960
   gaaagcccta agagtttttg aaactttacc tggtttactc aatttgggaa tggcagagca 205020
   gaattcagtc cttgaatatc ctcccactgc aggttcatgc tctttgatct aggtgtaaca 205080
   tttactctga gtaaactagg actctgggct aacagagatg aagcaagaca ggctggatat 205140
   taggagaatc taagagcaat ctaacgacca ttataataaa atcatgagtt ctagacttaa 205200
   aaaaagggaa aaacctgttt ttttgcttat gcgtatacca taatatttac attatttatt 205260
   tttttctcaa attcaaccta tactgtgtca agtaattttt tttaatataa cattttcctt 205320
   taacttaatt tcaattcatt tttctgtgtc tacttacaac tttggcacta gaattcacaa 205380
   ttttttttta gaggtatatc tccttaaagg gaagggttct gacactgtta catgttctca 205440
   attgtttgca aataggttaa taattattcc agtgtctcta agtacatatc aaccatgcca 205500
   gtgttcagcc tccataattt tattagcttc tgtgcttatt ttggaaaaac atttcccatt 205560
   accatgaaag acctcagttt aggatggttt ggtatgttag cctgatttct gcattcgtct 205620
   catgcaaagg aaaataggaa acgaagaact gaaattacct attgatacaa aatcaaagta 205680
   gcatttgaaa ccataaaact taagtagggc ttttcatcct ttctcgttag acagcaacag 205740
   agaatgggaa gaaaaactaa agtgatgggt ttgtgataca attccagtaa cataaagagc 205800
   aaggagaagt agttttgttg tgtttatgtt taatattcaa agctcaacct aaaagtattt 205860
   ttcattatca aacttccttc tagaataaat gattaaaact tgatttaaaa tatacaaatt 205920
   ctcctttata atacctcaaa atggagctac cccattgagt tttaagcttg tgattaaaat 205980
   attacgaaaa caaaggggaa gttgtaatag gtagaacaag cagtagtcta ggcattaggg 206040
   gatctggtgc tggctctgtg catcatgtgg tttcaggcaa cttttcaaat tttctacgca 206100
   aattttctta tcaataaaat aaacagttgg gccagaggat ctctgagtct ctttcagctt 206160
   tcagtgttta taagattgga gaagttggtg ggaaagcttt aagtggagtg taagtaattg 206220
   cagctgcatg tacagttaaa gagttgcctt cagccaagcc acgggatctt gcataaaaag 206280
   tgaaatcaaa tagaaaatgg tccaaactct gggtttgacc acagatgact tcagctagga 206340
   tctgagtgta gagcaatgag ctgaactcct gatatccaga tgttagcaag acttggaggc 206400
   cttctaaggc agagcaacaa ccagtatctg tcctggtgct gacctgatct tactagcaat 206460
   tgggcctcca tttgggtcca ttgtacaaaa caacaacaac aacaacaata aaatctccaa 206520
   acacccaaaa ttcaaaattt agatggagag atactattcc cagaattcta gagatatttg 206580
   gaaagcagaa aactatactt gccatgctga tgaagtccaa ttattgctct tttaaataca 206640
   tttagctact tctgaatata aaatgagtat ctactaatta tttacaaaat cacttggtaa 206700
   atatagaaag tcacaaagaa tgaagtgatc atcctgtttt gtaacccaga aatagtcatt 206760
   actggcactt gtgtgaatca gtttctattc ctgtatgtgg atgtgcacag cgtatcctgc 206820
   tttgtacact agagtactag catttttcta atgtaattca atattgtcga aaacatttta 206880
   aaatagcttc catcacaata atctatcaaa ttgacttgcc agactctcat tattaggtta 206940
   atttatctct aacattatgc agtcatgagt aatactacaa aggatatttt tggacacaat 207000
   ttttcatcta tgcctttctt tataatcctt catcctaagg tcacagatta tgaatatctt 207060
   taaagtacgg acaagtcttt taaattttgt gtgcaaaaac agtgcaaagc cttgaatgat 207120
   aaaatagagg tttgatatat gtgttttttt gtttgtttgt tttgagacgg attcctgctc 207180
   tgtcccccaa gctgtagtgc agtggcacga tcttggctca ctgcaacctt tgcctcttgg 207240
   gttcaagcaa ttatcctgcc tcagcctcct tagtagcagg gtctacaggc atgtgccacc 207300
   acacccggct gtttttgtat ttttagtaga gatggggttt caccatgttg gccaggatga 207360
   tctcgaacac ctgacctcaa gtgatccacc cacctcagtc tcccaaagtg ctgggattac 207420
   aggtgtgagc cactgcaccc ggccgataca tgtgttttta aagtcacaga aatttcagat 207480
   gtcttgaagg attttaagca atttaaaaaa taaagtcata gaagcttcaa tttaggaatg 207540
   aatggaaaat tgatgatatt cttaggatat ggattttccc taaaagaaac aaatgtatgc 207600
   atccccaaag ataatttgat tagtatacaa atattaaatt aaacatgtcc atatttagag 207660
   ccatgaattc tctttgcctg tcacaatagc tggatttatt cacaattgta gtaattagtc 207720
   cctgttcatt ataattttct aggtgatatg aagactttgt cagtccaagc aagtgtccac 207780
   attgtgtgta gcaaacatga gaataaacat tttaaacttt taaatgtaat acatattagt 207840
   gttatgtaat gtcatccttc atgttcgaag gcacatggaa cattgttctg gtggtacaga 207900
   ggggagagaa acaccatcag aatgaaagga aagaccgctc tggaaccttc ctccttagct 207960
   cttgagctta gtttaattgt cctgtcttat ggtctgctac aagcaatacc actcttcacc 208020
   ttcgcatgct tctctgtggt ttgataaagt acatgcaatt tttcatttaa ttcttccagc 208080
   tgcactaaga aaggagcctt atctttattg aacagatgag gaaatgaatg attagagaat 208140
   ttaaatgact agctctaggt cacacagctg gaacttacag ccagatttcc ttttaacaat 208200
   cctgtaacca aaagcatacc agtagtgccc cataaaatgt aagttataga gctgtgttgg 208260
   gtcaaaactt ttactgatgc taagaggagg caacattaac aaggggaaat tatttgtgta 208320
   ttatgttttg gattatgttc tctccataga taaaagactg tcgtagtaaa agagattcag 208380
   ggcacaggga aactccacca caaagcgtgg taccatttcc cacagaagct aaatggacgg 208440
   gaagcctgcc accaggaaag gtaaagccac tgctcttgtt tgcaggctat gttaataagc 208500
   tgaagcttat tccgacacat ttacacatct ctgcatcaca ctgacccttc gtaaagatac 208560
   tcccagtgta acattggagc cagctccagc ccctgatcct gttgcttttt ccttagcccc 208620
   atgaaatcat ctgtgagaaa ttaagccaaa taagcaataa atcctgggat ctagggagtg 208680
   gaataagttt tgggaaagtc tttttttttt ttttttttga ctgagtcttg ctctgtctca 208740
   caggctggag tgcagtggtg cgatctcggc tcactgcaac ctctgcctcc cgggttcaag 208800
   tgattctcct gcctcagcct cccgagtagc ttggactaca ggcacacacc accatgccca 208860
   gatgaatttt tgtattttta gtagagatgg agtttcgccg tgttagccag gatggtctcg 208920
   atctcctgac ctcgtgatcc accggcctcg gcctcccaaa gtgctgggat tacaggcatg 208980
   ggccaccacg cctggcccgg gaaagtcatt ttaaaccaac ctatgtatga atccctacta 209040
   taatattctc accaagcggc tggctctttc tcctgagctt ggaaacctcc agtaaaatgg 209100
   aaataattat ttcccagacc accactctta tctgtgagct tttttggcca ttaaaaatta 209160
   tttcttccat tatattttta tctgtgtctt cacaggtttt ctctttcttt cactttagtg 209220
   cttttcttca aataagcagg aaaaatccaa tctatcatgc acatgggaac cctttcaata 209280
   ttggtctgtg gttgttccat tttatgggga tgcttttaaa gaaaaaattt gtcctttcaa 209340
   tatattgaat atcttccagc accacatcac ctgcaagctt tgtaaaaata gttctacata 209400
   ttaatttttt tttttttttt gagattgagt ctcattctgt cacccaggct ggagtacagt 209460
   gacatgatct tggctcattg caacctctgc ctcctgggtt caagtgattc tcctgactca 209520
   gcctcccgag tagctgggat tacaggcatg catcaccatg cctgggtaat ttttgtattt 209580
   ttagtagaga tggggtttca ccatgttgac caggctggtc tcaaactcct gacctcaagt 209640
   gatccacctg ccttagcctc ccaaaatgct gggactacag gcgtgagcca ctgcacccca 209700
   cgtagttttt tttttttttt aagttgaaca tatgtgaagg caggacctag tgacacatag 209760
   caataacatt tccaagtaga cattacacta gggaattagt cgaagtgctc atttaaagta 209820
   ccatctctca aatgtattaa aagagaatcc ttggatgtgc aataccttaa ttcaaaggca 209880
   gctcgttatg tataaactct caagctttgt gataaacaaa tgtgcataac agatgggact 209940
   attcacttac agcccaggga attttattga cgctgagaag gttatgtgac tggctctgcc 210000
   actgtcatcc ccattcactt cattttggag caatatgaca taaatgcctt acatgtgggt 210060
   tttctctatt tatcatgtgt ttcctatccc cttgaaagat ggccatattt gctttacttg 210120
   gttataagat cccatattcg ctgtcttgaa gccaaccaaa taatttgaca aagtgggttt 210180
   gtagtgctgg ctattttggt gaaaaaaaga caatgagact tcatgtgtca tccaaagttc 210240
   tatcagatcg agctgtgaga gaaaggaaaa gaaaggggtc tcagtcagga tgctcactac 210300
   atacatctgt gttgttgtct aggtccagat ttctgttcat tacgctatgg gctggctctt 210360
   atcatgcact tctcaaactt caccatgata acgcagcgtg tgagtctgag cattgcgatc 210420
   atcgccatgg tgaacaccac tcagcagcaa ggtctatcta atgcctccac tgaggggcct 210480
   gttgcagatg ccttcaataa ctccagcata tccatcaagg aatttgatac aaaggtaagt 210540
   atgatggaaa atagggctct ttgttgagag aaaaaacttt gaaaggaagg catagatctt 210600
   gattctgtgg agtatggaag tatacatttc caatgacaaa ttaaaactga ctggaactat 210660
   ttttctttga gacattgctt acttcaataa taaaaataag atttcattga ggttattatg 210720
   attataaggt gggggaactg tagagttaaa tgtgaaaaat ttaaaaatgg aacagtttat 210780
   gtgatgtctt caatgaaaaa ctaggtatta cctgggcaca ttcttatagg ttactcaatc 210840
   ctattcagtt ctctgcctgt tttattgttt ctgagcaatt ttatatccct gtaaattcta 210900
   tataaccaat agaaatgcaa acgattcttg tccatagctt tgcaaataaa ttttgccaag 210960
   agaaaaatca gttaaaactt ttctccactc acctcccagt tgaattagcc aattttgctg 211020
   tttgtttgtt tgtttgtttt ttgagataga gtcttcctct gtcattcagg ctggagtgca 211080
   gtggcatgat ctcagctcac tgcagcctcc gcctcccggg ttcaagagat tttcctgtct 211140
   cggcctccca agtagctggg agtaaggggg catgccaccg cggctggcta atttttgtat 211200
   ttttagtaga gacagggttt cactaggctg gtctcgaact cctgacctca ggtgatccac 211260
   ccgcctcggc ctcccaaagt gttgggatta caggtgtgag ccactgtgcc aggctctgct 211320
   gtatatttaa agtctatttc agcattgctt cctgcttgtg ttatgcgtga ttctttgagt 211380
   tttcctttga accagttata acatcttact tacttcctcc attaatcaat gagttaaata 211440
   aaatctttgt tgtatgttta ttttacattt atatgaaaac catgaattta cccaattaaa 211500
   aaaattatcc tttaaattat cttgtactgt acatttccca tgtcatccct ataattcatg 211560
   attaatgatt ttattacatt ggacctagct tatttacaat gagtacataa atttattgtc 211620
   tccagtcttt cctccattat cccgtctaca tatccacact gagtagattc actactcagg 211680
   aatcttggac accttcaagt tgccaaacat gcagtgttca ctggacatgc tgtgttcctt 211740
   cagaatttgg gcctgcttct cagcacactc acatctgcta tcaatgaccc atggaaagtt 211800
   tttgccctga gcaagccaga gtccctgtta gtttcttcca aatgctacaa gttcactttt 211860
   gctatttttt ccgatgagat aaaattttcc tttttgactt tctacaaatc atagtcattt 211920
   ttcaagggat agttcaagta ttgcttcctt tctgggacct tcccaaatta ttattttctc 211980
   ctctcaaagt ctctgtttta tttatgttca tcctcaaatc ttgattctca catgaatcat 212040
   ataccttgta ttatttatag tttttttgag tgggtaaaat atttcatatt ttatattctt 212100
   tggctctcta ctttatagca tgatgccaga tatttagggg ccttattgca tttatttttt 212160
   attttatttt aaaatctatt ttatttttta tttatttatt ttaaaatcta tttattttta 212220
   ggtaaatatt caggtaatat aatttatgta attatttagg aattttaggt agttatttta 212280
   aaataattca aattatttat tgagttatat cagaagaatg tgatcttatt catttgtaat 212340
   atgtgtttta ggaactcagt tcagccaggg cagaccatga ttcccaaact tgacttttct 212400
   ttttaattag gcactgattt tggttaagag ttcagtaaag ttttgtgtgt gtgttttaaa 212460
   aaattctttg atataagagt caagatgtta ctcaactttt actagaagca aaatagagga 212520
   agtgctttca cagatgaaat atctctcaat gttttcttcc atttacttct tcctattatt 212580
   catctatata atcattttct ttacctcttt tcttcatttc ttctgttttt ctctccttct 212640
   actaagacaa gcaaattagg ggtataattg gttatttggg aaggtaggaa gaatatagag 212700
   agaaacaaaa atcaatattt tatactaggg tctcactaac ctcaagcaac tctgactgta 212760
   aagtagattt tcataatagg acttcttgac aaagagtttt cctatttttc ccccaggcct 212820
   ctgtgtatca atggagccca gaaactcagg gtatcatctt tagctccatc aactatggga 212880
   taatactgac tctgatccca agtggatatt tagcagggat atttggagca aaaaaaatgc 212940
   ttggtgctgg tttgctgatc tcttcccttc tcaccctctt tacaccactg gctgctgact 213000
   tcggagtgat tttggtcatc atggttcgga cagtccaggg cttggcccag gtatccagat 213060
   actttctcat tcttggtggg atccagattt ctgaattcta caaaatatca aaggtcttaa 213120
   tgattttcat ttcagggaat ggcatggaca ggtcagttta ctatttgggc aaagtgggct 213180
   cctccacttg aacgaagcaa gctcaccacc attgcaggat caggtaagtg tgcacagatg 213240
   ggtcatagct ttgtcatctg ttccatccca ctgtgtctta tcttctatga atcaaatggt 213300
   ttggggaaga gagagaaaaa gtactgctga aaaattcaac aatataagac acttgcatca 213360
   caaataggaa agatgcatct gtgcagtaaa gacattgaag cttagaagta gaaaaaacca 213420
   ttgtgagcta ggtttcagct cagaaaagcc ttagtagtca gaaaagcctt agtagtcaga 213480
   aaagccttgt cggaaaaagt ttaaaccttt aagaattgca cacatggaaa aagatcaagt 213540
   aagctatata tacaccatct tagcaatgat tttgaagtga gaattaaggc taccacagct 213600
   ccaggtggta aggagagaaa tcaggctgga agagtttgaa gtttctgtat tattctaagc 213660
   tctttactat tctattatga gctcattaat tctcacaaca accctctcat ataagtacca 213720
   ttttaaattc ttattttaca gagaagggag ttaaggaagg tggagattaa gaaaattgcc 213780
   caaatacaaa tagccagcag gtggtaggtc tgagatttaa gcccatgcag atttagcccc 213840
   cagagcagac attctcaatc actatgctag actgcctttc catggtatgt gatcctactc 213900
   aggcctctac agctttatca ttgctgttct ccccagcctg tcgtgctgag agtatatact 213960
   cgaagagcag aactaaaatt ccatccagct tctcactcct aggtccacta cacagctgca 214020
   tcctgcagac ttttacctca agcaaccctc ctgcgttctt gcttccttcc atcatagttg 214080
   taaccatctc ctctatttgc aaatactatc tgctgatctc tctcttctag actggtttct 214140
   ttcaaccttc ttcccaccaa aaccaagtta gcttgctaaa ataaagatgg cacattttta 214200
   ctcacccgct tgagaatttt caatgtgttc cttcatgctt acagagtaaa gcctgacctc 214260
   tttattgcat gaatacaaaa gttcttagcc atctggcccc aaccttgttc cactcaactc 214320
   ccctgtgcaa gcatggctcc agtggcactg gacattggct gctctccaca tagatctgca 214380
   ctgcacttcc ctctggctct gctcccgtta gtttatatgc ctggaaagtt ctttgcccct 214440
   gttccttgtg ccaaaattcc atctatccta ttgcatagct tatgtaaaaa cttcctaaac 214500
   cttttttttt tttttttttt tttttttttg agacggtgtc tcactctttc gcccaggccg 214560
   gactgcagta gcgctatctc ggctcactgc aagctccgcc tcccgggttc acgccatttt 214620
   cctgcctcag cctcccgagt agctgggact acaggcgcct gccaccatga ccggctaatt 214680
   ttttgtattt ttagtagaga cggggtttca agccaggatg gtctcaatct cctgacctcg 214740
   tgatccgccc gcctcggcct cccaaagtgc tgggattaca ggcgtgagcc accgcgcccg 214800
   gccaaaactt cctaaatctt ataattatta tcaatttatc ctcagatata cttccacgta 214860
   cattgtagtt ttattatatt tatattttac atcttttttt tcaaatttca gtttgggacc 214920
   cattagtgag tcataaaatc cattgagcgg gttaaaatca ttattttaaa aaatgaatag 214980
   aatagaatag aaattgttgg agtgcattgg acatggtaaa gttaaatatc gattcatgaa 215040
   accatcgttt gaggcatatg tgtgtggttg tatgtacaag tgtttatgca tattggtgtg 215100
   tgtgttatgt taccctgtaa aatgcatttc ttactatagg tctctgtgaa atatgtgtct 215160
   tgttgttttt taatgtagac ttccaaagcc tacatggcat ttcactagtg acaatcaatt 215220
   ttattcacat ttttctctcc aattggacca gaagctcttt gagggcaggg gctgtatctt 215280
   accgattttt gtaagtcttt catttcctgc ccctagcctc atattagatc atgcaagaat 215340
   gcaactgtaa tcacaagaaa atgctaatgg gctgtgatag cagagagtta ctgtgacaaa 215400
   ctaagggatt tagatttggt cacattggtg ttgaggagcc attgaagaat cagagagtgt 215460
   gttactatta tttgttaatt ttaattatat catattactt tactggggaa aatctgtgag 215520
   ctattttaga aataaatact ctcattgccc aataattcta agtctgccac ctcactgttg 215580
   ggacattgtt tagggaggcc acgaagtctc agcctttgat attttcataa gtgtttttct 215640
   ccctttttcc tttagggtca gcatttggat ccttcatcat cctctgtgtg gggggactaa 215700
   tctcacaggc cttgagctgg ccttttatct tctacatctt tggtgagtca ctttctctta 215760
   aatcctaacg cctccatttc ctgagcatcc attttggcac ctacaccacc cacattcttc 215820
   ctatatgaaa gaaaatgtcc tttatcaaat ggaagatgat aaaaaatgtc aacggttggt 215880
   atcattttta atctagtcac acaacctgat taacaccttc ctggtggttc tgggaagcca 215940
   cacgcacaag gtagaggagt tgactattca catggcaccc accgacttgt gatgcagtct 216000
   tgtccttcca tatcaagcac cttctgcaga atctctacca ccacatctga agtgcctgct 216060
   atatgcagtt aagatgtcaa agatagtgaa gtacattttc aatgtgtctt catatttcat 216120
   tataattatt atttctgtcc aagatgcctt tcacctgttc tctaccaagt taatcttgca 216180
   aagttcaatt caaatgttcc cttccccatg ggcccttcca gggcttaccc tatcagattc 216240
   tggcattctc tcctttatga tatttcctct ctaggttatg ttggtgtgta attatttatt 216300
   tctccttttc tttccactag actgtgaaat gcttgaggca aggaatccat tctatgtttt 216360
   catcacttgg gtgtcatcat ggtgcctgat ttttagcttt aaaataaaag aatcagtgaa 216420
   tccagtaatt agaggggatt taaagaaaac tagtcctcag aatcttttaa catagaatgt 216480
   tcttcaaata aggaattcca ataataagac aattttctac acttgatttt gtttttatag 216540
   ccaaatggtg tcattaaata tagtcctggc ctgaatggct ttctcattaa tgatgctaat 216600
   tattttggtt tgtacatgtt aaccaggtat tgtacaaaaa tatttctttt gggaatccat 216660
   aatggatgta tggcttgaat acaaataata ctgtctcttg taagtgcatt ggaaattttt 216720
   ccctgccaca tgatttcatg gaaggttgtt tcgtgtatgt atgactgcaa acctgactat 216780
   tcagatcttc cgcaacaaga caacttatgt gtgcattaag aagttgctgc ctaaaataca 216840
   taacactgta atcattggag actttaaagt aattaatcag ctatgcaatg ccacgctcct 216900
   gttatctcca gagggctctg acattgacaa atggtggctt tctatttgag acgtaatatc 216960
   taaaaagctt taacaggttt gtagaaggat tgaaagaaag aatgggaaca tttaggtcct 217020
   tatggtagaa taagcattaa ttgattagtg tgtagaaggg agaggcatgc cacttcagag 217080
   gaaacttcct tcccccagta aacaaatcta cctaaaaact aattttatcc cttcttccca 217140
   ggtagcactg gctgtgtctg ctgtctccta tggttcacag tgatttatga tgaccccatg 217200
   catcacccgt gcataagtgt tagggaaaag gagcacatcc tgtcctcact ggctcaacag 217260
   gtacagtgca caccttgtac ctgtggccca tgcagaggtc tctagggcag ggtgtggatc 217320
   tcctctgaga ggcaccatct tggctgctct aatactcatg ctgattagat ctttcttttc 217380
   agcccagttc tcctggacga gctgtcccca taaaggcgat ggtcacatgc ctaccacttt 217440
   gggccatttt cctgggtttt ttcagccatt tctggttgtg caccatcatc ctaacatacc 217500
   taccaacgta tatcagtact ctgctccatg ttaacatcag agatgtgagt ttacttccta 217560
   tacttctacg aaaatgataa tggtaataag gagaaacagt tctgtgttac ctattacatt 217620
   ctggctttac atataaccat taatttaacc ttcacaatga ccttgagaga ggcattgtta 217680
   taattccctt ttcacagatg tggaaacagg acacttagag gtgagataac ttgccccagg 217740
   ttgcacaata ctaagtgata gagctgctgc agcatccata ttcttaacca ctatgctata 217800
   ctaccacacc agctgattcc aaagcttctt ttagaaataa tattgctggg ccaggcatgg 217860
   tggctcatgc ctgtaattcc agcactttgg gaggccgagg caggcagatc atgaggtcag 217920
   gaatgcaaga ccagcctgac caatatggtt tactaaatat catctactaa aaatacaaaa 217980
   attagccagg tgtggtggca ggcacctgta atcccagcta ttcaggaggc tgagacagga 218040
   gaatcgcttg aacccaggag gtggaggttg cattgagcca agatcatgcc actgcactcc 218100
   agcctgggcg acagagtaag actccgtttc aaaaacaaaa aacccaagaa attaatattg 218160
   cttttatctg gagcccagag tgatgcagct tctggccctc ttatctgaga cagtgttctt 218220
   ttagtgtgaa aaaggatgct aattttcccc caaacaaccc acagtatcat gggggtaagt 218280
   taatggctgg tctgtgtaac tgacaaattt tggtgctaac gtatctctat aactactctg 218340
   tataaacttc cttccttcag agtggagttc tgtcctccct gccttttatt gctgctgcaa 218400
   gctgtacaat tttaggaggt cagctggcag atttcctttt gtccaggaat cttctcagat 218460
   tgatcactgt gcgaaagctc ttttcatctc ttggtaagga taagcgtgtg ggcccattta 218520
   accaatccct tttctgcaca tggtctcaga gggttccctg acagcatgtc ctcattgccc 218580
   agggctcctc cttccatcaa tatgtgctgt ggccctgccc tttgtggcct ccagttacgt 218640
   gataaccatt attttgctga tacttattcc tgggaccagt aacctatgtg actcagggtt 218700
   tatcatcaac accttagata tcgcccccag gtaagagctc tacctgtttt ttcccctcct 218760
   ccagacccct ccagaggtgt tagacctcag tggtcgccgt gaaactcttt aatgttactg 218820
   acattgcact aatggcagaa tgacaaataa ctacaaatat ctgtctgtgg ccatttttag 218880
   aacaacaaat gtggcatttt tagaacaaca atttccaatc ttggccagta atcattttga 218940
   caaaaacctt cccaagcttc cctaacagag attgaactgt gtatgctggg aaaaggccca 219000
   cacacaggtg atttggaaaa gtttccatgg tgttgttcat attagctacc atatatatat 219060
   atatatatat atatatatat atacagtcac aataagccag ctcctgtgcc aagacttgcc 219120
   atatatcaac acatctaatc ctcacagtta tattaggtag gccctattgt tatccccatt 219180
   ttataaggga gaaggctgag gcacaaggag gttaaatggt gtgactatgg tcacataaag 219240
   gcagagccag gatttggact gggggagtct ggctttggag tctgtgtcct gcccgttgca 219300
   caaactggct tctccactga gcagccgggg taaagaaacg tggttcccag agagactgca 219360
   ttgctccctg gttattgact tggtagattg gtaatttcag gtttggcaaa tagacattgc 219420
   cctgaatgtc tttaggtgaa tgaaaaactg cattaagcaa aatgactttg ccattagagc 219480
   tgaattgcat taaagttgag ttgctgcaga agctgtaggt ggctttctat ataaaatcat 219540
   ttataaaatc atcttcccac agatatgcaa gtttcctcat gggaatctca aggggatttg 219600
   ggctcatcgc aggaatcatc tcttccactg ccactggatt cctcatcagt caggttgggc 219660
   cagtttattg aacatcttca agtggcaggt attgttttag gtgttggaga tacacacggt 219720
   gctctaaaga tctggatggc aacacaatta ctctatttac atgagcctct aaatcagact 219780
   ctggtaggtc agatttccca gaggaagaaa aatataagct tattttctca agatgaatag 219840
   atgttagatt gattaaaatg agctgttccg gtgcagaaga cagcacgtgt gacttcctag 219900
   aggtacatga gcatgaaaca gttcttagtt atgaccagaa tgaaagacac atgtcaagga 219960
   atagcaagag acgaagacag aggggcaaaa gaagatcatg aagaatatgt tcagactaat 220020
   ccaattttta aaaaatcaca aaagggaaac aaagtgtcct aggccagttt aaagataatt 220080
   taatgtctgg aaacagatcg gctgtgagac attgcaagga ggcttgctcg gtgtttggaa 220140
   atgcaggctc atgaggaaga tgaaaagaca gacccaggca gggatggaag gactgacgag 220200
   aaccaactta caaagagaag ttttgttttt actacatttc tatgtgatca agttcccagg 220260
   ttaatatttg actaaactgc taggaatcca ctgtgactat aatgctggaa atgacttagt 220320
   agggctttct gaggagggtc acacagaaga ccaaagagaa ctcatgttga attgagatgg 220380
   gttgtagtga tagttgtcaa cagccaatac agaaacaaaa aaaaacaaaa caaacagcaa 220440
   caacaacaac aaaaaaaaac agagaagaca caaacacaat gccacaatgc cattttaggc 220500
   ataattttaa atgagtaata ttatatgttg aaatccaaat tttcagaaaa acattagtgt 220560
   attttatttt tgtttaaaga aataaccatc tcaactcaga accccatgtg cattttggcc 220620
   attttgtttc caatagtttc ataaactttc ttaagtaact actgcacatt gttccttata 220680
   ttccttgtga tcaacattgc aatacacaac tgggagggct actagaactg gtgtagaagg 220740
   aacttgtgag attgatcatt ttctctgttt tttacatcta ggattttgag tctggttgga 220800
   ggaatgtctt tttcctgtct gctgcagtca acatgtttgg cctggtcttt tacctcacgt 220860
   ttggacaagc agaacttcaa gactgggcca aagagaggac ccttacccgc ctctgaggac 220920
   ataaagttac aaacttaaat gtggtactga gcatgaactt tttaaacatt ttttacttct 220980
   ctccatattc ctgaccatag actcagcagt tcttaactct ggctgtgtgt tagtcttccc 221040
   tggggagcct ttataagaca ctgatacttg ggacccactc cagagattct gaatgaattg 221100
   gtctggggtg gaacccagat actactaatt tttagatact ccttagaggt ttctagcatg 221160
   cgcccggggt tgacaacagc tggacaaact tgaaaagtca attcatgtgg cctttgaatt 221220
   ttcctcattg gaaagtacta aataaataaa aattcatgtg aaaatgatca ctgataaata 221280
   tcttcatggt ggggcaggtt attggatgca gagaagatct gctcggaatt gtagccatat 221340
   gttacagatc tcagcaccga tcggaactgt aaagctataa tccccagaat taaagttttt 221400
   attatttttt atacattgta aaacatagac gtttatttat gtgattaaat tctattaaaa 221460
   tttacatgct aaaataaaat agaccatttt caaattattt agatccagat atttccatca 221520
   gattaaacag atatttattt atcctagccc aattgcaaga gattaatgat gagaaaatga 221580
   ccaatacaag attaaataaa tgaggttaac ttagaaatca aggacagaga agatagaact 221640
   ggaaggcttg tattgtgaga agaatgaatg tgaaggaagg caatgtagac acttccagaa 221700
   gggatagcaa tatagtttag accatataat gaaaattgga gagagatgac agagacactt 221760
   tcaagtgaaa tgacaattta tatgggggag aaaaatattg aagacataac aagatgagaa 221820
   aaggcataga aatgtatcac atacaaggca tagaagtgta tcacatacaa gagaagttcc 221880
   ttttgagcgt agaaaaagat aatttaacct tcttcatatt tttcttactt tcccaagata 221940
   ctcagatagg cagcgtcaac tctaacagga attaatttgg ctcctaacac ttaagacata 222000
   tcctttagtt tgtctcctca cacagaactg attctggttt tgccacaaca tgtctagaga 222060
   agaagttccc accatatttt aaatcctatt aaaaaactgc ttggacaaga accttgggtt 222120
   aattcagcag atgaagagaa tctcctaatg caaatcaatg ggtatttttg agcaagtttt 222180
   tcagaaaaac agagtgtcag gccctgaggg tggtactaag atgagaacat tgattttgcc 222240
   ttcatgatat tgacaacaca aagaggaaag ggggtttgca gaaaactaaa agaagaagta 222300
   gaagaaaaaa gaaagacata gtataatagg tagtcaaatt atgtacagaa aaaagagaaa 222360
   aaaaaaacaa aaaagggtgg gggacagaca acccaactaa aaaatgggcc aatgacttga 222420
   acagggactt cataaaagag aaaatgtaag tggctcctta acatataaaa agatgttcaa 222480
   cttcattagt cattacagaa atgaaaatca aaactacaat gaaataccac tataaaatta 222540
   actaatggat aaaatgaaag gagatggaaa acaaaatgtt gccagacatg tggagcaact 222600
   ggaactttca tacgttacga atgtgaactt tggaaagctg ctcggcaata tctcctaaag 222660
   ctaaatgtac aattccagtg actcaaacat tttacttaga aatgcacata tacatccata 222720
   aaacatgtac aacaatgttc ataggagcac tatctgtaat agcctgaaca ggaagttgtc 222780
   tgttaaaaaa agaatgagta aataaaccac ggtctatttg tatagcaatg agaattaaca 222840
   gaccccaata tataatagat gaatgggtct cataagcaca atattgatta aaggaagaca 222900
   aaacgcacat tcttttaaag gtttataaaa tactttttaa aaacagctac aaccaatctg 222960
   tcctgttaaa aatcagtgag cgatttccct tgtgcaggga tgggggttgt ggctggatgg 223020
   atggtactta agaagtgctc ctggggtact agaaatattt tatttcttga cttggatgtg 223080
   tgtttacttt gtgaatattg tacatttatg atttgtgcac gtttatgaat gtagaaaata 223140
   aaacagaaag caaattcaaa gtatcatcct tttgagagct tctgctctga cttcgttttg 223200
   accaatggag cagttgggaa ggggtcttgg tccttcggtc ctttgctttt tttttttttt 223260
   tttttttttt tagacagagt cttactctgt cgcccgggct ggagtgcagt ggctcgatct 223320
   tagctcactg aaagctttgc ctcccgggtt catgccattc tcctgcctca gcctccccag 223380
   tagctgggac tacaggcacc tgccaccatg cccggctaat tttttgtatt ttttagtaga 223440
   gacggggttt caccatgtta gccaggatgg tctcgatctc ctgacctcgt gatccgccca 223500
   cctgagcctc ccaaagtgct gggattacag gtgtgagcca ccgcgcccgg cccctggtcc 223560
   tctgctttca tgttcttctt ggtcctgttc ctcctcctct tttgttggaa cttccagtat 223620
   cagagcagga aggaaggcaa tgggtcaatc gatgctgtca gcttttggat caaactgcaa 223680
   gttctcaaac agcaaaatta atgagctcag gctttgaaga aaccatgacc ctgaaagcat 223740
   cagttgcttc caattgcatc agttgccacg ggtgataaga acaatgatga ctcagaatgc 223800
   ctaggttttc ccagcagctt ctctgaggtt ttcccagcag cttctctgat tgattcctga 223860
   cagatgactt cggtgtgtca gactttcagg gtatctttcc ttatgtgatg gtttgaggaa 223920
   gagttaccat tcacattcct aatggcttca gaatagatgc aattgtgaac tgataggaaa 223980
   catttctaat tcatctcccc tccccatccc taaaggattg tttctaacaa tagtcatgaa 224040
   aattaattca cttttctcaa atagtttatt gtcatctacc taatgatgag atgacttact 224100
   ttttctcctt gactgttaaa tattatgaat tatattaatg tatttcttaa tgttgagctt 224160
   tcccttgaat attcttttga tgtacgacag aatttgattc actaatagtt tatttaggac 224220
   tttggctgat gtactgatat atgagattgg ctctgtatgc atacatgtgt tttgtgtatc 224280
   ttttttgtgt ctggatatgg agcttatgct gatttcaaaa acaagaaagg agaactttcc 224340
   tttttcccca ttactctgaa aaagattgac tagaatggaa tttttataat tgctgttgtt 224400
   atttgaaagc ttgaaagcat tggtttgtaa aaatcatgca ggctgaaagc cattttgagg 224460
   agactttgat aactttctca atttccttca gttactggtc ttttaagggg ttttatattt 224520
   ttctttgatc aattttgacc atttatgtta tcttggagga tcatctattt tacacactat 224580
   ttaaagtata tttgcaaaaa ttcaactgtt ttatcaggct atctttttaa taatatattc 224640
   attttatcta tatctgaggt tttagcttct ttgtacttct gacccaattg catgtgtgct 224700
   ttctttctcc ttcattagac tacttagtca tttactaatt ttaagaatag cttgtctttt 224760
   atttatttac ttatttattt ttgagacgga gtctcactct gtcacccagg ctggagtgca 224820
   gtggcgcgat ctcggctcac tgcaacctcc gcctcccggg ttcaagtgat tctcctgcct 224880
   cagactcccg agtagctggg attacagtca tgcaccacca tgtctggcta atttctgtat 224940
   ttttaataga gatggggttt tgctatgttg gccaagctgg tctcaaactc ctgaccttag 225000
   atgatctacc caccttggcc tcccaaagtg ctgggattac aggcatgagc cactgcgccc 225060
   agccctgctt gtctttttat tttatatttg attagcttta tcttttatca agcttatgtc 225120
   ctatttccct ttgctttact tcatataaat tttgttttgg atagtttatt tatttttcat 225180
   ttaattatga aacaggttaa agcttagagg aaaattgctc ctctaagtcc aattttgtgg 225240
   gcagattaca ttttgctgtg ttgtgctccc aaattcattg ttcttttaat gctttatttc 225300
   tcaagttaat aacctatata gtaaaaaagt ggctgttgac tctcagcttt tttttttttt 225360
   tttttttttt gtagatacag ggatcttgct gtgttgctca ggctggtctg aaactgctgg 225420
   cttcaaggga tcctcctgcc ttggtctcac aaaatgctgg gatgacagac atgagacacc 225480
   atgcctagcc atgtctctct ccttatatat aataagaaaa cagacacact gaggcatcct 225540
   atcatctcac tcttggtttc actactgttc tctggaagtt ttgctctgac cttttgcagt 225600
   taatgtatta attttgcatt gagtagtttc catagaagaa ttatagcatt tgcattctgt 225660
   tgggtattat acttttcact gttatttgaa cataatttga gggctgaaac caagatgagg 225720
   caagtgaggt gcccaggaag caatatttaa ggaggcatcc tttcttaggc tcatgcaaga 225780
   acagaattgg cacatgagag tgagtgcctc cttaattttg agtgctggac acttcttgct 225840
   cacttagcat acccctggac aatgaagtgt tttttgtttt gttttttcat gtccatcctt 225900
   tatccttctt catctcaaaa catttcaatg gagtattttt ttggagcagt acttggatga 225960
   gcctctgagt cccacagtag ctgagaattt atttcatagt actctttatg atcactgtgg 226020
   agccttaaaa cattgtaata ttaacttagc tgggaacaga aattttgttc cacaatttgt 226080
   cttattcaga acagtattga cttcctgcta gtctcttctg atgtccaata tgaggaagtc 226140
   tagttagcca gctacttttt gtaggagagc tatgtttagg ctaggtgcta taggattctc 226200
   tttatcctgg aattccttca ccaagatgtg ccaaggtgtt aatcattttc tcttgctttt 226260
   tggctggtgg tcttagagtt tccttcgatt ttgttttatt tagtgattgt cctcaatttg 226320
   ttttctttac taagaatctc tcttctattt atctgtatgg taaaaccttg ttgcccatct 226380
   ttctggtttc tgctgacttt catttttgga ccttttactt tgctttctcc atggactttt 226440
   tggtagtgga ggcaggcaaa cactttccaa agtctttctc aatttccatc aatttcaact 226500
   tatttcctaa aattgcctca gaatgtgcct atgtccacaa tatccctcct tccactttag 226560
   aaaggaaagg catccacact ttatttaggt gcaatgcctg aagtgtaaac actttctggt 226620
   tgtcaacaaa ggagtacttc caaatattgg tttggggata acctgctaat gattaacaca 226680
   ttcaccttgg ctcttggttt gcctgctccc tcttctttta tctgctgtgt gtattttttt 226740
   taatcactga gaatatgcac agtattgtat gttttattat aagagaggac tggccagagt 226800
   gggaatgttc tgaattcaga ataactgaag cagtacagga taggaactca ttctttcaaa 226860
   tgaagctggc atattttccc agagcaccaa atttcaatat atatttaaaa aacttgatat 226920
   gaatgataca ataaagtggt tagaactttt attaaaataa acttatgtca tgaaatactt 226980
   attctaatta tagtcactct tcatcttatt tcatcttata acatgtttaa tgttttcttt 227040
   tatttacaaa acaatttatt ttttgatgaa aagttttaga aatcaagtta aaaatattca 227100
   aaggaatgcc taaagttttc aaaattcttt tacatgttgt acaatcaaaa gagtctgaag 227160
   accatttagc tatccaaatt gtttattttt aagcagtatc ccttctaata tttactattt 227220
   ataatcctta aaaatttgcc ttagcacagg agaattgctt gaacccagga gacggaggtt 227280
   gcagtgagcc aacacagtgc cactgccctc cagcctcggc gacagagtga gactctgtct 227340
   caaaaaaaaa aaaaaaaaaa aaaaaaaaag gccaaaaaca aataaacaaa caaaaaaatc 227400
   cgccttaaca ttatttgttc attaaaaact ttctttaata ctactagttt ccctttcctc 227460
   tcagcccatt gtcatatttt gatttttatc acttgctttg taggacatat gaggtttttg 227520
   tttttttttt tttttggaga tgcagtctcc ctctgttgcc cgtgctggag tgcaatggcg 227580
   caatcttggc tcactgcaac ctctgcctcc tgggttcaag caattctcct gcctcagcct 227640
   tccaagtagc tgggattaca ggcacccact accacgcctg gctaattttt gtatttctgg 227700
   tagagacggg gtttcaccat gttggccagg ctggtctcga actcctgacc tcaagtgatc 227760
   cacaatcctt ggcctcccaa agtgctatga ttacaagcat gagccacctg cccagccaga 227820
   atatatgttc attttgagtc ctttaacaaa gtcataagaa ttttaggaat tcagttactt 227880
   tcttgagaaa atctctgaaa agatgccaat aatttgtagc caattatatt gatttctctt 227940
   tttcatattg agaattgttt tttaaaaagt ttgtatgtgt gaagattttt gcactgtagt 228000
   taaagaaacc acctgtgtgt tggttaagcc ataagtacat gtattcaaat aaattgaggt 228060
   ggggttactc tgagaatcaa aggaaaacct gaagaaacag gcagcctcaa aaggtcttag 228120
   ctgtagcaac ttgctccatt gttgaaataa ataggcttga acttgtattt tccctctact 228180
   caacatttaa ggtctcagaa gataatataa ttggtgaaat ttaagtaaag tgctcactct 228240
   tttgctttaa caaaccctag agagctggta ggcagagcct caacagaccg ttttagcttc 228300
   caaagggagt tcaggacacc atgattcacg accacaatac atcacacata attgagaaaa 228360
   gatagttcca ccaaataaag ttgaaatgct gacaagaagg ggtaagaaat cttggaaata 228420
   agtttatata aaatttattt tttccttttt tattgttatg gaataggacc agttctactt 228480
   aagccaccca tttgccaaaa taaagtgaga atcgtttctt ttggggactc ctctttgtag 228540
   ctccaagtgc cactaacaat tcttaggacc tgagctataa gccaggtgat ttcagttaat 228600
   atgatcaatt atttcattta aatggctcta atgtgcagag ggaacggagc ccatcagcat 228660
   tccctgcagg gaactgcagt ggcttttatc aacttgaaca gctagctttc aactgttttg 228720
   aaatcacttt cagggtggtc atgtagttgc ttttttgaaa tcagaagatg attctgcctc 228780
   ttttaatatg tgactcctca gattcagaaa gtgctcgcta gtcttaagag tgaattaccc 228840
   tcagtggtcc agcgcttatg aacccacatc taaccctatc ccctggggga actatcagag 228900
   aaattggtgc catggacata agaggaaggc acagtgaagc agagagcccc gcatgatgaa 228960
   aatcagtgga cagcatcatt atttacaact ttgtaatcac ccaggagcat gaaaatccag 229020
   gccaatctgg caccatgagc tctaattttt gttggagttc ttggaaccga ttctgatgaa 229080
   tgactgttta gccattttag agtgtggcat acgtggctgc tggcatacag aggttggatg 229140
   taaacgggcc tttgccctct cttatgaaca tagacaggaa ctaaactgtg tcacataggt 229200
   tccaaatggt ggcctgaata ctatttacaa ctaaggtaca atgaaattga gtaagtcttt 229260
   tcctcttttg cagataccat cattattcat atatttcttc aaagttaact atttgtattt 229320
   ggtaattttt aatagaaatg taataattgc ttctcaagtt tagtctttag tcttaaggtt 229380
   gatgctctcc atgtccttcc aaaaaaaggt atgttgcttt tattatatcc tcgccttcag 229440
   atgggattat tccattttgt tctttgttaa tatatacttt gagccacttt ttttgtggct 229500
   ctgggtgaga tgctataggt acaatgacaa gtgatacgtg tgttgtccct gtcacaaaag 229560
   tggatagcct aagtggtgac ttttacctcc actccaaata tatgtatcac acaccagccg 229620
   tatgccaggc accactctag gtgctaggga tacagcagta aacagacaaa tgcaacccct 229680
   gcccatgtga aagagaataa gacaataaat aagtaaagtg catgttatat ggaggtggca 229740
   aatgctaaaa agaaaaatta agcaggcaag aggactcatt gaaaagatga catttgggta 229800
   aaagcccatg tatatatgtt ctattggttt tatttctctg gagagccctg actaatacac 229860
   aatgactttg agaagttact ggcttttgat ttatcacact attcggagtg ctgagagcct 229920
   tcttagtgtg tattcagtgt tttaagagag cttgtggatg aataataaat aggacaaaat 229980
   ttatccaaac ttaagccttg ctttaggtaa aagggctcct cttacaaggt agaaggttat 230040
   tatttggcat ttaaatccaa ctgaagacta ataagactaa ttaattaaaa gtttttaaat 230100
   cacaactggg tgcaaaataa atggaactgc catgctcgcc aagtgtgcat gagtggtgtg 230160
   catgggagac agcacgaagc taatcccact catcttgcag gttgctccat ttttctccta 230220
   aaatcagtaa gacagaagct ggtcagatta tcaagagccc tagttaaaca cagcagtagc 230280
   atttggaagg ggttgctctc attaggcagt gcctgaccac aacaagagat gaacaagccc 230340
   tgtatctgaa gccatcatgc ctagttatgg tcccccactg ttcatgatgc ctgaaaggga 230400
   ggccccctgc accctagaaa gctgggtggg ttctactgtc tgctttactg ctaaaaaccc 230460
   tcttctttgg atctggactt tacctctatc tgattttttt ttctaatata tgatttggca 230520
   ctgagtctgt cactgctgct aactcagcag ttctagggtc attgccccat tgcctcacag 230580
   aaagaatttc atagcttcca gcatcctctc tccttcatta tactttgatt tcagcattgc 230640
   tattttttct cttgggtgtt gcagctctct ctctccttcc catgtcttgt tggttttctg 230700
   ctaactcctg ctttttttct tttttttttt ttgagacgga gtctcgttct gtcacccagg 230760
   ctggagtgca gtggcacaat ctcggctcac tgcaacctcc gcctcccggg ttcaagctat 230820
   tctcctgcct cagcctccca agtagctggg actacaggcg ctcaccacta tgccccacta 230880
   atttttgtat ttttagtatt gctgtcatca atccacatgt ccagaagcac ctagaaactc 230940
   taattctttg taggtatcaa accctaggac tctttcctct aatcacaata tataatccct 231000
   gattcccaaa cacggtcttt tcatatacat tttccactgt acatactttc tgacctggaa 231060
   agctcttaca caaacacgcc ctcccctagg aagcctttat aaatgttccc aggaagaatc 231120
   agtcacccaa cagtgtcctt gtcacatctt aggttctaca cctttatttg ttctatctga 231180
   atgtaatctc ccagagggtg ttatcatctt tttttttgag atggaatctt gctttgctgc 231240
   ccaggctgga gtgcagtggc atgatctcgg ctcacagcaa cctccacctc ctgggttcaa 231300
   gtgattctcc tgcctcagcc tcctgagtag ctgggattac agacgtgtgt caccacacct 231360
   ggctaatttt tgtattttta gtagagacag ggtttcaccg tgttggcaag gctttcctcg 231420
   aactcccaaa ctcaggtgat ccacccgcct cagcctccca aagtgctggg attacaggtg 231480
   tgagccacca tgtccagccc catctttttc ttttagttta gttcttaaca aatagtctga 231540
   cacaaagtgg atataacaat attttgaatt atgaataact aaatgaatat ttccagattt 231600
   cctggtgctc tcaaagtttt atgttacaaa agaaaaacaa gtctaaaata cctgcctcaa 231660
   gtttttatct gtactatgat ttcaaaccaa ataaaaaaca ggtggggtaa aaactgaaac 231720
   aggaaataca tataactgaa aaattttggt atgttagtat gataatacta ggtcattttt 231780
   cctgtttccc caacttcatt ttctatagca ataaaaagaa acaagtaaat gtatattaat 231840
   ttaatttaaa agaagtagtc taccatctct tctgttaaaa agaaaaaagt attttaaaaa 231900
   attatctctg gaaggataca cagggaacat tgctctggtt tcttccaaga gagaaatgag 231960
   gaactagaga gcatggccaa gtggggtttt gcttttgttt ttgtttgtct atctgttagc 232020
   tttttattat tttcttttgt aggtttgaat ttcaaaccac ataaatctgt tacatgctca 232080
   taataataag tttaaaataa aacttttggc tgggtgcaat gacttacacc tgtaatccca 232140
   gcgctttggg aagcagaggt gggaggatac ttgaggccag gaatttgaga tcagcctggg 232200
   caacatagtg agaccctgcc tctgtagaaa taaacaaaaa ttagctggat atggtggtgc 232260
   atgcttgtac tcctagctac ttgggaggtt gaggcaggag gatcctttga gtccaggagt 232320
   ttgaggctgc agtgagctat aatcacccac tgcactatag catgggcaat aaggtgagaa 232380
   cttgtctcaa aaaaaaaaaa aggggggggg aaacaaataa ataaatataa acaaaacttt 232440
   tgtttcaaaa tatgtaatat ttagcactaa agaattctga attgtagagc taaaaagtac 232500
   ttaaaagtta ataattattg tctcctttaa aagaattgtt atcaaagtat aatttttatc 232560
   cagaaaatca tccatatcag caagctaaac tttctcaaaa tgacatatcc atgtaattag 232620
   ctcccaggta attagcaggc agcctctact caggttgagt attcctaatc taaaaattgg 232680
   aaattcaaaa tgctccaaaa tcggcaactt tttgaatgct aacatgattc tcaaaggagt 232740
   gctcatggaa tatttcagat tttggatttt tggatttgag atactcagta taatgcaaac 232800
   attccaaatc tgaaaaaatc tgaaatactt ctggttctaa gcataaggga tactcaacgt 232860
   gtgttagcta attagaccct tcatggtctc ttctagacct cagcttcttc aaggtaacct 232920
   ctatcctcac ttctaatagc atgaactttt ctgttttaga ataatttgga ttttcaggaa 232980
   agttgcaaag atagtacaaa gacagtacag gagagttccc atatatcttt cacctagctt 233040
   tcccccattg ttaggatttt acattattat gatacatttg tcaaatataa gcaactcaca 233100
   ttgatacatg aaactctatt aaccaaaccc tagactttat gtggatttca ccactgtttc 233160
   cactaatgtt ttctttctgt tccaaggtcc aatctggaat accacactgc attttcttgt 233220
   catatctccc tagtcttttt ttgtctgtga caatgtctca gtcttttctt gcttttcatg 233280
   accttaacag tcctgaagat catttgcttt tttttcataa ttacaccgga gttatagatt 233340
   ttttgaaata ataccacaag ggcaaagggc ccttcttgtc acatcatttt agggagaaca 233400
   tgatatccac atgacatcac tgatattaac cttcatcatg tggtttaggt aatgtttcag 233460
   gtttctctac tgcaaagtga tttttttccc ttaatttagc ccacctgaac ttatcaattt 233520
   tgttttcttc catgactaat acttttgtta ttatagctaa aacttcattg gggccaaatc 233580
   ttagatcatg taaattttct tctatatttt attctaaaag cttgtaatgt ttgatacatt 233640
   ctaaaagatg taatgtttga tacattacat ctagtccttt gatttatttt tagttacttt 233700
   tgtataaggt gtgagagatg tctccagttt cactttatta acacattgtg gtgttccagt 233760
   actatttgtt gctaagacta tcttttttcc attgattacc tttgccttag ttggcaatat 233820
   ttttgttggt ttatttctag actgtttatc tcattccact gatttgtgtc tatctttttg 233880
   acaaaactgt tgattacagt aagctttgaa atagttcatt ttttgtgtca acttgactga 233940
   gtcaggggat aaccagctat ctggttaaac attatttctg gctgtgtttg tgagcgtgtt 234000
   tctggatgag attagccttt gaataggtga tcctagtaaa gtaaactgtc tttcccagtg 234060
   tggatggcat tatgccacct gatattcagg gtctgaatag aagaaaaggc agaggaaggg 234120
   ggaatttggg cctttttttc tgcctcactg cttgagctgg gacatctcat ctggtctcct 234180
   gctcttgaac tgggatttac atcatcagtt cctctggttc tcaggccttc agattcagac 234240
   tgaatcatac caccagcttt cctgggtctc cagcttgcag attacagatc atgggactcc 234300
   tcatcttcca taaatgcatg agccaattca gtctatgtcc ttgaaaactg ccccactgca 234360
   gattaaggct tttttccact aggtgaaata aagaagcttg ttagacagat ttcccttcat 234420
   ccagtgccct ctcctcttta agttacaaca cattggctac acctaagtgc aggggtgggg 234480
   atgagggtat agtcctcttg tttgctgaga agagaactgt attgggaaag ctctagaagt 234540
   gtttgataca tacataaaca aggcatggtt tttgcactta atttcacatt acatttttcc 234600
   cagaaaaaaa ggaatgtata ggcatcacgt aactgtacta gctggagtca ttcttcctga 234660
   ttatcaaagg taaacagtta ttaatcctat accaagatgt caaggagaag tacttttgga 234720
   acacaaggaa ttctctggga gtccttacta ctctcaagcc cagtgaaaaa gttaatgaaa 234780
   aactatagta ccttcctata agctggatga ctaattacca ggctcattta ggaatttgcc 234840
   ttaccaagta aaacataagg gcagctgagg tgctgactga agacaaatgg agcatagaat 234900
   aagagtagta aagaatgcca aaaatgctgt catgtatcca ttgacaaaag gagctataaa 234960
   gcctttaggt attttcacac ttgctctgtt acgtaaatgt atgtgtgtgt gtgtgtgtgt 235020
   gtgtgtgtgt gtg 235033
<210> 21
   <211> 237326
   <212> DNA
   <213> Homo sapien
<400> 21
<210> 22
   <211> 15
   <212> DNA
   <213> Homo sapien
<400> 22
   tcacacaccg attgg 15
<210> 23
   <211> 16
   <212> DNA
   <213> Homo sapien
<400> 23
   ccaaactgat ctttga 16
<210> 24
   <211> 14
   <212> DNA
   <213> Homo sapien
<400> 24
   tatatatata tata 14
<210> 25
   <211> 14
   <212> DNA
   <213> Homo sapien
<400> 25
   cttttttttt tttt 14
<210> 26
   <211> 14
   <212> DNA
   <213> Homo sapien
<400> 26
   aaaaaaaaaa aaaa 14
<210> 27
   <211> 10
   <212> DNA
   <213> Homo sapien
<400> 27
   aggatggtct 10
<210> 28
   <211> 12
   <212> DNA
   <213> Homo sapien
<400> 28
   ttgttgttgt tg 12
<210> 29
   <211> 20
   <212> DNA
   <213> Homo sapien
<400> 29
   atatatatat atatatatat 20
<210> 30
   <211> 12
   <212> DNA
   <213> Homo sapien
<400> 30
   aataataata at 12
<210> 31
   <211> 11
   <212> DNA
   <213> Homo sapien
<400> 31
   tttttttttt t 11
<210> 32
   <211> 20
   <212> DNA
   <213> Artificial Sequence
<220>
   <223> Primer
<400> 32
   cctgatgctc gagtgaattc 20
<210> 33
   <211> 20
   <212> DNA
   <213> Artificial Sequence
<220>
   <223> Gel purified oligo
<400> 33
   cgacccagca acctggagat 20
<210> 34
   <211> 20
   <212> DNA
   <213> Artificial Sequence
<220>
   <223> Gel purified oligo
<400> 34
   agctcgagcg gccgctgcag 20
<210> 35
   <211> 20
   <212> DNA
   <213> Artificial Sequence
<220>
   <223> Gel purified oligo
<400> 35
   agaccccaac ccacaagaag 20
<210> 36
   <211> 20
   <212> DNA
   <213> Artificial Sequence
<220>
   <223> MAP1
<400> 36
   cgttagaacg cggctacaat 20
<210> 37
   <211> 20
   <212> DNA
   <213> Artificial Sequence
<220>
   <223> MAP2
<400> 37
   gccgattcat taatgcaggt 20
<210> 38
   <211> 20
   <212> DNA
   <213> Artificial Sequence
<220>
   <223> -21
<400> 38
   ctgtaaaacg acggccagtc 20
<210> 39
   <211> 19
   <212> DNA
   <213> Artificial Sequence
<220>
   <223> REV
<400> 39
   gcaggaaaca gctatgacc 19
<210> 40
   <211> 21
   <212> DNA
   <213> Artificial Sequence
<220>
   <223> Primer
<400> 40
   gcatcagcga ttaacttcta c 21
<210> 41
   <211> 22
   <212> DNA
   <213> Artificial Sequence
<220>
   <223> Primer
<400> 41
   ttgcattgtg gtgaaatcag gg 22
<210> 42
   <211> 21
   <212> DNA
   <213> Artificial Sequence
<220>
   <221> misc difference
   <222> (1) .. (1)
   <223> N = biotinylated cysteine
<223> Primer
<400> 42
   ntgagtaatt gtttaaggtg c 21
<210> 43
   <211> 21
   <212> DNA
   <213> Artificial Sequence
<220>
   <221> misc_difference
   <222> (1)... (1)
   <223> N = biotinylated cysteine
<223> Primer
<400> 43
   ntgagtaatt gtttaaggtg t 21
<210> 44
   <212> 20
   <212> DNA
   <213> Artificial Sequence
<220>
   <221> misc difference
   <222> (1)...(1)
   <223> N = phosphorylated digoxigenin adenine
<223> Primer
   <400> 44
   ngaagagata gatatggtgg 20
<210> 45
   <211> 20
   <212> DNA
   <213> Artificial Sequence
<220>
   <223> Primer
<400> 45
   gaatgtgacc gtcccatgag 20
<210> 46
   <211> 28
   <212> DNA
   <213> Artificial Sequence
<220>
   <223> Primer
<400> 46
   caactgaata tgcagaaaaa agtacacc 28
<210> 47
   <211> 21
   <212> DNA
   <213> Artificial Sequence
<220>
   <221> misc difference
   <222> (1) .. (1)
   <223> N = biotinylated adenine
<223> Primer
<400> 47
   ngtagctggg actcacggtg t 21
<210> 48
   <211> 21
   <212> DNA
   <213> Artificial Sequence
<220>
   <221> misc difference
   <222> (1) ... (1)
   <223> N = biotinylated adenine
<223> Primer
<400> 48
   ngtagctggg actcacggtg c 21
<210> 49
   <211> 21
   <212> DNA
   <213> Artificial Sequence
<220>
   <221> misc difference
   <222> (1)...(1)
   <223> N = phosphorylated digoxigenin guanine
<223> Primer
<400> 49
   ncgccaccac tcccagctca t 21